(19) Europäisches Patentamt
European Patent Office
Office européen des brevets

(11) EP 4 239 083 A2

(12) EUROPEAN PATENT APPLICATION
published in accordance with Art. 153(4) EPC

(43) Date of publication:
06.09.2023 Bulletin 2023/36

(51) International Patent Classification (IPC):
C12Q 1/70 (2006.01)    C12Q 1/6825 (2018.01)

(21) Application number: 21886987.3

(52) Cooperative Patent Classification (CPC):
C12Q 1/6825; C12Q 1/70

(22) Date of filing: 02.11.2021

(86) International application number:
PCT/KR2021/015742

(87) International publication number:
WO 2022/092995 (05.05.2022 Gazette 2022/18)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(72) Inventors:
• KIM, V. Narry
  Seoul 08826 (KR)
• LEE, Sungyul
  Daejeon 34051 (KR)
• LEE, Young-suk
  Daejeon 34141 (KR)

(30) Priority: 02.11.2020 KR 20200144787

(74) Representative: J A Kemp LLP
80 Turnmill Street
London EC1M 5QU (GB)

(71) Applicants:
• Seoul National University R & DB Foundation
  Seoul 08826 (KR)
• Institute for Basic Science
  Yuseong-gu
  Daejeon 34126 (KR)

Remarks:
The complete document including Reference
Table(s) and the Sequence Listing(s) can be
downloaded from the EPO website

(54) **RNA INTERACTOME CAPTURING PROTOCOL AND ANTIVIRAL COMPOSITION DISCOVERED USING SAME**

(57) The present invention relates to an RNA interactome capturing protocol and an antiviral composition discovered using same. When used, the antiviral composition and pharmaceutical composition of the present invention can effectively inhibit viral infection and suppress viral proliferation in vivo after viral infection.

【FIG. 5c】

## Description

## Technical Field

This patent application claims priority to Korean Patent Application No. 10-2020-0144787 filed with the Korean Intellectual Property Office on November 02, 2020, the disclosure of which is incorporated herein by reference.

The present invention relates to an RNA interactome capture protocol and an antiviral composition discovered using the same.

## Background Art

Coronaviruses (CoVs) are a group of enveloped viruses with nonsegmented, single-stranded, positive-sense (+) RNA genomes, which belong to order Nidovirales, family Coronaviridae, and subfamily Coronavirinae (Lai and Cavanagh, 1997). They are classified into four genera: Alphacoronavirus and Betacoronavirus which exclusively infect mammals and Gammacoronavirus and Deltacoronavirus which primarily infect birds (Woo et al., 2012). Human CoVs such as Alphacoronavirus HCoV-229E and Betacoronavirus HCoV-OC43 have been known since the 1960s (Hamre and Procknow, 1966) as etiologic agents of the common cold. However, with the beginning of the 21st century, the world experienced the emergence of five novel human coronavirus species including highly pathogenic Severe acute respiratory syndrome coronavirus (SARS-CoV) in 2002 (Peiris et al., 2003), Middle East respiratory syndrome coronavirus (MERS-CoV) in 2012 (de Groot et al., 2013), and SARS-CoV-2 in 2019 (Zhou et al., 2020).

At the core of the coronavirus particle, the RNA genome is encapsulated in nucleocapsid (N) protein and surrounded by the viral membrane that contains spike (S) protein, membrane (M) protein, and envelope (E) protein (Lai and Cavanagh, 1997). The coronaviral RNA genome is ~30 kb which is the longest among RNA viruses and contains a 5'-cap structure and a 3' poly(A) tail (Bouvet et al., 2010; Lai and Stohlman, 1981). Upon cell entry, the genomic RNA (gRNA) acts as an mRNA to produce nonstructural proteins (nsps) that are required for viral RNA production (Perlman and Netland, 2009). The ORF1a encodes polypeptide 1a (ppla, 440-500 kDa) that is cleaved into 11 nsps. The -1 ribosomal frameshift occurs immediately upstream of the ORFIa stop codon, allowing translation of downstream ORF1b, yielding a large polypeptide (pp1ab, 740-810 kDa) that is cleaved into 15 nsps. Together, 16 different nsp fragments are generated to construct double-membrane vesicles (DMV) and mediate subsequent steps of viral RNA synthesis.

After this initial stage of viral translation, the gRNA is used as the template for the synthesis of negative-strand (-) RNA intermediates which in turn serve as the templates for positive-sense (+) RNA synthesis (Snijder et al., 2016; Sola et al., 2015). Ten different canonical (+) RNA species are produced from the SARS-CoV-2 genome, which include one full-length gRNA and nine subgenomic RNAs (sgRNAs) (Kim et al., 2020a). All canonical viral (+) RNAs share the common 5' end sequence called the leader sequence and the 3' end sequences. The sgRNAs are generated via discontinuous transcription which leads to the fusion between the 5' leader sequence and the "body" parts containing the downstream open reading frames (Sola et al., 2015) that encode structural proteins (S, E, M, and N) and accessory proteins (3a, 3c, 6, 7a, 7b, 8, and 9b) (Kim et al., 2020a).

To accomplish this, coronaviruses employ unique strategies to evade, modulate, and utilize the host machinery (Fung and Liu, 2019). For example, the gRNA molecules must be kept in an intricate balance between translation, transcription, and encapsulation by recruiting the right host RNA-binding proteins (RBPs) and forming specific ribonucleoprotein (RNP) complexes. As host cells counteract by launching RBPs such as RIG-I, MDA5, and Toll-like receptors (TLRs) to recognize and eliminate viral RNAs, the virus needs to evade the immune system using its components to win the arms race between virus and host. How such stealthy devices are genetically coded in this compact RNA genome is yet to be explored (Snijder et al., 2016). Thus, the identification of the RBPs that bind to viral transcripts (or the SARS-CoV-2 RNA interactome) is key to uncovering the molecular rewiring of viral gene regulation and the activation of antiviral defense systems.

Biochemical techniques for studying RNA-protein interactions have been developed (Ramanathan et al., 2019) with the advancement in protein-centric methods such as CLIP-seq (crosslinking immunoprecipitation followed by sequencing) (Ule et al., 2018). In CLIP-seq experiments, RNP complexes are crosslinked by UV irradiation within cells to identify direct RNA-protein interactions. The protein of interest is immunoprecipitated to identify the associated RNAs (Lee and Ule, 2018; Van Nostrand et al., 2020). More recently, RNA-centric methods have also been developed to profile the mRNA interactome and RNP complexes (Roth and Diederichs, 2015). After UV irradiation, the RNA of interest is purified with oligonucleotide probes and the crosslinked proteins are identified by mass spectrometry. For example, RAP-MS exhibits compelling evidence of highly confident profiling of proteins that bind to a specific RNA owing to a combination of long hybridization probes and harsh denaturing condition (Engreitz et al., 2013; McHugh et al., 2015).

**Disclosure of Invention**

**Technical Problem**

Leading to the present disclosure, intensive and thorough research conducted by the present inventors into the development of a process that facilitates analyzing the binding of RNA viruses, more specifically, the genomic RNA of coronaviruses to the virus itself and/or host proteins. As a result, the inventors completed the development of a powerful RNP capture protocol to define the repertoire of viral and host proteins related to the transcriptome of coronavirus, and utilized it to identify new targets for suppression of coronavirus.

Therefore, an aspect of the present disclosure is to provide an antiviral composition.

Another aspect of the present invention is to provide a pharmaceutical composition for preventing or treating coronavirus infection.

Another aspect of the present invention is to provide a method for screening coronavirus inhibitors.

Another aspect of the present invention is to provide a method for establishing an RNA interactome.

**Solution to Problem**

According to an aspect thereof, the present disclosure provides an antiviral composition comprising one or more proteins selected from the group consisting of LARP1, FUBP3, FUBP1, FAM120A, FAM120C, EIF4H, RPS3, RPS9, SND1, CELF1, RALY, CNBP, TRIM25, ZC3HAV1, PARP12, PPP1CA, HDLBP, CNBP, TIAL, UPF1, and SHFL as an active ingredient.

The present inventors have made intensive research efforts to develop a process that facilitates analyzing the binding of RNA viruses, more specifically, the genomic RNA of coronaviruses to the virus itself and/or host proteins. As a result, we completed the development of a powerful RNP capture protocol to define the repertoire of viral and host proteins related to the transcriptome of coronavirus, and utilized it to identify new targets for suppression of coronavirus.

One embodiment of the RNP capture protocol designed to derive a component having antiviral activity and a component having proviral activity of the present invention includes the following steps: (a) inducing the RNA-protein cross-linking by irradiating a sample containing target RNA and protein with UV light; (b) performing Denaturation by DNase treatment; (c) capturing denatured RNP complexes in a sequence-specific manner using a biotinylated antisense oligonucleotide probe pool (wherein the biotinylated antisense oligonucleotides included in the probe pool are designed based on the target RNA sequence in a consecutive manner, and have the same length ranging from 45 to 135 nt, preferably from 60 to 120 nt, more preferably from 75 to 105 nt, or from 80 to 100 nt, or at 90 nt and their starting positions are spaced at regular intervals to induce partial overlap between the oligonucleotides; (d) performing Digestion by on-bead trypsin treatment; and (e) obtaining interactome information through analysis of the digestion products.

Through the RNP capture protocol including the above steps, the present inventors were able to obtain detailed information about the interactome, which is a set of proteins that form crosslinks with the genomic RNA (gRNA) of the coronavirus. The term "interactome" in the present invention refers to the collective set of proteins that bind to a target RNA sequence, specifically to a genomic RNA sequence of the coronavirus. More specifically, for example, in the case of the "SARS-CoV-2 RNA interactome," it refers to the set of 109 proteins that were discovered through the RNP capture protocol.

The antiviral composition of the present disclosure comprises one or more proteins selected from the group consisting of LARP1, FUBP3, FUBP1, FAM120A, FAM120C, EIF4H, RPS3, RPS9, SND1, CELF1, RALY, CNBP, TRIM25, ZC3HAV1, PARP12, PPP1CA, HDLBP, CNBP, TIAL, UPF1, and SHFL as an active ingredient. The present inventors revealed through knock-down experiments that depletion of the above-described proteins leads to substantial upregulation of viral RNA, indicating that one or more proteins selected from the above-described protein group possess antiviral functions.

"LARP1" in the present disclosure is known to stabilizes 5' TOP mRNAs encoding ribosomal proteins and translation factors, which contain the 5' terminal oligopyrimidine (5' TOP) motif in the 5' UTR, and encodes ribosomal proteins and translation factors, and inhibits the translation of 5' TOP mRNA in response to metabolic stress in an mTORC1-dependent manner.

"FUBP3" in the present disclosure is known as a nuclear protein with four KH domains, which binds to the 3'UTR of cellular mRNAs regulating mRNA localization, while its connection to the lifecycle of coronavirus is unknown.

"FUBP1" in the present disclosure is a single-strnaded DNA-binding protein that binds to various DNA elements, including the FUSE (Far upstream element) located upstream of c-myc. However, its connection to the lifecycle of coronaviruses is not known.

"FAM120A" in the present disclosure is known to be involved in mRNA transport in the cytoplasm, and acts as a scaffolding protein that activates src family kinases and allows src family kinases to phosphorylate and activate PI3-kinase.

"FAM120C" in the present disclosure is a potential transmembrane protein, which is known to be encoded by a gene located in the region associated with intellectual disability and autism and functions.

"EIF4H" of the present disclosure, it is known to act as a cofactor of RNA helicase EIF4A together with EIF4B, and its depletion is known to lead to the formation of RNA granules.

"RPS3" of the present disclosure is a component of the 40S ribosomal subunit in eukaryotes, and it is known to be a multifunctional protein involved in DNA repair, apoptosis, and regulation of innate immune response to bacterial infections. However, its connection to viral inhibition is not known.

"RPS9" in the present disclosure is known as a component of the 40S ribosomal subunit in eukaryotes, and its Various expressions have been reported in colorectal cancer, but the correlation between its expression levels and the severity of the disease has not been established. Additionally, its connection to viral inhibition is also not known.

"SND1" of the present disclosure is known to act as an oncogene in the progression of various carcinomas, and in particular, it is known to promote tumor angiogenesis in human hepatocellular carcinoma through a novel pathway including NF-kappaB and miR-221.

"CELF1" of the present disclosure is a protein known to enhance cell migration, invasion, and chemical resistance by targeting ETS2 in colon cancer, and corresponds to an RNA-binding protein, but its connection to virus inhibition is not known.

"RALY" of the present disclosure is known as an RNA binding protein. Infectious mononucleosis produces anti-EBNA-1 antibodies that cross-react with several normal human proteins, and RALY is one such antigen and is known to be a member of the heterogeneous nuclear ribonucleoprotein gene family.

"CNBP" of the present disclosure is found in many tissues of the body, and is the most abundant protein in the heart and muscle (skeletal muscle) used for exercise. It regulates the activity of other genes, and plays an essential role in normal development before birth, especially the normal development of muscles.

"TRIM25" of the present disclosure is known as a protein that binds to the CARD domain of RIG-I.

"ZC3HAV1 (ZAP/PARP13)" of the present disclosure is a protein that has been reported to degrade HIV RNA by recognizing CpG and recruiting decay factors.

"PARP12" of the present disclosure is a substance that belongs to the ADP-ribosyl transferase family, and its function is not been clearly identified.

"PPP1CA" of the present disclosure is a protein that associates with over 200 regulatory proteins to form holoenzymes which dephosphorylate their biological targets with high specificity.

"HDLBP" of the present disclosure is a conserved protein that contains 14 KH domains and has been implicated in viral translation of dengue virus.

"CNBP" of the present disclosure is a CCHC-type zinc finger nucleic acid binding protein, and it is known to form bonds to DNA and RNA. It is known to functionally act in cap-dependent translation of ornithine decarboxylase mRNA, and it is known to function in the sterol-mediated transcriptional regulation.

"TIAL" of the present disclosure is a type of RNA-binding protein that contains three RNA recognition motifs (RRMs). It is known to bind to adenine- and uridine-rich elements in mRNA and pre-mRNA.

"UPF1" of the present disclosure is a protein that is part of a multiprotein complex after splicing as an exon junction complex involved in nuclear release of mRNA.

"SHFL" of the present disclosure is a protein known to be associated to Dengue virus and autoimmune peripheral neuropathy.

The above-mentioned proteins are not known to be associated with the inhibition or lifecycle of the coronavirus.

According to another aspect thereof, the present disclosure provides an antiviral composition comprising nucleic acids encoding one or more proteins selected from the group consisting of LARP1, FUBP3, FUBP1, FAM120A, FAM120C, EIF4H, RPS3, RPS9, SND1, CELF1, RALY, CNBP, TRIM25, ZC3HAV1, PARP12, PPP1CA, HDLBP, CNBP, TIAL, UPF1, and SHFL as an active ingredient.

A nucleotide sequence of the above-mentioned nucleic acid molecule encoding the proteins of the present disclosure is understood to be the nucleotide sequence encoding the amino acid sequence constituting the fusion protein, and is not limited to any specific nucleotide sequence. This is evident to a person skilled in the art because even if variations occur in the nucleotide sequence, there are cases where the mutated nucleotide sequence, when expressed as a protein, does not result in changes in the protein sequence. This is known as codon degeneracy. Therefore, the nucleotide sequence includes nucleotide sequences that encode functionally equivalent codons or the same amino acids (for example, due to codon degeneracy, there are six codons for arginine or serine), or codons that encode biologically equivalent amino acids.

As used herein, the term "nucleic acid" refers to comprehensively including DNA (gDNA and cDNA) and RNA molecules, and the nucleotide as a basic constituent unit in the nucleic acid molecule includes naturally occurring nucleotides, and analogues with modified sugars or bases (Scheit, Nucleotide Analogs, John Wiley, New York (1980); and Uhlman & Peyman, Chemical Reviews, 90:543-584(1990)).

Considering the above-described mutation having biologically equivalent activity, it should be construed that the nucleic acid molecules of the present disclosure encoding the amino acid sequences constituting the fusion proteins also include sequences showing substantial identity therewith. The substantial identity refers to a sequence showing at least 60%, more preferably at least 70%, still more preferably at least 80%, still more preferably at least 90% nucleotide, and most specifically at least 95% identity when the sequence of the present invention and any other sequence are correspondingly aligned as much as possible and the aligned sequence is analyzed using algorithms commonly used in the art. Methods of alignment for sequence comparison are known in the art. Various methods and algorithms for alignment are disclosed in Smith and Waterman, Adv. Appl. Math. 2:482(1981); Needleman and Wunsch, J. Mol. Bio. 48:443(1970); Pearson and Lipman, Methods in Mol. Biol. 24: 307-31(1988); Higgins and Sharp, Gene 73:237-44(1988); Higgins and Sharp, CABIOS 5:151-3(1989); Corpet et al., Nuc. Acids Res. 16:10881-90(1988); Huang et al., Comp. Appl. BioSci. 8:155-65(1992); and Pearson et al., Meth. Mol. Biol. 24:307-31(1994), but are not limited thereto.

In an embodiment of the present disclosure, the virus is a coronavirus.

As used herein, the term "coronavirus" refers to a collective term for RNA viruses belonging to the subfamily *Coronavirinae* in the family Coronaviridae. They cause respiratory and gastrointestinal infections in humans and animals and are easily transmitted through mucosal and droplet transmission. Recently, they have emerged as a prominent virus causing severe infections in humans. Coronaviruses are known to be responsible for a significant portion of common colds in humans and can lead to direct viral pneumonia, secondary bacterial pneumonia, viral bronchitis, or secondary bacterial bronchitis.

In an embodiment of the present disclosure, the coronavirus is selected from the group consisting of 229E, OC43, NL63, HKU1, MERS-CoV, SARS-CoV and SARS-CoV-2, but is not limited thereto. The coronaviruses correspond to variants of coronavirus that can infect humans and are known to share a significant portion of genetic information in their gRNA. By applying the antiviral composition of the present invention, replication of gRNA and protein synthesis of the coronaviruses can be inhibited, thereby suppressing virus proliferation and activation.

According to another aspect thereof, the present disclosure provides an antiviral composition comprising a recombinant vector that contains nucleic acids encoding one or more proteins selected from the group consisting of LARP1, FUBP3, FUBP1, FAM120A, FAM120C, EIF4H, RPS3, RPS9, SND1, CELF1, RALY, CNBP, TRIM25, ZC3HAV1, PARP12, PPP1CA, HDLBP, CNBP, TIAL, UPF1, and SHFL as an active ingredient.

As used herein, the term "vector" refers to a means for expressing a gene of interest in a host cell, which comprising a plasmid vector; cosmid vector; and viral vectors such as bacteriophage vectors, adenoviral vectors, retroviral vectors, and adeno-associated viral vectors, and the like.

In an embodiment of the present disclosure, in the vector of the present invention, the nucleic acid molecule encoding one or more proteins selected from the group consisting of LARP1, FUBP3, FUBP1, FAM120A, FAM120C, EIF4H, RPS3, RPS9, SND1, CELF1, RALY, CNBP, TRIM25, ZC3HAV1, PARP12, PPP1CA, HDLBP, CNBP, TIAL, UPF1 and SHFL is operatively linked to the promoter of the vector.

As used herein, the term "operatively linked" refers to a functional linkage between a nucleic acid expression regulatory sequence (e.g., promoters, signal sequences, or arrays of transcription factor binding sites) and another nucleic acid sequence, whereby Control sequences will control the transcription and/or translation of said other nucleic acid sequences.

The recombinant vector system of the present invention can be constructed by various methods known in the art, and a specific method thereof is disclosed in Sambrook et al., Molecular Cloning, A Laboratory Manual, Cold Spring Harbor Laboratory Press (2001), which is incorporated herein by reference.

The vector of the present invention may be typically constructed as a vector for cloning or a vector for expression. In addition, the vector of the present invention may be constructed by using a prokaryotic or eukaryotic cell as a host.

For example, in cases where the vector of the present invention is an expression vector and an eukaryotic cell is used as a host cell, a promoter derived from the genome of a mammalian cell (e.g., metallothionein promoter, β-actin promoter, human hemoglobin promoter, and human muscle creatine promoter) or a promoter derived from mammalian viruses (e.g., adenovirus late promoter, vaccinia virus 7.5K promoter, SV40 promoter, cytomegalovirus promoter, tk promoter of HSV, mouse mammary tumor virus (MMTV) promoter, LTR promoter of HIV, promoter of Moloney virus, Epstein-Barr virus (EBV), and Rous sarcoma virus (RSV)) may be used, and a polyadenylated sequence may be commonly used as the transcription termination sequence.

The vector of the present invention may be fused with the other sequences to facilitate the purification of the antibody expressed therefrom. Examples of the fusion sequence include, for example, glutathione S-transferase (Pharmacia, USA), maltose binding protein (NEB, USA), FLAG™ (IBI, USA), and 6×His (hexahistidine (SEQ ID NO: 23); QIAGEN™, USA).

Meanwhile, the expression vector of the present invention includes, as a selective marker, an antibiotic agent-resistant gene that is ordinarily used in the art, and may include resistant genes against ampicillin, gentamycin, carbenicillin, chloramphenicol, streptomycin, kanamycin, geneticin, neomycin, and tetracycline.

According to another aspect thereof, the present disclosure provides an antiviral composition comprising an expression inhibitor or activity inhibitor of one or more proteins selected from the group consisting of EIF3A, EIF3D, CSDE1, HNRNPM, YBX3, FMR1, RTCB, and NUFIP2.

"EIF3A" of the present disclosure is an RNA-binding component of the mammalian EIF3 complex and is an evolutionarily conserved protein along with EIF3B and EIF3C.

"EIF3D" of the present disclosure is known to interact with the mRNA cap and corresponds to a protein required for initiation of translation.

"CSDE1(Unr)" of the present disclosure is known to be required for IRES-dependent translation in human rhinovirus (*Picornaviridae*) and poliovirus (*Picornaviridae*).

"HNRNPM" of the present disclosure is a protein involved in the spliceosome mechanism through interaction with the CDC5L/PLRG1 spliceosome subcomplex, and the HNRNPM complex with nuclear IMP-3 is known to play an important role in efficient synthesis of CCND1, D3 and G1, and in the proliferation of human cancer cells.

"YBX3" of the present disclosure is known to inhibit influenza A virus by interacting with the viral ribonucleoprotein complex and impairing its function. However, its connection to coronavirus suppression is not identified.

"FMR1" of the present disclosure is a protein that binds to RNA and is associated with polysomes, and corresponds to a protein known to be associated with mRNA transport from the nucleus to the cytoplasm.

The "RTCB" of the present disclosure is an RNA ligase component of the E.coli RNA repair operon, and is a protein known to be able to catalyze *in vivo* tRNA splicing and HAC1 mRNA splicing.

"NUFIP2" of the present disclosure is an RNA-binding protein that exhibits cell cycle-dependent intracellular localization.

In an embodiment of the present disclosure, the expression inhibitor of the proteins is selected from the group consisting of miRNA, siRNA, shRNA, antisense oligonucleotide, CRISPRi, and combinations thereof that complementarily bind to the nucleic acid sequences encoding the proteins.

As used herein, the term "miRNA, siRNA or shRNA" of the present disclosure refers to a nucleic acid molecule that binds to mRNA transcribed from a target gene and inhibits translation of the mRNA in order to mediate RNA interference or gene silencing. Since the siRNA or shRNA can inhibit the expression of a target gene, it can be used in an efficient gene knockdown method or gene therapy method, and for the purpose of the present invention, it can be used to inhibit the expression of any one or more proteins selected from the group consisting of EIF3A, EIF3D, CSDE1, HNRNPM, YBX3, FMR1, RTCB and NUFIP2.

In an embodiment of the present disclosure, specific examples of siRNAs for EIF3A, EIF3D, CSDE1, HNRNPM, YBX3, FMR1, RTCB, and NUFIP2 of the present invention are as follows, and one or more siRNAs selected from the following siRNA sequences can be used to inhibit protein expression:

siEIF3A: 5'-GAGAAGACUUGGCGAUAGU-3'(SEQ ID NO: 1) / 5'-AGACAGAGAUUUAUUCGUA-3'(SEQ ID NO: 2) / 5'-GAAGAAAGGCAUAAUCGAU-3'(SEQ ID NO: 3) / 5'-UCACAAAGGUUCUAAAUUG-3'(SEQ ID NO: 4)

siEIF3D: 5'-GUUCAAGCCUAAUGAGUUU-3'(SEQ ID NO: 5) / 5'-CAAGAUAAGAGGUACACAA-3'(SEQ ID NO: 6) / 5'-AGACAAAGAUCGUCGGAAC-3'(SEQ ID NO: 7) / 5'-CGAAUGAGAUUUGCCCAGA-3'(SEQ ID NO: 8)

siCSDE1: 5'-AGACCGACGUGACAAAUUA-3'(SEQ ID NO: 9) / 5'-UCAAAUAGGAGAUAAGGUU-3'(SEQ ID NO: 10) / 5'-GAAAGAAAGAUCCGUCAAG-3'(SEQ ID NO: 11) / 5'-GGUGAGGUCUAUCCAUUUG-3'(SEQ ID NO: 12)

siHNRNPM: 5'-UGAAAUGGCAGUCACUUAA-3'(SEQ ID NO: 13) / 5'-GAAUGGAAGGCAUAGGAUU-3'(SEQ ID NO: 14) / 5'-GAUGAGAGGGCCUUACCAA-3'(SEQ ID NO: 15) / 5'-GAGAUUGACGUUCGAAUUG-3'(SEQ ID NO: 16)

siYBX3: 5'-GGAAGGGAGUCGUUACGCU-3'(SEQ ID NO: 17) / 5'-GAUGAAGGAUGGAGUCCCA-3'(SEQ ID NO: 18) / 5'-GCAAUAAAGUGGAAGACUA-3'(SEQ ID NO: 19) / 5'-GCACGGAAGACAAGAGAGU-3'(SEQ ID NO: 20)

siFMR1: 5'-CCAAAGAGGCGGCACAUAA-3'(SEQ ID NO: 21) / 5'-GAUGAUAAAGGGUGAGUUU-3'(SEQ ID NO: 22) / 5'-CACCAAAUCGUACAGAUAA-3'(SEQ ID NO: 23) / 5'-AAAGCUAUGUGACUGAUGA-3'(SEQ ID NO: 24)

siRTCB: 5'-GGAAUUGUUCAUCGAUCUA-3'(SEQ ID NO: 25) / 5'-GAACGGACACUGUUAGUAC-3'(SEQ ID NO: 26) / 5'-GGUGUCCGCUUGCUAAGAA-3'(SEQ ID NO: 27) / 5'-GUCAACCGCUCUUCCAUGA-3'(SEQ ID NO: 28)

siNUFIP2: 5'-AGGAAAGCUAGGCGCAAUA-3'(SEQ ID NO: 29) / 5'-GGGUGAUAUGCUUCGGAAA-3'(SEQ ID NO: 30) / 5'-AAUUAAGCCCUGCGAGAAU-3'(SEQ ID NO: 31) / 5'-AUGGUGAACUAAACGGUAA-3'(SEQ ID NO: 32)

As used herein, the term "antisense oligonucleotide" of the present invention refers to DNA, RNA, or derivatives thereof containing a nucleic acid sequence complementary to a specific mRNA sequence, which binds to a complementary sequence in mRNA and inhibits translation of mRNA into protein. For the purpose of the present invention, it can be used to inhibit the expression of any one or more proteins selected from the group consisting of EIF3A, EIF3D, CSDE1, HNRNPM, YBX3, FMR1, RTCB, and NUFIP2.

As used herein, the term "CRISPRi (Clustered regularly interspaced short palindromic repeats interference)" of the present disclosure refers to a system capable of inducing gene suppression at the target site by using dCas in which the DNA cleavage activity of Cas is deficient (removed), in order to use the CRISPR-Cas system for the purpose of regulating gene expression. CRISPR, a protein involved in the adaptive immune system in Eubacteria and Archaea, has been utilized as a gene editing tool that can easily and quickly create gene insertion and modification at the target site using Cas and sgRNA. However, due to the potential for off-target mutations, CRISPRi (CRISPR interference) was developed as a system to regulate gene expression instead of gene editing at the target site by utilizing dCas, which lacks DNA cleavage activity. By using CRISPRi, gene expression at specific target locations can be suppressed without inducing mutations, allowing for precise gene regulation without genetic modifications. CRISPRi is used as an efficient tool for DNA sequence-specific regulation of gene expression in various organisms and can be used to inhibit the expression of one or more proteins selected from the group consisting of EIF3A, EIF3D, CSDE1, HNRNPM, YBX3, FMR1, RTCB, and NUFIP2, for the purposes of the present invention.

In an embodiment of the present disclosure, the activity inhibitor of the proteins is selected from the group consisting of antibodies, aptamers, small molecule compounds, and combinations thereof that specifically bind to the proteins.

As used herein, the term "antibody" in the present disclosure refers to a proteinaceous molecule capable of specifically binding to the antigenic epitope of a protein or peptide molecule. The antibodies can be prepared by cloning each gene into an expression vector according to a conventional method to obtain a protein encoded by the gene, and then using the obtained protein by a conventional method.

In the present disclosure, the antibody can be utilized as a means capable of inhibiting the activity of the protein, by binding to the activated EIF3A, EIF3D, CSDE1, HNRNPM, YBX3, FMR1, RTCB, or NUFIP2 protein.

As specific examples, the antibodies of the present invention may include polyclonal antibodies, monoclonal antibodies, or antigen-binding molecules that can specifically bind to EIF3A, EIF3D, CSDE1, HNRNPM, YBX3, FMR1, RTCB, or NUFIP2, and includes any portion thereof that possesses antigen-binding properties, and also encompasses specialized antibodies, including humanized antibodies and other special antibodies, in addition to including all immunoglobulin antibodies. In addition, the antibody may be a complete form having two full-length light chains and two full-length heavy chains, as well as a form including functional fragments of the antibody molecule. A functional fragment of an antibody molecule refers to a fragment having at least an antigen-binding function, and may include Fab, F(ab'), F(ab') 2 and Fv.

As used herein, the term "aptamer" of the present disclosure refers to a nucleic acid molecule having binding activity to a specific target molecule. The aptamer may be RNA, DNA, modified nucleic acid, or a mixture thereof, and may be linear or cyclic forms. In general, it is known that the shorter the nucleotide sequence constituting the aptamer facilitates the easier the chemical synthesis and mass production, the better the cost advantage, the easier the chemical modification, the better the stability in vivo, and the lower the toxicity. In the present disclosure, the aptamer binds to the activated EIF3A, EIF3D, CSDE1, HNRNPM, YBX3, FMR1, RTCB, or NUFIP2 protein and can be utilized as a means to inhibit the activity of the protein.

As used herein, the term "small molecules" of the present disclosure refers to organic compounds having a small molecular weight, which bind to and regulate the function of biological polymers such as proteins. It may be of natural origin or artificially synthesized, and may inhibit protein functions or interfere with protein-protein interactions, but is not limited thereto.

For the purpose of the present invention, the small molecule compound includes, without limitation, any molecule that inhibits the activity of the activated EIF3A, EIF3D, CSDE1, HNRNPM, YBX3, FMR1, RTCB, or NUFIP2 protein, and specifically includes a molecule that binds to and inhibits the activity of activated EIF3A, EIF3D, CSDE1, HNRNPM, YBX3, FMR1, RTCB, or NUFIP2, but is not limited thereto.

According to another aspect thereof, the present disclosure provides a pharmaceutical composition for the prevention or treatment of coronavirus infection comprising the antiviral composition according to any one of the antiviral compositions mentioned above.

As used herein, the term "coronavirus infection" refers to a symptom or disease state caused by infection with a coronavirus selected from the group consisting of 229E, OC43, NL63, HKU1, MERS-CoV, SARS-CoV and SARS-CoV-2. Specifically, for example, it is known that infection with coronavirus is the cause of a significant portion of common colds in humans, and can cause direct viral pneumonia, secondary bacterial pneumonia, viral bronchitis, secondary bacterial bronchitis, and the like. It is known that there is, and by preventing coronavirus infection, the development of the above-mentioned diseases can be prevented. In addition, for a cold, viral pneumonia, or viral bronchitis, which is directly caused by a coronavirus, by suppressing viral growth, the above-mentioned diseases can be treated.

As used herein, the term "prevention" in the present specification refers to any activity that suppresses or delays viral infection itself or the onset of diseases associated with viral infection by administration of the pharmaceutical composition of the present invention.

As used herein, the term "treatment" refers to all activities that inhibit viral growth by administering the pharmaceutical composition of the present invention, thereby improving or beneficially altering symptoms and diseases caused by viral infection.

The pharmaceutical compositions of the present disclosure comprise a pharmaceutically acceptable carrier in addition to the active ingredient. The pharmaceutically acceptable carriers included in the pharmaceutical compositions of the disclosure are those commonly utilized in pharmaceutical formulations, such as lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil. In addition to the above components, the pharmaceutical compositions of the present disclosure may further comprise lubricants, wetting agents, sweeteners, flavoring agents, emulsifiers, suspending agents, preservatives, and the like. Suitable pharmaceutically acceptable carriers and formulations are described in detail in Remington's Pharmaceutical Sciences (19th ed., 1995).

A suitable dosage of the pharmaceutical composition of the present disclosure is variously prescribed by factors such as formulation method, administration method, patient's age, weight, sex, medical condition, food, administration time, administration route, excretion rate and response sensitivity. In one aspect, the dosage of the pharmaceutical composition of the present disclosure is preferably 0.001-1000 mg/kg (body weight) per day.

The pharmaceutical composition of the present disclosure can be administered orally or parenterally, and in the case of parenteral administration, it can be administered by topical application to the skin, intravenous injection, subcutaneous injection, intramuscular injection, intraperitoneal injection, transdermal administration, etc..

Considering that the coronavirus of the present disclosure is transmitted through mucosal transmission and droplet transmission, resulting in damage to the bronchi, lungs, and the like, the pharmaceutical compositions of the present disclosure are preferably administered to a patient by topical application to mucosal sites, oral administration, intravenous injection, subcutaneous injection, and the like.

The pharmaceutical composition of the present disclosure is prepared in unit dosage form by formulation using a pharmaceutically acceptable carrier and/or excipient according to a method that can be easily performed by those skilled in the art. or it may be prepared by incorporating into a multi-dose container. In this case, the formulation may be in the form of a solution, suspension or emulsion in an oil or aqueous medium, or may be in the form of an extract, powder, granule, tablet or capsule, and may additionally contain a dispersing agent or stabilizer.

According to another aspect of the present disclosure, the present disclosure provides a screening method for a coronavirus inhibitor comprising the following steps:

(a) contacting coronavirus-infected cells with a candidate substance for a coronavirus inhibitor; and
(b) analyzing whether the expression level of one or more proteins selected from the group consisting of EIF3A, EIF3D, CSDE1, HNRNPM, YBX3, FMR1, RTCB, and NUFIP2 is reduced compared to a control group of cells not treated with the candidate substance, to determine the candidate substance as a coronavirus inhibitor if the expression level of the proteins is reduced.

Since the EIF3A, EIF3D, CSDE1, HNRNPM, YBX3, FMR1, RTCB and NUFIP2 proteins of the present disclosure have proviral activity, they can be targets of newly discovered pharmaceutical compositions and can be used as drug candidates in cells transfected with coronavirus. By processing, it is possible to discover a coronavirus inhibitor by confirming the expression level of any one or more of the above-mentioned proteins.

According to another aspect of the present disclosure, the present disclosure provides a method for establishing an RNA interactome comprising the following steps:

(a) inducing the RNA-protein cross-linking by irradiating a sample containing target RNA and protein with UV light;
(b) performing Denaturation by DNase treatment;
(c) capturing denatured RNP complexes in a sequence-specific manner using a biotinylated antisense oligonucleotide probe pool; wherein the biotinylated antisense oligonucleotides included in the probe pool are designed based on the target RNA sequence in a consecutive manner, and have the same length ranging from 45 to 135 nt, and their starting positions are spaced at regular intervals to induce partial overlap between the oligonucleotides;
(d) performing Digestion by on-bead trypsin treatment; and
(e) obtaining interactome information through analysis of the digestion products.

In one embodiment of the present disclosure, wherein the probe pool comprises a first probe pool that specifically hybridizes with a gRNA molecule and a second probe pool that hybridizes with both gRNA and gsRNA.

**Advantageous Effects of Invention**

The features and advantages of the present disclosure are summarized as follows:

(a) The present disclosure provides an antiviral composition.
(b) The present disclosure provides a pharmaceutical composition for preventing or treating coronavirus infection.
(c) The present disclosure provides a method for screening coronavirus inhibitors.

(d) The present disclosure provides a method for establishing an RNA interactome.

(e) In the case of using the antiviral composition and pharmaceutical composition of the present disclosure, viral infection can be effectively inhibited, and viral proliferation in the body after viral infection can be inhibited.

**Brief description of drawings**

FIG. 1 shows comprehensive identification host and viral proteins that directly interact with the SARS-CoV-2 RNAs

FIG. 1a shows schematic of the modified RAP-MS protocol in SARS-CoV-2-infected Vero cells.

FIG. 1b shows schematic of two separate pools of 90-nt antisense oligonucleotides and their SARS-CoV-2 RNA coverage. The first probe set "Probe I" consists of 707 oligonucleotides that cover the unique region of gRNA, and the second probe set "Probe II" consists of 275 oligonucleotides that cover the common region of gRNA and sgRNAs.

FIG. 1c shows spectral count ratio of Probe I (x-axis) and Probe II (y-axis) experiments over no-probe control in SARS-CoV-2-infected Vero cells (n = 3 technical replicates). Host proteins are marked by grey circles, and viral proteins (n = 9) are marked and labelled in black. The mean spectral count ratio of Probe I and of Probe II experiments are marked by vertical and horizontal dashed lines, respectively.

FIG. 1d shows statistical analysis of the quantity of viral proteins over no-probe control (i.e. probe binding). Adjusted p-values (adj. P-value) of Probe I experiments and of Probe II experiments are shown in yellow and green, respectively. Threshold for statistical significance (adj. p-value < 0.01) is indicated by horizontal dashed lines.

FIG. 1e shows spectral count ratio of Probe I (x-axis) and Probe II (y-axis) experiments in SARS-CoV-2-infected Vero cells compared to RNP capture experiments in uninfected cells (n = 3 technical replicates). Statistically significant host proteins (n = 37, adj. P-value < 0.05) in both Probe I and Probe II experiments are marked by black circles. Of those, representative host proteins are labelled. The mean spectral count ratio of Probe I and of Probe II experiments are marked by vertical and horizontal dashed lines, respectively.

FIG. 1f shows statistical analysis of host proteins enriched in both Probe I and Probe II experiment (i.e. probe enriched). Adjusted p-values (adj. P-value) of Probe I experiments and of Probe II experiments are shown in yellow and green, respectively.

FIG. 1g shows Statistical analysis of host proteins enriched in only Probe I experiment and (H) in only Probe II experiment.

FIG.2 shows comparison of SARS-CoV-2 and HCoV-OC43 RNA interactome.

FIG. 2a shows schematic of time-course RNP capture experiment in HCoV-OC43-infected HCT-8 cells.

FIG. 2b shows LFQ intensity of abundant viral proteins identified in HCoV-OC43 RNP capture experiment at 12, 24, 36, and 48 hours post-infection (hpi).

FIG. 2c shows heatmap of normalized LFQ intensity (nLFQ) of HCoV-OC43 experiment of host proteins enriched in (left) both SARS-CoV-2 Probe I and Probe II experiments, (middle) only SARS-CoV- 2 Probe I experiment, and (right) only SARS-CoV-2 Probe II experiment. nLFQ is the log10 fold change over the median LFQ intensity across each probe set (i.e. Probe I or Probe II). A pseudo-value of $1 \times 10^6$ was added to handle missing values.

FIG. 2d shows the spectral count ratio of Probe I (x-axis) and Probe II (y-axis) experiments over no-probe control in HCoV-OC43-infected HCT-8 cells of 36 hpi. Host proteins are marked by grey circles, and viral proteins (n = 14) are marked and labelled in black. The mean spectral count ratio of Probe I and of Probe II experiments are marked by vertical and horizontal dashed lines, respectively.

FIG. 2e shows the spectral count ratio of Probe I (x-axis) and Probe II (y-axis) experiments in HCoV-OC43- infected HCT-8 cells compared to uninfected cells of 36 hpi. Statistically significant host proteins (n = 38, adj. P-value < 0.05) in both Probe I and Probe II experiments are marked by black circles. Of those, representative host proteins are labelled. The mean spectral count ratio of Probe I and of Probe II experiments are marked by vertical and horizontal dashed lines, respectively.

FIG. 2f shows upset plot of HCoV-OC43 Probe I-enriched (n = 67), HCoV-OC43 Probe II-enriched (n = 70), SARS-CoV-2 Probe I-enriched (n = 73), and SARS-CoV-2 Probe II-enriched (n = 72) proteins. 14 host proteins were enriched in all four experiments.

FIG. 3 shows molecular network of the SARS-CoV-2 RNA interactome.

FIG. 3a shows the physical-interaction network of SARS-CoV-2 RNA interactome but excluding ribosomal proteins and EIF3 proteins. Host factors are marked in red. Host and viral proteins are marked by circles and diamonds, respectively. The size of the network node is proportional to the number of network edges (i.e. degree) of the node. Largest connected component of the network was chosen for visualization and interpretation.

FIG. 3b shows Gene Ontology Biological Process term enrichment analysis of proteins retrieved in FIG. 3a.

FIG. 3c shows the subcellular localization of the SARS-CoV-2 RNA interactome and cellular mRNA interactomes. The number of proteins with localization information is shown in parentheses.

FIG. 4 shows the regulation of the SARS-CoV-2 RNA interactome.

FIG. 4a shows the differential expression analysis of ACE2-expressing A549 cells (A549-ACE2) after SARSCoV-2 infection (MOI = 2.0). mRNA fold change (FC) of -1 and 1 are marked by vertical dashed lines. 1% FDR is marked by a horizontal dashed line. Host genes of the SARS-CoV-2 RNA interactome are marked in red if differentially expressed (FDR < 1% and |FC| > 1) or otherwise in black.

FIG. 4b shows the comparison of mRNA fold change of SARS-CoV-2-infected (MOI = 2.0) A549-ACE2 cells after treatment of ruxolitinib. Host genes of the SARS-CoV-2 RNA interactome are marked in black.

FIG. 4c shows the differential expression analysis of normal human bronchial epithelial (NHBE) cells after INFβ treatment. Graph notations as in FIG. 4d.

FIG. 4d shows the MA plot of published mRNA-seq data of postmortem lung samples from COVID-19 patients and healthy lung tissue from uninfected individuals. Mean fold change is marked by a horizontal dashed line. Host genes of the SARS-CoV-2 RNA interactome are marked in red if differentially expressed (FDR < 5%) or otherwise in black. Other differentially expressed genes are in dark grey.

FIG. 5 shows the effects of RBP depletion on viral RNA abundance.

FIG. 5a shows a schematic of siRNA knockdown in SARS-CoV-2-infected Calu-3 cells.

FIG. 5b shows cell viability of siRNA-treated cells measured by MTT assay. Data are represented as mean ± s.e.m. (n = 3 independent experiments). siNC was used as negative control.

FIG. 5c shows the change in gRNA (amplicon nsp12, dark grey) and N sgRNA (amplicon N, light grey) levels after siRNA depletion were measured by RT-qPCR. Data are represented as mean ± s.e.m. (n = 3 or 6 independent experiments) . RNA levels in siNC-treated cells were used as negative control and marked by a horizontal dashed line. 18S rRNA was used for normalization. * $P < 0.05$, ** $P < 0.01$; two-sided Student's t-test.

FIG. 6 shows the experimental and statistical validation of modified RAP-MS experiments on SARS-CoV-2 RNAs.

FIG. 6a shows validation of antisense oligo-based RNA-protein (RNP) capture by silver staining and western blotting in SARS-CoV-2-infected Vero cells. SARS-CoV-2 nucleocapsid and RPS7 were used as control for rRNA and SARS-CoV-2 RNA (Probe I and Probe II) RNP capture experiments. CL: cross-linked.

FIG. 6b shows the RNA yield of antisense oligo-based RNA-protein (RNP) capture (rRNA, Probe I, and Probe II) measured by RT-qPCR (n = 2 technical replicates). Note that the SARS-CoV-2 N sgRNA qPCR primer targets the shared region of gRNA and sgRNAs, and gRNA qPCR primer targets nsp12.

FIG. 6c shows the protein domain enrichment analysis of host factors identified after statistical comparison over no-probe control experiment (i.e. Probe I and Probe II binding). Threshold for statistical significance (P-value < 0.05) is indicated by vertical dashed lines.

FIG. 6d shows the validation of RNP capture by silver staining in SARS-CoV-2-infected and uninfected Vero cells.

FIG. 6e shows the distribution of peptide length with "on-bead" digestion in SARS-CoV-2-infected and uninfected Vero cells (n = 3 technical replicates).

FIG. 6f shows Gene Ontology (GO) Biological Process (BP) term enrichment analysis of 37 host proteins statistically enriched in both Probe I and Probe II experiments.

FIG. 7 shows the experimental and statistical validation of RNP capture on HCoV-OC43 RNAsresults of RNP capture for HCoV-OC43 RNA.

FIG. 7a shows the relative HCoV-OC43 gRNA (amplicon nsp12) level measured by RT-qPCR and FIG. 7b shows the HCoVOC43 N protein level measured by western blotting for multiple viral loads ($1\times$, $5\times$, and $25\times$) across post-infection timepoints of 12, 24, 36, and 48 hours. RNA levels are relative to gRNA levels at 12 hours after 1x viral load infection.

FIG. 7c shows the confirmation of RNP capture by silver staining in HCoV-OC43-infected HCT-8 cells.

FIG. 7d shows the Protein domain enrichment analysis of host factors identified after statistical comparison over no-probe control experiment (i.e. Probe I and Probe II binding) for each post-infection timepoint RNP capture. Threshold for statistical significance (P-value < 0.05) is indicated by vertical dashed lines.

FIG. 7e shows the LFQ intensity of viral proteins identified in HCoV-OC43 RNP capture at 12, 24, 36, and 48 hours post-infection (hpi) not shown in FIG. 2b.

FIG. 7f shows Gene Ontology (GO) Biological Process (BP) term enrichment analysis of the 38 host proteins statistically enriched in both OC43 Probe I and Probe II experiments. txn: transcription

FIG. 8 shows the SARS-CoV-2 RNA interactome in the context of the physical-interaction network and gene-regulatory network

FIG. 8a shows the physical-interaction network of host and viral proteins enriched in both SARS-CoV-2 Probe I and Probe II experiments which are designated in red. Host and viral proteins are marked by circles and diamonds, respectively. The size of the network node is proportional to the number of network edges (i.e. degree) of the node. Largest connected component of the network was chosen for visualization and interpretation.

FIG. 8b shows the GO BP term enrichment analysis of host proteins retrieved in FIG. 8a.

FIG. 8c shows the differential expression analysis of published proteomic data of HeLa cells after 24 hours of INFβ stimulation. Protein-level fold change (FC) of -1 and 1 are marked by vertical dashed lines. 1% FDR is marked by a horizontal dashed line. Host proteins of the SARS-CoV-2 RNA interactome are marked in red if differentially expressed (FDR < 1% and |FC| > 1) or otherwise in black.

FIG. 9 shows the experimental confirmation of siRNA knockdown in Calu-3 cells.

FIG. 9a shows the results of siRNA knockdown confirmation by RT-qPCR in Calu-3 cells and FIG. 9b shows the results of siRNA knockdown confirmation by RT-qPCR in SARS-CoV-2-infected Calu-3 cells, 24 hours after infection at 0.05 MOI. Data are represented as mean ± s.e.m. (n = 3 independent experiments). 18S rRNA was used for normalization.

## Mode for Carrying Out the Invention

Hereinafter, the present disclosure will be described in more detail through examples. These examples are only for explaining the present disclosure in more detail, and it will be apparent to those skilled in the art that the scope of the present disclosure is not limited by these examples according to the gist of the present disclosure.

## Example

## Reagent and Resource

| REAGENT or RESOURCE | SOURCE | IDENTIFIER |
|---|---|---|
| **Antibodies** | | |
| Anti-SARS Coronavirus Nucleocapsid antibody | Invitrogen | Cat#PA1-41098 |
| Anti-RPS7 antibody | Abcam | Cat#ab57637 |
| Anti-Coronavirus antibody, OC-43 strain, clone 541-8 | Merck | Cat#MAB9012 |
| Anti-GAPDH antibody (6C5) | Abcam | Cat#ab8245 |
| **Virus Strains** | | |
| SARS-CoV-2 | National Culture Collection for Pathogens, Korea National Institute of Health, Korea | NCCP 43326 |
| HCoV-OC-43 | Korea Bank for Pathogenic Viruses | KBPV-VR-8 |
| **Chemicals and reagents** | | |
| Antibiotic-Antimycotic | Gibco | Cat#15240-062 |
| TRIzol LS | Invitrogen | Cat#10296-028 |
| DMEM, High glucose | Welgene | Cat#LM001-05 |
| RPMI 1640, HEPES | Welgene | Cat#LM011-03 |
| FBS | Gibco | Cat#10082147 |
| TURBO DNase I | Invitrogen | Cat#AM2239 |
| QiAquick PCR purification kit | QIAGEN | Cat#28106 |
| RNase-Free DNase Set | QIAGEN | Cat#79254 |
| RNeasy Mini Kit | QIAGEN | Cat#74106 |
| RevertAid Reverse Transcriptase | Thermo Scientific | Cat#EP0442 |
| MEGAscript T7 Transcription Kit | Invitrogen | Cat#AMB13345 |
| AMPure XP | Beckman | Cat#A63881 |
| RNAClean XP | Beckman | Cat#A63987 |
| SUPERase•In RNase Inhibitor | Invitrogen | Cat#AM2696 |
| Streptavidin magnetic beads | New England Biolabs | Cat#S1420S |

| Proteinase K | Roche | Cat#03 115 879 001 |
|---|---|---|
| Lithium chloride 8 M solution | Sigma | Cat#L7026 |
| Lithium dodecyle sulfate | Sigma | Cat#L9781 |
| Urea | Sigma | Cat#U6504 |
| Poly(ethylene glycol) | Sigma | Cat#P3015 |
| Iodoacetamide | Sigma | Cat#I1149 |
| Sodium hydroxide solution | Sigma | Cat#72068 |
| Ammonium bicarbonate | Sigma | Cat#09830 |
| DTT | Thermo Scientific | Cat#R0861 |
| Acetic acid (glacial) 100% | Merck | Cat#100063 |
| Tris-Cl pH 7.0 | Invitrogen | Cat#AM9851 |
| Tris-Cl pH 8.0 | Invitrogen | Cat#AM9856 |
| EDTA (0.5 M), pH 8.0, RNase-free | Invitrogen | Cat#AM9261 |
| GlycoBlue | Invitrogen | Cat#AM9516 |
| PowerSYBR Green PCR Master Mix | Applied Biosystems | Cat#AB4367659 |
| KAPA HiFi Hot Start ReadyMix PCR kit | Roche | Cat#KK2602 |
| Magnetic stand | Invitrogen | Cat#12321D |
| DEPC-treated Water | Invitrogen | Cat#4387937 |
| Pierce Trypsin Protease, MS Grade | Thermo Scientific | Cat#90058 |
| Benzonase® Nuclease | Millipore | Cat#E1014 |
| Direct-zol RNA Miniprep | Zymo Research | Cat#R2051 |
| HiPPR Detergent Removal Spin Columns, 0.1 mL | Thermo Scientific | Cat#88305 |
| HyperSep C18 Cartridges | Thermo Scientific | Cat#60108 |
| Novex WedgeWell 10 to 20%, Tris-Glycine, 1.0 mm, Mini Protein Gel, 15-well | Invitrogen | Cat#XP10205BOX |
| PageRuler Plus Prestained Protein Ladder | Thermo Scientific | Cat#26619 |
| Lipofectamine RNAiMAX Transfection Reagent | Invitrogen | Cat#13778150 |
| **Critical Commercial Assays** | | |
| EzWay Protein-Silver Staining Kit | KOMA Biotech | Cat#K14040D |
| CellTiter 96® Non-Radioactive Cell Proliferation Assay (MTT) | Promega | G4000 |

| Experimental Models: Cell Lines | | |
|---|---|---|
| Vero | ATCC | Cat#CCL-81 |
| HCT-8 | KCLB | 10244 |
| Calu-3 | KCLB | 30055 |
| **Oligonucleotides** | | |
| The oligonucleotides used in this study were listed in Table S6 | This study | N/A |
| ON-TARGETplus Human CELF1 siRNA SMARTpool | Horizon Discovery | L-020166-00 |
| ON-TARGETplus Human CNBP siRNA SMARTpool | Horizon Discovery | L-019778-00 |
| ON-TARGETplus Human CSDE1 siRNA SMARTpool | Horizon Discovery | L-015834-00 |
| ON-TARGETplus Human EIF3A siRNA SMARTpool | Horizon Discovery | L-019534-00 |
| ON-TARGETplus Human EIF3D siRNA SMARTpool | Horizon Discovery | L-017556-00 |
| ON-TARGETplus Human EIF4H siRNA SMARTpool | Horizon Discovery | L-013054-00 |
| ON-TARGETplus Human FAM120A siRNA SMARTpool | Horizon Discovery | L-014186-01 |
| ON-TARGETplus Human FAM120C siRNA SMARTpool | Horizon Discovery | L-032024-00 |
| ON-TARGETplus Human FUBP3 siRNA SMARTpool | Horizon Discovery | L-029452-01 |
| ON-TARGETplus Human G3BP1 siRNA SMARTpool | Horizon Discovery | L-012099-00 |
| ON-TARGETplus Human HDLBP siRNA SMARTpool | Horizon Discovery | L-019956-00 |
| ON-TARGETplus Human KHDRBS1 siRNA SMARTpool | Horizon Discovery | L-020019-00 |
| ON-TARGETplus Human LARP1 siRNA SMARTpool | Horizon Discovery | L-027187-00 |
| ON-TARGETplus Human MATR3 siRNA SMARTpool | Horizon Discovery | L-017382-00 |
| ON-TARGETplus Human MOV10 siRNA SMARTpool | Horizon Discovery | L-014162-00 |
| ON-TARGETplus Human NONO siRNA SMARTpool | Horizon Discovery | L-007756-01 |
| ON-TARGETplus Human PARP12 siRNA SMARTpool | Horizon Discovery | L-013740-00 |
| ON-TARGETplus Human RALY siRNA SMARTpool | Horizon Discovery | L-012392-01 |
| ON-TARGETplus Human RPS3 siRNA SMARTpool | Horizon Discovery | L-013607-00 |
| ON-TARGETplus Human RPS9 siRNA SMARTpool | Horizon Discovery | L-011131-00 |
| ON-TARGETplus Human SHFL (C19orf66) siRNA SMARTpool | Horizon Discovery | L-020478-02 |
| ON-TARGETplus Human SND1 siRNA SMARTpool | Horizon Discovery | L-010657-01 |
| ON-TARGETplus Human SYNCRIP siRNA SMARTpool | Horizon Discovery | L-016218-00 |
| ON-TARGETplus Human TRIM25 siRNA SMARTpool | Horizon Discovery | L-006585-00 |

| ON-TARGETplus Human UPF1 siRNA SMARTpool | Horizon Discovery | L-011763-00 |
| ON-TARGETplus Human ZC3HAV1 siRNA SMARTpool | Horizon Discovery | L-017449-01 |
| **Other: Instrument** | | |
| Spectrolinker™ XL-1500 UV Crosslinker | Spectronics | |
| BIO-LINK BLX-254 UV Crosslinker | Vilber Lourmat | |
| Orbitrap Fusion Lumos Tribrid Mass Spectrometer | Thermo Scientific | |
| UltiMate 3000 RSLCnano System | Thermo Scientific | |
| QuantStudio 3 Real-Time PCR Instrument | Applied Biosystems | |

## Methods

### Example 1: Preparation of antisense oligonucleotide templates

By scanning the genomic RNAs of SARS-CoV-2 (NCBI RefSeq accession NC_045512.2) and HCoV-OC43 (GenBank accession AY391777.1) from head to tail, partially overlapping 90 nt tiles were enumerated. These tiles were designed to have 30 nt spacing, so adjacent tiles share a subsequence of 60 nt. To avoid ambiguous targeting, tiles were aligned to the human transcriptome (version of Oct 14, 2019) using bowtie 2 (Langmead and Salzberg, 2012) and multi-mapped sequences were discarded. To prepare biotinylated antisense oligonucleotides (ASOs) in bulk, the sequence elements for in vitro transcription (IVT), reverse transcription (RT)and PCR were added to the 90 nt tiles. The T7 promoter (5'-TAA TAG GAC TCA CTA TAG GG-3') and a pad for RT priming (5'-TGG AAT TCT CGG GTG CCA AGG-3') were added to the head and tail of each tile, respectively. We grouped ASOs into two sets for each viral genome: "Probe I" targets the unique region of genomic RNA ([265:21553] of NC_045512.2; [:21506] of AY391777.1) and "Probe II" aims at both genomic and subgenomic RNAs ([21562:] of NC_045512.2; [21506:] of AY391777.1) . The templates of four ASO groups have distinct PCR primer binding sites on both ends. Accordingly, each ASO set can be selectively amplified from a single mixture. The final ASO templates (167 nt) were prepared via the oligo pool synthesis service of Genscript and stored at -80°C. The ASO templates used in this study are listed in Table 2.

### Example 2: Mass production of biotin-labeled ASO

ASO templates were amplified using KAPA HiFi HotStart ReadyMix (Roche) and PCR primers for an ASO pool. PCR products were purified by QIAquick PCR purification kit (QIAGEN). RNA intermediates were then transcribed using 5X MEGAscript T7 transcription kit (Invitrogen), and DNA templates were degraded by TURBO DNase (Invitrogen). To clean up enzymes and other reagents, 1.8× reaction volume of AMPure XP (Beckman) was applied and polyethylene glycol was added to be final 20%. The size selection was carried out according to the manufacturer's protocol. Biotinylated ASOs were synthesized by RevertAid Reverse Transcriptase (Thermo Scientific) and 5' biotin-TEG primer. RNA intermediates were hydrolyzed at 0.1 M NaOH and neutralized with acetic acid. Finally, ASO purification was performed in the same manner as IVT RNA selection. The primer sequences used for PCR and reverse transcription are listed in Table 6.

[Table 6]

| No | Name | 5' - Sequence - 3' |
| --- | --- | --- |
| **Probe PCR and RT primers** | | |
| 1 | SARS2-ProbeI_fwd | CTGATGAGGGAGAAACAT |
| 2 | SARS2-ProbeI_rev | TCCAAGGGTCTTTTAGCC |
| 3 | SARS2-ProbeII_fwd | TAGAAAACGATAGAAGTA |
| 4 | SARS2-ProbeII_rev | CTGGAGCGATTGTTATTG |
| 5 | OC43-ProbeI_fwd | TGTGAGAGATTCCCTTCG |
| 6 | OC43-ProbeI_rev | AATGAAGGTTTCTTGGTA |
| 7 | OC43-ProbeII_fwd | GTATTAGAGTTTCCCCTC |
| 8 | OC43-ProbeII_rev | TTTACCATGAGATTTAGC |
| 9 | rRNA_fwd | GAGGACTAGGAACAGACG |
| 10 | rRNA_rev | GACATCTAAATCTTCCAT |
| 11 | Biotin RT | /5BiotinTEG/CCTTGGCACCCGAGAATTCCA |

| No | Name | 5' - Sequence - 3' |
|----|------|--------------------|
| **qPCR primers** | | |
| 1 | PARP12_fwd | CCTGGTCTATGGCACAACTAAA |
| 2 | PARP12_rev | GGGCCTGGAAACTTGGTATT |
| 3 | FUBP3_fwd | AATGGGAGGAGGCAGTATAGA |
| 4 | FUBP3_rev | GAATCCTCACACCAGCATCA |
| 5 | RPS9_fwd | CCTGGGCCTGAAGATAGAGG |
| 6 | RPS9_rev | TGCTTGCGGACCCTGATATG |
| 7 | ZC3HAV1_fwd | CAGGTTGACTCTCTCTGGAATG |
| 8 | ZC3HAV1_rev | GAGCCCAAATGGGCAATTAAC |
| 9 | EIF4H_fwd | TTCAGGGCGACATAGATGCT |
| 10 | EIF4H_rev | GCAATGTCCACACGAAGTGA |
| 11 | FAM120A_fwd | GGCGTCCAAGAGGAGTTATTT |
| 12 | FAM120A_rev | GAAGCAGCATAAGGAGGTCTG |
| 13 | FAM120C_fwd | TGCACTGCTAGGTAACCACA |
| 14 | FAM120C_rev | GTCACAGGGAGGAAGAACCA |
| 15 | SND1_fwd | TCAGCGAGGAAGCTACACTT |
| 16 | SND1_rev | TGTGCAGCCAGCCGATAAA |
| 17 | CELF1_fwd | CTACTCGGGTATCCAGCAATATG |
| 18 | CELF1_rev | TTCCAGCAGCACCAATACTC |
| 19 | TRIM25_fwd | CAGACCTTGAAGGAGGAGATTG |
| 20 | TRIM25_rev | GGGATGTAGACTGGCTTTGTT |
| 21 | RALY_fwd | CCTGACACAAACCCTACATCTC |
| 22 | RALY_rev | TGTCACTGGAGTACGCTCTAT |
| 23 | RPS3_fwd | GCAAGATGGCAGTGCAAATATC |
| 24 | RPS3_rev | GCTCCCGAGTAAGAAACTCATT |
| 25 | HDLBP_fwd | CAGGACCTGATCACCATCATT |
| 26 | HDLBP_rev | GGAGTCTTCCACCACATTATCC |
| 27 | SHFL_fwd | GGAGAAGTTTCATGGGAAGGT |
| 28 | SHFL_rev | GGGCATCCAGATCGTTACTTAG |
| 29 | CNBP_fwd | GCAGGGTTCTGTCAGTTCAT |
| 30 | CNBP_rev | TTCTTGCTTGCTCAGGACTATT |
| 31 | MATR3_fwd | TCTCAACCCAGTGCTTGATTAT |
| 32 | MATR3_rev | CATCCCTACACCTTTCTCCATC |
| 33 | UPF1_fwd | CTCAACAACCTGCGTGAGAG |
| 34 | UPF1_rev | CTGGTCATGGGTCTGGAAGT |

| No | Name | 5' - Sequence - 3' |
|---|---|---|
| 35 | G3BP1_fwd | CTCAGCCGCGTAGGTTGAAT |
| 36 | G3BP1_rev | AGACTGCATCTGCTGGCTTT |
| 37 | KHDRBS1_fwd | GATACCTTTGCCTCCACCTC |
| 38 | KHDRBS1_rev | CCCTTGACTCTGGCTGTAATAG |
| 39 | SYNCRIP_fwd | CGATACCATCGGACAGGATTTC |
| 40 | SYNCRIP_rev | ATGGGCTCTTCAGTACCATTTC |
| 41 | NONO_fwd | TGCCATAGTGCATCCCTTAC |
| 42 | NONO_rev | CAATGACTACAGCCCTCTCTAC |
| 43 | EIF3A_fwd | TGCCCAGCAAGCTTTCAAAT |
| 44 | EIF3A_rev | GCGCTGAATCTGCGATAAGT |
| 45 | MOV10_fwd | ATGGTGTGGATGTGGAAGTC |
| 46 | MOV10_rev | AGAGGTGAGTGAGGGTAACA |
| 47 | CSDE1_fwd | ATGTGGCAACTCTGAAGGATAA |
| 48 | CSDE1_rev | GGCTATCAACATCACCAGAGAA |
| 49 | EIF3D_fwd | GTGGAGGACGACATGGATAAG |
| 50 | EIF3D_rev | GTGCTCACAACGGACAATAAG |
| 51 | LARP1_fwd | GCTGCCTTAATGAGCGGAAA |
| 52 | LARP1_rev | AAGAAGGACCAGAAGCGGAA |
| 53 | 18S_rRNA_fwd | CACGGACAGGATTGACAGATT |
| 54 | 18S_rRNA_rev | GCCAGAGTCTCGTTCGTTATC |
| 55 | 28S_rRNA_fwd | TTAGACCGTCGTGAGACAGG |
| 56 | 28S_rRNA_rev | CCTCAGCCAAGCACATACAC |
| 57 | OC43_gRNA_fwd | CACTGTTGCTGGTGTTTCCA |
| 58 | OC43_gRNA_rev | GCGGCGTAACATATCATCCC |
| 59 | SARS-CoV-2_nsp12_fwd | CAGAGAGCTAGGTGTTGTAC |
| 60 | SARS-CoV-2_nsp12_rev | AAGCACGTAGTGCGTTTATC |
| 61 | SARS-CoV-2_N_fwd | GGCCAGAAGCTGGACTTCCC |
| 62 | SARS-CoV-2_N_rev | AGGATTGCGGGTGCCAATGT |

**Example 3: Compilation of proteome databases**

The Uniprot reference proteome sets for human (UP000005640, canonical, SwissProt) and African green monkey (Chlorocebus sabaeus; UP000029965, canonical, SwissProt and TrEMBL) were used to identify host proteins in each mass spectrometry experiment (version 03/21/2020) (UniProt Consortium, 2019). The reference proteome set for the Severe acute respiratory syndrome coronavirus 2 (SARS-CoV-2) was manually curated largely based on the NCBI Reference Sequence (NC_045512.2) and related literature of other accessory proteins (e.g. ORF3b, ORF9b and ORF9c). The reference proteome set for the Human coronavirus OC43 (HCoV-OC43) was compiled based on the Uniprot Swiss-Prot proteins for HCoV-OC43 (taxonomy:31631) except for HCoV-OC43 Protein I which was separated into Protein Ia and Protein Ib (or N2) (Vijgen et al., 2005).

**Example 4: Cell culture, transfection and virus infection**

Virus experiments were carried out in accordance with the biosafety guideline by the Korea Centers for Disease Control & Prevention (KCDC). The Institutional Biosafety Committee of Seoul National University (SNUIBC) approved the protocols used in this study, including SNUIBC-R200331-1-1 for BL2 experiments and SNUIBC-200508-1 for BL3 experiments. Vero and HCT-8 cells were maintained in DMEM (Welgene) and RPMI 1640 (Welgene) respectively, both with 1X Antibiotic-Antimycotic (Gibco) and 10% FBS (Gibco) and cultured in CO2 incubator with 5% CO2 at 37 °C. For SARS-CoV-2 infection, $7 \times 10^6$ Vero cells were plated in T-175 flasks 24 hours before infection. Cells were washed with serum-free media and incubated with 5 mL virus-diluted media for 30 minutes at 0.1 MOI, as determined by plaque assay. After infection, virus containing media was replaced with reduced-serum media (2% FBS) and cultured until the harvest. For HCoV-OC43 infection, a similar protocol was used except for incubation temperature lowered to 33 °C. For siRNA transfection, $3.5 \times 10^5$ Calu-3 cells, maintained in DMEM with 1X Antibiotic-Antimycotic and 10% FBS in CO2 incubator with 5% CO2 at 37 °C, were plated in 12 well plate and final 50 nM siRNAs were reverse-transfected using Lipofectamine RNAiMAX (Invitrogen) and ON-TARGETplus SMARTpool siRNAs (Horizon Discovery). Cell viability after siRNA knockdown was measured by splitting 1/100th of cells from uninfected cells, 48 hours after transfection into 96 well plates in triplicates and cell number was measured by MTT assay (Promega) at 4 hours after addition of tetrazolium dye.

**Example 5: RNA purification and RT-qPCR**

For total RNA purification from virus-infected cells, 1 mL TRIzol LS (Invitorgen) were added to media-removed cell monolayers per single well of 12 well plates followed by on-column DNA digestion and purification (Zymo Research). For RNA purification from RNP capture sample, bead-captured RNAs were digested with 100 ng Proteinase K (PCR grade, Roche) and incubated at 37°C for 1 hour, followed by RNA isolation by TRIzol LS with GlycoBlue (Invitrogen). 1-5 μg RNA were reverse-transcribed using RevertAid transcriptase (Thermo Scientific) and random hexamer. qPCR was performed with primer pairs listed in Table S6 and PowerSYBR Green (Applied Biosystems) and analyzed with QuantStudio 5 (Thermo Scientific).

**Example 6: Modified RNA antisense purification coupled with mass spectrometry (RAP-MS)**

Virus infected cells were detached from culture vessels by trypsin and cell pellets were resuspended with ice-cold PBS. 12 mL cell suspensions were dispersed in 150 mm dishes to irradiate 254 nm UV for 2.5 J/cm2 using BIO-LINK BLX-254 for SARS-CoV-2 or 0.8 J/cm2 using Spectrolinker XL-1500 for HCoV-OC43. UV-crosslinked cells were pelleted by centrifugation and resuspended in TURBO DNase solution (150 Units per flask) and incubated at 37°C for 30 minutes. DNA digested cells were supplemented with equal volume of pre-heated 2X lysis buffer (40 mM Tris-Cl at pH 7.5, 1 M LiCl, 1% LDS, 2 mM EDTA, 10 mM DTT and 8 M urea) and denatured by incubating at 68°C for 30 minutes. Per replicate, cell lysate from 1 flask (T-175) were mixed with 20 μg biotin probe pools (Probe I or Probe II) and hybridized by incubating at 68°C for 30 minutes in final 1 mL volume. Biotin-labeled RNP lysates were supplemented with streptavidin beads (1 mL per replicate, New England Biolabs) and captured by rotating at room temperature overnight. Probe-enriched RNP beads were washed with 1X lysis buffer twice and transferred to fresh tubes, followed by final wash with detergent-free wash buffer (20 mM Tris-Cl at pH 7.5, 0.5 M LiCl, 1 mM EDTA). 1/10th of beads were set aside for assessment of RNA contents by RT-qPCR and another 1/10th of beads were used for silver staining (KOMA biotech). The remaining 8/10th of beads were digested with 100 units of Benzonase nuclease (Millipore) at 37°C for 1 hour. For on-bead peptide digestion, nuclease treated beads were suspended to final 8 M urea and reduced with 10 mM dithiothreitol (DTT), alkylated with 40 mM iodoacetamide (IAA) for 1 hour each at 37°C, and diluted with 50 mM ammonium bicarbonate (ABC) to final 1 M urea. These bead suspensions were supplemented with 300 ng Trypsin (Thermo Scientific, MS grade) and 1 mM CaCl2 and digested overnight at 37°C. Peptide solutions were separated from magnetic beads and further processed with HiPPR detergent removal spin columns (Thermo Scientific) and desalted by reverse phase C18 ziptip (Millipore). After the clean up and dry down, the samples were reconstituted with 20 pL of 25 mM ammonium bicarbonate buffer for LC-MS/MS analysis.

**Example 7: LC-MS/MS analysis**

LC-MS/MS analysis was carried out using Orbitrap Fusion Lumos Tribrid MS (Thermo Fisher Scientific) coupled with nanoAcquity UPLC system (Waters). Both analytical capillary columns (100 cm × 75 um i.d.) and the trap columns (3 cm × 150 um i.d) were packed in-house with 3 um Jupiter C18 particles (Phenomenex, Torrance). The long analytical column was placed in a column heater (Analytical Sales and Services) regulated to a temperature of 45°C. The LC flow rate was 300 nL/min and the 100-min linear gradient ranged from 95% solvent A (H2O with 0.1% formic acid (Merck)) to 40% solvent B (100% acetonitrile with 0.1% formic acid). Precursor ions were acquired at 120 K resolving power at m/z 200 and the isolation of precursor for MS/MS analysis was performed with a 1.4 Th. Higher-energy collisional dissociation (HCD) with 30% collision energy was used for sequencing with a target value of 1E5 ions determined by automatic gain control. Resolving power for acquired MS2 spectra was set to 30 K at m/z 200 with 120 ms maximum injection time.

Mass spectrometric raw data files were processed for Label-Free Quantification with MaxQuant (version 1.6.15.0) (Cox and Mann, 2008) using the built-in Andromeda search engine (Cox et al., 2011) at default settings with a few exceptions. Briefly, for peptide-spectrum match (PSM) search, cysteine carbamidomethylation was set as fixed modifications, and methionine oxidation and N-terminal acetylation were set as variable modifications. Tolerance for the first and main PSM search were 20 and 4.5 ppm, respectively. Peptides from common contaminant proteins were identified by utilizing the contaminant database provided by MaxQuant. FDR threshold of 1% was used for both the peptide and protein level. The match-between-runs option was enabled with default parameters in the identification step. Finally, LFQ was performed for those with a minimum ratio count of 1.

### Example 8: Statistical analysis for RNP capture experiment

To identify host and viral proteins that interact with the particular RNA species of interest (e.g. sgRNA or gRNA), we utilized the results from the "bead only" and "probe only" samples as technical backgrounds. Specifically, the "bead only" (or no-probe) experiment in infected cells was used to account for nonspecific interactors and biotin-containing carboxylases (e.g. PCCA, ACACA, and ACACB) and determine the set of host and viral proteins that in a broad sense bind to the RNA, which we call Probe I/II "binding" proteins. The probe experiment in uninfected cells (i.e. "probe only") was then used as the technical background against target RNAindependent interactors and determine the set of host proteins that are enriched for the target RNA, which we call Probe I/II "enriched" proteins.

To accomplish this, we considered the protein spectra count data as a multinomial distribution and applied a statistical test for spectra count enrichment. Specifically, let Np be the number of identified spectra counts for protein group p from the case experiment (e.g. Probe I experiment in infected cells), and Mp be the respective count number from the control experiment (e.g. noprobe experiment in infected cells). For each protein i with $N_i \geq 1$, the statistical significance of enrichment is:

$$P(X \geq N_i) = \sum_{k=N_i}^{N} B(k\,;\,N,\theta_i)$$

$$\theta_i = \frac{M_i + 1}{\sum_i (M_i + 1)}$$

where $N = \sum_i N_i$ is the total spectra count, $\theta_i$ is the background probability, and $B(k\,;\,n,\,p)$ is the binomial distribution of $k$ successes in $n$ trails with success probability $p$. Finally, the Benjamini-Hochberg method was used to adjust the p-values and control the false discovery rate.

### Example 9: Gene Ontology (GO) enrichment analysis

We conducted enrichment analyses of Gene Ontology (GO) terms (Gene Ontology Consortium, 2001) by means of summarizing the function of tens of host proteins identified in the RNP capture experiment. In general, Fisher's exact test is used to estimate the statistical significance of the association (i.e. contingency) between a particular GO term and the gene set of interest.

To improve the explanatory power of this analysis, we used the weight01 algorithm (Alexa et al., 2006) from the topGO R package which accounts for the GO graph structure and reduces local dependencies between GO terms.

Detailed information of the Gene Ontology was from the GO.db R package (version 3.8.2), and GO gene annotations were from the org.Hs.eg.db R package (version 3.8.2).

**Example 10: Protein-protein interaction network analysis**

We integrated protein-protein interaction data from the BioGRID database (Release 3.5.187) (Stark et al., 2006) and retrieved other proteins that do not necessarily bind to the SARS-CoV-2 RNA but form either transient or stable physical interactions with the host proteins identified from the RNP capture experiments. In detail, we considered only human protein-protein interactions that were (1) found from at least two different types of experiments and (2) reported by at least three publication records which resulted in a total of 65,625 interactions covering 12,143 human proteins. Physical interactions between SARS-CoV-2 proteins and human proteins were by affinity capture and mass spectrometry in SARS-CoV-2 protein expressing cells (Gordon et al., 2020). The network R package and the ggnet2 function of the GGally R package was used for graph visualization.

**Example 11: Protein domain enrichment analysis**

Pfam database (version 33.1) (El-Gebali et al., 2019) was used for protein domain enrichment analysis. Taxon 9606 (human) and Taxon 60711 (green monkey) protein domain annotations were used to analyze RNP capture results of HCoV-OC43 and SARS-CoV-2, respectively. Onesided Fisher's exact test was applied to estimate the statistical enrichment of a particular protein domain for the specific gene set (e.g. SARS-CoV-2 Probe I binding proteins). We utilized the set of all proteins identified in the RNP capture experiments and all protein domains annotated to those proteins as the statistical background of the enrichment analysis.

**Example 12: Subcellular localization analysis**

To investigate the subcellular localizations of the SARS-CoV-2 interactome, we leveraged the protein subcellular localization information from the Human cell map database v1 (Go et al., 2019). Information from the SAFE algorithm was used primarily but then supplemented by 29 information from the NMF algorithm in case of "no prediction" or "-" localizations. Localization terms of the NMF algorithm were matched to terms of the SAFE algorithm in general, but few were mapped to the higher term of the SAFE algorithm. For example, the "cell junction" term of the NMF algorithm was merged to the "cell junction, plasma membrane" term of the SAFE algorithm.

**Results**

**1. SARS-CoV-2 RNP purification**

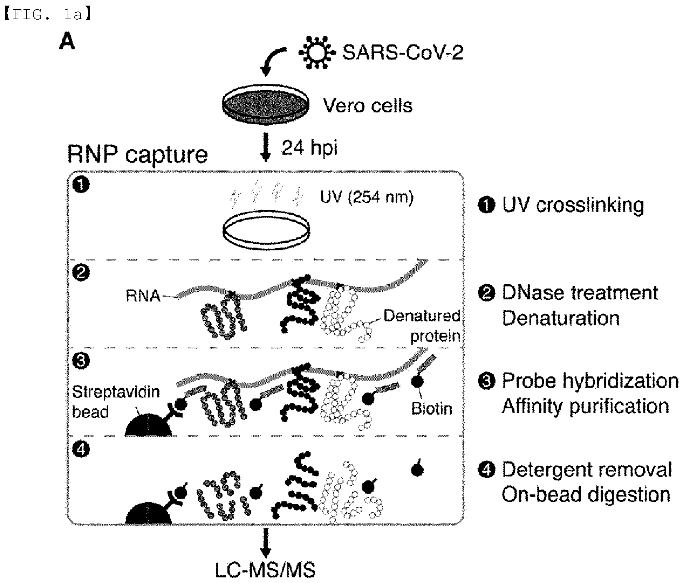

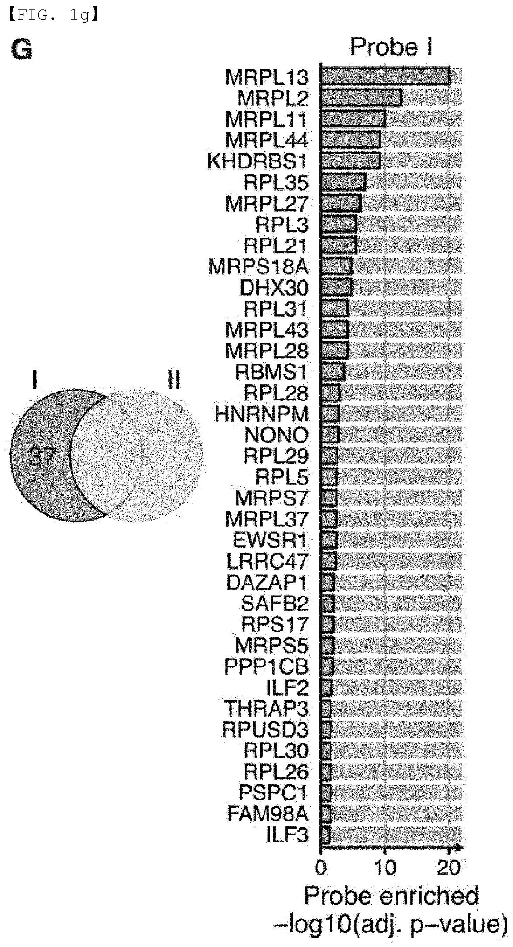

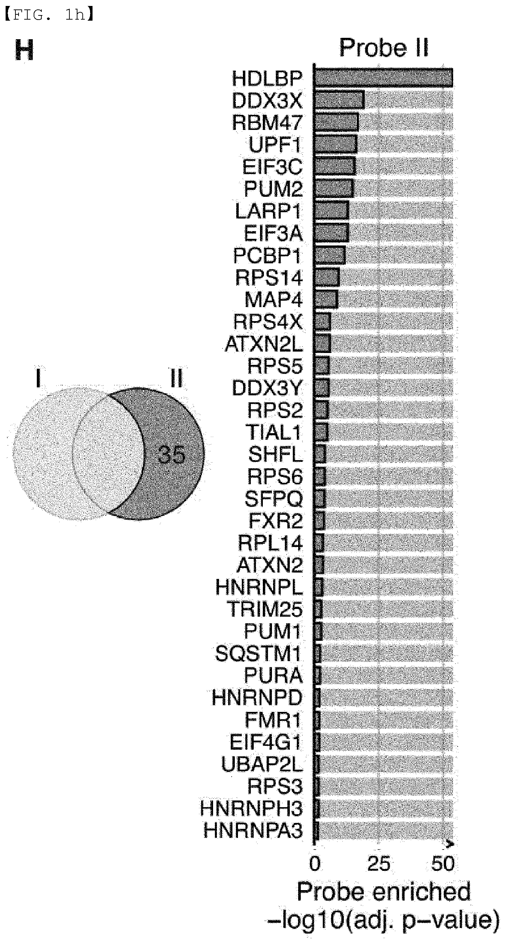

To identify the viral and host proteins that directly interact with the genomic and subgenomic RNAs of SARS-CoV-2, we modified the RNA antisense purification coupled with mass spectrometry (RAP-MS) (McHugh and Guttman, 2018) protocol which was developed to profile the interacting proteins of a particular RNA species (FIG. 1a).

Briefly, cells were first detached from culture vessels and then irradiated with 254 nm UV to induce RNA-protein crosslink while preserving RNA integrity. Crosslinked cells were treated with DNase and lysed with an optimized buffer condition to homogenize and denature the proteins in high concentration. Massive pools of biotinylated antisense 90-nt probes were used to capture the denatured RNP complexes in a sequence-specific manner. After stringent washing and detergent removal, the RNP complexes were released and digested by serial benzonase and on-bead trypsin treatment. These modifications to the RAP-MS protocol enabled robust and sensitive identification of proteins directly bound to the RNA target of interest.

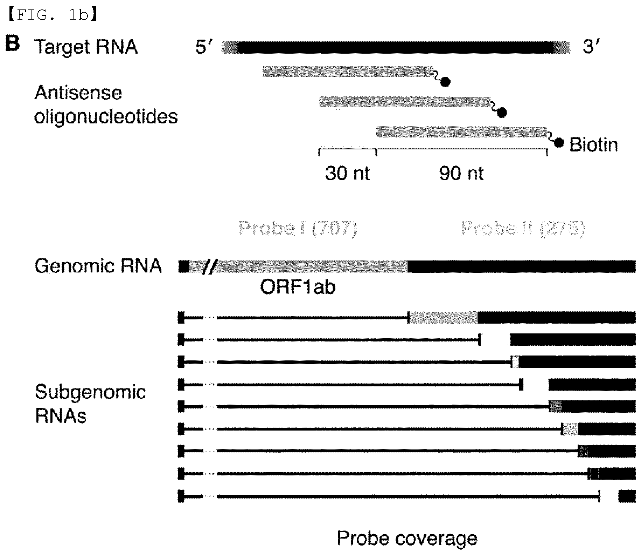

We designed two separate pools of densely overlapping 90-nt antisense probes to achieve an unbiased perspective of the SARS-CoV-2 RNA interactome (FIG. 1b and Table 1).

[Table 1]

| ASO ID | Accession | Group ID | Custom Array sequence |
|---|---|---|---|
| SARS2-Probe I.0 | NC_045512.2 | SARS2-Probe I | CTGATGAGGGAGGAGAAAACATTAATACGACTCACTATAGGG... AAAAGACCCTTGGA |
| SARS2-Probe I.1 | NC_045512.2 | SARS2-Probe I | CTGATGAGGGAGGAGAAAACATTAATACGACTCACTATAGGG... AAAAGACCCTTGGA |
| SARS2-Probe I.2 | NC_045512.2 | SARS2-Probe I | CTGATGAGGGAGGAGAAAACATTAATACGACTCACTATAGGG... AAAAGACCCTTGGA |
| SARS2-Probe I.3 | NC_045512.2 | SARS2-Probe I | CTGATGAGGGAGGAGAAAACATTAATACGACTCACTATAGGG... AAAAGACCCTTGGA |
| SARS2-Probe I.4 | NC_045512.2 | SARS2-Probe I | CTGATGAGGGAGGAGAAAACATTAATACGACTCACTATAGGG... AAAAGACCCTTGGA |
| SARS2-Probe I.5 | NC_045512.2 | SARS2-Probe I | CTGATGAGGGAGGAGAAAACATTAATACGACTCACTATAGGG... AAAAGACCCTTGGA |
| SARS2-Probe I.6 | NC_045512.2 | SARS2-Probe I | CTGATGAGGGAGGAGAAAACATTAATACGACTCACTATAGGG... AAAAGACCCTTGGA |
| SARS2-Probe I.7 | NC_045512.2 | SARS2-Probe I | CTGATGAGGGAGGAGAAAACATTAATACGACTCACTATAGGG... AAAAGACCCTTGGA |
| SARS2-Probe I.8 | NC_045512.2 | SARS2-Probe I | CTGATGAGGGAGGAGAAAACATTAATACGACTCACTATAGGG... AAAAGACCCTTGGA |
| SARS2-Probe I.9 | NC_045512.2 | SARS2-Probe I | CTGATGAGGGAGGAGAAAACATTAATACGACTCACTATAGGG... AAAAGACCCTTGGA |
| SARS2-Probe I.10 | NC_045512.2 | SARS2-Probe I | CTGATGAGGGAGGAGAAAACATTAATACGACTCACTATAGGG... AAAAGACCCTTGGA |
| SARS2-Probe I.11 | NC_045512.2 | SARS2-Probe I | CTGATGAGGGAGGAGAAAACATTAATACGACTCACTATAGGG... AAAAGACCCTTGGA |
| SARS2-Probe I.12 | NC_045512.2 | SARS2-Probe I | CTGATGAGGGAGGAGAAAACATTAATACGACTCACTATAGGG... AAAAGACCCTTGGA |
| SARS2-Probe I.13 | NC_045512.2 | SARS2-Probe I | CTGATGAGGGAGGAGAAAACATTAATACGACTCACTATAGGG... AAAAGACCCTTGGA |
| SARS2-Probe I.14 | NC_045512.2 | SARS2-Probe I | CTGATGAGGGAGGAGAAAACATTAATACGACTCACTATAGGG... AAAAGACCCTTGGA |
| SARS2-Probe I.15 | NC_045512.2 | SARS2-Probe I | CTGATGAGGGAGGAGAAAACATTAATACGACTCACTATAGGG... AAAAGACCCTTGGA |
| SARS2-Probe I.16 | NC_045512.2 | SARS2-Probe I | CTGATGAGGGAGGAGAAAACATTAATACGACTCACTATAGGG... AAAAGACCCTTGGA |
| SARS2-Probe I.17 | NC_045512.2 | SARS2-Probe I | CTGATGAGGGAGGAGAAAACATTAATACGACTCACTATAGGG... AAAAGACCCTTGGA |
| SARS2-Probe I.18 | NC_045512.2 | SARS2-Probe I | CTGATGAGGGAGGAGAAAACATTAATACGACTCACTATAGGG... AAAAGACCCTTGGA |
| SARS2-Probe I.19 | NC_045512.2 | SARS2-Probe I | CTGATGAGGGAGGAGAAAACATTAATACGACTCACTATAGGG... AAAAGACCCTTGGA |
| SARS2-Probe I.20 | NC_045512.2 | SARS2-Probe I | CTGATGAGGGAGGAGAAAACATTAATACGACTCACTATAGGG... AAAAGACCCTTGGA |
| SARS2-Probe I.21 | NC_045512.2 | SARS2-Probe I | CTGATGAGGGAGGAGAAAACATTAATACGACTCACTATAGGG... AAAAGACCCTTGGA |
| SARS2-Probe I.22 | NC_045512.2 | SARS2-Probe I | CTGATGAGGGAGGAGAAAACATTAATACGACTCACTATAGGG... AAAAGACCCTTGGA |
| SARS2-Probe I.23 | NC_045512.2 | SARS2-Probe I | CTGATGAGGGAGGAGAAAACATTAATACGACTCACTATAGGG... AAAAGACCCTTGGA |
| SARS2-Probe I.24 | NC_045512.2 | SARS2-Probe I | CTGATGAGGGAGGAGAAAACATTAATACGACTCACTATAGGG... AAAAGACCCTTGGA |

| ASO ID | Accession | Group ID | Custom Array sequence |
|---|---|---|---|
| SARS2-Probe I.25 | NC_045512.2 | SARS2-Probe I | CTGATGAGGGAGAAACATTAATACGACTCACTATAGGGTTGCAGACACCTTTTGAAATTAAATTGGCAAAGAAATTTGACACCTTCAATGGGGAATGTCCAAATTTTGTATTTCCCTTAAATTCCATATGGAATTCTCGGGTGCCAAGGGGCTAAAAGACCCTTGGA |
| SARS2-Probe I.26 | NC_045512.2 | SARS2-Probe I | CTGATGAGGGAGAAACATTAATACGACTCACTATAGGGAAGAAATTTGACACCTTCAATGGGGAATGTCCAAATTTTGTATTTCCCTTAAATTCCATAATCAAGACTATTCAACCAAGGGTTGAAAAGTGGAATTCTCGGGTGCCAAGGGGCTAAAAGACCCTTGGA |
| SARS2-Probe I.27 | NC_045512.2 | SARS2-Probe I | CTGATGAGGGAGAAACATTAATACGACTCACTATAGGGCCAAATTTTGTATTTCCCTTAAATTCCATAATCAAGACTATTCAACCAAGGGTTGAAAAGAAAAAGCTTGATGGCTTTATGGGTAGAATTTGGAATTCTCGGGTGCCAAGGGGCTAAAAGACCCTTGGA |
| SARS2-Probe I.28 | NC_045512.2 | SARS2-Probe I | CTGATGAGGGAGAAACATTAATACGACTCACTATAGGGATCAAGACTATTCAACCAAGGGTTGAAAAGAAAAAGCTTGATGGCTTTATGGGTAGAATTCGATCTGTCTATCCAGTTGCGTCACCAAATTGGAATTCTCGGGTGCCAAGGGGCTAAAAGACCCTTGGA |
| SARS2-Probe I.29 | NC_045512.2 | SARS2-Probe I | CTGATGAGGGAGAAACATTAATACGACTCACTATAGGGAAAAAGCTTGATGGCTTTATGGGTAGAATTCGATCTGTCTATCCAGTTGCGTCACCAAATGAATGCAACCAAATGTGCCTTTCAACTCTCTGGAATTCTCGGGTGCCAAGGGGCTAAAAGACCCTTGGA |
| SARS2-Probe I.30 | NC_045512.2 | SARS2-Probe I | CTGATGAGGGAGAAACATTAATACGACTCACTATAGGGCGATCTGTCTATCCAGTTGCGTCACCAAATGAATGCAACCAAATGTGCCTTTCAACTCTCATGAAGTGTGATCATTGTGGTGAAACTTCATGGAATTCTCGGGTGCCAAGGGGCTAAAAGACCCTTGGA |
| SARS2-Probe I.31 | NC_045512.2 | SARS2-Probe I | CTGATGAGGGAGAAACATTAATACGACTCACTATAGGGGAATGCAACCAAATGTGCCTTTCAACTCTCATGAAGTGTGATCATTGTGGTGAAACTTCATGGCAGACGGGCGATTTTGTTAAAGCCACTTGGAATTCTCGGGTGCCAAGGGGCTAAAAGACCCTTGGA |
| SARS2-Probe I.32 | NC_045512.2 | SARS2-Probe I | CTGATGAGGGAGAAACATTAATACGACTCACTATAGGGATGAAGTGTGATCATTGTGGTGAAACTTCATGGCAGACGGGCGATTTTGTTAAAGCCACTTGCGAATTTTGTGGCACTGAGAATTTGACTTGGAATTCTCGGGTGCCAAGGGGCTAAAAGACCCTTGGA |
| SARS2-Probe I.33 | NC_045512.2 | SARS2-Probe I | CTGATGAGGGAGAAACATTAATACGACTCACTATAGGGTGGCAGACGGGCGATTTTGTTAAAGCCACTTGCGAATTTTGTGGCACTGAGAATTTGACTAAAGAAGGTGCCACTACTTGTGGTTACTTATGGAATTCTCGGGTGCCAAGGGGCTAAAAGACCCTTGGA |
| SARS2-Probe I.34 | NC_045512.2 | SARS2-Probe I | CTGATGAGGGAGAAACATTAATACGACTCACTATAGGGTGCGAATTTTGTGGCACTGAGAATTTGACTAAAGAAGGTGCCACTACTTGTGGTTACTTACCCCAAAATGCTGTTGTTAAAATTTATTGTTGGAATTCTCGGGTGCCAAGGGGCTAAAAGACCCTTGGA |
| SARS2-Probe I.35 | NC_045512.2 | SARS2-Probe I | CTGATGAGGGAGAAACATTAATACGACTCACTATAGGGAAAGAAGGTGCCACTACTTGTGGTTACTTACCCCAAAATGCTGTTGTTAAAATTTATTGTCCAGCATGTCACAATTCAGAAGTAGGACCTTGGAATTCTCGGGTGCCAAGGGGCTAAAAGACCCTTGGA |
| SARS2-Probe I.36 | NC_045512.2 | SARS2-Probe I | CTGATGAGGGAGAAACATTAATACGACTCACTATAGGGCCCCAAAATGCTGTTGTTAAAATTTATTGTCCAGCATGTCACAATTCAGAAGTAGGACCTGAGCATAGTCTTGCCGAATACCATAATGAATGGAATTCTCGGGTGCCAAGGGGCTAAAAGACCCTTGGA |
| SARS2-Probe I.37 | NC_045512.2 | SARS2-Probe I | CTGATGAGGGAGAAACATTAATACGACTCACTATAGGGCCAGCATGTCACAATTCAGAAGTAGGACCTGAGCATAGTCTTGCCGAATACCATAATGAATCTGGCTTGAAAACCATTCTTCGTAAGGGTTGGAATTCTCGGGTGCCAAGGGGCTAAAAGACCCTTGGA |
| SARS2-Probe I.38 | NC_045512.2 | SARS2-Probe I | CTGATGAGGGAGAAACATTAATACGACTCACTATAGGGGAGCATAGTCTTGCCGAATACCATAATGAATCTGGCTTGAAAACCATTCTTCGTAAGGGTGGTCGCACTATTGCCTTTGGAGGCTGTGTGTGGAATTCTCGGGTGCCAAGGGGCTAAAAGACCCTTGGA |
| SARS2-Probe I.39 | NC_045512.2 | SARS2-Probe I | CTGATGAGGGAGAAACATTAATACGACTCACTATAGGGTCTGGCTTGAAAACCATTCTTCGTAAGGGTGGTCGCACTATTGCCTTTGGAGGCTGTGTGTGTTCTCTTATGTTGGTTGCCATAACAAGTGTTGGAATTCTCGGGTGCCAAGGGGCTAAAAGACCCTTGGA |
| SARS2-Probe I.40 | NC_045512.2 | SARS2-Probe I | CTGATGAGGGAGAAACATTAATACGACTCACTATAGGGGGTCGCACTATTGCCTTTGGAGGCTGTGTGTTCTCTTATGTTGGTTGCCATAACAAGTGTGCCTATTGGGTTCCACGTGCTAGCGCTAACTGGAATTCTCGGGTGCCAAGGGGCTAAAAGACCCTTGGA |
| SARS2-Probe I.41 | NC_045512.2 | SARS2-Probe I | CTGATGAGGGAGAAACATTAATACGACTCACTATAGGGTTCTCTTATGTTGGTTGCCATAACAAGTGTGCCTATTGGGTTCCACGTGCTAGCGCTAACATAGGTTGTAACCATACAGGTGTTGTTGGATGGAATTCTCGGGTGCCAAGGGGCTAAAAGACCCTTGGA |
| SARS2-Probe I.42 | NC_045512.2 | SARS2-Probe I | CTGATGAGGGAGAAACATTAATACGACTCACTATAGGGGCCTATTGGGTTCCACGTGCTAGCGCTAACATAGGTTGTAACCATACAGGTGTTGTTGGAGAAGGTTCCGAAGGTCTTAATGACAACCTTTGGAATTCTCGGGTGCCAAGGGGCTAAAAGACCCTTGGA |
| SARS2-Probe I.43 | NC_045512.2 | SARS2-Probe I | CTGATGAGGGAGAAACATTAATACGACTCACTATAGGGATAGGTTGTAACCATACAGGTGTTGTTGGAGAAGGTTCCGAAGGTCTTAATGACAACCTTCTTGAAATACTCCAAAAAGAGAAAGTCAACTGGAATTCTCGGGTGCCAAGGGGCTAAAAGACCCTTGGA |
| SARS2-Probe I.44 | NC_045512.2 | SARS2-Probe I | CTGATGAGGGAGAAACATTAATACGACTCACTATAGGGGAAGGTTCCGAAGGTCTTAATGACAACCTTCTTGAAATACTCCAAAAAGAGAAAGTCAACATCAATATTGTTGGTGACTTTAAACTTAATTGGAATTCTCGGGTGCCAAGGGGCTAAAAGACCCTTGGA |
| SARS2-Probe I.45 | NC_045512.2 | SARS2-Probe I | CTGATGAGGGAGAAACATTAATACGACTCACTATAGGGCTTGAAATACTCCAAAAAGAGAAAGTCAACATCAATATTGTTGGTGACTTTAAACTTAATGAAGAGATCGCCATTATTTTGGCATCTTTTTGGAATTCTCGGGTGCCAAGGGGCTAAAAGACCCTTGGA |
| SARS2-Probe I.46 | NC_045512.2 | SARS2-Probe I | CTGATGAGGGAGAAACATTAATACGACTCACTATAGGGATCAATATTGTTGGTGACTTTAAACTTAATGAAGAGATCGCCATTATTTTGGCATCTTTTTCTGCTTCCACAAGTGCTTTTGTGGAAACTTGGAATTCTCGGGTGCCAAGGGGCTAAAAGACCCTTGGA |
| SARS2-Probe I.47 | NC_045512.2 | SARS2-Probe I | CTGATGAGGGAGAAACATTAATACGACTCACTATAGGGGAAGAGATCGCCATTATTTTGGCATCTTTTTCTGCTTCCACAAGTGCTTTTGTGGAAACTGTGAAAGGTTTGGATTATAAAGCATTCAAATGGAATTCTCGGGTGCCAAGGGGCTAAAAGACCCTTGGA |
| SARS2-Probe I.48 | NC_045512.2 | SARS2-Probe I | CTGATGAGGGAGAAACATTAATACGACTCACTATAGGGTCTGCTTCCACAAGTGCTTTTGTGGAAACTGTGAAAGGTTTGGATTATAAAGCATTCAAACAAATTGTTGAATCCTGTGGTAATTTTAAATGGAATTCTCGGGTGCCAAGGGGCTAAAAGACCCTTGGA |
| SARS2-Probe I.49 | NC_045512.2 | SARS2-Probe I | CTGATGAGGGAGAAACATTAATACGACTCACTATAGGGGTGAAAGGTTTGGATTATAAAGCATTCAAACAAATTGTTGAATCCTGTGGTAATTTTAAAGTTACAAAAGGAAAAGCTAAAAAAGGTGCCTGGAATTCTCGGGTGCCAAGGGGCTAAAAGACCCTTGGA |

24

| ASO ID | Accession | Group ID | Custom Array sequence |
|---|---|---|---|
| SARS2-Probe I.50 | NC_045512.2 | SARS2-Probe I | CTGATGAGGGAGAAACATTAATACGACTCACTATAGGGCAAATTGTTGAATCCTGTGGTAATTTTAAAGTTACAAAAGGAAAAGCTAAAAAAGGTGCCTGGAATATTGGTGAACAGAAATCAATACTGTGGAATTCTCGGGTGCCAAGGGGCTAAAAGACCCTTGGA |
| SARS2-Probe I.51 | NC_045512.2 | SARS2-Probe I | CTGATGAGGGAGAAACATTAATACGACTCACTATAGGGGTTACAAAAGGAAAAGCTAAAAAAGGTGCCTGGAATATTGGTGAACAGAAATCAATACTGAGTCCTCTTTATGCATTTGCATCAGAGGCTTGGAATTCTCGGGTGCCAAGGGGCTAAAAGACCCTTGGA |
| SARS2-Probe I.52 | NC_045512.2 | SARS2-Probe I | CTGATGAGGGAGAAACATTAATACGACTCACTATAGGGTGGAATATTGGTGAACAGAAATCAATACTGAGTCCTCTTTATGCATTTGCATCAGAGGCTGCTCGTGTTGTACGATCAATTTTCTCCCGCTGGAATTCTCGGGTGCCAAGGGGCTAAAAGACCCTTGGA |
| SARS2-Probe I.53 | NC_045512.2 | SARS2-Probe I | CTGATGAGGGAGAAACATTAATACGACTCACTATAGGGAGTCCTCTTTATGCATTTGCATCAGAGGCTGCTCGTGTTGTACGATCAATTTTCTCCCGCACTCTTGAAACTGCTCAAAATTCTGTGCGTTGGAATTCTCGGGTGCCAAGGGGCTAAAAGACCCTTGGA |
| SARS2-Probe I.54 | NC_045512.2 | SARS2-Probe I | CTGATGAGGGAGAAACATTAATACGACTCACTATAGGGGCTCGTGTTGTACGATCAATTTTCTCCCGCACTCTTGAAACTGCTCAAAATTCTGTGCGTGTTTTACAGAAGGCCGCTATAACAATACTATGGAATTCTCGGGTGCCAAGGGGCTAAAAGACCCTTGGA |
| SARS2-Probe I.55 | NC_045512.2 | SARS2-Probe I | CTGATGAGGGAGAAACATTAATACGACTCACTATAGGGACTCTTGAAACTGCTCAAAATTCTGTGCGTGTTTTACAGAAGGCCGCTATAACAATACTAGATGGAATTTCACAGTATTCACTGAGACTCTGGAATTCTCGGGTGCCAAGGGGCTAAAAGACCCTTGGA |
| SARS2-Probe I.56 | NC_045512.2 | SARS2-Probe I | CTGATGAGGGAGAAACATTAATACGACTCACTATAGGGGTTTTACAGAAGGCCGCTATAACAATACTAGATGGAATTTCACAGTATTCACTGAGACTCATTGATGCTATGATGTTCACATCTGATTTGTGGAATTCTCGGGTGCCAAGGGGCTAAAAGACCCTTGGA |
| SARS2-Probe I.57 | NC_045512.2 | SARS2-Probe I | CTGATGAGGGAGAAACATTAATACGACTCACTATAGGGGATGGAATTTCACAGTATTCACTGAGACTCATTGATGCTATGATGTTCACATCTGATTTGGCTACTAACAATCTAGTTGTAATGGCCTACTGGAATTCTCGGGTGCCAAGGGGCTAAAAGACCCTTGGA |
| SARS2-Probe I.58 | NC_045512.2 | SARS2-Probe I | CTGATGAGGGAGAAACATTAATACGACTCACTATAGGGATTGATGCTATGATGTTCACATCTGATTTGGCTACTAACAATCTAGTTGTAATGGCCTACATTACAGGTGGTGTTGTTCAGTTGACTTCGTGGAATTCTCGGGTGCCAAGGGGCTAAAAGACCCTTGGA |
| SARS2-Probe I.59 | NC_045512.2 | SARS2-Probe I | CTGATGAGGGAGAAACATTAATACGACTCACTATAGGGGCTACTAACAATCTAGTTGTAATGGCCTACATTACAGGTGGTGTTGTTCAGTTGACTTCGCAGTGGCTAACTAACATCTTTGGCACTGTTTGGAATTCTCGGGTGCCAAGGGGCTAAAAGACCCTTGGA |
| SARS2-Probe I.60 | NC_045512.2 | SARS2-Probe I | CTGATGAGGGAGAAACATTAATACGACTCACTATAGGGATTACAGGTGGTGTTGTTCAGTTGACTTCGCAGTGGCTAACTAACATCTTTGGCACTGTTTATGAAAAACTCAAACCCGTCCTTGATTGGTGGAATTCTCGGGTGCCAAGGGGCTAAAAGACCCTTGGA |
| SARS2-Probe I.61 | NC_045512.2 | SARS2-Probe I | CTGATGAGGGAGAAACATTAATACGACTCACTATAGGGCAGTGGCTAACTAACATCTTTGGCACTGTTTATGAAAAACTCAAACCCGTCCTTGATTGGCTTGAAGAGAAGTTTAAGGAAGGTGTAGAGTGGAATTCTCGGGTGCCAAGGGGCTAAAAGACCCTTGGA |
| SARS2-Probe I.62 | NC_045512.2 | SARS2-Probe I | CTGATGAGGGAGAAACATTAATACGACTCACTATAGGGTATGAAAAACTCAAACCCGTCCTTGATTGGCTTGAAGAGAAGTTTAAGGAAGGTGTAGAGTTTCTTAGAGACGGTTGGGAAATTGTTAAATGGAATTCTCGGGTGCCAAGGGGCTAAAAGACCCTTGGA |
| SARS2-Probe I.63 | NC_045512.2 | SARS2-Probe I | CTGATGAGGGAGAAACATTAATACGACTCACTATAGGGCTTGAAGAGAAGTTTAAGGAAGGTGTAGAGTTTCTTAGAGACGGTTGGGAAATTGTTAAATTTATCTCAACCTGTGCTTGTGAAATTGTCTGGAATTCTCGGGTGCCAAGGGGCTAAAAGACCCTTGGA |
| SARS2-Probe I.64 | NC_045512.2 | SARS2-Probe I | CTGATGAGGGAGAAACATTAATACGACTCACTATAGGGTTTCTTAGAGACGGTTGGGAAATTGTTAAATTTATCTCAACCTGTGCTTGTGAAATTGTCGGTGGACAAATTGTCACCTGTGCAAAGGAATGGAATTCTCGGGTGCCAAGGGGCTAAAAGACCCTTGGA |
| SARS2-Probe I.65 | NC_045512.2 | SARS2-Probe I | CTGATGAGGGAGAAACATTAATACGACTCACTATAGGGTTTATCTCAACCTGTGCTTGTGAAATTGTCGGTGGACAAATTGTCACCTGTGCAAAGGAAATTAAGGAGAGTGTTCAGACATTCTTTAAGTGGAATTCTCGGGTGCCAAGGGGCTAAAAGACCCTTGGA |
| SARS2-Probe I.66 | NC_045512.2 | SARS2-Probe I | CTGATGAGGGAGAAACATTAATACGACTCACTATAGGGGGGTGGACAAATTGTCACCTGTGCAAAGGAAATTAAGGAGAGTGTTCAGACATTCTTTAAGCTTGTAAATAAATTTTTGGCTTTGTGTGCTTGGAATTCTCGGGTGCCAAGGGGCTAAAAGACCCTTGGA |
| SARS2-Probe I.67 | NC_045512.2 | SARS2-Probe I | CTGATGAGGGAGAAACATTAATACGACTCACTATAGGGATTAAGGAGAGTGTTCAGACATTCTTTAAGCTTGTAAATAAATTTTTGGCTTTGTGTGCTGACTCTATCATTATTGGTGGAGCTAAACTTTGGAATTCTCGGGTGCCAAGGGGCTAAAAGACCCTTGGA |
| SARS2-Probe I.68 | NC_045512.2 | SARS2-Probe I | CTGATGAGGGAGAAACATTAATACGACTCACTATAGGGCTTGTAAATAAATTTTTGGCTTTGTGTGCTGACTCTATCATTATTGGTGGAGCTAAACTTAAAGCCTTGAATTTAGGTGAAACATTTGTCTGGAATTCTCGGGTGCCAAGGGGCTAAAAGACCCTTGGA |
| SARS2-Probe I.69 | NC_045512.2 | SARS2-Probe I | CTGATGAGGGAGAAACATTAATACGACTCACTATAGGGGACTCTATCATTATTGGTGGAGCTAAACTTAAAGCCTTGAATTTAGGTGAAACATTTGTCACGCACTCAAAGGGATTGTACAGAAAGTGTTGGAATTCTCGGGTGCCAAGGGGCTAAAAGACCCTTGGA |
| SARS2-Probe I.70 | NC_045512.2 | SARS2-Probe I | CTGATGAGGGAGAAACATTAATACGACTCACTATAGGGAAAGCCTTGAATTTAGGTGAAACATTTGTCACGCACTCAAAGGGATTGTACAGAAAGTGTGTTAAATCCAGAGAAGAAACTGGCCTACTCTGGAATTCTCGGGTGCCAAGGGGCTAAAAGACCCTTGGA |
| SARS2-Probe I.71 | NC_045512.2 | SARS2-Probe I | CTGATGAGGGAGAAACATTAATACGACTCACTATAGGGACGCACTCAAAGGGATTGTACAGAAAGTGTGTTAAATCCAGAGAAGAAACTGGCCTACTCATGCCTCTAAAAGCCCCAAAAGAAATTATCTGGAATTCTCGGGTGCCAAGGGGCTAAAAGACCCTTGGA |
| SARS2-Probe I.72 | NC_045512.2 | SARS2-Probe I | CTGATGAGGGAGAAACATTAATACGACTCACTATAGGGGTTAAATCCAGAGAAGAAACTGGCCTACTCATGCCTCTAAAAGCCCCAAAAGAAATTATCTTCTTAGAGGGAGAAACACTTCCCACAGAATGGAATTCTCGGGTGCCAAGGGGCTAAAAGACCCTTGGA |
| SARS2-Probe I.73 | NC_045512.2 | SARS2-Probe I | CTGATGAGGGAGAAACATTAATACGACTCACTATAGGGATGCCTCTAAAAGCCCCAAAAGAAATTATCTTCTTAGAGGGAGAAACACTTCCCACAGAAGTGTTAACAGAGGAAGTTGTCTTGAAAACTTGGAATTCTCGGGTGCCAAGGGGCTAAAAGACCCTTGGA |
| SARS2-Probe I.74 | NC_045512.2 | SARS2-Probe I | CTGATGAGGGAGAAACATTAATACGACTCACTATAGGGTTCTTAGAGGGAGAAACACTTCCCACAGAAGTGTTAACAGAGGAAGTTGTCTTGAAAACTGGTGATTTACAACCATTAGAACAACCTACTTGGAATTCTCGGGTGCCAAGGGGCTAAAAGACCCTTGGA |

| ASO ID | Accession | Group ID | Custom Array sequence |
|---|---|---|---|
| SARS2-Probe I.75 | NC_045512.2 | SARS2-Probe I | CTGATGAGGGAGAAACATTAATACGACTCACTATAGGGGTGTTAACAGAGGAAGTTGTCTTGAAAACTGGTGATTTACAACCATTAGAACAACCTACTAGTGAAGCTGTTGAAGCTCCATTGGTTGGTTGGAATTCTCGGGTGCCAAGGGGCTAAAAGACCCTTGGA |
| SARS2-Probe I.76 | NC_045512.2 | SARS2-Probe I | CTGATGAGGGAGAAACATTAATACGACTCACTATAGGGGGGTGATTTACAACCATTAGAACAACCTACTAGTGAAGCTGTTGAAGCTCCATTGGTTGGTACACCAGTTTGTATTAACGGGCTTATGTTGTGGAATTCTCGGGTGCCAAGGGGCTAAAAGACCCTTGGA |
| SARS2-Probe I.77 | NC_045512.2 | SARS2-Probe I | CTGATGAGGGAGAAACATTAATACGACTCACTATAGGGGAGTGAAGCTGTTGAAGCTCCATTGGTTGGTACACCAGTTTGTATTAACGGGCTTATGTTGCTCGAAATCAAAGACACAGAAAAGTACTGTTGGAATTCTCGGGTGCCAAGGGGCTAAAAGACCCTTGGA |
| SARS2-Probe I.78 | NC_045512.2 | SARS2-Probe I | CTGATGAGGGAGAAACATTAATACGACTCACTATAGGGACACCAGTTTGTATTAACGGGCTTATGTTGCTCGAAATCAAAGACACAGAAAAGTACTGTGCCCTTGCACCTAATATGATGGTAACAAACTGGAATTCTCGGGTGCCAAGGGGCTAAAAGACCCTTGGA |
| SARS2-Probe I.79 | NC_045512.2 | SARS2-Probe I | CTGATGAGGGAGAAACATTAATACGACTCACTATAGGGCTCGAAATCAAAGACACAGAAAAGTACTGTGCCCTTGCACCTAATATGATGGTAACAAACAATACCTTCACACTCAAAGGCGGTGCACCATGGAATTCTCGGGTGCCAAGGGGCTAAAAGACCCTTGGA |
| SARS2-Probe I.80 | NC_045512.2 | SARS2-Probe I | CTGATGAGGGAGAAACATTAATACGACTCACTATAGGGGCCCTTGCACCTAATATGATGGTAACAAACAATACCTTCACACTCAAAGGCGGTGCACCAACAAAGGTTACTTTTGGTGATGACACTGTGTGGAATTCTCGGGTGCCAAGGGGCTAAAAGACCCTTGGA |
| SARS2-Probe I.81 | NC_045512.2 | SARS2-Probe I | CTGATGAGGGAGAAACATTAATACGACTCACTATAGGGAATACCTTCACACTCAAAGGCGGTGCACCAACAAAGGTTACTTTTGGTGATGACACTGTGATAGAAGTGCAAGGTTACAAGAGTGTGAATTGGAATTCTCGGGTGCCAAGGGGCTAAAAGACCCTTGGA |
| SARS2-Probe I.82 | NC_045512.2 | SARS2-Probe I | CTGATGAGGGAGAAACATTAATACGACTCACTATAGGGACAAAGGTTACTTTTGGTGATGACACTGTGATAGAAGTGCAAGGTTACAAGAGTGTGAATATCACTTTTGAACTTGATGAAAGGATTGATTGGAATTCTCGGGTGCCAAGGGGCTAAAAGACCCTTGGA |
| SARS2-Probe I.83 | NC_045512.2 | SARS2-Probe I | CTGATGAGGGAGAAACATTAATACGACTCACTATAGGGATAGAAGTGCAAGGTTACAAGAGTGTGAATATCACTTTTGAACTTGATGAAAGGATTGATAAAGTACTTAATGAGAAGTGCTCTGCCTATTGGAATTCTCGGGTGCCAAGGGGCTAAAAGACCCTTGGA |
| SARS2-Probe I.84 | NC_045512.2 | SARS2-Probe I | CTGATGAGGGAGAAACATTAATACGACTCACTATAGGGATCACTTTTGAACTTGATGAAAGGATTGATAAAGTACTTAATGAGAAGTGCTCTGCCTATACAGTTGAACTCGGTACAGAAGTAAATGAGTGGAATTCTCGGGTGCCAAGGGGCTAAAAGACCCTTGGA |
| SARS2-Probe I.85 | NC_045512.2 | SARS2-Probe I | CTGATGAGGGAGAAACATTAATACGACTCACTATAGGGAAAGTACTTAATGAGAAGTGCTCTGCCTATACAGTTGAACTCGGTACAGAAGTAAATGAGTTCGCCTGTGTTGTGGCAGATGCTGTCATATGGAATTCTCGGGTGCCAAGGGGCTAAAAGACCCTTGGA |
| SARS2-Probe I.86 | NC_045512.2 | SARS2-Probe I | CTGATGAGGGAGAAACATTAATACGACTCACTATAGGGACAGTTGAACTCGGTACAGAAGTAAATGAGTTCGCCTGTGTTGTGGCAGATGCTGTCATAAAAACTTTGCAACCAGTATCTGAATTACTTTGGAATTCTCGGGTGCCAAGGGGCTAAAAGACCCTTGGA |
| SARS2-Probe I.87 | NC_045512.2 | SARS2-Probe I | CTGATGAGGGAGAAACATTAATACGACTCACTATAGGGTTCGCCTGTGTTGTGGCAGATGCTGTCATAAAAACTTTGCAACCAGTATCTGAATTACTTACACCACTGGGCATTGATTTAGATGAGTGGTGGAATTCTCGGGTGCCAAGGGGCTAAAAGACCCTTGGA |
| SARS2-Probe I.88 | NC_045512.2 | SARS2-Probe I | CTGATGAGGGAGAAACATTAATACGACTCACTATAGGGAAAAACTTTGCAACCAGTATCTGAATTACTTACACCACTGGGCATTGATTTAGATGAGTGGAGTATGGCTACATACTACTTATTTGATGAGTGGAATTCTCGGGTGCCAAGGGGCTAAAAGACCCTTGGA |
| SARS2-Probe I.89 | NC_045512.2 | SARS2-Probe I | CTGATGAGGGAGAAACATTAATACGACTCACTATAGGGACACCACTGGGCATTGATTTAGATGAGTGGAGTATGGCTACATACTACTTATTTGATGAGTCTGGTGAGTTTAAATTGGCTTCACATATGTGGAATTCTCGGGTGCCAAGGGGCTAAAAGACCCTTGGA |
| SARS2-Probe I.90 | NC_045512.2 | SARS2-Probe I | CTGATGAGGGAGAAACATTAATACGACTCACTATAGGGAGTATGGCTACATACTACTTATTTGATGAGTCTGGTGAGTTTAAATTGGCTTCACATATGTATTGTTCTTTCTACCCTCCAGATGAGGATTGGAATTCTCGGGTGCCAAGGGGCTAAAAGACCCTTGGA |
| SARS2-Probe I.91 | NC_045512.2 | SARS2-Probe I | CTGATGAGGGAGAAACATTAATACGACTCACTATAGGGTCTGGTGAGTTTAAATTGGCTTCACATATGTATTGTTCTTTCTACCCTCCAGATGAGGATGAAGAAGAAGGTGATTGTGAAGAAGAAGAGTGGAATTCTCGGGTGCCAAGGGGCTAAAAGACCCTTGGA |
| SARS2-Probe I.92 | NC_045512.2 | SARS2-Probe I | CTGATGAGGGAGAAACATTAATACGACTCACTATAGGGTATTGTTCTTTCTACCCTCCAGATGAGGATGAAGAAGAAGGTGATTGTGAAGAAGAAGAGTTGAGCCATCAACTCAATATGAGTATGGTTGGAATTCTCGGGTGCCAAGGGGCTAAAAGACCCTTGGA |
| SARS2-Probe I.93 | NC_045512.2 | SARS2-Probe I | CTGATGAGGGAGAAACATTAATACGACTCACTATAGGGGAAGAAGAAGGTGATTGTGAAGAAGAAGAGTTTGAGCCATCAACTCAATATGAGTATGGTACTGAAGATGATTACCAAGGTAAACCTTTGTGGAATTCTCGGGTGCCAAGGGGCTAAAAGACCCTTGGA |
| SARS2-Probe I.94 | NC_045512.2 | SARS2-Probe I | CTGATGAGGGAGAAACATTAATACGACTCACTATAGGGTTTGAGCCATCAACTCAATATGAGTATGGTACTGAAGATGATTACCAAGGTAAACCTTTGGAATTTGGTGCCACTTCTGCTGCTCTTCAATGGAATTCTCGGGTGCCAAGGGGCTAAAAGACCCTTGGA |
| SARS2-Probe I.95 | NC_045512.2 | SARS2-Probe I | CTGATGAGGGAGAAACATTAATACGACTCACTATAGGGACTGAAGATGATTACCAAGGTAAACCTTTGGAATTTGGTGCCACTTCTGCTGCTCTTCAACCTGAAGAAGAGCAAGAAGAAGATTGGTTATGGAATTCTCGGGTGCCAAGGGGCTAAAAGACCCTTGGA |
| SARS2-Probe I.96 | NC_045512.2 | SARS2-Probe I | CTGATGAGGGAGAAACATTAATACGACTCACTATAGGGGAATTTGGTGCCACTTCTGCTGCTCTTCAACCTGAAGAAGAGCAAGAAGAAGATTGGTTAGATGATGATAGTCAACAAACTGTTGGTCAATGGAATTCTCGGGTGCCAAGGGGCTAAAAGACCCTTGGA |
| SARS2-Probe I.97 | NC_045512.2 | SARS2-Probe I | CTGATGAGGGAGAAACATTAATACGACTCACTATAGGGCCTGAAGAAGAGCAAGAAGAAGATTGGTTAGATGATGATAGTCAACAAACTGTTGGTCAACAAGACGGCAGTGAGGACAATCAGACAACTTGGAATTCTCGGGTGCCAAGGGGCTAAAAGACCCTTGGA |
| SARS2-Probe I.98 | NC_045512.2 | SARS2-Probe I | CTGATGAGGGAGAAACATTAATACGACTCACTATAGGGGGATGATGATAGTCAACAAACTGTTGGTCAACAAGACGGCAGTGAGGACAATCAGACAACTACTATTCAAACAATTGTTGAGGTTCAACCTTGGAATTCTCGGGTGCCAAGGGGCTAAAAGACCCTTGGA |
| SARS2-Probe I.99 | NC_045512.2 | SARS2-Probe I | CTGATGAGGGAGAAACATTAATACGACTCACTATAGGGCAAGACGGCAGTGAGGACAATCAGACAACTACTATTCAAACAATTGTTGAGGTTCAACCTCAATTAGAGATGGAACTTACACCAGTTGTTTGGAATTCTCGGGTGCCAAGGGGCTAAAAGACCCTTGGA |

| ASO ID | Accession | Group ID | Custom Array sequence |
|---|---|---|---|
| SARS2: Probe I1100 | NC_045512.2 | SARS2: Probe I | CTGATGAGGGAGGGAGAAACATTAATACGACTCACTATAGGGACTATAGGAGGTTCAACCTCAAACAATTGTTGAGGTCAACTCAGATATTGAAGTGAATAGTTTTAGTGGTTGGTGAATTCTCGGGTGCCAAGGGGCTAAAAGACCCTTGGA |
| SARS2: Probe I1101 | NC_045512.2 | SARS2: Probe I | CTGATGAGGGAGGGAGAAACATTAATACGACTCACTATAGGGACTATAGGAGGTTCACCAGGTTCAGATATTGTTCAGATTATTAAAACTTACTGACAATTGTTAAAACTGTATACATTTCGGGTGCCAAGGGGCTAAAAGACCCTTGGA |
| SARS2: Probe I1102 | NC_045512.2 | SARS2: Probe I | CTGATGAGGGAGGGAGAAACATTAATACGACTCACTATAGGGACTATAGGCAGACTATTGAAGTGAATAGTTTTAGTGGTTGGTTATTTAAAACTTACTGACAATGTGTGGAAGAAGCTAAAACCAACAGTGGTTAATTCTCGGGTGCCAAGGGGCTAAAAGACCCTTGGA |
| SARS2: Probe I1103 | NC_045512.2 | SARS2: Probe I | CTGATGAGGGAGGGAGAAACATTAATACGACTCACTATAGGGACTATAGGGTATTTAAAACTTACTGACAATGTGTGGAAGAAGCTAAAACCAACAGTGGTTAATTCTCGGGTGCCAAGGGGCTAAAAGACCCTTGGA |
| SARS2: Probe I1104 | NC_045512.2 | SARS2: Probe I | CTGATGAGGGAGGGAGAAACATTAATACGACTCACTATAGGGACTATAGGGAAGAAGCTAAAACCAACAGTGGTTGTTAATGCCAGCCAATGTTGTTAATGCAGCCAATGTTAATCTCGGGTGCCAAGGGGCTAAAAGACCCTTGGA |
| SARS2: Probe I1105 | NC_045512.2 | SARS2: Probe I | CTGATGAGGGAGGGAGAAACATTAATACGACTCACTATAGGGACTATAGGGAAAATGCAGCCAATGTTAATGCAGCCAATGTTAATCATGGAGGAGGTTGGTAATTCTCGGGTGCCAAGGGGCTAAAAGACCCTTGGA |
| SARS2: Probe I1106 | NC_045512.2 | SARS2: Probe I | CTGATGAGGGAGGGAGAAACATTAATACGACTCACTATAGGGACTATAGGGTTAATGCAGCCAATGTTAATCATGGAGGAGGTGTTGCAGGAGCCTTAAATAAGGCTACTAACAATGCCATGCAAGTAATTCTCGGGTGCCAAGGGGCTAAAAGACCCTTGGA |
| SARS2: Probe I1107 | NC_045512.2 | SARS2: Probe I | CTGATGAGGGAGGGAGAAACATTAATACGACTCACTATAGGGACTATAGGGATCATGGAGGAGGTGTTGCAGGAGCCTTAAATAAGGCTACTAACAATGCCATGCAAGTCAAGCTTAAAGATGATGTCAAAAATTACTGTGGCGGCCAATTCTCGGGTGCCAAGGGGCTAAAAGACCCTTGGA |
| SARS2: Probe I1108 | NC_045512.2 | SARS2: Probe I | CTGATGAGGGAGGGAGAAACATTAATACGACTCACTATAGGGACTATAGGGAATGCCATGCAAGTCAAGCTTAAAGATGATGTTTTTAAAGCGGCACAATCTTGCTCTAAACACTGTCTTCATGGTGTTTTTAAGCGGACACAATCTTGCTCTAATTCTCGGGTGCCAAGGGGCTAAAAGACCCTTGGA |
| SARS2: Probe I1109 | NC_045512.2 | SARS2: Probe I | CTGATGAGGGAGGGAGAAACATTAATACGACTCACTATAGGGACTATAGGGGTGGTAGTTGCTAAACACTGTCTTCATGGTGTTTTAAGCGGACACAATCTTGCTCTCATGTTGTCGGCCCAAATGTTAAACTGTTAACAAATCTCGGGTGCCAAGGGGCTAAAAGACCCTTGGA |
| SARS2: Probe I1110 | NC_045512.2 | SARS2: Probe I | CTGATGAGGGAGGGAGAAACATTAATACGACTCACTATAGGGACTATAGGGGTAGTTGTTTTTAAGCGGACACAATCTTGCTCTCATGTTGTCGGCCCAAATGTTAAACTGTTAACAAATCTCAACTTCAACTTCTTAAGGTTAACAAATCTCGGGTGCCAAGGGGCTAAAAGACCCTTGGA |
| SARS2: Probe I1111 | NC_045512.2 | SARS2: Probe I | CTGATGAGGGAGGGAGAAACATTAATACGACTCACTATAGGGACTATAGGGGTTGTCGGCCCAAATGTTAAACTGTTAACAAATCTCAACTTCAACTTCTTAAGGTTAACAAAGGTGAAGACATTTAATCTCGGGTGCCAAGGGGCTAAAAGACCCTTGGA |
| SARS2: Probe I1112 | NC_045512.2 | SARS2: Probe I | CTGATGAGGGAGGGAGAAACATTAATACGACTCACTATAGGGACTATAGGGAATGTTAACAAAGGTGAAGACATTCAACTTCTTAAGGTTAACAAAGGTGAAGACATTTAATCAGACACGACGAAGTTAATCTCGGGTGCCAAGGGGCTAAAAGACCCTTGGA |
| SARS2: Probe I1113 | NC_045512.2 | SARS2: Probe I | CTGATGAGGGAGGGAGAAACATTAATACGACTCACTATAGGGACTATAGGGCTTAAGGAGTTGTTATGAAAAATTTAATCAGACACGACGAAGTTCTTATTATGCCAACTAATTATTGCGACCACCTATTGGTGTTGTATATTTGGTGTTGTATATTTGTGTAATTCTCGGGTGCCAAGGGGCTAAAAGACCCTTGGA |
| SARS2: Probe I1114 | NC_045512.2 | SARS2: Probe I | CTGATGAGGGAGGGAGAAACATTAATACGACTCACTATAGGGACTATAGGGGCTTAAGGAGTTGTTATGAAAAATTTAATCAGACACGACGAAGTTCTTATTATGCCAACTAATTATTGCGACCACCTATTGTCTACTACTACTCATCTTAAGAGTCATCTTAAGAGTCATCTTCAATTCTCGGGTGCCAAGGGGCTAAAAGACCCTTGGA |
| SARS2: Probe I1115 | NC_045512.2 | SARS2: Probe I | CTGATGAGGGAGGGAGAAACATTAATACGACTCACTATAGGGACTATAGGGCTGGTATTTTGATAAAATGTCGCACACACTGTTCGCACAAATGTCTTCGCACAAATGTCTTAGTAGAGTTTGTGTAGAGTTTGTGTAGACTTAGTTGTCTACTAGCTGTCTTAATCTCGGGTGCCAAGGGGCTAAAAGACCCTTGGA |
| SARS2: Probe I1116 | NC_045512.2 | SARS2: Probe I | CTGATGAGGGAGGGAGAAACATTAATACGACTCACTATAGGGACTATAGGGCTTTGATAAAATGTCGCACACACTGTTCGCACAAATGTCTTAGTAGAGTTTGTGTAGACTTAGCTGTCTTAATCTCTGAATTCTCGGGTGCCAAGGGGCTAAAAGACCCTTGGA |
| SARS2: Probe I1117 | NC_045512.2 | SARS2: Probe I | CTGATGAGGGAGGGAGAAACATTAATACGACTCACTATAGGGACTATAGGGGTTCAAGCTTTGTTTGAATGAAGAGTGAAAAGCAAGTTGAATGGAATTGAATGGAATTGAATGGAATTGGAATGGAATTCTCGGGTGCCAAGGGGCTAAAAGACCCTTGGA |
| SARS2: Probe I1118 | NC_045512.2 | SARS2: Probe I | CTGATGAGGGAGGGAGAAACATTAATACGACTCACTATAGGGACTATAGGGGACAAAATCTGTCTTTTTTGGAAATGAGTGAAAAGCAAGTTGAATGGGATATCCTGAGATTCCTAAAGCAAGTTGAATGGAATTCTCGGGTGCCAAGGGGCTAAAAGACCCTTGGA |
| SARS2: Probe I1119 | NC_045512.2 | SARS2: Probe I | CTGATGAGGGAGGGAGAAACATTAATACGACTCACTATAGGGACTATAGGGGAAATGAGTGAAAAGCAAGTTGAACAAAGGAATCGTGCTGAGATTCCTAAAGCAAGTTGAACAAAGGAATCCTGAGATTCCTAAAGCAAGTTGAATTCTCGGGTGCCAAGGGGCTAAAAGACCCTTGGA |
| SARS2: Probe I1120 | NC_045512.2 | SARS2: Probe I | CTGATGAGGGAGGGAGAAACATTAATACGACTCACTATAGGGACTATAGGGGGAAATCCTGAGATTCCTAAAGCAAGTTGAAAATGATAAAACTAACTACTTAACTGAAGAGAGACAAGAAAACAAGATGTAAGGCTAAATCTCGGGTGCCAAGGGGCTAAAAGACCCTTGGA |
| SARS2: Probe I1121 | NC_045512.2 | SARS2: Probe I | CTGATGAGGGAGGGAGAAACATTAATACGACTCACTATAGGGACTATAGGGGAGATTCCTAAAGCAAGTTGAAAATGATAAAACTAACTACTTAACTGAAGAGAGACAAGAAAACAAGATGTAAGGCTACATGGTTGTTGAAGAGTTATCTCGGGTGCCAAGGGGCTAAAAGACCCTTGGA |
| SARS2: Probe I1122 | NC_045512.2 | SARS2: Probe I | CTGATGAGGGAGGGAGAAACATTAATACGACTCACTATAGGGACTATAGGGGAAGCTTCAGTTCTCTCCATGGTTGTTGAAGAGTTACAACCTCTCTGGAAGAAAATCAACTCATTTACTTTATTTGAACATTAATGGAATTCTCGGGTGCCAAGGGGCTAAAAGACCCTTGGA |
| SARS2: Probe I1123 | NC_045512.2 | SARS2: Probe I | CTGATGAGGGAGGGAGAAACATTAATACGACTCACTATAGGGACTATAGGGGTGTTGAAGAGTTACAACCTCTCTGGAAGAAAATCAACTCATTTACTTTACTTTATTGACACTTATATTGTTACTTATATTGTTAACTTATATTGGAATTCTCGGGTGCCAAGGGGCTAAAAGACCCTTGGA |
| SARS2: Probe I1124 | NC_045512.2 | SARS2: Probe I | CTGATGAGGGAGGGAGAAACATTAATACGACTCACTATAGGGACTATAGGGGAATTCAGTTGACATTTTACTTTATTGACACTTATATTGTTGCCGCCAGATTCGTCGCCAGATTCATCCCAGATTCATCCGCCAATGGCAACTCTTTGTTACTTCTGCCAATGGCAACTCTTTGTTACTTCTGCCAATGGCAACTAATGGCAACTCTTTGTTACTTCTGCCAATGGCAACTCTTTGTTACTTCTGCCAATGGCAACTCTTTGTTACTTCTGCCAATGGCAACTAATGGCAACTCTTTGTTACTTCTGCCAGTATCTGCCGAGTATCGTCGCCAGTATCGTCATCCAGTATCGTCATCCAGATTCGTCGCCAGATTCGTCATCCAGTATCGTCATCCAGTATCGTCATCCAGTATCATCCGCCAATGGCAACTCTTTGTTACTTCTGCCAGTATCGTCATCCAGTATCATCCGCCAATGGCAACTCTTTGTTACTTCTGCCAGTATCATCCGCCAATGGCAACTAATGGCAACTCTTTGTTACTTCTGCCGAGTATTAATTGACACTTATATTGGAATTCTCGGGTGCCAAGGGGCTAAAAGACCCTTGGA |

| ASO ID | Accession | Group ID | Custom Array sequence |
|---|---|---|---|
| SARS2-Probe I.125 | NC_045512.2 | SARS2-Probe I | CTGATGAGGGAGAAACATTAATACGACTCACTATAGGGGAAAACTTGTTACTTTATATTGACATTAATGGCAATCTTCATCCAGATTCTGCCCACTCTTGTTAGTGACATTGACATCACTTTCTTAAAGTGGAATTCTCGGGTGCCAAGGGGCTAAAAGACCCCTTGGA |
| SARS2-Probe I.126 | NC_045512.2 | SARS2-Probe I | CTGATGAGGGAGAAACATTAATACGACTCACTATAGGGGGCAATCTTCATCCAGATTCTGCCCACTCTTGTTAGTGACATTGACATCACTTTCTTAAAGAAAGATGCTCCATATATAGTGGGTGATGTTTGGAATTCTCGGGTGCCAAGGGGCTAAAAGACCCCTTGGA |
| SARS2-Probe I.127 | NC_045512.2 | SARS2-Probe I | CTGATGAGGGAGAAACATTAATACGACTCACTATAGGGGTTAGTGACATTGACATCACTTTCTTAAAGAAAGATGCTCCATATATAGTGGGTGATGTTGTTCAAGAGGGTGTTTTAACTGCTGTGGTTTGGAATTCTCGGGTGCCAAGGGGCTAAAAGACCCCTTGGA |
| SARS2-Probe I.128 | NC_045512.2 | SARS2-Probe I | CTGATGAGGGAGAAACATTAATACGACTCACTATAGGGAAAGATGCTCCATATATAGTGGGTGATGTTGTTCAAGAGGGTGTTTTAACTGCTGTGGTTATACCTACTAAAAAGGCTGGTGGCACTACTTGGAATTCTCGGGTGCCAAGGGGCTAAAAGACCCCTTGGA |
| SARS2-Probe I.129 | NC_045512.2 | SARS2-Probe I | CTGATGAGGGAGAAACATTAATACGACTCACTATAGGGGTTCAAGAGGGTGTTTTAACTGCTGTGGTTATACCTACTAAAAAGGCTGGTGGCACTACTGAAATGCTAGCGAAAGCTTTGAGAAAAGTGTGGAATTCTCGGGTGCCAAGGGGCTAAAAGACCCCTTGGA |
| SARS2-Probe I.130 | NC_045512.2 | SARS2-Probe I | CTGATGAGGGAGAAACATTAATACGACTCACTATAGGGGATACCTACTAAAAAGGCTGGTGGCACTACTGAAATGCTAGCGAAAGCTTTGAGAAAAGTGCCAACAGACAATTATATAACCACTTACCCGTGGAATTCTCGGGTGCCAAGGGGCTAAAAGACCCCTTGGA |
| SARS2-Probe I.131 | NC_045512.2 | SARS2-Probe I | CTGATGAGGGAGAAACATTAATACGACTCACTATAGGGGAAATGCTAGCGAAAGCTTTGAGAAAAGTGCCAACAGACAATTATATAACCACTTACCCGGGTCAGGGTTTAAATGGTTACACTGTAGAGTGGAATTCTCGGGTGCCAAGGGGCTAAAAGACCCCTTGGA |
| SARS2-Probe I.132 | NC_045512.2 | SARS2-Probe I | CTGATGAGGGAGAAACATTAATACGACTCACTATAGGGCCAACAGACAATTATATAACCACTTACCCGGGTCAGGGTTTAAATGGTTACACTGTAGAGGAGGCAAAGACAGTGCTTAAAAAGTGTAAATGGAATTCTCGGGTGCCAAGGGGCTAAAAGACCCCTTGGA |
| SARS2-Probe I.133 | NC_045512.2 | SARS2-Probe I | CTGATGAGGGAGAAACATTAATACGACTCACTATAGGGGGTCAGGGTTTAAATGGTTACACTGTAGAGGAGGCAAAGACAGTGCTTAAAAAGTGTAAAGTGCCTTTTACATTCTACCATCTATTATCTGGAATTCTCGGGTGCCAAGGGGCTAAAAGACCCCTTGGA |
| SARS2-Probe I.134 | NC_045512.2 | SARS2-Probe I | CTGATGAGGGAGAAACATTAATACGACTCACTATAGGGGGAGGCAAAGACAGTGCTTAAAAAGTGTAAAGTGCCTTTTACATTCTACCATCTATTATCTCTAATGAGAAGCAAGAAATTCTTGGAACTTGGAATTCTCGGGTGCCAAGGGGCTAAAAGACCCCTTGGA |
| SARS2-Probe I.135 | NC_045512.2 | SARS2-Probe I | CTGATGAGGGAGAAACATTAATACGACTCACTATAGGGAGTGCCTTTTACATTCTACCATCTATTATCTCTAATGAGAAGCAAGAAATTCTTGGAACTGTTTCTTGGAATTTGCGAGAAATGCTTGCATGGAATTCTCGGGTGCCAAGGGGCTAAAAGACCCCTTGGA |
| SARS2-Probe I.136 | NC_045512.2 | SARS2-Probe I | CTGATGAGGGAGAAACATTAATACGACTCACTATAGGGTCTAATGAGAAGCAAGAAATTCTTGGAACTGTTTCTTGGAATTTGCGAGAAATGCTTGCACATGCAGAAGAAACACGCAAATTAATGCCTTGGAATTCTCGGGTGCCAAGGGGCTAAAAGACCCCTTGGA |
| SARS2-Probe I.137 | NC_045512.2 | SARS2-Probe I | CTGATGAGGGAGAAACATTAATACGACTCACTATAGGGGTTTCTTGGAATTTGCGAGAAATGCTTGCACATGCAGAAGAAACACGCAAATTAATGCCTGTCTGTGTGGAAACTAAAGCCATAGTTTCATGGAATTCTCGGGTGCCAAGGGGCTAAAAGACCCCTTGGA |
| SARS2-Probe I.138 | NC_045512.2 | SARS2-Probe I | CTGATGAGGGAGAAACATTAATACGACTCACTATAGGGCATGCAGAAGAAACACGCAAATTAATGCCTGTCTGTGTGGAAACTAAAGCCATAGTTTCAACTATACAGCGTAAATATAAGGGTATTAAATGGAATTCTCGGGTGCCAAGGGGCTAAAAGACCCCTTGGA |
| SARS2-Probe I.139 | NC_045512.2 | SARS2-Probe I | CTGATGAGGGAGAAACATTAATACGACTCACTATAGGGGTCTGTGTGGAAACTAAAGCCATAGTTTCAACTATACAGCGTAAATATAAGGGTATTAAAATACAAGAGGGTGTGGTTGATTATGGTGCTTGGAATTCTCGGGTGCCAAGGGGCTAAAAGACCCCTTGGA |
| SARS2-Probe I.140 | NC_045512.2 | SARS2-Probe I | CTGATGAGGGAGAAACATTAATACGACTCACTATAGGGACTATACAGCGTAAATATAAGGGTATTAAAATACAAGAGGGTGTGGTTGATTATGGTGCTAGATTTTACTTTTACACCAGTAAAACAACTTGGAATTCTCGGGTGCCAAGGGGCTAAAAGACCCCTTGGA |
| SARS2-Probe I.141 | NC_045512.2 | SARS2-Probe I | CTGATGAGGGAGAAACATTAATACGACTCACTATAGGGATACAAGAGGGTGTGGTTGATTATGGTGCTAGATTTTACTTTTACACCAGTAAAACAACTGTAGCGTCACTTATCAACACACTTAACGATTGGAATTCTCGGGTGCCAAGGGGCTAAAAGACCCCTTGGA |
| SARS2-Probe I.142 | NC_045512.2 | SARS2-Probe I | CTGATGAGGGAGAAACATTAATACGACTCACTATAGGGAGATTTTACTTTTACACCAGTAAAACAACTGTAGCGTCACTTATCAACACACTTAACGATCTAAATGAAACTCTTGTTACAATGCCACTTTGGAATTCTCGGGTGCCAAGGGGCTAAAAGACCCCTTGGA |
| SARS2-Probe I.143 | NC_045512.2 | SARS2-Probe I | CTGATGAGGGAGAAACATTAATACGACTCACTATAGGGGTAGCGTCACTTATCAACACACTTAACGATCTAAATGAAACTCTTGTTACAATGCCACTTGGCTATGTAACACATGGCTTAAATTTGGAATGGAATTCTCGGGTGCCAAGGGGCTAAAAGACCCCTTGGA |
| SARS2-Probe I.144 | NC_045512.2 | SARS2-Probe I | CTGATGAGGGAGAAACATTAATACGACTCACTATAGGGCTAAATGAAACTCTTGTTACAATGCCACTTGGCTATGTAACACATGGCTTAAATTTGGAAGAAGCTGCTCGGTATATGAGATCTCTCAAATGGAATTCTCGGGTGCCAAGGGGCTAAAAGACCCCTTGGA |
| SARS2-Probe I.145 | NC_045512.2 | SARS2-Probe I | CTGATGAGGGAGAAACATTAATACGACTCACTATAGGGGGGCTATGTAACACATGGCTTAAATTTGGAAGAAGCTGCTCGGTATATGAGATCTCTCAAAGTGCCAGCTACAGTTTCTGTTTCTTCACCTTGGAATTCTCGGGTGCCAAGGGGCTAAAAGACCCCTTGGA |
| SARS2-Probe I.146 | NC_045512.2 | SARS2-Probe I | CTGATGAGGGAGAAACATTAATACGACTCACTATAGGGGAAGCTGCTCGGTATATGAGATCTCTCAAAGTGCCAGCTACAGTTTCTGTTTCTTCACCTGATGCTGTTACAGCGTATAATGGTTATCTTTGGAATTCTCGGGTGCCAAGGGGCTAAAAGACCCCTTGGA |
| SARS2-Probe I.147 | NC_045512.2 | SARS2-Probe I | CTGATGAGGGAGAAACATTAATACGACTCACTATAGGGGTGCCAGCTACAGTTTCTGTTTCTTCACCTGATGCTGTTACAGCGTATAATGGTTATCTTACTTCTTCTTCTAAAACACCTGAAGAACATTGGAATTCTCGGGTGCCAAGGGGCTAAAAGACCCCTTGGA |
| SARS2-Probe I.148 | NC_045512.2 | SARS2-Probe I | CTGATGAGGGAGAAACATTAATACGACTCACTATAGGGGATGCTGTTACAGCGTATAATGGTTATCTTACTTCTTCTTCTAAAACACCTGAAGAACATTTTATTGAAACCATCTCACTTGCTGGTTCCTGGAATTCTCGGGTGCCAAGGGGCTAAAAGACCCCTTGGA |
| SARS2-Probe I.149 | NC_045512.2 | SARS2-Probe I | CTGATGAGGGAGAAACATTAATACGACTCACTATAGGGACTTCTTCTTCTAAAACACCTGAAGAACATTTTATTGAAACCATCTCACTTGCTGGTTCCTATAAAGATTGGTCCTATTCTGGACAATCTTGGAATTCTCGGGTGCCAAGGGGCTAAAAGACCCCTTGGA |

| ASO ID | Accession | Group ID | Custom Array sequence |
|---|---|---|---|
| SARS2-Probe I.150 | NC_045512.2 | SARS2-Probe I | CTGATGAGGGAGAAACATTAATACGACTCACTATAGGGTTTATTGAAACCATCTCACTTGCTGGTTCCTATAAAGATTGGTCCTATTCTGGACAATCTACACAACTAGGTATAGAATTTCTTAAGAGATGGAATTCTCGGGTGCCAAGGGGCTAAAAGACCCTTGGA |
| SARS2-Probe I.151 | NC_045512.2 | SARS2-Probe I | CTGATGAGGGAGAAACATTAATACGACTCACTATAGGGTATAAAGATTGGTCCTATTCTGGACAATCTACACAACTAGGTATAGAATTTCTTAAGAGAGGTGATAAAAGTGTATATTACACTAGTAATTGGAATTCTCGGGTGCCAAGGGGCTAAAAGACCCTTGGA |
| SARS2-Probe I.152 | NC_045512.2 | SARS2-Probe I | CTGATGAGGGAGAAACATTAATACGACTCACTATAGGGACACAACTAGGTATAGAATTTCTTAAGAGAGGTGATAAAAGTGTATATTACACTAGTAATCCTACCACATTCCACCTAGATGGTGAAGTTTGGAATTCTCGGGTGCCAAGGGGCTAAAAGACCCTTGGA |
| SARS2-Probe I.153 | NC_045512.2 | SARS2-Probe I | CTGATGAGGGAGAAACATTAATACGACTCACTATAGGGGGGTGATAAAAGTGTATATTACACTAGTAATCCTACCACATTCCACCTAGATGGTGAAGTTATCACCTTTGACAATCTTAAGACACTTCTTTGGAATTCTCGGGTGCCAAGGGGCTAAAAGACCCTTGGA |
| SARS2-Probe I.154 | NC_045512.2 | SARS2-Probe I | CTGATGAGGGAGAAACATTAATACGACTCACTATAGGGCCTACCACATTCCACCTAGATGGTGAAGTTATCACCTTTGACAATCTTAAGACACTTCTTTCTTTGAGAGAAGTGAGGACTATTAAGGTGTGGAATTCTCGGGTGCCAAGGGGCTAAAAGACCCTTGGA |
| SARS2-Probe I.155 | NC_045512.2 | SARS2-Probe I | CTGATGAGGGAGAAACATTAATACGACTCACTATAGGGATCACCTTTGACAATCTTAAGACACTTCTTTCTTTGAGAGAAGTGAGGACTATTAAGGTGTTTACAACAGTAGACAACATTAACCTCCACTGGAATTCTCGGGTGCCAAGGGGCTAAAAGACCCTTGGA |
| SARS2-Probe I.156 | NC_045512.2 | SARS2-Probe I | CTGATGAGGGAGAAACATTAATACGACTCACTATAGGGTCTTTGAGAGAAGTGAGGACTATTAAGGTGTTTACAACAGTAGACAACATTAACCTCCACACGCAAGTTGTGGACATGTCAATGACATATTGGAATTCTCGGGTGCCAAGGGGCTAAAAGACCCTTGGA |
| SARS2-Probe I.157 | NC_045512.2 | SARS2-Probe I | CTGATGAGGGAGAAACATTAATACGACTCACTATAGGGTTTACAACAGTAGACAACATTAACCTCCACACGCAAGTTGTGGACATGTCAATGACATATGGACAACAGTTTGGTCCAACTTATTTGGATTGGAATTCTCGGGTGCCAAGGGGCTAAAAGACCCTTGGA |
| SARS2-Probe I.158 | NC_045512.2 | SARS2-Probe I | CTGATGAGGGAGAAACATTAATACGACTCACTATAGGGACGCAAGTTGTGGACATGTCAATGACATATGGACAACAGTTTGGTCCAACTTATTTGGATGGAGCTGATGTTACTAAAATAAAACCTCATTGGAATTCTCGGGTGCCAAGGGGCTAAAAGACCCTTGGA |
| SARS2-Probe I.159 | NC_045512.2 | SARS2-Probe I | CTGATGAGGGAGAAACATTAATACGACTCACTATAGGGGGGCAACAGTTTGGTCCAACTTATTTGGATGGAGCTGATGTTACTAAAATAAAACCTCATAATTCACATGAAGGTAAAACATTTTATGTTTGGAATTCTCGGGTGCCAAGGGGCTAAAAGACCCTTGGA |
| SARS2-Probe I.160 | NC_045512.2 | SARS2-Probe I | CTGATGAGGGAGAAACATTAATACGACTCACTATAGGGGGGAGCTGATGTTACTAAAATAAAACCTCATAATTCACATGAAGGTAAAACATTTTATGTTTACCTAATGATGACACTCTACGTGTTGAGTGGAATTCTCGGGTGCCAAGGGGCTAAAAGACCCTTGGA |
| SARS2-Probe I.161 | NC_045512.2 | SARS2-Probe I | CTGATGAGGGAGAAACATTAATACGACTCACTATAGGGAATTCACATGAAGGTAAAACATTTTATGTTTACCTAATGATGACACTCTACGTGTTGAGGCTTTTGAGTACTACCACACAACTGATCCTTGGAATTCTCGGGTGCCAAGGGGCTAAAAGACCCTTGGA |
| SARS2-Probe I.162 | NC_045512.2 | SARS2-Probe I | CTGATGAGGGAGAAACATTAATACGACTCACTATAGGGTTACCTAATGATGACACTCTACGTGTTGAGGCTTTTGAGTACTACCACACAACTGATCCTAGTTTTCTGGGTAGGTACATGTCAGCATTATGGAATTCTCGGGTGCCAAGGGGCTAAAAGACCCTTGGA |
| SARS2-Probe I.163 | NC_045512.2 | SARS2-Probe I | CTGATGAGGGAGAAACATTAATACGACTCACTATAGGGGCTTTTGAGTACTACCACACAACTGATCCTAGTTTTCTGGGTAGGTACATGTCAGCATTAAATCACACTAAAAAGTGGAAATACCCACAATGGAATTCTCGGGTGCCAAGGGGCTAAAAGACCCTTGGA |
| SARS2-Probe I.164 | NC_045512.2 | SARS2-Probe I | CTGATGAGGGAGAAACATTAATACGACTCACTATAGGGAGTTTTCTGGGTAGGTACATGTCAGCATTAAATCACACTAAAAAGTGGAAATACCCACAAGTTAATGGTTTAACTTCTATTAAATGGGCATGGAATTCTCGGGTGCCAAGGGGCTAAAAGACCCTTGGA |
| SARS2-Probe I.165 | NC_045512.2 | SARS2-Probe I | CTGATGAGGGAGAAACATTAATACGACTCACTATAGGGAATCACACTAAAAAGTGGAAATACCCACAAGTTAATGGTTTAACTTCTATTAAATGGGCAGATAACAACTGTTATCTTGCCACTGCATTGTGGAATTCTCGGGTGCCAAGGGGCTAAAAGACCCTTGGA |
| SARS2-Probe I.166 | NC_045512.2 | SARS2-Probe I | CTGATGAGGGAGAAACATTAATACGACTCACTATAGGGGTTAATGGTTTAACTTCTATTAAATGGGCAGATAACAACTGTTATCTTGCCACTGCATTGTTAACACTCCAACAAATAGAGTTGAAGTTTGGAATTCTCGGGTGCCAAGGGGCTAAAAGACCCTTGGA |
| SARS2-Probe I.167 | NC_045512.2 | SARS2-Probe I | CTGATGAGGGAGAAACATTAATACGACTCACTATAGGGGATAACAACTGTTATCTTGCCACTGCATTGTTAACACTCCAACAAATAGAGTTGAAGTTTAATCCACCTGCTCTACAAGATGCTTATTACTGGAATTCTCGGGTGCCAAGGGGCTAAAAGACCCTTGGA |
| SARS2-Probe I.168 | NC_045512.2 | SARS2-Probe I | CTGATGAGGGAGAAACATTAATACGACTCACTATAGGGTTAACACTCCAACAAATAGAGTTGAAGTTTAATCCACCTGCTCTACAAGATGCTTATTACAGAGCAAGGGCTGGTGAAGCTGCTAACTTTTGGAATTCTCGGGTGCCAAGGGGCTAAAAGACCCTTGGA |
| SARS2-Probe I.169 | NC_045512.2 | SARS2-Probe I | CTGATGAGGGAGAAACATTAATACGACTCACTATAGGGAATCCACCTGCTCTACAAGATGCTTATTACAGAGCAAGGGCTGGTGAAGCTGCTAACTTTTGTGCACTTATCTTAGCCTACTGTAATAAGTGGAATTCTCGGGTGCCAAGGGGCTAAAAGACCCTTGGA |
| SARS2-Probe I.170 | NC_045512.2 | SARS2-Probe I | CTGATGAGGGAGAAACATTAATACGACTCACTATAGGGAGAGCAAGGGCTGGTGAAGCTGCTAACTTTTGTGCACTTATCTTAGCCTACTGTAATAAGACAGTAGGTGAGTTAGGTGATGTTAGAGAATGGAATTCTCGGGTGCCAAGGGGCTAAAAGACCCTTGGA |
| SARS2-Probe I.171 | NC_045512.2 | SARS2-Probe I | CTGATGAGGGAGAAACATTAATACGACTCACTATAGGGTGTGCACTTATCTTAGCCTACTGTAATAAGACAGTAGGTGAGTTAGGTGATGTTAGAGAAACAATGAGTTACTTGTTTCAACATGCCAATTGGAATTCTCGGGTGCCAAGGGGCTAAAAGACCCTTGGA |
| SARS2-Probe I.172 | NC_045512.2 | SARS2-Probe I | CTGATGAGGGAGAAACATTAATACGACTCACTATAGGGACAGTAGGTGAGTTAGGTGATGTTAGAGAAACAATGAGTTACTTGTTTCAACATGCCAATTTAGATTCTTGCAAAAGAGTCTTGAACGTGTGGAATTCTCGGGTGCCAAGGGGCTAAAAGACCCTTGGA |
| SARS2-Probe I.173 | NC_045512.2 | SARS2-Probe I | CTGATGAGGGAGAAACATTAATACGACTCACTATAGGGACAATGAGTTACTTGTTTCAACATGCCAATTTAGATTCTTGCAAAAGAGTCTTGAACGTGGTGTGTAAAACTTGTGGACAACAGCAGACATGGAATTCTCGGGTGCCAAGGGGCTAAAAGACCCTTGGA |
| SARS2-Probe I.174 | NC_045512.2 | SARS2-Probe I | CTGATGAGGGAGAAACATTAATACGACTCACTATAGGGTTAGATTCTTGCAAAAGAGTCTTGAACGTGGTGTGTAAAACTTGTGGACAACAGCAGACAACCCTTAAGGGTGTAGAAGCTGTTATGTACTGGAATTCTCGGGTGCCAAGGGGCTAAAAGACCCTTGGA |

| ASO ID | Accession | Group ID | Custom Array sequence |
|---|---|---|---|
| SARS2-Probe I.175 | NC_045512.2 | SARS2-Probe I | CTGATGAGGGAGAAACATTAATACGACTCACTATAGGGGTGTGTAAAACTTGTGGACAACAGCAGACAACCCTTAAGGGTGTAGAAGCTGTTATGTACATGGGCACACTTTCTTATGAACAATTTAAGTGGAATTCTCGGGTGCCAAGGGGCTAAAAGACCCTTGGA |
| SARS2-Probe I.176 | NC_045512.2 | SARS2-Probe I | CTGATGAGGGAGAAACATTAATACGACTCACTATAGGGACCCTTAAGGGTGTAGAAGCTGTTATGTACATGGGCACACTTTCTTATGAACAATTTAAGAAAGGTGTTCAGATACCTTGTACGTGTGGTTGGAATTCTCGGGTGCCAAGGGGCTAAAAGACCCTTGGA |
| SARS2-Probe I.177 | NC_045512.2 | SARS2-Probe I | CTGATGAGGGAGAAACATTAATACGACTCACTATAGGGGATGGGCACACTTTCTTATGAACAATTTAAGAAAGGTGTTCAGATACCTTGTACGTGTGGTAAACAAGCTACAAAATATCTAGTACAACAGTGGAATTCTCGGGTGCCAAGGGGCTAAAAGACCCTTGGA |
| SARS2-Probe I.178 | NC_045512.2 | SARS2-Probe I | CTGATGAGGGAGAAACATTAATACGACTCACTATAGGGAAAGGTGTTCAGATACCTTGTACGTGTGGTAAACAAGCTACAAAATATCTAGTACAACAGGAGTCACCTTTTGTTATGATGTCAGCACCATGGAATTCTCGGGTGCCAAGGGGCTAAAAGACCCTTGGA |
| SARS2-Probe I.179 | NC_045512.2 | SARS2-Probe I | CTGATGAGGGAGAAACATTAATACGACTCACTATAGGGAAACAAGCTACAAAATATCTAGTACAACAGGAGTCACCTTTTGTTATGATGTCAGCACCACCTGCTCAGTATGAACTTAAGCATGGTACATGGAATTCTCGGGTGCCAAGGGGCTAAAAGACCCTTGGA |
| SARS2-Probe I.180 | NC_045512.2 | SARS2-Probe I | CTGATGAGGGAGAAACATTAATACGACTCACTATAGGGGAGTCACCTTTTGTTATGATGTCAGCACCACCTGCTCAGTATGAACTTAAGCATGGTACATTTACTTGTGCTAGTGAGTACACTGGTAATTGGAATTCTCGGGTGCCAAGGGGCTAAAAGACCCTTGGA |
| SARS2-Probe I.181 | NC_045512.2 | SARS2-Probe I | CTGATGAGGGAGAAACATTAATACGACTCACTATAGGGCCTGCTCAGTATGAACTTAAGCATGGTACATTTACTTGTGCTAGTGAGTACACTGGTAATTACCAGTGTGGTCACTATAAACATATAACTTGGAATTCTCGGGTGCCAAGGGGCTAAAAGACCCTTGGA |
| SARS2-Probe I.182 | NC_045512.2 | SARS2-Probe I | CTGATGAGGGAGAAACATTAATACGACTCACTATAGGGTTTACTTGTGCTAGTGAGTACACTGGTAATTACCAGTGTGGTCACTATAAACATATAACTTCTAAAGAAACTTTGTATTGCATAGACGGTGGAATTCTCGGGTGCCAAGGGGCTAAAAGACCCTTGGA |
| SARS2-Probe I.183 | NC_045512.2 | SARS2-Probe I | CTGATGAGGGAGAAACATTAATACGACTCACTATAGGGTACCAGTGTGGTCACTATAAACATATAACTTCTAAAGAAACTTTGTATTGCATAGACGGTGCTTTACTTACAAAGTCCTCAGAATACAAATGGAATTCTCGGGTGCCAAGGGGCTAAAAGACCCTTGGA |
| SARS2-Probe I.184 | NC_045512.2 | SARS2-Probe I | CTGATGAGGGAGAAACATTAATACGACTCACTATAGGGTCTAAAGAAACTTTGTATTGCATAGACGGTGCTTTACTTACAAAGTCCTCAGAATACAAAGGTCCTATTACGGATGTTTTCTACAAAGAATGGAATTCTCGGGTGCCAAGGGGCTAAAAGACCCTTGGA |
| SARS2-Probe I.185 | NC_045512.2 | SARS2-Probe I | CTGATGAGGGAGAAACATTAATACGACTCACTATAGGGGCTTTACTTACAAAGTCCTCAGAATACAAAGGTCCTATTACGGATGTTTTCTACAAAGAAAACAGTTACACAACAACCATAAAACCAGTTTGGAATTCTCGGGTGCCAAGGGGCTAAAAGACCCTTGGA |
| SARS2-Probe I.186 | NC_045512.2 | SARS2-Probe I | CTGATGAGGGAGAAACATTAATACGACTCACTATAGGGGGGTCCTATTACGGATGTTTTCTACAAAGAAAACAGTTACACAACAACCATAAAACCAGTTACTTATAAATTGGATGGTGTTGTTTGTACATGGAATTCTCGGGTGCCAAGGGGCTAAAAGACCCTTGGA |
| SARS2-Probe I.187 | NC_045512.2 | SARS2-Probe I | CTGATGAGGGAGAAACATTAATACGACTCACTATAGGGAACAGTTACACAACAACCATAAAACCAGTTACTTATAAATTGGATGGTGTTGTTTGTACAGAAATTGACCCTAAGTTGGACAATTATTATTGGAATTCTCGGGTGCCAAGGGGCTAAAAGACCCTTGGA |
| SARS2-Probe I.188 | NC_045512.2 | SARS2-Probe I | CTGATGAGGGAGAAACATTAATACGACTCACTATAGGGACTTATAAATTGGATGGTGTTGTTTGTACAGAAATTGACCCTAAGTTGGACAATTATTATAAGAAAGACAATTCTTATTTCACAGAGCAATGGAATTCTCGGGTGCCAAGGGGCTAAAAGACCCTTGGA |
| SARS2-Probe I.189 | NC_045512.2 | SARS2-Probe I | CTGATGAGGGAGAAACATTAATACGACTCACTATAGGGGAAATTGACCCTAAGTTGGACAATTATTATAAGAAAGACAATTCTTATTTCACAGAGCAACCAATTGATCTTGTACCAAACCAACCATATTGGAATTCTCGGGTGCCAAGGGGCTAAAAGACCCTTGGA |
| SARS2-Probe I.190 | NC_045512.2 | SARS2-Probe I | CTGATGAGGGAGAAACATTAATACGACTCACTATAGGGGAAGAAAGACAATTCTTATTTCACAGAGCAACCAATTGATCTTGTACCAAACCAACCATATCCAAACGCAAGCTTCGATAATTTTAAGTTTTGGAATTCTCGGGTGCCAAGGGGCTAAAAGACCCTTGGA |
| SARS2-Probe I.191 | NC_045512.2 | SARS2-Probe I | CTGATGAGGGAGAAACATTAATACGACTCACTATAGGGGCCAATTGATCTTGTACCAAACCAACCATATCCAAACGCAAGCTTCGATAATTTTAAGTTTGTATGTGATAATATCAAATTTGCTGATGATTGGAATTCTCGGGTGCCAAGGGGCTAAAAGACCCTTGGA |
| SARS2-Probe I.192 | NC_045512.2 | SARS2-Probe I | CTGATGAGGGAGAAACATTAATACGACTCACTATAGGGCCAAACGCAAGCTTCGATAATTTTAAGTTTGTATGTGATAATATCAAATTTGCTGATGATTTAAACCAGTTAACTGGTTATAAGAAACCTTGGAATTCTCGGGTGCCAAGGGGCTAAAAGACCCTTGGA |
| SARS2-Probe I.193 | NC_045512.2 | SARS2-Probe I | CTGATGAGGGAGAAACATTAATACGACTCACTATAGGGGTATGTGATAATATCAAATTTGCTGATGATTTAAACCAGTTAACTGGTTATAAGAAACCTGCTTCAAGAGAGCTTAAAGTTACATTTTTCTGGAATTCTCGGGTGCCAAGGGGCTAAAAGACCCTTGGA |
| SARS2-Probe I.194 | NC_045512.2 | SARS2-Probe I | CTGATGAGGGAGAAACATTAATACGACTCACTATAGGGTTAAACCAGTTAACTGGTTATAAGAAACCTGCTTCAAGAGAGCTTAAAGTTACATTTTTCCCTGACTTAAATGGTGATGTGGTGGCTATTTGGAATTCTCGGGTGCCAAGGGGCTAAAAGACCCTTGGA |
| SARS2-Probe I.195 | NC_045512.2 | SARS2-Probe I | CTGATGAGGGAGAAACATTAATACGACTCACTATAGGGGCTTCAAGAGAGCTTAAAGTTACATTTTTCCCTGACTTAAATGGTGATGTGGTGGCTATTGATTATAAACACTACACACCCTCTTTTAAGTGGAATTCTCGGGTGCCAAGGGGCTAAAAGACCCTTGGA |
| SARS2-Probe I.196 | NC_045512.2 | SARS2-Probe I | CTGATGAGGGAGAAACATTAATACGACTCACTATAGGGCCTGACTTAAATGGTGATGTGGTGGCTATTGATTATAAACACTACACACCCTCTTTTAAGAAAAGGAGCTAAATTGTTACATAAACCTATTTGGAATTCTCGGGTGCCAAGGGGCTAAAAGACCCTTGGA |
| SARS2-Probe I.197 | NC_045512.2 | SARS2-Probe I | CTGATGAGGGAGAAACATTAATACGACTCACTATAGGGGATTATAAACACTACACACCCTCTTTTAAGAAAAGGAGCTAAATTGTTACATAAACCTATTGTTTGGCATGTTAACAATGCAACTAATAAATGGAATTCTCGGGTGCCAAGGGGCTAAAAGACCCTTGGA |
| SARS2-Probe I.198 | NC_045512.2 | SARS2-Probe I | CTGATGAGGGAGAAACATTAATACGACTCACTATAGGGAAAGGAGCTAAATTGTTACATAAACCTATTGTTTGGCATGTTAACAATGCAACTAATAAATGCCACGTATAAACCAAATACCTGGTGTATATGGAATTCTCGGGTGCCAAGGGGCTAAAAGACCCTTGGA |
| SARS2-Probe I.199 | NC_045512.2 | SARS2-Probe I | CTGATGAGGGAGAAACATTAATACGACTCACTATAGGGGTTTGGCATGTTAACAATGCAACTAATAAATGCCACGTATAAACCAAATACCTGGTGTATACGTTGTCTTTGGAGCACAAAACCAGTTGAATGGAATTCTCGGGTGCCAAGGGGCTAAAAGACCCTTGGA |

| ASO ID | Accession | Group ID | Custom Array sequence |
|---|---|---|---|
| SARS2-Probe I.200 | NC_045512.2 | SARS2-Probe I | CTGATGAGGGAGAAACATTAATACGACTCACTATAGGGGCCACGTATAAACCAAATACCTGGTGTATACGTTGTCTTTGGAGCACAAAACCAGTTGAAACATCAAATTCGTTTGATGTACTGAAGTCATGGAATTCTCGGGTGCCAAGGGGCTAAAAGACCCTTGGA |
| SARS2-Probe I.201 | NC_045512.2 | SARS2-Probe I | CTGATGAGGGAGAAACATTAATACGACTCACTATAGGGCGTTGTCTTTGGAGCACAAAACCAGTTGAAACATCAAATTCGTTTGATGTACTGAAGTCAGAGGACGCGCAGGGAATGGATAATCTTGCCTGGAATTCTCGGGTGCCAAGGGGCTAAAAGACCCTTGGA |
| SARS2-Probe I.202 | NC_045512.2 | SARS2-Probe I | CTGATGAGGGAGAAACATTAATACGACTCACTATAGGGACATCAAATTCGTTTGATGTACTGAAGTCAGAGGACGCGCAGGGAATGGATAATCTTGCCTGCGAAGATCTAAAACCAGTCTCTGAAGAATGGAATTCTCGGGTGCCAAGGGGCTAAAAGACCCTTGGA |
| SARS2-Probe I.203 | NC_045512.2 | SARS2-Probe I | CTGATGAGGGAGAAACATTAATACGACTCACTATAGGGGGAGGACGCGCAGGGAATGGATAATCTTGCCTGCGAAGATCTAAAACCAGTCTCTGAAGAAGTAGTGGAAAATCCTACCATACAGAAAGACTGGAATTCTCGGGTGCCAAGGGGCTAAAAGACCCTTGGA |
| SARS2-Probe I.204 | NC_045512.2 | SARS2-Probe I | CTGATGAGGGAGAAACATTAATACGACTCACTATAGGGTGCGAAGATCTAAAACCAGTCTCTGAAGAAGTAGTGGAAAATCCTACCATACAGAAAGACGTTCTTGAGTGTAATGTGAAAACTACCGAATGGAATTCTCGGGTGCCAAGGGGCTAAAAGACCCTTGGA |
| SARS2-Probe I.205 | NC_045512.2 | SARS2-Probe I | CTGATGAGGGAGAAACATTAATACGACTCACTATAGGGGTAGTGGAAAATCCTACCATACAGAAAGACGTTCTTGAGTGTAATGTGAAAACTACCGAAGTTGTAGGAGACATTATACTTAAACCAGCATGGAATTCTCGGGTGCCAAGGGGCTAAAAGACCCTTGGA |
| SARS2-Probe I.206 | NC_045512.2 | SARS2-Probe I | CTGATGAGGGAGAAACATTAATACGACTCACTATAGGGGTTCTTGAGTGTAATGTGAAAACTACCGAAGTTGTAGGAGACATTATACTTAAACCAGCAAATAATAGTTTAAAAATTACAGAAGAGGTTTGGAATTCTCGGGTGCCAAGGGGCTAAAAGACCCTTGGA |
| SARS2-Probe I.207 | NC_045512.2 | SARS2-Probe I | CTGATGAGGGAGAAACATTAATACGACTCACTATAGGGGTTGTAGGAGACATTATACTTAAACCAGCAAATAATAGTTTAAAAATTACAGAAGAGGTTGGCCACACAGATCTAATGGCTGCTTATGTATGGAATTCTCGGGTGCCAAGGGGCTAAAAGACCCTTGGA |
| SARS2-Probe I.208 | NC_045512.2 | SARS2-Probe I | CTGATGAGGGAGAAACATTAATACGACTCACTATAGGGAATAATAGTTTAAAAATTACAGAAGAGGTTGGCCACACAGATCTAATGGCTGCTTATGTAGACAATTCTAGTCTTACTATTAAGAAACCTTGGAATTCTCGGGTGCCAAGGGGCTAAAAGACCCTTGGA |
| SARS2-Probe I.209 | NC_045512.2 | SARS2-Probe I | CTGATGAGGGAGAAACATTAATACGACTCACTATAGGGGGGCCACACAGATCTAATGGCTGCTTATGTAGACAATTCTAGTCTTACTATTAAGAAACCTAATGAATTATCTAGAGTATTAGGTTTGAAATGGAATTCTCGGGTGCCAAGGGGCTAAAAGACCCTTGGA |
| SARS2-Probe I.210 | NC_045512.2 | SARS2-Probe I | CTGATGAGGGAGAAACATTAATACGACTCACTATAGGGGACAATTCTAGTCTTACTATTAAGAAACCTAATGAATTATCTAGAGTATTAGGTTTGAAAACCCTTGCTACTCATGGTTTAGCTGCTGTTTGGAATTCTCGGGTGCCAAGGGGCTAAAAGACCCTTGGA |
| SARS2-Probe I.211 | NC_045512.2 | SARS2-Probe I | CTGATGAGGGAGAAACATTAATACGACTCACTATAGGGAATGAATTATCTAGAGTATTAGGTTTGAAAACCCTTGCTACTCATGGTTTAGCTGCTGTTAATAGTGTCCCTTGGGATACTATAGCTAATTGGAATTCTCGGGTGCCAAGGGGCTAAAAGACCCTTGGA |
| SARS2-Probe I.212 | NC_045512.2 | SARS2-Probe I | CTGATGAGGGAGAAACATTAATACGACTCACTATAGGGACCCTTGCTACTCATGGTTTAGCTGCTGTTAATAGTGTCCCTTGGGATACTATAGCTAATTATGCTAAGCCTTTTCTTAACAAAGTTGTTTGGAATTCTCGGGTGCCAAGGGGCTAAAAGACCCTTGGA |
| SARS2-Probe I.213 | NC_045512.2 | SARS2-Probe I | CTGATGAGGGAGAAACATTAATACGACTCACTATAGGGAATAGTGTCCCTTGGGATACTATAGCTAATTATGCTAAGCCTTTTCTTAACAAAGTTGTTAGTACAACTACTAACATAGTTACACGGTGTTGGAATTCTCGGGTGCCAAGGGGCTAAAAGACCCTTGGA |
| SARS2-Probe I.214 | NC_045512.2 | SARS2-Probe I | CTGATGAGGGAGAAACATTAATACGACTCACTATAGGGTATGCTAAGCCTTTTCTTAACAAAGTTGTTAGTACAACTACTAACATAGTTACACGGTGTTAAACCGTGTTTGTACTAATTATATGCCTTGGAATTCTCGGGTGCCAAGGGGCTAAAAGACCCTTGGA |
| SARS2-Probe I.215 | NC_045512.2 | SARS2-Probe I | CTGATGAGGGAGAAACATTAATACGACTCACTATAGGGAGTACAACTACTAACATAGTTACACGGTGTTTAAACCGTGTTTGTACTAATTATATGCCTTATTTCTTTACTTTATTGCTACAATTGTGTTGGAATTCTCGGGTGCCAAGGGGCTAAAAGACCCTTGGA |
| SARS2-Probe I.216 | NC_045512.2 | SARS2-Probe I | CTGATGAGGGAGAAACATTAATACGACTCACTATAGGGTTAAACCGTGTTTGTACTAATTATATGCCTTATTTCTTTACTTTATTGCTACAATTGTGTACTTTTACTAGAAGTACAAATTCTAGAATTTGGAATTCTCGGGTGCCAAGGGGCTAAAAGACCCTTGGA |
| SARS2-Probe I.217 | NC_045512.2 | SARS2-Probe I | CTGATGAGGGAGAAACATTAATACGACTCACTATAGGGTATTTCTTTACTTTATTGCTACAATTGTGTACTTTTACTAGAAGTACAAATTCTAGAATTAAAGCATCTATGCCGACTACTATAGCAAAGTGGAATTCTCGGGTGCCAAGGGGCTAAAAGACCCTTGGA |
| SARS2-Probe I.218 | NC_045512.2 | SARS2-Probe I | CTGATGAGGGAGAAACATTAATACGACTCACTATAGGGACTTTTACTAGAAGTACAAATTCTAGAATTAAAGCATCTATGCCGACTACTATAGCAAAGAATACTGTTAAGAGTGTCGGTAAATTTTGTTGGAATTCTCGGGTGCCAAGGGGCTAAAAGACCCTTGGA |
| SARS2-Probe I.219 | NC_045512.2 | SARS2-Probe I | CTGATGAGGGAGAAACATTAATACGACTCACTATAGGGAAAGCATCTATGCCGACTACTATAGCAAAGAATACTGTTAAGAGTGTCGGTAAATTTTGTCTAGAGGCTTCATTTAATTATTTGAAGTCATGGAATTCTCGGGTGCCAAGGGGCTAAAAGACCCTTGGA |
| SARS2-Probe I.220 | NC_045512.2 | SARS2-Probe I | CTGATGAGGGAGAAACATTAATACGACTCACTATAGGGAATACTGTTAAGAGTGTCGGTAAATTTTGTCTAGAGGCTTCATTTAATTATTTGAAGTCACCTAATTTTTCTAAACTGATAAATATTATATGGAATTCTCGGGTGCCAAGGGGCTAAAAGACCCTTGGA |
| SARS2-Probe I.221 | NC_045512.2 | SARS2-Probe I | CTGATGAGGGAGAAACATTAATACGACTCACTATAGGGCTAGAGGCTTCATTTAATTATTTGAAGTCACCTAATTTTTCTAAACTGATAAATATTATAATTTGGTTTTTACTATTAAGTGTTTGCCTATGGAATTCTCGGGTGCCAAGGGGCTAAAAGACCCTTGGA |
| SARS2-Probe I.222 | NC_045512.2 | SARS2-Probe I | CTGATGAGGGAGAAACATTAATACGACTCACTATAGGGCCTAATTTTTCTAAACTGATAAATATTATAATTTGGTTTTTACTATTAAGTGTTTGCCTAGGTTCTTTAATCTACTCAACCGCTGCTTTATGGAATTCTCGGGTGCCAAGGGGCTAAAAGACCCTTGGA |
| SARS2-Probe I.223 | NC_045512.2 | SARS2-Probe I | CTGATGAGGGAGAAACATTAATACGACTCACTATAGGGATTTGGTTTTTACTATTAAGTGTTTGCCTAGGTTCTTTAATCTACTCAACCGCTGCTTTAGGTGTTTTAATGTCTAATTTAGGCATGCCTTGGAATTCTCGGGTGCCAAGGGGCTAAAAGACCCTTGGA |
| SARS2-Probe I.224 | NC_045512.2 | SARS2-Probe I | CTGATGAGGGAGAAACATTAATACGACTCACTATAGGGGGTTCTTTAATCTACTCAACCGCTGCTTTAGGTGTTTTAATGTCTAATTTAGGCATGCCTTCTTACTGTACTGGTTACAGAGAAGGCTATTGGAATTCTCGGGTGCCAAGGGGCTAAAAGACCCTTGGA |

| ASO ID | Accession | Group ID | Custom Array sequence |
|---|---|---|---|
| SARS2-Probe I.225 | NC_045512.2 | SARS2-Probe I | CTGATGAGGGAGAAACATTAATACGACTCACTATAGGGGGTGTGTTTAATGTCTAATTTAGGCATGCCTTCTTACTGTACTGGTTACAGAGAAGGCTATTTGAACTCTACTAATGTCACTATTGCAACCTGGAATTCTCGGGTGCCAAGGGGCTAAAAGACCCTTGGA |
| SARS2-Probe I.226 | NC_045512.2 | SARS2-Probe I | CTGATGAGGGAGAAACATTAATACGACTCACTATAGGGTCTTACTGTACTGGTTACAGAGAAGGCTATTTGAACTCTACTAATGTCACTATTGCAACCTACTGTACTGGTTCTATACCTTGTAGTGTTTGGAATTCTCGGGTGCCAAGGGGCTAAAAGACCCTTGGA |
| SARS2-Probe I.227 | NC_045512.2 | SARS2-Probe I | CTGATGAGGGAGAAACATTAATACGACTCACTATAGGGTTGAACTCTACTAATGTCACTATTGCAACCTACTGTACTGGTTCTATACCTTGTAGTGTTTGTCTTAGTGGTTTAGAGTTCTTTAGACACCTGGAATTCTCGGGTGCCAAGGGGCTAAAAGACCCTTGGA |
| SARS2-Probe I.228 | NC_045512.2 | SARS2-Probe I | CTGATGAGGGAGAAACATTAATACGACTCACTATAGGGTACTGTACTGGTTCTATACCTTGTAGTGTTTGTCTTAGTGGTTTAGATTCTTTAGACACCTATCCTTCTTTAGAAACTATACACAAATTACCTGGAATTCTCGGGTGCCAAGGGGCTAAAAGACCCTTGGA |
| SARS2-Probe I.229 | NC_045512.2 | SARS2-Probe I | CTGATGAGGGAGAAACATTAATACGACTCACTATAGGGTGTCTTAGTGGTTTAGATTCTTTAGACACCTATCCTTCTTTAGAAACTATACACAAATTACCATTTCATCTTTTAAATGGGATTTAACTGCTTGGAATTCTCGGGTGCCAAGGGGCTAAAAGACCCTTGGA |
| SARS2-Probe I.230 | NC_045512.2 | SARS2-Probe I | CTGATGAGGGAGAAACATTAATACGACTCACTATAGGGTATCCTTCTTTAGAAACTATACAAATTACCATTTCATCTTTTAAATGGGATTTAACTGCTTTTGGCTTAGTTGCAGAGTGGTTTTTGGCATGGAATTCTCGGGTGCCAAGGGGCTAAAAGACCCTTGGA |
| SARS2-Probe I.231 | NC_045512.2 | SARS2-Probe I | CTGATGAGGGAGAAACATTAATACGACTCACTATAGGGATTTCATCTTTTAAATGGGATTTAACTGCTTTTGGCTTAGTTGCAGAGTGGTTTTTGGCATATATTCTTTTCACTAGGTTTTCTATGTATGGAATTCTCGGGTGCCAAGGGGCTAAAAGACCCTTGGA |
| SARS2-Probe I.232 | NC_045512.2 | SARS2-Probe I | CTGATGAGGGAGAAACATTAATACGACTCACTATAGGGTTTGGCTTAGTTGCAGAGTGGTTTTTGGCATATATTCTTTTCACTAGGTTTTCTATGTACTTGGATTGGCTGCAATCATGCAATTGTTTTGGAATTCTCGGGTGCCAAGGGGCTAAAAGACCCTTGGA |
| SARS2-Probe I.233 | NC_045512.2 | SARS2-Probe I | CTGATGAGGGAGAAACATTAATACGACTCACTATAGGGTATATTCTTTTCACTAGGTTTTCTATGTACTTGGATTGGCTGCAATCATGCAATTGTTTTTCAGCTATTTTGCAGTACATTTTATTAGTTGGAATTCTCGGGTGCCAAGGGGCTAAAAGACCCTTGGA |
| SARS2-Probe I.234 | NC_045512.2 | SARS2-Probe I | CTGATGAGGGAGAAACATTAATACGACTCACTATAGGGCTTGGATTGGCTGCAATCATGCAATTGTTTTTCAGCTATTTTGCAGTACATTTTATTAGTAATTCTTGGCTTATGTGGTTAATAATTAATTGGAATTCTCGGGTGCCAAGGGGCTAAAAGACCCTTGGA |
| SARS2-Probe I.235 | NC_045512.2 | SARS2-Probe I | CTGATGAGGGAGAAACATTAATACGACTCACTATAGGGTTCAGCTATTTTGCAGTACATTTTATTAGTAATTCTTGGCTTATGTGGTTAATAATTAATCTTGTACAAATGGCCCCGATTTCAGCTATGTGGAATTCTCGGGTGCCAAGGGGCTAAAAGACCCTTGGA |
| SARS2-Probe I.236 | NC_045512.2 | SARS2-Probe I | CTGATGAGGGAGAAACATTAATACGACTCACTATAGGGAATTCTTGGCTTATGTGGTTAATAATTAATCTTGTACAAATGGCCCCGATTTCAGCTATGGTTAGAATGTACATCTTCTTTGCATCATTTTGGAATTCTCGGGTGCCAAGGGGCTAAAAGACCCTTGGA |
| SARS2-Probe I.237 | NC_045512.2 | SARS2-Probe I | CTGATGAGGGAGAAACATTAATACGACTCACTATAGGGCTTGTACAAATGGCCCCGATTTCAGCTATGGTTAGAATGTACATCTTCTTTGCATCATTTTATTATGTATGGAAAGTTATGTGCATGTTTGGAATTCTCGGGTGCCAAGGGGCTAAAAGACCCTTGGA |
| SARS2-Probe I.238 | NC_045512.2 | SARS2-Probe I | CTGATGAGGGAGAAACATTAATACGACTCACTATAGGGGTTAGAATGTACATCTTCTTTGCATCATTTTATTATGTATGGAAAGTTATGTGCATGTTGTAGACGGTTGTAATTCATCAACTTGTATGTGGAATTCTCGGGTGCCAAGGGGCTAAAAGACCCTTGGA |
| SARS2-Probe I.239 | NC_045512.2 | SARS2-Probe I | CTGATGAGGGAGAAACATTAATACGACTCACTATAGGGTATTATGTATGGAAAGTTATGTGCATGTTGTAGACGGTTGTAATTCATCAACTTGTATGATGTGTTACAAACGTAATAGAGCAACAAGATGGAATTCTCGGGTGCCAAGGGGCTAAAAGACCCTTGGA |
| SARS2-Probe I.240 | NC_045512.2 | SARS2-Probe I | CTGATGAGGGAGAAACATTAATACGACTCACTATAGGGGTAGACGGTTGTAATTCATCAACTTGTATGATGTGTTACAAACGTAATAGAGCAACAAGAGTCGAATGTACAACTATTGTTAATGGTGTTTGGAATTCTCGGGTGCCAAGGGGCTAAAAGACCCTTGGA |
| SARS2-Probe I.241 | NC_045512.2 | SARS2-Probe I | CTGATGAGGGAGAAACATTAATACGACTCACTATAGGGGATGTGTTACAAACGTAATAGAGCAACAAGAGTCGAATGTACAACTATTGTTAATGGTGTTAGAAGGTCCTTTTATGTCTATGCTAATGGATGGAATTCTCGGGTGCCAAGGGGCTAAAAGACCCTTGGA |
| SARS2-Probe I.242 | NC_045512.2 | SARS2-Probe I | CTGATGAGGGAGAAACATTAATACGACTCACTATAGGGGTCGAATGTACAACTATTGTTAATGGTGTTAGAAGGTCCTTTTATGTCTATGCTAATGGAGGTAAAGGCTTTTGCAAACTACACAATTGGTGGAATTCTCGGGTGCCAAGGGGCTAAAAGACCCTTGGA |
| SARS2-Probe I.243 | NC_045512.2 | SARS2-Probe I | CTGATGAGGGAGAAACATTAATACGACTCACTATAGGGGAGAAGGTCCTTTTATGTCTATGCTAATGGAGGTAAAGGCTTTTGCAAACTACACAATTGGAATTGTGTTAATTGTGATACATTCTGTGCTTGGAATTCTCGGGTGCCAAGGGGCTAAAAGACCCTTGGA |
| SARS2-Probe I.244 | NC_045512.2 | SARS2-Probe I | CTGATGAGGGAGAAACATTAATACGACTCACTATAGGGGGTAAAGGCTTTTGCAAACTACACAATTGGAATTGTGTTAATTGTGATACATTCTGTGCTGGTAGTACATTTATTAGTGATGAAGTTGCGTGAATTCTCGGGTGCCAAGGGGCTAAAAGACCCTTGGA |
| SARS2-Probe I.245 | NC_045512.2 | SARS2-Probe I | CTGATGAGGGAGAAACATTAATACGACTCACTATAGGGAATTGTGTTAATTGTGATACATTCTGTGCTGGTAGTACATTTATTAGTGATGAAGTTGCGAGAGACTTGTCACTACAGTTTAAAAGACCATGGAATTCTCGGGTGCCAAGGGGCTAAAAGACCCTTGGA |
| SARS2-Probe I.246 | NC_045512.2 | SARS2-Probe I | CTGATGAGGGAGAAACATTAATACGACTCACTATAGGGGGTAGTACATTTATTAGTGATGAAGTTGCGAGAGACTTGTCACTACAGTTTAAAAGACCAATAAATCCTACTGACCAGTCTTCTTACATCTGGAATTCTCGGGTGCCAAGGGGCTAAAAGACCCTTGGA |
| SARS2-Probe I.247 | NC_045512.2 | SARS2-Probe I | CTGATGAGGGAGAAACATTAATACGACTCACTATAGGGGAGAGACTTGTCACTACAGTTTAAAAGACCAATAAATCCTACTGACCAGTCTTCTTACATCGTTGATAGTGTTACAGTGAAGAATGGTTCCTGGAATTCTCGGGTGCCAAGGGGCTAAAAGACCCTTGGA |
| SARS2-Probe I.248 | NC_045512.2 | SARS2-Probe I | CTGATGAGGGAGAAACATTAATACGACTCACTATAGGGGATAAATCCTACTGACCAGTCTTCTTACATCGTTGATAGTGTTACAGTGAAGAATGGTTCCATCCATCTTTACTTTGATAAAGCTGGTCAATGGAATTCTCGGGTGCCAAGGGGCTAAAAGACCCTTGGA |
| SARS2-Probe I.249 | NC_045512.2 | SARS2-Probe I | CTGATGAGGGAGAAACATTAATACGACTCACTATAGGGGTTGATAGTGTTACAGTGAAGAATGGTTCCATCCATCTTTACTTTGATAAAGCTGGTCAAAAGACTTATGAAAGACATTCTCTCTCTCATTGGAATTCTCGGGTGCCAAGGGGCTAAAAGACCCTTGGA |

31

| ASO ID | Accession | Group ID | Custom Array sequence |
|---|---|---|---|
| SARS2-Probe I.250 | NC_045512.2 | SARS2-Probe I | CTGATGAGGGAGAAACATTAATACGACTCACTATAGGGATCCATCTTTACTTTGATAAAGCTGGTCAAAAGACTTATGAAAGACATTCTCTCTCTCATTTTGTTAACTTAGACAACCTGAGAGCTAATTGGAATTCTCGGGTGCCAAGGGGCTAAAAGACCCTTGGA |
| SARS2-Probe I.251 | NC_045512.2 | SARS2-Probe I | CTGATGAGGGAGAAACATTAATACGACTCACTATAGGGAAGACTTATGAAAGACATTCTCTCTCTCATTTTGTTAACTTAGACAACCTGAGAGCTAATAACACTAAAGGTTCATTGCCTATTAATGTTTGGAATTCTCGGGTGCCAAGGGGCTAAAAGACCCTTGGA |
| SARS2-Probe I.252 | NC_045512.2 | SARS2-Probe I | CTGATGAGGGAGAAACATTAATACGACTCACTATAGGGTTTGTTAACTTAGACAACCTGAGAGCTAATAACACTAAAGGTTCATTGCCTATTAATGTTATAGTTTTTGATGGTAAATCAAAATGTGAATGGAATTCTCGGGTGCCAAGGGGCTAAAAGACCCTTGGA |
| SARS2-Probe I.253 | NC_045512.2 | SARS2-Probe I | CTGATGAGGGAGAAACATTAATACGACTCACTATAGGGAACACTAAAGGTTCATTGCCTATTAATGTTATAGTTTTTGATGGTAAATCAAAATGTGAAGAATCATCTGCAAAATCAGCGTCTGTTTACTGGAATTCTCGGGTGCCAAGGGGCTAAAAGACCCTTGGA |
| SARS2-Probe I.254 | NC_045512.2 | SARS2-Probe I | CTGATGAGGGAGAAACATTAATACGACTCACTATAGGGATAGTTTTTGATGGTAAATCAAAATGTGAAGAATCATCTGCAAAATCAGCGTCTGTTTACTACAGTCAGCTTATGTGTCAACCTATACTGTGGAATTCTCGGGTGCCAAGGGGCTAAAAGACCCTTGGA |
| SARS2-Probe I.255 | NC_045512.2 | SARS2-Probe I | CTGATGAGGGAGAAACATTAATACGACTCACTATAGGGGAATCATCTGCAAAATCAGCGTCTGTTTACTACAGTCAGCTTATGTGTCAACCTATACTGTTACTAGATCAGGCATTAGTGTCTGATGTTTGGAATTCTCGGGTGCCAAGGGGCTAAAAGACCCTTGGA |
| SARS2-Probe I.256 | NC_045512.2 | SARS2-Probe I | CTGATGAGGGAGAAACATTAATACGACTCACTATAGGGTACAGTCAGCTTATGTGTCAACCTATACTGTTACTAGATCAGGCATTAGTGTCTGATGTTGGTGATAGTGCGGAAGTTGCAGTTAAAATGTGGAATTCTCGGGTGCCAAGGGGCTAAAAGACCCTTGGA |
| SARS2-Probe I.257 | NC_045512.2 | SARS2-Probe I | CTGATGAGGGAGAAACATTAATACGACTCACTATAGGGTTACTAGATCAGGCATTAGTGTCTGATGTTGGTGATAGTGCGGAAGTTGCAGTTAAAATGTTTGATGCTTACGTTAATACGTTTTCATCATGGAATTCTCGGGTGCCAAGGGGCTAAAAGACCCTTGGA |
| SARS2-Probe I.258 | NC_045512.2 | SARS2-Probe I | CTGATGAGGGAGAAACATTAATACGACTCACTATAGGGGGGTGATAGTGCGGAAGTTGCAGTTAAAATGTTTGATGCTTACGTTAATACGTTTTCATCAACTTTTAACGTACCAATGGAAAAACTCAAATGGAATTCTCGGGTGCCAAGGGGCTAAAAGACCCTTGGA |
| SARS2-Probe I.259 | NC_045512.2 | SARS2-Probe I | CTGATGAGGGAGAAACATTAATACGACTCACTATAGGGTTTGATGCTTACGTTAATACGTTTTCATCAACTTTTAACGTACCAATGGAAAAACTCAAAACACTAGTTGCAACTGCAGAAGCTGAACTTTGGAATTCTCGGGTGCCAAGGGGCTAAAAGACCCTTGGA |
| SARS2-Probe I.260 | NC_045512.2 | SARS2-Probe I | CTGATGAGGGAGAAACATTAATACGACTCACTATAGGGACTTTTAACGTACCAATGGAAAAACTCAAAACACTAGTTGCAACTGCAGAAGCTGAACTTGCAAAGAATGTGTCCTTAGACAATGTCTTATGGAATTCTCGGGTGCCAAGGGGCTAAAAGACCCTTGGA |
| SARS2-Probe I.261 | NC_045512.2 | SARS2-Probe I | CTGATGAGGGAGAAACATTAATACGACTCACTATAGGGACACTAGTTGCAACTGCAGAAGCTGAACTTGCAAAGAATGTGTCCTTAGACAATGTCTTATCTACTTTTATTTCAGCAGCTCGGCAAGGGTGGAATTCTCGGGTGCCAAGGGGCTAAAAGACCCTTGGA |
| SARS2-Probe I.262 | NC_045512.2 | SARS2-Probe I | CTGATGAGGGAGAAACATTAATACGACTCACTATAGGGGCAAAGAATGTGTCCTTAGACAATGTCTTATCTACTTTTATTTCAGCAGCTCGGCAAGGGTTTGTTGATTCAGATGTAGAAACTAAAGATTGGAATTCTCGGGTGCCAAGGGGCTAAAAGACCCTTGGA |
| SARS2-Probe I.263 | NC_045512.2 | SARS2-Probe I | CTGATGAGGGAGAAACATTAATACGACTCACTATAGGGTCTACTTTTATTTCAGCAGCTCGGCAAGGGTTTGTTGATTCAGATGTAGAAACTAAAGATGTTGTTGAATGTCTTAAATTGTCACATCAATGGAATTCTCGGGTGCCAAGGGGCTAAAAGACCCTTGGA |
| SARS2-Probe I.264 | NC_045512.2 | SARS2-Probe I | CTGATGAGGGAGAAACATTAATACGACTCACTATAGGGTTTGTTGATTCAGATGTAGAAACTAAAGATGTTGTTGAATGTCTTAAATTGTCACATCAATCTGACATAGAAGTTACTGGCGATAGTTGTTGGAATTCTCGGGTGCCAAGGGGCTAAAAGACCCTTGGA |
| SARS2-Probe I.265 | NC_045512.2 | SARS2-Probe I | CTGATGAGGGAGAAACATTAATACGACTCACTATAGGGTTGTTGAATGTCTTAAATTGTCACATCAATCTGACATAGAAGTTACTGGCGATAGTTGTAATAACTATATGCTCACCTATAACAAAGTTTGGAATTCTCGGGTGCCAAGGGGCTAAAAGACCCTTGGA |
| SARS2-Probe I.266 | NC_045512.2 | SARS2-Probe I | CTGATGAGGGAGAAACATTAATACGACTCACTATAGGGTCTGACATAGAAGTTACTGGCGATAGTTGTAATAACTATATGCTCACCTATAACAAAGTTGAAAACATGACACCCCGTGACCTTGGTGCTTGGAATTCTCGGGTGCCAAGGGGCTAAAAGACCCTTGGA |
| SARS2-Probe I.267 | NC_045512.2 | SARS2-Probe I | CTGATGAGGGAGAAACATTAATACGACTCACTATAGGGAATAACTATATGCTCACCTATAACAAAGTTGAAAACATGACACCCCGTGACCTTGGTGCTTGTATTGACTGTAGTGCGCGTCATATTAATTGGAATTCTCGGGTGCCAAGGGGCTAAAAGACCCTTGGA |
| SARS2-Probe I.268 | NC_045512.2 | SARS2-Probe I | CTGATGAGGGAGAAACATTAATACGACTCACTATAGGGGAAAACATGACACCCCGTGACCTTGGTGCTTGTATTGACTGTAGTGCGCGTCATATTAATGCGCAGGTAGCAAAAAGTCACAACATTGCTTGGAATTCTCGGGTGCCAAGGGGCTAAAAGACCCTTGGA |
| SARS2-Probe I.269 | NC_045512.2 | SARS2-Probe I | CTGATGAGGGAGAAACATTAATACGACTCACTATAGGGTGTATTGACTGTAGTGCGCGTCATATTAATGCGCAGGTAGCAAAAAGTCACAACATTGCTTTGATATGGAACGTTAAAGATTTCATGTCATGGAATTCTCGGGTGCCAAGGGGCTAAAAGACCCTTGGA |
| SARS2-Probe I.270 | NC_045512.2 | SARS2-Probe I | CTGATGAGGGAGAAACATTAATACGACTCACTATAGGGGCGCAGGTAGCAAAAAGTCACAACATTGCTTTGATATGGAACGTTAAAGATTTCATGTCATTGTCTGAACAACTACGAAAACAAATACGTTGGAATTCTCGGGTGCCAAGGGGCTAAAAGACCCTTGGA |
| SARS2-Probe I.271 | NC_045512.2 | SARS2-Probe I | CTGATGAGGGAGAAACATTAATACGACTCACTATAGGGTTGATATGGAACGTTAAAGATTTCATGTCATTGTCTGAACAACTACGAAAACAAATACGTAGTGCTGCTAAAAAGAATAACTTACCTTTTTGGAATTCTCGGGTGCCAAGGGGCTAAAAGACCCTTGGA |
| SARS2-Probe I.272 | NC_045512.2 | SARS2-Probe I | CTGATGAGGGAGAAACATTAATACGACTCACTATAGGGTTGTCTGAACAACTACGAAAACAAATACGTAGTGCTGCTAAAAAGAATAACTTACCTTTTTAAGTTGACATGTGCAACTACTAGACAAGTTTGGAATTCTCGGGTGCCAAGGGGCTAAAAGACCCTTGGA |
| SARS2-Probe I.273 | NC_045512.2 | SARS2-Probe I | CTGATGAGGGAGAAACATTAATACGACTCACTATAGGGAGTGCTGCTAAAAAGAATAACTTACCTTTTTAAGTTGACATGTGCAACTACTAGACAAGTTGTTAATGTTGTAACAACAAAGATAGCACTTTGGAATTCTCGGGTGCCAAGGGGCTAAAAGACCCTTGGA |
| SARS2-Probe I.274 | NC_045512.2 | SARS2-Probe I | CTGATGAGGGAGAAACATTAATACGACTCACTATAGGGAAGTTGACATGTGCAACTACTAGACAAGTTGTTAATGTTGTAACAACAAAGATAGCACTTAAGGGTGGTAAAATTGTTAATAATTGGTTGTGGAATTCTCGGGTGCCAAGGGGCTAAAAGACCCTTGGA |

32

| ASO ID | Accession | Group ID | Custom Array sequence |
|---|---|---|---|
| SARS2-Probe I.275 | NC_045512.2 | SARS2-Probe I | CTGATGAGGGAGAAACATTAATACGACTCACTATAGGGGTTAATGTTGTAACAACAAAGATAGCACTTAAGGGTGGTAAAATTGTTAATAATTGGTTGAAGCAGTTAATTAAAGTTACACTTGTGTTCTGGAATTCTCGGGTGCCAAGGGGCTAAAAGACCCTTGGA |
| SARS2-Probe I.276 | NC_045512.2 | SARS2-Probe I | CTGATGAGGGAGAAACATTAATACGACTCACTATAGGGAAGGGTGGTAAAATTGTTAATAATTGGTTGAAGCAGTTAATTAAAGTTACACTTGTGTTCCTTTTTGTTGCTGCTATTTTCTATTTAATATGGAATTCTCGGGTGCCAAGGGGCTAAAAGACCCTTGGA |
| SARS2-Probe I.277 | NC_045512.2 | SARS2-Probe I | CTGATGAGGGAGAAACATTAATACGACTCACTATAGGGAAGCAGTTAATTAAAGTTACACTTGTGTTCCTTTTTGTTGCTGCTATTTTCTATTTAATAACACCTGTTCATGTCATGTCTAAACATACTTGGAATTCTCGGGTGCCAAGGGGCTAAAAGACCCTTGGA |
| SARS2-Probe I.278 | NC_045512.2 | SARS2-Probe I | CTGATGAGGGAGAAACATTAATACGACTCACTATAGGGCTTTTTGTTGCTGCTATTTTCTATTTAATAACACCTGTTCATGTCATGTCTAAACATACTGACTTTTCAAGTGAAATCATAGGATACAAGTGGAATTCTCGGGTGCCAAGGGGCTAAAAGACCCTTGGA |
| SARS2-Probe I.279 | NC_045512.2 | SARS2-Probe I | CTGATGAGGGAGAAACATTAATACGACTCACTATAGGGACACCTGTTCATGTCATGTCTAAACATACTGACTTTTCAAGTGAAATCATAGGATACAAGGCTATTGATGGTGGTGTCACTCGTGACATATGGAATTCTCGGGTGCCAAGGGGCTAAAAGACCCTTGGA |
| SARS2-Probe I.280 | NC_045512.2 | SARS2-Probe I | CTGATGAGGGAGAAACATTAATACGACTCACTATAGGGGACTTTTCAAGTGAAATCATAGGATACAAGGCTATTGATGGTGGTGTCACTCGTGACATAGCATCTACAGATACTTGTTTTGCTAACAAATGGAATTCTCGGGTGCCAAGGGGCTAAAAGACCCTTGGA |
| SARS2-Probe I.281 | NC_045512.2 | SARS2-Probe I | CTGATGAGGGAGAAACATTAATACGACTCACTATAGGGGCTATTGATGGTGGTGTCACTCGTGACATAGCATCTACAGATACTTGTTTTGCTAACAAACATGCTGATTTTGACACATGGTTTAGCCAGTGGAATTCTCGGGTGCCAAGGGGCTAAAAGACCCTTGGA |
| SARS2-Probe I.282 | NC_045512.2 | SARS2-Probe I | CTGATGAGGGAGAAACATTAATACGACTCACTATAGGGGCATCTACAGATACTTGTTTTGCTAACAAACATGCTGATTTTGACACATGGTTTAGCCAGCGTGGTGGTAGTTATACTAATGACAAAGCTTGGAATTCTCGGGTGCCAAGGGGCTAAAAGACCCTTGGA |
| SARS2-Probe I.283 | NC_045512.2 | SARS2-Probe I | CTGATGAGGGAGAAACATTAATACGACTCACTATAGGGCATGCTGATTTTGACACATGGTTTAGCCAGCGTGGTGGTAGTTATACTAATGACAAAGCTTGCCCATTGATTGCTGCAGTCATAACAAGATGGAATTCTCGGGTGCCAAGGGGCTAAAAGACCCTTGGA |
| SARS2-Probe I.284 | NC_045512.2 | SARS2-Probe I | CTGATGAGGGAGAAACATTAATACGACTCACTATAGGGCGTGGTGGTAGTTATACTAATGACAAAGCTTGCCCATTGATTGCTGCAGTCATAACAAGAGAAGTGGGTTTTGTCGTGCCTGGTTTGCCTTGGAATTCTCGGGTGCCAAGGGGCTAAAAGACCCTTGGA |
| SARS2-Probe I.285 | NC_045512.2 | SARS2-Probe I | CTGATGAGGGAGAAACATTAATACGACTCACTATAGGGTGCCCATTGATTGCTGCAGTCATAACAAGAGAAGTGGGTTTTGTCGTGCCTGGTTTGCCTGGCACGATATTACGCACAACTAATGGTGACTGGAATTCTCGGGTGCCAAGGGGCTAAAAGACCCTTGGA |
| SARS2-Probe I.286 | NC_045512.2 | SARS2-Probe I | CTGATGAGGGAGAAACATTAATACGACTCACTATAGGGGAAGTGGGTTTTGTCGTGCCTGGTTTGCCTGGCACGATATTACGCACAACTAATGGTGACTTTTGCATTTCTTACCTAGAGTTTTTAGTTGGAATTCTCGGGTGCCAAGGGGCTAAAAGACCCTTGGA |
| SARS2-Probe I.287 | NC_045512.2 | SARS2-Probe I | CTGATGAGGGAGAAACATTAATACGACTCACTATAGGGGGGCACGATATTACGCACAACTAATGGTGACTTTTGCATTTCTTACCTAGAGTTTTTAGTTGCAGTTGGTAACATCTGTTACACACCATCATGGAATTCTCGGGTGCCAAGGGGCTAAAAGACCCTTGGA |
| SARS2-Probe I.288 | NC_045512.2 | SARS2-Probe I | CTGATGAGGGAGAAACATTAATACGACTCACTATAGGGGTTTTTGCATTTCTTACCTAGAGTTTTTAGTTGCAGTTGGTAACATCTGTTACACACCATCAAAACTTATAGAGTACACTGACTTTGCAACATGGAATTCTCGGGTGCCAAGGGGCTAAAAGACCCTTGGA |
| SARS2-Probe I.289 | NC_045512.2 | SARS2-Probe I | CTGATGAGGGAGAAACATTAATACGACTCACTATAGGGGCAGTTGGTAACATCTGTTACACACCATCAAAACTTATAGAGTACACTGACTTTGCAACATCAGCTTGTGTGTTTGGCTGCTGAATGTACATGGAATTCTCGGGTGCCAAGGGGCTAAAAGACCCTTGGA |
| SARS2-Probe I.290 | NC_045512.2 | SARS2-Probe I | CTGATGAGGGAGAAACATTAATACGACTCACTATAGGGAAACTTATAGAGTACACTGACTTTGCAACATCAGCTTGTGTGTTTGGCTGCTGAATGTACAATTTTTAAAGATGCTTCTGGTAAGCCAGTATGGAATTCTCGGGTGCCAAGGGGCTAAAAGACCCTTGGA |
| SARS2-Probe I.291 | NC_045512.2 | SARS2-Probe I | CTGATGAGGGAGAAACATTAATACGACTCACTATAGGGTCAGCTTGTGTGTTTGGCTGCTGAATGTACAATTTTTAAAGATGCTTCTGGTAAGCCAGTACCATATTGTTATGATACCAATGTACTAGAATGGAATTCTCGGGTGCCAAGGGGCTAAAAGACCCTTGGA |
| SARS2-Probe I.292 | NC_045512.2 | SARS2-Probe I | CTGATGAGGGAGAAACATTAATACGACTCACTATAGGGATTTTTAAAGATGCTTCTGGTAAGCCAGTACCATCATATTGTTATGATACCAATGTACTAGAAGGTTCTGTTGCTTATGAAAGTTTACGCCCTTGGAATTCTCGGGTGCCAAGGGGCTAAAAGACCCTTGGA |
| SARS2-Probe I.293 | NC_045512.2 | SARS2-Probe I | CTGATGAGGGAGAAACATTAATACGACTCACTATAGGGCCATATTGTTATGATACCAATGTACTAGAAGGTTCTGTTGCTTATGAAAGTTTACGCCCTGACACACGTTATGTGCTCATGGATGGCTCTTGGAATTCTCGGGTGCCAAGGGGCTAAAAGACCCTTGGA |
| SARS2-Probe I.294 | NC_045512.2 | SARS2-Probe I | CTGATGAGGGAGAAACATTAATACGACTCACTATAGGGGTTCTGTTGCTTATGAAAGTTTACGCCCTGACACACGTTATGTGCTCATGGATGGCTCTATTATTCAATTTCCTAACACCTACCTTGAATGGAATTCTCGGGTGCCAAGGGGCTAAAAGACCCTTGGA |
| SARS2-Probe I.295 | NC_045512.2 | SARS2-Probe I | CTGATGAGGGAGAAACATTAATACGACTCACTATAGGGGACACACGTTATGTGCTCATGGATGGCTCTATTATTCAATTTCCTAACACCTACCTTGAAGGTTCTGTTAGAGTGGTAACAACTTTTGATTGGAATTCTCGGGTGCCAAGGGGCTAAAAGACCCTTGGA |
| SARS2-Probe I.296 | NC_045512.2 | SARS2-Probe I | CTGATGAGGGAGAAACATTAATACGACTCACTATAGGGATTATTCAATTTCCTAACACCTACCTTGAAGGTTCTGTTAGAGTGGTAACAACTTTTGATTCTGAGTACTGTAGGCACGGCACTTGTGAATGGAATTCTCGGGTGCCAAGGGGCTAAAAGACCCTTGGA |
| SARS2-Probe I.297 | NC_045512.2 | SARS2-Probe I | CTGATGAGGGAGAAACATTAATACGACTCACTATAGGGGGGTTCTGTTAGAGTGGTAACAACTTTTGATTCTGAGTACTGTAGGCACGGCACTTGTGAAAGATCAGAAGCTGGTGTTTGTGTATCTACTTGGAATTCTCGGGTGCCAAGGGGCTAAAAGACCCTTGGA |
| SARS2-Probe I.298 | NC_045512.2 | SARS2-Probe I | CTGATGAGGGAGAAACATTAATACGACTCACTATAGGGTCTGAGTACTGTAGGCACGGCACTTGTGAAAGATCAGAAGCTGGTGTTTGTGTATCTACTAGTGGTAGATGGGTACTTAACAATGATTATTGGAATTCTCGGGTGCCAAGGGGCTAAAAGACCCTTGGA |
| SARS2-Probe I.299 | NC_045512.2 | SARS2-Probe I | CTGATGAGGGAGAAACATTAATACGACTCACTATAGGGAGATCAGAAGCTGGTGTTTGTGTATCTACTAGTGGTAGATGGGTACTTAACAATGATTATTACAGATCTTTACCAGGAGTTTTCTGTGGTTGGAATTCTCGGGTGCCAAGGGGCTAAAAGACCCTTGGA |

33

| ASO ID | Accession | Group ID | Custom Array sequence |
|---|---|---|---|
| SARS2-Probe I.300 | NC_045512.2 | SARS2-Probe I | CTGATGAGGGAGAAACATTAATACGACTCACTATAGGGAGTGGTAGATGGGTACTTAACAATGATTATTACAGATCTTTACCAGGAGTTTTCTGTGGTGTAGATGCTGTAAATTTACTTACTAATATGTGGAATTCTCGGGTGCCAAGGGGCTAAAAGACCCTTGGA |
| SARS2-Probe I.301 | NC_045512.2 | SARS2-Probe I | CTGATGAGGGAGAAACATTAATACGACTCACTATAGGGTACAGATCTTTACCAGGAGTTTTCTGTGGTGTAGATGCTGTAAATTTACTTACTAATATGTTTACACCACTAATTCAACCTATTGGTGCTTGGAATTCTCGGGTGCCAAGGGGCTAAAAGACCCTTGGA |
| SARS2-Probe I.302 | NC_045512.2 | SARS2-Probe I | CTGATGAGGGAGAAACATTAATACGACTCACTATAGGGGTAGATGCTGTAAATTTACTTACTAATATGTTTACACCACTAATTCAACCTATTGGTGCTTTGGACATATCAGCATCTATAGTAGCTGGTTGGAATTCTCGGGTGCCAAGGGGCTAAAAGACCCTTGGA |
| SARS2-Probe I.303 | NC_045512.2 | SARS2-Probe I | CTGATGAGGGAGAAACATTAATACGACTCACTATAGGGTTTACACCACTAATTCAACCTATTGGTGCTTTGGACATATCAGCATCTATAGTAGCTGGTGGTATTGTAGCTATCGTAGTAACATGCCTTTGGAATTCTCGGGTGCCAAGGGGCTAAAAGACCCTTGGA |
| SARS2-Probe I.304 | NC_045512.2 | SARS2-Probe I | CTGATGAGGGAGAAACATTAATACGACTCACTATAGGGTTGGACATATCAGCATCTATAGTAGCTGGTGGTATTGTAGCTATCGTAGTAACATGCCTTGCCTACTATTTTATGAGGTTTAGAAGAGCTTGGAATTCTCGGGTGCCAAGGGGCTAAAAGACCCTTGGA |
| SARS2-Probe I.305 | NC_045512.2 | SARS2-Probe I | CTGATGAGGGAGAAACATTAATACGACTCACTATAGGGGGTATTGTAGCTATCGTAGTAACATGCCTTGCCTACTATTTTATGAGGTTTAGAAGAGCTTTTGGTGAATACAGTCATGTAGTTGCCTTTTGGAATTCTCGGGTGCCAAGGGGCTAAAAGACCCTTGGA |
| SARS2-Probe I.306 | NC_045512.2 | SARS2-Probe I | CTGATGAGGGAGAAACATTAATACGACTCACTATAGGGGCCTACTATTTTATGAGGTTTAGAAGAGCTTTTGGTGAATACAGTCATGTAGTTGCCTTTAATACTTTACTATTCCTTATGTCATTCACTTGGAATTCTCGGGTGCCAAGGGGCTAAAAGACCCTTGGA |
| SARS2-Probe I.307 | NC_045512.2 | SARS2-Probe I | CTGATGAGGGAGAAACATTAATACGACTCACTATAGGGTTTGGTGAATACAGTCATGTAGTTGCCTTTAATACTTTACTATTCCTTATGTCATTCACTGTACTCTGTTTAACACCAGTTTACTCATTCTGGAATTCTCGGGTGCCAAGGGGCTAAAAGACCCTTGGA |
| SARS2-Probe I.308 | NC_045512.2 | SARS2-Probe I | CTGATGAGGGAGAAACATTAATACGACTCACTATAGGGAATACTTTACTATTCCTTATGTCATTCACTGTACTCTGTTTAACACCAGTTTACTCATTCTTACCTGGTGTTTATTCTGTTATTTACTTGTGTGGAATTCTCGGGTGCCAAGGGGCTAAAAGACCCTTGGA |
| SARS2-Probe I.309 | NC_045512.2 | SARS2-Probe I | CTGATGAGGGAGAAACATTAATACGACTCACTATAGGGGTACTCTGTTTAACACCAGTTTACTCATTCTTACCTGGTGTTTATTCTGTTATTTACTTGTACTTGACATTTATCTTACTAATGATGTTTGGAATTCTCGGGTGCCAAGGGGCTAAAAGACCCTTGGA |
| SARS2-Probe I.310 | NC_045512.2 | SARS2-Probe I | CTGATGAGGGAGAAACATTAATACGACTCACTATAGGGTTACCTGGTGTTTATTCTGTTATTTACTTGTACTTGACATTTATCTTACTAATGATGTTTCTTTTTTAGCACATATTCAGTGGATGGTTTGGAATTCTCGGGTGCCAAGGGGCTAAAAGACCCTTGGA |
| SARS2-Probe I.311 | NC_045512.2 | SARS2-Probe I | CTGATGAGGGAGAAACATTAATACGACTCACTATAGGGTACTTGACATTTATCTTACTAATGATGTTTCTTTTTTAGCACATATTCAGTGGATGGTTATGTTCACACCTTTAGTACCTTTCTGGATATGGAATTCTCGGGTGCCAAGGGGCTAAAAGACCCTTGGA |
| SARS2-Probe I.312 | NC_045512.2 | SARS2-Probe I | CTGATGAGGGAGAAACATTAATACGACTCACTATAGGGTCTTTTTTAGCACATATTCAGTGGATGGTTATGTTCACACCTTTAGTACCTTTCTGGATAACAATTGCTTATATCATTTGTATTTCCACATGGAATTCTCGGGTGCCAAGGGGCTAAAAGACCCTTGGA |
| SARS2-Probe I.313 | NC_045512.2 | SARS2-Probe I | CTGATGAGGGAGAAACATTAATACGACTCACTATAGGGATGTTCACACCTTTAGTACCTTTCTGGATAACAATTGCTTATATCATTTGTATTTCCACAAAGCATTTCTATTGGTTCTTTAGTAATTACTGGAATTCTCGGGTGCCAAGGGGCTAAAAGACCCTTGGA |
| SARS2-Probe I.314 | NC_045512.2 | SARS2-Probe I | CTGATGAGGGAGAAACATTAATACGACTCACTATAGGGACAATTGCTTATATCATTTGTATTTCCACAAAGCATTTCTATTGGTTCTTTAGTAATTACCTAAAGAGACGTGTAGTCTTTAATGGTGTTTGGAATTCTCGGGTGCCAAGGGGCTAAAAGACCCTTGGA |
| SARS2-Probe I.315 | NC_045512.2 | SARS2-Probe I | CTGATGAGGGAGAAACATTAATACGACTCACTATAGGGAAGCATTTCTATTGGTTCTTTAGTAATTACCTAAAGAGACGTGTAGTCTTTAATGGTGTTTCCTTTAGTACTTTTGAAGAAGCTGCGCTGTGGAATTCTCGGGTGCCAAGGGGCTAAAAGACCCTTGGA |
| SARS2-Probe I.316 | NC_045512.2 | SARS2-Probe I | CTGATGAGGGAGAAACATTAATACGACTCACTATAGGGCTAAAGAGACGTGTAGTCTTTAATGGTGTTTCCTTTAGTACTTTTGAAGAAGCTGCGCTGTGCACCTTTTTGTTAAATAAAGAAATGTATTGGAATTCTCGGGTGCCAAGGGGCTAAAAGACCCTTGGA |
| SARS2-Probe I.317 | NC_045512.2 | SARS2-Probe I | CTGATGAGGGAGAAACATTAATACGACTCACTATAGGGTCCTTTAGTACTTTTGAAGAAGCTGCGCTGTGCACCTTTTTGTTAAATAAAGAAATGTATCTAAAGTTGCGTAGTGATGTGCTATTACCTTGGAATTCTCGGGTGCCAAGGGGCTAAAAGACCCTTGGA |
| SARS2-Probe I.318 | NC_045512.2 | SARS2-Probe I | CTGATGAGGGAGAAACATTAATACGACTCACTATAGGGTGCACCTTTTTGTTAAATAAAGAAATGTATCTAAAGTTGCGTAGTGATGTGCTATTACCTCTTACGCAATATAATAGATACTTAGCTCTTTGGAATTCTCGGGTGCCAAGGGGCTAAAAGACCCTTGGA |
| SARS2-Probe I.319 | NC_045512.2 | SARS2-Probe I | CTGATGAGGGAGAAACATTAATACGACTCACTATAGGGCTAAAGTTGCGTAGTGATGTGCTATTACCTCTTACGCAATATAATAGATACTTAGCTCTTTATAATAAGTACAAGTATTTTAGTGGAGCATGGAATTCTCGGGTGCCAAGGGGCTAAAAGACCCTTGGA |
| SARS2-Probe I.320 | NC_045512.2 | SARS2-Probe I | CTGATGAGGGAGAAACATTAATACGACTCACTATAGGGCTTACGCAATATAATAGATACTTAGCTCTTTATAATAAGTACAAGTATTTTAGTGGAGCAATGGATACAACTAGCTACAGAGAAGCTGCTTGGAATTCTCGGGTGCCAAGGGGCTAAAAGACCCTTGGA |
| SARS2-Probe I.321 | NC_045512.2 | SARS2-Probe I | CTGATGAGGGAGAAACATTAATACGACTCACTATAGGGTATAATAAGTACAAGTATTTTAGTGGAGCAATGGATACAACTAGCTACAGAGAAGCTGCTTGTTGTCATCTCGCAAAGGCTCTCAATGACTGGAATTCTCGGGTGCCAAGGGGCTAAAAGACCCTTGGA |
| SARS2-Probe I.322 | NC_045512.2 | SARS2-Probe I | CTGATGAGGGAGAAACATTAATACGACTCACTATAGGGATGGATACAACTAGCTACAGAGAAGCTGCTTGTTGTCATCTCGCAAAGGCTCTCAATGACTTCAGTAACTCAGGTTCTGATGTTCTTTACTGGAATTCTCGGGTGCCAAGGGGCTAAAAGACCCTTGGA |
| SARS2-Probe I.323 | NC_045512.2 | SARS2-Probe I | CTGATGAGGGAGAAACATTAATACGACTCACTATAGGGTGTTGTCATCTCGCAAAGGCTCTCAATGACTTCAGTAACTCAGGTTCTGATGTTCTTTACCAACCACCACAAACCTCTATCACCTCAGCTTGGAATTCTCGGGTGCCAAGGGGCTAAAAGACCCTTGGA |
| SARS2-Probe I.324 | NC_045512.2 | SARS2-Probe I | CTGATGAGGGAGAAACATTAATACGACTCACTATAGGGTTCAGTAACTCAGGTTCTGATGTTCTTTACCAACCACCACAAACCTCTATCACCTCAGCTGTTTTGCAGAGTGGTTTTAGAAAAATGGCATGGAATTCTCGGGTGCCAAGGGGCTAAAAGACCCTTGGA |

| ASO ID | Accession | Group ID | Custom Array sequence |
|---|---|---|---|
| SARS2-Probe I.325 | NC_045512.2 | SARS2-Probe I | CTGATGAGGGAGAAACATTAATACGACTCACTATAGGGCAACCACCACAAACCTCTATCACCTCAGCTGTTTTGCAGAGTGGTTTTAGAAAAATGGCATTCCCATCTGGTAAAGTTGAGGGTTGTATGTGGAATTCTCGGGTGCCAAGGGGCTAAAAGACCCTTGGA |
| SARS2-Probe I.326 | NC_045512.2 | SARS2-Probe I | CTGATGAGGGAGAAACATTAATACGACTCACTATAGGGGTTTTGCAGAGTGGTTTTAGAAAAATGGCATTCCCATCTGGTAAAGTTGAGGGTTGTATGGTACAAGTAACTTGTGGTACAACTACACTTTGGAATTCTCGGGTGCCAAGGGGCTAAAAGACCCTTGGA |
| SARS2-Probe I.327 | NC_045512.2 | SARS2-Probe I | CTGATGAGGGAGAAACATTAATACGACTCACTATAGGGTTCCCATCTGGTAAAGTTGAGGGTTGTATGGTACAAGTAACTTGTGGTACAACTACACTTAACGGTCTTTGGCTTGATGACGTAGTTTACTGGAATTCTCGGGTGCCAAGGGGCTAAAAGACCCTTGGA |
| SARS2-Probe I.328 | NC_045512.2 | SARS2-Probe I | CTGATGAGGGAGAAACATTAATACGACTCACTATAGGGGTACAAGTAACTTGTGGTACAACTACACTTAACGGTCTTTGGCTTGATGACGTAGTTTACTGTCCAAGACATGTGATCTGCACCTCTGAATGGAATTCTCGGGTGCCAAGGGGCTAAAAGACCCTTGGA |
| SARS2-Probe I.329 | NC_045512.2 | SARS2-Probe I | CTGATGAGGGAGAAACATTAATACGACTCACTATAGGGAACGGTCTTTGGCTTGATGACGTAGTTTACTGTCCAAGACATGTGATCTGCACCTCTGAAGACATGCTTAACCCTAATTATGAAGATTTATGGAATTCTCGGGTGCCAAGGGGCTAAAAGACCCTTGGA |
| SARS2-Probe I.330 | NC_045512.2 | SARS2-Probe I | CTGATGAGGGAGAAACATTAATACGACTCACTATAGGGTGTCCAAGACATGTGATCTGCACCTCTGAAGACATGCTTAACCCTAATTATGAAGATTTACTCATTCGTAAGTCTAATCATAATTTCTTGTGGAATTCTCGGGTGCCAAGGGGCTAAAAGACCCTTGGA |
| SARS2-Probe I.331 | NC_045512.2 | SARS2-Probe I | CTGATGAGGGAGAAACATTAATACGACTCACTATAGGGGACATGCTTAACCCTAATTATGAAGATTTACTCATTCGTAAGTCTAATCATAATTTCTTGGTACAGGCTGGTAATGTTCAACTCAGGGTTTGGAATTCTCGGGTGCCAAGGGGCTAAAAGACCCTTGGA |
| SARS2-Probe I.332 | NC_045512.2 | SARS2-Probe I | CTGATGAGGGAGAAACATTAATACGACTCACTATAGGGCTCATTCGTAAGTCTAATCATAATTTCTTGGTACAGGCTGGTAATGTTCAACTCAGGGTTATTGGACATTCTATGCAAAATTGTGTACTTTGGAATTCTCGGGTGCCAAGGGGCTAAAAGACCCTTGGA |
| SARS2-Probe I.333 | NC_045512.2 | SARS2-Probe I | CTGATGAGGGAGAAACATTAATACGACTCACTATAGGGGTACAGGCTGGTAATGTTCAACTCAGGGTTATTGGACATTCTATGCAAAATTGTGTACTTAAGCTTAAGGTTGATACAGCCAATCCTAAGTGGAATTCTCGGGTGCCAAGGGGCTAAAAGACCCTTGGA |
| SARS2-Probe I.334 | NC_045512.2 | SARS2-Probe I | CTGATGAGGGAGAAACATTAATACGACTCACTATAGGGATTGGACATTCTATGCAAAATTGTGTACTTAAGCTTAAGGTTGATACAGCCAATCCTAAGACACCTAAGTATAAGTTTGTTCGCATTCAATGGAATTCTCGGGTGCCAAGGGGCTAAAAGACCCTTGGA |
| SARS2-Probe I.335 | NC_045512.2 | SARS2-Probe I | CTGATGAGGGAGAAACATTAATACGACTCACTATAGGGAAGCTTAAGGTTGATACAGCCAATCCTAAGACACCTAAGTATAAGTTTGTTCGCATTCAACCAGGACAGACTTTTTCAGTGTTAGCTTGTTGGAATTCTCGGGTGCCAAGGGGCTAAAAGACCCTTGGA |
| SARS2-Probe I.336 | NC_045512.2 | SARS2-Probe I | CTGATGAGGGAGAAACATTAATACGACTCACTATAGGGGACACCTAAGTATAAGTTTGTTCGCATTCAACCAGGACAGACTTTTTCAGTGTTAGCTTGTTACAATGGTTCACCATCTGGTGTTTACCAATGGAATTCTCGGGTGCCAAGGGGCTAAAAGACCCTTGGA |
| SARS2-Probe I.337 | NC_045512.2 | SARS2-Probe I | CTGATGAGGGAGAAACATTAATACGACTCACTATAGGGCCAGGACAGACTTTTTCAGTGTTAGCTTGTTACAATGGTTCACCATCTGGTGTTTACCAATGTGCTATGAGGCCCAATTTCACTATTAAGTGGAATTCTCGGGTGCCAAGGGGCTAAAAGACCCTTGGA |
| SARS2-Probe I.338 | NC_045512.2 | SARS2-Probe I | CTGATGAGGGAGAAACATTAATACGACTCACTATAGGGTACAATGGTTCACCATCTGGTGTTTACCAATGTGCTATGAGGCCCAATTTCACTATTAAGGGTTCATTCCTTAATGGTTCATGTGGTAGTTGGAATTCTCGGGTGCCAAGGGGCTAAAAGACCCTTGGA |
| SARS2-Probe I.339 | NC_045512.2 | SARS2-Probe I | CTGATGAGGGAGAAACATTAATACGACTCACTATAGGGTGTGCTATGAGGCCCAATTTCACTATTAAGGGTTCATTCCTTAATGGTTCATGTGGTAGTGTTGGTTTTAACATAGATTATGACTGTGTCTGGAATTCTCGGGTGCCAAGGGGCTAAAAGACCCTTGGA |
| SARS2-Probe I.340 | NC_045512.2 | SARS2-Probe I | CTGATGAGGGAGAAACATTAATACGACTCACTATAGGGGGTTCATTCCTTAATGGTTCATGTGGTAGTGTTGGTTTTAACATAGATTATGACTGTGTCTCTTTTTGTTACATGCACCATATGGAATTATGGAATTCTCGGGTGCCAAGGGGCTAAAAGACCCTTGGA |
| SARS2-Probe I.341 | NC_045512.2 | SARS2-Probe I | CTGATGAGGGAGAAACATTAATACGACTCACTATAGGGGTTGGTTTTAACATAGATTATGACTGTGTCTCTTTTTGTTACATGCACCATATGGAATTACCAACTGGAGTTCATGCTGGCACAGACTTATGGAATTCTCGGGTGCCAAGGGGCTAAAAGACCCTTGGA |
| SARS2-Probe I.342 | NC_045512.2 | SARS2-Probe I | CTGATGAGGGAGAAACATTAATACGACTCACTATAGGGTCTTTTTGTTACATGCACCATATGGAATTACCAACTGGAGTTCATGCTGGCACAGACTTAGAAGGTAACTTTTATGGACCTTTTGTTGACTGGAATTCTCGGGTGCCAAGGGGCTAAAAGACCCTTGGA |
| SARS2-Probe I.343 | NC_045512.2 | SARS2-Probe I | CTGATGAGGGAGAAACATTAATACGACTCACTATAGGGCCAACTGGAGTTCATGCTGGCACAGACTTAGAAGGTAACTTTTATGGACCTTTTGTTGACAGGCAAACAGCACAAGCAGCTGGTACGGACTGGAATTCTCGGGTGCCAAGGGGCTAAAAGACCCTTGGA |
| SARS2-Probe I.344 | NC_045512.2 | SARS2-Probe I | CTGATGAGGGAGAAACATTAATACGACTCACTATAGGGGAAGGTAACTTTTATGGACCTTTTGTTGACAGGCAAACAGCACAAGCAGCTGGTACGGACACAACTATTACAGTTAATGTTTTAGCTTGGTGGAATTCTCGGGTGCCAAGGGGCTAAAAGACCCTTGGA |
| SARS2-Probe I.345 | NC_045512.2 | SARS2-Probe I | CTGATGAGGGAGAAACATTAATACGACTCACTATAGGGAGGCAAACAGCACAAGCAGCTGGTACGGACACAACTATTACAGTTAATGTTTTAGCTTGGTTGTACGCTGCTGTTATAAATGGAGACAGGTGGAATTCTCGGGTGCCAAGGGGCTAAAAGACCCTTGGA |
| SARS2-Probe I.346 | NC_045512.2 | SARS2-Probe I | CTGATGAGGGAGAAACATTAATACGACTCACTATAGGGGACAACTATTACAGTTAATGTTTTAGCTTGGTTGTACGCTGCTGTTATAAATGGAGACAGGTGGTTTCTCAATCGATTTACCACAACTCTTTGGAATTCTCGGGTGCCAAGGGGCTAAAAGACCCTTGGA |
| SARS2-Probe I.347 | NC_045512.2 | SARS2-Probe I | CTGATGAGGGAGAAACATTAATACGACTCACTATAGGGTTGTACGCTGCTGTTATAAATGGAGACAGGTGGTTTCTCAATCGATTTACCACAACTCTTAATGACTTTAACCTTGTGGCTATGAAGTACAATTATGGAATTCTCGGGTGCCAAGGGGCTAAAAGACCCTTGGA |
| SARS2-Probe I.348 | NC_045512.2 | SARS2-Probe I | CTGATGAGGGAGAAACATTAATACGACTCACTATAGGGTGGTTTCTCAATCGATTTACCACAACTCTTAATGACTTTAACCTTGTGGCTATGAAGTACAATTATGAACCTCTAACACAAGACCATGTTTGGAATTCTCGGGTGCCAAGGGGCTAAAAGACCCTTGGA |
| SARS2-Probe I.349 | NC_045512.2 | SARS2-Probe I | CTGATGAGGGAGAAACATTAATACGACTCACTATAGGGAATGACTTTAACCTTGTGGCTATGAAGTACAATTATGAACCTCTAACACAAGACCATGTTGACATACTAGGACCTCTTTCTGCTCAAACTTGGAATTCTCGGGTGCCAAGGGGCTAAAAGACCCTTGGA |

EP 4 239 083 A2

| ASO ID | Accession | Group ID | Custom Array sequence |
|---|---|---|---|
| SARS2-Probe I.350 | NC_045512.2 | SARS2-Probe I | CTGATGAGGGAGAAACATTAATACGACTCACTATAGGGAATTATGAACCTCTAACACAAGACCATGTTGACATACTAGGACCTCTTTCTGCTCAAACTGGAATTGCCGTTTTAGATATGTGTGCTTCATGGAATTCTCGGGTGCCAAGGGGCTAAAAGACCCTTGGA |
| SARS2-Probe I.351 | NC_045512.2 | SARS2-Probe I | CTGATGAGGGAGAAACATTAATACGACTCACTATAGGGGACATACTAGGACCTCTTTCTGCTCAAACTGGAATTGCCGTTTTAGATATGTGTGCTTCATTAAAAGAATTACTGCAAAATGGTATGAATTGGAATTCTCGGGTGCCAAGGGGCTAAAAGACCCTTGGA |
| SARS2-Probe I.352 | NC_045512.2 | SARS2-Probe I | CTGATGAGGGAGAAACATTAATACGACTCACTATAGGGGGAATTGCCGTTTTAGATATGTGTGCTTCATTAAAAGAATTACTGCAAAATGGTATGAATGGACGTACCATATTGGGTAGTGCTTTATTATGGAATTCTCGGGTGCCAAGGGGCTAAAAGACCCTTGGA |
| SARS2-Probe I.353 | NC_045512.2 | SARS2-Probe I | CTGATGAGGGAGAAACATTAATACGACTCACTATAGGGTTAAAAGAATTACTGCAAAATGGTATGAATGGACGTACCATATTGGGTAGTGCTTTATTAGAAGATGAATTTACACCTTTTGATGTTGTTTGGAATTCTCGGGTGCCAAGGGGCTAAAAGACCCTTGGA |
| SARS2-Probe I.354 | NC_045512.2 | SARS2-Probe I | CTGATGAGGGAGAAACATTAATACGACTCACTATAGGGGGACGTACCATATTGGGTAGTGCTTTATTAGAAGATGAATTTACACCTTTTGATGTTGTTAGACAATGCTCAGGTGTTACTTTCCAAAGTTGGAATTCTCGGGTGCCAAGGGGCTAAAAGACCCTTGGA |
| SARS2-Probe I.355 | NC_045512.2 | SARS2-Probe I | CTGATGAGGGAGAAACATTAATACGACTCACTATAGGGGAAGATGAATTTACACCTTTTGATGTTGTTAGACAATGCTCAGGTGTTACTTTCCAAAGTGCAGTGAAAAGAACAATCAAGGGTACACACTGGAATTCTCGGGTGCCAAGGGGCTAAAAGACCCTTGGA |
| SARS2-Probe I.356 | NC_045512.2 | SARS2-Probe I | CTGATGAGGGAGAAACATTAATACGACTCACTATAGGGAGACAATGCTCAGGTGTTACTTTCCAAAGTGCAGTGAAAAGAACAATCAAGGGTACACACCACTGGTTGTTACTCACAATTTTGACTTCATGGAATTCTCGGGTGCCAAGGGGCTAAAAGACCCTTGGA |
| SARS2-Probe I.357 | NC_045512.2 | SARS2-Probe I | CTGATGAGGGAGAAACATTAATACGACTCACTATAGGGGCAGTGAAAAGAACAATCAAGGGTACACACCACTGGTTGTTACTCACAATTTTGACTTCACTTTTAGTTTTAGTCCAGAGTACTCAATGGTGGAATTCTCGGGTGCCAAGGGGCTAAAAGACCCTTGGA |
| SARS2-Probe I.358 | NC_045512.2 | SARS2-Probe I | CTGATGAGGGAGAAACATTAATACGACTCACTATAGGGCACTGGTTGTTACTCACAATTTTGACTTCACTTTTAGTTTTAGTCCAGAGTACTCAATGGTCTTTGTTCTTTTTTTTGTATGAAAATGCCTGGAATTCTCGGGTGCCAAGGGGCTAAAAGACCCTTGGA |
| SARS2-Probe I.359 | NC_045512.2 | SARS2-Probe I | CTGATGAGGGAGAAACATTAATACGACTCACTATAGGGCTTTTAGTTTTAGTCCAGAGTACTCAATGGTCTTTGTTCTTTTTTTTGTATGAAAATGCCTTTTTACCTTTTGCTATGGGTATTATTGCTTGGAATTCTCGGGTGCCAAGGGGCTAAAAGACCCTTGGA |
| SARS2-Probe I.360 | NC_045512.2 | SARS2-Probe I | CTGATGAGGGAGAAACATTAATACGACTCACTATAGGGTCTTTGTTCTTTTTTTTGTATGAAAATGCCTTTTTACCTTTTGCTATGGGTATTATTGCTATGTCTGCTTTTGCAATGATGTTTGTCAAATGGAATTCTCGGGTGCCAAGGGGCTAAAAGACCCTTGGA |
| SARS2-Probe I.361 | NC_045512.2 | SARS2-Probe I | CTGATGAGGGAGAAACATTAATACGACTCACTATAGGGTTTTTACCTTTTGCTATGGGTATTATTGCTATGTCTGCTTTTGCAATGATGTTTGTCAAACATAAGCATGCATTTCTCTGTTTGTTTTTGTGGAATTCTCGGGTGCCAAGGGGCTAAAAGACCCTTGGA |
| SARS2-Probe I.362 | NC_045512.2 | SARS2-Probe I | CTGATGAGGGAGAAACATTAATACGACTCACTATAGGGATGTCTGCTTTTGCAATGATGTTTGTCAAACATAAGCATGCATTTCTCTGTTTGTTTTTGTTACCTTCTCTTGCCACTGTAGCTTATTTTTGGAATTCTCGGGTGCCAAGGGGCTAAAAGACCCTTGGA |
| SARS2-Probe I.363 | NC_045512.2 | SARS2-Probe I | CTGATGAGGGAGAAACATTAATACGACTCACTATAGGGCATAAGCATGCATTTCTCTGTTTGTTTTTGTTACCTTCTCTTGCCACTGTAGCTTATTTTAATATGGTCTATATGCCTGCTAGTTGGGTGTGGAATTCTCGGGTGCCAAGGGGCTAAAAGACCCTTGGA |
| SARS2-Probe I.364 | NC_045512.2 | SARS2-Probe I | CTGATGAGGGAGAAACATTAATACGACTCACTATAGGGTTACCTTCTCTTGCCACTGTAGCTTATTTTAATATGGTCTATATGCCTGCTAGTTGGGTGATGCGTATTATGACATGGTTGGATATGGTTTGGAATTCTCGGGTGCCAAGGGGCTAAAAGACCCTTGGA |
| SARS2-Probe I.365 | NC_045512.2 | SARS2-Probe I | CTGATGAGGGAGAAACATTAATACGACTCACTATAGGGAATATGGTCTATATGCCTGCTAGTTGGGTGATGCGTATTATGACATGGTTGGATATGGTTGATACTAGTTTGTCTGGTTTTAAGCTAAAATGGAATTCTCGGGTGCCAAGGGGCTAAAAGACCCTTGGA |
| SARS2-Probe I.366 | NC_045512.2 | SARS2-Probe I | CTGATGAGGGAGAAACATTAATACGACTCACTATAGGGATGCGTATTATGACATGGTTGGATATGGTTGATACTAGTTTGTCTGGTTTTAAGCTAAAAGACTGTGTTATGTATGCATCAGCTGTAGTGTGGAATTCTCGGGTGCCAAGGGGCTAAAAGACCCTTGGA |
| SARS2-Probe I.367 | NC_045512.2 | SARS2-Probe I | CTGATGAGGGAGAAACATTAATACGACTCACTATAGGGGATACTAGTTTGTCTGGTTTTAAGCTAAAAGACTGTGTTATGTATGCATCAGCTGTAGTGTTACTAATCCTTATGACAGCAAGAACTGTGTGGAATTCTCGGGTGCCAAGGGGCTAAAAGACCCTTGGA |
| SARS2-Probe I.368 | NC_045512.2 | SARS2-Probe I | CTGATGAGGGAGAAACATTAATACGACTCACTATAGGGGACTGTGTTATGTATGCATCAGCTGTAGTGTTACTAATCCTTATGACAGCAAGAACTGTGTATGATGATGGTGCTAGGAGAGTGTGGACATGGAATTCTCGGGTGCCAAGGGGCTAAAAGACCCTTGGA |
| SARS2-Probe I.369 | NC_045512.2 | SARS2-Probe I | CTGATGAGGGAGAAACATTAATACGACTCACTATAGGGGTTACTAATCCTTATGACAGCAAGAACTGTGTATGATGATGGTGCTAGGAGAGTGTGGACACTTATGAATGTCTTGACACTCGTTTATAAATGGAATTCTCGGGTGCCAAGGGGCTAAAAGACCCTTGGA |
| SARS2-Probe I.370 | NC_045512.2 | SARS2-Probe I | CTGATGAGGGAGAAACATTAATACGACTCACTATAGGGTATGATGATGGTGCTAGGAGAGTGTGGACACTTATGAATGTCTTGACACTCGTTTATAAAGTTTATTATGGTAATGCTTTAGATCAAGCCTGGAATTCTCGGGTGCCAAGGGGCTAAAAGACCCTTGGA |
| SARS2-Probe I.371 | NC_045512.2 | SARS2-Probe I | CTGATGAGGGAGAAACATTAATACGACTCACTATAGGGCTTATGAATGTCTTGACACTCGTTTATAAAGTTTATTATGGTAATGCTTTAGATCAAGCCATTTCCATGTGGGCTCTTATAATCTCTGTTTGGAATTCTCGGGTGCCAAGGGGCTAAAAGACCCTTGGA |
| SARS2-Probe I.372 | NC_045512.2 | SARS2-Probe I | CTGATGAGGGAGAAACATTAATACGACTCACTATAGGGGTTTATTATGGTAATGCTTTAGATCAAGCCATTTCCATGTGGGCTCTTATAATCTCTGTTACTTCTAACTACTCAGGTGTAGTTACAACTTGGAATTCTCGGGTGCCAAGGGGCTAAAAGACCCTTGGA |
| SARS2-Probe I.373 | NC_045512.2 | SARS2-Probe I | CTGATGAGGGAGAAACATTAATACGACTCACTATAGGGATTTCCATGTGGGCTCTTATAATCTCTGTTACTTCTAACTACTCAGGTGTAGTTACAACTGTCATGTTTTTGGCCAGAGGTATTGTTTTTTGGAATTCTCGGGTGCCAAGGGGCTAAAAGACCCTTGGA |
| SARS2-Probe I.374 | NC_045512.2 | SARS2-Probe I | CTGATGAGGGAGAAACATTAATACGACTCACTATAGGGACTTCTAACTACTCAGGTGTAGTTACAACTGTCATGTTTTTGGCCAGAGGTATTGTTTTTATGTGTGTTGAGTATTGCCCTATTTTCTTCTGGAATTCTCGGGTGCCAAGGGGCTAAAAGACCCTTGGA |

36

| ASO ID | Accession | Group ID | Custom Array sequence |
|---|---|---|---|
| SARS2-Probe I.375 | NC_045512.2 | SARS2-Probe I | CTGATGAGGGAGAAACATTAATACGACTCACTATAGGGGTCATGTTTTTGGCCAGAGGTATTGTTTTTATGTGTGTTGAGTATTGCCCTATTTTCTTCATAACTGGTAATACACTTCAGTGTATAATGTGGAATTCTCGGGTGCCAAGGGGCTAAAAGACCCTTGGA |
| SARS2-Probe I.376 | NC_045512.2 | SARS2-Probe I | CTGATGAGGGAGAAACATTAATACGACTCACTATAGGGGATGTGTGTTGAGTATTGCCCTATTTTCTTCATAACTGGTAATACACTTCAGTGTATAATGCTAGTTTATTGTTTCTTAGGCTATTTTTGTTGGAATTCTCGGGTGCCAAGGGGCTAAAAGACCCTTGGA |
| SARS2-Probe I.377 | NC_045512.2 | SARS2-Probe I | CTGATGAGGGAGAAACATTAATACGACTCACTATAGGGGATAACTGGTAATACACTTCAGTGTATAATGCTAGTTTATTGTTTCTTAGGCTATTTTTGTACTTGTTACTTTGGCCTCTTTTGTTTACTCTGGAATTCTCGGGTGCCAAGGGGCTAAAAGACCCTTGGA |
| SARS2-Probe I.378 | NC_045512.2 | SARS2-Probe I | CTGATGAGGGAGAAACATTAATACGACTCACTATAGGGCTAGTTTATTGTTTCTTAGGCTATTTTTGTACTTGTTACTTTGGCCTCTTTTGTTTACTCAACCGCTACTTTAGACTGACTCTTGGTGTTTGGAATTCTCGGGTGCCAAGGGGCTAAAAGACCCTTGGA |
| SARS2-Probe I.379 | NC_045512.2 | SARS2-Probe I | CTGATGAGGGAGAAACATTAATACGACTCACTATAGGGACTTGTTACTTTGGCCTCTTTTGTTTACTCAACCGCTACTTTAGACTGACTCTTGGTGTTTATGATTACTTAGTTTCTACACAGGAGTTTTGGAATTCTCGGGTGCCAAGGGGCTAAAAGACCCTTGGA |
| SARS2-Probe I.380 | NC_045512.2 | SARS2-Probe I | CTGATGAGGGAGAAACATTAATACGACTCACTATAGGGAACCGCTACTTTAGACTGACTCTTGGTGTTTATGATTACTTAGTTTCTACACAGGAGTTTAGATATATGAATTCACAGGGACTACTCCCATGGAATTCTCGGGTGCCAAGGGGCTAAAAGACCCTTGGA |
| SARS2-Probe I.381 | NC_045512.2 | SARS2-Probe I | CTGATGAGGGAGAAACATTAATACGACTCACTATAGGGTATGATTACTTAGTTTCTACACAGGAGTTTAGATATATGAATTCACAGGGACTACTCCCACCCAAGAATAGCATAGATGCCTTCAAACTCTGGAATTCTCGGGTGCCAAGGGGCTAAAAGACCCTTGGA |
| SARS2-Probe I.382 | NC_045512.2 | SARS2-Probe I | CTGATGAGGGAGAAACATTAATACGACTCACTATAGGGAGATATATGAATTCACAGGGACTACTCCCACCCAAGAATAGCATAGATGCCTTCAAACTCAACATTAAATTGTTGGGTGTTGGTGGCAAATGGAATTCTCGGGTGCCAAGGGGCTAAAAGACCCTTGGA |
| SARS2-Probe I.383 | NC_045512.2 | SARS2-Probe I | CTGATGAGGGAGAAACATTAATACGACTCACTATAGGGCCCAAGAATAGCATAGATGCCTTCAAACTCAACATTAAATTGTTGGGTGTTGGTGGCAAACCTTGTATCAAAGTAGCCACTGTACAGTCTTGGAATTCTCGGGTGCCAAGGGGCTAAAAGACCCTTGGA |
| SARS2-Probe I.384 | NC_045512.2 | SARS2-Probe I | CTGATGAGGGAGAAACATTAATACGACTCACTATAGGGAACATTAAATTGTTGGGTGTTGGTGGCAAACCTTGTATCAAAGTAGCCACTGTACAGTCTAAAATGTCAGATGTAAAGTGCACATCAGTATGGAATTCTCGGGTGCCAAGGGGCTAAAAGACCCTTGGA |
| SARS2-Probe I.385 | NC_045512.2 | SARS2-Probe I | CTGATGAGGGAGAAACATTAATACGACTCACTATAGGGCCTTGTATCAAAGTAGCCACTGTACAGTCTAAAATGTCAGATGTAAAGTGCACATCAGTAGTCTTACTCTCAGTTTTGCAACAACTCAGATGGAATTCTCGGGTGCCAAGGGGCTAAAAGACCCTTGGA |
| SARS2-Probe I.386 | NC_045512.2 | SARS2-Probe I | CTGATGAGGGAGAAACATTAATACGACTCACTATAGGGAAAATGTCAGATGTAAAGTGCACATCAGTAGTCTTACTCTCAGTTTTGCAACAACTCAGATAGAATCATCATCTAAATTGTGGGCTCAATGGAATTCTCGGGTGCCAAGGGGCTAAAAGACCCTTGGA |
| SARS2-Probe I.387 | NC_045512.2 | SARS2-Probe I | CTGATGAGGGAGAAACATTAATACGACTCACTATAGGGGTCTTACTCTCAGTTTTGCAACAACTCAGATAGAATCATCATCTAAATTGTGGGCTCAATGTGTCCAGTTACACAATGACATTCTCTTATGGAATTCTCGGGTGCCAAGGGGCTAAAAGACCCTTGGA |
| SARS2-Probe I.388 | NC_045512.2 | SARS2-Probe I | CTGATGAGGGAGAAACATTAATACGACTCACTATAGGGGTAGAATCATCATCTAAATTGTGGGCTCAATGTGTCCAGTTACACAATGACATTCTCTTAGCTAAAGATACTACTGAAGCCTTTGAAAAATGGAATTCTCGGGTGCCAAGGGGCTAAAAGACCCTTGGA |
| SARS2-Probe I.389 | NC_045512.2 | SARS2-Probe I | CTGATGAGGGAGAAACATTAATACGACTCACTATAGGGTGTGTCCAGTTACACAATGACATTCTCTTAGCTAAAGATACTACTGAAGCCTTTGAAAAATGGTTTCACTACTTTCTGTTTTGCTTTCCTGGAATTCTCGGGTGCCAAGGGGCTAAAAGACCCTTGGA |
| SARS2-Probe I.390 | NC_045512.2 | SARS2-Probe I | CTGATGAGGGAGAAACATTAATACGACTCACTATAGGGGCTAAAGATACTACTGAAGCCTTTGAAAAAATGGTTTCACTACTTTCTGTTTTGCTTTCCATGCAGGGTGCTGTAGACATAAACAAGCTTTGGAATTCTCGGGTGCCAAGGGGCTAAAAGACCCTTGGA |
| SARS2-Probe I.391 | NC_045512.2 | SARS2-Probe I | CTGATGAGGGAGAAACATTAATACGACTCACTATAGGGGATGGTTTCACTACTTTCTGTTTTGCTTTCCATGCAGGGTGCTGTAGACATAAACAAGCTTTGTGAAGAAATGCTGGACAACAGGGCAACCTGGAATTCTCGGGTGCCAAGGGGCTAAAAGACCCTTGGA |
| SARS2-Probe I.392 | NC_045512.2 | SARS2-Probe I | CTGATGAGGGAGAAACATTAATACGACTCACTATAGGGGATGCAGGGTGCTGTAGACATAAACAAGCTTTGTGAAGAAATGCTGGACAACAGGGCAACCTTACAAGCTATAGCCTCAGAGTTTAGTTCCTGGAATTCTCGGGTGCCAAGGGGCTAAAAGACCCTTGGA |
| SARS2-Probe I.393 | NC_045512.2 | SARS2-Probe I | CTGATGAGGGAGAAACATTAATACGACTCACTATAGGGTGTGAAGAAATGCTGGACAACAGGGCAACCTTACAAGCTATAGCCTCAGAGTTTAGTTCCCTTCCATCATATGCAGCTTTTGCTACTGCTTGGAATTCTCGGGTGCCAAGGGGCTAAAAGACCCTTGGA |
| SARS2-Probe I.394 | NC_045512.2 | SARS2-Probe I | CTGATGAGGGAGAAACATTAATACGACTCACTATAGGGTTACAAGCTATAGCCTCAGAGTTTAGTTCCCTTCCATCATATGCAGCTTTTGCTACTGCTCAAGAAGCTTATGAGCAGGCTGTTGCTAATTGGAATTCTCGGGTGCCAAGGGGCTAAAAGACCCTTGGA |
| SARS2-Probe I.395 | NC_045512.2 | SARS2-Probe I | CTGATGAGGGAGAAACATTAATACGACTCACTATAGGGCTTCCATCATATGCAGCTTTTGCTACTGCTCAAGAAGCTTATGAGCAGGCTGTTGCTAATGGTGATTCTGAAGTTGTTCTTAAAAAGTTGTGGAATTCTCGGGTGCCAAGGGGCTAAAAGACCCTTGGA |
| SARS2-Probe I.396 | NC_045512.2 | SARS2-Probe I | CTGATGAGGGAGAAACATTAATACGACTCACTATAGGGCAAGAAGCTTATGAGCAGGCTGTTGCTAATGGTGATTCTGAAGTTGTTCTTAAAAAGTTGAAGAAGTCTTTGAATGTGGCTAAATCTGAATGGAATTCTCGGGTGCCAAGGGGCTAAAAGACCCTTGGA |
| SARS2-Probe I.397 | NC_045512.2 | SARS2-Probe I | CTGATGAGGGAGAAACATTAATACGACTCACTATAGGGGGGTGATTCTGAAGTTGTTCTTAAAAAGTTGAAGAAGTCTTTGAATGTGGCTAAATCTGAATTTGACCGTGATGCAGCCATGCAACGTAAGTGGAATTCTCGGGTGCCAAGGGGCTAAAAGACCCTTGGA |
| SARS2-Probe I.398 | NC_045512.2 | SARS2-Probe I | CTGATGAGGGAGAAACATTAATACGACTCACTATAGGGAAGAAGTCTTTGAATGTGGCTAAATCTGAATTTGACCGTGATGCAGCCATGCAACGTAAGTTGGAAAAGATGGCTGATCAAGCTATGACCTGGAATTCTCGGGTGCCAAGGGGCTAAAAGACCCTTGGA |
| SARS2-Probe I.399 | NC_045512.2 | SARS2-Probe I | CTGATGAGGGAGAAACATTAATACGACTCACTATAGGGTTTGACCGTGATGCAGCCATGCAACGTAAGTTGGAAAAGATGGCTGATCAAGCTATGACCCAAATGTATAAACAGGCTAGATCTGAGGACTGGAATTCTCGGGTGCCAAGGGGCTAAAAGACCCTTGGA |

38

| ASO ID | Accession | Group ID | Custom Array sequence |
|---|---|---|---|
| SARS2-Probe I.400 | NC_045512.2 | SARS2-Probe I | CTGATGAGGGAGAAACATTAATACGACTCACTATAGGGTTGGAAAAGATGGCTGATCAAGCTATGACCCAAATGTATAAACAGGCTAGATCTGAGGACAAGAGGGCAAAAGTTACTAGTGCTATGCAGTGGAATTCTCGGGTGCCAAGGGGCTAAAAGACCCTTGGA |
| SARS2-Probe I.401 | NC_045512.2 | SARS2-Probe I | CTGATGAGGGAGAAACATTAATACGACTCACTATAGGGCAAATGTATAAACAGGCTAGATCTGAGGACAAGAGGGCAAAAGTTACTAGTGCTATGCAGACAATGCTTTTCACTATGCTTAGAAAGTTGTGGAATTCTCGGGTGCCAAGGGGCTAAAAGACCCTTGGA |
| SARS2-Probe I.402 | NC_045512.2 | SARS2-Probe I | CTGATGAGGGAGAAACATTAATACGACTCACTATAGGGAAGAGGGCAAAAGTTACTAGTGCTATGCAGACAATGCTTTTCACTATGCTTAGAAAGTTGGATAATGATGCACTCAACAACATTATCAACTGGAATTCTCGGGTGCCAAGGGGCTAAAAGACCCTTGGA |
| SARS2-Probe I.403 | NC_045512.2 | SARS2-Probe I | CTGATGAGGGAGAAACATTAATACGACTCACTATAGGGACAATGCTTTTCACTATGCTTAGAAAGTTGGATAATGATGCACTCAACAACATTATCAACAATGCAAGAGATGGTTGTGTTCCCTTGAACTGGAATTCTCGGGTGCCAAGGGGCTAAAAGACCCTTGGA |
| SARS2-Probe I.404 | NC_045512.2 | SARS2-Probe I | CTGATGAGGGAGAAACATTAATACGACTCACTATAGGGGATAATGATGCACTCAACAACATTATCAACAATGCAAGAGATGGTTGTGTTCCCTTGAACATAATACCTCTTACAACAGCAGCCAAACTATGGAATTCTCGGGTGCCAAGGGGCTAAAAGACCCTTGGA |
| SARS2-Probe I.405 | NC_045512.2 | SARS2-Probe I | CTGATGAGGGAGAAACATTAATACGACTCACTATAGGGAATGCAAGAGATGGTTGTGTTCCCTTGAACATAATACCTCTTACAACAGCAGCCAAACTAATGGTTGTCATACCAGACTATAACACATATTGGAATTCTCGGGTGCCAAGGGGCTAAAAGACCCTTGGA |
| SARS2-Probe I.406 | NC_045512.2 | SARS2-Probe I | CTGATGAGGGAGAAACATTAATACGACTCACTATAGGGATAATACCTCTTACAACAGCAGCCAAACTAATGGTTGTCATACCAGACTATAACACATATAAAAATACGTGTGATGGTACAACATTTACTTGGAATTCTCGGGTGCCAAGGGGCTAAAAGACCCTTGGA |
| SARS2-Probe I.407 | NC_045512.2 | SARS2-Probe I | CTGATGAGGGAGAAACATTAATACGACTCACTATAGGGATGGTTGTCATACCAGACTATAACACATATAAAAATACGTGTGATGGTACAACATTTACTTATGCATCAGCATTGTGGGAAATCCAACAGTGGAATTCTCGGGTGCCAAGGGGCTAAAAGACCCTTGGA |
| SARS2-Probe I.408 | NC_045512.2 | SARS2-Probe I | CTGATGAGGGAGAAACATTAATACGACTCACTATAGGGAAAAATACGTGTGATGGTACAACATTTACTTATGCATCAGCATTGTGGGAAATCCAACAGGTTGTAGATGCAGATAGTAAAATTGTTCAATGGAATTCTCGGGTGCCAAGGGGCTAAAAGACCCTTGGA |
| SARS2-Probe I.409 | NC_045512.2 | SARS2-Probe I | CTGATGAGGGAGAAACATTAATACGACTCACTATAGGGTATGCATCAGCATTGTGGGAAATCCAACAGGTTGTAGATGCAGATAGTAAAATTGTTCAACTTAGTGAAATTAGTATGGACAATTCACCTTGGAATTCTCGGGTGCCAAGGGGCTAAAAGACCCTTGGA |
| SARS2-Probe I.410 | NC_045512.2 | SARS2-Probe I | CTGATGAGGGAGAAACATTAATACGACTCACTATAGGGGTTGTAGATGCAGATAGTAAAATTGTTCAACTTAGTGAAATTAGTATGGACAATTCACCTAATTTAGCATGGCCTCTTATTGTAACAGCTTGGAATTCTCGGGTGCCAAGGGGCTAAAAGACCCTTGGA |
| SARS2-Probe I.411 | NC_045512.2 | SARS2-Probe I | CTGATGAGGGAGAAACATTAATACGACTCACTATAGGGCTTAGTGAAATTAGTATGGACAATTCACCTAATTTAGCATGGCCTCTTATTGTAACAGCTTTAAGGGCCAATTCTGCTGTCAAATTACAGTGGAATTCTCGGGTGCCAAGGGGCTAAAAGACCCTTGGA |
| SARS2-Probe I.412 | NC_045512.2 | SARS2-Probe I | CTGATGAGGGAGAAACATTAATACGACTCACTATAGGGAATTTAGCATGGCCTCTTATTGTAACAGCTTTAAGGGCCAATTCTGCTGTCAAATTACAGAATAATGAGCTTAGTCCTGTTGCACTACGATGGAATTCTCGGGTGCCAAGGGGCTAAAAGACCCTTGGA |
| SARS2-Probe I.413 | NC_045512.2 | SARS2-Probe I | CTGATGAGGGAGAAACATTAATACGACTCACTATAGGGTTAAGGGCCAATTCTGCTGTCAAATTACAGAATAATGAGCTTAGTCCTGTTGCACTACGACAGATGTCTTGTGCTGCCGGTACTACACAATGGAATTCTCGGGTGCCAAGGGGCTAAAAGACCCTTGGA |
| SARS2-Probe I.414 | NC_045512.2 | SARS2-Probe I | CTGATGAGGGAGAAACATTAATACGACTCACTATAGGGAATAATGAGCTTAGTCCTGTTGCACTACGACAGATGTCTTGTGCTGCCGGTACTACACAAACTGCTTGCACTGATGACAATGCGTTAGCTTGGAATTCTCGGGTGCCAAGGGGCTAAAAGACCCTTGGA |
| SARS2-Probe I.415 | NC_045512.2 | SARS2-Probe I | CTGATGAGGGAGAAACATTAATACGACTCACTATAGGGCAGATGTCTTGTGCTGCCGGTACTACACAAACTGCTTGCACTGATGACAATGCGTTAGCTTACTACAACACAACAAAGGGAGGTAGGTTTTGGAATTCTCGGGTGCCAAGGGGCTAAAAGACCCTTGGA |
| SARS2-Probe I.416 | NC_045512.2 | SARS2-Probe I | CTGATGAGGGAGAAACATTAATACGACTCACTATAGGGACTGCTTGCACTGATGACAATGCGTTAGCTTACTACAACACAACAAAGGGAGGTAGGTTTGTACTTGCACTGTTATCCGATTTACAGGATTGGAATTCTCGGGTGCCAAGGGGCTAAAAGACCCTTGGA |
| SARS2-Probe I.417 | NC_045512.2 | SARS2-Probe I | CTGATGAGGGAGAAACATTAATACGACTCACTATAGGGTACTACAACACAACAAAGGGAGGTAGGTTTGTACTTGCACTGTTATCCGATTTACAGGATTTGAAATGGGCTAGATTCCCTAAGAGTGATTGGAATTCTCGGGTGCCAAGGGGCTAAAAGACCCTTGGA |
| SARS2-Probe I.418 | NC_045512.2 | SARS2-Probe I | CTGATGAGGGAGAAACATTAATACGACTCACTATAGGGGTACTTGCACTGTTATCCGATTTACAGGATTTGAAATGGGCTAGATTCCCTAAGAGTGATGGAACTGGTACTATCTATACAGAACTGGAATGGAATTCTCGGGTGCCAAGGGGCTAAAAGACCCTTGGA |
| SARS2-Probe I.419 | NC_045512.2 | SARS2-Probe I | CTGATGAGGGAGAAACATTAATACGACTCACTATAGGGTTTGAAATGGGCTAGATTCCCTAAGAGTGATGGAACTGGTACTATCTATACAGAACTGGAACCACCTTGTAGGTTTGTTACAGACACACCTTGGAATTCTCGGGTGCCAAGGGGCTAAAAGACCCTTGGA |
| SARS2-Probe I.420 | NC_045512.2 | SARS2-Probe I | CTGATGAGGGAGAAACATTAATACGACTCACTATAGGGGGGAACTGGTACTATCTATACAGAACTGGAACCACCTTGTAGGTTTGTTACAGACACACCTAAAGGTCCTAAAGTGAAGTATTTATACTTTTGGAATTCTCGGGTGCCAAGGGGCTAAAAGACCCTTGGA |
| SARS2-Probe I.421 | NC_045512.2 | SARS2-Probe I | CTGATGAGGGAGAAACATTAATACGACTCACTATAGGGCCACCTTGTAGGTTTGTTACAGACACACCTAAAGGTCCTAAAGTGAAGTATTTATACTTTATTAAAGGATTAAACAACCTAAATAGAGGTTGGAATTCTCGGGTGCCAAGGGGCTAAAAGACCCTTGGA |
| SARS2-Probe I.422 | NC_045512.2 | SARS2-Probe I | CTGATGAGGGAGAAACATTAATACGACTCACTATAGGGAAAGGTCCTAAAGTGAAGTATTTATACTTTATTAAAGGATTAAACAACCTAAATAGAGGTATGGTACTTGGTAGTTTAGCTGCCACAGTATGGAATTCTCGGGTGCCAAGGGGCTAAAAGACCCTTGGA |
| SARS2-Probe I.423 | NC_045512.2 | SARS2-Probe I | CTGATGAGGGAGAAACATTAATACGACTCACTATAGGGATTAAAGGATTAAACAACCTAAATAGAGGTATGGTACTTGGTAGTTTAGCTGCCACAGTACGTCTACAAGCTGGTAATGCAACAGAAGTGTGGAATTCTCGGGTGCCAAGGGGCTAAAAGACCCTTGGA |
| SARS2-Probe I.424 | NC_045512.2 | SARS2-Probe I | CTGATGAGGGAGAAACATTAATACGACTCACTATAGGGATGGTACTTGGTAGTTTAGCTGCCACAGTACGTCTACAAGCTGGTAATGCAACAGAAGTGCCTGCCAATTCAACTGTATTATCTTTCTGTTGGAATTCTCGGGTGCCAAGGGGCTAAAAGACCCTTGGA |

| ASO ID | Accession | Group ID | Custom Array sequence |
|---|---|---|---|
| SARS2-Probe I.425 | NC_045512.2 | SARS2-Probe I | CTGATGAGGGAGAAACATTAATACGACTCACTATAGGGCGTCTACAAGCTGGTAATGCAACAGAAGTGCCTGCCAATTCAACTGTATTATCTTTCTGTGCTTTTGCTGTAGATGCTGCTAAAGCTTACTGGAATTCTCGGGTGCCAAGGGGCTAAAAGACCCTTGGA |
| SARS2-Probe I.426 | NC_045512.2 | SARS2-Probe I | CTGATGAGGGAGAAACATTAATACGACTCACTATAGGGCCTGCCAATTCAACTGTATTATCTTTCTGTGCTTTTGCTGTAGATGCTGCTAAAGCTTACAAAGATTATCTAGCTAGTGGGGGACAACCATGGAATTCTCGGGTGCCAAGGGGCTAAAAGACCCTTGGA |
| SARS2-Probe I.427 | NC_045512.2 | SARS2-Probe I | CTGATGAGGGAGAAACATTAATACGACTCACTATAGGGGCTTTTGCTGTAGATGCTGCTAAAGCTTACAAAGATTATCTAGCTAGTGGGGGACAACCAATCACTAATTGTGTTAAGATGTTGTGTACATGGAATTCTCGGGTGCCAAGGGGCTAAAAGACCCTTGGA |
| SARS2-Probe I.428 | NC_045512.2 | SARS2-Probe I | CTGATGAGGGAGAAACATTAATACGACTCACTATAGGGAAAGATTATCTAGCTAGTGGGGGACAACCAATCACTAATTGTGTTAAGATGTTGTGTACACACACTGGTACTGGTCAGGCAATAACAGTTTGGAATTCTCGGGTGCCAAGGGGCTAAAAGACCCTTGGA |
| SARS2-Probe I.429 | NC_045512.2 | SARS2-Probe I | CTGATGAGGGAGAAACATTAATACGACTCACTATAGGGATCACTAATTGTGTTAAGATGTTGTGTACACACACTGGTACTGGTCAGGCAATAACAGTTACACCGGAAGCCAATATGGATCAAGAATCCTGGAATTCTCGGGTGCCAAGGGGCTAAAAGACCCTTGGA |
| SARS2-Probe I.430 | NC_045512.2 | SARS2-Probe I | CTGATGAGGGAGAAACATTAATACGACTCACTATAGGGCACACTGGTACTGGTCAGGCAATAACAGTTACACCGGAAGCCAATATGGATCAAGAATCCTTTGGTGGTGCATCGTGTTGTCTGTACTGCTGGAATTCTCGGGTGCCAAGGGGCTAAAAGACCCTTGGA |
| SARS2-Probe I.431 | NC_045512.2 | SARS2-Probe I | CTGATGAGGGAGAAACATTAATACGACTCACTATAGGGACACCGGAAGCCAATATGGATCAAGAATCCTTTGGTGGTGCATCGTGTTGTCTGTACTGCCGTTGCCACATAGATCATCCAAATCCTAAATGGAATTCTCGGGTGCCAAGGGGCTAAAAGACCCTTGGA |
| SARS2-Probe I.432 | NC_045512.2 | SARS2-Probe I | CTGATGAGGGAGAAACATTAATACGACTCACTATAGGGTTTGGTGGTGCATCGTGTTGTCTGTACTGCCGTTGCCACATAGATCATCCAAATCCTAAAGGATTTTGTGACTTAAAAGGTAAGTATGTATGGAATTCTCGGGTGCCAAGGGGCTAAAAGACCCTTGGA |
| SARS2-Probe I.433 | NC_045512.2 | SARS2-Probe I | CTGATGAGGGAGAAACATTAATACGACTCACTATAGGGCGTTGCCACATAGATCATCCAAATCCTAAAGGATTTTGTGACTTAAAAGGTAAGTATGTACAAATACCTACAACTTGTGCTAATGACCCTTGGAATTCTCGGGTGCCAAGGGGCTAAAAGACCCTTGGA |
| SARS2-Probe I.434 | NC_045512.2 | SARS2-Probe I | CTGATGAGGGAGAAACATTAATACGACTCACTATAGGGGGGATTTTGTGACTTAAAAGGTAAGTATGTACAAATACCTACAACTTGTGCTAATGACCCTGTGGGTTTTACACTTAAAAACACAGTCTGTTGGAATTCTCGGGTGCCAAGGGGCTAAAAGACCCTTGGA |
| SARS2-Probe I.435 | NC_045512.2 | SARS2-Probe I | CTGATGAGGGAGAAACATTAATACGACTCACTATAGGGCAAATACCTACAACTTGTGCTAATGACCCTGTGGGTTTTACACTTAAAAACACAGTCTGTACCGTCTGCGGTATGTGGAAAGGTTATGGCTGGAATTCTCGGGTGCCAAGGGGCTAAAAGACCCTTGGA |
| SARS2-Probe I.436 | NC_045512.2 | SARS2-Probe I | CTGATGAGGGAGAAACATTAATACGACTCACTATAGGGGTGGGTTTTACACTTAAAAACACAGTCTGTACCGTCTGCGGTATGTGGAAAGGTTATGGCTGTAGTTGTGATCAACTCCGCGAACCCATGTGGAATTCTCGGGTGCCAAGGGGCTAAAAGACCCTTGGA |
| SARS2-Probe I.437 | NC_045512.2 | SARS2-Probe I | CTGATGAGGGAGAAACATTAATACGACTCACTATAGGGACCGTCTGCGGTATGTGGAAAGGTTATGGCTGTAGTTGTGATCAACTCCGCGAACCCATGCTTCAGTCAGCTGATGCACAATCGTTTTTATGGAATTCTCGGGTGCCAAGGGGCTAAAAGACCCTTGGA |
| SARS2-Probe I.438 | NC_045512.2 | SARS2-Probe I | CTGATGAGGGAGAAACATTAATACGACTCACTATAGGGTGTAGTTGTGATCAACTCCGCGAACCCATGCTTCAGTCAGCTGATGCACAATCGTTTTTAAACGGGTTTGCGGTGTAAGTGCAGCCCGTCTGGAATTCTCGGGTGCCAAGGGGCTAAAAGACCCTTGGA |
| SARS2-Probe I.439 | NC_045512.2 | SARS2-Probe I | CTGATGAGGGAGAAACATTAATACGACTCACTATAGGGCTTCAGTCAGCTGATGCACAATCGTTTTTAAACGGGTTTGCGGTGTAAGTGCAGCCCGTCTTACACCGTGCGGCACAGGCACTAGTACTGTGGAATTCTCGGGTGCCAAGGGGCTAAAAGACCCTTGGA |
| SARS2-Probe I.440 | NC_045512.2 | SARS2-Probe I | CTGATGAGGGAGAAACATTAATACGACTCACTATAGGGAACGGGTTTGCGGTGTAAGTGCAGCCCGTCTTACACCGTGCGGCACAGGCACTAGTACTGATGTCGTATACAGGGCTTTTGACATCTACATGGAATTCTCGGGTGCCAAGGGGCTAAAAGACCCTTGGA |
| SARS2-Probe I.441 | NC_045512.2 | SARS2-Probe I | CTGATGAGGGAGAAACATTAATACGACTCACTATAGGGTTACACCGTGCGGCACAGGCACTAGTACTGATGTCGTATACAGGGCTTTTGACATCTACAATGATAAAGTAGCTGGTTTTGCTAAATTCCTGGAATTCTCGGGTGCCAAGGGGCTAAAAGACCCTTGGA |
| SARS2-Probe I.442 | NC_045512.2 | SARS2-Probe I | CTGATGAGGGAGAAACATTAATACGACTCACTATAGGGATGTCGTATACAGGGCTTTTGACATCTACAATGATAAAGTAGCTGGTTTTGCTAAATTCCTAAAAACTAATTGTTGTCGCTTCCAAGAAATGGAATTCTCGGGTGCCAAGGGGCTAAAAGACCCTTGGA |
| SARS2-Probe I.443 | NC_045512.2 | SARS2-Probe I | CTGATGAGGGAGAAACATTAATACGACTCACTATAGGGATGATAAAGTAGCTGGTTTTGCTAAATTCCTAAAAACTAATTGTTGTCGCTTCCAAGAAAAGGACGAAGATGACAATTTAATTGATTCTTTGGAATTCTCGGGTGCCAAGGGGCTAAAAGACCCTTGGA |
| SARS2-Probe I.444 | NC_045512.2 | SARS2-Probe I | CTGATGAGGGAGAAACATTAATACGACTCACTATAGGGTAAAAACTAATTGTTGTCGCTTCCAAGAAAAGGACGAAGATGACAATTTAATTGATTCTTACTTTGTAGTTAAGAGACACACTTTCTCTATGGAATTCTCGGGTGCCAAGGGGCTAAAAGACCCTTGGA |
| SARS2-Probe I.445 | NC_045512.2 | SARS2-Probe I | CTGATGAGGGAGAAACATTAATACGACTCACTATAGGGAGGACGAAGATGACAATTTAATTGATTCTTACTTTGTAGTTAAGAGACACACTTTCTCTAACTACCAACATGAAGAAACAATTTATAATTTGGAATTCTCGGGTGCCAAGGGGCTAAAAGACCCTTGGA |
| SARS2-Probe I.446 | NC_045512.2 | SARS2-Probe I | CTGATGAGGGAGAAACATTAATACGACTCACTATAGGGACTTTGTAGTTAAGAGACACACTTTCTCTAACTACCAACATGAAGAAACAATTTATAATTTACTTAAGGATTGTCCAGCTGTTGCTAAACTGGAATTCTCGGGTGCCAAGGGGCTAAAAGACCCTTGGA |
| SARS2-Probe I.447 | NC_045512.2 | SARS2-Probe I | CTGATGAGGGAGAAACATTAATACGACTCACTATAGGGACTACCAACATGAAGAAACAATTTATAATTTACTTAAGGATTGTCCAGCTGTTGCTAAACATGACTTCTTTAAGTTTAGAATAGACGGTGTGGAATTCTCGGGTGCCAAGGGGCTAAAAGACCCTTGGA |
| SARS2-Probe I.448 | NC_045512.2 | SARS2-Probe I | CTGATGAGGGAGAAACATTAATACGACTCACTATAGGGTACTTAAGGATTGTCCAGCTGTTGCTAAACATGACTTCTTTAAGTTTAGAATAGACGGTGACATGGTACCACATATATCACGTCAACGTCTGGAATTCTCGGGTGCCAAGGGGCTAAAAGACCCTTGGA |
| SARS2-Probe I.449 | NC_045512.2 | SARS2-Probe I | CTGATGAGGGAGAAACATTAATACGACTCACTATAGGGATGACTTCTTTAAGTTTAGAATAGACGGTGACATGGTACCACATATATCACGTCAACGTCTTACTAAATACACAATGGCAGACCTCGTCTTGGAATTCTCGGGTGCCAAGGGGCTAAAAGACCCTTGGA |

| ASO ID | Accession | Group ID | Custom Array sequence |
|---|---|---|---|
| SARS2-Probe I.450 | NC_045512.2 | SARS2-Probe I | CTGATGAGGGAGAAACATTAATACGACTCACTATAGGGACATGGTACCACATATATCACGTCAACGTCTTACTAAATACACAATGGCAGACCTCGTCTATGCTTTAAGGCATTTTGATGAAGGTAATTTGGAATTCTCGGGTGCCAAGGGGCTAAAAGACCCTTGGA |
| SARS2-Probe I.451 | NC_045512.2 | SARS2-Probe I | CTGATGAGGGAGAAACATTAATACGACTCACTATAGGGTTACTAAATACACAATGGCAGACCTCGTCTATGCTTTAAGGCATTTTGATGAAGGTAATTGTGACACATTAAAAGAAATACTTGTCACATTGGAATTCTCGGGTGCCAAGGGGCTAAAAGACCCTTGGA |
| SARS2-Probe I.452 | NC_045512.2 | SARS2-Probe I | CTGATGAGGGAGAAACATTAATACGACTCACTATAGGGATGCTTTAAGGCATTTTGATGAAGGTAATTGTGACACATTAAAAGAAATACTTGTCACATACAATTGTTGTGATGATGATTATTTCAATATGGAATTCTCGGGTGCCAAGGGGCTAAAAGACCCTTGGA |
| SARS2-Probe I.453 | NC_045512.2 | SARS2-Probe I | CTGATGAGGGAGAAACATTAATACGACTCACTATAGGGGTGACACATTAAAAGAAATACTTGTCACATACAATTGTTGTGATGATGATTATTTCAATAAAAAGGACTGGTATGATTTTGTAGAAAACCTGGAATTCTCGGGTGCCAAGGGGCTAAAAGACCCTTGGA |
| SARS2-Probe I.454 | NC_045512.2 | SARS2-Probe I | CTGATGAGGGAGAAACATTAATACGACTCACTATAGGGGACAATTGTTGTGATGATGATTATTTCAATAAAAAGGACTGGTATGATTTTGTAGAAAACCCAGATATATTACGCGTATACGCCAACTTAGTGGAATTCTCGGGTGCCAAGGGGCTAAAAGACCCTTGGA |
| SARS2-Probe I.455 | NC_045512.2 | SARS2-Probe I | CTGATGAGGGAGAAACATTAATACGACTCACTATAGGGAAAAGGACTGGTATGATTTTGTAGAAAACCCAGATATATTACGCGTATACGCCAACTTAGGTGAACGTGTACGCCAAGCTTTGTTAAAAATGGAATTCTCGGGTGCCAAGGGGCTAAAAGACCCTTGGA |
| SARS2-Probe I.456 | NC_045512.2 | SARS2-Probe I | CTGATGAGGGAGAAACATTAATACGACTCACTATAGGGCAGATATATTACGCGTATACGCCAACTTAGGTGAACGTGTACGCCAAGCTTTGTTAAAAACAGTACAATTCTGTGATGCCATGCGAAATGTGGAATTCTCGGGTGCCAAGGGGCTAAAAGACCCTTGGA |
| SARS2-Probe I.457 | NC_045512.2 | SARS2-Probe I | CTGATGAGGGAGAAACATTAATACGACTCACTATAGGGGTGAACGTGTACGCCAAGCTTTGTTAAAAACAGTACAATTCTGTGATGCCATGCGAAATGCTGGTATTGTTGGTGTACTGACATTAGATATGGAATTCTCGGGTGCCAAGGGGCTAAAAGACCCTTGGA |
| SARS2-Probe I.458 | NC_045512.2 | SARS2-Probe I | CTGATGAGGGAGAAACATTAATACGACTCACTATAGGGCAGTACAATTCTGTGATGCCATGCGAAATGCTGGTATTGTTGGTGTACTGACATTAGATAATCAAGATCTCAATGGTAACTGGTATGATTTGGAATTCTCGGGTGCCAAGGGGCTAAAAGACCCTTGGA |
| SARS2-Probe I.459 | NC_045512.2 | SARS2-Probe I | CTGATGAGGGAGAAACATTAATACGACTCACTATAGGGCTGGTATTGTTGGTGTACTGACATTAGATAATCAAGATCTCAATGGTAACTGGTATGATTTCGGTGATTTCATACAAACCACGCCAGGTATGGAATTCTCGGGTGCCAAGGGGCTAAAAGACCCTTGGA |
| SARS2-Probe I.460 | NC_045512.2 | SARS2-Probe I | CTGATGAGGGAGAAACATTAATACGACTCACTATAGGGATCAAGATCTCAATGGTAACTGGTATGATTTCGGTGATTTCATACAAACCACGCCAGGTAGTGGAGTTCCTGTTGTAGATTCTTATTATTTGGAATTCTCGGGTGCCAAGGGGCTAAAAGACCCTTGGA |
| SARS2-Probe I.461 | NC_045512.2 | SARS2-Probe I | CTGATGAGGGAGAAACATTAATACGACTCACTATAGGGTCGGTGATTTCATACAAACCACGCCAGGTAGTGGAGTTCCTGTTGTAGATTCTTATTATTCATTGTTAATGCCTATATTAACCTTGACCATGGAATTCTCGGGTGCCAAGGGGCTAAAAGACCCTTGGA |
| SARS2-Probe I.462 | NC_045512.2 | SARS2-Probe I | CTGATGAGGGAGAAACATTAATACGACTCACTATAGGGGTGGAGTTCCTGTTGTAGATTCTTATTATTCATTGTTAATGCCTATATTAACCTTGACCAGGGCTTTAACTGCAGAGTCACATGTTGACATGGAATTCTCGGGTGCCAAGGGGCTAAAAGACCCTTGGA |
| SARS2-Probe I.463 | NC_045512.2 | SARS2-Probe I | CTGATGAGGGAGAAACATTAATACGACTCACTATAGGGCATTGTTAATGCCTATATTAACCTTGACCAGGGCTTTAACTGCAGAGTCACATGTTGACACTGACTTAACAAAGCCTTACATTAAGTGGGTGGAATTCTCGGGTGCCAAGGGGCTAAAAGACCCTTGGA |
| SARS2-Probe I.464 | NC_045512.2 | SARS2-Probe I | CTGATGAGGGAGAAACATTAATACGACTCACTATAGGGGGGCTTTAACTGCAGAGTCACATGTTGACACTGACTTAACAAAGCCTTACATTAAGTGGGATTTGTTAAAATATGACTTCACGGAAGAGATGGAATTCTCGGGTGCCAAGGGGCTAAAAGACCCTTGGA |
| SARS2-Probe I.465 | NC_045512.2 | SARS2-Probe I | CTGATGAGGGAGAAACATTAATACGACTCACTATAGGGCTGACTTAACAAAGCCTTACATTAAGTGGGATTTGTTAAAATATGACTTCACGGAAGAGAGGTTAAAACTCTTTGACCGTTATTTTAAATTGGAATTCTCGGGTGCCAAGGGGCTAAAAGACCCTTGGA |
| SARS2-Probe I.466 | NC_045512.2 | SARS2-Probe I | CTGATGAGGGAGAAACATTAATACGACTCACTATAGGGGATTTGTTAAAATATGACTTCACGGAAGAGAGGTTAAAACTCTTTGACCGTTATTTTAAATATTGGGATCAGACATACCACCCAAATTGTGTGGAATTCTCGGGTGCCAAGGGGCTAAAAGACCCTTGGA |
| SARS2-Probe I.467 | NC_045512.2 | SARS2-Probe I | CTGATGAGGGAGAAACATTAATACGACTCACTATAGGGGGGTTAAAACTCTTTGACCGTTATTTTAAATATTGGGATCAGACATACCACCCAAATTGTGTTAACTGTTTGGATGACAGATGCATTCTGCTGGAATTCTCGGGTGCCAAGGGGCTAAAAGACCCTTGGA |
| SARS2-Probe I.468 | NC_045512.2 | SARS2-Probe I | CTGATGAGGGAGAAACATTAATACGACTCACTATAGGGATTGGGATCAGACATACCACCCAAATTGTGTTAACTGTTTGGATGACAGATGCATTCTGCATTGTGCAAACTTTAATGTTTTATTCTCTATGGAATTCTCGGGTGCCAAGGGGCTAAAAGACCCTTGGA |
| SARS2-Probe I.469 | NC_045512.2 | SARS2-Probe I | CTGATGAGGGAGAAACATTAATACGACTCACTATAGGGTTAACTGTTTGGATGACAGATGCATTCTGCATTGTGCAAACTTTAATGTTTTATTCTCTACAGTGTTCCCACCTACAAGTTTTGGACCACTGGAATTCTCGGGTGCCAAGGGGCTAAAAGACCCTTGGA |
| SARS2-Probe I.470 | NC_045512.2 | SARS2-Probe I | CTGATGAGGGAGAAACATTAATACGACTCACTATAGGGATTGTGCAAACTTTAATGTTTTATTCTCTACAGTGTTCCCACCTACAAGTTTTGGACCACTAGTGAGAAAAATATTTGTTGATGGTGTTCTGGAATTCTCGGGTGCCAAGGGGCTAAAAGACCCTTGGA |
| SARS2-Probe I.471 | NC_045512.2 | SARS2-Probe I | CTGATGAGGGAGAAACATTAATACGACTCACTATAGGGCAGTGTTCCCACCTACAAGTTTTGGACCACTAGTGAGAAAAATATTTGTTGATGGTGTTCCATTTGTAGTTTCAACTGGATACCACTTCATGGAATTCTCGGGTGCCAAGGGGCTAAAAGACCCTTGGA |
| SARS2-Probe I.472 | NC_045512.2 | SARS2-Probe I | CTGATGAGGGAGAAACATTAATACGACTCACTATAGGGTAGTGAGAAAAATATTTGTTGATGGTGTTCCATTTGTAGTTTCAACTGGATACCACTTCAGAGAGCTAGGTGTTGTACATAATCAGGATGTGGAATTCTCGGGTGCCAAGGGGCTAAAAGACCCTTGGA |
| SARS2-Probe I.473 | NC_045512.2 | SARS2-Probe I | CTGATGAGGGAGAAACATTAATACGACTCACTATAGGGCATTTGTAGTTTCAACTGGATACCACTTCAGAGAGCTAGGTGTTGTACATAATCAGGATGTAAACTTACATAGCTCTAGACTTAGTTTTATGGAATTCTCGGGTGCCAAGGGGCTAAAAGACCCTTGGA |
| SARS2-Probe I.474 | NC_045512.2 | SARS2-Probe I | CTGATGAGGGAGAAACATTAATACGACTCACTATAGGGGAGAGCTAGGTGTTGTACATAATCAGGATGTAAACTTACATAGCTCTAGACTTAGTTTTAAGGAATTACTTGTGTATGCTGCTGACCCTGTGGAATTCTCGGGTGCCAAGGGGCTAAAAGACCCTTGGA |

| ASO ID | Accession | Group ID | Custom Array sequence |
|---|---|---|---|
| SARS2-Probe I.475 | NC_045512.2 | SARS2-Probe I | CTGATGAGGGAGAAACATTAATACGACTCACTATAGGGTAAACTTACATAGCTCTAGACTTAGTTTTAAGGAATTACTTGTGTATGCTGCTGACCCTGCTATGCACGCTGCTTCTGGTAATCTATTACTGGAATTCTCGGGTGCCAAGGGGCTAAAAGACCCTTGGA |
| SARS2-Probe I.476 | NC_045512.2 | SARS2-Probe I | CTGATGAGGGAGAAACATTAATACGACTCACTATAGGGAGGAATTACTTGTGTATGCTGCTGACCCTGCTATGCACGCTGCTTCTGGTAATCTATTACTAGATAAACGCACTACGTGCTTTTCAGTAGTGGAATTCTCGGGTGCCAAGGGGCTAAAAGACCCTTGGA |
| SARS2-Probe I.477 | NC_045512.2 | SARS2-Probe I | CTGATGAGGGAGAAACATTAATACGACTCACTATAGGGCTATGCACGCTGCTTCTGGTAATCTATTACTAGATAAACGCACTACGTGCTTTTCAGTAGCTGCACTTACTAACAATGTTGCTTTTCAAATGGAATTCTCGGGTGCCAAGGGGCTAAAAGACCCTTGGA |
| SARS2-Probe I.478 | NC_045512.2 | SARS2-Probe I | CTGATGAGGGAGAAACATTAATACGACTCACTATAGGGTAGATAAACGCACTACGTGCTTTTCAGTAGCTGCACTTACTAACAATGTTGCTTTTCAAACTGTCAAACCCGGTAATTTTAACAAAGACTTGGAATTCTCGGGTGCCAAGGGGCTAAAAGACCCTTGGA |
| SARS2-Probe I.479 | NC_045512.2 | SARS2-Probe I | CTGATGAGGGAGAAACATTAATACGACTCACTATAGGGCTGCACTTACTAACAATGTTGCTTTTCAAACTGTCAAACCCGGTAATTTTAACAAAGACTTCTATGACTTTGCTGTGTCTAAGGGTTTCTTGGAATTCTCGGGTGCCAAGGGGCTAAAAGACCCTTGGA |
| SARS2-Probe I.480 | NC_045512.2 | SARS2-Probe I | CTGATGAGGGAGAAACATTAATACGACTCACTATAGGGCTGTCAAACCCGGTAATTTTAACAAAGACTTCTATGACTTTGCTGTGTCTAAGGGTTTCTTAAGGAAGGAAGTTCTGTTGAATTAAAACTGGAATTCTCGGGTGCCAAGGGGCTAAAAGACCCTTGGA |
| SARS2-Probe I.481 | NC_045512.2 | SARS2-Probe I | CTGATGAGGGAGAAACATTAATACGACTCACTATAGGGTCTATGACTTTGCTGTGTCTAAGGGTTTCTTAAGGAAGGAAGTTCTGTTGAATTAAAACACTTCTTCTTTGCTCAGGATGGTAATGCTGTGTGGAATTCTCGGGTGCCAAGGGGCTAAAAGACCCTTGGA |
| SARS2-Probe I.482 | NC_045512.2 | SARS2-Probe I | CTGATGAGGGAGAAACATTAATACGACTCACTATAGGGTTAAGGAAGGAAGTTCTGTTGAATTAAAACACTTCTTCTTTGCTCAGGATGGTAATGCTGCTATCAGCGATTATGACTACTATCGTTATATGGAATTCTCGGGTGCCAAGGGGCTAAAAGACCCTTGGA |
| SARS2-Probe I.483 | NC_045512.2 | SARS2-Probe I | CTGATGAGGGAGAAACATTAATACGACTCACTATAGGGACTTCTTCTTTGCTCAGGATGGTAATGCTGCTATCAGCGATTATGACTACTATCGTTATAATCTACCAACAATGTGTGATATCAGACAACTGGAATTCTCGGGTGCCAAGGGGCTAAAAGACCCTTGGA |
| SARS2-Probe I.484 | NC_045512.2 | SARS2-Probe I | CTGATGAGGGAGAAACATTAATACGACTCACTATAGGGCTATCAGCGATTATGACTACTATCGTTATAATCTACCAACAATGTGTGATATCAGACAACTACTATTTGTAGTTGAAGTTGTTGATAAGTGGAATTCTCGGGTGCCAAGGGGCTAAAAGACCCTTGGA |
| SARS2-Probe I.485 | NC_045512.2 | SARS2-Probe I | CTGATGAGGGAGAAACATTAATACGACTCACTATAGGGATCTACCAACAATGTGTGATATCAGACAACTACTATTTGTAGTTGAAGTTGTTGATAAGTACTTTGATTGTTACGATGGTGGCTGTATTATGGAATTCTCGGGTGCCAAGGGGCTAAAAGACCCTTGGA |
| SARS2-Probe I.486 | NC_045512.2 | SARS2-Probe I | CTGATGAGGGAGAAACATTAATACGACTCACTATAGGGTACTATTTGTAGTTGAAGTTGTTGATAAGTACTTTGATTGTTACGATGGTGGCTGTATTAATGCTAACCAAGTCATCGTCAACAACCTAGTGGAATTCTCGGGTGCCAAGGGGCTAAAAGACCCTTGGA |
| SARS2-Probe I.487 | NC_045512.2 | SARS2-Probe I | CTGATGAGGGAGAAACATTAATACGACTCACTATAGGGACTTTGATTGTTACGATGGTGGCTGTATTAATGCTAACCAAGTCATCGTCAACAACCTAGACAAATCAGCTGGTTTTCCATTTAATAAATTGGAATTCTCGGGTGCCAAGGGGCTAAAAGACCCTTGGA |
| SARS2-Probe I.488 | NC_045512.2 | SARS2-Probe I | CTGATGAGGGAGAAACATTAATACGACTCACTATAGGGATGCTAACCAAGTCATCGTCAACAACCTAGACAAATCAGCTGGTTTTCCATTTAATAAATGGGGTAAGGCTAGACTTTATTATGATTCAATGGAATTCTCGGGTGCCAAGGGGCTAAAAGACCCTTGGA |
| SARS2-Probe I.489 | NC_045512.2 | SARS2-Probe I | CTGATGAGGGAGAAACATTAATACGACTCACTATAGGGACAAATCAGCTGGTTTTCCATTTAATAAATGGGGTAAGGCTAGACTTTATTATGATTCAATGAGTTATGAGGATCAAGATGCACTTTTCGTGGAATTCTCGGGTGCCAAGGGGCTAAAAGACCCTTGGA |
| SARS2-Probe I.490 | NC_045512.2 | SARS2-Probe I | CTGATGAGGGAGAAACATTAATACGACTCACTATAGGGGGGGTAAGGCTAGACTTTATTATGATTCAATGAGTTATGAGGATCAAGATGCACTTTTCGCATATACAAAACGTAATGTCATCCCTACTATGGAATTCTCGGGTGCCAAGGGGCTAAAAGACCCTTGGA |
| SARS2-Probe I.491 | NC_045512.2 | SARS2-Probe I | CTGATGAGGGAGAAACATTAATACGACTCACTATAGGGTGAGTTATGAGGATCAAGATGCACTTTTCGCATATACAAAACGTAATGTCATCCCTACTATAACTCAAATGAATCTTAAGTATGCCATTATGGAATTCTCGGGTGCCAAGGGGCTAAAAGACCCTTGGA |
| SARS2-Probe I.492 | NC_045512.2 | SARS2-Probe I | CTGATGAGGGAGAAACATTAATACGACTCACTATAGGGCATATACAAAACGTAATGTCATCCCTACTATAACTCAAATGAATCTTAAGTATGCCATTAGTGCAAAGAATAGAGCTCGCACCGTAGCTGTGTGGAATTCTCGGGTGCCAAGGGGCTAAAAGACCCTTGGA |
| SARS2-Probe I.493 | NC_045512.2 | SARS2-Probe I | CTGATGAGGGAGAAACATTAATACGACTCACTATAGGGTAACTCAAATGAATCTTAAGTATGCCATTAGTGCAAAGAATAGAGCTCGCACCGTAGCTGGTGTCTCTATCTGTAGTACTATGACCAATATGGAATTCTCGGGTGCCAAGGGGCTAAAAGACCCTTGGA |
| SARS2-Probe I.494 | NC_045512.2 | SARS2-Probe I | CTGATGAGGGAGAAACATTAATACGACTCACTATAGGGTGCAAAGAATAGAGCTCGCACCGTAGCTGGTGTCTCTATCTGTAGTACTATGACCAATAGACAGTTTCATCAAAAATTATTGAAATCAATGGAATTCTCGGGTGCCAAGGGGCTAAAAGACCCTTGGA |
| SARS2-Probe I.495 | NC_045512.2 | SARS2-Probe I | CTGATGAGGGAGAAACATTAATACGACTCACTATAGGGGTGTCTCTATCTGTAGTACTATGACCAATAGACAGTTTCATCAAAAATTATTGAAATCAATAGCCGCCACTAGAGGAGCTACTGTAGTAATGGAATTCTCGGGTGCCAAGGGGCTAAAAGACCCTTGGA |
| SARS2-Probe I.496 | NC_045512.2 | SARS2-Probe I | CTGATGAGGGAGAAACATTAATACGACTCACTATAGGGGACAGTTTCATCAAAAATTATTGAAATCAATAGCCGCCACTAGAGGAGCTACTGTAGTAATTGGAACAAGCAAATTCTATGGTGGTTGGCTGGAATTCTCGGGTGCCAAGGGGCTAAAAGACCCTTGGA |
| SARS2-Probe I.497 | NC_045512.2 | SARS2-Probe I | CTGATGAGGGAGAAACATTAATACGACTCACTATAGGGTAGCCGCCACTAGAGGAGCTACTGTAGTAATTGGAACAAGCAAATTCTATGGTGGTTGGCACAACATGTTAAAAACTGTTTATAGTGATGTGGAATTCTCGGGTGCCAAGGGGCTAAAAGACCCTTGGA |
| SARS2-Probe I.498 | NC_045512.2 | SARS2-Probe I | CTGATGAGGGAGAAACATTAATACGACTCACTATAGGGTTGGAACAAGCAAATTCTATGGTGGTTGGCACAACATGTTAAAAACTGTTTATAGTGATGTAGAAAACCCTCACCTTATGGGTTGGGATTTGGAATTCTCGGGTGCCAAGGGGCTAAAAGACCCTTGGA |
| SARS2-Probe I.499 | NC_045512.2 | SARS2-Probe I | CTGATGAGGGAGAAACATTAATACGACTCACTATAGGGACAACATGTTAAAAACTGTTTATAGTGATGTAGAAAACCCTCACCTTATGGGTTGGGATTATCCTAAATGTGATAGAGCCATGCCTAACATGGAATTCTCGGGTGCCAAGGGGCTAAAAGACCCTTGGA |

| ASO ID | Accession | Group ID | Custom Array sequence |
|---|---|---|---|
| SARS2-Probe I.500 | NC_045512.2 | SARS2-Probe I | CTGATGAGGGAGAAACATTAATACGACTCACTATAGGGTAGAAAACCCTCACCTTATGGGTTGGGATTATCCTAAATGTGATAGAGCCATGCCTAACATGCTTAGAATTATGGCCTCACTTGTTCTTGTGGAATTCTCGGGTGCCAAGGGGCTAAAAGACCCTTGGA |
| SARS2-Probe I.501 | NC_045512.2 | SARS2-Probe I | CTGATGAGGGAGAAACATTAATACGACTCACTATAGGGATCCTAAATGTGATAGAGCCATGCCTAACATGCTTAGAATTATGGCCTCACTTGTTCTTGCTCGCAAACATACAACGTGTTGTAGCTTGTTGGAATTCTCGGGTGCCAAGGGGCTAAAAGACCCTTGGA |
| SARS2-Probe I.502 | NC_045512.2 | SARS2-Probe I | CTGATGAGGGAGAAACATTAATACGACTCACTATAGGGTGCTTAGAATTATGGCCTCACTTGTTCTTGCTCGCAAACATACAACGTGTTGTAGCTTGTCACACCGTTTCTATAGATTAGCTAATGAGTTGGAATTCTCGGGTGCCAAGGGGCTAAAAGACCCTTGGA |
| SARS2-Probe I.503 | NC_045512.2 | SARS2-Probe I | CTGATGAGGGAGAAACATTAATACGACTCACTATAGGGCTCGCAAACATACAACGTGTTGTAGCTTGTCACACCGTTTCTATAGATTAGCTAATGAGTGTGCTCAAGTATTGAGTGAAATGGTCATGTTGGAATTCTCGGGTGCCAAGGGGCTAAAAGACCCTTGGA |
| SARS2-Probe I.504 | NC_045512.2 | SARS2-Probe I | CTGATGAGGGAGAAACATTAATACGACTCACTATAGGGCACACCGTTTCTATAGATTAGCTAATGAGTGTGCTCAAGTATTGAGTGAAATGGTCATGTGTGGCGGTTCACTATATGTTAAACCAGGTGTGGAATTCTCGGGTGCCAAGGGGCTAAAAGACCCTTGGA |
| SARS2-Probe I.505 | NC_045512.2 | SARS2-Probe I | CTGATGAGGGAGAAACATTAATACGACTCACTATAGGGTGCTCAAGTATTGAGTGAAATGGTCATGTGTGGCGGTTCACTATATGTTAAACCAGGTGGAACCTCATCAGGAGATGCCACAACTGCTTTGGAATTCTCGGGTGCCAAGGGGCTAAAAGACCCTTGGA |
| SARS2-Probe I.506 | NC_045512.2 | SARS2-Probe I | CTGATGAGGGAGAAACATTAATACGACTCACTATAGGGGTGGCGGTTCACTATATGTTAAACCAGGTGGAACCTCATCAGGAGATGCCACAACTGCTTATGCTAATAGTGTTTTTAACATTTGTCAAGTGGAATTCTCGGGTGCCAAGGGGCTAAAAGACCCTTGGA |
| SARS2-Probe I.507 | NC_045512.2 | SARS2-Probe I | CTGATGAGGGAGAAACATTAATACGACTCACTATAGGGGAACCTCATCAGGAGATGCCACAACTGCTTATGCTAATAGTGTTTTTAACATTTGTCAAGCTGTCACGGCCAATGTTAATGCACTTTTATTGGAATTCTCGGGTGCCAAGGGGCTAAAAGACCCTTGGA |
| SARS2-Probe I.508 | NC_045512.2 | SARS2-Probe I | CTGATGAGGGAGAAACATTAATACGACTCACTATAGGGATGCTAATAGTGTTTTTAACATTTGTCAAGCTGTCACGGCCAATGTTAATGCACTTTTATCTACTGATGGTAACAAAATTGCCGATAAGTTGGAATTCTCGGGTGCCAAGGGGCTAAAAGACCCTTGGA |
| SARS2-Probe I.509 | NC_045512.2 | SARS2-Probe I | CTGATGAGGGAGAAACATTAATACGACTCACTATAGGGCTGTCACGGCCAATGTTAATGCACTTTTATCTACTGATGGTAACAAAATTGCCGATAAGTATGTCCGCAATTTACAACACAGACTTTATGTGGAATTCTCGGGTGCCAAGGGGCTAAAAGACCCTTGGA |
| SARS2-Probe I.510 | NC_045512.2 | SARS2-Probe I | CTGATGAGGGAGAAACATTAATACGACTCACTATAGGGCTACTGATGGTAACAAAATTGCCGATAAGTATGTCCGCAATTTACAACACAGACTTTATGAGTGTCTCTATAGAAATAGAGATGTTGACATGGAATTCTCGGGTGCCAAGGGGCTAAAAGACCCTTGGA |
| SARS2-Probe I.511 | NC_045512.2 | SARS2-Probe I | CTGATGAGGGAGAAACATTAATACGACTCACTATAGGGATGTCCGCAATTTACAACACAGACTTTATGAGTGTCTCTATAGAAATAGAGATGTTGACACAGACTTTGTGAATGAGTTTTACGCATATTTGGAATTCTCGGGTGCCAAGGGGCTAAAAGACCCTTGGA |
| SARS2-Probe I.512 | NC_045512.2 | SARS2-Probe I | CTGATGAGGGAGAAACATTAATACGACTCACTATAGGGAGTGTCTCTATAGAAATAGAGATGTTGACACAGACTTTGTGAATGAGTTTTACGCATATTTGCGTAAACATTTCTCAATGATGATACTCTTGGAATTCTCGGGTGCCAAGGGGCTAAAAGACCCTTGGA |
| SARS2-Probe I.513 | NC_045512.2 | SARS2-Probe I | CTGATGAGGGAGAAACATTAATACGACTCACTATAGGGCAGACTTTGTGAATGAGTTTTACGCATATTTGCGTAAACATTTCTCAATGATGATACTCTCTGACGATGCTGTTGTGTGTTTCAATAGCATGGAATTCTCGGGTGCCAAGGGGCTAAAAGACCCTTGGA |
| SARS2-Probe I.514 | NC_045512.2 | SARS2-Probe I | CTGATGAGGGAGAAACATTAATACGACTCACTATAGGGTGCGTAAACATTTCTCAATGATGATACTCTCTGACGATGCTGTTGTGTGTTTCAATAGCACTTATGCATCTCAAGGTCTAGTGGCTAGCATGGAATTCTCGGGTGCCAAGGGGCTAAAAGACCCTTGGA |
| SARS2-Probe I.515 | NC_045512.2 | SARS2-Probe I | CTGATGAGGGAGAAACATTAATACGACTCACTATAGGGCTGACGATGCTGTTGTGTGTTTCAATAGCACTTATGCATCTCAAGGTCTAGTGGCTAGCATAAAGAACTTTAAGTCAGTTCTTTATTATCTGGAATTCTCGGGTGCCAAGGGGCTAAAAGACCCTTGGA |
| SARS2-Probe I.516 | NC_045512.2 | SARS2-Probe I | CTGATGAGGGAGAAACATTAATACGACTCACTATAGGGCTTATGCATCTCAAGGTCTAGTGGCTAGCATAAAGAACTTTAAGTCAGTTCTTTATTATCAAAACAATGTTTTTATGTCTGAAGCAAAATTGGAATTCTCGGGTGCCAAGGGGCTAAAAGACCCTTGGA |
| SARS2-Probe I.517 | NC_045512.2 | SARS2-Probe I | CTGATGAGGGAGAAACATTAATACGACTCACTATAGGGTAAAGAACTTTAAGTCAGTTCTTTATTATCAAAACAATGTTTTTATGTCTGAAGCAAATGTTGGACTGAGACTGACCTTACTAAAGGACTGGAATTCTCGGGTGCCAAGGGGCTAAAAGACCCTTGGA |
| SARS2-Probe I.518 | NC_045512.2 | SARS2-Probe I | CTGATGAGGGAGAAACATTAATACGACTCACTATAGGGAAAACAATGTTTTTATGTCTGAAGCAAATGTTGGACTGAGACTGACCTTACTAAAGGACCTCATGAATTTTGCTCTCAACATACAATGCTGGAATTCTCGGGTGCCAAGGGGCTAAAAGACCCTTGGA |
| SARS2-Probe I.519 | NC_045512.2 | SARS2-Probe I | CTGATGAGGGAGAAACATTAATACGACTCACTATAGGGGTTGGACTGAGACTGACCTTACTAAAGGACCTCATGAATTTTGCTCTCAACATACAATGCTAGTTAAACAGGGTGATGATTATGTGTACCTGGAATTCTCGGGTGCCAAGGGGCTAAAAGACCCTTGGA |
| SARS2-Probe I.520 | NC_045512.2 | SARS2-Probe I | CTGATGAGGGAGAAACATTAATACGACTCACTATAGGGCTCATGAATTTTGCTCTCAACATACAATGCTAGTTAAACAGGGTGATGATTATGTGTACCTTCCTTACCCAGATCCATCAAGAATCCTAGTGGAATTCTCGGGTGCCAAGGGGCTAAAAGACCCTTGGA |
| SARS2-Probe I.521 | NC_045512.2 | SARS2-Probe I | CTGATGAGGGAGAAACATTAATACGACTCACTATAGGGTAGTTAAACAGGGTGATGATTATGTGTACCTTCCTTACCCAGATCCATCAAGAATCCTAGGGGCCGGCTGTTTGTAGATGATATCGTAATGGAATTCTCGGGTGCCAAGGGGCTAAAAGACCCTTGGA |
| SARS2-Probe I.522 | NC_045512.2 | SARS2-Probe I | CTGATGAGGGAGAAACATTAATACGACTCACTATAGGGTTCCTTACCCAGATCCATCAAGAATCCTAGGGGCCGGCTGTTTGTAGATGATATCGTAAAAACAGATGGTACACTTATGATTGAACGGTTGGAATTCTCGGGTGCCAAGGGGCTAAAAGACCCTTGGA |
| SARS2-Probe I.523 | NC_045512.2 | SARS2-Probe I | CTGATGAGGGAGAAACATTAATACGACTCACTATAGGGGGGGCCGGCTGTTTGTAGATGATATCGTAAAAACAGATGGTACACTTATGATTGAACGGTTCGTGTCTTTAGCTATAGATGCTTACCCACTGGAATTCTCGGGTGCCAAGGGGCTAAAAGACCCTTGGA |
| SARS2-Probe I.524 | NC_045512.2 | SARS2-Probe I | CTGATGAGGGAGAAACATTAATACGACTCACTATAGGGAAACAGATGGTACACTTATGATTGAACGGTTCGTGTCTTTAGCTATAGATGCTTACCCACTTACTAAACATCCTAATCAGGAGTATGCTGTGGAATTCTCGGGTGCCAAGGGGCTAAAAGACCCTTGGA |

| ASO ID | Accession | Group ID | Custom Array sequence |
|---|---|---|---|
| SARS2: Probe 1525 | NC_045512.2 | SARS2: Probe I | CTGATGAGGGAGGGAGAAACATTAATACGACTCACTATAGGGTCGTGTCTTTAGCTATAGATGTCTACCCACTTACTAAACATCCTAATCAGGATATGCTGATGTCTTCATTTGTACTACAATACATACATAAGAAAGCTACATGAGTTAACACATAAGACACATGGAATTCTCGGGTGCCAAGGGGCTAAAAGACCCTTGGA |
| SARS2: Probe 1526 | NC_045512.2 | SARS2: Probe I | CTGATGAGGGAGGGAGAAACATTAATACGACTCACTATAGGGTTACTAACATCCTAATCAGGATATGCTGATGTCTTCATTTGTACTACAATACATACATAAGAAAGCTACATGAGTTAACAGGACACATGGAATTCTCGGGTGCCAAGGGGCTAAAAGACCCTTGGA |
| SARS2: Probe 1527 | NC_045512.2 | SARS2: Probe I | CTGATGAGGGAGGGAGAAACATTAATACGACTCACTATAGGGATGTCTTCATTTGTACTACAATACATAAGAAAGCTACATGAGTTAGAACGTTATGCTTAGAACATGTTAGAACGTGATTATGTATTCTGTGTTAATGCTCTGGAACCTGTAGGGAACTGTAAAAGACCCTTGGA |
| SARS2: Probe 1528 | NC_045512.2 | SARS2: Probe I | CTGATGAGGGAGGGAGAAACATTAATACGACTCACTATAGGGTGTTAGACACTTAGAACATGTTAGAACGTGATTATGTATTCTGTGTTAATGCTCTGGAACCTGTAGGGAACTGTAAAAGACCCTTGGA |
| SARS2: Probe 1529 | NC_045512.2 | SARS2: Probe I | CTGATGAGGGAGGGAGAAACATTAATACGACTCACTATAGGGTGTTAGACATGTTAGTATTCTGTGTTAATGATAAACAACTTAGATGGGGACCTGTAACTTATGAGAAGTTTGTCTGGGGAACCTGTAGGGAACTGTAAAAGACCCTTGGA |
| SARS2: Probe 1530 | NC_045512.2 | SARS2: Probe I | CTGATGAGGGAGGGAGAAACATTAATACGACTCACTATAGGGATTGGGGAACCTGAGTTTATGGGGCTATGTGTTCTGGGGGTTGTTCTGGGGCTATGTTGGGGCTATGTGTTCTGGGGCTATGAGTTCACAGGCTGGGGCTAAAAGACCCTTGGA |
| SARS2: Probe 1531 | NC_045512.2 | SARS2: Probe I | CTGATGAGGGAGGGAGAAACATTAATACGACTCACTATAGGGATTACACTTACAGGCTATGTGACACCGCATACAGTTCTTACAGGCTGTTGTGTTCTTTGCAATTCACAGGCTGTTGGGGCTAAGGGCTAAAAGACCCTTGGA |
| SARS2: Probe 1532 | NC_045512.2 | SARS2: Probe I | CTGATGAGGGAGGGAGAAACATTAATACGACTCACTATAGGGTTGTGTTCTTTGCAATTCACAGACTTCTTATGTTTGCATACGTGTTACGAACCCATCTTATGTTTGCAATGTTTGGGGCTGCCAAGGGGCTAAAAGACCCTTGGA |
| SARS2: Probe 1533 | NC_045512.2 | SARS2: Probe I | CTGATGAGGGAGGGAGAAACATTAATACGACTCACTATAGGGTTTGCATACGTGTTACGAACTCTTATGTGTAAATGCTCATTTGTAAATGCTGTTAAATGCTCATGTTGTCTGTCTTATGGTGCCAAGGGGCTAAAAGACCCTTGGA |
| SARS2: Probe 1534 | NC_045512.2 | SARS2: Probe I | CTGATGAGGGAGGGAGAAACATTAATACGACTCACTATAGGGTGCTTGCATACGTGTTGTAAATGCTCATATCAACATCACATAAATTAGTCTTGTCTGTTAATCTGATGTTGTGATGTGCCAAGGGGCTAAAAGACCCTTGGA |
| SARS2: Probe 1535 | NC_045512.2 | SARS2: Probe I | CTGATGAGGGAGGGAGAAACATTAATACGACTCACTATAGGGTCTTATGTTAAATGCTCATATCAACATCACATAAATTAGTCTTGTCTGTTAATCCGATATGTCGTGTTAATCCGATGTGCCAAGGGGCTAAAAGACCCTTGGA |
| SARS2: Probe 1536 | NC_045512.2 | SARS2: Probe I | CTGATGAGGGAGGGAGAAACATTAATACGACTCACTATAGGGCATCACATAAATTAGTCTTGTCTGTTAATCCGATATGTTTGCAATGTTTGCAATGTCACAGATGTGATGTGCCAAGGGGCTAAAAGACCCTTGGA |
| SARS2: Probe 1537 | NC_045512.2 | SARS2: Probe I | CTGATGAGGGAGGGAGAAACATTAATACGACTCACTATAGGGTGTCTGTTAATCCGATATGTTTGCAATGTTTACTTACTAGGAGTGGAATTCTCGGGTGTCACAGATGTGATGCTATTATTCGGGTGCCAAGGGGCTAAAAGACCCTTGGA |
| SARS2: Probe 1538 | NC_045512.2 | SARS2: Probe I | CTGATGAGGGAGGGAGAAACATTAATACGACTCACTATAGGGTCACAGATGTGATGCTATTATTCGGGTGACTCAACTTTACTTATTATTCGGGTGACTCAACTTTATTATTATCACATAAACTCACATAACCACCCATTAGTTTCATTGTGCTAATGTGCCAAGGGGCTAAAAGACCCTTGGA |
| SARS2: Probe 1539 | NC_045512.2 | SARS2: Probe I | CTGATGAGGGAGGGAGAAACATTAATACGACTCACTATAGGGTATGGGGCTATTATTCGGGTGACTCAACTTTATTATTATCACATAAACTCACATAACCACCCATTAGTTTCATTGTGCTAATGTTTATAAAAATACATTGGAATTCTCGGGTGCCAAGGGGCTAAAAGACCCTTGGA |
| SARS2: Probe 1540 | NC_045512.2 | SARS2: Probe I | CTGATGAGGGAGGGAGAAACATTAATACGACTCACTATAGGGTGACTCAACTTTATTAAAAATACATTATTATAAAAATACATTGTTTATAAAAATACATTGGTTATATAAAATACATTGTTGGTAGCGATAATGTTTATGTGCCAAGGGGCTAAAAGACCCTTGGA |
| SARS2: Probe 1541 | NC_045512.2 | SARS2: Probe I | CTGATGAGGGAGGGAGAAACATTAATACGACTCACTATAGGGTTTTTGGTTTATAAAAATACATGTTTAATGCAATTGCAACAATGTGACAATGTGACTTAATGTTACTGACCGATAATGTGCTAGCGATAATGTGCCAAGGGGCTAAAAGACCCTTGGA |
| SARS2: Probe 1542 | NC_045512.2 | SARS2: Probe I | CTGATGAGGGAGGGAGAAACATTAATACGACTCACTATAGGGTGTTGGTAGCGATAATGTTACTGACTTTAATGCAATTGCAACAATGTTTAATGCAATTGCAACAATCATTTAGCTGACAATATGTGCCAAGGGGCTAAAAGACCCTTGGA |
| SARS2: Probe 1543 | NC_045512.2 | SARS2: Probe I | CTGATGAGGGAGGGAGAAACATTAATACGACTCACTATAGGGATGCAATTGCAACAATCATTTAGCTGACACCTGTGACTAACACCTGTTAGCTAACACCTGTTAGCTAACACCTGTGACTAACACCTAGTCGTGTGCCAAGGGGCTAAAAGACCCTTGGA |
| SARS2: Probe 1544 | NC_045512.2 | SARS2: Probe I | CTGATGAGGGAGGGAGAAACATTAATACGACTCACTATAGGGATGTGACTAACACCTGTGGTGGTTTTTGCAGCAGAAACGCTCAAGCTTTTTGCAGCAGAAACGCTCAAGCTACTGAGGAGACTCAAGTGCCAAGGGGCTAAAAGACCCTTGGA |
| SARS2: Probe 1545 | NC_045512.2 | SARS2: Probe I | CTGATGAGGGAGGGAGAAACATTAATACGACTCACTATAGGGTGGTTTTTGCAGCAGAAACGCTCAAGCTACTGAGGAGACTCAAGTGCTACTGTACGTGAAGTGCTCTTATGGTGCTACTGTACGTGAAGTGCCAAGGGGCTAAAAGACCCTTGGA |
| SARS2: Probe 1546 | NC_045512.2 | SARS2: Probe I | CTGATGAGGGAGGGAGAAACATTAATACGACTCACTATAGGGTGCTACTGAGGAGACTCAAGTGCTTATGGTGCTACTGTACGTGAAGTGCTCATTTCATGGAAGTTGGTGTCGTGTCTGACAGAGAATATGTGCCAAGGGGCTAAAAGACCCTTGGA |
| SARS2: Probe 1547 | NC_045512.2 | SARS2: Probe I | CTGATGAGGGAGGGAGAAACATTAATACGACTCACTATAGGGTCTTATGGTGCTACTGTACGTGAAGTGCTGAAGTGCGAAATTAGTCTTTCATGGGAAGTTGGTGTCGTGTCTGACAGAGAATACATCTTTCATGTGCCAAGGGGCTAAAAGACCCTTGGA |
| SARS2: Probe 1548 | NC_045512.2 | SARS2: Probe I | CTGATGAGGGAGGGAGAAACATTAATACGACTCACTATAGGGTGGTGTCGTGTCTGACAGAGAATATGTGTACTACTGAGAAGTGCTACTGTACGTGAAGTGCTCAGAGAATACATCTTTACTGTGTCTTTACTGTGTCTGGTTATCGTGTGCCAAGGGGCTAAAAGACCCTTGGA |
| SARS2: Probe 1549 | NC_045512.2 | SARS2: Probe I | CTGATGAGGGAGGGGGAGAAACATTAATACGACTCACTATAGGGGGGGAAGTTGGTGTCGTGTCTGACAGAGAATACATCTTTACTGTGTCTGGTTATCGTGTGTAACTAGAAACCTAGTCGTGTAACTAGAAACCAGTAGAGTGAAATTATGTCTTTACTGTGTGTATCGTGTGCCAAGGGGCTAAAAGACCCTTGGA |

| ASO ID | Accession | Group ID | Custom Array sequence |
|---|---|---|---|
| SARS2-Probe I.550 | NC_045512.2 | SARS2-Probe I | CTGATGAGGGAGAAACATTAATACGACTCACTATAGGGACCGAAATTATGTCTTTACTGGTTATCGTGTAACTAAAAACAGTAAAGTACAAATAGGAGAGTACACCTTTGAAAAAGGTGACTATGGTGTGGAATTCTCGGGTGCCAAGGGGCTAAAAGACCCTTGGA |
| SARS2-Probe I.551 | NC_045512.2 | SARS2-Probe I | CTGATGAGGGAGAAACATTAATACGACTCACTATAGGGTAACTAAAAACAGTAAAGTACAAATAGGAGAGTACACCTTTGAAAAAGGTGACTATGGTGATGCTGTTGTTTACCGAGGTACAACAACTTTGGAATTCTCGGGTGCCAAGGGGCTAAAAGACCCTTGGA |
| SARS2-Probe I.552 | NC_045512.2 | SARS2-Probe I | CTGATGAGGGAGAAACATTAATACGACTCACTATAGGGAGTACACCTTTGAAAAAGGTGACTATGGTGATGCTGTTGTTTACCGAGGTACAACAACTTACAAATTAAATGTTGGTGATTATTTTGTGCTGGAATTCTCGGGTGCCAAGGGGCTAAAAGACCCTTGGA |
| SARS2-Probe I.553 | NC_045512.2 | SARS2-Probe I | CTGATGAGGGAGAAACATTAATACGACTCACTATAGGGATGCTGTTGTTTACCGAGGTACAACAACTTACAAATTAAATGTTGGTGATTATTTTGTGCTGACATCACATACAGTAATGCCATTAAGTGTGGAATTCTCGGGTGCCAAGGGGCTAAAAGACCCTTGGA |
| SARS2-Probe I.554 | NC_045512.2 | SARS2-Probe I | CTGATGAGGGAGAAACATTAATACGACTCACTATAGGGACAAATTAAATGTTGGTGATTATTTTGTGCTGACATCACATACAGTAATGCCATTAAGTGCACCTACACTAGTGCCACAAGAGCACTATGTGGAATTCTCGGGTGCCAAGGGGCTAAAAGACCCTTGGA |
| SARS2-Probe I.555 | NC_045512.2 | SARS2-Probe I | CTGATGAGGGAGAAACATTAATACGACTCACTATAGGGTGACATCACATACAGTAATGCCATTAAGTGCACCTACACTAGTGCCACAAGAGCACTATGTTAGAATTACTGGCTTATACCCAACACTCATGGAATTCTCGGGTGCCAAGGGGCTAAAAGACCCTTGGA |
| SARS2-Probe I.556 | NC_045512.2 | SARS2-Probe I | CTGATGAGGGAGAAACATTAATACGACTCACTATAGGGCACCTACACTAGTGCCACAAGAGCACTATGTTAGAATTACTGGCTTATACCCAACACTCAATATCTCAGATGAGTTTTCTAGCAATGTTGTGGAATTCTCGGGTGCCAAGGGGCTAAAAGACCCTTGGA |
| SARS2-Probe I.557 | NC_045512.2 | SARS2-Probe I | CTGATGAGGGAGAAACATTAATACGACTCACTATAGGGTTAGAATTACTGGCTTATACCCAACACTCAATATCTCAGATGAGTTTTCTAGCAATGTTGCAAATTATCAAAAGGTTGGTATGCAAAAGTTGGAATTCTCGGGTGCCAAGGGGCTAAAAGACCCTTGGA |
| SARS2-Probe I.558 | NC_045512.2 | SARS2-Probe I | CTGATGAGGGAGAAACATTAATACGACTCACTATAGGGATATCTCAGATGAGTTTTCTAGCAATGTTGCAAATTATCAAAAGGTTGGTATGCAAAAGTATTCTACACTCCAGGGACCACCTGGTACTGTGGAATTCTCGGGTGCCAAGGGGCTAAAAGACCCTTGGA |
| SARS2-Probe I.559 | NC_045512.2 | SARS2-Probe I | CTGATGAGGGAGAAACATTAATACGACTCACTATAGGGCAAATTATCAAAAGGTTGGTATGCAAAAGTATTCTACACTCCAGGGACCACCTGGTACTGGTAAGAGTCATTTTGCTATTGGCCTAGCTCTGGAATTCTCGGGTGCCAAGGGGCTAAAAGACCCTTGGA |
| SARS2-Probe I.560 | NC_045512.2 | SARS2-Probe I | CTGATGAGGGAGAAACATTAATACGACTCACTATAGGGATTCTACACTCCAGGGACCACCTGGTACTGGTAAGAGTCATTTTGCTATTGGCCTAGCTCTCTACTACCCTTCTGCTCGCATAGTGTATATGGAATTCTCGGGTGCCAAGGGGCTAAAAGACCCTTGGA |
| SARS2-Probe I.561 | NC_045512.2 | SARS2-Probe I | CTGATGAGGGAGAAACATTAATACGACTCACTATAGGGGTAAGAGTCATTTTGCTATTGGCCTAGCTCTCTACTACCCTTCTGCTCGCATAGTGTATACAGCTTGCTCTCATGCCGCTGTTGATGCACTGGAATTCTCGGGTGCCAAGGGGCTAAAAGACCCTTGGA |
| SARS2-Probe I.562 | NC_045512.2 | SARS2-Probe I | CTGATGAGGGAGAAACATTAATACGACTCACTATAGGGTCTACTACCCTTCTGCTCGCATAGTGTATACAGCTTGCTCTCATGCCGCTGTTGATGCACTATGTGAGAAGGCATTAAAATATTTGCCTATGGAATTCTCGGGTGCCAAGGGGCTAAAAGACCCTTGGA |
| SARS2-Probe I.563 | NC_045512.2 | SARS2-Probe I | CTGATGAGGGAGAAACATTAATACGACTCACTATAGGGCAGCTTGCTCTCATGCCGCTGTTGATGCACTATGTGAGAAGGCATTAAAATATTTGCCTATAGATAAATGTAGTAGAATTATACCTGCACTGGAATTCTCGGGTGCCAAGGGGCTAAAAGACCCTTGGA |
| SARS2-Probe I.564 | NC_045512.2 | SARS2-Probe I | CTGATGAGGGAGAAACATTAATACGACTCACTATAGGGTATGTGAGAAGGCATTAAAATATTTGCCTATAGATAAATGTAGTAGAATTATACCTGCACGTGCTCGTGTAGAGTGTTTTGATAAATTCATGGAATTCTCGGGTGCCAAGGGGCTAAAAGACCCTTGGA |
| SARS2-Probe I.565 | NC_045512.2 | SARS2-Probe I | CTGATGAGGGAGAAACATTAATACGACTCACTATAGGGTAGATAAATGTAGTAGAATTATACCTGCACGTGCTCGTGTAGAGTGTTTTGATAAATTCAAAGTGAATTCAACATTAGAACAGTATGTCTTGGAATTCTCGGGTGCCAAGGGGCTAAAAGACCCTTGGA |
| SARS2-Probe I.566 | NC_045512.2 | SARS2-Probe I | CTGATGAGGGAGAAACATTAATACGACTCACTATAGGGTGCTCGTGTAGAGTGTTTTGATAAATTCAAAGTGAATTCAACATTAGAACAGTATGTCTTTGTACTGTAAATGCATTGCCTGAGACGATGGAATTCTCGGGTGCCAAGGGGCTAAAAGACCCTTGGA |
| SARS2-Probe I.567 | NC_045512.2 | SARS2-Probe I | CTGATGAGGGAGAAACATTAATACGACTCACTATAGGGAAGTGAATTCAACATTAGAACAGTATGTCTTTGTACTGTAAATGCATTGCCTGAGACGACAGCAGATATAGTTGTCTTTGATGAAATTTTGGAATTCTCGGGTGCCAAGGGGCTAAAAGACCCTTGGA |
| SARS2-Probe I.568 | NC_045512.2 | SARS2-Probe I | CTGATGAGGGAGAAACATTAATACGACTCACTATAGGGTTTGTACTGTAAATGCATTGCCTGAGACGACAGCAGATATAGTTGTCTTTGATGAAATTTCAATGGCCACAAATTATGATTTGAGTGTTGTGGAATTCTCGGGTGCCAAGGGGCTAAAAGACCCTTGGA |
| SARS2-Probe I.569 | NC_045512.2 | SARS2-Probe I | CTGATGAGGGAGAAACATTAATACGACTCACTATAGGGCAGCAGATATAGTTGTCTTTGATGAAATTTCAATGGCCACAAATTATGATTTGAGTGTTGTCAATGCCAGATTACGTGCTAAGCACTATGTGGAATTCTCGGGTGCCAAGGGGCTAAAAGACCCTTGGA |
| SARS2-Probe I.570 | NC_045512.2 | SARS2-Probe I | CTGATGAGGGAGAAACATTAATACGACTCACTATAGGGCAATGGCCACAAATTATGATTTGAGTGTTGTCAATGCCAGATTACGTGCTAAGCACTATGTGTACATTGGCGACCCTGCTCAATTACCTGTGGAATTCTCGGGTGCCAAGGGGCTAAAAGACCCTTGGA |
| SARS2-Probe I.571 | NC_045512.2 | SARS2-Probe I | CTGATGAGGGAGAAACATTAATACGACTCACTATAGGGTCAATGCCAGATTACGTGCTAAGCACTATGTGTACATTGGCGACCCTGCTCAATTACCTGCACCACGCACATTGCTAACTAAGGGCACACTGGAATTCTCGGGTGCCAAGGGGCTAAAAGACCCTTGGA |
| SARS2-Probe I.572 | NC_045512.2 | SARS2-Probe I | CTGATGAGGGAGAAACATTAATACGACTCACTATAGGGTGTACATTGGCGACCCTGCTCAATTACCTGCACCACGCACATTGCTAACTAAGGGCACACTAGAACCAGAATATTTCAATTCAGTGTGTATGGAATTCTCGGGTGCCAAGGGGCTAAAAGACCCTTGGA |
| SARS2-Probe I.573 | NC_045512.2 | SARS2-Probe I | CTGATGAGGGAGAAACATTAATACGACTCACTATAGGGCACCACGCACATTGCTAACTAAGGGCACACTAGAACCAGAATATTTCAATTCAGTGTGTAGACTTATGAAAACTATAGGTCCAGACATGTTGGAATTCTCGGGTGCCAAGGGGCTAAAAGACCCTTGGA |
| SARS2-Probe I.574 | NC_045512.2 | SARS2-Probe I | CTGATGAGGGAGAAACATTAATACGACTCACTATAGGGTAGAACCAGAATATTTCAATTCAGTGTGTAGACTTATGAAAACTATAGGTCCAGACATGTTCCTCGGAACTTGTCGGCGTTGTCCTGCTGTGGAATTCTCGGGTGCCAAGGGGCTAAAAGACCCTTGGA |

| ASO ID | Accession | Group ID | Custom Array sequence |
|---|---|---|---|
| SARS2-Probe I.575 | NC_045512.2 | SARS2-Probe I | CTGATGAGGGAGAAACATTAATACGACTCACTATAGGGGACTTATGAAAACTATAGGTCCAGACATGTTCCTCGGAACTTGTCGGCGTTGTCCTGCTGAAATTGTTGACACTGTGAGTGCTTTGGTTTTGGAATTCTCGGGTGCCAAGGGGCTAAAAGACCCTTGGA |
| SARS2-Probe I.576 | NC_045512.2 | SARS2-Probe I | CTGATGAGGGAGAAACATTAATACGACTCACTATAGGGTCCTCGGAACTTGTCGGCGTTGTCCTGCTGAAATTGTTGACACTGTGAGTGCTTTGGTTTATGATAATAAGCTTAAAGCACATAAAGACATGGAATTCTCGGGTGCCAAGGGGCTAAAAGACCCTTGGA |
| SARS2-Probe I.577 | NC_045512.2 | SARS2-Probe I | CTGATGAGGGAGAAACATTAATACGACTCACTATAGGGAAATTGTTGACACTGTGAGTGCTTTGGTTTATGATAATAAGCTTAAAGCACATAAAGACAAATCAGCTCAATGCTTTAAAATGTTTTATATGGAATTCTCGGGTGCCAAGGGGCTAAAAGACCCTTGGA |
| SARS2-Probe I.578 | NC_045512.2 | SARS2-Probe I | CTGATGAGGGAGAAACATTAATACGACTCACTATAGGGATGATAATAAGCTTAAAGCACATAAAGACAAATCAGCTCAATGCTTTAAAATGTTTTATAAGGGTGTTATCACGCATGATGTTTCATCTGTGGAATTCTCGGGTGCCAAGGGGCTAAAAGACCCTTGGA |
| SARS2-Probe I.579 | NC_045512.2 | SARS2-Probe I | CTGATGAGGGAGAAACATTAATACGACTCACTATAGGGAATCAGCTCAATGCTTTAAAATGTTTTATAAGGGTGTTATCACGCATGATGTTTCATCTGCAATTAACAGGCCACAAATAGGCGTGGTAATGGAATTCTCGGGTGCCAAGGGGCTAAAAGACCCTTGGA |
| SARS2-Probe I.580 | NC_045512.2 | SARS2-Probe I | CTGATGAGGGAGAAACATTAATACGACTCACTATAGGGAGGGTGTTATCACGCATGATGTTTCATCTGCAATTAACAGGCCACAAATAGGCGTGGTAAGAGAATTCCTTACACGTAACCCTGCTTGGATGGAATTCTCGGGTGCCAAGGGGCTAAAAGACCCTTGGA |
| SARS2-Probe I.581 | NC_045512.2 | SARS2-Probe I | CTGATGAGGGAGAAACATTAATACGACTCACTATAGGGCAATTAACAGGCCACAAATAGGCGTGGTAAGAGAATTCCTTACACGTAACCCTGCTTGGAGAAAAGCTGTCTTTATTTCACCTTATAATTTGGAATTCTCGGGTGCCAAGGGGCTAAAAGACCCTTGGA |
| SARS2-Probe I.582 | NC_045512.2 | SARS2-Probe I | CTGATGAGGGAGAAACATTAATACGACTCACTATAGGGGGAGAATTCCTTACACGTAACCCTGCTTGGAGAAAAGCTGTCTTTATTTCACCTTATAATTCACAGAATGCTGTAGCCTCAAAGATTTTGGTGGAATTCTCGGGTGCCAAGGGGCTAAAAGACCCTTGGA |
| SARS2-Probe I.583 | NC_045512.2 | SARS2-Probe I | CTGATGAGGGAGAAACATTAATACGACTCACTATAGGGGAAAAGCTGTCTTTATTTCACCTTATAATTCACAGAATGCTGTAGCCTCAAAGATTTTGGGACTACCAACTCAAACTGTTGATTCATCACTGGAATTCTCGGGTGCCAAGGGGCTAAAAGACCCTTGGA |
| SARS2-Probe I.584 | NC_045512.2 | SARS2-Probe I | CTGATGAGGGAGAAACATTAATACGACTCACTATAGGGCACAGAATGCTGTAGCCTCAAAGATTTTGGGACTACCAACTCAAACTGTTGATTCATCACAGGGCTCAGAATATGACTATGTCATATTCATGGAATTCTCGGGTGCCAAGGGGCTAAAAGACCCTTGGA |
| SARS2-Probe I.585 | NC_045512.2 | SARS2-Probe I | CTGATGAGGGAGAAACATTAATACGACTCACTATAGGGGACTACCAACTCAAACTGTTGATTCATCACAGGGCTCAGAATATGACTATGTCATATTCACTCAAACCACTGAAACAGCTCACTCTTGTATGGAATTCTCGGGTGCCAAGGGGCTAAAAGACCCTTGGA |
| SARS2-Probe I.586 | NC_045512.2 | SARS2-Probe I | CTGATGAGGGAGAAACATTAATACGACTCACTATAGGGAGGGCTCAGAATATGACTATGTCATATTCACTCAAACCACTGAAACAGCTCACTCTTGTAATGTAAACAGATTTAATGTTGCTATTACCATGGAATTCTCGGGTGCCAAGGGGCTAAAAGACCCTTGGA |
| SARS2-Probe I.587 | NC_045512.2 | SARS2-Probe I | CTGATGAGGGAGAAACATTAATACGACTCACTATAGGGCTCAAACCACTGAAACAGCTCACTCTTGTAATGTAAACAGATTTAATGTTGCTATTACCAGAGCAAAAGTAGGCCATACTTTGCATAATGTTGGAATTCTCGGGTGCCAAGGGGCTAAAAGACCCTTGGA |
| SARS2-Probe I.588 | NC_045512.2 | SARS2-Probe I | CTGATGAGGGAGAAACATTAATACGACTCACTATAGGGATGTAAACAGATTTAATGTTGCTATTACCAGAGCAAAAGTAGGCCATACTTTGCATAATGTCTGATAGAGACCTTTATGACAAGTTGCAATTGGAATTCTCGGGTGCCAAGGGGCTAAAAGACCCTTGGA |
| SARS2-Probe I.589 | NC_045512.2 | SARS2-Probe I | CTGATGAGGGAGAAACATTAATACGACTCACTATAGGGGAGCAAAAGTAGGCCATACTTTGCATAATGTCTGATAGAGACCTTTATGACAAGTTGCAATTTACAAGTCTTGAAATTCCACGTAGGAATGTGGAATTCTCGGGTGCCAAGGGGCTAAAAGACCCTTGGA |
| SARS2-Probe I.590 | NC_045512.2 | SARS2-Probe I | CTGATGAGGGAGAAACATTAATACGACTCACTATAGGGCTGATAGAGACCTTTATGACAAGTTGCAATTTACAAGTCTTGAAATTCCACGTAGGAATGTGGCAACTTTACAAGCTGAAAATGTAACAGTGGAATTCTCGGGTGCCAAGGGGCTAAAAGACCCTTGGA |
| SARS2-Probe I.591 | NC_045512.2 | SARS2-Probe I | CTGATGAGGGAGAAACATTAATACGACTCACTATAGGGTTACAAGTCTTGAAATTCCACGTAGGAATGTGGCAACTTTACAAGCTGAAAATGTAACAGGACTCTTTAAAGATTGTAGTAAGGTAATCATGGAATTCTCGGGTGCCAAGGGGCTAAAAGACCCTTGGA |
| SARS2-Probe I.592 | NC_045512.2 | SARS2-Probe I | CTGATGAGGGAGAAACATTAATACGACTCACTATAGGGTGGCAACTTTACAAGCTGAAAATGTAACAGGACTCTTTAAAGATTGTAGTAAGGTAATCACTGGGTTACATCCTACACAGGCACCTACACTGGAATTCTCGGGTGCCAAGGGGCTAAAAGACCCTTGGA |
| SARS2-Probe I.593 | NC_045512.2 | SARS2-Probe I | CTGATGAGGGAGAAACATTAATACGACTCACTATAGGGGACTCTTTAAAGATTGTAGTAAGGTAATCACTGGGTTACATCCTACACAGGCACCTACACACCTCAGTGTTGACACTAAATTCAAAACTGTGGAATTCTCGGGTGCCAAGGGGCTAAAAGACCCTTGGA |
| SARS2-Probe I.594 | NC_045512.2 | SARS2-Probe I | CTGATGAGGGAGAAACATTAATACGACTCACTATAGGGCTGGGTTACATCCTACACAGGCACCTACACACCTCAGTGTTGACACTAAATTCAAAACTGAAGGTTTATGTGTTGACATACCTGGCATACTGGAATTCTCGGGTGCCAAGGGGCTAAAAGACCCTTGGA |
| SARS2-Probe I.595 | NC_045512.2 | SARS2-Probe I | CTGATGAGGGAGAAACATTAATACGACTCACTATAGGGACCTCAGTGTTGACACTAAATTCAAAACTGAAGGTTTATGTGTTGACATACCTGGCATACCTAAGGACATGACCTATAGAAGACTCATCTTGGAATTCTCGGGTGCCAAGGGGCTAAAAGACCCTTGGA |
| SARS2-Probe I.596 | NC_045512.2 | SARS2-Probe I | CTGATGAGGGAGAAACATTAATACGACTCACTATAGGGAAGGTTTATGTGTTGACATACCTGGCATACCTAAGGACATGACCTATAGAAGACTCATCTCTATGATGGGTTTTAAAATGAATTATCAAGTGGAATTCTCGGGTGCCAAGGGGCTAAAAGACCCTTGGA |
| SARS2-Probe I.597 | NC_045512.2 | SARS2-Probe I | CTGATGAGGGAGAAACATTAATACGACTCACTATAGGGCTAAGGACATGACCTATAGAAGACTCATCTCTATGATGGGTTTTAAAATGAATTATCAAGTTAATGGTTACCCTAACATGTTTATCACCCTGGAATTCTCGGGTGCCAAGGGGCTAAAAGACCCTTGGA |
| SARS2-Probe I.598 | NC_045512.2 | SARS2-Probe I | CTGATGAGGGAGAAACATTAATACGACTCACTATAGGGCTATGATGGGTTTTAAAATGAATTATCAAGTTAATGGTTACCCTAACATGTTTATCACCCGCGAAGAAGCTATAAGACATGTACGTGCATTGGAATTCTCGGGTGCCAAGGGGCTAAAAGACCCTTGGA |
| SARS2-Probe I.599 | NC_045512.2 | SARS2-Probe I | CTGATGAGGGAGAAACATTAATACGACTCACTATAGGGTTAATGGTTACCCTAACATGTTTATCACCCGCGAAGAAGCTATAAGACATGTACGTGCATGGATTGGCTTCGATGTCGAGGGGTGTCATGTGGAATTCTCGGGTGCCAAGGGGCTAAAAGACCCTTGGA |

| ASO ID | Accession | Group ID | Custom Array sequence |
|---|---|---|---|
| SARS2-Probe I.600 | NC_045512.2 | SARS2-Probe I | CTGATGAGGGAGAAACATTAATACGACTCACTATAGGGGCGAAGAAGCTATAAGACATGTACGTGCATGGATTGGCTTCGATGTCGAGGGGTGTCATGCTACTAGAGAAGCTGTTGGTACCAATTTACTGGAATTCTCGGGTGCCAAGGGGCTAAAAGACCCTTGGA |
| SARS2-Probe I.601 | NC_045512.2 | SARS2-Probe I | CTGATGAGGGAGAAACATTAATACGACTCACTATAGGGGGGATTGGCTTCGATGTCGAGGGGTGTCATGCTACTAGAGAAGCTGTTGGTACCAATTTACCTTTACAGCTAGGTTTTTCTACAGGTGTTATGGAATTCTCGGGTGCCAAGGGGCTAAAAGACCCTTGGA |
| SARS2-Probe I.602 | NC_045512.2 | SARS2-Probe I | CTGATGAGGGAGAAACATTAATACGACTCACTATAGGGCTACTAGAGAAGCTGTTGGTACCAATTTACCTTTACAGCTAGGTTTTTCTACAGGTGTTAACCTAGTTGCTGTACCTACAGGTTATGTTGTGTGGAATTCTCGGGTGCCAAGGGGCTAAAAGACCCTTGGA |
| SARS2-Probe I.603 | NC_045512.2 | SARS2-Probe I | CTGATGAGGGAGAAACATTAATACGACTCACTATAGGGCTTTACAGCTAGGTTTTTCTACAGGTGTTAACCTAGTTGCTGTACCTACAGGTTATGTTGATACACCTAATAATACAGATTTTTCCAGAGTGGAATTCTCGGGTGCCAAGGGGCTAAAAGACCCTTGGA |
| SARS2-Probe I.604 | NC_045512.2 | SARS2-Probe I | CTGATGAGGGAGAAACATTAATACGACTCACTATAGGGACCTAGTTGCTGTACCTACAGGTTATGTTGATACACCTAATAATACAGATTTTTCCAGAGTTAGTGCTAAACCACCGCCTGGAGATCAATTGGAATTCTCGGGTGCCAAGGGGCTAAAAGACCCTTGGA |
| SARS2-Probe I.605 | NC_045512.2 | SARS2-Probe I | CTGATGAGGGAGAAACATTAATACGACTCACTATAGGGGATACACCTAATAATACAGATTTTTCCAGAGTTAGTGCTAAACCACCGCCTGGAGATCAATTTAAACACCTCATACCACTTATGTACAAAGTGGAATTCTCGGGTGCCAAGGGGCTAAAAGACCCTTGGA |
| SARS2-Probe I.606 | NC_045512.2 | SARS2-Probe I | CTGATGAGGGAGAAACATTAATACGACTCACTATAGGGTTAGTGCTAAACCACCGCCTGGAGATCAATTTAAACACCTCATACCACTTATGTACAAAGGACTTCCTTGGAATGTAGTGCGTATAAAGATGGAATTCTCGGGTGCCAAGGGGCTAAAAGACCCTTGGA |
| SARS2-Probe I.607 | NC_045512.2 | SARS2-Probe I | CTGATGAGGGAGAAACATTAATACGACTCACTATAGGGTTAAACACCTCATACCACTTATGTACAAAGGACTTCCTTGGAATGTAGTGCGTATAAAGATTGTACAAATGTTAAGTGACACACTTAAAATGGAATTCTCGGGTGCCAAGGGGCTAAAAGACCCTTGGA |
| SARS2-Probe I.608 | NC_045512.2 | SARS2-Probe I | CTGATGAGGGAGAAACATTAATACGACTCACTATAGGGGACTTCCTTGGAATGTAGTGCGTATAAAGATTGTACAAATGTTAAGTGACACACTTAAAAATCTCTCTGACAGAGTCGTATTTGTCTTATTGGAATTCTCGGGTGCCAAGGGGCTAAAAGACCCTTGGA |
| SARS2-Probe I.609 | NC_045512.2 | SARS2-Probe I | CTGATGAGGGAGAAACATTAATACGACTCACTATAGGGTTGTACAAATGTTAAGTGACACACTTAAAAATCTCTCTGACAGAGTCGTATTTGTCTTATGGGCACATGGCTTTGAGTTGACATCTATGATGGAATTCTCGGGTGCCAAGGGGCTAAAAGACCCTTGGA |
| SARS2-Probe I.610 | NC_045512.2 | SARS2-Probe I | CTGATGAGGGAGAAACATTAATACGACTCACTATAGGGATCTCTCTGACAGAGTCGTATTTGTCTTATGGGCACATGGCTTTGAGTTGACATCTATGAAGTATTTTGTGAAAATAGGACCTGAGCGCATGGAATTCTCGGGTGCCAAGGGGCTAAAAGACCCTTGGA |
| SARS2-Probe I.611 | NC_045512.2 | SARS2-Probe I | CTGATGAGGGAGAAACATTAATACGACTCACTATAGGGGGGGCACATGGCTTTGAGTTGACATCTATGAAGTATTTTGTGAAAATAGGACCTGAGCGCACCTGTTGTCTATGTGATAGACGTGCCACATTGGAATTCTCGGGTGCCAAGGGGCTAAAAGACCCTTGGA |
| SARS2-Probe I.612 | NC_045512.2 | SARS2-Probe I | CTGATGAGGGAGAAACATTAATACGACTCACTATAGGGAGTATTTTGTGAAAATAGGACCTGAGCGCACCTGTTGTCTATGTGATAGACGTGCCACATGCTTTTCCACTGCTTCAGACACTTATGCCTTGGAATTCTCGGGTGCCAAGGGGCTAAAAGACCCTTGGA |
| SARS2-Probe I.613 | NC_045512.2 | SARS2-Probe I | CTGATGAGGGAGAAACATTAATACGACTCACTATAGGGCCTGTTGTCTATGTGATAGACGTGCCACATGCTTTTCCACTGCTTCAGACACTTATGCCTGTTGGCATCATTCTATTGGATTTGATTACGTGGAATTCTCGGGTGCCAAGGGGCTAAAAGACCCTTGGA |
| SARS2-Probe I.614 | NC_045512.2 | SARS2-Probe I | CTGATGAGGGAGAAACATTAATACGACTCACTATAGGGGCTTTTCCACTGCTTCAGACACTTATGCCTGTTGGCATCATTCTATTGGATTTGATTACGTCTATAATCCGTTTATGATTGATGTTCAACTGGAATTCTCGGGTGCCAAGGGGCTAAAAGACCCTTGGA |
| SARS2-Probe I.615 | NC_045512.2 | SARS2-Probe I | CTGATGAGGGAGAAACATTAATACGACTCACTATAGGGGTTGGCATCATTCTATTGGATTTGATTACGTCTATAATCCGTTTATGATTGATGTTCAACAATGGGGTTTTACAGGTAACCTACAAAGCATGGAATTCTCGGGTGCCAAGGGGCTAAAAGACCCTTGGA |
| SARS2-Probe I.616 | NC_045512.2 | SARS2-Probe I | CTGATGAGGGAGAAACATTAATACGACTCACTATAGGGTCTATAATCCGTTTATGATTGATGTTCAACAATGGGGTTTTACAGGTAACCTACAAAGCAACCATGATCTGTATTGTCAAGTCCATGGTATGGAATTCTCGGGTGCCAAGGGGCTAAAAGACCCTTGGA |
| SARS2-Probe I.617 | NC_045512.2 | SARS2-Probe I | CTGATGAGGGAGAAACATTAATACGACTCACTATAGGGAATGGGGTTTTACAGGTAACCTACAAAGCAACCATGATCTGTATTGTCAAGTCCATGGTAATGCACATGTAGCTAGTTGTGATGCAATCATGGAATTCTCGGGTGCCAAGGGGCTAAAAGACCCTTGGA |
| SARS2-Probe I.618 | NC_045512.2 | SARS2-Probe I | CTGATGAGGGAGAAACATTAATACGACTCACTATAGGGACCATGATCTGTATTGTCAAGTCCATGGTAATGCACATGTAGCTAGTTGTGATGCAATCATGACTAGGTGTCTAGCTGTCCACGAGTGCTTGGAATTCTCGGGTGCCAAGGGGCTAAAAGACCCTTGGA |
| SARS2-Probe I.619 | NC_045512.2 | SARS2-Probe I | CTGATGAGGGAGAAACATTAATACGACTCACTATAGGGATGCACATGTAGCTAGTTGTGATGCAATCATGACTAGGTGTCTAGCTGTCCACGAGTGCTTTGTTAAGCGTGTTGACTGGACTATTGAATTGGAATTCTCGGGTGCCAAGGGGCTAAAAGACCCTTGGA |
| SARS2-Probe I.620 | NC_045512.2 | SARS2-Probe I | CTGATGAGGGAGAAACATTAATACGACTCACTATAGGGTGACTAGGTGTCTAGCTGTCCACGAGTGCTTTGTTAAGCGTGTTGACTGGACTATTGAATATCCTATAATTGGTGATGAACTGAAGATTATGGAATTCTCGGGTGCCAAGGGGCTAAAAGACCCTTGGA |
| SARS2-Probe I.621 | NC_045512.2 | SARS2-Probe I | CTGATGAGGGAGAAACATTAATACGACTCACTATAGGGTTGTTAAGCGTGTTGACTGGACTATTGAATATCCTATAATTGGTGATGAACTGAAGATTAATGCGGCTTGTAGAAAAGGTTCAACACATGGTGGAATTCTCGGGTGCCAAGGGGCTAAAAGACCCTTGGA |
| SARS2-Probe I.622 | NC_045512.2 | SARS2-Probe I | CTGATGAGGGAGAAACATTAATACGACTCACTATAGGGGATCCTATAATTGGTGATGAACTGAAGATTAATGCGGCTTGTAGAAAAGGTTCAACACATGGTTGTTAAAGCTGCATTATTAGCAGACAAATTGGAATTCTCGGGTGCCAAGGGGCTAAAAGACCCTTGGA |
| SARS2-Probe I.623 | NC_045512.2 | SARS2-Probe I | CTGATGAGGGAGAAACATTAATACGACTCACTATAGGGGATGCGGCTTGTAGAAAAGGTTCAACACATGGTTGTTAAAGCTGCATTATTAGCAGACAAATTCCCAGTTCTTCACGACATTGGTAACCCTATGGAATTCTCGGGTGCCAAGGGGCTAAAAGACCCTTGGA |
| SARS2-Probe I.624 | NC_045512.2 | SARS2-Probe I | CTGATGAGGGAGAAACATTAATACGACTCACTATAGGGTTGTTAAAGCTGCATTATTAGCAGACAAATTCCCAGTTCTTCACGACATTGGTAACCCTAAAGCTATTAAGTGTGTACCTCAAGCTGATGTGGAATTCTCGGGTGCCAAGGGGCTAAAAGACCCTTGGA |

| ASO ID | Accession | Group ID | Custom Array sequence |
|---|---|---|---|
| SARS2-Probe I.625 | NC_045512.2 | SARS2-Probe I | CTGATGAGGGAGAAACATTAATACGACTCACTATAGGGTCCCAGTTCTTCACGACATTGGTAACCCTAAAGCTATTAAGTGTGTACCTCAAGCTGATGTAGAATGGAAGTTCTATGATGCACAGCCTTTGGAATTCTCGGGTGCCAAGGGGCTAAAAGACCCTTGGA |
| SARS2-Probe I.626 | NC_045512.2 | SARS2-Probe I | CTGATGAGGGAGAAACATTAATACGACTCACTATAGGGAAGCTATTAAGTGTGTACCTCAAGCTGATGTAGAATGGAAGTTCTATGATGCACAGCCTTGTAGTGACAAAGCTTATAAAATAGAAGAATTGGAATTCTCGGGTGCCAAGGGGCTAAAAGACCCTTGGA |
| SARS2-Probe I.627 | NC_045512.2 | SARS2-Probe I | CTGATGAGGGAGAAACATTAATACGACTCACTATAGGGTAGAATGGAAGTTCTATGATGCACAGCCTTGTAGTGACAAAGCTTATAAAATAGAAGAATTATTCTATTCTTATGCCACACATTCTGACATGGAATTCTCGGGTGCCAAGGGGCTAAAAGACCCTTGGA |
| SARS2-Probe I.628 | NC_045512.2 | SARS2-Probe I | CTGATGAGGGAGAAACATTAATACGACTCACTATAGGGGTAGTGACAAAGCTTATAAAATAGAAGAATTATTCTATTCTTATGCCACACATTCTGACAAATTCACAGATGGTGTATGCCTATTTTGGAATTCTCGGGTGCCAAGGGGCTAAAAGACCCTTGGA |
| SARS2-Probe I.629 | NC_045512.2 | SARS2-Probe I | CTGATGAGGGAGAAACATTAATACGACTCACTATAGGGTATTCTATTCTTATGCCACACATTCTGACAAATTCACAGATGGTGTATGCCTATTTTGGAATTGCAATGTCGATAGATATCCTGCTAATTTGGAATTCTCGGGTGCCAAGGGGCTAAAAGACCCTTGGA |
| SARS2-Probe I.630 | NC_045512.2 | SARS2-Probe I | CTGATGAGGGAGAAACATTAATACGACTCACTATAGGGAATTCACAGATGGTGTATGCCTATTTTGGAATTGCAATGTCGATAGATATCCTGCTAATTCCATTGTTTGTAGATTTGACACTAGAGTGCTGGAATTCTCGGGTGCCAAGGGGCTAAAAGACCCTTGGA |
| SARS2-Probe I.631 | NC_045512.2 | SARS2-Probe I | CTGATGAGGGAGAAACATTAATACGACTCACTATAGGGATTGCAATGTCGATAGATATCCTGCTAATTCCATTGTTTGTAGATTTGACACTAGAGTGCTATCTAACCTTAACTTGCCTGGTTGTGATGTGGAATTCTCGGGTGCCAAGGGGCTAAAAGACCCTTGGA |
| SARS2-Probe I.632 | NC_045512.2 | SARS2-Probe I | CTGATGAGGGAGAAACATTAATACGACTCACTATAGGGCCATTGTTTGTAGATTTGACACTAGAGTGCTATCTAACCTTAACTTGCCTGGTTGTGATGGTGGCAGTTTGTATGTAAATAAACATGCCATTGGAATTCTCGGGTGCCAAGGGGCTAAAAGACCCTTGGA |
| SARS2-Probe I.633 | NC_045512.2 | SARS2-Probe I | CTGATGAGGGAGAAACATTAATACGACTCACTATAGGGTATCTAACCTTAACTTGCCTGGTTGTGATGGTGGCAGTTTGTATGTAAATAAACATGCATTCCACACACCAGCTTTTGATAAAAGTGCTTTGGAATTCTCGGGTGCCAAGGGGCTAAAAGACCCTTGGA |
| SARS2-Probe I.634 | NC_045512.2 | SARS2-Probe I | CTGATGAGGGAGAAACATTAATACGACTCACTATAGGGGTGGCAGTTTGTATGTAAATAAACATGCATTCCACACACCAGCTTTTGATAAAAGTGCTTTTGTTAATTTAAAACAATTACCATTTTTCTTGGAATTCTCGGGTGCCAAGGGGCTAAAAGACCCTTGGA |
| SARS2-Probe I.635 | NC_045512.2 | SARS2-Probe I | CTGATGAGGGAGAAACATTAATACGACTCACTATAGGGTCCACACACCAGCTTTTGATAAAAGTGCTTTTGTTAATTTAAAACAATTACCATTTTTCTATTACTCTGACAGTCCATGTGAGTCTCATGTGGAATTCTCGGGTGCCAAGGGGCTAAAAGACCCTTGGA |
| SARS2-Probe I.636 | NC_045512.2 | SARS2-Probe I | CTGATGAGGGAGAAACATTAATACGACTCACTATAGGGGTTGTTAATTTAAAACAATTACCATTTTTCTATTACTCTGACAGTCCATGTGAGTCTCATGGAAAACAAGTAGTGTCAGATATAGATTATGTGGAATTCTCGGGTGCCAAGGGGCTAAAAGACCCTTGGA |
| SARS2-Probe I.637 | NC_045512.2 | SARS2-Probe I | CTGATGAGGGAGAAACATTAATACGACTCACTATAGGGATTACTCTGACAGTCCATGTGAGTCTCATGGAAAACAAGTAGTGTCAGATATAGATTATGTACCACTAAAGTCTGCTACGTGTATAACACTGGAATTCTCGGGTGCCAAGGGGCTAAAAGACCCTTGGA |
| SARS2-Probe I.638 | NC_045512.2 | SARS2-Probe I | CTGATGAGGGAGAAACATTAATACGACTCACTATAGGGGAAAACAAGTAGTGTCAGATATAGATTATGTACCACTAAAGTCTGCTACGTGTATAACACGTTGCAATTTAGGTGGTGCTGTCTGTAGACTGGAATTCTCGGGTGCCAAGGGGCTAAAAGACCCTTGGA |
| SARS2-Probe I.639 | NC_045512.2 | SARS2-Probe I | CTGATGAGGGAGAAACATTAATACGACTCACTATAGGGTACCACTAAAGTCTGCTACGTGTATAACACGTTGCAATTTAGGTGGTGCTGTCTGTAGACATCATGCTAATGAGTACAGATTGTATCTCGTGGAATTCTCGGGTGCCAAGGGGCTAAAAGACCCTTGGA |
| SARS2-Probe I.640 | NC_045512.2 | SARS2-Probe I | CTGATGAGGGAGAAACATTAATACGACTCACTATAGGGGTTGCAATTTAGGTGGTGCTGTCTGTAGACATCATGCTAATGAGTACAGATTGTATCTCGATGCTTATAACATGATGATCTCAGCTGGCTTGGAATTCTCGGGTGCCAAGGGGCTAAAAGACCCTTGGA |
| SARS2-Probe I.641 | NC_045512.2 | SARS2-Probe I | CTGATGAGGGAGAAACATTAATACGACTCACTATAGGGATCATGCTAATGAGTACAGATTGTATCTCGATGCTTATAACATGATGATCTCAGCTGGCTTTAGCTTGTGGGTTTACAAACAATTTGATATGGAATTCTCGGGTGCCAAGGGGCTAAAAGACCCTTGGA |
| SARS2-Probe I.642 | NC_045512.2 | SARS2-Probe I | CTGATGAGGGAGAAACATTAATACGACTCACTATAGGGATGCTTATAACATGATGATCTCAGCTGGCTTTAGCTTGTGGGTTTACAAACAATTTGATACTTATAACCTCTGGAACACTTTTACAAGACTGGAATTCTCGGGTGCCAAGGGGCTAAAAGACCCTTGGA |
| SARS2-Probe I.643 | NC_045512.2 | SARS2-Probe I | CTGATGAGGGAGAAACATTAATACGACTCACTATAGGGTTAGCTTGTGGGTTTACAAACAATTTGATACTTATAACCTCTGGAACACTTTTACAAGACTTCAGATTTAGAAAATGTGGCTTTTAATGTGGAATTCTCGGGTGCCAAGGGGCTAAAAGACCCTTGGA |
| SARS2-Probe I.644 | NC_045512.2 | SARS2-Probe I | CTGATGAGGGAGAAACATTAATACGACTCACTATAGGGCTTATAACCTCTGGAACACTTTTACAAGACTTCAGATTTAGAAAATGTGGCTTTTAATGTTGTAAATAAGGGACACTTTGATGGACAACTGGAATTCTCGGGTGCCAAGGGGCTAAAAGACCCTTGGA |
| SARS2-Probe I.645 | NC_045512.2 | SARS2-Probe I | CTGATGAGGGAGAAACATTAATACGACTCACTATAGGGTTCAGATTTAGAAAATGTGGCTTTTAATGTTGTAAATAAGGGACACTTTGATGGACAACAGGGTGAAGTACCAGTTTCTATCATTAATATGGAATTCTCGGGTGCCAAGGGGCTAAAAGACCCTTGGA |
| SARS2-Probe I.646 | NC_045512.2 | SARS2-Probe I | CTGATGAGGGAGAAACATTAATACGACTCACTATAGGGTTGTAAATAAGGGACACTTTGATGGACAACAGGGTGAAGTACCAGTTTCTATCATTAATAACACTGTTTACACAAAAGTTGATGGTGTTGTGGAATTCTCGGGTGCCAAGGGGCTAAAAGACCCTTGGA |
| SARS2-Probe I.647 | NC_045512.2 | SARS2-Probe I | CTGATGAGGGAGAAACATTAATACGACTCACTATAGGGAGGGTGAAGTACCAGTTTCTATCATTAATAACACTGTTTACACAAAAGTTGATGGTGTTGATGTAGAATTGTTTGAAAATAAAACAACATTGGAATTCTCGGGTGCCAAGGGGCTAAAAGACCCTTGGA |
| SARS2-Probe I.648 | NC_045512.2 | SARS2-Probe I | CTGATGAGGGAGAAACATTAATACGACTCACTATAGGGACACTGTTTACACAAAAGTTGATGGTGTTGATGTAGAATTGTTTGAAAATAAAACAACATTACCTGTTAATGTAGCATTTGAGCTTTGGGTGGAATTCTCGGGTGCCAAGGGGCTAAAAGACCCTTGGA |
| SARS2-Probe I.649 | NC_045512.2 | SARS2-Probe I | CTGATGAGGGAGAAACATTAATACGACTCACTATAGGGATGTAGAATTGTTTGAAAATAAAACAACATTACCTGTTAATGTAGCATTTGAGCTTTGGGCTAAGCGCAACATTAAACCAGTACCAGAGGTGGAATTCTCGGGTGCCAAGGGGCTAAAAGACCCTTGGA |

| ASO ID | Accession | Group ID | Custom Array sequence |
|---|---|---|---|
| SARS2-Probe I.650 | NC_045512.2 | SARS2-Probe I | CTGATGAGGGAGAAACATTAATACGACTCACTATAGGGTACCTGTTAATGTAGCATTTGAGCTTTGGGCTAAGCGCAACATTAAACCAGTACCAGAGGTGAAAATACTCAATAATTTGGGTGTGGACATGGAATTCTCGGGTGCCAAGGGGCTAAAAGACCCTTGGA |
| SARS2-Probe I.651 | NC_045512.2 | SARS2-Probe I | CTGATGAGGGAGAAACATTAATACGACTCACTATAGGGCTAAGCGCAACATTAAACCAGTACCAGAGGTGAAAATACTCAATAATTTGGGTGTGGACATTGCTGCTAATACTGTGATCTGGGACTACATGGAATTCTCGGGTGCCAAGGGGCTAAAAGACCCTTGGA |
| SARS2-Probe I.652 | NC_045512.2 | SARS2-Probe I | CTGATGAGGGAGAAACATTAATACGACTCACTATAGGGTGAAAATACTCAATAATTTGGGTGTGGACATTGCTGCTAATACTGTGATCTGGGACTACAAAAGAGATGCTCCAGCACATATATCTACTATGGAATTCTCGGGTGCCAAGGGGCTAAAAGACCCTTGGA |
| SARS2-Probe I.653 | NC_045512.2 | SARS2-Probe I | CTGATGAGGGAGAAACATTAATACGACTCACTATAGGGTTGCTGCTAATACTGTGATCTGGGACTACAAAAGAGATGCTCCAGCACATATATCTACTATTGGTGTTTGTTCTATGACTGACATAGCCATGGAATTCTCGGGTGCCAAGGGGCTAAAAGACCCTTGGA |
| SARS2-Probe I.654 | NC_045512.2 | SARS2-Probe I | CTGATGAGGGAGAAACATTAATACGACTCACTATAGGGAAAGAGATGCTCCAGCACATATATCTACTATTGGTGTTTGTTCTATGACTGACATAGCCAAGAAACCAACTGAAACGATTTGTGCACCACTGGAATTCTCGGGTGCCAAGGGGCTAAAAGACCCTTGGA |
| SARS2-Probe I.655 | NC_045512.2 | SARS2-Probe I | CTGATGAGGGAGAAACATTAATACGACTCACTATAGGGTTGGTGTTTGTTCTATGACTGACATAGCCAAGAAACCAACTGAAACGATTTGTGCACCACTCACTGTCTTTTTTGATGGTAGAGTTGATGTGGAATTCTCGGGTGCCAAGGGGCTAAAAGACCCTTGGA |
| SARS2-Probe I.656 | NC_045512.2 | SARS2-Probe I | CTGATGAGGGAGAAACATTAATACGACTCACTATAGGGAGAAACCAACTGAAACGATTTGTGCACCACTCACTGTCTTTTTTGATGGTAGAGTTGATGGTCAAGTAGACTTATTTAGAAATGCCCGTATGGAATTCTCGGGTGCCAAGGGGCTAAAAGACCCTTGGA |
| SARS2-Probe I.657 | NC_045512.2 | SARS2-Probe I | CTGATGAGGGAGAAACATTAATACGACTCACTATAGGGTCACTGTCTTTTTTGATGGTAGAGTTGATGGTCAAGTAGACTTATTTAGAAATGCCCGTAATGGTGTTCTTATTACAGAAGGTAGTGTTATGGAATTCTCGGGTGCCAAGGGGCTAAAAGACCCTTGGA |
| SARS2-Probe I.658 | NC_045512.2 | SARS2-Probe I | CTGATGAGGGAGAAACATTAATACGACTCACTATAGGGGTCAAGTAGACTTATTTAGAAATGCCCGTAATGGTGTTCTTATTACAGAAGGTAGTGTTAAAGGTTTACAACCATCTGTAGGTCCCAAACTGGAATTCTCGGGTGCCAAGGGGCTAAAAGACCCTTGGA |
| SARS2-Probe I.659 | NC_045512.2 | SARS2-Probe I | CTGATGAGGGAGAAACATTAATACGACTCACTATAGGGATGGTGTTCTTATTACAGAAGGTAGTGTTAAAGGTTTACAACCATCTGTAGGTCCCAAACAAGCTAGTCTTAATGGAGTCACATTAATTGTGTGGAATTCTCGGGTGCCAAGGGGCTAAAAGACCCTTGGA |
| SARS2-Probe I.660 | NC_045512.2 | SARS2-Probe I | CTGATGAGGGAGAAACATTAATACGACTCACTATAGGGAAGGTTTACAACCATCTGTAGGTCCCAAACAAGCTAGTCTTAATGGAGTCACATTAATTGGAGAAGCCGTAAAAACACAGTTCAATTATTTGGAATTCTCGGGTGCCAAGGGGCTAAAAGACCCTTGGA |
| SARS2-Probe I.661 | NC_045512.2 | SARS2-Probe I | CTGATGAGGGAGAAACATTAATACGACTCACTATAGGGAAGCTAGTCTTAATGGAGTCACATTAATTGGAGAAGCCGTAAAAACACAGTTCAATTATTATAAGAAAGTTGATGGTGTTGTCCAACAATTGGAATTCTCGGGTGCCAAGGGGCTAAAAGACCCTTGGA |
| SARS2-Probe I.662 | NC_045512.2 | SARS2-Probe I | CTGATGAGGGAGAAACATTAATACGACTCACTATAGGGGAGAAGCCGTAAAAACACAGTTCAATTATTATAAGAAAGTTGATGGTGTTGTCCAACAATTACCTGAAACTTACTTTACTCAGAGTAGAATGGAATTCTCGGGTGCCAAGGGGCTAAAAGACCCTTGGA |
| SARS2-Probe I.663 | NC_045512.2 | SARS2-Probe I | CTGATGAGGGAGAAACATTAATACGACTCACTATAGGGATAAGAAAGTTGATGGTGTTGTCCAACAATTACCTGAAACTTACTTTACTCAGAGTAGAAATTTACAAGAATTTAAACCCAGGAGTCAAATGGAATTCTCGGGTGCCAAGGGGCTAAAAGACCCTTGGA |
| SARS2-Probe I.664 | NC_045512.2 | SARS2-Probe I | CTGATGAGGGAGAAACATTAATACGACTCACTATAGGGTACCTGAAACTTACTTTACTCAGAGTAGAAATTTACAAGAATTTAAACCCAGGAGTCAAATGGAAATTGATTTCTTAGAATTAGCTATGGTGGAATTCTCGGGTGCCAAGGGGCTAAAAGACCCTTGGA |
| SARS2-Probe I.665 | NC_045512.2 | SARS2-Probe I | CTGATGAGGGAGAAACATTAATACGACTCACTATAGGGATTTACAAGAATTTAAACCCAGGAGTCAAATGGAAATTGATTTCTTAGAATTAGCTATGGATGAATTCATTGAACGGTATAAATTAGAAGTGGAATTCTCGGGTGCCAAGGGGCTAAAAGACCCTTGGA |
| SARS2-Probe I.666 | NC_045512.2 | SARS2-Probe I | CTGATGAGGGAGAAACATTAATACGACTCACTATAGGGTGGAAATTGATTTCTTAGAATTAGCTATGGATGAATTCATTGAACGGTATAAATTAGAAGGCTATGCCTTCGAACATATCGTTTATGGAGTGGAATTCTCGGGTGCCAAGGGGCTAAAAGACCCTTGGA |
| SARS2-Probe I.667 | NC_045512.2 | SARS2-Probe I | CTGATGAGGGAGAAACATTAATACGACTCACTATAGGGATGAATTCATTGAACGGTATAAATTAGAAGGCTATGCCTTCGAACATATCGTTTATGGAGATTTTAGTCATAGTCAGTTAGGTGGTTTACTGGAATTCTCGGGTGCCAAGGGGCTAAAAGACCCTTGGA |
| SARS2-Probe I.668 | NC_045512.2 | SARS2-Probe I | CTGATGAGGGAGAAACATTAATACGACTCACTATAGGGCTATGCCTTCGAACATATCGTTTATGGAGATTTTAGTCATAGTCAGTTAGGTGGTTTACATCTACTGATTGGACTAGCTAAACGTTTTATGGAATTCTCGGGTGCCAAGGGGCTAAAAGACCCTTGGA |
| SARS2-Probe I.669 | NC_045512.2 | SARS2-Probe I | CTGATGAGGGAGAAACATTAATACGACTCACTATAGGGATTTTAGTCATAGTCAGTTAGGTGGTTTACATCTACTGATTGGACTAGCTAAACGTTTTAAGGAATCACCTTTTGAATTAGAAGATTTTATGGAATTCTCGGGTGCCAAGGGGCTAAAAGACCCTTGGA |
| SARS2-Probe I.670 | NC_045512.2 | SARS2-Probe I | CTGATGAGGGAGAAACATTAATACGACTCACTATAGGGATCTACTGATTGGACTAGCTAAACGTTTTAAGGAATCACCTTTTGAATTAGAAGATTTTATTCCTATGGACAGTACAGTTAAAAACTATTTGGAATTCTCGGGTGCCAAGGGGCTAAAAGACCCTTGGA |
| SARS2-Probe I.671 | NC_045512.2 | SARS2-Probe I | CTGATGAGGGAGAAACATTAATACGACTCACTATAGGGAGGAATCACCTTTTGAATTAGAAGATTTTATTCCTATGGACAGTACAGTTAAAAACTATTTCATAACAGATGCGCAAACAGGTTCATCTATGGAATTCTCGGGTGCCAAGGGGCTAAAAGACCCTTGGA |
| SARS2-Probe I.672 | NC_045512.2 | SARS2-Probe I | CTGATGAGGGAGAAACATTAATACGACTCACTATAGGGTTCCTATGGACAGTACAGTTAAAAACTATTTCATAACAGATGCGCAAACAGGTTCATCTAAGTGTGTGTGTTCTGTTATTGATTTATTACTGGAATTCTCGGGTGCCAAGGGGCTAAAAGACCCTTGGA |
| SARS2-Probe I.673 | NC_045512.2 | SARS2-Probe I | CTGATGAGGGAGAAACATTAATACGACTCACTATAGGGTCATAACAGATGCGCAAACAGGTTCATCTAAGTGTGTGTGTTCTGTTATTGATTTATTACTTGATGATTTTGTTGAAATAATAAAATCCCTGGAATTCTCGGGTGCCAAGGGGCTAAAAGACCCTTGGA |
| SARS2-Probe I.674 | NC_045512.2 | SARS2-Probe I | CTGATGAGGGAGAAACATTAATACGACTCACTATAGGGAGTGTGTGTGTTCTGTTATTGATTTATTACTTGATGATTTTGTTGAAATAATAAAATCCCAAGATTTATCTGTAGTTTCTAAGGTTGTCATGGAATTCTCGGGTGCCAAGGGGCTAAAAGACCCTTGGA |

| ASO ID | Accession | Group ID | Custom Array sequence |
|---|---|---|---|
| SARS2-Probe I.675 | NC_045512.2 | SARS2-Probe I | CTGATGAGGGAGAAACATTAATACGACTCACTATAGGGTTGATGATTTTGTTGAAATAATAAAATCCCAAGATTTATCTGTAGTTTCTAAGGTTGTCAAAGTGACTATTGACTATACAGAAATTTCATTGGAATTCTCGGGTGCCAAGGGGCTAAAAGACCCTTGGA |
| SARS2-Probe I.676 | NC_045512.2 | SARS2-Probe I | CTGATGAGGGAGAAACATTAATACGACTCACTATAGGGAAGATTTATCTGTAGTTTCTAAGGTTGTCAAAGTGACTATTGACTATACAGAAATTTCATTTATGCTTTGGTGTAAAGATGGCCATGTAGTGGAATTCTCGGGTGCCAAGGGGCTAAAAGACCCTTGGA |
| SARS2-Probe I.677 | NC_045512.2 | SARS2-Probe I | CTGATGAGGGAGAAACATTAATACGACTCACTATAGGGAAGTGACTATTGACTATACAGAAATTTCATTTATGCTTTGGTGTAAAGATGGCCATGTAGAAACATTTTACCCAAAATTACAATCTAGTCTGGAATTCTCGGGTGCCAAGGGGCTAAAAGACCCTTGGA |
| SARS2-Probe I.678 | NC_045512.2 | SARS2-Probe I | CTGATGAGGGAGAAACATTAATACGACTCACTATAGGGTTATGCTTTGGTGTAAAGATGGCCATGTAGAAACATTTTACCCAAAATTACAATCTAGTCAAGCGTGGCAACCGGGTGTTGCTATGCCTATGGAATTCTCGGGTGCCAAGGGGCTAAAAGACCCTTGGA |
| SARS2-Probe I.679 | NC_045512.2 | SARS2-Probe I | CTGATGAGGGAGAAACATTAATACGACTCACTATAGGGAAACATTTTACCCAAAATTACAATCTAGTCAAGCGTGGCAACCGGGTGTTGCTATGCCTAATCTTTACAAAATGCAAAGAATGCTATTAGTGGAATTCTCGGGTGCCAAGGGGCTAAAAGACCCTTGGA |
| SARS2-Probe I.680 | NC_045512.2 | SARS2-Probe I | CTGATGAGGGAGAAACATTAATACGACTCACTATAGGGAAGCGTGGCAACCGGGTGTTGCTATGCCTAATCTTTACAAAATGCAAAGAATGCTATTAGAAAAGTGTGACCTTCAAAATTATGGTGATATGGAATTCTCGGGTGCCAAGGGGCTAAAAGACCCTTGGA |
| SARS2-Probe I.681 | NC_045512.2 | SARS2-Probe I | CTGATGAGGGAGAAACATTAATACGACTCACTATAGGGATCTTTACAAAATGCAAAGAATGCTATTAGAAAAGTGTGACCTTCAAAATTATGGTGATAGTGCAACATTACCTAAAGGCATAATGATGATGGAATTCTCGGGTGCCAAGGGGCTAAAAGACCCTTGGA |
| SARS2-Probe I.682 | NC_045512.2 | SARS2-Probe I | CTGATGAGGGAGAAACATTAATACGACTCACTATAGGGAAAAGTGTGACCTTCAAAATTATGGTGATAGTGCAACATTACCTAAAGGCATAATGATGAATGTCGCAAAATATACTCAACTGTGTCAATTGGAATTCTCGGGTGCCAAGGGGCTAAAAGACCCTTGGA |
| SARS2-Probe I.683 | NC_045512.2 | SARS2-Probe I | CTGATGAGGGAGAAACATTAATACGACTCACTATAGGGGTGCAACATTACCTAAAGGCATAATGATGAATGTCGCAAAATATACTCAACTGTGTCAATATTTAAACACATTAACATTAGCTGTACCCTTGGAATTCTCGGGTGCCAAGGGGCTAAAAGACCCTTGGA |
| SARS2-Probe I.684 | NC_045512.2 | SARS2-Probe I | CTGATGAGGGAGAAACATTAATACGACTCACTATAGGGATGTCGCAAAATATACTCAACTGTGTCAATATTTAAACACATTAACATTAGCTGTACCCTATAATATGAGAGTTATACATTTTGGTGCTGTGGAATTCTCGGGTGCCAAGGGGCTAAAAGACCCTTGGA |
| SARS2-Probe I.685 | NC_045512.2 | SARS2-Probe I | CTGATGAGGGAGAAACATTAATACGACTCACTATAGGGATTTAAACACATTAACATTAGCTGTACCCTATAATATGAGAGTTATACATTTTGGTGCTGGTTCTGATAAAGGAGTTGCACCAGGTACAGTGGAATTCTCGGGTGCCAAGGGGCTAAAAGACCCTTGGA |
| SARS2-Probe I.686 | NC_045512.2 | SARS2-Probe I | CTGATGAGGGAGAAACATTAATACGACTCACTATAGGGATAATATGAGAGTTATACATTTTGGTGCTGGTTCTGATAAAGGAGTTGCACCAGGTACAGTGTTTAAGACAGTGGTTGCCTACGGGTATGGAATTCTCGGGTGCCAAGGGGCTAAAAGACCCTTGGA |
| SARS2-Probe I.687 | NC_045512.2 | SARS2-Probe I | CTGATGAGGGAGAAACATTAATACGACTCACTATAGGGGTTCTGATAAAGGAGTTGCACCAGGTACAGTCAGCTGTTTAAGACAGTGGTTGCCTACGGGTACGCTGCTTGTCGATTCAGATCTTAATGACTTGGAATTCTCGGGTGCCAAGGGGCTAAAAGACCCTTGGA |
| SARS2-Probe I.688 | NC_045512.2 | SARS2-Probe I | CTGATGAGGGAGAAACATTAATACGACTCACTATAGGGCTGTTTTAAGACAGTGGTTGCCTACGGGTACGCTGCTTGTCGATTCAGATCTTAATGACTTTGTCTCTGATGCAGATTCAACTTTGATTGTGGAATTCTCGGGTGCCAAGGGGCTAAAAGACCCTTGGA |
| SARS2-Probe I.689 | NC_045512.2 | SARS2-Probe I | CTGATGAGGGAGAAACATTAATACGACTCACTATAGGGCGCTGCTTGTCGATTCAGATCTTAATGACTTTGTCTCTGATGCAGATTCAACTTTGATTGGTGATTGTGCAACTGTACATACAGCTAATATGGAATTCTCGGGTGCCAAGGGGCTAAAAGACCCTTGGA |
| SARS2-Probe I.690 | NC_045512.2 | SARS2-Probe I | CTGATGAGGGAGAAACATTAATACGACTCACTATAGGGTTGTCTCTGATGCAGATTCAACTTTGATTGGTGATTGTGCAACTGTACATACAGCTAATAAATGGGATCTCATTATTAGTGATATGTACGTGGAATTCTCGGGTGCCAAGGGGCTAAAAGACCCTTGGA |
| SARS2-Probe I.691 | NC_045512.2 | SARS2-Probe I | CTGATGAGGGAGAAACATTAATACGACTCACTATAGGGGTGATTGTGCAACTGTACATACAGCTAATAAATGGGATCTCATTATTAGTGATATGTACGACCCTAAGACTAAAAATGTTACAAAAGAAATGGAATTCTCGGGTGCCAAGGGGCTAAAAGACCCTTGGA |
| SARS2-Probe I.692 | NC_045512.2 | SARS2-Probe I | CTGATGAGGGAGAAACATTAATACGACTCACTATAGGGAATGGGATCTCATTATTAGTGATATGTACGACCCTAAGACTAAAAATGTTACAAAAGAAATGACTCTAAAGAGGGTTTTTTCACTTACATGGAATTCTCGGGTGCCAAGGGGCTAAAAGACCCTTGGA |
| SARS2-Probe I.693 | NC_045512.2 | SARS2-Probe I | CTGATGAGGGAGAAACATTAATACGACTCACTATAGGGACCCTAAGACTAAAAATGTTACAAAAGAAATGACTCTAAAGAGGGTTTTTTCACTTACATTTGTGGGTTTATACAACAAAAGCTAGCTCTGGAATTCTCGGGTGCCAAGGGGCTAAAAGACCCTTGGA |
| SARS2-Probe I.694 | NC_045512.2 | SARS2-Probe I | CTGATGAGGGAGAAACATTAATACGACTCACTATAGGGATGACTCTAAAGAGGGTTTTTTCACTTACATTTGTGGGTTTATACAACAAAAGCTAGCTCTTGGAGGTTCCGTGGCTATAAAGATAACAGTGGAATTCTCGGGTGCCAAGGGGCTAAAAGACCCTTGGA |
| SARS2-Probe I.695 | NC_045512.2 | SARS2-Probe I | CTGATGAGGGAGAAACATTAATACGACTCACTATAGGGTTTGTGGGTTTATACAACAAAAGCTAGCTCTTGGAGGTTCCGTGGCTATAAAGATAACAGAACATTCTTGGAATGCTGATCTTTATAAGCTGGAATTCTCGGGTGCCAAGGGGCTAAAAGACCCTTGGA |
| SARS2-Probe I.696 | NC_045512.2 | SARS2-Probe I | CTGATGAGGGAGAAACATTAATACGACTCACTATAGGGTTGGAGGTTCCGTGGCTATAAAGATAACAGAACATTCTTGGAATGCTGATCTTTATAAGCTCATGGGACACTTCGCATGGTGGACAGCCTTGGAATTCTCGGGTGCCAAGGGGCTAAAAGACCCTTGGA |
| SARS2-Probe I.697 | NC_045512.2 | SARS2-Probe I | CTGATGAGGGAGAAACATTAATACGACTCACTATAGGGAACATTCTTGGAATGCTGATCTTTATAAGCTCATGGGACACTTCGCATGGTGGACAGCCTTTGTTACTAATGTGAATGCGTCATCATCTGTGGAATTCTCGGGTGCCAAGGGGCTAAAAGACCCTTGGA |
| SARS2-Probe I.698 | NC_045512.2 | SARS2-Probe I | CTGATGAGGGAGAAACATTAATACGACTCACTATAGGGTCATGGGACACTTCGCATGGTGGACAGCCTTTGTTACTAATGTGAATGCGTCATCATCTGAAGCATTTTTAATTGGATGTAATTATCTTGTGGAATTCTCGGGTGCCAAGGGGCTAAAAGACCCTTGGA |
| SARS2-Probe I.699 | NC_045512.2 | SARS2-Probe I | CTGATGAGGGAGAAACATTAATACGACTCACTATAGGGTTGTTACTAATGTGAATGCGTCATCATCTGAAGCATTTTTAATTGGATGTAATTATCTTGGCAAACCACGCGAACAAATAGATGGTTATGTGGAATTCTCGGGTGCCAAGGGGCTAAAAGACCCTTGGA |

49

| ASO ID | Accession | Group ID | Custom Array sequence |
|---|---|---|---|
| SARS2-Probe I.700 | NC_045512.2 | SARS2-Probe I | CTGATGAGGGAGAAACATTAATACGACTCACTATAGGGAAGCATTTTTAATTGGATGTAATTATCTTGGCAAACCACGCGAACAAATAGATGGTTATGTCATGCATGCAAATTACATATTTTGGAGGATGGAATTCTCGGGTGCCAAGGGGCTAAAAGACCCTTGGA |
| SARS2-Probe I.701 | NC_045512.2 | SARS2-Probe I | CTGATGAGGGAGAAACATTAATACGACTCACTATAGGGGCAAACCACGCGAACAAATAGATGGTTATGTCATGCATGCAAATTACATATTTTGGAGGAATACAAATCCAATTCAGTTGTCTTCCTATTTGGAATTCTCGGGTGCCAAGGGGCTAAAAGACCCTTGGA |
| SARS2-Probe I.702 | NC_045512.2 | SARS2-Probe I | CTGATGAGGGAGAAACATTAATACGACTCACTATAGGGTCATGCATGCAAATTACATATTTTGGAGGAATACAAATCCAATTCAGTTGTCTTCCTATTCTTTATTTGACATGAGTAAATTTCCCCTTATGGAATTCTCGGGTGCCAAGGGGCTAAAAGACCCTTGGA |
| SARS2-Probe I.703 | NC_045512.2 | SARS2-Probe I | CTGATGAGGGAGAAACATTAATACGACTCACTATAGGGATACAAATCCAATTCAGTTGTCTTCCTATTCTTTATTTGACATGAGTAAATTTCCCCTTAAATTAAGGGGTACTGCTGTTATGTCTTTAATGGAATTCTCGGGTGCCAAGGGGCTAAAAGACCCTTGGA |
| SARS2-Probe I.704 | NC_045512.2 | SARS2-Probe I | CTGATGAGGGAGAAACATTAATACGACTCACTATAGGGCTTTATTTGACATGAGTAAATTTCCCCTTAAATTAAGGGGTACTGCTGTTATGTCTTTAAAAGAAGGTCAAATCAATGATATGATTTTATTGGAATTCTCGGGTGCCAAGGGGCTAAAAGACCCTTGGA |
| SARS2-Probe I.705 | NC_045512.2 | SARS2-Probe I | CTGATGAGGGAGAAACATTAATACGACTCACTATAGGGAATTAAGGGGTACTGCTGTTATGTCTTTAAAAGAAGGTCAAATCAATGATATGATTTTATCTCTTCTTAGTAAAGGTAGACTTATAATTATGGAATTCTCGGGTGCCAAGGGGCTAAAAGACCCTTGGA |
| SARS2-Probe I.706 | NC_045512.2 | SARS2-Probe I | CTGATGAGGGAGAAACATTAATACGACTCACTATAGGGAAGAAGGTCAAATCAATGATATGATTTTATCTCTTCTTAGTAAAGGTAGACTTATAATTAGAGAAAACAACAGAGTTGTTATTTCTAGTGTGGAATTCTCGGGTGCCAAGGGGCTAAAAGACCCTTGGA |
| SARS2-Probe II.0 | NC_045512.2 | SARS2-Probe II | TAGAAAACGATAGAAGTATAATACGACTCACTATAGGGATGTTTGTTTTTCTTGTTTTATTGCCACTAGTCTCTAGTCAGTGTGTTAATCTTACAACCAGAACTCAATTACCCCCTGCATACACTAATTGGAATTCTCGGGTGCCAAGGCAATAACAATCGCTCCAG |
| SARS2-Probe II.1 | NC_045512.2 | SARS2-Probe II | TAGAAAACGATAGAAGTATAATACGACTCACTATAGGGGTCTCTAGTCAGTGTGTTAATCTTACAACCAGAACTCAATTACCCCCTGCATACACTAATTCTTTCACACGTGGTGTTTATTACCCTGACTGGAATTCTCGGGTGCCAAGGCAATAACAATCGCTCCAG |
| SARS2-Probe II.2 | NC_045512.2 | SARS2-Probe II | TAGAAAACGATAGAAGTATAATACGACTCACTATAGGGAGAACTCAATTACCCCCTGCATACACTAATTCTTTCACACGTGGTGTTTATTACCCTGACAAAGTTTTCAGATCCTCAGTTTTACATTCATGGAATTCTCGGGTGCCAAGGCAATAACAATCGCTCCAG |
| SARS2-Probe II.3 | NC_045512.2 | SARS2-Probe II | TAGAAAACGATAGAAGTATAATACGACTCACTATAGGGTCTTTCACACGTGGTGTTTATTACCCTGACAAAGTTTTCAGATCCTCAGTTTTACATTCAACTCAGGACTTGTTCTTACCTTTCTTTTCCTGGAATTCTCGGGTGCCAAGGCAATAACAATCGCTCCAG |
| SARS2-Probe II.4 | NC_045512.2 | SARS2-Probe II | TAGAAAACGATAGAAGTATAATACGACTCACTATAGGGAAAGTTTTCAGATCCTCAGTTTTACATTCAACTCAGGACTTGTTCTTACCTTTCTTTTCCAATGTTACTTGGTTCCATGCTATACATGTCTGGAATTCTCGGGTGCCAAGGCAATAACAATCGCTCCAG |
| SARS2-Probe II.5 | NC_045512.2 | SARS2-Probe II | TAGAAAACGATAGAAGTATAATACGACTCACTATAGGGACTCAGGACTTGTTCTTACCTTTCTTTTCCAATGTTACTTGGTTCCATGCTATACATGTCTCTGGGACCAATGGTACTAAGAGGTTTGATTGGAATTCTCGGGTGCCAAGGCAATAACAATCGCTCCAG |
| SARS2-Probe II.6 | NC_045512.2 | SARS2-Probe II | TAGAAAACGATAGAAGTATAATACGACTCACTATAGGGAATGTTACTTGGTTCCATGCTATACATGTCTCTGGGACCAATGGTACTAAGAGGTTTGATAACCCTGTCCTACCATTTAATGATGGTGTTTGGAATTCTCGGGTGCCAAGGCAATAACAATCGCTCCAG |
| SARS2-Probe II.7 | NC_045512.2 | SARS2-Probe II | TAGAAAACGATAGAAGTATAATACGACTCACTATAGGGTCTGGGACCAATGGTACTAAGAGGTTTGATAACCCTGTCCTACCATTTAATGATGGTGTTTATTTTGCTTCCACTGAGAAGTCTAACATATGGAATTCTCGGGTGCCAAGGCAATAACAATCGCTCCAG |
| SARS2-Probe II.8 | NC_045512.2 | SARS2-Probe II | TAGAAAACGATAGAAGTATAATACGACTCACTATAGGGAACCCTGTCCTACCATTTAATGATGGTGTTTATTTTGCTTCCACTGAGAAGTCTAACATAATAAGAGGCTGGATTTTTGGTACTACTTTATGGAATTCTCGGGTGCCAAGGCAATAACAATCGCTCCAG |
| SARS2-Probe II.9 | NC_045512.2 | SARS2-Probe II | TAGAAAACGATAGAAGTATAATACGACTCACTATAGGGTATTTTGCTTCCACTGAGAAGTCTAACATAATAAGAGGCTGGATTTTTGGTACTACTTTAGATTCGAAGACCCAGTCCCTACTTATTGTTTGGAATTCTCGGGTGCCAAGGCAATAACAATCGCTCCAG |
| SARS2-Probe II.10 | NC_045512.2 | SARS2-Probe II | TAGAAAACGATAGAAGTATAATACGACTCACTATAGGGATAAGAGGCTGGATTTTTGGTACTACTTTAGATTCGAAGACCCAGTCCCTACTTATTGTTAATAACGCTACTAATGTTGTTATTAAAGTCTGGAATTCTCGGGTGCCAAGGCAATAACAATCGCTCCAG |
| SARS2-Probe II.11 | NC_045512.2 | SARS2-Probe II | TAGAAAACGATAGAAGTATAATACGACTCACTATAGGGGATTCGAAGACCCAGTCCCTACTTATTGTTAATAACGCTACTAATGTTGTTATTAAAGTCTGTGAATTTCAATTTTGTAATGATCCATTTTGGAATTCTCGGGTGCCAAGGCAATAACAATCGCTCCAG |
| SARS2-Probe II.12 | NC_045512.2 | SARS2-Probe II | TAGAAAACGATAGAAGTATAATACGACTCACTATAGGGAATAACGCTACTAATGTTGTTATTAAAGTCTGTGAATTTCAATTTTGTAATGATCCATTTTGGGTGTTTATTACCACAAAAACAACAAATGGAATTCTCGGGTGCCAAGGCAATAACAATCGCTCCAG |
| SARS2-Probe II.13 | NC_045512.2 | SARS2-Probe II | TAGAAAACGATAGAAGTATAATACGACTCACTATAGGGTGTGAATTTCAATTTTGTAATGATCCATTTTGGGTGTTTATTACCACAAAAACAACAAAAGTTGGATGGAAAGTGAGTTCAGAGTTTATTGGAATTCTCGGGTGCCAAGGCAATAACAATCGCTCCAG |
| SARS2-Probe II.14 | NC_045512.2 | SARS2-Probe II | TAGAAAACGATAGAAGTATAATACGACTCACTATAGGGTTGGGTGTTTATTACCACAAAAACAACAAAAGTTGGATGGAAAGTGAGTTCAGAGTTTATTCTAGTGCGAATAATTGCACTTTTGAATATTGGAATTCTCGGGTGCCAAGGCAATAACAATCGCTCCAG |
| SARS2-Probe II.15 | NC_045512.2 | SARS2-Probe II | TAGAAAACGATAGAAGTATAATACGACTCACTATAGGGAGTTGGATGGAAAGTGAGTTCAGAGTTTATTCTAGTGCGAATAATTGCACTTTTGAATATGTCTCTCAGCCTTTTCTTATGGACCTTGAATGGAATTCTCGGGTGCCAAGGCAATAACAATCGCTCCAG |
| SARS2-Probe II.16 | NC_045512.2 | SARS2-Probe II | TAGAAAACGATAGAAGTATAATACGACTCACTATAGGGTCTAGTGCGAATAATTGCACTTTTGAATATGTCTCTCAGCCTTTTCTTATGGACCTTGAAGGAAAACAGGGTAATTTCAAAAATCTTAGGTGGAATTCTCGGGTGCCAAGGCAATAACAATCGCTCCAG |
| SARS2-Probe II.17 | NC_045512.2 | SARS2-Probe II | TAGAAAACGATAGAAGTATAATACGACTCACTATAGGGGTCTCTCAGCCTTTTCTTATGGACCTTGAAGGAAAACAGGGTAATTTCAAAAATCTTAGGGAATTTGTGTTTAAGAATATTGATGGTTATTGGAATTCTCGGGTGCCAAGGCAATAACAATCGCTCCAG |

| ASO ID | Accession | Group ID | Custom Array sequence |
|---|---|---|---|
| SARS2-Probe II.18 | NC_045512.2 | SARS2-Probe II | TAGAAAACGATAGAAGTATAATACGACTCACTATAGGGGGAAAACAGGGTAATTTCAAAAATCTTAGGGAATTTGTGTTTAAGAATATTGATGGTTATTTTAAAATATATTCTAAGCACACGCCTATTTGGAATTCTCGGGTGCCAAGGCAATAACAATCGCTCCAG |
| SARS2-Probe II.19 | NC_045512.2 | SARS2-Probe II | TAGAAAACGATAGAAGTATAATACGACTCACTATAGGGGGAATTTGTGTTTAAGAATATTGATGGTTATTTTAAAATATATTCTAAGCACACGCCTATTAATTTAGTGCGTGATCTCCCTCAGGGTTTTTGGAATTCTCGGGTGCCAAGGCAATAACAATCGCTCCAG |
| SARS2-Probe II.20 | NC_045512.2 | SARS2-Probe II | TAGAAAACGATAGAAGTATAATACGACTCACTATAGGGTTTAAAATATATTCTAAGCACACGCCTATTAATTTAGTGCGTGATCTCCCTCAGGGTTTTTCGGCTTTAGAACCATTGGTAGATTTGCCATGGAATTCTCGGGTGCCAAGGCAATAACAATCGCTCCAG |
| SARS2-Probe II.21 | NC_045512.2 | SARS2-Probe II | TAGAAAACGATAGAAGTATAATACGACTCACTATAGGGAATTTAGTGCGTGATCTCCCTCAGGGTTTTTCGGCTTTAGAACCATTGGTAGATTTGCCAATAGGTATTAACATCACTAGGTTTCAAACTTGGAATTCTCGGGTGCCAAGGCAATAACAATCGCTCCAG |
| SARS2-Probe II.22 | NC_045512.2 | SARS2-Probe II | TAGAAAACGATAGAAGTATAATACGACTCACTATAGGGTCGGCTTTAGAACCATTGGTAGATTTGCCAATAGGTATTAACATCACTAGGTTTCAAACTTTACTTGCTTTACATAGAAGTTATTTGACTTGGAATTCTCGGGTGCCAAGGCAATAACAATCGCTCCAG |
| SARS2-Probe II.23 | NC_045512.2 | SARS2-Probe II | TAGAAAACGATAGAAGTATAATACGACTCACTATAGGGGATAGGTATTAACATCACTAGGTTTCAAACTTTACTTGCTTTACATAGAAGTTATTTGACTCCTGGTGATTCTTCTTCAGGTTGGACAGCTTGGAATTCTCGGGTGCCAAGGCAATAACAATCGCTCCAG |
| SARS2-Probe II.24 | NC_045512.2 | SARS2-Probe II | TAGAAAACGATAGAAGTATAATACGACTCACTATAGGGTTACTTGCTTTACATAGAAGTTATTTGACTCCTGGTGATTCTTCTTCAGGTTGGACAGCTGGTGCTGCAGCTTATTATGTGGGTTATCTTTGGAATTCTCGGGTGCCAAGGCAATAACAATCGCTCCAG |
| SARS2-Probe II.25 | NC_045512.2 | SARS2-Probe II | TAGAAAACGATAGAAGTATAATACGACTCACTATAGGGCCTGGTGATTCTTCTTCAGGTTGGACAGCTGGTGCTGCAGCTTATTATGTGGGTTATCTTCAACCTAGGACTTTTCTATTAAAATATAATTGGAATTCTCGGGTGCCAAGGCAATAACAATCGCTCCAG |
| SARS2-Probe II.26 | NC_045512.2 | SARS2-Probe II | TAGAAAACGATAGAAGTATAATACGACTCACTATAGGGGGGTGCTGCAGCTTATTATGTGGGTTATCTTCAACCTAGGACTTTTCTATTAAAATATAATGAAAATGGAACCATTACAGATGCTGTAGACTGGAATTCTCGGGTGCCAAGGCAATAACAATCGCTCCAG |
| SARS2-Probe II.27 | NC_045512.2 | SARS2-Probe II | TAGAAAACGATAGAAGTATAATACGACTCACTATAGGGCAACCTAGGACTTTTCTATTAAAATATAATGAAAATGGAACCATTACAGATGCTGTAGACTGTGCACTTGACCCTCTCTCAGAAACAAAGTGGAATTCTCGGGTGCCAAGGCAATAACAATCGCTCCAG |
| SARS2-Probe II.28 | NC_045512.2 | SARS2-Probe II | TAGAAAACGATAGAAGTATAATACGACTCACTATAGGGGGAAAATGGAACCATTACAGATGCTGTAGACTGTGCACTTGACCCTCTCTCAGAAACAAAGTGTACGTTGAAATCCTTCACTGTAGAAAAATGGAATTCTCGGGTGCCAAGGCAATAACAATCGCTCCAG |
| SARS2-Probe II.29 | NC_045512.2 | SARS2-Probe II | TAGAAAACGATAGAAGTATAATACGACTCACTATAGGGTGTGCACTTGACCCTCTCTCAGAAACAAAGTGTACGTTGAAATCCTTCACTGTAGAAAAAGGAATCTATCAAACTTCTAACTTTAGAGTCTGGAATTCTCGGGTGCCAAGGCAATAACAATCGCTCCAG |
| SARS2-Probe II.30 | NC_045512.2 | SARS2-Probe II | TAGAAAACGATAGAAGTATAATACGACTCACTATAGGGTGTACGTTGAAATCCTTCACTGTAGAAAAAGGAATCTATCAAACTTCTAACTTTAGAGTCCAACCAACAGAATCTATTGTTAGATTTCCTTGGAATTCTCGGGTGCCAAGGCAATAACAATCGCTCCAG |
| SARS2-Probe II.31 | NC_045512.2 | SARS2-Probe II | TAGAAAACGATAGAAGTATAATACGACTCACTATAGGGGGGAATCTATCAAACTTCTAACTTTAGAGTCCAACCAACAGAATCTATTGTTAGATTTCCTAATATTACAAACTTGTGCCCTTTTGGTGAATGGAATTCTCGGGTGCCAAGGCAATAACAATCGCTCCAG |
| SARS2-Probe II.32 | NC_045512.2 | SARS2-Probe II | TAGAAAACGATAGAAGTATAATACGACTCACTATAGGGCAACCAACAGAATCTATTGTTAGATTTCCTAATATTACAAACTTGTGCCCTTTTGGTGAAGTTTTTAACGCCACCAGATTTGCATCTGTTTGGAATTCTCGGGTGCCAAGGCAATAACAATCGCTCCAG |
| SARS2-Probe II.33 | NC_045512.2 | SARS2-Probe II | TAGAAAACGATAGAAGTATAATACGACTCACTATAGGGAATATTACAAACTTGTGCCCTTTTGGTGAAGTTTTTAACGCCACCAGATTTGCATCTGTTTATGCTTGGAACAGGAAGAGAATCAGCAACTGGAATTCTCGGGTGCCAAGGCAATAACAATCGCTCCAG |
| SARS2-Probe II.34 | NC_045512.2 | SARS2-Probe II | TAGAAAACGATAGAAGTATAATACGACTCACTATAGGGGTTTTTAACGCCACCAGATTTGCATCTGTTTATGCTTGGAACAGGAAGAGAATCAGCAACTGTGTTGCTGATTATTCTGTCCTATATAATTGGAATTCTCGGGTGCCAAGGCAATAACAATCGCTCCAG |
| SARS2-Probe II.35 | NC_045512.2 | SARS2-Probe II | TAGAAAACGATAGAAGTATAATACGACTCACTATAGGGTATGCTTGGAACAGGAAGAGAATCAGCAACTGTGTTGCTGATTATTCTGTCCTATATAATTCCGCATCATTTTCCACTTTTAAGTGTTATTGGAATTCTCGGGTGCCAAGGCAATAACAATCGCTCCAG |
| SARS2-Probe II.36 | NC_045512.2 | SARS2-Probe II | TAGAAAACGATAGAAGTATAATACGACTCACTATAGGGTGTGTTGCTGATTATTCTGTCCTATATAATTCCGCATCATTTTCCACTTTTAAGTGTTATGGAGTGTCTCCTACTAAATTAAATGATCTGGAATTCTCGGGTGCCAAGGCAATAACAATCGCTCCAG |
| SARS2-Probe II.37 | NC_045512.2 | SARS2-Probe II | TAGAAAACGATAGAAGTATAATACGACTCACTATAGGGTCCGCATCATTTTCCACTTTTAAGTGTTATGGAGTGTCTCCTACTAAATTAAATGATCTGCTTTACTAATGTCTATGCAGATTCATTTTGGAATTCTCGGGTGCCAAGGCAATAACAATCGCTCCAG |
| SARS2-Probe II.38 | NC_045512.2 | SARS2-Probe II | TAGAAAACGATAGAAGTATAATACGACTCACTATAGGGGGGAGTGTCTCCTACTAAATTAAATGATCTCTGCTTTACTAATGTCTATGCAGATTCATTTGTAATTAGAGGTGATGAAGTCAGACAAATCTGGAATTCTCGGGTGCCAAGGCAATAACAATCGCTCCAG |
| SARS2-Probe II.39 | NC_045512.2 | SARS2-Probe II | TAGAAAACGATAGAAGTATAATACGACTCACTATAGGGTGCTTTACTAATGTCTATGCAGATTCATTTGTAATTAGAGGTGATGAAGTCAGACAAATCGCTCCAGGGCAAACTGGAAAGATTGCTGATTGGAATTCTCGGGTGCCAAGGCAATAACAATCGCTCCAG |
| SARS2-Probe II.40 | NC_045512.2 | SARS2-Probe II | TAGAAAACGATAGAAGTATAATACGACTCACTATAGGGGTAATTAGAGGTGATGAAGTCAGACAAATCGCTCCAGGGCAAACTGGAAAGATTGCTGATTATAATTATAAATTACCAGATGATTTTCACATGGAATTCTCGGGTGCCAAGGCAATAACAATCGCTCCAG |
| SARS2-Probe II.41 | NC_045512.2 | SARS2-Probe II | TAGAAAACGATAGAAGTATAATACGACTCACTATAGGGGGCTCCAGGGCAAACTGGAAAGATTGCTGATTATAATTATAAATTACCAGATGATTTTACAGGCTGCGTTATAGCTTGGAATTCTAACAATTGGAATTCTCGGGTGCCAAGGCAATAACAATCGCTCCAG |
| SARS2-Probe II.42 | NC_045512.2 | SARS2-Probe II | TAGAAAACGATAGAAGTATAATACGACTCACTATAGGGTATAATTATAAATTACCAGATGATTTTACAGGCTGCGTTATAGCTTGGAATTCTAACAATCTTGATTCTAAGGTTGGTGGTAATTATAATTGGAATTCTCGGGTGCCAAGGCAATAACAATCGCTCCAG |

| ASO ID | Accession | Group ID | Custom Array sequence |
|---|---|---|---|
| SARS2-Probe II.43 | NC_045512.2 | SARS2-Probe II | TAGAAAACGATAGAAGTATAATACGACTCACTATAGGGGGGCTGCGTTATAGCTTGGAATTCTAACAATCTTGATTCTAAGGTTGGTGGTAATTATAATTACCTGTATAGATTGTTTAGGAAGTCTAATTGGAATTCTCGGGTGCCAAGGCAATAACAATCGCTCCAG |
| SARS2-Probe II.44 | NC_045512.2 | SARS2-Probe II | TAGAAAACGATAGAAGTATAATACGACTCACTATAGGGGCTTGATTCTAAGGTTGGTGGTAATTATAATTACCTGTATAGATTGTTTAGGAAGTCTAATCTCAAACCTTTTGAGAGAGATATTTCAACTTGGAATTCTCGGGTGCCAAGGCAATAACAATCGCTCCAG |
| SARS2-Probe II.45 | NC_045512.2 | SARS2-Probe II | TAGAAAACGATAGAAGTATAATACGACTCACTATAGGGTACCTGTATAGATTGTTTAGGAAGTCTAATCTCAAACCTTTTGAGAGAGATATTTCAACTGAAATCTATCAGGCCGGTAGCACACCTTGTTGGAATTCTCGGGTGCCAAGGCAATAACAATCGCTCCAG |
| SARS2-Probe II.46 | NC_045512.2 | SARS2-Probe II | TAGAAAACGATAGAAGTATAATACGACTCACTATAGGGCTCAAACCTTTTGAGAGAGATATTTCAACTGAAATCTATCAGGCCGGTAGCACACCTTGTAATGGTGTTGAAGGTTTTAATTGTTACTTTTGGAATTCTCGGGTGCCAAGGCAATAACAATCGCTCCAG |
| SARS2-Probe II.47 | NC_045512.2 | SARS2-Probe II | TAGAAAACGATAGAAGTATAATACGACTCACTATAGGGGAAATCTATCAGGCCGGTAGCACACCTTGTAATGGTGTTGAAGGTTTTAATTGTTACTTTCCTTTACAATCATATGGTTTCCAACCCACTTGGAATTCTCGGGTGCCAAGGCAATAACAATCGCTCCAG |
| SARS2-Probe II.48 | NC_045512.2 | SARS2-Probe II | TAGAAAACGATAGAAGTATAATACGACTCACTATAGGGGAATGGTGTTGAAGGTTTTAATTGTTACTTTCCTTTACAATCATATGGTTTCCAACCCACTAATGGTGTTGGTTACCAACCATACAGAGTATGGAATTCTCGGGTGCCAAGGCAATAACAATCGCTCCAG |
| SARS2-Probe II.49 | NC_045512.2 | SARS2-Probe II | TAGAAAACGATAGAAGTATAATACGACTCACTATAGGGCCTTTACAATCATATGGTTTCCAACCCACTAATGGTGTTGGTTACCAACCATACAGAGTAGTAGTACTTTCTTTTGAACTTCTACATGCATGGAATTCTCGGGTGCCAAGGCAATAACAATCGCTCCAG |
| SARS2-Probe II.50 | NC_045512.2 | SARS2-Probe II | TAGAAAACGATAGAAGTATAATACGACTCACTATAGGGAATGGTGTTGGTTACCAACCATACAGAGTAGTAGTACTTTCTTTTGAACTTCTACATGCACCAGCAACTGTTTGTGGACCTAAAAAGTCTTGGAATTCTCGGGTGCCAAGGCAATAACAATCGCTCCAG |
| SARS2-Probe II.51 | NC_045512.2 | SARS2-Probe II | TAGAAAACGATAGAAGTATAATACGACTCACTATAGGGGTAGTACTTTCTTTTGAACTTCTACATGCACCAGCAACTGTTTGTGGACCTAAAAAGTCTACTAATTTGGTTAAAAACAAATGTGTCAATTGGAATTCTCGGGTGCCAAGGCAATAACAATCGCTCCAG |
| SARS2-Probe II.52 | NC_045512.2 | SARS2-Probe II | TAGAAAACGATAGAAGTATAATACGACTCACTATAGGGCCAGCAACTGTTTGTGGACCTAAAAAGTCTACTAATTTGGTTAAAAACAAATGTGTCAATTCAACTTCAATGGTTTAACAGGCACAGGTTGGAATTCTCGGGTGCCAAGGCAATAACAATCGCTCCAG |
| SARS2-Probe II.53 | NC_045512.2 | SARS2-Probe II | TAGAAAACGATAGAAGTATAATACGACTCACTATAGGGACTAATTTGGTTAAAAACAAATGTGTCAATTCAACTTCAATGGTTTAACAGGCACAGGTGTTCTTACTGAGTCTAACAAAAAGTTTCTGTGTGGAATTCTCGGGTGCCAAGGCAATAACAATCGCTCCAG |
| SARS2-Probe II.54 | NC_045512.2 | SARS2-Probe II | TAGAAAACGATAGAAGTATAATACGACTCACTATAGGGTTCAACTTCAATGGTTTAACAGGCACAGGTGTTCTTACTGAGTCTAACAAAAAGTTTCTGCCTTTCCAACAATTTGGCAGAGACATTGCTTGGAATTCTCGGGTGCCAAGGCAATAACAATCGCTCCAG |
| SARS2-Probe II.55 | NC_045512.2 | SARS2-Probe II | TAGAAAACGATAGAAGTATAATACGACTCACTATAGGGGTTCTTACTGAGTCTAACAAAAAGTTTCTGCCTTTCCAACAATTTGGCAGAGACATTGCTGACACTACTGATGCTGTCCGTGATCCACAGTGGAATTCTCGGGTGCCAAGGCAATAACAATCGCTCCAG |
| SARS2-Probe II.56 | NC_045512.2 | SARS2-Probe II | TAGAAAACGATAGAAGTATAATACGACTCACTATAGGGCCTTTCCAACAATTTGGCAGAGACATTGCTGACACTACTGATGCTGTCCGTGATCCACAGACACTTGAGATTCTTGACATTACACCATGTTGGAATTCTCGGGTGCCAAGGCAATAACAATCGCTCCAG |
| SARS2-Probe II.57 | NC_045512.2 | SARS2-Probe II | TAGAAAACGATAGAAGTATAATACGACTCACTATAGGGGACACTACTGATGCTGTCCGTGATCCACAGACACTTGAGATTCTTGACATTACACCATGTTCTTTTGGTGGTGTCAGTGTTATAACACCATGGAATTCTCGGGTGCCAAGGCAATAACAATCGCTCCAG |
| SARS2-Probe II.58 | NC_045512.2 | SARS2-Probe II | TAGAAAACGATAGAAGTATAATACGACTCACTATAGGGACACTTGAGATTCTTGACATTACACCATGTTCTTTTGGTGGTGTCAGTGTTATAACACCAGGAACAAATACTTCTAACCAGGTTGCTGTTTGGAATTCTCGGGTGCCAAGGCAATAACAATCGCTCCAG |
| SARS2-Probe II.59 | NC_045512.2 | SARS2-Probe II | TAGAAAACGATAGAAGTATAATACGACTCACTATAGGGTCTTTTGGTGGTGTCAGTGTTATAACACCAGGAACAAATACTTCTAACCAGGTTGCTGTTCTTTATCAGGATGTTAACTGCACAGAAGTCTGGAATTCTCGGGTGCCAAGGCAATAACAATCGCTCCAG |
| SARS2-Probe II.60 | NC_045512.2 | SARS2-Probe II | TAGAAAACGATAGAAGTATAATACGACTCACTATAGGGGGAACAAATACTTCTAACCAGGTTGCTGTTCTTTATCAGGATGTTAACTGCACAGAAGTCCCTGTTGCTATTCATGCAGATCAACTTACTTGGAATTCTCGGGTGCCAAGGCAATAACAATCGCTCCAG |
| SARS2-Probe II.61 | NC_045512.2 | SARS2-Probe II | TAGAAAACGATAGAAGTATAATACGACTCACTATAGGGCTTTATCAGGATGTTAACTGCACAGAAGTCCCTGTTGCTATTCATGCAGATCAACTTACTCCTACTTGGCGTGTTTATTCTACAGGTTCTTGGAATTCTCGGGTGCCAAGGCAATAACAATCGCTCCAG |
| SARS2-Probe II.62 | NC_045512.2 | SARS2-Probe II | TAGAAAACGATAGAAGTATAATACGACTCACTATAGGGCCTGTTGCTATTCATGCAGATCAACTTACTCCTACTTGGCGTGTTTATTCTACAGGTTCTAATGTTTTTCAAACACGTGCAGGCTGTTTATGGAATTCTCGGGTGCCAAGGCAATAACAATCGCTCCAG |
| SARS2-Probe II.63 | NC_045512.2 | SARS2-Probe II | TAGAAAACGATAGAAGTATAATACGACTCACTATAGGGCCTACTTGGCGTGTTTATTCTACAGGTTCTAATGTTTTTCAAACACGTGCAGGCTGTTTAATAGGGGCTGAACATGTCAACAACTCATATTGGAATTCTCGGGTGCCAAGGCAATAACAATCGCTCCAG |
| SARS2-Probe II.64 | NC_045512.2 | SARS2-Probe II | TAGAAAACGATAGAAGTATAATACGACTCACTATAGGGAATGTTTTTCAAACACGTGCAGGCTGTTTAATAGGGGCTGAACATGTCAACAACTCATATGAGTGTGACATACCCATTGGTGCAGGTATATGGAATTCTCGGGTGCCAAGGCAATAACAATCGCTCCAG |
| SARS2-Probe II.65 | NC_045512.2 | SARS2-Probe II | TAGAAAACGATAGAAGTATAATACGACTCACTATAGGGGATAGGGGCTGAACATGTCAACAACTCATATGAGTGTGACATACCCATTGGTGCAGGTATATGCGCTAGTTATCAGACTCAGACTAATTCTTGGAATTCTCGGGTGCCAAGGCAATAACAATCGCTCCAG |
| SARS2-Probe II.66 | NC_045512.2 | SARS2-Probe II | TAGAAAACGATAGAAGTATAATACGACTCACTATAGGGGGAGTGTGACATACCCATTGGTGCAGGTATATGCGCTAGTTATCAGACTCAGACTAATTCTCCTCGGCGGGCACGTAGTGTAGCTAGTCAATGGAATTCTCGGGTGCCAAGGCAATAACAATCGCTCCAG |
| SARS2-Probe II.67 | NC_045512.2 | SARS2-Probe II | TAGAAAACGATAGAAGTATAATACGACTCACTATAGGGTGCGCTAGTTATCAGACTCAGACTAATTCTCCTCGGCGGGCACGTAGTGTAGCTAGTCAATCCATCATTGCCTACACTATGTCACTTGGTTGGAATTCTCGGGTGCCAAGGCAATAACAATCGCTCCAG |

| ASO ID | Accession | Group ID | Custom Array sequence |
|---|---|---|---|
| SARS2-Probe II.68 | NC_045512.2 | SARS2-Probe II | TAGAAAACGATAGAAGTATAATACGACTCACTATAGGGCCTCGGCGGGCACGTAGTGTAGCTAGTCAATCCATCATTGCCTACACTATGTCACTTGGTGCAGAAAATTCAGTTGCTTACTCTAATAACTGGAATTCTCGGGTGCCAAGGCAATAACAATCGCTCCAG |
| SARS2-Probe II.69 | NC_045512.2 | SARS2-Probe II | TAGAAAACGATAGAAGTATAATACGACTCACTATAGGGTCCATCATTGCCTACACTATGTCACTTGGTGCAGAAAATTCAGTTGCTTACTCTAATAACTCTATTGCCATACCCACAAATTTTACTATTTGGAATTCTCGGGTGCCAAGGCAATAACAATCGCTCCAG |
| SARS2-Probe II.70 | NC_045512.2 | SARS2-Probe II | TAGAAAACGATAGAAGTATAATACGACTCACTATAGGGGCAGAAAATTCAGTTGCTTACTCTAATAACTCTATTGCCATACCCACAAATTTTACTATTAGTGTTACCACAGAAATTCTACCAGTGTCTTGGAATTCTCGGGTGCCAAGGCAATAACAATCGCTCCAG |
| SARS2-Probe II.71 | NC_045512.2 | SARS2-Probe II | TAGAAAACGATAGAAGTATAATACGACTCACTATAGGGTCTATTGCCATACCCACAAATTTTACTATTAGTGTTACCACAGAAATTCTACCAGTGTCTATGACCAAGACATCAGTAGATTGTACAATGTGGAATTCTCGGGTGCCAAGGCAATAACAATCGCTCCAG |
| SARS2-Probe II.72 | NC_045512.2 | SARS2-Probe II | TAGAAAACGATAGAAGTATAATACGACTCACTATAGGGGAGTGTTACCACAGAAATTCTACCAGTGTCTATGACCAAGACATCAGTAGATTGTACAATGTACATTTGTGGTGATTCAACTGAATGCAGCTGGAATTCTCGGGTGCCAAGGCAATAACAATCGCTCCAG |
| SARS2-Probe II.73 | NC_045512.2 | SARS2-Probe II | TAGAAAACGATAGAAGTATAATACGACTCACTATAGGGATGACCAAGACATCAGTAGATTGTACAATGTACATTTGTGGTGATTCAACTGAATGCAGCAATCTTTTGTTGCAATATGGCAGTTTTTGTTGGAATTCTCGGGTGCCAAGGCAATAACAATCGCTCCAG |
| SARS2-Probe II.74 | NC_045512.2 | SARS2-Probe II | TAGAAAACGATAGAAGTATAATACGACTCACTATAGGGTACATTTGTGGTGATTCAACTGAATGCAGCAATCTTTTGTTGCAATATGGCAGTTTTTGTACACAATTAAACCGTGCTTTAACTGGAATATGGAATTCTCGGGTGCCAAGGCAATAACAATCGCTCCAG |
| SARS2-Probe II.75 | NC_045512.2 | SARS2-Probe II | TAGAAAACGATAGAAGTATAATACGACTCACTATAGGGAATCTTTTGTTGCAATATGGCAGTTTTTGTACACAATTAAACCGTGCTTTAACTGGAATAGCTGTTGAACAAGACAAAAACACCCAAGAATGGAATTCTCGGGTGCCAAGGCAATAACAATCGCTCCAG |
| SARS2-Probe II.76 | NC_045512.2 | SARS2-Probe II | TAGAAAACGATAGAAGTATAATACGACTCACTATAGGGACACAATTAAACCGTGCTTTAACTGGAATAGCTGTTGAACAAGACAAAAACACCCAAGAAGTTTTTGCACAAGTCAAACAAATTTACAAATGGAATTCTCGGGTGCCAAGGCAATAACAATCGCTCCAG |
| SARS2-Probe II.77 | NC_045512.2 | SARS2-Probe II | TAGAAAACGATAGAAGTATAATACGACTCACTATAGGGGCTGTTGAACAAGACAAAAACACCCAAGAAGTTTTTGCACAAGTCAAACAAATTTACAAAACACCACCAATTAAAGATTTTGGTGGTTTTTGGAATTCTCGGGTGCCAAGGCAATAACAATCGCTCCAG |
| SARS2-Probe II.78 | NC_045512.2 | SARS2-Probe II | TAGAAAACGATAGAAGTATAATACGACTCACTATAGGGGTTTTTGCACAAGTCAAACAAATTTACAAAACACCACCAATTAAAGATTTTGGTGGTTTTAATTTTTCACAAATATTACCAGATCCATCATGGAATTCTCGGGTGCCAAGGCAATAACAATCGCTCCAG |
| SARS2-Probe II.79 | NC_045512.2 | SARS2-Probe II | TAGAAAACGATAGAAGTATAATACGACTCACTATAGGGACACCACCAATTAAAGATTTTGGTGGTTTTAATTTTTCACAAATATTACCAGATCCATCAAAACCAAGCAAGAGGTCATTTATTGAAGATTGGAATTCTCGGGTGCCAAGGCAATAACAATCGCTCCAG |
| SARS2-Probe II.80 | NC_045512.2 | SARS2-Probe II | TAGAAAACGATAGAAGTATAATACGACTCACTATAGGGAATTTTTCACAAATATTACCAGATCCATCAAAACCAAGCAAGAGGTCATTTATTGAAGATCTACTTTTCAACAAAGTGACACTTGCAGATTGGAATTCTCGGGTGCCAAGGCAATAACAATCGCTCCAG |
| SARS2-Probe II.81 | NC_045512.2 | SARS2-Probe II | TAGAAAACGATAGAAGTATAATACGACTCACTATAGGGAAACCAAGCAAGAGGTCATTTATTGAAGATCTACTTTTCAACAAAGTGACACTTGCAGATGCTGGCTTCATCAAACAATATGGTGATTGCTGGAATTCTCGGGTGCCAAGGCAATAACAATCGCTCCAG |
| SARS2-Probe II.82 | NC_045512.2 | SARS2-Probe II | TAGAAAACGATAGAAGTATAATACGACTCACTATAGGGCTACTTTTCAACAAAGTGACACTTGCAGATGCTGGCTTCATCAAACAATATGGTGATTGCCTTGGTGATATTGCTGCTAGAGACCTCATTTGGAATTCTCGGGTGCCAAGGCAATAACAATCGCTCCAG |
| SARS2-Probe II.83 | NC_045512.2 | SARS2-Probe II | TAGAAAACGATAGAAGTATAATACGACTCACTATAGGGGCTGGCTTCATCAAACAATATGGTGATTGCCTTGGTGATATTGCTGCTAGAGACCTCATTTGTGCACAAAAGTTTAACGGCCTTACTGTTTGGAATTCTCGGGTGCCAAGGCAATAACAATCGCTCCAG |
| SARS2-Probe II.84 | NC_045512.2 | SARS2-Probe II | TAGAAAACGATAGAAGTATAATACGACTCACTATAGGGCTTGGTGATATTGCTGCTAGAGACCTCATTTGTGCACAAAAGTTTAACGGCCTTACTGTTTGCCACCTTTGCTCACAGATGAAATGATTTGGAATTCTCGGGTGCCAAGGCAATAACAATCGCTCCAG |
| SARS2-Probe II.85 | NC_045512.2 | SARS2-Probe II | TAGAAAACGATAGAAGTATAATACGACTCACTATAGGGTGTGCACAAAAGTTTAACGGCCTTACTGTTTGCCACCTTTGCTCACAGATGAAATGATTGCTCAATACACTTCTGCACTGTTAGCGGGTTGGAATTCTCGGGTGCCAAGGCAATAACAATCGCTCCAG |
| SARS2-Probe II.86 | NC_045512.2 | SARS2-Probe II | TAGAAAACGATAGAAGTATAATACGACTCACTATAGGGTTGCCACCTTTGCTCACAGATGAAATGATTGCTCAATACACTTCTGCACTGTTAGCGGGTACAATCACTTCTGGTTGGACCTTTGGTGCATGGAATTCTCGGGTGCCAAGGCAATAACAATCGCTCCAG |
| SARS2-Probe II.87 | NC_045512.2 | SARS2-Probe II | TAGAAAACGATAGAAGTATAATACGACTCACTATAGGGGCTCAATACACTTCTGCACTGTTAGCGGGTACAATCACTTCTGGTTGGACCTTTGGTGCAGGTGCTGCATTACAAATACCATTTGCTATGTGGAATTCTCGGGTGCCAAGGCAATAACAATCGCTCCAG |
| SARS2-Probe II.88 | NC_045512.2 | SARS2-Probe II | TAGAAAACGATAGAAGTATAATACGACTCACTATAGGGACAATCACTTCTGGTTGGACCTTTGGTGCAGGTGCTGCATTACAAATACCATTTGCTATGCAAATGGCTTATAGGTTTAATGGTATTGGATGGAATTCTCGGGTGCCAAGGCAATAACAATCGCTCCAG |
| SARS2-Probe II.89 | NC_045512.2 | SARS2-Probe II | TAGAAAACGATAGAAGTATAATACGACTCACTATAGGGGGTGCTGCATTACAAATACCATTTGCTATGCAAATGGCTTATAGGTTTAATGGTATTGGAGTTACACAGAATGTTCTCTATGAGAACCAATGGAATTCTCGGGTGCCAAGGCAATAACAATCGCTCCAG |
| SARS2-Probe II.90 | NC_045512.2 | SARS2-Probe II | TAGAAAACGATAGAAGTATAATACGACTCACTATAGGGCAAATGGCTTATAGGTTTAATGGTATTGGAGTTACACAGAATGTTCTCTATGAGAACCAAAAATTGATTGCCAACCAATTTAATAGTGCTTGGAATTCTCGGGTGCCAAGGCAATAACAATCGCTCCAG |
| SARS2-Probe II.91 | NC_045512.2 | SARS2-Probe II | TAGAAAACGATAGAAGTATAATACGACTCACTATAGGGGTTACACAGAATGTTCTCTATGAGAACCAAAAATTGATTGCCAACCAATTTAATAGTGCTATTGGCAAAATTCAAGACTCACTTTCTTCCTGGAATTCTCGGGTGCCAAGGCAATAACAATCGCTCCAG |
| SARS2-Probe II.92 | NC_045512.2 | SARS2-Probe II | TAGAAAACGATAGAAGTATAATACGACTCACTATAGGGAAATTGATTGCCAACCAATTTAATAGTGCTATTGGCAAAATTCAAGACTCACTTTCTTCCACAGCAAGTGCACTTGGAAAACTTCAAGATTGGAATTCTCGGGTGCCAAGGCAATAACAATCGCTCCAG |

| ASO ID | Accession | Group ID | Custom Array sequence |
|---|---|---|---|
| SARS2-Probe II.93 | NC_045512.2 | SARS2-Probe II | TAGAAAACGATAGAAGTATAATACGACTCACTATAGGGATTGGCAAAATTCAAGACTCACTTTCTTCCACAGCAAGTGCACTTGGAAAACTTCAAGATGTGGTCAACCAAAATGCACAAGCTTTAAACTGGAATTCTCGGGTGCCAAGGCAATAACAATCGCTCCAG |
| SARS2-Probe II.94 | NC_045512.2 | SARS2-Probe II | TAGAAAACGATAGAAGTATAATACGACTCACTATAGGGACAGCAAGTGCACTTGGAAAACTTCAAGATGTGGTCAACCAAAATGCACAAGCTTTAAACACGCTTGTTAAACAACTTAGCTCCAATTTTTGGAATTCTCGGGTGCCAAGGCAATAACAATCGCTCCAG |
| SARS2-Probe II.95 | NC_045512.2 | SARS2-Probe II | TAGAAAACGATAGAAGTATAATACGACTCACTATAGGGGTGGTCAACCAAAATGCACAAGCTTTAAACACGCTTGTTAAACAACTTAGCTCCAATTTTTGGTGCAATTTCAAGTGTTTTAAATGATATCTGGAATTCTCGGGTGCCAAGGCAATAACAATCGCTCCAG |
| SARS2-Probe II.96 | NC_045512.2 | SARS2-Probe II | TAGAAAACGATAGAAGTATAATACGACTCACTATAGGGACGCTTGTTAAACAACTTAGCTCCAATTTTGGTGCAATTTCAAGTGTTTTAAATGATATCCTTTCACGTCTTGACAAAGTTGAGGCTGAATGGAATTCTCGGGTGCCAAGGCAATAACAATCGCTCCAG |
| SARS2-Probe II.97 | NC_045512.2 | SARS2-Probe II | TAGAAAACGATAGAAGTATAATACGACTCACTATAGGGGGTGCAATTTCAAGTGTTTTAAATGATATCCTTTCACGTCTTGACAAAGTTGAGGCTGAAGTGCAAATTGATAGGTTGATCACAGGCAGATGGAATTCTCGGGTGCCAAGGCAATAACAATCGCTCCAG |
| SARS2-Probe II.98 | NC_045512.2 | SARS2-Probe II | TAGAAAACGATAGAAGTATAATACGACTCACTATAGGGCTTTCACGTCTTGACAAAGTTGAGGCTGAAGTGCAAATTGATAGGTTGATCACAGGCAGACTTCAAAGTTTGCAGACATATGTGACTCAATGGAATTCTCGGGTGCCAAGGCAATAACAATCGCTCCAG |
| SARS2-Probe II.99 | NC_045512.2 | SARS2-Probe II | TAGAAAACGATAGAAGTATAATACGACTCACTATAGGGGGTGCAAATTGATAGGTTGATCACAGGCAGACTTCAAAGTTTGCAGACATATGTGACTCAACAATTAATTAGAGCTGCAGAAATCAGAGCTTGGAATTCTCGGGTGCCAAGGCAATAACAATCGCTCCAG |
| SARS2-Probe II.100 | NC_045512.2 | SARS2-Probe II | TAGAAAACGATAGAAGTATAATACGACTCACTATAGGGCTTCAAAGTTTGCAGACATATGTGACTCAACAATTAATTAGAGCTGCAGAAATCAGAGCTTCTGCTAATCTTGCTGCTACTAAAATGTCATGGAATTCTCGGGTGCCAAGGCAATAACAATCGCTCCAG |
| SARS2-Probe II.101 | NC_045512.2 | SARS2-Probe II | TAGAAAACGATAGAAGTATAATACGACTCACTATAGGGCAATTAATTAGAGCTGCAGAAATCAGAGCTTCTGCTAATCTTGCTGCTACTAAAATGTCAGAGTGTGTACTTGGACAATCAAAAAGAGTTTGGAATTCTCGGGTGCCAAGGCAATAACAATCGCTCCAG |
| SARS2-Probe II.102 | NC_045512.2 | SARS2-Probe II | TAGAAAACGATAGAAGTATAATACGACTCACTATAGGGTCTGCTAATCTTGCTGCTACTAAAATGTCAGAGTGTGTACTTGGACAATCAAAAAGAGTTGATTTTTGTGGAAAAGGGCTATCATCTTATGTGGAATTCTCGGGTGCCAAGGCAATAACAATCGCTCCAG |
| SARS2-Probe II.103 | NC_045512.2 | SARS2-Probe II | TAGAAAACGATAGAAGTATAATACGACTCACTATAGGGGAGTGTGTACTTGGACAATCAAAAAGAGTTGATTTTTGTGGAAAGGGCTATCATCTTATGTCCTTCCCTCAGTCAGCACCTCATGGTGTATGGAATTCTCGGGTGCCAAGGCAATAACAATCGCTCCAG |
| SARS2-Probe II.104 | NC_045512.2 | SARS2-Probe II | TAGAAAACGATAGAAGTATAATACGACTCACTATAGGGGATTTTTGTGGAAAGGGCTATCATCTTATGTCCTTCCCTCAGTCAGCACCTCATGGTGTAGTCTTCGTCATGTGACTTATGTCCCTGCATGGAATTCTCGGGTGCCAAGGCAATAACAATCGCTCCAG |
| SARS2-Probe II.105 | NC_045512.2 | SARS2-Probe II | TAGAAAACGATAGAAGTATAATACGACTCACTATAGGGGTCCTTCCCTCAGTCAGCACCTCATGGTGTAGTCTTCGTCATGTGACTTATGTCCCTGCACAAGAAAAGAACTTCACAACTGCTCCTGCCTGGAATTCTCGGGTGCCAAGGCAATAACAATCGCTCCAG |
| SARS2-Probe II.106 | NC_045512.2 | SARS2-Probe II | TAGAAAACGATAGAAGTATAATACGACTCACTATAGGGGTCTTCGTCATGTGACTTATGTCCCTGCACAAGAAAAGAACTTCACAACTGCTCCTGCCATTTGTCATGATGGAAAAGCACACTTTCCTTGGAATTCTCGGGTGCCAAGGCAATAACAATCGCTCCAG |
| SARS2-Probe II.107 | NC_045512.2 | SARS2-Probe II | TAGAAAACGATAGAAGTATAATACGACTCACTATAGGGCAAGAAAAGAACTTCACAACTGCTCCTGCCATTTGTCATGATGGAAAAGCACACTTTCCTCGTGAAGGTGTCTTTGTTTCAAATGGCACATGGAATTCTCGGGTGCCAAGGCAATAACAATCGCTCCAG |
| SARS2-Probe II.108 | NC_045512.2 | SARS2-Probe II | TAGAAAACGATAGAAGTATAATACGACTCACTATAGGGATTTGTCATGATGGAAAAGCACACTTTCCTCGTGAAGGTGTCTTTGTTTCAAATGGCACACACTGGTTTGTAACACAAAGGAATTTTTATTGGAATTCTCGGGTGCCAAGGCAATAACAATCGCTCCAG |
| SARS2-Probe II.109 | NC_045512.2 | SARS2-Probe II | TAGAAAACGATAGAAGTATAATACGACTCACTATAGGGCGTGAAGGTGTCTTTGTTTCAAATGGCACACACTGGTTTGTAACACAAAGGAATTTTTATGAACCACAAATCATTACTACAGACAACACATGGAATTCTCGGGTGCCAAGGCAATAACAATCGCTCCAG |
| SARS2-Probe II.110 | NC_045512.2 | SARS2-Probe II | TAGAAAACGATAGAAGTATAATACGACTCACTATAGGGCACTGGTTTGTAACACAAAGGAATTTTTATGAACCACAAATCATTACTACAGACAACACATTTGTGTCTGGTAACTGTGATGTTGTAATATGGAATTCTCGGGTGCCAAGGCAATAACAATCGCTCCAG |
| SARS2-Probe II.111 | NC_045512.2 | SARS2-Probe II | TAGAAAACGATAGAAGTATAATACGACTCACTATAGGGGAACCACAAATCATTACTACAGACAACACATTTGTGTCTGGTAACTGTGATGTTGTAATAGGAATTGTCAACAACACAGTTTATGATCCTTGGAATTCTCGGGTGCCAAGGCAATAACAATCGCTCCAG |
| SARS2-Probe II.112 | NC_045512.2 | SARS2-Probe II | TAGAAAACGATAGAAGTATAATACGACTCACTATAGGGTTTGTGTCTGGTAACTGTGATGTTGTAATAGGAATTGTCAACAACACAGTTTATGATCCTTTGCAACCTGAATTAGACTCATTCAAGGAGTGGAATTCTCGGGTGCCAAGGCAATAACAATCGCTCCAG |
| SARS2-Probe II.113 | NC_045512.2 | SARS2-Probe II | TAGAAAACGATAGAAGTATAATACGACTCACTATAGGGGGAATTGTCAACAACACAGTTTATGATCCTTTGCAACCTGAATTAGACTCATTCAAGGAGGAGTTAGATAAATATTTTAAGAATCATACATGGAATTCTCGGGTGCCAAGGCAATAACAATCGCTCCAG |
| SARS2-Probe II.114 | NC_045512.2 | SARS2-Probe II | TAGAAAACGATAGAAGTATAATACGACTCACTATAGGGGTTGCAACCTGAATTAGACTCATTCAAGGAGGAGTTAGATAAATATTTTAAGAATCATACATCACCAGATGTTGATTTAGGTGACATCTCTTGGAATTCTCGGGTGCCAAGGCAATAACAATCGCTCCAG |
| SARS2-Probe II.115 | NC_045512.2 | SARS2-Probe II | TAGAAAACGATAGAAGTATAATACGACTCACTATAGGGGGAGTTAGATAAATATTTTAAGAATCATACATCACCAGATGTTGATTTAGGTGACATCTCTGGCATTAATGCTTCAGTTGTAAACATTCAATGGAATTCTCGGGTGCCAAGGCAATAACAATCGCTCCAG |
| SARS2-Probe II.116 | NC_045512.2 | SARS2-Probe II | TAGAAAACGATAGAAGTATAATACGACTCACTATAGGGTCACCAGATGTTGATTTAGGTGACATCTCTGGCATTAATGCTTCAGTTGTAAACATTCAAAAAGAAATTGACCGCCTCAATGAGGTTGCCTGGAATTCTCGGGTGCCAAGGCAATAACAATCGCTCCAG |
| SARS2-Probe II.117 | NC_045512.2 | SARS2-Probe II | TAGAAAACGATAGAAGTATAATACGACTCACTATAGGGGGCATTAATGCTTCAGTTGTAAACATTCAAAAAGAAATTGACCGCCTCAATGAGGTTGCCAAGAATTTAAATGAATCTCTCATCGATCTCTGGAATTCTCGGGTGCCAAGGCAATAACAATCGCTCCAG |

EP 4 239 083 A2

| ASO ID | Accession | Group ID | Custom Array sequence |
|---|---|---|---|
| SARS2-Probe II.118 | NC_045512.2 | SARS2-Probe II | TAGAAAACGATAGAAGTATAATACGACTCACTATAGGGAAAGAAATTGACCGCCTCAATGAGGTTGCCAAGAATTTAAATGAATCTCTCATCGATCTCCAAGAACTTGGAAAGTATGAGCAGTATATATGGAATTCTCGGGTGCCAAGGCAATAACAATCGCTCCAG |
| SARS2-Probe II.119 | NC_045512.2 | SARS2-Probe II | TAGAAAACGATAGAAGTATAATACGACTCACTATAGGGAAGAAATTTAAATGAATCTCTCATCGATCTCCAAGAACTTGGAAAGTATGAGCAGTATATAAAATGGCCATGGTACATTTGGCTAGGTTTTTGGAATTCTCGGGTGCCAAGGCAATAACAATCGCTCCAG |
| SARS2-Probe II.120 | NC_045512.2 | SARS2-Probe II | TAGAAAACGATAGAAGTATAATACGACTCACTATAGGGCAAGAACTTGGAAAGTATGAGCAGTATATAAAATGGCCATGGTACATTTGGCTAGGTTTTTATAGCTGGCTTGATTGCCATAGTAATGGTGTGGAATTCTCGGGTGCCAAGGCAATAACAATCGCTCCAG |
| SARS2-Probe II.121 | NC_045512.2 | SARS2-Probe II | TAGAAAACGATAGAAGTATAATACGACTCACTATAGGGAAATGGCCATGGTACATTTGGCTAGGTTTTTATAGCTGGCTTGATTGCCATAGTAATGGTGACAATTATGCTTTGCTGTATGACCAGTTGCTGGAATTCTCGGGTGCCAAGGCAATAACAATCGCTCCAG |
| SARS2-Probe II.122 | NC_045512.2 | SARS2-Probe II | TAGAAAACGATAGAAGTATAATACGACTCACTATAGGGATAGCTGGCTTGATTGCCATAGTAATGGTGACAATTATGCTTTGCTGTATGACCAGTTGCTGTAGTTGTCTCAAGGGCTGTTGTTCTTGTTGGAATTCTCGGGTGCCAAGGCAATAACAATCGCTCCAG |
| SARS2-Probe II.123 | NC_045512.2 | SARS2-Probe II | TAGAAAACGATAGAAGTATAATACGACTCACTATAGGGACAATTATGCTTTGCTGTATGACCAGTTGCTGTAGTTGTCTCAAGGGCTGTTGTTCTTGTGGATCCTGCTGCAAATTTGATGAAGACGACTCGGAATTCTCGGGTGCCAAGGCAATAACAATCGCTCCAG |
| SARS2-Probe II.124 | NC_045512.2 | SARS2-Probe II | TAGAAAACGATAGAAGTATAATACGACTCACTATAGGGTGTAGTTGTCTCAAGGGCTGTTGTTCTTGTGGATCCTGCTGCAAATTTGATGAAGACGACTCTGAGCCAGTGCTCAAAGGAGTCAAATTATGGAATTCTCGGGTGCCAAGGCAATAACAATCGCTCCAG |
| SARS2-Probe II.125 | NC_045512.2 | SARS2-Probe II | TAGAAAACGATAGAAGTATAATACGACTCACTATAGGGGGGATCCTGCTGCAAATTTGATGAAGACGACTCTGAGCCAGTGCTCAAAGGAGTCAAATTACATTACACATAAACGAACTTATGGATTTGTTGGAATTCTCGGGTGCCAAGGCAATAACAATCGCTCCAG |
| SARS2-Probe II.126 | NC_045512.2 | SARS2-Probe II | TAGAAAACGATAGAAGTATAATACGACTCACTATAGGGTCTGAGCCAGTGCTCAAAGGAGTCAAATTACATTACACATAAACGAACTTATGGATTTGTTTATGAGAATCTTCACAATTGGAACTGTAATGGAATTCTCGGGTGCCAAGGCAATAACAATCGCTCCAG |
| SARS2-Probe II.127 | NC_045512.2 | SARS2-Probe II | TAGAAAACGATAGAAGTATAATACGACTCACTATAGGGCATTACACATAAACGAACTTATGGATTTGTTTATGAGAATCTTCACAATTGGAACTGTAACTTTGAAGCAAGGTGAAATCAAGGATGCTATGGAATTCTCGGGTGCCAAGGCAATAACAATCGCTCCAG |
| SARS2-Probe II.128 | NC_045512.2 | SARS2-Probe II | TAGAAAACGATAGAAGTATAATACGACTCACTATAGGGTTATGAGAATCTTCACAATTGGAACTGTAACTTTGAAGCAAGGTGAAATCAAGGATGCTACTCCTTCAGATTTTGTTCGCGCTACTGCAATGGAATTCTCGGGTGCCAAGGCAATAACAATCGCTCCAG |
| SARS2-Probe II.129 | NC_045512.2 | SARS2-Probe II | TAGAAAACGATAGAAGTATAATACGACTCACTATAGGGCTTTGAAGCAAGGTGAAATCAAGGATGCTACTCCTTCAGATTTTGTTCGCGCTACTGCAACGATACCGATACAAGCCTCACTCCCTTTCGTGGAATTCTCGGGTGCCAAGGCAATAACAATCGCTCCAG |
| SARS2-Probe II.130 | NC_045512.2 | SARS2-Probe II | TAGAAAACGATAGAAGTATAATACGACTCACTATAGGGCTCCTTCAGATTTTGTTCGCGCTACTGCAACGATACCGATACAAGCCTCACTCCCTTTCGGATGGCTTATTGTTGGCGTTGCACTTCTTGTGTGGAATTCTCGGGTGCCAAGGCAATAACAATCGCTCCAG |
| SARS2-Probe II.131 | NC_045512.2 | SARS2-Probe II | TAGAAAACGATAGAAGTATAATACGACTCACTATAGGGCGATACCGATACAAGCCTCACTCCCTTTCGGATGGCTTATTGTTGGCGTTGCACTTCTTGCTGTTTTTCAGAGCGCTTCCAAAATCATAATGGAATTCTCGGGTGCCAAGGCAATAACAATCGCTCCAG |
| SARS2-Probe II.132 | NC_045512.2 | SARS2-Probe II | TAGAAAACGATAGAAGTATAATACGACTCACTATAGGGGATGGCTTATTGTTGGCGTTGCACTTCTTGCTGTTTTTCAGAGCGCTTCCAAAATCATAACCCTCAAAAAGAGATGGCAACTAGCACTCTTGGAATTCTCGGGTGCCAAGGCAATAACAATCGCTCCAG |
| SARS2-Probe II.133 | NC_045512.2 | SARS2-Probe II | TAGAAAACGATAGAAGTATAATACGACTCACTATAGGGCTGTTTTTCAGAGCGCTTCCAAAATCATAACCCTCAAAAAGAGATGGCAACTAGCACTCTCCAAGGGTGTTCACTTTGTTTGCAACTTGCTGGAATTCTCGGGTGCCAAGGCAATAACAATCGCTCCAG |
| SARS2-Probe II.134 | NC_045512.2 | SARS2-Probe II | TAGAAAACGATAGAAGTATAATACGACTCACTATAGGGCCCTCAAAAAGAGATGGCAACTAGCACTCTCCAAGGGTGTTCACTTTGTTTGCAACTTGCTGTTGTTGTTTGTAACAGTTTACTCACACCTGGAATTCTCGGGTGCCAAGGCAATAACAATCGCTCCAG |
| SARS2-Probe II.135 | NC_045512.2 | SARS2-Probe II | TAGAAAACGATAGAAGTATAATACGACTCACTATAGGGCCAAGGGTGTTCACTTTGTTTGCAACTTGCTGTTGTTGTTTGTAACAGTTTACTCACACCTTTGCTCGTTGCTGCTGGCCTTGAAGCCCTGGAATTCTCGGGTGCCAAGGCAATAACAATCGCTCCAG |
| SARS2-Probe II.136 | NC_045512.2 | SARS2-Probe II | TAGAAAACGATAGAAGTATAATACGACTCACTATAGGGTGTTGTTGTTTGTAACAGTTTACTCACACCTTTGCTCGTTGCTGCTGGCCTTGAAGCCCCTTTTCTCTATCTTTATGCTTTAGTCTACTTGGAATTCTCGGGTGCCAAGGCAATAACAATCGCTCCAG |
| SARS2-Probe II.137 | NC_045512.2 | SARS2-Probe II | TAGAAAACGATAGAAGTATAATACGACTCACTATAGGGTTTTGCTCGTTGCTGCTGGCCTTGAAGCCCCTTTTCTCTATCTTTATGCTTTAGTCTACTTCTTGCAGAGTATAAACTTTGTAAGAATAATGGAATTCTCGGGTGCCAAGGCAATAACAATCGCTCCAG |
| SARS2-Probe II.138 | NC_045512.2 | SARS2-Probe II | TAGAAAACGATAGAAGTATAATACGACTCACTATAGGGCTTTTCTCTATCTTTATGCTTTAGTCTACTTCTTGCAGAGTATAAACTTTGTAAGAATAATAATGAGGCTTTGGCTTTGCTGGAAATGCCTGGAATTCTCGGGTGCCAAGGCAATAACAATCGCTCCAG |
| SARS2-Probe II.139 | NC_045512.2 | SARS2-Probe II | TAGAAAACGATAGAAGTATAATACGACTCACTATAGGGTCTTGCAGAGTATAAACTTTGTAAGAATAATAATGAGGCTTTGGCTTTGCTGGAAATGCCGTTCCAAAAACCCATTACTTTATGATGCCATGGAATTCTCGGGTGCCAAGGCAATAACAATCGCTCCAG |
| SARS2-Probe II.140 | NC_045512.2 | SARS2-Probe II | TAGAAAACGATAGAAGTATAATACGACTCACTATAGGGTAATGAGGCTTTGGCTTTGCTGGAAATGCCGTTCCAAAAACCCATTACTTTATGATGCCAACTATTTTCTTTGCTGGCATACTAATTGTTTGGAATTCTCGGGTGCCAAGGCAATAACAATCGCTCCAG |
| SARS2-Probe II.141 | NC_045512.2 | SARS2-Probe II | TAGAAAACGATAGAAGTATAATACGACTCACTATAGGGGTTCCAAAAACCCATTACTTTATGATGCCAACTATTTTCTTTGCTGGCATACTAATTGTTACGACTATTGTATACCTTACAATAGTGTAATGGAATTCTCGGGTGCCAAGGCAATAACAATCGCTCCAG |
| SARS2-Probe II.142 | NC_045512.2 | SARS2-Probe II | TAGAAAACGATAGAAGTATAATACGACTCACTATAGGGACTATTTTCTTTGCTGGCATACTAATTGTTACGACTATTGTATACCTTACAATAGTGTAACTTCTTCAATTGTCATTACTTCAGGTGATGTGGAATTCTCGGGTGCCAAGGCAATAACAATCGCTCCAG |

| ASO ID | Accession | Group ID | Custom Array sequence |
|---|---|---|---|
| SARS2-Probe II.143 | NC_045512.2 | SARS2-Probe II | TAGAAAACGATAGAAGTATAATACGACTCACTATAGGGGACGACTATTGTATACCTTACAATAGTGTAACTTCTTCAATTGTCATTACTTCAGGTGATGGCACAACAAGTCCTATTTCTGAACATGACTTGGAATTCTCGGGTGCCAAGGCAATAACAATCGCTCCAG |
| SARS2-Probe II.144 | NC_045512.2 | SARS2-Probe II | TAGAAAACGATAGAAGTATAATACGACTCACTATAGGGGCTTCTTCAATTGTCATTACTTCAGGTGATGGCACAACAAGTCCTATTTCTGAACATGACTACCAGATTGGTGGTTATACTGAAAAATGGGTGGAATTCTCGGGTGCCAAGGCAATAACAATCGCTCCAG |
| SARS2-Probe II.145 | NC_045512.2 | SARS2-Probe II | TAGAAAACGATAGAAGTATAATACGACTCACTATAGGGGCACAACAAGTCCTATTTCTGAACATGACTACCAGATTGGTGGTTATACTGAAAAATGGGAATCTGGAGTAAAAGACTGTGTTGTATTACTGGAATTCTCGGGTGCCAAGGCAATAACAATCGCTCCAG |
| SARS2-Probe II.146 | NC_045512.2 | SARS2-Probe II | TAGAAAACGATAGAAGTATAATACGACTCACTATAGGGACCAGATTGGTGGTTATACTGAAAAATGGGAATCTGGAGTAAAAGACTGTGTTGTATTACACAGTTACTTCACTTCAGACTATTACCAGCTGGAATTCTCGGGTGCCAAGGCAATAACAATCGCTCCAG |
| SARS2-Probe II.147 | NC_045512.2 | SARS2-Probe II | TAGAAAACGATAGAAGTATAATACGACTCACTATAGGGAATCTGGAGTAAAAGACTGTGTTGTATTACACAGTTACTTCACTTCAGACTATTACCAGCTGTACTCAACTCAATTGAGTACAGACACTGTGGAATTCTCGGGTGCCAAGGCAATAACAATCGCTCCAG |
| SARS2-Probe II.148 | NC_045512.2 | SARS2-Probe II | TAGAAAACGATAGAAGTATAATACGACTCACTATAGGGACAGTTACTTCACTTCAGACTATTACCAGCTGTACTCAACTCAATTGAGTACAGACACTGGTGTTGAACATGTTACCTTCTTCATCTACATGGAATTCTCGGGTGCCAAGGCAATAACAATCGCTCCAG |
| SARS2-Probe II.149 | NC_045512.2 | SARS2-Probe II | TAGAAAACGATAGAAGTATAATACGACTCACTATAGGGTGTACTCAACTCAATTGAGTACAGACACTGGTGTTGAACATGTTACCTTCTTCATCTACAATAAAATTGTTGATGAGCCTGAAGAACATGTGGAATTCTCGGGTGCCAAGGCAATAACAATCGCTCCAG |
| SARS2-Probe II.150 | NC_045512.2 | SARS2-Probe II | TAGAAAACGATAGAAGTATAATACGACTCACTATAGGGGTGTTGAACATGTTACCTTCTTCATCTACAATAAAATTGTTGATGAGCCTGAAGAACATGTCCAAATTCACACAATCGACGGTTCATCCGTGGAATTCTCGGGTGCCAAGGCAATAACAATCGCTCCAG |
| SARS2-Probe II.151 | NC_045512.2 | SARS2-Probe II | TAGAAAACGATAGAAGTATAATACGACTCACTATAGGGATAAAATTGTTGATGAGCCTGAAGAACATGTCCAAATTCACACAATCGACGGTTCATCCGGAGTTGTTAATCCAGTAATGGAACCAATTTTGGAATTCTCGGGTGCCAAGGCAATAACAATCGCTCCAG |
| SARS2-Probe II.152 | NC_045512.2 | SARS2-Probe II | TAGAAAACGATAGAAGTATAATACGACTCACTATAGGGTCCAAATTCACACAATCGACGGTTCATCCGGAGTTGTTAATCCAGTAATGGAACCAATTTATGATGAACCGACGACGACTACTAGCGTGCTGGAATTCTCGGGTGCCAAGGCAATAACAATCGCTCCAG |
| SARS2-Probe II.153 | NC_045512.2 | SARS2-Probe II | TAGAAAACGATAGAAGTATAATACGACTCACTATAGGGGAGTTGTTAATCCAGTAATGGAACCAATTTATGATGAACCGACGACGACTACTAGCGTGCCTTTGTAAGCACAAGCTGATGAGTACGAACTGGAATTCTCGGGTGCCAAGGCAATAACAATCGCTCCAG |
| SARS2-Probe II.154 | NC_045512.2 | SARS2-Probe II | TAGAAAACGATAGAAGTATAATACGACTCACTATAGGGATGATGAACCGACGACGACTACTAGCGTGCCTTTGTAAGCACAAGCTGATGAGTACGAACTTATGTACTCATTCGTTCGGAAGAGACAGTGGAATTCTCGGGTGCCAAGGCAATAACAATCGCTCCAG |
| SARS2-Probe II.155 | NC_045512.2 | SARS2-Probe II | TAGAAAACGATAGAAGTATAATACGACTCACTATAGGGCTTTGTAAGCACAAGCTGATGAGTACGAACTTATGTACTCATTCGTTCGGAAGAGACAGGTACGTTAATAGTTAATAGCGTACTTCTTTTGGAATTCTCGGGTGCCAAGGCAATAACAATCGCTCCAG |
| SARS2-Probe II.156 | NC_045512.2 | SARS2-Probe II | TAGAAAACGATAGAAGTATAATACGACTCACTATAGGGTTATGTACTCATTCGTTCGGAAGAGACAGGTACGTTAATAGTTAATAGCGTACTTCTTTTTCTTGCTTTCGTGGTATTCTTGCTAGTTATGGAATTCTCGGGTGCCAAGGCAATAACAATCGCTCCAG |
| SARS2-Probe II.157 | NC_045512.2 | SARS2-Probe II | TAGAAAACGATAGAAGTATAATACGACTCACTATAGGGGTACGTTAATAGTTAATAGCGTACTTCTTTTTCTTGCTTTCGTGGTATTCTTGCTAGTTACACTAGCCATCCTTACTGCGCTTCGATTGTTGGAATTCTCGGGTGCCAAGGCAATAACAATCGCTCCAG |
| SARS2-Probe II.158 | NC_045512.2 | SARS2-Probe II | TAGAAAACGATAGAAGTATAATACGACTCACTATAGGGTTCTTGCTTTCGTGGTATTCTTGCTAGTTACACTAGCCATCCTTACTGCGCTTCGATTGTGTGCGTACTGCTGCAATATTGTTAACGTGATGGAATTCTCGGGTGCCAAGGCAATAACAATCGCTCCAG |
| SARS2-Probe II.159 | NC_045512.2 | SARS2-Probe II | TAGAAAACGATAGAAGTATAATACGACTCACTATAGGGCACTAGCCATCCTTACTGCGCTTCGATTGTGTGCGTACTGCTGCAATATTGTTAACGTGAGTCTTGTAAAACCTTCTTTTTACGTTTACTTGGAATTCTCGGGTGCCAAGGCAATAACAATCGCTCCAG |
| SARS2-Probe II.160 | NC_045512.2 | SARS2-Probe II | TAGAAAACGATAGAAGTATAATACGACTCACTATAGGGGTGCGTACTGCTGCAATATTGTTAACGTGAGTCTTGTAAAACCTTCTTTTTACGTTTACTCTCGTGTTAAAAATCTGAATTCTTCTAGAGTGGAATTCTCGGGTGCCAAGGCAATAACAATCGCTCCAG |
| SARS2-Probe II.161 | NC_045512.2 | SARS2-Probe II | TAGAAAACGATAGAAGTATAATACGACTCACTATAGGGTCTTGTAAAACCTTCTTTTTACGTTTACTCTCGTGTTAAAAATCTGAATTCTTCTAGAGTTCCTGATCTTCTGGTCTAAACGAACTAAATGGAATTCTCGGGTGCCAAGGCAATAACAATCGCTCCAG |
| SARS2-Probe II.162 | NC_045512.2 | SARS2-Probe II | TAGAAAACGATAGAAGTATAATACGACTCACTATAGGGCTCGTGTTAAAAATCTGAATTCTTCTAGAGTTCCTGATCTTCTGGTCTAAACGAACTAAATATTATATTAGTTTTTCTGTTTGGAACTTTTGGAATTCTCGGGTGCCAAGGCAATAACAATCGCTCCAG |
| SARS2-Probe II.163 | NC_045512.2 | SARS2-Probe II | TAGAAAACGATAGAAGTATAATACGACTCACTATAGGGTTCCTGATCTTCTGGTCTAAACGAACTAAATATTATATTAGTTTTTCTGTTTGGAACTTTAATTTTAGCCATGGCAGATTCCAACGGTACTGGAATTCTCGGGTGCCAAGGCAATAACAATCGCTCCAG |
| SARS2-Probe II.164 | NC_045512.2 | SARS2-Probe II | TAGAAAACGATAGAAGTATAATACGACTCACTATAGGGTATTATATTAGTTTTTCTGTTTGGAACTTTAATTTTAGCCATGGCAGATTCCAACGGTACTATTACCGTTGAAGAGCTTAAAAAGCTCCTTGGAATTCTCGGGTGCCAAGGCAATAACAATCGCTCCAG |
| SARS2-Probe II.165 | NC_045512.2 | SARS2-Probe II | TAGAAAACGATAGAAGTATAATACGACTCACTATAGGGAATTTTAGCCATGGCAGATTCCAACGGTACTATTACCGTTGAAGAGCTTAAAAAGCTCCTTGAACAATGGAACCTAGTAATAGGTTTCCTTGGAATTCTCGGGTGCCAAGGCAATAACAATCGCTCCAG |
| SARS2-Probe II.166 | NC_045512.2 | SARS2-Probe II | TAGAAAACGATAGAAGTATAATACGACTCACTATAGGGATTACCGTTGAAGAGCTTAAAAAGCTCCTTGAACAATGGAACCTAGTAATAGGTTTCCTATTCCTTACATGGATTTGTCTTCTACAATTTGGAATTCTCGGGTGCCAAGGCAATAACAATCGCTCCAG |
| SARS2-Probe II.167 | NC_045512.2 | SARS2-Probe II | TAGAAAACGATAGAAGTATAATACGACTCACTATAGGGTGAACAATGGAACCTAGTAATAGGTTTCCTATTCCTTACATGGATTTGTCTTCTACAATTTGCCTATGCCAACAGGAATAGGTTTTTGTATGGAATTCTCGGGTGCCAAGGCAATAACAATCGCTCCAG |

EP 4 239 083 A2

| ASO ID | Accession | Group ID | Custom Array sequence |
|---|---|---|---|
| SARS2-Probe II.168 | NC_045512.2 | SARS2-Probe II | TAGAAAACGATAGAAGTATAATACGACTCACTATAGGGATTCCTTACATGGATTTGTCTTCTACAATTTGCCTATGCCAACAGGAATAGGTTTTTGTATATAATTAAGTTAATTTTCCTCTGGCTGTTTGGAATTCTCGGGTGCCAAGGCAATAACAATCGCTCCAG |
| SARS2-Probe II.169 | NC_045512.2 | SARS2-Probe II | TAGAAAACGATAGAAGTATAATACGACTCACTATAGGGTGCCTATGCCAACAGGAATAGGTTTTTGTATATAATTAAGTTAATTTTCCTCTGGCTGTTATGGCCAGTAACTTTAGCTTGTTTTGTGCTTGGAATTCTCGGGTGCCAAGGCAATAACAATCGCTCCAG |
| SARS2-Probe II.170 | NC_045512.2 | SARS2-Probe II | TAGAAAACGATAGAAGTATAATACGACTCACTATAGGGTATAATTAAGTTAATTTTCCTCTGGCTGTTATGGCCAGTAACTTTAGCTTGTTTTGTGCTTGCTGCTGTTTACAGAATAAATTGGATCACTGGAATTCTCGGGTGCCAAGGCAATAACAATCGCTCCAG |
| SARS2-Probe II.171 | NC_045512.2 | SARS2-Probe II | TAGAAAACGATAGAAGTATAATACGACTCACTATAGGGATGGCCAGTAACTTTAGCTTGTTTTGTGCTTGCTGCTGTTTACAGAATAAATTGGATCACCGGTGGAATTGCTATCGCAATGGCTTGTCTTGGAATTCTCGGGTGCCAAGGCAATAACAATCGCTCCAG |
| SARS2-Probe II.172 | NC_045512.2 | SARS2-Probe II | TAGAAAACGATAGAAGTATAATACGACTCACTATAGGGTGCTGCTGTTTACAGAATAAATTGGATCACCGGTGGAATTGCTATCGCAATGGCTTGTCTTGTAGGCTTGATGTGGCTCAGCTACTTCATTGGAATTCTCGGGTGCCAAGGCAATAACAATCGCTCCAG |
| SARS2-Probe II.173 | NC_045512.2 | SARS2-Probe II | TAGAAAACGATAGAAGTATAATACGACTCACTATAGGGCGGTGGAATTGCTATCGCAATGGCTTGTCTTGTAGGCTTGATGTGGCTCAGCTACTTCATTGCTTCTTTCAGACTGTTTGCGCGTACGCGTGGAATTCTCGGGTGCCAAGGCAATAACAATCGCTCCAG |
| SARS2-Probe II.174 | NC_045512.2 | SARS2-Probe II | TAGAAAACGATAGAAGTATAATACGACTCACTATAGGGTGTAGGCTTGATGTGGCTCAGCTACTTCATTGCTTCTTTCAGACTGTTTGCGCGTACGCGTTCCATGTGGTCATTCAATCCAGAAACTAATGGAATTCTCGGGTGCCAAGGCAATAACAATCGCTCCAG |
| SARS2-Probe II.175 | NC_045512.2 | SARS2-Probe II | TAGAAAACGATAGAAGTATAATACGACTCACTATAGGGTGCTTCTTTCAGACTGTTTGCGCGTACGCGTTCCATGTGGTCATTCAATCCAGAAACTAACATTCTTCTCAACGTGCCACTCCATGGCACTGGAATTCTCGGGTGCCAAGGCAATAACAATCGCTCCAG |
| SARS2-Probe II.176 | NC_045512.2 | SARS2-Probe II | TAGAAAACGATAGAAGTATAATACGACTCACTATAGGGTTCCATGTGGTCATTCAATCCAGAAACTAACATTCTTCTCAACGTGCCACTCCATGGCACTATTCTGACCAGACCGCTTCTAGAAAGTGATGGAATTCTCGGGTGCCAAGGCAATAACAATCGCTCCAG |
| SARS2-Probe II.177 | NC_045512.2 | SARS2-Probe II | TAGAAAACGATAGAAGTATAATACGACTCACTATAGGGCATTCTTCTCAACGTGCCACTCCATGGCACTATTCTGACCAGACCGCTTCTAGAAAGTGAACTCGTAATCGGAGCTGTGATCCTTCGTGGTGGAATTCTCGGGTGCCAAGGCAATAACAATCGCTCCAG |
| SARS2-Probe II.178 | NC_045512.2 | SARS2-Probe II | TAGAAAACGATAGAAGTATAATACGACTCACTATAGGGTATTCTGACCAGACCGCTTCTAGAAAGTGAACTCGTAATCGGAGCTGTGATCCTTCGTGGACATCTTCGTATTGCTGGACACCATCTAGGTGGAATTCTCGGGTGCCAAGGCAATAACAATCGCTCCAG |
| SARS2-Probe II.179 | NC_045512.2 | SARS2-Probe II | TAGAAAACGATAGAAGTATAATACGACTCACTATAGGGACTCGTAATCGGAGCTGTGATCCTTCGTGGACATCTTCGTATTGCTGGACACCATCTAGGACGCTGTGACATCAAGGACCTGCCTAAAGATGGAATTCTCGGGTGCCAAGGCAATAACAATCGCTCCAG |
| SARS2-Probe II.180 | NC_045512.2 | SARS2-Probe II | TAGAAAACGATAGAAGTATAATACGACTCACTATAGGGACATCTTCGTATTGCTGGACACCATCTAGGACGCTGTGACATCAAGGACCTGCCTAAAGAAATCACTGTTGCTACATCACGAACGCTTTCTGGAATTCTCGGGTGCCAAGGCAATAACAATCGCTCCAG |
| SARS2-Probe II.181 | NC_045512.2 | SARS2-Probe II | TAGAAAACGATAGAAGTATAATACGACTCACTATAGGGACGCTGTGACATCAAGGACCTGCCTAAAGAAATCACTGTTGCTACATCACGAACGCTTTCTTATTACAAATTGGGAGCTTCGCAGCGTGTTGGAATTCTCGGGTGCCAAGGCAATAACAATCGCTCCAG |
| SARS2-Probe II.182 | NC_045512.2 | SARS2-Probe II | TAGAAAACGATAGAAGTATAATACGACTCACTATAGGGAATCACTGTTGCTACATCACGAACGCTTTCTTATTACAAATTGGGAGCTTCGCAGCGTGTAGCAGGTGACTCAGGTTTTGCTGCATACAGTGGAATTCTCGGGTGCCAAGGCAATAACAATCGCTCCAG |
| SARS2-Probe II.183 | NC_045512.2 | SARS2-Probe II | TAGAAAACGATAGAAGTATAATACGACTCACTATAGGGTTATTACAAATTGGGAGCTTCGCAGCGTGTAGCAGGTGACTCAGGTTTTGCTGCATACAGTCGCTACAGGATTGGCAACTATAAATTAAATGGAATTCTCGGGTGCCAAGGCAATAACAATCGCTCCAG |
| SARS2-Probe II.184 | NC_045512.2 | SARS2-Probe II | TAGAAAACGATAGAAGTATAATACGACTCACTATAGGGAGCAGGTGACTCAGGTTTTGCTGCATACAGTCGCTACAGGATTGGCAACTATAAATTAAACACAGACCATTCCAGTAGCAGTGACAATATTGGAATTCTCGGGTGCCAAGGCAATAACAATCGCTCCAG |
| SARS2-Probe II.185 | NC_045512.2 | SARS2-Probe II | TAGAAAACGATAGAAGTATAATACGACTCACTATAGGGTCGCTACAGGATTGGCAACTATAAATTAAACACAGACCATTCCAGTAGCAGTGACAATATTGCTTTGCTTGTACAGTAAGTGACAACAGATGGAATTCTCGGGTGCCAAGGCAATAACAATCGCTCCAG |
| SARS2-Probe II.186 | NC_045512.2 | SARS2-Probe II | TAGAAAACGATAGAAGTATAATACGACTCACTATAGGGCACAGACCATTCCAGTAGCAGTGACAATATTGCTTTGCTTGTACAGTAAGTGACAACAGATGTTTCATCTCGTTGACTTTCAGGTTACTATGGAATTCTCGGGTGCCAAGGCAATAACAATCGCTCCAG |
| SARS2-Probe II.187 | NC_045512.2 | SARS2-Probe II | TAGAAAACGATAGAAGTATAATACGACTCACTATAGGGTGCTTTGCTTGTACAGTAAGTGACAACAGATGTTTCATCTCGTTGACTTTCAGGTTACTATAGCAGAGATATTACTAATTATTATGAGGATGGAATTCTCGGGTGCCAAGGCAATAACAATCGCTCCAG |
| SARS2-Probe II.188 | NC_045512.2 | SARS2-Probe II | TAGAAAACGATAGAAGTATAATACGACTCACTATAGGGTGTTTCATCTCGTTGACTTTCAGGTTACTATAGCAGAGATATTACTAATTATTATGAGGACTTTTAAAGTTTCCATTTGGAATCTTGATTTGGAATTCTCGGGTGCCAAGGCAATAACAATCGCTCCAG |
| SARS2-Probe II.189 | NC_045512.2 | SARS2-Probe II | TAGAAAACGATAGAAGTATAATACGACTCACTATAGGGAGCAGAGATATTACTAATTATTATGAGGACTTTTAAAGTTTCCATTTGGAATCTTGATTACATCATAAACCTCATAATTAAAAATTTATTGGAATTCTCGGGTGCCAAGGCAATAACAATCGCTCCAG |
| SARS2-Probe II.190 | NC_045512.2 | SARS2-Probe II | TAGAAAACGATAGAAGTATAATACGACTCACTATAGGGCTTTTAAAGTTTCCATTTGGAATCTTGATTACATCATAAACCTCATAATTAAAAATTTATCTAAGTCACTAACTGAGAATAAATATTCTCTGGAATTCTCGGGTGCCAAGGCAATAACAATCGCTCCAG |
| SARS2-Probe II.191 | NC_045512.2 | SARS2-Probe II | TAGAAAACGATAGAAGTATAATACGACTCACTATAGGGACATCATAAACCTCATAATTAAAAATTTATCTAAGTCACTAACTGAGAATAAATATTCTCAATTAGATGAAGAGCAACCAATGGAGATTGTGGAATTCTCGGGTGCCAAGGCAATAACAATCGCTCCAG |
| SARS2-Probe II.192 | NC_045512.2 | SARS2-Probe II | TAGAAAACGATAGAAGTATAATACGACTCACTATAGGGCTAAGTCACTAACTGAGAATAAATATTCTCAATTAGATGAAGAGCAACCAATGGAGATTGATTAAACGAACATGAAAATTATTCTTTTCTTGGAATTCTCGGGTGCCAAGGCAATAACAATCGCTCCAG |

58

| ASO ID | Accession | Group ID | Custom Array sequence |
|---|---|---|---|
| SARS2-Probe II.193 | NC_045512.2 | SARS2-Probe II | TAGAAAACGATAGAAGTATAATACGACTCACTATAGGGAATTAGATGAAGAGCAACCAATGGAGATTGATTAAACGAACATGAAAATTATTCTTTTCTTGGCACTGATAACACTCGCTACTTGTGAGCTGGAATTCTCGGGTGCCAAGGCAATAACAATCGCTCCAG |
| SARS2-Probe II.194 | NC_045512.2 | SARS2-Probe II | TAGAAAACGATAGAAGTATAATACGACTCACTATAGGGATTAAACGAACATGAAAATTATTCTTTTCTTGGCACTGATAACACTCGCTACTTGTGAGCTTTATCACTACCAAGAGTGTGTTAGAGGTATGGAATTCTCGGGTGCCAAGGCAATAACAATCGCTCCAG |
| SARS2-Probe II.195 | NC_045512.2 | SARS2-Probe II | TAGAAAACGATAGAAGTATAATACGACTCACTATAGGGTGGCACTGATAACACTCGCTACTTGTGAGCTTTATCACTACCAAGAGTGTGTTAGAGGTACAACAGTACTTTTAAAAGAACCTTGCTCTTTGGAATTCTCGGGTGCCAAGGCAATAACAATCGCTCCAG |
| SARS2-Probe II.196 | NC_045512.2 | SARS2-Probe II | TAGAAAACGATAGAAGTATAATACGACTCACTATAGGGTTTATCACTACCAAGAGTGTGTTAGAGGTACAACAGTACTTTTAAAAGAACCTTGCTCTTCTGGAACATACGAGGGCAATTCACCATTTCTGGAATTCTCGGGTGCCAAGGCAATAACAATCGCTCCAG |
| SARS2-Probe II.197 | NC_045512.2 | SARS2-Probe II | TAGAAAACGATAGAAGTATAATACGACTCACTATAGGGCAACAGTACTTTTAAAAGAACCTTGCTCTTCTGGAACATACGAGGGCAATTCACCATTTCATCCTCTAGCTGATAACAAATTTGCACTGATGGAATTCTCGGGTGCCAAGGCAATAACAATCGCTCCAG |
| SARS2-Probe II.198 | NC_045512.2 | SARS2-Probe II | TAGAAAACGATAGAAGTATAATACGACTCACTATAGGGCTGGAACATACGAGGGCAATTCACCATTTCATCCTCTAGCTGATAACAAATTTGCACTGACTTGCTTTAGCACTCAATTTGCTTTTGCTTTGGAATTCTCGGGTGCCAAGGCAATAACAATCGCTCCAG |
| SARS2-Probe II.199 | NC_045512.2 | SARS2-Probe II | TAGAAAACGATAGAAGTATAATACGACTCACTATAGGGATCCTCTAGCTGATAACAAATTTGCACTGACTTGCTTTAGCACTCAATTTGCTTTTGCTTGTCCTGACGGCGTAAAACACGTCTATCAGTTGGAATTCTCGGGTGCCAAGGCAATAACAATCGCTCCAG |
| SARS2-Probe II.200 | NC_045512.2 | SARS2-Probe II | TAGAAAACGATAGAAGTATAATACGACTCACTATAGGGCTTGCTTTAGCACTCAATTTGCTTTTGCTTGTCCTGACGGCGTAAAACACGTCTATCAGTTACGTGCCAGATCAGTTTCACCTAAACTGTTGGAATTCTCGGGTGCCAAGGCAATAACAATCGCTCCAG |
| SARS2-Probe II.201 | NC_045512.2 | SARS2-Probe II | TAGAAAACGATAGAAGTATAATACGACTCACTATAGGGGTCCTGACGGCGTAAAACACGTCTATCAGTTACGTGCCAGATCAGTTTCACCTAAACTGTTCATCAGACAAGAGGAAGTTCAAGAACTTTTGGAATTCTCGGGTGCCAAGGCAATAACAATCGCTCCAG |
| SARS2-Probe II.202 | NC_045512.2 | SARS2-Probe II | TAGAAAACGATAGAAGTATAATACGACTCACTATAGGGTACGTGCCAGATCAGTTTCACCTAAACTGTTCATCAGACAAGAGGAAGTTCAAGAACTTTACTCTCCAATTTTTCTTATTGTTGCGGCAATGGAATTCTCGGGTGCCAAGGCAATAACAATCGCTCCAG |
| SARS2-Probe II.203 | NC_045512.2 | SARS2-Probe II | TAGAAAACGATAGAAGTATAATACGACTCACTATAGGGTCATCAGACAAGAGGAAGTTCAAGAACTTTACTCTCCAATTTTTCTTATTGTTGCGGCAATAGTGTTTATAACACTTTGCTTCACACTCATGGAATTCTCGGGTGCCAAGGCAATAACAATCGCTCCAG |
| SARS2-Probe II.204 | NC_045512.2 | SARS2-Probe II | TAGAAAACGATAGAAGTATAATACGACTCACTATAGGGACTCTCCAATTTTTCTTATTGTTGCGGCAATAGTGTTTATAACACTTTGCTTCACACTCAAAAGAAAGACAGAATGATTGAACTTTCATTTGGAATTCTCGGGTGCCAAGGCAATAACAATCGCTCCAG |
| SARS2-Probe II.205 | NC_045512.2 | SARS2-Probe II | TAGAAAACGATAGAAGTATAATACGACTCACTATAGGGTAGTGTTTATAACACTTTGCTTCACACTCAAAAGAAAGACAGAATGATTGAACTTTCATTAATTGACTTCTATTTGTGCTTTTTAGCCTTTGGAATTCTCGGGTGCCAAGGCAATAACAATCGCTCCAG |
| SARS2-Probe II.206 | NC_045512.2 | SARS2-Probe II | TAGAAAACGATAGAAGTATAATACGACTCACTATAGGGAAAGAAAGACAGAATGATTGAACTTTCATTAATTGACTTCTATTTGTGCTTTTTAGCCTTTCTGCTATTCCTTGTTTTAATTATGCTTATTGGAATTCTCGGGTGCCAAGGCAATAACAATCGCTCCAG |
| SARS2-Probe II.207 | NC_045512.2 | SARS2-Probe II | TAGAAAACGATAGAAGTATAATACGACTCACTATAGGGAATTGACTTCTATTTGTGCTTTTTAGCCTTTCTGCTATTCCTTGTTTTAATTATGCTTATTATCTTTTGGTTCTCACTTGAACTGCAAGATGGAATTCTCGGGTGCCAAGGCAATAACAATCGCTCCAG |
| SARS2-Probe II.208 | NC_045512.2 | SARS2-Probe II | TAGAAAACGATAGAAGTATAATACGACTCACTATAGGGTCTGCTATTCCTTGTTTTAATTATGCTTATTATCTTTTGGTTCTCACTTGAACTGCAAGATCATAATGAAACTTGTCACGCCTAAACGAATGGAATTCTCGGGTGCCAAGGCAATAACAATCGCTCCAG |
| SARS2-Probe II.209 | NC_045512.2 | SARS2-Probe II | TAGAAAACGATAGAAGTATAATACGACTCACTATAGGGTATCTTTTGGTTCTCACTTGAACTGCAAGATCATAATGAAACTTGTCACGCCTAAACGAACATGAAATTTCTTGTTTTCTTAGGAATCATTGGAATTCTCGGGTGCCAAGGCAATAACAATCGCTCCAG |
| SARS2-Probe II.210 | NC_045512.2 | SARS2-Probe II | TAGAAAACGATAGAAGTATAATACGACTCACTATAGGGTCATAATGAAACTTGTCACGCCTAAACGAACATGAAATTTCTTGTTTTCTTAGGAATCATCACAACTGTAGCTGCATTTCACCAAGAATGTGGAATTCTCGGGTGCCAAGGCAATAACAATCGCTCCAG |
| SARS2-Probe II.211 | NC_045512.2 | SARS2-Probe II | TAGAAAACGATAGAAGTATAATACGACTCACTATAGGGCATGAAATTTCTTGTTTTCTTAGGAATCATCACAACTGTAGCTGCATTTCACCAAGAATGTAGTTTACAGTCATGTACTCAACATCAACCTGGAATTCTCGGGTGCCAAGGCAATAACAATCGCTCCAG |
| SARS2-Probe II.212 | NC_045512.2 | SARS2-Probe II | TAGAAAACGATAGAAGTATAATACGACTCACTATAGGGCACAACTGTAGCTGCATTTCACCAAGAATGTAGTTTACAGTCATGTACTCAACATCAACCATATGTAGTTGATGACCCGTGTCCTATTCATGGAATTCTCGGGTGCCAAGGCAATAACAATCGCTCCAG |
| SARS2-Probe II.213 | NC_045512.2 | SARS2-Probe II | TAGAAAACGATAGAAGTATAATACGACTCACTATAGGGTAGTTTACAGTCATGTACTCAACATCAACCATATGTAGTTGATGACCCGTGTCCTATTCACTTCTATTCTAAATGGTATATTAGAGTAGGTGGAATTCTCGGGTGCCAAGGCAATAACAATCGCTCCAG |
| SARS2-Probe II.214 | NC_045512.2 | SARS2-Probe II | TAGAAAACGATAGAAGTATAATACGACTCACTATAGGGATATGTAGTTGATGACCCGTGTCCTATTCACTTCTATTCTAAATGGTATATTAGAGTAGGAGCTAGAAAATCAGCACCTTTAATTGAATTTGGAATTCTCGGGTGCCAAGGCAATAACAATCGCTCCAG |
| SARS2-Probe II.215 | NC_045512.2 | SARS2-Probe II | TAGAAAACGATAGAAGTATAATACGACTCACTATAGGGCTTCTATTCTAAATGGTATATTAGAGTAGGAGCTAGAAAATCAGCACCTTTAATTGAATTGTGCGTGGATGAGGCTGGTTCTAAATCACCTGGAATTCTCGGGTGCCAAGGCAATAACAATCGCTCCAG |
| SARS2-Probe II.216 | NC_045512.2 | SARS2-Probe II | TAGAAAACGATAGAAGTATAATACGACTCACTATAGGGGAGCTAGAAAATCAGCACCTTTAATTGAATTGTGCGTGGATGAGGCTGGTTCTAAATCACCCATTCAGTACATCGATATCGGTAATTATACTGGAATTCTCGGGTGCCAAGGCAATAACAATCGCTCCAG |
| SARS2-Probe II.217 | NC_045512.2 | SARS2-Probe II | TAGAAAACGATAGAAGTATAATACGACTCACTATAGGGGTGCGTGGATGAGGCTGGTTCTAAATCACCCATTCAGTACATCGATATCGGTAATTATACAGTTTCCTGTTTACCTTTTACAATTAATTGTGGAATTCTCGGGTGCCAAGGCAATAACAATCGCTCCAG |

| ASO ID | Accession | Group ID | Custom Array sequence |
|---|---|---|---|
| SARS2-Probe II.218 | NC_045512.2 | SARS2-Probe II | TAGAAAACGATAGAAGTATAATACGACTCACTATAGGGCATTCAGTACATCGATATCGGTAATTATACAGTTTCCTGTTTACCTTTTACAATTAATTGCCAGGAACCTAAATTGGGTAGTCTTGTAGTTGGAATTCTCGGGTGCCAAGGCAATAACAATCGCTCCAG |
| SARS2-Probe II.219 | NC_045512.2 | SARS2-Probe II | TAGAAAACGATAGAAGTATAATACGACTCACTATAGGGAGTTTCCTGTTTACCTTTTACAATTAATTGCCAGGAACCTAAATTGGGTAGTCTTGTAGTGCGTTGTTCGTTCTATGAAGACTTTTTAGATGGAATTCTCGGGTGCCAAGGCAATAACAATCGCTCCAG |
| SARS2-Probe II.220 | NC_045512.2 | SARS2-Probe II | TAGAAAACGATAGAAGTATAATACGACTCACTATAGGGCCAGGAACCTAAATTGGGTAGTCTTGTAGTGCGTTGTTCGTTCTATGAAGACTTTTTAGAGTATCATGACGTTCGTGTTGTTTTAGATTTTGGAATTCTCGGGTGCCAAGGCAATAACAATCGCTCCAG |
| SARS2-Probe II.221 | NC_045512.2 | SARS2-Probe II | TAGAAAACGATAGAAGTATAATACGACTCACTATAGGGGCGTTGTTCGTTCTATGAAGACTTTTTAGAGTATCATGACGTTCGTGTTGTTTTAGATTTCATCTAAACGAACAAACTAAAATGTCTGATTGGAATTCTCGGGTGCCAAGGCAATAACAATCGCTCCAG |
| SARS2-Probe II.222 | NC_045512.2 | SARS2-Probe II | TAGAAAACGATAGAAGTATAATACGACTCACTATAGGGGTATCATGACGTTCGTGTTGTTTTAGATTTCATCTAAACGAACAAACTAAAATGTCTGATAATGGACCCCAAAATCAGCGAAATGCACCCTGGAATTCTCGGGTGCCAAGGCAATAACAATCGCTCCAG |
| SARS2-Probe II.223 | NC_045512.2 | SARS2-Probe II | TAGAAAACGATAGAAGTATAATACGACTCACTATAGGGCATCTAAACGAACAAACTAAAATGTCTGATAATGGACCCCAAAATCAGCGAAATGCACCCCGCATTACGTTTGGTGGACCCTCAGATTCATGGAATTCTCGGGTGCCAAGGCAATAACAATCGCTCCAG |
| SARS2-Probe II.224 | NC_045512.2 | SARS2-Probe II | TAGAAAACGATAGAAGTATAATACGACTCACTATAGGGAATGGACCCCAAAATCAGCGAAATGCACCCCGCATTACGTTTGGTGGACCCTCAGATTCAACTGGCAGTAACCAGAATGGAGAACGCAGTTGGAATTCTCGGGTGCCAAGGCAATAACAATCGCTCCAG |
| SARS2-Probe II.225 | NC_045512.2 | SARS2-Probe II | TAGAAAACGATAGAAGTATAATACGACTCACTATAGGGCGCATTACGTTTGGTGGACCCTCAGATTCAACTGGCAGTAACCAGAATGGAGAACGCAGTGGGGCGCGATCAAAACAACGTCGGCCCCAATGGAATTCTCGGGTGCCAAGGCAATAACAATCGCTCCAG |
| SARS2-Probe II.226 | NC_045512.2 | SARS2-Probe II | TAGAAAACGATAGAAGTATAATACGACTCACTATAGGGACTGGCAGTAACCAGAATGGAGAACGCAGTGGGGCGCGATCAAAACAACGTCGGCCCCAAGGTTTACCCAATAATACTGCGTCTTGGTTCTGGAATTCTCGGGTGCCAAGGCAATAACAATCGCTCCAG |
| SARS2-Probe II.227 | NC_045512.2 | SARS2-Probe II | TAGAAAACGATAGAAGTATAATACGACTCACTATAGGGGGGGCGCGATCAAAACAACGTCGGCCCCAAGGTTTACCCAATAATACTGCGTCTTGGTTCACCGCTCTCACTCAACATGGCAAGGAAGACTGGAATTCTCGGGTGCCAAGGCAATAACAATCGCTCCAG |
| SARS2-Probe II.228 | NC_045512.2 | SARS2-Probe II | TAGAAAACGATAGAAGTATAATACGACTCACTATAGGGGGTTTACCCAATAATACTGCGTCTTGGTTCACCGCTCTCACTCAACATGGCAAGGAAGACCTTAAATTCCCTCGAGGACAAGGCGTTCCATGGAATTCTCGGGTGCCAAGGCAATAACAATCGCTCCAG |
| SARS2-Probe II.229 | NC_045512.2 | SARS2-Probe II | TAGAAAACGATAGAAGTATAATACGACTCACTATAGGGACCGCTCTCACTCAACATGGCAAGGAAGACCTTAAATTCCCTCGAGGACAAGGCGTTCCAATTAACACCAATAGCAGTCCAGATGACCAATGGAATTCTCGGGTGCCAAGGCAATAACAATCGCTCCAG |
| SARS2-Probe II.230 | NC_045512.2 | SARS2-Probe II | TAGAAAACGATAGAAGTATAATACGACTCACTATAGGGCTTAAATTCCCTCGAGGACAAGGCGTTCCAATTAACACCAATAGCAGTCCAGATGACCAAATTGGCTACTACCGAAGAGCTACCAGACGATGGAATTCTCGGGTGCCAAGGCAATAACAATCGCTCCAG |
| SARS2-Probe II.231 | NC_045512.2 | SARS2-Probe II | TAGAAAACGATAGAAGTATAATACGACTCACTATAGGGATTAACACCAATAGCAGTCCAGATGACCAAATTGGCTACTACCGAAGAGCTACCAGACGAATTCGTGGTGGTGACGGTAAAATGAAAGATTGGAATTCTCGGGTGCCAAGGCAATAACAATCGCTCCAG |
| SARS2-Probe II.232 | NC_045512.2 | SARS2-Probe II | TAGAAAACGATAGAAGTATAATACGACTCACTATAGGGATTGGCTACTACCGAAGAGCTACCAGACGAATTCGTGGTGGTGACGGTAAAATGAAAGATCTCAGTCCAAGATGGTATTTCTACTACCTATGGAATTCTCGGGTGCCAAGGCAATAACAATCGCTCCAG |
| SARS2-Probe II.233 | NC_045512.2 | SARS2-Probe II | TAGAAAACGATAGAAGTATAATACGACTCACTATAGGGATTCGTGGTGGTGACGGTAAAATGAAAGATCTCAGTCCAAGATGGTATTTCTACTACCTAGGAACTGGGCCAGAAGCTGGACTTCCCTATTGGAATTCTCGGGTGCCAAGGCAATAACAATCGCTCCAG |
| SARS2-Probe II.234 | NC_045512.2 | SARS2-Probe II | TAGAAAACGATAGAAGTATAATACGACTCACTATAGGGCTCAGTCCAAGATGGTATTTCTACTACCTAGGAACTGGGCCAGAAGCTGGACTTCCCTATGGTGCTAACAAAGACGGCATCATATGGGTTTGGAATTCTCGGGTGCCAAGGCAATAACAATCGCTCCAG |
| SARS2-Probe II.235 | NC_045512.2 | SARS2-Probe II | TAGAAAACGATAGAAGTATAATACGACTCACTATAGGGGGAACTGGGCCAGAAGCTGGACTTCCCTATGGTGCTAACAAAGACGGCATCATATGGGTTGCAACTGAGGGGAGCCTTGAATACACCAAAATGGAATTCTCGGGTGCCAAGGCAATAACAATCGCTCCAG |
| SARS2-Probe II.236 | NC_045512.2 | SARS2-Probe II | TAGAAAACGATAGAAGTATAATACGACTCACTATAGGGGGTGCTAACAAAGACGGCATCATATGGGTTGCAACTGAGGGGAGCCTTGAATACACCAAAAGATCACATTGGCACCCGCAATCCTGCTAACTGGAATTCTCGGGTGCCAAGGCAATAACAATCGCTCCAG |
| SARS2-Probe II.237 | NC_045512.2 | SARS2-Probe II | TAGAAAACGATAGAAGTATAATACGACTCACTATAGGGGCAACTGAGGGAGCCTTGAATACACCAAAAGATCACATTGGCACCCGCAATCCTGCTAACAATGCTGCAATCGTGCTACAACTTCCTCAATGGAATTCTCGGGTGCCAAGGCAATAACAATCGCTCCAG |
| SARS2-Probe II.238 | NC_045512.2 | SARS2-Probe II | TAGAAAACGATAGAAGTATAATACGACTCACTATAGGGGGATCACATTGGCACCCGCAATCCTGCTAACAATGCTGCAATCGTGCTACAACTTCCTCAAGGAACAACATTGCCAAAAGGCTTCTACGCATGGAATTCTCGGGTGCCAAGGCAATAACAATCGCTCCAG |
| SARS2-Probe II.239 | NC_045512.2 | SARS2-Probe II | TAGAAAACGATAGAAGTATAATACGACTCACTATAGGGAATGCTGCAATCGTGCTACAACTTCCTCAAGGAACAACATTGCCAAAAGGCTTCTACGCAGAAGGGAGCAGAGGCGGCAGTCAAGCCTCTTGGAATTCTCGGGTGCCAAGGCAATAACAATCGCTCCAG |
| SARS2-Probe II.240 | NC_045512.2 | SARS2-Probe II | TAGAAAACGATAGAAGTATAATACGACTCACTATAGGGGGGAACAACATTGCCAAAAGGCTTCTACGCAGAAGGGAGCAGAGGCGGCAGTCAAGCCTCTTCTCGTTCCTCATCACGTAGTCGCAACAGTTGGAATTCTCGGGTGCCAAGGCAATAACAATCGCTCCAG |
| SARS2-Probe II.241 | NC_045512.2 | SARS2-Probe II | TAGAAAACGATAGAAGTATAATACGACTCACTATAGGGAAGGGAGCAGAGGCGGCAGTCAAGCCTCTTCTCGTTCCTCATCACGTAGTCGCAACAGTTCAAGAAATTCAACTCCAGGCAGCAGTAGGTGGAATTCTCGGGTGCCAAGGCAATAACAATCGCTCCAG |
| SARS2-Probe II.242 | NC_045512.2 | SARS2-Probe II | TAGAAAACGATAGAAGTATAATACGACTCACTATAGGGTCTCGTTCCTCATCACGTAGTCGCAACAGTTCAAGAAATTCAACTCCAGGCAGCAGTAGGGGAACTTCTCCTGCTAGAATGGCTGGCAATTGGAATTCTCGGGTGCCAAGGCAATAACAATCGCTCCAG |

| ASO ID | Accession | Group ID | Custom Array sequence |
|---|---|---|---|
| SARS2-Probe II.243 | NC_045512.2 | SARS2-Probe II | TAGAAAACGATAGAAGTATAATACGACTCACTATAGGGTCAAGAAATTCAACTCCAGGCAGCAGTAGGGGAACTTCTCCTGCTAGAATGGCTGGCAATGGCGGTGATGCTGCTCTTGCTTTGCTGCTGTGGAATTCTCGGGTGCCAAGGCAATAACAATCGCTCCAG |
| SARS2-Probe II.244 | NC_045512.2 | SARS2-Probe II | TAGAAAACGATAGAAGTATAATACGACTCACTATAGGGGGAACTTCTCCTGCTAGAATGGCTGGCAATGGCGGTGATGCTGCTCTTGCTTTGCTGCTGCTTGACAGATTGAACCAGCTTGAGAGCAAATGGAATTCTCGGGTGCCAAGGCAATAACAATCGCTCCAG |
| SARS2-Probe II.245 | NC_045512.2 | SARS2-Probe II | TAGAAAACGATAGAAGTATAATACGACTCACTATAGGGGGCGGTGATGCTGCTCTTGCTTTGCTGCTGCTTGACAGATTGAACCAGCTTGAGAGCAAAATGTCTGGTAAAGGCCAACAACAACAAGGCTGGAATTCTCGGGTGCCAAGGCAATAACAATCGCTCCAG |
| SARS2-Probe II.246 | NC_045512.2 | SARS2-Probe II | TAGAAAACGATAGAAGTATAATACGACTCACTATAGGGCTTGACAGATTGAACCAGCTTGAGAGCAAAATGTCTGGTAAAGGCCAACAACAACAAGGCCAAACTGTCACTAAGAAATCTGCTGCTGAGTGGAATTCTCGGGTGCCAAGGCAATAACAATCGCTCCAG |
| SARS2-Probe II.247 | NC_045512.2 | SARS2-Probe II | TAGAAAACGATAGAAGTATAATACGACTCACTATAGGGATGTCTGGTAAAGGCCAACAACAACAAGGCCAAACTGTCACTAAGAAATCTGCTGCTGAGGCTTCTAAGAAGCCTCGGCAAAAACGTACTTGGAATTCTCGGGTGCCAAGGCAATAACAATCGCTCCAG |
| SARS2-Probe II.248 | NC_045512.2 | SARS2-Probe II | TAGAAAACGATAGAAGTATAATACGACTCACTATAGGGCAAACTGTCACTAAGAAATCTGCTGCTGAGGCTTCTAAGAAGCCTCGGCAAAAACGTACTGCCACTAAAGCATACAATGTAACACAAGCTTGGAATTCTCGGGTGCCAAGGCAATAACAATCGCTCCAG |
| SARS2-Probe II.249 | NC_045512.2 | SARS2-Probe II | TAGAAAACGATAGAAGTATAATACGACTCACTATAGGGGCTTCTAAGAAGCCTCGGCAAAAACGTACTGCCACTAAAGCATACAATGTAACACAAGCTTTCGGCAGACGTGGTCCAGAACAAACCCAATGGAATTCTCGGGTGCCAAGGCAATAACAATCGCTCCAG |
| SARS2-Probe II.250 | NC_045512.2 | SARS2-Probe II | TAGAAAACGATAGAAGTATAATACGACTCACTATAGGGGCCCACTAAAGCATACAATGTAACACAAGCTTTCGGCAGACGTGGTCCAGAACAAACCCAAGGAAATTTTGGGGACCAGGAACTAATCAGATGGAATTCTCGGGTGCCAAGGCAATAACAATCGCTCCAG |
| SARS2-Probe II.251 | NC_045512.2 | SARS2-Probe II | TAGAAAACGATAGAAGTATAATACGACTCACTATAGGGTTCGGCAGACGTGGTCCAGAACAAACCCAAGGAAATTTTGGGGACCAGGAACTAATCAGACAAGGAACTGATTACAAACATTGGCCGCAATGGAATTCTCGGGTGCCAAGGCAATAACAATCGCTCCAG |
| SARS2-Probe II.252 | NC_045512.2 | SARS2-Probe II | TAGAAAACGATAGAAGTATAATACGACTCACTATAGGGGGAAATTTTGGGGACCAGGAACTAATCAGACAAGGAACTGATTACAAACATTGGCCGCAAATTGCACAATTTGCCCCCAGCGCTTCAGCGTGGAATTCTCGGGTGCCAAGGCAATAACAATCGCTCCAG |
| SARS2-Probe II.253 | NC_045512.2 | SARS2-Probe II | TAGAAAACGATAGAAGTATAATACGACTCACTATAGGGCAAGGAACTGATTACAAACATTGGCCGCAAATTGCACAATTTGCCCCCAGCGCTTCAGCGTTCTTCGGAATGTCGCGCATTGGCATGGAATGGAATTCTCGGGTGCCAAGGCAATAACAATCGCTCCAG |
| SARS2-Probe II.254 | NC_045512.2 | SARS2-Probe II | TAGAAAACGATAGAAGTATAATACGACTCACTATAGGGATTGCACAATTTGCCCCCAGCGCTTCAGCGTTCTTCGGAATGTCGCGCATTGGCATGGAAGTCACACCTTCGGGAACGTGGTTGACCTACTGGAATTCTCGGGTGCCAAGGCAATAACAATCGCTCCAG |
| SARS2-Probe II.255 | NC_045512.2 | SARS2-Probe II | TAGAAAACGATAGAAGTATAATACGACTCACTATAGGGTTCTTCGGAATGTCGCGCATTGGCATGGAAGTCACACCTTCGGGAACGTGGTTGACCTACACAGGTGCCATCAAATTGGATGACAAAGATTGGAATTCTCGGGTGCCAAGGCAATAACAATCGCTCCAG |
| SARS2-Probe II.256 | NC_045512.2 | SARS2-Probe II | TAGAAAACGATAGAAGTATAATACGACTCACTATAGGGGTCACACCTTCGGGAACGTGGTTGACCTACACAGGTGCCATCAAATTGGATGACAAAGATCCAAATTTCAAAGATCAAGTCATTTTGCTGTGGAATTCTCGGGTGCCAAGGCAATAACAATCGCTCCAG |
| SARS2-Probe II.257 | NC_045512.2 | SARS2-Probe II | TAGAAAACGATAGAAGTATAATACGACTCACTATAGGGACAGGTGCCATCAAATTGGATGACAAAGATCCAAATTTCAAAGATCAAGTCATTTTGCTGAATAAGCATATTGACGCATACAAAACATTCTGGAATTCTCGGGTGCCAAGGCAATAACAATCGCTCCAG |
| SARS2-Probe II.258 | NC_045512.2 | SARS2-Probe II | TAGAAAACGATAGAAGTATAATACGACTCACTATAGGGCCAAATTTCAAAGATCAAGTCATTTTGCTGAATAAGCATATTGACGCATACAAAACATTCCCACCAACAGAGCCTAAAAAGGACAAAAAGTGGAATTCTCGGGTGCCAAGGCAATAACAATCGCTCCAG |
| SARS2-Probe II.259 | NC_045512.2 | SARS2-Probe II | TAGAAAACGATAGAAGTATAATACGACTCACTATAGGGAATAAGCATATTGACGCATACAAAACATTCCCACCAACAGAGCCTAAAAAGGACAAAAAGAAGAAGGCTGATGAAACTCAAGCCTTACCGTGGAATTCTCGGGTGCCAAGGCAATAACAATCGCTCCAG |
| SARS2-Probe II.260 | NC_045512.2 | SARS2-Probe II | TAGAAAACGATAGAAGTATAATACGACTCACTATAGGGCCACCAACAGAGCCTAAAAAGGACAAAAAGAAGAAGGCTGATGAAACTCAAGCCTTACCGCAGAGACAGAAGAAACAGCAAACTGTGACTTGGAATTCTCGGGTGCCAAGGCAATAACAATCGCTCCAG |
| SARS2-Probe II.261 | NC_045512.2 | SARS2-Probe II | TAGAAAACGATAGAAGTATAATACGACTCACTATAGGGAAGAAGGCTGATGAAACTCAAGCCTTACCGCAGAGACAGAAGAAACAGCAAACTGTGACTCTTCTTCCTGCTGCAGATTTGGATGATTTCTGGAATTCTCGGGTGCCAAGGCAATAACAATCGCTCCAG |
| SARS2-Probe II.262 | NC_045512.2 | SARS2-Probe II | TAGAAAACGATAGAAGTATAATACGACTCACTATAGGGCAGAGACAGAAGAAACAGCAAACTGTGACTCTTCTTCCTGCTGCAGATTTGGATGATTTCTCCAAACAATTGCAACAATCCATGAGCAGTTGGAATTCTCGGGTGCCAAGGCAATAACAATCGCTCCAG |
| SARS2-Probe II.263 | NC_045512.2 | SARS2-Probe II | TAGAAAACGATAGAAGTATAATACGACTCACTATAGGGCTTCTTCCTGCTGCAGATTTGGATGATTTCTCCAAACAATTGCAACAATCCATGAGCAGTGCTGACTCAACTCAGGCCTAAACTCATGCATGGAATTCTCGGGTGCCAAGGCAATAACAATCGCTCCAG |
| SARS2-Probe II.264 | NC_045512.2 | SARS2-Probe II | TAGAAAACGATAGAAGTATAATACGACTCACTATAGGGTCCAAACAATTGCAACAATCCATGAGCAGTGCTGACTCAACTCAGGCCTAAACTCATGCAGACCACACAAGGCAGATGGGCTATATAAACTGGAATTCTCGGGTGCCAAGGCAATAACAATCGCTCCAG |
| SARS2-Probe II.265 | NC_045512.2 | SARS2-Probe II | TAGAAAACGATAGAAGTATAATACGACTCACTATAGGGGCTGACTCAACTCAGGCCTAAACTCATGCAGACCACACAAGGCAGATGGGCTATATAAACGTTTTCGCTTTTCCGTTTACGATATATAGTTGGAATTCTCGGGTGCCAAGGCAATAACAATCGCTCCAG |
| SARS2-Probe II.266 | NC_045512.2 | SARS2-Probe II | TAGAAAACGATAGAAGTATAATACGACTCACTATAGGGGACCACACAAGGCAGATGGGCTATATAAACGTTTTCGCTTTTCCGTTTACGATATATAGTCTACTCTTGTGCAGAATGAATTCTCGTAACTGGAATTCTCGGGTGCCAAGGCAATAACAATCGCTCCAG |
| SARS2-Probe II.267 | NC_045512.2 | SARS2-Probe II | TAGAAAACGATAGAAGTATAATACGACTCACTATAGGGGTTTTCGCTTTTCCGTTTACGATATATAGTCTACTCTTGTGCAGAATGAATTCTCGTAACTACATAGCACAAGTAGATGTAGTTAACTTTTGGAATTCTCGGGTGCCAAGGCAATAACAATCGCTCCAG |

| ASO ID | Accession | Group ID | Custom Array sequence |
|---|---|---|---|
| SARS2-Probe II.268 | NC_045512.2 | SARS2-Probe II | TAGAAAACGATAGAAGTATAATACGACTCACTATAGGGCTACTCTTGTGCAGAATGAATTCTCGTAACTACATAGCACAAGTAGATGTAGTTAACTTTAATCTCACATAGCAATCTTTAATCAGTGTGTGGAATTCTCGGGTGCCAAGGCAATAACAATCGCTCCAG |
| SARS2-Probe II.269 | NC_045512.2 | SARS2-Probe II | TAGAAAACGATAGAAGTATAATACGACTCACTATAGGGTACATAGCACAAGTAGATGTAGTTAACTTTAATCTCACATAGCAATCTTTAATCAGTGTGTAACATTAGGGAGGACTTGAAAGAGCCACCTGGAATTCTCGGGTGCCAAGGCAATAACAATCGCTCCAG |
| SARS2-Probe II.270 | NC_045512.2 | SARS2-Probe II | TAGAAAACGATAGAAGTATAATACGACTCACTATAGGGAATCTCACATAGCAATCTTTAATCAGTGTGTAACATTAGGGAGGACTTGAAAGAGCCACCACATTTTCACCGAGGCCACGCGGAGTACGATGGAATTCTCGGGTGCCAAGGCAATAACAATCGCTCCAG |
| SARS2-Probe II.271 | NC_045512.2 | SARS2-Probe II | TAGAAAACGATAGAAGTATAATACGACTCACTATAGGGTAACATTAGGGAGGACTTGAAAGAGCCACCACATTTTCACCGAGGCCACGCGGAGTACGATCGAGTGTACAGTGAACAATGCTAGGGAGATGGAATTCTCGGGTGCCAAGGCAATAACAATCGCTCCAG |
| SARS2-Probe II.272 | NC_045512.2 | SARS2-Probe II | TAGAAAACGATAGAAGTATAATACGACTCACTATAGGGACATTTTCACCGAGGCCACGCGGAGTACGATCGAGTGTACAGTGAACAATGCTAGGGAGAGCTGCCTATATGGAAGAGCCCTAATGTGTATGGAATTCTCGGGTGCCAAGGCAATAACAATCGCTCCAG |
| SARS2-Probe II.273 | NC_045512.2 | SARS2-Probe II | TAGAAAACGATAGAAGTATAATACGACTCACTATAGGGTCGAGTGTACAGTGAACAATGCTAGGGAGAGCTGCCTATATGGAAGAGCCCTAATGTGTAAAATTAATTTTAGTAGTGCTATCCCCATGTTGATTTTAATAGCTTCTTAGGAGAATGACAATGGAATTCTCGGGTGCCAAGGCAATAACAATCGCTCCAG |
| SARS2-Probe II.274 | NC_045512.2 | SARS2-Probe II | TAGAAAACGATAGAAGTATAATACGACTCACTATAGGGGCTGCCTATATGGAAGAGCCCTAATGTGTAAAATTAATTTTAGTAGTGCTATCCCCATGTGATTTTAATAGCTTCTTAGGAGAATGACAATGGAATTCTCGGGTGCCAAGGCAATAACAATCGCTCCAG |
| OC43-Probe I.0 | AY391777.1 | OC43-Probe I | TGTGAGAGATTCCCTTCGTAATACGACTCACTATAGGGGATTGTGAGCGATTTGCGTGCGTGCATCCCGCTTCACTGATCTCTTGTTAGATCTTTTTGTAATCTAAACTTTATAAAAACATCCACTCCTGGAATTCTCGGGTGCCAAGGTACCAAGAAACCTTCATT |
| OC43-Probe I.1 | AY391777.1 | OC43-Probe I | TGTGAGAGATTCCCTTCGTAATACGACTCACTATAGGGGCTTCACTGATCTCTTGTTAGATCTTTTTGTAATCTAAACTTTATAAAAACATCCACTCCCTGTAATCTATGCTTGTGGGCGTAGATTTTTGGAATTCTCGGGTGCCAAGGTACCAAGAAACCTTCATT |
| OC43-Probe I.2 | AY391777.1 | OC43-Probe I | TGTGAGAGATTCCCTTCGTAATACGACTCACTATAGGGTAATCTAAACTTTATAAAAACATCCACTCCCTGTAATCTATGCTTGTGGGCGTAGATTTTTCATAGTGGTGTTTATATTCATTTCTGCTGTGGAATTCTCGGGTGCCAAGGTACCAAGAAACCTTCATT |
| OC43-Probe I.3 | AY391777.1 | OC43-Probe I | TGTGAGAGATTCCCTTCGTAATACGACTCACTATAGGGCGTGAATCTATGCTTGTGGGCGTAGATTTTTCATAGTGGTGTTTATATTCATTTCTGCTGTTAACAGCTTTCAGCCAGGGACGTGTTGTATGGAATTCTCGGGTGCCAAGGTACCAAGAAACCTTCATT |
| OC43-Probe I.4 | AY391777.1 | OC43-Probe I | TGTGAGAGATTCCCTTCGTAATACGACTCACTATAGGGTCATAGTGGTGTTTATATTCATTTCTGCTGTTAACAGCTTTCAGCCAGGGACGTGTTGTATCCTAGGCAGTGGCCCGCCCATAGGTCACATGGAATTCTCGGGTGCCAAGGTACCAAGAAACCTTCATT |
| OC43-Probe I.5 | AY391777.1 | OC43-Probe I | TGTGAGAGATTCCCTTCGTAATACGACTCACTATAGGGTTAACAGCTTTCAGCCAGGGACGTGTTGTATCCTAGGCAGTGGCCCGCCCATAGGTCACAATGTCGAAGATCAACAAATACGGTCTCGAATGGAATTCTCGGGTGCCAAGGTACCAAGAAACCTTCATT |
| OC43-Probe I.6 | AY391777.1 | OC43-Probe I | TGTGAGAGATTCCCTTCGTAATACGACTCACTATAGGGTCCTAGGCAGTGGCCCGCCCATAGGTCACAATGTCGAAGATCAACAAATACGGTCTCGAACTACACTGGGCTCCAGAATTTCCATGGATGTGGAATTCTCGGGTGCCAAGGTACCAAGAAACCTTCATT |
| OC43-Probe I.7 | AY391777.1 | OC43-Probe I | TGTGAGAGATTCCCTTCGTAATACGACTCACTATAGGGGATGTCGAAGATCAACAAATACGGTCTCGAACTACACTGGGCTCCAGAATTTCCATGGATGTTTGAGGACGCAGAGGAAGTGGATAACTGGAATTCTCGGGTGCCAAGGTACCAAGAAACCTTCATT |
| OC43-Probe I.8 | AY391777.1 | OC43-Probe I | TGTGAGAGATTCCCTTCGTAATACGACTCACTATAGGGCTACACTGGGCTCCAGAATTTCCATGGATGTTTGAGGACGCAGAGGAGAAGTTGGATAACCCTAGTAGTTCAGAGGTGGATATGATTTGCTGGAATTCTCGGGTGCCAAGGTACCAAGAAACCTTCATT |
| OC43-Probe I.9 | AY391777.1 | OC43-Probe I | TGTGAGAGATTCCCTTCGTAATACGACTCACTATAGGGTTTGAGGACGCAGAGGAGAAGTTGGATAACCCTAGTAGTTCAGAGGTGGATATGATTTGCTCCACCACTGCGCAAAAGCTGGAAACAGACTGGAATTCTCGGGTGCCAAGGTACCAAGAAACCTTCATT |
| OC43-Probe I.10 | AY391777.1 | OC43-Probe I | TGTGAGAGATTCCCTTCGTAATACGACTCACTATAGGGCCTAGTAGTTCAGAGGTGGATATGATTTGCTCCACCACTGCGCAAAAGCTGGAAACAGACGGAATTTGTCCTGAAAATCATGTGATGGTGTGGAATTCTCGGGTGCCAAGGTACCAAGAAACCTTCATT |
| OC43-Probe I.11 | AY391777.1 | OC43-Probe I | TGTGAGAGATTCCCTTCGTAATACGACTCACTATAGGGTCCACCACTGCGCAAAAGCTGGAAACAGACGGAATTTGTCCTGAAAATCATGTGATGGTGGATTGTCGCCGACTTCTTAAACAAGAGTGTTGGAATTCTCGGGTGCCAAGGTACCAAGAAACCTTCATT |
| OC43-Probe I.12 | AY391777.1 | OC43-Probe I | TGTGAGAGATTCCCTTCGTAATACGACTCACTATAGGGGGAATTTGTCCTGAAAATCATGTGATGGTGGATTGTCGCCGACTTCTTAAACAAGAGTGTTGTGTGCAGTCTAGCCTAATACGTGAAATTTGGAATTCTCGGGTGCCAAGGTACCAAGAAACCTTCATT |
| OC43-Probe I.13 | AY391777.1 | OC43-Probe I | TGTGAGAGATTCCCTTCGTAATACGACTCACTATAGGGGATTGTCGCCGACTTCTTAAACAAGAGTGTTGTGTGCAGTCTAGCCTAATACGTGAAATTGTTATGAATGCAAGTCCATATGATTTGGAGTGGAATTCTCGGGTGCCAAGGTACCAAGAAACCTTCATT |
| OC43-Probe I.14 | AY391777.1 | OC43-Probe I | TGTGAGAGATTCCCTTCGTAATACGACTCACTATAGGGTGTGTGCAGTCTAGCCTAATACGTGAAATTGTTATGAATGCAAGTCCATATGATTTGGAGGTGCTACTTCAAGATGCTTTGCAGTCCCGTTGGAATTCTCGGGTGCCAAGGTACCAAGAAACCTTCATT |
| OC43-Probe I.15 | AY391777.1 | OC43-Probe I | TGTGAGAGATTCCCTTCGTAATACGACTCACTATAGGGTTATGAATGCAAGTCCATATGATTTGGAGGTGCTACTTCAAGATGCTTTGCAGTCCCGTGAAGCAGTTTTGGTTACAACCCCCTTAGGTTGGAATTCTCGGGTGCCAAGGTACCAAGAAACCTTCATT |
| OC43-Probe I.16 | AY391777.1 | OC43-Probe I | TGTGAGAGATTCCCTTCGTAATACGACTCACTATAGGGGCTACTTCAAGATGCTTTGCAGTCCCGTGAAGCAGTTTTGGTTACAACCCCCTTAGGTATGTGTCTTTAGAGGCATGCTATGTGAGAGGTTGGAATTCTCGGGTGCCAAGGTACCAAGAAACCTTCATT |
| OC43-Probe I.17 | AY391777.1 | OC43-Probe I | TGTGAGAGATTCCCTTCGTAATACGACTCACTATAGGGGAAGCAGTTTTGGTTACAACCCCCTTAGGTATGTGTCTTTAGAGGCATGCTATGTGAGAGGTTGTAATCCTAAAGGATGGACCATGGGTTTGTGGAATTCTCGGGTGCCAAGGTACCAAGAAACCTTCATT |

| ASO ID | Accession | Group ID | Custom Array sequence |
|---|---|---|---|
| OC43-Probe I.18 | AY391777.1 | OC43-Probe I | TGTGAGAGATTCCCTTCGTAATACGACTCACTATAGGGATGTCTTTAGAGGCATGCTATGTGAGAGGTTGTAATCCTAAAGGATGGACCATGGGTTTGTTTCGGCGTAGAAGTGTGTGTAACACTGGTTGGAATTCTCGGGTGCCAAGGTACCAAGAAACCTTCATT |
| OC43-Probe I.19 | AY391777.1 | OC43-Probe I | TGTGAGAGATTCCCTTCGTAATACGACTCACTATAGGGTGTAATCCTAAAGGATGGACCATGGGTTTGTTTCGGCGTAGAAGTGTGTGTAACACTGGTCGTTGCACTGTTAATAAGCATGTGGCCTATTGGAATTCTCGGGTGCCAAGGTACCAAGAAACCTTCATT |
| OC43-Probe I.20 | AY391777.1 | OC43-Probe I | TGTGAGAGATTCCCTTCGTAATACGACTCACTATAGGGTTTCGGCGTAGAAGTGTGTGTAACACTGGTCGTTGCACTGTTAATAAGCATGTGGCCTATCAGTTATATATGATTGATCCTGCAGGTGTCTGGAATTCTCGGGTGCCAAGGTACCAAGAAACCTTCATT |
| OC43-Probe I.21 | AY391777.1 | OC43-Probe I | TGTGAGAGATTCCCTTCGTAATACGACTCACTATAGGGCGTTGCACTGTTAATAAGCATGTGGCCTATCAGTTATATATGATTGATCCTGCAGGTGTCTGTCTTGGTGCAGGTCAATTCGTGGGTTGGTGGAATTCTCGGGTGCCAAGGTACCAAGAAACCTTCATT |
| OC43-Probe I.22 | AY391777.1 | OC43-Probe I | TGTGAGAGATTCCCTTCGTAATACGACTCACTATAGGGCAGTTATATATGATTGATCCTGCAGGTGTCTGTCTTGGTGCAGGTCAATTCGTGGGTTGGGTCATACCCTTAGCCTTTATGCCTGTGCAATGGAATTCTCGGGTGCCAAGGTACCAAGAAACCTTCATT |
| OC43-Probe I.23 | AY391777.1 | OC43-Probe I | TGTGAGAGATTCCCTTCGTAATACGACTCACTATAGGGTGTCTTGGTGCAGGTCAATTCGTGGGTTGGGTCATACCCTTAGCCTTTATGCCTGTGCAATCCCGGAAATTTATTGTTCCATGGGTTATGTGGAATTCTCGGGTGCCAAGGTACCAAGAAACCTTCATT |
| OC43-Probe I.24 | AY391777.1 | OC43-Probe I | TGTGAGAGATTCCCTTCGTAATACGACTCACTATAGGGGTCATACCCTTAGCCTTTATGCCTGTGCAATCCCGGAAATTTATTGTTCCATGGGTTATGTACTTGCGTAAGCGTGGCGAAAAGGGTGCTTGGAATTCTCGGGTGCCAAGGTACCAAGAAACCTTCATT |
| OC43-Probe I.25 | AY391777.1 | OC43-Probe I | TGTGAGAGATTCCCTTCGTAATACGACTCACTATAGGGTCCCGGAAATTTATTGTTCCATGGGTTATGTACTTGCGTAAGCGTGGCGAAAAGGGTGCTTACAATAAAGATCATGGACGTGGCGGTTTTTGGAATTCTCGGGTGCCAAGGTACCAAGAAACCTTCATT |
| OC43-Probe I.26 | AY391777.1 | OC43-Probe I | TGTGAGAGATTCCCTTCGTAATACGACTCACTATAGGGTACTTGCGTAAGCGTGGCGAAAAGGGTGCTTACAATAAAGATCATGGACGTGGCGGTTTTGGACATGTTTATGATTTTAAAGTTGAAGATTGGAATTCTCGGGTGCCAAGGTACCAAGAAACCTTCATT |
| OC43-Probe I.27 | AY391777.1 | OC43-Probe I | TGTGAGAGATTCCCTTCGTAATACGACTCACTATAGGGTACAATAAAGATCATGGACGTGGCGGTTTTGGACATGTTTATGATTTTAAAGTTGAAGATGCTTATGACCAGGTGCATGATGAGCCTAAGTGGAATTCTCGGGTGCCAAGGTACCAAGAAACCTTCATT |
| OC43-Probe I.28 | AY391777.1 | OC43-Probe I | TGTGAGAGATTCCCTTCGTAATACGACTCACTATAGGGGGACATGTTTATGATTTTAAAGTTGAAGATGCTTATGACCAGGTGCATGATGAGCCTAAGGGTAAGTTTTCTAAGAAGGCTTATGCTTTATGGAATTCTCGGGTGCCAAGGTACCAAGAAACCTTCATT |
| OC43-Probe I.29 | AY391777.1 | OC43-Probe I | TGTGAGAGATTCCCTTCGTAATACGACTCACTATAGGGGCTTATGACCAGGTGCATGATGAGCCTAAGGGTAAGTTTTCTAAGAAGGCTTATGCTTTAATTAGAGGGTATCGTGGTGTTAAACCACTTTGGAATTCTCGGGTGCCAAGGTACCAAGAAACCTTCATT |
| OC43-Probe I.30 | AY391777.1 | OC43-Probe I | TGTGAGAGATTCCCTTCGTAATACGACTCACTATAGGGGGTAAGTTTTCTAAGAAGGCTTATGCTTTAATTAGAGGGTATCGTGGTGTTAAACCACTTCTCTATGTAGACCAGTATGGTTGTGATTATTGGAATTCTCGGGTGCCAAGGTACCAAGAAACCTTCATT |
| OC43-Probe I.31 | AY391777.1 | OC43-Probe I | TGTGAGAGATTCCCTTCGTAATACGACTCACTATAGGGATTAGAGGGTATCGTGGTGTTAAACCACTTCTCTATGTAGACCAGTATGGTTGTGATTATACTGGTAGTCTTGCAGATGGCTTAGAGGCTTGGAATTCTCGGGTGCCAAGGTACCAAGAAACCTTCATT |
| OC43-Probe I.32 | AY391777.1 | OC43-Probe I | TGTGAGAGATTCCCTTCGTAATACGACTCACTATAGGGCTCTATGTAGACCAGTATGGTTGTGATTATACTGGTAGTCTTGCAGATGGCTTAGAGGCTTATGCTGATAAGACATTGCAAGAAATGAAGTGGAATTCTCGGGTGCCAAGGTACCAAGAAACCTTCATT |
| OC43-Probe I.33 | AY391777.1 | OC43-Probe I | TGTGAGAGATTCCCTTCGTAATACGACTCACTATAGGGACTGGTAGTCTTGCAGATGGCTTAGAGGCTTATGCTGATAAGACATTGCAAGAAATGAAGGCATTATTTCCTACTTGGAGTCAGGAACTCTGGAATTCTCGGGTGCCAAGGTACCAAGAAACCTTCATT |
| OC43-Probe I.34 | AY391777.1 | OC43-Probe I | TGTGAGAGATTCCCTTCGTAATACGACTCACTATAGGGTATGCTGATAAGACATTGCAAGAAATGAAGGCATTATTTCCTACTTGGAGTCAGGAACTCCTTTTTGATGTAATTGTGGCATGGCATGTTTGGAATTCTCGGGTGCCAAGGTACCAAGAAACCTTCATT |
| OC43-Probe I.35 | AY391777.1 | OC43-Probe I | TGTGAGAGATTCCCTTCGTAATACGACTCACTATAGGGGCATTATTTCCTACTTGGAGTCAGGAACTCCTTTTTGATGTAATTGTGGCATGGCATGTTGTGCGTGATCCACGTTATGTTATGAGATTGTGGAATTCTCGGGTGCCAAGGTACCAAGAAACCTTCATT |
| OC43-Probe I.36 | AY391777.1 | OC43-Probe I | TGTGAGAGATTCCCTTCGTAATACGACTCACTATAGGGCTTTTTGATGTAATTGTGGCATGGCATGTTGTGCGTGATCCACGTTATGTTATGAGATTGCAGAGTGCTGCTACTATACGTAGTGTTGCATGGAATTCTCGGGTGCCAAGGTACCAAGAAACCTTCATT |
| OC43-Probe I.37 | AY391777.1 | OC43-Probe I | TGTGAGAGATTCCCTTCGTAATACGACTCACTATAGGGGTGCGTGATCCACGTTATGTTATGAGATTGCAGAGTGCTGCTACTATACGTAGTGTTGCATATGTTGCTAATCCTACTGAAGACTTGTGTTGGAATTCTCGGGTGCCAAGGTACCAAGAAACCTTCATT |
| OC43-Probe I.38 | AY391777.1 | OC43-Probe I | TGTGAGAGATTCCCTTCGTAATACGACTCACTATAGGGCAGAGTGCTGCTACTATACGTAGTGTTGCATATGTTGCTAATCCTACTGAAGACTTGTGTGATGGTTCTGTTGTTATAAAAGAACCTGTGTGGAATTCTCGGGTGCCAAGGTACCAAGAAACCTTCATT |
| OC43-Probe I.39 | AY391777.1 | OC43-Probe I | TGTGAGAGATTCCCTTCGTAATACGACTCACTATAGGGTATGTTGCTAATCCTACTGAAGACTTGTGTGATGGTTCTGTTGTTATAAAAGAACCTGTGCATGTTTATGCAGATGACTCTATTATTTTATGGAATTCTCGGGTGCCAAGGTACCAAGAAACCTTCATT |
| OC43-Probe I.40 | AY391777.1 | OC43-Probe I | TGTGAGAGATTCCCTTCGTAATACGACTCACTATAGGGGATGGTTCTGTTGTTATAAAAGAACCTGTGCATGTTTATGCAGATGACTCTATTATTTTACGTCAATATAATTTAGTTGACATTATGAGTTGGAATTCTCGGGTGCCAAGGTACCAAGAAACCTTCATT |
| OC43-Probe I.41 | AY391777.1 | OC43-Probe I | TGTGAGAGATTCCCTTCGTAATACGACTCACTATAGGGCATGTTTATGCAGATGACTCTATTATTTTACGTCAATATAATTTAGTTGACATTATGAGTCATTTTTATATGGAGGCAGATACAGTTGTATGGAATTCTCGGGTGCCAAGGTACCAAGAAACCTTCATT |
| OC43-Probe I.42 | AY391777.1 | OC43-Probe I | TGTGAGAGATTCCCTTCGTAATACGACTCACTATAGGGCGTCAATATAATTTAGTTGACATTATGAGTCATTTTTATATGGAGGCAGATACAGTTGTAAATGCTTTTTATGGTGTTGCTTTGAAAGATTGGAATTCTCGGGTGCCAAGGTACCAAGAAACCTTCATT |

| ASO ID | Accession | Group ID | Custom Array sequence |
|---|---|---|---|
| OC43-Probe I.43 | AY391777.1 | OC43-Probe I | TGTGAGAGATTCCCTTCGTAATACGACTCACTATAGGGCATTTTTATATGGAGGCAGATACAGTTGTAAATGCTTTTTATGGTGTTGCTTTGAAAGATTGCGGTTTTGTTATGCAGTTTGGTTACATTTGGAATTCTCGGGTGCCAAGGTACCAAGAAACCTTCATT |
| OC43-Probe I.44 | AY391777.1 | OC43-Probe I | TGTGAGAGATTCCCTTCGTAATACGACTCACTATAGGGAATGCTTTTTATGGTGTTGCTTTGAAAGATTGCGGTTTTGTTATGCAGTTTGGTTACATTGATTGCGAACAAGACTCGTGTGATTTAAATGGAATTCTCGGGTGCCAAGGTACCAAGAAACCTTCATT |
| OC43-Probe I.45 | AY391777.1 | OC43-Probe I | TGTGAGAGATTCCCTTCGTAATACGACTCACTATAGGGTGCGGTTTTGTTATGCAGTTTGGTTACATTGATTGCGAACAAGACTCGTGTGATTTTAAAGGTTGGATTCCTGGTAACATGATAGATGGTTGGAATTCTCGGGTGCCAAGGTACCAAGAAACCTTCATT |
| OC43-Probe I.46 | AY391777.1 | OC43-Probe I | TGTGAGAGATTCCCTTCGTAATACGACTCACTATAGGGGATTGCGAACAAGACTCGTGTGATTTTAAAGGTTGGATTCCTGGTAACATGATAGATGGTTTTGCTTGCACCACTTGTGGTCATGTTTATTGGAATTCTCGGGTGCCAAGGTACCAAGAAACCTTCATT |
| OC43-Probe I.47 | AY391777.1 | OC43-Probe I | TGTGAGAGATTCCCTTCGTAATACGACTCACTATAGGGGGGTTGGATTCCTGGTAACATGATAGATGGTTTTGCTTGCACCACTTGTGGTCATGTTTATGAAGTAGGTGATTTGATAGCACAATCTTCATGGAATTCTCGGGTGCCAAGGTACCAAGAAACCTTCATT |
| OC43-Probe I.48 | AY391777.1 | OC43-Probe I | TGTGAGAGATTCCCTTCGTAATACGACTCACTATAGGGTTTGCTTGCACCACTTGTGGTCATGTTTATGAAGTAGGTGATTTGATAGCACAATCTTCAGGTGTTTTGCCTGTTAACCCTGTATTGCATTGGAATTCTCGGGTGCCAAGGTACCAAGAAACCTTCATT |
| OC43-Probe I.49 | AY391777.1 | OC43-Probe I | TGTGAGAGATTCCCTTCGTAATACGACTCACTATAGGGGAAGTAGGTGATTTGATAGCACAATCTTCAGGTGTTTTGCCTGTTAACCCTGTATTGCATACTAAGAGTGCAGCAGGCTATGGTGGTTTTTGGAATTCTCGGGTGCCAAGGTACCAAGAAACCTTCATT |
| OC43-Probe I.50 | AY391777.1 | OC43-Probe I | TGTGAGAGATTCCCTTCGTAATACGACTCACTATAGGGGGGTGTTTGCCTGTTAACCCTGTATTGCATACTAAGAGTGCAGCAGGCTATGGTGGTTTTGGTTGTAAAGATTCTTTTACTCTGTATGGCTGGAATTCTCGGGTGCCAAGGTACCAAGAAACCTTCATT |
| OC43-Probe I.51 | AY391777.1 | OC43-Probe I | TGTGAGAGATTCCCTTCGTAATACGACTCACTATAGGGACTAAGAGTGCAGCAGGCTATGGTGGTTTTGGTTGTAAAGATTCTTTTACTCTGTATGGCCAAACTGTAGTTTATTTTGGAGGTTGTGTGTGGAATTCTCGGGTGCCAAGGTACCAAGAAACCTTCATT |
| OC43-Probe I.52 | AY391777.1 | OC43-Probe I | TGTGAGAGATTCCCTTCGTAATACGACTCACTATAGGGGGGTTGTAAAGATTCTTTTACTCTGTATGGCCAAACTGTAGTTTATTTTGGAGGTTGTGTGTATTGGAGTCCAGCACGTAATATATGGATTTGGAATTCTCGGGTGCCAAGGTACCAAGAAACCTTCATT |
| OC43-Probe I.53 | AY391777.1 | OC43-Probe I | TGTGAGAGATTCCCTTCGTAATACGACTCACTATAGGGCAAACTGTAGTTTATTTTGGAGGTTGTGTGTATTGGAGTCCAGCACGTAATATATGGATTCCTATATTAAAATCCTCTGTTAAGTCATATTGGAATTCTCGGGTGCCAAGGTACCAAGAAACCTTCATT |
| OC43-Probe I.54 | AY391777.1 | OC43-Probe I | TGTGAGAGATTCCCTTCGTAATACGACTCACTATAGGGTATTGGAGTCCAGCACGTAATATATGGATTCCTATATTAAAATCCTCTGTTAAGTCATATGCAGTTTGGTTTATACTGGAGTTTTTAGGTTGGAATTCTCGGGTGCCAAGGTACCAAGAAACCTTCATT |
| OC43-Probe I.55 | AY391777.1 | OC43-Probe I | TGTGAGAGATTCCCTTCGTAATACGACTCACTATAGGGCCTATATTAAAATCCTCTGTTAAGTCATATGCAGTTTGGTTTATACTGGAGTTTTTAGGTTGCAAGGCTATTGTAAAGGAAACAAATCTCTGGAATTCTCGGGTGCCAAGGTACCAAGAAACCTTCATT |
| OC43-Probe I.56 | AY391777.1 | OC43-Probe I | TGTGAGAGATTCCCTTCGTAATACGACTCACTATAGGGGACAGTTTGGTTTATACTGGAGTTTTTAGGTTGCAAGGCTATTGTAAAGGAAACAAATCTCATTTGCAAAGCTTTGTACCTTGATTATGTTTGGAATTCTCGGGTGCCAAGGTACCAAGAAACCTTCATT |
| OC43-Probe I.57 | AY391777.1 | OC43-Probe I | TGTGAGAGATTCCCTTCGTAATACGACTCACTATAGGGTGCAAGGCTATTGTAAAGGAAACAAATCTCATTTGCAAAGCTTTGTACCTTGATTATGTTCAACACAAGTGTGGCAATTTACACCAACGGTGGAATTCTCGGGTGCCAAGGTACCAAGAAACCTTCATT |
| OC43-Probe I.58 | AY391777.1 | OC43-Probe I | TGTGAGAGATTCCCTTCGTAATACGACTCACTATAGGGATTTGCAAAGCTTTGTACCTTGATTATGTTCAACACAAGTGTGGCAATTTACACCAACGGGAGTTGCTAGGTGTTTCAGATGTGTGGCATTGGAATTCTCGGGTGCCAAGGTACCAAGAAACCTTCATT |
| OC43-Probe I.59 | AY391777.1 | OC43-Probe I | TGTGAGAGATTCCCTTCGTAATACGACTCACTATAGGGCAACACAAGTGTGGCAATTTACACCAACGGGAGTTGCTAGGTGTTTCAGATGTGTGGCATAAACAATTGCTATTAAATAGAGGTGTTTATTGGAATTCTCGGGTGCCAAGGTACCAAGAAACCTTCATT |
| OC43-Probe I.60 | AY391777.1 | OC43-Probe I | TGTGAGAGATTCCCTTCGTAATACGACTCACTATAGGGGAGTTGCTAGGTGTTTCAGATGTGTGGCATAAACAATTGCTATTAAATAGAGGTGTTTATAAACCTCTGTTAGAGAATATTGATTATTTTTGGAATTCTCGGGTGCCAAGGTACCAAGAAACCTTCATT |
| OC43-Probe I.61 | AY391777.1 | OC43-Probe I | TGTGAGAGATTCCCTTCGTAATACGACTCACTATAGGGGAAACAATTGCTATTAAATAGAGGTGTTTATAAACCTCTGTTAGAGAATATTGATTATTTTAATATGCGGCGCGCTAAATTTAGTTTAGAATGGAATTCTCGGGTGCCAAGGTACCAAGAAACCTTCATT |
| OC43-Probe I.62 | AY391777.1 | OC43-Probe I | TGTGAGAGATTCCCTTCGTAATACGACTCACTATAGGGGAAACCTCTGTTAGAGAATATTGATTATTTTAATATGCGGCGCGCTAAATTTAGTTTAGAAACTTTTACTGTTTGTGCAGATGGCTTTATGTGGAATTCTCGGGTGCCAAGGTACCAAGAAACCTTCATT |
| OC43-Probe I.63 | AY391777.1 | OC43-Probe I | TGTGAGAGATTCCCTTCGTAATACGACTCACTATAGGGAATATGCGGCGCGCTAAATTTAGTTTAGAAACTTTTACTGTTTGTGCAGATGGCTTTATGCCTTTTCTTTTAGATGATTTAGTTCCACGCTGGAATTCTCGGGTGCCAAGGTACCAAGAAACCTTCATT |
| OC43-Probe I.64 | AY391777.1 | OC43-Probe I | TGTGAGAGATTCCCTTCGTAATACGACTCACTATAGGGACTTTTACTGTTTGTGCAGATGGCTTTATGCCTTTTCTTTTAGATGATTTAGTTCCACGCGCATATTATTTGGCAGTAAGTGGTCAAGCATGGAATTCTCGGGTGCCAAGGTACCAAGAAACCTTCATT |
| OC43-Probe I.65 | AY391777.1 | OC43-Probe I | TGTGAGAGATTCCCTTCGTAATACGACTCACTATAGGGCCTTTTCTTTTAGATGATTTAGTTCCACGCGCATATTATTTGGCAGTAAGTGGTCAAGCATTTTGTGATTATGCAGATAAACTTTGCCATTGGAATTCTCGGGTGCCAAGGTACCAAGAAACCTTCATT |
| OC43-Probe I.66 | AY391777.1 | OC43-Probe I | TGTGAGAGATTCCCTTCGTAATACGACTCACTATAGGGGGCATATTATTTGGCAGTAAGTGGTCAAGCATTTTGTGATTATGCAGATAAACTTTGCCATGCCGTTGTGTCTAAGAGTAAAGAGTTACTTTGGAATTCTCGGGTGCCAAGGTACCAAGAAACCTTCATT |
| OC43-Probe I.67 | AY391777.1 | OC43-Probe I | TGTGAGAGATTCCCTTCGTAATACGACTCACTATAGGGTTTTGTGATTATGCAGATAAACTTTGCCATGCCGTTGTGTCTAAGAGTAAAGAGTTACTTGATGTGTCTCTGGATTCTTTAGGTGCAGCTTGGAATTCTCGGGTGCCAAGGTACCAAGAAACCTTCATT |

EP 4 239 083 A2

| ASO ID | Accession | Group ID | Custom Array sequence |
|---|---|---|---|
| OC43-Probe I.68 | AY391777.1 | OC43-Probe I | TGTGAGAGATTCCCTTCGTAATACGACTCACTATAGGGGCCGTTGTGTCTAAGAGTAAAGAGTTACTTGATGTGTCTCTGGATTCTTTAGGTGCAGCTATACATTATTTGAATTCTAAGATTGTTGATTGGAATTCTCGGGTGCCAAGGTACCAAGAAACCTTCATT |
| OC43-Probe I.69 | AY391777.1 | OC43-Probe I | TGTGAGAGATTCCCTTCGTAATACGACTCACTATAGGGGATGTGTCTCTGGATTCTTTAGGTGCAGCTATACATTATTTGAATTCTAAGATTGTTGATTGGCTCAACATTTTAGTGATTTTGGAACATGGAATTCTCGGGTGCCAAGGTACCAAGAAACCTTCATT |
| OC43-Probe I.70 | AY391777.1 | OC43-Probe I | TGTGAGAGATTCCCTTCGTAATACGACTCACTATAGGGGATACATTATTTGAATTCTAAGATTGTTGATTGGCTCAACATTTTAGTGATTTTGGAACAAGTTTCGTTTCTAAAATTGTTCATTTCTTTTGGAATTCTCGGGTGCCAAGGTACCAAGAAACCTTCATT |
| OC43-Probe I.71 | AY391777.1 | OC43-Probe I | TGTGAGAGATTCCCTTCGTAATACGACTCACTATAGGGGTTGGCTCAACATTTTAGTGATTTTGGAACAAGTTTCGTTTCTAAAATTGTTCATTTCTTTAAGACTTTTACTACTAGCACTGCTCTTGCATGGAATTCTCGGGTGCCAAGGTACCAAGAAACCTTCATT |
| OC43-Probe I.72 | AY391777.1 | OC43-Probe I | TGTGAGAGATTCCCTTCGTAATACGACTCACTATAGGGGAGTTTCGTTTCTAAAATTGTTCATTTCTTTAAGACTTTTACTACTAGCACTGCTCTTGCATTTGCATGGGTTTTATTTCATGTTTTGCATTGGAATTCTCGGGTGCCAAGGTACCAAGAAACCTTCATT |
| OC43-Probe I.73 | AY391777.1 | OC43-Probe I | TGTGAGAGATTCCCTTCGTAATACGACTCACTATAGGGAAGACTTTTACTACTAGCACTGCTCTTGCATTTGCATGGGTTTTATTTCATGTTTTGCATGGTGCTTATATAGTAGTGGAGAGTGATATATGGAATTCTCGGGTGCCAAGGTACCAAGAAACCTTCATT |
| OC43-Probe I.74 | AY391777.1 | OC43-Probe I | TGTGAGAGATTCCCTTCGTAATACGACTCACTATAGGGGTTTGCATGGGTTTTATTTCATGTTTTGCATGGTGCTTATATAGTAGTGGAGAGTGATATATATTTTGTTAAAAACATTCCTCGTTATGCTTGGAATTCTCGGGTGCCAAGGTACCAAGAAACCTTCATT |
| OC43-Probe I.75 | AY391777.1 | OC43-Probe I | TGTGAGAGATTCCCTTCGTAATACGACTCACTATAGGGGGGTGCTTATATAGTAGTGGAGAGTGATATATATTTTGTTAAAAACATTCCTCGTTATGCTAGTGCTGTTGCACAAGCATTTCAGAGTGTTTGGAATTCTCGGGTGCCAAGGTACCAAGAAACCTTCATT |
| OC43-Probe I.76 | AY391777.1 | OC43-Probe I | TGTGAGAGATTCCCTTCGTAATACGACTCACTATAGGGTATTTTGTTAAAAACATTCCTCGTTATGCTAGTGCTGTTGCACAAGCATTTCAGAGTGTTGCTAAAGTTGTACTGGACTCTTTAAGAGTTTGGAATTCTCGGGTGCCAAGGTACCAAGAAACCTTCATT |
| OC43-Probe I.77 | AY391777.1 | OC43-Probe I | TGTGAGAGATTCCCTTCGTAATACGACTCACTATAGGGGAGTGCTGTTGCACAAGCATTTCAGAGTGTTGCTAAAGTTGTACTGGACTCTTTAAGAGTTACTTTTATTGATGGCCTTTCTTGTTTTAAGTGGAATTCTCGGGTGCCAAGGTACCAAGAAACCTTCATT |
| OC43-Probe I.78 | AY391777.1 | OC43-Probe I | TGTGAGAGATTCCCTTCGTAATACGACTCACTATAGGGGCTAAAGTTGTACTGGACTCTTTAAGAGTTACTTTTATTGATGGCCTTTCTTGTTTTAAGTGGACGTAGAAGAAATTTGTCTTTCAGGCTGGAATTCTCGGGTGCCAAGGTACCAAGAAACCTTCATT |
| OC43-Probe I.79 | AY391777.1 | OC43-Probe I | TGTGAGAGATTCCCTTCGTAATACGACTCACTATAGGGGACTTTTATTGATGGCCTTTCTTGTTTTAAGATTGGACGTAGAAGAATTTGTCTTTCAGGCAGAAAAATTTATGAAGTTGAGCGTGGCTTGTGGAATTCTCGGGTGCCAAGGTACCAAGAAACCTTCATT |
| OC43-Probe I.80 | AY391777.1 | OC43-Probe I | TGTGAGAGATTCCCTTCGTAATACGACTCACTATAGGGGATTGGACGTAGAAGAATTTGTCTTTCAGGCAGAAAAATTTATGAAGTTGAGCGTGGCTTGTTACATTCATCCCAATTGCCATTAGATGTTTGGAATTCTCGGGTGCCAAGGTACCAAGAAACCTTCATT |
| OC43-Probe I.81 | AY391777.1 | OC43-Probe I | TGTGAGAGATTCCCTTCGTAATACGACTCACTATAGGGGAGAAAAATTTATGAAGTTGAGCGTGGCTTGTTACATTCATCCCAATTGCCATTAGATGTTTATGATTTAACCATGCCTAGTCAAGTTCAGTGGAATTCTCGGGTGCCAAGGTACCAAGAAACCTTCATT |
| OC43-Probe I.82 | AY391777.1 | OC43-Probe I | TGTGAGAGATTCCCTTCGTAATACGACTCACTATAGGGTTACATTCATCCCAATTGCCATTAGATGTTTATGATTTAACCATGCCTAGTCAAGTTCAGAAAGCCAAGCAAAAACCTATTTATTTAAAATGGAATTCTCGGGTGCCAAGGTACCAAGAAACCTTCATT |
| OC43-Probe I.83 | AY391777.1 | OC43-Probe I | TGTGAGAGATTCCCTTCGTAATACGACTCACTATAGGGTATGATTTAACCATGCCTAGTCAAGTTCAGAAAGCCAAGCAAAAACCTATTTATTTAAAAGGTTCTGGTTCTGATTTTTCATTAGCGGATTGGAATTCTCGGGTGCCAAGGTACCAAGAAACCTTCATT |
| OC43-Probe I.84 | AY391777.1 | OC43-Probe I | TGTGAGAGATTCCCTTCGTAATACGACTCACTATAGGGGAAAGCCAAGCAAAAACCTATTTATTTAAAAGGTTCTGGTTCTGATTTTTCATTAGCGGATAGTGTAGTTGAAGTTGTTACAACTTCACTTTGGAATTCTCGGGTGCCAAGGTACCAAGAAACCTTCATT |
| OC43-Probe I.85 | AY391777.1 | OC43-Probe I | TGTGAGAGATTCCCTTCGTAATACGACTCACTATAGGGGGGTTCTGGTTCTGATTTTTCATTAGCGGATAGTGTAGTTGAAGTTGTTACAACTTCACTTACACCATGTGGTTATTCTGAACCACCTAAATGGAATTCTCGGGTGCCAAGGTACCAAGAAACCTTCATT |
| OC43-Probe I.86 | AY391777.1 | OC43-Probe I | TGTGAGAGATTCCCTTCGTAATACGACTCACTATAGGGGAGTGTAGTTGAAGTTGTTACAACTTCACTTACACCATGTGGTTATTCTGAACCACCTAAAGTTGCAGATAAAATTTGCATTGTGGATAATGGAATTCTCGGGTGCCAAGGTACCAAGAAACCTTCATT |
| OC43-Probe I.87 | AY391777.1 | OC43-Probe I | TGTGAGAGATTCCCTTCGTAATACGACTCACTATAGGGGACACCATGTGGTTATTCTGAACCACCTAAAGTTGCAGATAAAATTTGCATTGTGGATAATGTTTATATGGCCAAGGCTGGTGACAAATATTGGAATTCTCGGGTGCCAAGGTACCAAGAAACCTTCATT |
| OC43-Probe I.88 | AY391777.1 | OC43-Probe I | TGTGAGAGATTCCCTTCGTAATACGACTCACTATAGGGGGTTGCAGATAAAATTTGCATTGTGGATAATGTTTATATGGCCAAGGCTGGTGACAAATATTACCCTGTTGTGGTTGATGATCATGTTGGATGGAATTCTCGGGTGCCAAGGTACCAAGAAACCTTCATT |
| OC43-Probe I.89 | AY391777.1 | OC43-Probe I | TGTGAGAGATTCCCTTCGTAATACGACTCACTATAGGGGTTTATATGGCCAAGGCTGGTGACAAATATTACCCTGTTGTGGTTGATGATCATGTTGGACTCTTGGATCAAGCATGGAGAGTTCCTTGTTGGAATTCTCGGGTGCCAAGGTACCAAGAAACCTTCATT |
| OC43-Probe I.90 | AY391777.1 | OC43-Probe I | TGTGAGAGATTCCCTTCGTAATACGACTCACTATAGGGTACCCTGTTGTGGTTGATGATCATGTTGGACTCTTGGATCAAGCATGGAGAGTTCCTTGTGCTGGAAGGCGTGTTACATTTAAGGAACAGTGGAATTCTCGGGTGCCAAGGTACCAAGAAACCTTCATT |
| OC43-Probe I.91 | AY391777.1 | OC43-Probe I | TGTGAGAGATTCCCTTCGTAATACGACTCACTATAGGGGCTCTTGGATCAAGCATGGAGAGTTCCTTGTGCTGGAAGGCGTGTTACATTTAAGGAACAGCCTACAGTAAAGGAGATTATAAGCATGCCTTGGAATTCTCGGGTGCCAAGGTACCAAGAAACCTTCATT |
| OC43-Probe I.92 | AY391777.1 | OC43-Probe I | TGTGAGAGATTCCCTTCGTAATACGACTCACTATAGGGGCTGGAAGGCGTGTTACATTTAAGGAACAGCCTACAGTAAAGGAGATTATAAGCATGCCTAAGATTATTAAGGTTTTTTATGAGCTTGACTGGAATTCTCGGGTGCCAAGGTACCAAGAAACCTTCATT |

64

| ASO ID | Accession | Group ID | Custom Array sequence |
|---|---|---|---|
| OC43-Probe I.93 | AY391777.1 | OC43-Probe I | TGTGAGAGATTCCCTTCGTAATACGACTCACTATAGGGCCTACAGTAAAGGAGATTATAAGCATGCCTAAGATTATTAAGGTTTTTTATGAGCTTGACAACGATTTTAATACTATTTTAAATACTGCGTGGAATTCTCGGGTGCCAAGGTACCAAGAAACCTTCATT |
| OC43-Probe I.94 | AY391777.1 | OC43-Probe I | TGTGAGAGATTCCCTTCGTAATACGACTCACTATAGGGAAGATTATTAAGGTTTTTTATGAGCTTGACAACGATTTTAATACTATTTTAAATACTGCGTGTGGAGTGTTTGAAGTGGATGATACTGTTTGGAATTCTCGGGTGCCAAGGTACCAAGAAACCTTCATT |
| OC43-Probe I.95 | AY391777.1 | OC43-Probe I | TGTGAGAGATTCCCTTCGTAATACGACTCACTATAGGGAACGATTTTAATACTATTTTAAATACTGCGTGTGGAGTGTTTGAAGTGGATGATACTGTTGATATGGAGGAATTTTATGCTGTGGTGATTTGGAATTCTCGGGTGCCAAGGTACCAAGAAACCTTCATT |
| OC43-Probe I.96 | AY391777.1 | OC43-Probe I | TGTGAGAGATTCCCTTCGTAATACGACTCACTATAGGGTGTGGAGTGTTTGAAGTGGATGATACTGTTGATATGGAGGAATTTTATGCTGTGGTGATTGATGCCATAGAAGAGAAACTTTCTCCATGTTGGAATTCTCGGGTGCCAAGGTACCAAGAAACCTTCATT |
| OC43-Probe I.97 | AY391777.1 | OC43-Probe I | TGTGAGAGATTCCCTTCGTAATACGACTCACTATAGGGGATATGGAGGAATTTTATGCTGTGGTGATTGATGCCATAGAAGAGAAACTTTCTCCATGTAAGGAGCTTGAAGGTGTAGGTGCTAAAGTTTGGAATTCTCGGGTGCCAAGGTACCAAGAAACCTTCATT |
| OC43-Probe I.98 | AY391777.1 | OC43-Probe I | TGTGAGAGATTCCCTTCGTAATACGACTCACTATAGGGGATGCCATAGAAGAGAAACTTTCTCCATGTAAGGAGCTTGAAGGTGTAGGTGCTAAAGTTAGTGCCTTTTTACAGAAATTAGAGGATAATTGGAATTCTCGGGTGCCAAGGTACCAAGAAACCTTCATT |
| OC43-Probe I.99 | AY391777.1 | OC43-Probe I | TGTGAGAGATTCCCTTCGTAATACGACTCACTATAGGGAAGGAGCTTGAAGGTGTAGGTGCTAAAGTTAGTGCCTTTTTACAGAAATTAGAGGATAATCCCCTATTTTTATTTGATGAGGCTGGCGAGTGGAATTCTCGGGTGCCAAGGTACCAAGAAACCTTCATT |
| OC43-Probe I.100 | AY391777.1 | OC43-Probe I | TGTGAGAGATTCCCTTCGTAATACGACTCACTATAGGGAGTGCCTTTTTACAGAAATTAGAGGATAATCCCCTATTTTTATTTGATGAGGCTGGCGAGGAAGTTCTTGCTCCTAAATTGTATTGTGCCTGGAATTCTCGGGTGCCAAGGTACCAAGAAACCTTCATT |
| OC43-Probe I.101 | AY391777.1 | OC43-Probe I | TGTGAGAGATTCCCTTCGTAATACGACTCACTATAGGGCCCCTATTTTTATTTGATGAGGCTGGCGAGGAAGTTCTTGCTCCTAAATTGTATTGTGCCTTTACAGCTCCTGAAGATGATGACTTTCTTTGGAATTCTCGGGTGCCAAGGTACCAAGAAACCTTCATT |
| OC43-Probe I.102 | AY391777.1 | OC43-Probe I | TGTGAGAGATTCCCTTCGTAATACGACTCACTATAGGGGAAGTTCTTGCTCCTAAATTGTATTGTGCCTTTACAGCTCCTGAAGATGATGACTTTCTTGAGGAAAGTGATGTTGAAGAAGATGATGTATGGAATTCTCGGGTGCCAAGGTACCAAGAAACCTTCATT |
| OC43-Probe I.103 | AY391777.1 | OC43-Probe I | TGTGAGAGATTCCCTTCGTAATACGACTCACTATAGGGTTTACAGCTCCTGAAGATGATGACTTTCTTGAGGAAAGTGATGTTGAAGAAGATGATGTAGAAGGTGAGGAAACTGATTTAACTGTCACATGGAATTCTCGGGTGCCAAGGTACCAAGAAACCTTCATT |
| OC43-Probe I.104 | AY391777.1 | OC43-Probe I | TGTGAGAGATTCCCTTCGTAATACGACTCACTATAGGGGAGGAAAGTGATGTTGAAGAAGATGATGTAGAAGGTGAGGAAACTGATTTAACTGTCACAAGTGCTGGACAGCCTTGTGTTGCTAGTGAATGGAATTCTCGGGTGCCAAGGTACCAAGAAACCTTCATT |
| OC43-Probe I.105 | AY391777.1 | OC43-Probe I | TGTGAGAGATTCCCTTCGTAATACGACTCACTATAGGGGAAGGTGAGGAAACTGATTTAACTGTCACAAGTGCTGGACAGCCTTGTGTTGCTAGTGAACAGGAGGAGTCTTCTGAAGTCTTAGAGGACTGGAATTCTCGGGTGCCAAGGTACCAAGAAACCTTCATT |
| OC43-Probe I.106 | AY391777.1 | OC43-Probe I | TGTGAGAGATTCCCTTCGTAATACGACTCACTATAGGGGAGTGCTGGACAGCCTTGTGTTGCTAGTGAACAGGAGGAGTCTTCTGAAGTCTTAGAGGACACTTTGGATGATGGTCCAAGTGTGGAGACATGGAATTCTCGGGTGCCAAGGTACCAAGAAACCTTCATT |
| OC43-Probe I.107 | AY391777.1 | OC43-Probe I | TGTGAGAGATTCCCTTCGTAATACGACTCACTATAGGGCAGGAGGAGTCTTCTGAAGTCTTAGAGGACACTTTGGATGATGGTCCAAGTGTGGAGACATCTGATTCACAAGTTGAAGAAGATGTAGAATGGAATTCTCGGGTGCCAAGGTACCAAGAAACCTTCATT |
| OC43-Probe I.108 | AY391777.1 | OC43-Probe I | TGTGAGAGATTCCCTTCGTAATACGACTCACTATAGGGGACTTTGGATGATGGTCCAAGTGTGGAGACATCTGATTCACAAGTTGAAGAAGATGTAGAAAATGTCGGATTTTGTTGATCTTGAATCTGTGTGGAATTCTCGGGTGCCAAGGTACCAAGAAACCTTCATT |
| OC43-Probe I.109 | AY391777.1 | OC43-Probe I | TGTGAGAGATTCCCTTCGTAATACGACTCACTATAGGGTCTGATTCACAAGTTGAAGAAGATGTAGAAAATGTCGGATTTTGTTGATCTTGAATCTGTGATTCAGGATTATGAAAATGTTTGTTTTGAGTGGAATTCTCGGGTGCCAAGGTACCAAGAAACCTTCATT |
| OC43-Probe I.110 | AY391777.1 | OC43-Probe I | TGTGAGAGATTCCCTTCGTAATACGACTCACTATAGGGGATGTCGGATTTTGTTGATCTTGAATCTGTGATTCAGGATTATGAAAATGTTTGTTTTGAGTTTTATACTACAGAGCCAGAATTTGTTAAATGGAATTCTCGGGTGCCAAGGTACCAAGAAACCTTCATT |
| OC43-Probe I.111 | AY391777.1 | OC43-Probe I | TGTGAGAGATTCCCTTCGTAATACGACTCACTATAGGGGATTCAGGATTATGAAAATGTTTGTTTTGAGTTTTATACTACAGAGCCAGAATTTGTTAAAGTTTTGGGTCTGTATGTGCCTAAAGCAACTTGGAATTCTCGGGTGCCAAGGTACCAAGAAACCTTCATT |
| OC43-Probe I.112 | AY391777.1 | OC43-Probe I | TGTGAGAGATTCCCTTCGTAATACGACTCACTATAGGGTTTTATACTACAGAGCCAGAATTTGTTAAAGTTTTGGGTCTGTATGTGCCTAAAGCAACTCGCAACAATTGCTGGTTGCGATCAGTTTTGTGGAATTCTCGGGTGCCAAGGTACCAAGAAACCTTCATT |
| OC43-Probe I.113 | AY391777.1 | OC43-Probe I | TGTGAGAGATTCCCTTCGTAATACGACTCACTATAGGGGTTTTGGGTCTGTATGTGCCTAAAGCAACTCGCAACAATTGCTGGTTGCGATCAGTTTTGGCAGTGATGCAGAAATTGCCCTGTCAATTTTGGAATTCTCGGGTGCCAAGGTACCAAGAAACCTTCATT |
| OC43-Probe I.114 | AY391777.1 | OC43-Probe I | TGTGAGAGATTCCCTTCGTAATACGACTCACTATAGGGCGCAACAATTGCTGGTTGCGATCAGTTTTGGCAGTGATGCAGAAATTGCCCTGTCAATTTAAAGATAAAAATTTGCAGGATCTTTGGGTGTGGAATTCTCGGGTGCCAAGGTACCAAGAAACCTTCATT |
| OC43-Probe I.115 | AY391777.1 | OC43-Probe I | TGTGAGAGATTCCCTTCGTAATACGACTCACTATAGGGGCAGTGATGCAGAAATTGCCCTGTCAATTTAAAGATAAAAATTTGCAGGATCTTTGGGTGTTATACAAGCAACAGTATAGTCAGTTGTTTGTGGAATTCTCGGGTGCCAAGGTACCAAGAAACCTTCATT |
| OC43-Probe I.116 | AY391777.1 | OC43-Probe I | TGTGAGAGATTCCCTTCGTAATACGACTCACTATAGGGAAAGATAAAAATTTGCAGGATCTTTGGGTGTTATACAAGCAACAGTATAGTCAGTTGTTTGTTGATACCTTGGTTAATAAGATACCTGCTTGGAATTCTCGGGTGCCAAGGTACCAAGAAACCTTCATT |
| OC43-Probe I.117 | AY391777.1 | OC43-Probe I | TGTGAGAGATTCCCTTCGTAATACGACTCACTATAGGGTTATACAAGCAACAGTATAGTCAGTTGTTTGTTGATACCTTGGTTAATAAGATACCTGCTAATATTGTACTTCCACAAGGTGGTTATGTTTGGAATTCTCGGGTGCCAAGGTACCAAGAAACCTTCATT |

EP 4 239 083 A2

| ASO ID | Accession | Group ID | Custom Array sequence |
|---|---|---|---|
| OC43-Probe I.118 | AY391777.1 | OC43-Probe I | TGTGAGAGATTCCCTTCGTAATACGACTCACTATAGGGGGTTGATACCTTGGTTAATAAGATACCTGCTAATATTGTACTTCCACAAGGTGGTTATGTTGCTGATTTTGCATATTGGTTTTTAACCTTATGGAATTCTCGGGTGCCAAGGTACCAAGAAACCTTCATT |
| OC43-Probe I.119 | AY391777.1 | OC43-Probe I | TGTGAGAGATTCCCTTCGTAATACGACTCACTATAGGGAATATTGTACTTCCACAAGGTGGTTATGTTGCTGATTTTGCATATTGGTTTTTAACCTTATGTGATTGGCAGTGTGTTGCATACTGGAAATGGAATTCTCGGGTGCCAAGGTACCAAGAAACCTTCATT |
| OC43-Probe I.120 | AY391777.1 | OC43-Probe I | TGTGAGAGATTCCCTTCGTAATACGACTCACTATAGGGGGCTGATTTTGCATATTGGTTTTTAACCTTATGTGATTGGCAGTGTGTTGCATACTGGAAATGCATTAAATGTGATTTAGCTCTTAAGCTTTGGAATTCTCGGGTGCCAAGGTACCAAGAAACCTTCATT |
| OC43-Probe I.121 | AY391777.1 | OC43-Probe I | TGTGAGAGATTCCCTTCGTAATACGACTCACTATAGGGTGTGATTGGCAGTGTGTTGCATACTGGAAATGCATTAAATGTGATTTAGCTCTTAAGCTTAAAGGCTTGGATGCTATGTTCTTTTATGGTTGGAATTCTCGGGTGCCAAGGTACCAAGAAACCTTCATT |
| OC43-Probe I.122 | AY391777.1 | OC43-Probe I | TGTGAGAGATTCCCTTCGTAATACGACTCACTATAGGGTGCATTAAATGTGATTTAGCTCTTAAGCTTAAAGGCTTGGATGCTATGTTCTTTTATGGTGATGTTGTTTCACATATATGCAAGTGTGGTTGGAATTCTCGGGTGCCAAGGTACCAAGAAACCTTCATT |
| OC43-Probe I.123 | AY391777.1 | OC43-Probe I | TGTGAGAGATTCCCTTCGTAATACGACTCACTATAGGGAAAGGCTTGGATGCTATGTTCTTTTATGGTGATGTTGTTTCACATATATGCAAGTGTGGTGAGTCTATGGTACTTATTGATGTTGATGTGTGGAATTCTCGGGTGCCAAGGTACCAAGAAACCTTCATT |
| OC43-Probe I.124 | AY391777.1 | OC43-Probe I | TGTGAGAGATTCCCTTCGTAATACGACTCACTATAGGGGATGTTGTTTCACATATATGCAAGTGTGGTGAGTCTATGGTACTTATTGATGTTGATGTGCCATTTACAGCCCACTTTGCTCTTAAAGATTGGAATTCTCGGGTGCCAAGGTACCAAGAAACCTTCATT |
| OC43-Probe I.125 | AY391777.1 | OC43-Probe I | TGTGAGAGATTCCCTTCGTAATACGACTCACTATAGGGGAGTCTATGGTACTTATTGATGTTGATGTGCCATTTACAGCCCACTTTGCTCTTAAAGATAAGTTGTTTTGTGCATTTATTACTAAGCGTTGGAATTCTCGGGTGCCAAGGTACCAAGAAACCTTCATT |
| OC43-Probe I.126 | AY391777.1 | OC43-Probe I | TGTGAGAGATTCCCTTCGTAATACGACTCACTATAGGGGCCATTTACAGCCCACTTTGCTCTTAAAGATAAGTTGTTTTGTGCATTTATTACTAAGCGTATTGTGTATAAAGCAGCTTGTGTTGTGGATTGGAATTCTCGGGTGCCAAGGTACCAAGAAACCTTCATT |
| OC43-Probe I.127 | AY391777.1 | OC43-Probe I | TGTGAGAGATTCCCTTCGTAATACGACTCACTATAGGGAAGTTGTTTTGTGCATTTATTACTAAGCGTATTGTGTATAAAGCAGCTTGTGTTGTGGATGTTAATGATAGTCATTCTATGGCTGTTGTTTGGAATTCTCGGGTGCCAAGGTACCAAGAAACCTTCATT |
| OC43-Probe I.128 | AY391777.1 | OC43-Probe I | TGTGAGAGATTCCCTTCGTAATACGACTCACTATAGGGATTGTGTATAAAGCAGCTTGTGTTGTGGATGTTAATGATAGTCATTCTATGGCTGTTGTTGATGGTAAACAAATTGATGATCATCGTATCTGGAATTCTCGGGTGCCAAGGTACCAAGAAACCTTCATT |
| OC43-Probe I.129 | AY391777.1 | OC43-Probe I | TGTGAGAGATTCCCTTCGTAATACGACTCACTATAGGGGGTTAATGATAGTCATTCTATGGCTGTTGTTGATGGTAAACAAATTGATGATCATCGTATCCTAGTATTACTAGTGATAAGTTTGATTTTGGAATTCTCGGGTGCCAAGGTACCAAGAAACCTTCATT |
| OC43-Probe I.130 | AY391777.1 | OC43-Probe I | TGTGAGAGATTCCCTTCGTAATACGACTCACTATAGGGGATGGTAAACAAATTGATGATCATCGTATCCTAGTATTACTAGTGATAAGTTTGATTTTATTATTGGGCATGGTATGTCATTTTCAATGTGGAATTCTCGGGTGCCAAGGTACCAAGAAACCTTCATT |
| OC43-Probe I.131 | AY391777.1 | OC43-Probe I | TGTGAGAGATTCCCTTCGTAATACGACTCACTATAGGGGACTAGTATTACTAGTGATAAGTTTGATTTTATTATTGGGCATGGTATGTCATTTTCAATGACTACTTTTGAAATTGCCCAATTGTATGGTTGGAATTCTCGGGTGCCAAGGTACCAAGAAACCTTCATT |
| OC43-Probe I.132 | AY391777.1 | OC43-Probe I | TGTGAGAGATTCCCTTCGTAATACGACTCACTATAGGGATTATTGGGCATGGTATGTCATTTTCAATGACTACTTTTGAAATTGCCCAATTGTATGGTTCTTGTATAACACCTAATGTGTGTTTTTGTTGGAATTCTCGGGTGCCAAGGTACCAAGAAACCTTCATT |
| OC43-Probe I.133 | AY391777.1 | OC43-Probe I | TGTGAGAGATTCCCTTCGTAATACGACTCACTATAGGGACTACTTTTGAAATTGCCCAATTGTATGGTTCTTGTATAACACCTAATGTGTGTTTTTGTTAAAGGTGATATAATTAAAGTATCTAAGCTTTGGAATTCTCGGGTGCCAAGGTACCAAGAAACCTTCATT |
| OC43-Probe I.134 | AY391777.1 | OC43-Probe I | TGTGAGAGATTCCCTTCGTAATACGACTCACTATAGGGTCTTGTATAACACCTAATGTGTGTTTTTGTTAAAGGTGATATAATTAAAGTATCTAAGCTTGTTAAAGCAGAAGTTGTTGTAAACCCTGCTTGGAATTCTCGGGTGCCAAGGTACCAAGAAACCTTCATT |
| OC43-Probe I.135 | AY391777.1 | OC43-Probe I | TGTGAGAGATTCCCTTCGTAATACGACTCACTATAGGGAAAGGTGATATAATTAAAGTATCTAAGCTTGTTAAAGCAGAAGTTGTTGTAAACCCTGCTAATGGCCATATGGCACATGGTGGTGGTGTTTGGAATTCTCGGGTGCCAAGGTACCAAGAAACCTTCATT |
| OC43-Probe I.136 | AY391777.1 | OC43-Probe I | TGTGAGAGATTCCCTTCGTAATACGACTCACTATAGGGGGTTAAAGCAGAAGTTGTTGTAAACCCTGCTAATGGCCATATGGCACATGGTGGTGGTGTTGCAAAAGCTATTGCAGTAGCAGCTGGACAGTGGAATTCTCGGGTGCCAAGGTACCAAGAAACCTTCATT |
| OC43-Probe I.137 | AY391777.1 | OC43-Probe I | TGTGAGAGATTCCCTTCGTAATACGACTCACTATAGGGGAATGGCCATATGGCACATGGTGGTGGTGTTGCAAAAGCTATTGCAGTAGCAGCTGGACAGCAGTTTGTTAAAGAGACTACCGATATGGTTTGGAATTCTCGGGTGCCAAGGTACCAAGAAACCTTCATT |
| OC43-Probe I.138 | AY391777.1 | OC43-Probe I | TGTGAGAGATTCCCTTCGTAATACGACTCACTATAGGGGGCAAAAGCTATTGCAGTAGCAGCTGGACAGCAGTTTGTTAAAGAGACTACCGATATGGTTAAGTCTAAAGGAGTTTGTGCTACTGGAGATTGGAATTCTCGGGTGCCAAGGTACCAAGAAACCTTCATT |
| OC43-Probe I.139 | AY391777.1 | OC43-Probe I | TGTGAGAGATTCCCTTCGTAATACGACTCACTATAGGGCAGTTTGTTAAAGAGACTACCGATATGGTTAAGTCTAAAGGAGTTTGTGCTACTGGAGATTGTTATGTCTCTACAGGGGGCAAATTATGTTGGAATTCTCGGGTGCCAAGGTACCAAGAAACCTTCATT |
| OC43-Probe I.140 | AY391777.1 | OC43-Probe I | TGTGAGAGATTCCCTTCGTAATACGACTCACTATAGGGAAGTCTAAAGGAGTTTGTGCTACTGGAGATTGTTATGTCTCTACAGGGGGCAAATTATGTAAAACTGTGCTTAATGTTGTTGGACCTGATTGGAATTCTCGGGTGCCAAGGTACCAAGAAACCTTCATT |
| OC43-Probe I.141 | AY391777.1 | OC43-Probe I | TGTGAGAGATTCCCTTCGTAATACGACTCACTATAGGGTGTTATGTCTCTACAGGGGGCAAATTATGTAAAACTGTGCTTAATGTTGTTGGACCTGATGCGAGAACACAGGGTAAACAAAGTTATGTATGGAATTCTCGGGTGCCAAGGTACCAAGAAACCTTCATT |
| OC43-Probe I.142 | AY391777.1 | OC43-Probe I | TGTGAGAGATTCCCTTCGTAATACGACTCACTATAGGGAAAACTGTGCTTAATGTTGTTGGACCTGATGCGAGAACACAGGGTAAACAAAGTTATGTATTGTTAGAGCGTGTTTATAAACATCTTAACTGGAATTCTCGGGTGCCAAGGTACCAAGAAACCTTCATT |

| ASO ID | Accession | Group ID | Custom Array sequence |
|---|---|---|---|
| OC43-Probe I.143 | AY391777.1 | OC43-Probe I | TGTGAGAGATTCCCTTCGTAATACGACTCACTATAGGGGCGAGAACACAGGGTAAACAAAGTTATGTATTGTTAGAGCGTGTTTATAAACATCTTAACAACTATGACTGTGTTGTTACAACTTTGATCTGGAATTCTCGGGTGCCAAGGTACCAAGAAACCTTCATT |
| OC43-Probe I.144 | AY391777.1 | OC43-Probe I | TGTGAGAGATTCCCTTCGTAATACGACTCACTATAGGGTTGTTAGAGCGTGTTTATAAACATCTTAACAACTATGACTGTGTTGTTACAACTTTGATCTCAGCTGGTATATTTAGTGTGCCTTCTGATTGGAATTCTCGGGTGCCAAGGTACCAAGAAACCTTCATT |
| OC43-Probe I.145 | AY391777.1 | OC43-Probe I | TGTGAGAGATTCCCTTCGTAATACGACTCACTATAGGGAACTATGACTGTGTTGTTACAACTTTGATCTCAGCTGGTATATTTAGTGTGCCTTCTGATGTGTCTTTAACATATCTACTTGGTACTGCTTGGAATTCTCGGGTGCCAAGGTACCAAGAAACCTTCATT |
| OC43-Probe I.146 | AY391777.1 | OC43-Probe I | TGTGAGAGATTCCCTTCGTAATACGACTCACTATAGGGTCAGCTGGTATATTTAGTGTGCCTTCTGATGTGTCTTTAACATATCTACTTGGTACTGCTAAGACAACAAGTTGTTCTTGTTAGCAATAATTGGAATTCTCGGGTGCCAAGGTACCAAGAAACCTTCATT |
| OC43-Probe I.147 | AY391777.1 | OC43-Probe I | TGTGAGAGATTCCCTTCGTAATACGACTCACTATAGGGGTGTCTTTAACATATCTACTTGGTACTGCTAAGACAACAAGTTGTTCTTGTTAGCAATAATCAAGAGGATTTTGATCTTATTTCTAAGTGTTGGAATTCTCGGGTGCCAAGGTACCAAGAAACCTTCATT |
| OC43-Probe I.148 | AY391777.1 | OC43-Probe I | TGTGAGAGATTCCCTTCGTAATACGACTCACTATAGGGAAGACAACAAGTTGTTCTTGTTAGCAATAATCAAGAGGATTTTGATCTTATTTCTAAGTGTCAGATAACTGCTGTTGAGGGCACTAAGAAATGGAATTCTCGGGTGCCAAGGTACCAAGAAACCTTCATT |
| OC43-Probe I.149 | AY391777.1 | OC43-Probe I | TGTGAGAGATTCCCTTCGTAATACGACTCACTATAGGGCAAGAGGATTTTGATCTTATTTCTAAGTGTCAGATAACTGCTGTTGAGGGCACTAAGAAATTGGCAGCGCGTCTTTCTTTTAATGTTGGATGGAATTCTCGGGTGCCAAGGTACCAAGAAACCTTCATT |
| OC43-Probe I.150 | AY391777.1 | OC43-Probe I | TGTGAGAGATTCCCTTCGTAATACGACTCACTATAGGGCAGATAACTGCTGTTGAGGGCACTAAGAAATTGGCAGCGCGTCTTTCTTTTAATGTTGGACGTTCCATTGTTTACGAAACAGATGCTAATTGGAATTCTCGGGTGCCAAGGTACCAAGAAACCTTCATT |
| OC43-Probe I.151 | AY391777.1 | OC43-Probe I | TGTGAGAGATTCCCTTCGTAATACGACTCACTATAGGGTTGGCAGCGCGTCTTTCTTTTAATGTTGGACGTTCCATTGTTTACGAAACAGATGCTAATAAGTTGATTTTAATCAATGACGTTGCATTTTGGAATTCTCGGGTGCCAAGGTACCAAGAAACCTTCATT |
| OC43-Probe I.152 | AY391777.1 | OC43-Probe I | TGTGAGAGATTCCCTTCGTAATACGACTCACTATAGGGCGTTCCATTGTTTACGAAACAGATGCTAATAAGTTGATTTTAATCAATGACGTTGCATTTGTTTCGACATTTAATGTTTTACAGGATGTTTGGAATTCTCGGGTGCCAAGGTACCAAGAAACCTTCATT |
| OC43-Probe I.153 | AY391777.1 | OC43-Probe I | TGTGAGAGATTCCCTTCGTAATACGACTCACTATAGGGAAGTTGATTTTAATCAATGACGTTGCATTTGTTTCGACATTTAATGTTTTACAGGATGTTTATCCTTAAGACATGATATAGCACTTGATTGGAATTCTCGGGTGCCAAGGTACCAAGAAACCTTCATT |
| OC43-Probe I.154 | AY391777.1 | OC43-Probe I | TGTGAGAGATTCCCTTCGTAATACGACTCACTATAGGGGTTTCGACATTTAATGTTTTACAGGATGTTTATCCTTAAGACATGATATAGCACTTGATGATGATGCACGAACCTTCGTTCAGAGCAATTGGAATTCTCGGGTGCCAAGGTACCAAGAAACCTTCATT |
| OC43-Probe I.155 | AY391777.1 | OC43-Probe I | TGTGAGAGATTCCCTTCGTAATACGACTCACTATAGGGTTATCCTTAAGACATGATATAGCACTTGATGATGATGCACGAACCTTCGTTCAGAGCAATGTTGATGTTGTACCTGAGGGTTGGCGTGTTTGGAATTCTCGGGTGCCAAGGTACCAAGAAACCTTCATT |
| OC43-Probe I.156 | AY391777.1 | OC43-Probe I | TGTGAGAGATTCCCTTCGTAATACGACTCACTATAGGGGATGATGCACGAACCTTCGTTCAGAGCAATGTTGATGTTGTACCTGAGGGTTGGCGTGTTGTCAATAAGTTTTATCAAATTAATGGTGTTTGGAATTCTCGGGTGCCAAGGTACCAAGAAACCTTCATT |
| OC43-Probe I.157 | AY391777.1 | OC43-Probe I | TGTGAGAGATTCCCTTCGTAATACGACTCACTATAGGGGTTGATGTTGTACCTGAGGGTTGGCGTGTTGTCAATAAGTTTTATCAAATTAATGGTGTTAGAACCGTTAAGTATTTTGAGTGTACTGGATGGAATTCTCGGGTGCCAAGGTACCAAGAAACCTTCATT |
| OC43-Probe I.158 | AY391777.1 | OC43-Probe I | TGTGAGAGATTCCCTTCGTAATACGACTCACTATAGGGGTCAATAAGTTTTATCAAATTAATGGTGTTAGAACCGTTAAGTATTTTGAGTGTACTGGAGGCATAGATATATGCAGCCAGGATAAAGTTTGGAATTCTCGGGTGCCAAGGTACCAAGAAACCTTCATT |
| OC43-Probe I.159 | AY391777.1 | OC43-Probe I | TGTGAGAGATTCCCTTCGTAATACGACTCACTATAGGGGAGAACCGTTAAGTATTTTGAGTGTACTGGAGGCATAGATATATGCAGCCAGGATAAAGTTTTTGGTTATGTACAGCAGGGTATTTTTAATTGGAATTCTCGGGTGCCAAGGTACCAAGAAACCTTCATT |
| OC43-Probe I.160 | AY391777.1 | OC43-Probe I | TGTGAGAGATTCCCTTCGTAATACGACTCACTATAGGGGGGCATAGATATATGCAGCCAGGATAAAGTTTTTGGTTATGTACAGCAGGGTATTTTTAATAAGGCTACTGTTGCTCAAATTAAAGCCTTGTGGAATTCTCGGGTGCCAAGGTACCAAGAAACCTTCATT |
| OC43-Probe I.161 | AY391777.1 | OC43-Probe I | TGTGAGAGATTCCCTTCGTAATACGACTCACTATAGGGTTTGGTTATGTACAGCAGGGTATTTTTAATAAGGCTACTGTTGCTCAAATTAAAGCCTTGTTTTTGGATAAAGTGGACATCTTGCTAACTTGGAATTCTCGGGTGCCAAGGTACCAAGAAACCTTCATT |
| OC43-Probe I.162 | AY391777.1 | OC43-Probe I | TGTGAGAGATTCCCTTCGTAATACGACTCACTATAGGGAAGGCTACTGTTGCTCAAATTAAAGCCTTGTTTTTGGATAAAGTGGACATCTTGCTAACTGTTGATGGTGTTAATTTCACTAATAGGTTTTGGAATTCTCGGGTGCCAAGGTACCAAGAAACCTTCATT |
| OC43-Probe I.163 | AY391777.1 | OC43-Probe I | TGTGAGAGATTCCCTTCGTAATACGACTCACTATAGGGTTTTGGATAAAGTGGACATCTTGCTAACTGTTGATGGTGTTAATTTCACTAATAGGTTTGTGCCTGTTGGTGAAAGTTTGGTAAGAGTTGGAATTCTCGGGTGCCAAGGTACCAAGAAACCTTCATT |
| OC43-Probe I.164 | AY391777.1 | OC43-Probe I | TGTGAGAGATTCCCTTCGTAATACGACTCACTATAGGGGTTGATGGTGTTAATTTCACTAATAGGTTTGTGCCTGTTGGTGAAAGTTTGGTAAGAGTCTAGGAAATGTGTTTTGTGATGGAGTTAATGGAATTCTCGGGTGCCAAGGTACCAAGAAACCTTCATT |
| OC43-Probe I.165 | AY391777.1 | OC43-Probe I | TGTGAGAGATTCCCTTCGTAATACGACTCACTATAGGGGTGCCTGTTGGTGAAAGTTTGGTAAGAGTCTAGGAAATGTGTTTTGTGATGGAGTTAATGTCACGAAGCATAAGTGTGATATAAATTATTGGAATTCTCGGGTGCCAAGGTACCAAGAAACCTTCATT |
| OC43-Probe I.166 | AY391777.1 | OC43-Probe I | TGTGAGAGATTCCCTTCGTAATACGACTCACTATAGGGGCTAGGAAATGTGTTTTGTGATGGAGTTAATGTCACGAAGCATAAGTGTGATATAAATTATAAAGGTAAAGTCTTTTTCCAGTTTGATAATTGGAATTCTCGGGTGCCAAGGTACCAAGAAACCTTCATT |
| OC43-Probe I.167 | AY391777.1 | OC43-Probe I | TGTGAGAGATTCCCTTCGTAATACGACTCACTATAGGGGTCACGAAGCATAAGTGTGATATAAATTATAAAGGTAAAGTCTTTTTCCAGTTTGATAATCTTTCTAGTGAAGATTTAAAGGCTGTAAGATGGAATTCTCGGGTGCCAAGGTACCAAGAAACCTTCATT |

| ASO ID | Accession | Group ID | Custom Array sequence |
|---|---|---|---|
| OC43-Probe t.168 | AY391777.1 | OC43-Probe t | TGTGAGAGATCCCCTTCGTAATACGACTCACTATAGGGCAAAGGTAAAGTCTTTTTCCAGTTGATAATCTTTCTAGTGAAGAAGTTCCTTAATTTTGATCAGAGAGAATTGTGGAATTCTCGGGTGCCAAGGTACCAAGAAACCTTCATT |
| OC43-Probe t.169 | AY391777.1 | OC43-Probe t | TGTGAGAGATCCCCTTCGTAATACGACTCACTATAGGGCTTTCTAGTGAAGTCTTTCTAGTGAAGAAGATTTAAAGGCTGTAAGAAGGAATTGCTTGCCTATTACAACATGCTTAATTTTGATCAGAGAGAATTGTGGAATTCTCGGGTGCCAAGGTACCAAGAAACCTTCATT |
| OC43-Probe t.170 | AY391777.1 | OC43-Probe t | TGTGAGAGATCCCCTTCGTAATACGACTCACTATAGGGCTTCCTTAATTTTGATCAGAAGGAGTTCCTTACCATATTACAACATGCTTGTTAATTGTTTAAGTGGTAAGTGGAATTGTGGAATTCTCGGGTGCCAAGGTACCAAGAAACCTTCATT |
| OC43-Probe t.171 | AY391777.1 | OC43-Probe t | TGTGAGAGATCCCCTTCGTAATACGACTCACTATAGGGCTATTACAACATGCTTGCCTATTACAACATGCTTGTTAATTGTTTAAGTGGCAGGTATTTCACTTTTTGTTTAATGTTTCTTGTTAATGTTTCTTAATGGAATTCTCGGGTGCCAAGGTACCAAGAAACCTTCATT |
| OC43-Probe t.172 | AY391777.1 | OC43-Probe t | TGTGAGAGATCCCCTTCGTAATACGACTCACTATAGGGCTGTTGTTAATGGTAAGTGGCAGGTTTAAGTGGCAGGTATTTCACTTTTTGTTTAATGTTTCTTGCTTAATGCTCTGGAATTCTCGGGTGCCAAGGTACCAAGAAACCTTCATT |
| OC43-Probe t.173 | AY391777.1 | OC43-Probe t | TGTGAGAGATCCCCTTCGTAATACGACTCACTATAGGGCTGTTTAAGTGGCAGGTATTTCACTTTTTGTTTAATGTTTCTTGCTTAATGCTCTCCAGAGTTTGCATCTGACATTTAAAATTGTTCAATGGCAGGAATTCTCGGGTGCCAAGGTACCAAGAAACCTTCATT |
| OC43-Probe t.174 | AY391777.1 | OC43-Probe t | TGTGAGAGATCCCCTTCGTAATACGACTCACTATAGGGCTATTTCACTTTTTGTTTAATGTTTCTTGCTTAATGCTCTCCAGAGTTTGCATCTGACATTTAAAATTGTTCAATGGCAGAGGCATGGCTTGAATTCTGAATTCTCGGGTGCCAAGGTACCAAGAAACCTTCATT |
| OC43-Probe t.175 | AY391777.1 | OC43-Probe t | TGTGAGAGATCCCCTTCGTAATACGACTCACTATAGGGCTGCTTAATGCTCTCCAGAGTTTGCATCTGACATTTAAAATTGTTCAATGGCAGAGGCATGGCTTGAATTCGTTGTTGGATGATTTCGTTCGTTGGATTTCTCGGGTGCCAAGGTACCAAGAAACCTTCATT |
| OC43-Probe t.176 | AY391777.1 | OC43-Probe t | TGTGAGAGATCCCCTTCGTAATACGACTCACTATAGGGTCGCCGCCCTGCCTAGATTTGTCGTTCTGGCCAAAGGTGGGTTTAAATTTGGATGATTTCGTTCGTTGTGTTTAGTCAAGTTCAATTTGGATGATTTCGGGTGCCAAGGTACCAAGAAACCTTCATT |
| OC43-Probe t.177 | AY391777.1 | OC43-Probe t | TGTGAGAGATCCCCTTCGTAATACGACTCACTATAGGGTCTTGGCCAAAGGTGGGTTTAAATTTGGATGATTTCGTTCGTTGTGTTTAGTCAAGTTAGTCAAGTTGATTGACTGGGTGCAATATGTGGTTAGTCAAGTTCAATTCTCGGGTGCCAAGGTACCAAGAAACCTTCATT |
| OC43-Probe t.178 | AY391777.1 | OC43-Probe t | TGTGAGAGATCCCCTTCGTAATACGACTCACTATAGGGTTTAAATTTCGTTGCGTTGTGTTTAGTCAAGTTGATTTGACTGGGTGCAATATGTGTGTTAGTCAAGTTTGAAATTGAAATGCATGTATAGTAATGGTAAATGCTGGAATTCTCGGGTGCCAAGGTACCAAGAAACCTTCATT |
| OC43-Probe t.179 | AY391777.1 | OC43-Probe t | TGTGAGAGATCCCCTTCGTAATACGACTCACTATAGGGATCCTGCTGTTGTTGTTTAGTCAAGTTGACTGGGGCAATATGTGTTAGTCAAGTTTGAAATTGAAATTGCATGTATAAATGGTAAATGCTGGTTATGCATTTTGGTACTTGGAATTCTCGGGTGCCAAGGTACCAAGAAACCTTCATT |
| OC43-Probe t.180 | AY391777.1 | OC43-Probe t | TGTGAGAGATCCCCTTCGTAATACGACTCACTATAGGGGTGTGTTAGTCAAGTTGACTGGGCAATATGTGTTAGTCAAGTTTGAAATTGCATGTATAAATGGTAAATGCTGGTTATGCATTTTGGTAACATTGGTACTTCTCGGGTGCCAAGGTACCAAGAAACCTTCATT |
| OC43-Probe t.181 | AY391777.1 | OC43-Probe t | TGTGAGAGATCCCCTTCGTAATACGACTCACTATAGGGCAATATGTGATTTTGAAATTGCATGTATAAATGGTAAAGCAGGAACAGCGTACTGGGTCTGGACGCTGTTATGCATTTTGGTATACTTGGTGTAAAGCTGAGTCGTGAGAATCTGGAATTCTCGGGTGCCAAGGTACCAAGAAACCTTCATT |
| OC43-Probe t.182 | AY391777.1 | OC43-Probe t | TGTGAGAGATCCCCTTCGTAATACGACTCACTATAGGGTGTGTTATGCATTTTGGTAAAGCAGGAACAGCGTACTGGGTCTGGACGCTGTTATGCATTTTGGTATACTTGGTGTAAAGCTGAGTCGTGAGAATCTGGAAAAGCTAATTGATCTTGCCGGTAAAAAGCTAATTTTATCTCGGGTGCCAAGGTACCAAGAAACCTTCATT |
| OC43-Probe t.183 | AY391777.1 | OC43-Probe t | TGTGAGAGATCCCCTTCGTAATACGACTCACTATAGGGCTGGACGCTGTTATGCATTTTGGTATACTTGGTGTAAAGCTGAGTCGTGAGAATCTGGAAAAGCTAATTGATCTTGCCGGTAAAAAGCTAATTTTATGGAATTCATTTTATGGAATTCTCGGGTGCCAAGGTACCAAGAAACCTTCATT |
| OC43-Probe t.184 | AY391777.1 | OC43-Probe t | TGTGAGAGATCCCCTTCGTAATACGACTCACTATAGGGGTGTAAAGCTGAGTCGTGAGAATCTGGAAAAGCTAATTGATCTTGCCGGTAAAAAGCTAATTTTATGGAATTCATTTTATGGTACGATTTGATCATTTTATGGTACGATTTATGGAATTCTCGGGTGCCAAGGTACCAAGAAACCTTCATT |
| OC43-Probe t.185 | AY391777.1 | OC43-Probe t | TGTGAGAGATCCCCTTCGTAATACGACTCACTATAGGGGTGGACTGTTCTTGCCGGTAAAAAGCTAATTCATTGCAGTAATACCTGCTAGTGTAAATTGTACGATTTGATCATTTGCAGTAATACCTGCTAGTGTAAATATTTGGAATTCTCGGGTGCCAAGGTACCAAGAAACCTTCATT |
| OC43-Probe t.186 | AY391777.1 | OC43-Probe t | TGTGAGAGATCCCCTTCGTAATACGACTCACTATAGGGTCGTTGCCGGTAATACCCGTGATTTGATCATTTGCAGTAATACCTGCTAGTGTAAATATTACGTAGGAAGTGCAAATATTACGTAGGAAGTGCAAATATTTATTCTCGGGTGCCAAGGTACCAAGAAACCTTCATT |
| OC43-Probe t.187 | AY391777.1 | OC43-Probe t | TGTGAGAGATCCCCTTCGTAATACGACTCACTATAGGGCATTGTGACGATTTGATCATTTGCAGTAATACACTGCTAGTGTAAATATTACGTAGGAAGTGCAAATATTTTTATAGGTGTAAGGTGATAAGGTTTATAGGTGTAAGGTGATAAGGTTCTCGGGTGCCAAGGTACCAAGAAACCTTCATT |
| OC43-Probe t.188 | AY391777.1 | OC43-Probe t | TGTGAGAGATCCCCTTCGTAATACGACTCACTATAGGGGATTTACCTAAGGGTGTAGGAAGTGCAAATATTTTTATAGGTGTAAGGTGATAAGGTTGGTCATTATGTTTCATGTTTATGATGCTTCATCTCATTATGTTTCATGTTTATGATGCTTTCTCGGGTGCCAAGGTACCAAGAAACCTTCATT |
| OC43-Probe t.189 | AY391777.1 | OC43-Probe t | TGTGAGAGATCCCCTTCGTAATACGACTCACTATAGGGGTTTACCTAAGGGTTTATAGGTGTAAGGTGATAAGGTTAAGGTGAACAATCTTATCAGCTTTATGATGCTTCTAATGTTACAGATGTTCTCAGATGTCGTGCAAGTTGTCAGATTGTCTCGGGTGCCAAGGTACCAAGAAACCTTCATT |
| OC43-Probe t.190 | AY391777.1 | OC43-Probe t | TGTGAGAGATCCCCTTCGTAATACGACTCACTATAGGGGTGATTTCATGTTTATGATGCTTCTAATGTTACAGATGTTCTCAGATGTCGTGCAAGTTGTCAGATTGTCTGTATCTTTAAAATGTTGAAACAAACTTTATATCTTTAAAATGTTGAAACAAACTTCTCGGGTGCCAAGGTACCAAGAAACCTTCATT |
| OC43-Probe t.191 | AY391777.1 | OC43-Probe t | TGTGAGAGATCCCCTTCGTAATACGACTCACTATAGGGGATTGCAGTTGTCTCAGATGTCGTGCAAGTTGTCAGATTGTCTGTATCTTTAAAATGTTGAAACAAACTTTATATCTTTAAAATGTTGAAACAAACTTTGAAAACAAACTTTAAAATGTTGAACCACCTATTCTCGGGTGCCAAGGTACCAAGAAACCTTCATT |
| OC43-Probe t.192 | AY391777.1 | OC43-Probe t | TGTGAGAGATCCCCTTCGTAATACGACTCACTATAGGGGATGGTTCAGATTGTCTGTATCTTTAAAATGTTGAAACAAACTTTAAAATGTTGAACCACCTATTGCTGTTAACCACCTATTTCTCGGGTGCCAAGGTACCAAGAAACCTTCATT |

68

| ASO ID | Accession | Group ID | Custom Array sequence |
|---|---|---|---|
| OC43-Probe I.193 | AY391777.1 | OC43-Probe I | TGTGAGAGATTCCCTTCGTAATACGACTCACTATAGGGTATCTTAAAAATTTGAAACAAACTTTTAAATCGGTGTTAACCACCTATTATTTGGATGATGTTAAGAAAATTGAGTATAAACCTGACTTGTGGAATTCTCGGGTGCCAAGGTACCAAGAAACCTTCATT |
| OC43-Probe I.194 | AY391777.1 | OC43-Probe I | TGTGAGAGATTCCCTTCGTAATACGACTCACTATAGGGTCGGTGTTAACCACCTATTATTTGGATGATGTTAAGAAAATTGAGTATAAACCTGACTTGTCACAATATTATTGTGACGGAGGTAAGTATTGGAATTCTCGGGTGCCAAGGTACCAAGAAACCTTCATT |
| OC43-Probe I.195 | AY391777.1 | OC43-Probe I | TGTGAGAGATTCCCTTCGTAATACGACTCACTATAGGGGTTAAGAAAATTGAGTATAAACCTGACTTGTCACAATATTATTGTGACGGAGGTAAGTATTATACTCAGCGTATTATTAAAGCCCAATTTTGGAATTCTCGGGTGCCAAGGTACCAAGAAACCTTCATT |
| OC43-Probe I.196 | AY391777.1 | OC43-Probe I | TGTGAGAGATTCCCTTCGTAATACGACTCACTATAGGGTCACAATATTATTGTGACGGAGGTAAGTATTATACTCAGCGTATTATTAAAGCCCAATTTAAAACATTCGAGAAAGTAGATGGTGTGTATTGGAATTCTCGGGTGCCAAGGTACCAAGAAACCTTCATT |
| OC43-Probe I.197 | AY391777.1 | OC43-Probe I | TGTGAGAGATTCCCTTCGTAATACGACTCACTATAGGGTATACTCAGCGTATTATTAAAGCCCAATTTAAAACATTCGAGAAAGTAGATGGTGTGTATACTAATTTTAAATTGATAGGACACACCGTCTGGAATTCTCGGGTGCCAAGGTACCAAGAAACCTTCATT |
| OC43-Probe I.198 | AY391777.1 | OC43-Probe I | TGTGAGAGATTCCCTTCGTAATACGACTCACTATAGGGAAAACATTCGAGAAAGTAGATGGTGTGTATACTAATTTTAAATTGATAGGACACACCGTCTGTGACAGTCTTAATGCTAAGTTGGGTTTTGGAATTCTCGGGTGCCAAGGTACCAAGAAACCTTCATT |
| OC43-Probe I.199 | AY391777.1 | OC43-Probe I | TGTGAGAGATTCCCTTCGTAATACGACTCACTATAGGGACTAATTTTAAATTGATAGGACACACCGTCTGTGACAGTCTTAATGCTAAGTTGGGTTTTGATAGCTCTAAAGAGTTTGTTGAATATAAGTGGAATTCTCGGGTGCCAAGGTACCAAGAAACCTTCATT |
| OC43-Probe I.200 | AY391777.1 | OC43-Probe I | TGTGAGAGATTCCCTTCGTAATACGACTCACTATAGGGTGTGACAGTCTTAATGCTAAGTTGGGTTTTGATAGCTCTAAAGAGTTTGTTGAATATAAGTTACTGAGTGGCCAACAGCTACAGGTGATTGGAATTCTCGGGTGCCAAGGTACCAAGAAACCTTCATT |
| OC43-Probe I.201 | AY391777.1 | OC43-Probe I | TGTGAGAGATTCCCTTCGTAATACGACTCACTATAGGGGATAGCTCTAAAGAGTTTGTTGAATATAAGTTACTGAGTGGCCAACAGCTACAGGTGATGTGGTGTTGGCTACTGATGATTTGTATGTTTGGAATTCTCGGGTGCCAAGGTACCAAGAAACCTTCATT |
| OC43-Probe I.202 | AY391777.1 | OC43-Probe I | TGTGAGAGATTCCCTTCGTAATACGACTCACTATAGGGATTACTGAGTGGCCAACAGCTACAGGTGATGTGGTGTTGGCTACTGATGATTTGTATGTTAAGAGATATGAGAGGGGTTGTATTACTTTTTGGAATTCTCGGGTGCCAAGGTACCAAGAAACCTTCATT |
| OC43-Probe I.203 | AY391777.1 | OC43-Probe I | TGTGAGAGATTCCCTTCGTAATACGACTCACTATAGGGGTGGTGTTGGCTACTGATGATTTGTATGTTAAGAGATATGAGAGGGGTTGTATTACTTTTGGTAAACCTGTTATATGGTTAAGCCATGAGTGGAATTCTCGGGTGCCAAGGTACCAAGAAACCTTCATT |
| OC43-Probe I.204 | AY391777.1 | OC43-Probe I | TGTGAGAGATTCCCTTCGTAATACGACTCACTATAGGGAAGAGATATGAGAGGGGTTGTATTACTTTTGGTAAACCTGTTATATGGTTAAGCCATGAGAAAGCTTCCCTCAATTCTTTAACATATTTTTGGAATTCTCGGGTGCCAAGGTACCAAGAAACCTTCATT |
| OC43-Probe I.205 | AY391777.1 | OC43-Probe I | TGTGAGAGATTCCCTTCGTAATACGACTCACTATAGGGGGTAAACCTGTTATATGGTTAAGCCATGAGAAAGCTTCCCTCAATTCTTTAACATATTTTAATAGACCTTCATTGGTTGATGATAATAAATGGAATTCTCGGGTGCCAAGGTACCAAGAAACCTTCATT |
| OC43-Probe I.206 | AY391777.1 | OC43-Probe I | TGTGAGAGATTCCCTTCGTAATACGACTCACTATAGGGAAAGCTTCCCTCAATTCTTTAACATATTTTAATAGACCTTCATTGGTTGATGATAATAAATTTGATGTTTTAAAAGTGGATGATGTTGACTGGAATTCTCGGGTGCCAAGGTACCAAGAAACCTTCATT |
| OC43-Probe I.207 | AY391777.1 | OC43-Probe I | TGTGAGAGATTCCCTTCGTAATACGACTCACTATAGGGGAATAGACCTTCATTGGTTGATGATAATAAATTTGATGTTTTAAAAGTGGATGATGTTGACGATGGTGGTGACAGCTCAGAGAGTGGTGCCTGGAATTCTCGGGTGCCAAGGTACCAAGAAACCTTCATT |
| OC43-Probe I.208 | AY391777.1 | OC43-Probe I | TGTGAGAGATTCCCTTCGTAATACGACTCACTATAGGGTTTGATGTTTTAAAAGTGGATGATGTTGACGATGGTGGTGACAGCTCAGAGAGTGGTGCCAAAGAAACCAAAGAAATCAACATTATTAAGTGGAATTCTCGGGTGCCAAGGTACCAAGAAACCTTCATT |
| OC43-Probe I.209 | AY391777.1 | OC43-Probe I | TGTGAGAGATTCCCTTCGTAATACGACTCACTATAGGGGATGGTGGTGACAGCTCAGAGAGTGGTGCCAAAGAAACCAAAGAAATCAACATTATTAAGTTAAGTGGTGTTAAAAAACCATTTAAGGTTTGGAATTCTCGGGTGCCAAGGTACCAAGAAACCTTCATT |
| OC43-Probe I.210 | AY391777.1 | OC43-Probe I | TGTGAGAGATTCCCTTCGTAATACGACTCACTATAGGGGAAAGAAACCAAAGAAATCAACATTATTAAGTTAAGTGGTGTTAAAAAACCATTTAAGGTTGAAGATAGTGTCATTGTTAATGATGATACTTGGAATTCTCGGGTGCCAAGGTACCAAGAAACCTTCATT |
| OC43-Probe I.211 | AY391777.1 | OC43-Probe I | TGTGAGAGATTCCCTTCGTAATACGACTCACTATAGGGTTAAGTGGTGTTAAAAAACCATTTAAGGTTGAAGATAGTGTCATTGTTAATGATGATACTAGTGAAACCAAATATGTTAAGAGTTTGTCTTGGAATTCTCGGGTGCCAAGGTACCAAGAAACCTTCATT |
| OC43-Probe I.212 | AY391777.1 | OC43-Probe I | TGTGAGAGATTCCCTTCGTAATACGACTCACTATAGGGGAAGATAGTGTCATTGTTAATGATGATACTAGTGAAACCAAATATGTTAAGAGTTTGTCTATTGTTGATGTGTATGATATGTGGCTTACATGGAATTCTCGGGTGCCAAGGTACCAAGAAACCTTCATT |
| OC43-Probe I.213 | AY391777.1 | OC43-Probe I | TGTGAGAGATTCCCTTCGTAATACGACTCACTATAGGGGAGTGAAACCAAATATGTTAAGAGTTTGTCTATTGTTGATGTGTATGATATGTGGCTTACAGGTTGTAAGTATGTTGTTAGAACTGCTAATTGGAATTCTCGGGTGCCAAGGTACCAAGAAACCTTCATT |
| OC43-Probe I.214 | AY391777.1 | OC43-Probe I | TGTGAGAGATTCCCTTCGTAATACGACTCACTATAGGGGATTGTTGATGTGTATGATATGTGGCTTACAGGTTGTAAGTATGTTGTTAGAACTGCTAATGCTTTGAGCAGAGCAGTTAACGTACCTACATGGAATTCTCGGGTGCCAAGGTACCAAGAAACCTTCATT |
| OC43-Probe I.215 | AY391777.1 | OC43-Probe I | TGTGAGAGATTCCCTTCGTAATACGACTCACTATAGGGGGGTTGTAAGTATGTTGTTAGAACTGCTAATGCTTTGAGCAGAGCAGTTAACGTACCTACAATACGTAAGTTTATAAAATTTGGTATGACTTGGAATTCTCGGGTGCCAAGGTACCAAGAAACCTTCATT |
| OC43-Probe I.216 | AY391777.1 | OC43-Probe I | TGTGAGAGATTCCCTTCGTAATACGACTCACTATAGGGGCTTTGAGCAGAGCAGTTAACGTACCTACAATACGTAAGTTTATAAAATTTGGTATGACTCTTGTTAGTATACCAATTGATTTGTTAAATTTGGAATTCTCGGGTGCCAAGGTACCAAGAAACCTTCATT |
| OC43-Probe I.217 | AY391777.1 | OC43-Probe I | TGTGAGAGATTCCCTTCGTAATACGACTCACTATAGGGGATACGTAAGTTTATAAAATTTGGTATGACTCTTGTTAGTATACCAATTGATTTGTTAAATTTAAGAGAGATTAAGCCTGCTGTTAATGTGTGGAATTCTCGGGTGCCAAGGTACCAAGAAACCTTCATT |

| ASO ID | Accession | Group ID | Custom Array sequence |
|---|---|---|---|
| OC43-Probe I.218 | AY391777.1 | OC43-Probe I | TGTGAGAGATTCCCTTCGTAATACGACTCACTATAGGGCTTGTTAGTATACCAATTGATTTGTTAAATTTAAGAGAGATTAAGCCTGCTGTTAATGTGGTTAAAGCTGTGCGAAATAAAATTTCTGTATGGAATTCTCGGGTGCCAAGGTACCAAGAAACCTTCATT |
| OC43-Probe I.219 | AY391777.1 | OC43-Probe I | TGTGAGAGATTCCCTTCGTAATACGACTCACTATAGGGTTAAGAGAGATTAAGCCTGCTGTTAATGTGGTTAAAGCTGTGCGAAATAAAATTTCTGTATGCTTTAATTTTATTAAATGGCTTTTTGTCTGGAATTCTCGGGTGCCAAGGTACCAAGAAACCTTCATT |
| OC43-Probe I.220 | AY391777.1 | OC43-Probe I | TGTGAGAGATTCCCTTCGTAATACGACTCACTATAGGGGTTAAAGCTGTGCGAAATAAAATTTCTGTATGCTTTAATTTTATTAAATGGCTTTTTGTCTTATTATTTGGCTGGATTAAAATATCCGCTTGGAATTCTCGGGTGCCAAGGTACCAAGAAACCTTCATT |
| OC43-Probe I.221 | AY391777.1 | OC43-Probe I | TGTGAGAGATTCCCTTCGTAATACGACTCACTATAGGGTGCTTTAATTTTATTAAATGGCTTTTTGTCTTATTATTTGGCTGGATTAAAATATCCGCTGATAATAAAGTAATCTACACCACAGAAATTTGGAATTCTCGGGTGCCAAGGTACCAAGAAACCTTCATT |
| OC43-Probe I.222 | AY391777.1 | OC43-Probe I | TGTGAGAGATTCCCTTCGTAATACGACTCACTATAGGGTTATTATTTGGCTGGATTAAAATATCCGCTGATAATAAAGTAATCTACACCACAGAAATTGCATCAAAGCTTACGTGTAAGCTTGTAGCTTGGAATTCTCGGGTGCCAAGGTACCAAGAAACCTTCATT |
| OC43-Probe I.223 | AY391777.1 | OC43-Probe I | TGTGAGAGATTCCCTTCGTAATACGACTCACTATAGGGGATAATAAAGTAATCTACACCACAGAAATTGCATCAAAGCTTACGTGTAAGCTTGTAGCTTTAGCTTTTAAAAATGCATTTTTGACATTTTGGAATTCTCGGGTGCCAAGGTACCAAGAAACCTTCATT |
| OC43-Probe I.224 | AY391777.1 | OC43-Probe I | TGTGAGAGATTCCCTTCGTAATACGACTCACTATAGGGCATCAAAGCTTACGTGTAAGCTTGTAGCTTTAGCTTTTAAAAATGCATTTTTGACATTTAAGTGGAGTATGGTTGCTAGAGGTGCTTGCTGGAATTCTCGGGTGCCAAGGTACCAAGAAACCTTCATT |
| OC43-Probe I.225 | AY391777.1 | OC43-Probe I | TGTGAGAGATTCCCTTCGTAATACGACTCACTATAGGGTTAGCTTTTAAAAATGCATTTTTGACATTTAAGTGGAGTATGGTTGCTAGAGGTGCTTGCATTATAGCGACTATATTTCTATTGTGGTTTTGGAATTCTCGGGTGCCAAGGTACCAAGAAACCTTCATT |
| OC43-Probe I.226 | AY391777.1 | OC43-Probe I | TGTGAGAGATTCCCTTCGTAATACGACTCACTATAGGGAAGTGGAGTATGGTTGCTAGAGGTGCTTGCATTATAGCGACTATATTTCTATTGTGGTTTAATTTTATATATGCCAATGTAATTTTTTAGTTGGAATTCTCGGGTGCCAAGGTACCAAGAAACCTTCATT |
| OC43-Probe I.227 | AY391777.1 | OC43-Probe I | TGTGAGAGATTCCCTTCGTAATACGACTCACTATAGGGATTATAGCGACTATATTTCTATTGTGGTTTAATTTTATATATGCCAATGTAATTTTTAGTGATTTTTATTTGCCTAAAATCGGTTTCTTGTGGAATTCTCGGGTGCCAAGGTACCAAGAAACCTTCATT |
| OC43-Probe I.228 | AY391777.1 | OC43-Probe I | TGTGAGAGATTCCCTTCGTAATACGACTCACTATAGGGAATTTTATATATGCCAATGTAATTTTTAGTGATTTTTATTTGCCTAAAATCGGTTTCTTGCCGACTTTTGTTGGTAAGATTGCACAGTGGTGGAATTCTCGGGTGCCAAGGTACCAAGAAACCTTCATT |
| OC43-Probe I.229 | AY391777.1 | OC43-Probe I | TGTGAGAGATTCCCTTCGTAATACGACTCACTATAGGGGATTTTTATTTGCCTAAAATCGGTTTCTTGCCGACTTTTGTTGGTAAGATTGCACAGTGGATTAAGAACACTTTTAGTCTTGTAACTATTTGGAATTCTCGGGTGCCAAGGTACCAAGAAACCTTCATT |
| OC43-Probe I.230 | AY391777.1 | OC43-Probe I | TGTGAGAGATTCCCTTCGTAATACGACTCACTATAGGGCCGACTTTTGTTGGTAAGATTGCACAGTGGATTAAGAACACTTTTAGTCTTGTAACTATTTGTGATCTATATTCCATGCAGGATGTGGGTTGGAATTCTCGGGTGCCAAGGTACCAAGAAACCTTCATT |
| OC43-Probe I.231 | AY391777.1 | OC43-Probe I | TGTGAGAGATTCCCTTCGTAATACGACTCACTATAGGGATTAAGAACACTTTTAGTCTTGTAACTATTTGTGATCTATATTCCATGCAGGATGTGGGTTTTAAGAATCAGTATTGTAATGGAAGTATTTGGAATTCTCGGGTGCCAAGGTACCAAGAAACCTTCATT |
| OC43-Probe I.232 | AY391777.1 | OC43-Probe I | TGTGAGAGATTCCCTTCGTAATACGACTCACTATAGGGTGTGATCTATATTCCATGCAGGATGTGGGTTTTAAGAATCAGTATTGTAATGGAAGTATTGCATGTCAGTTCTGCTTGGCAGGATTTGATTGGAATTCTCGGGTGCCAAGGTACCAAGAAACCTTCATT |
| OC43-Probe I.233 | AY391777.1 | OC43-Probe I | TGTGAGAGATTCCCTTCGTAATACGACTCACTATAGGGTTTAAGAATCAGTATTGTAATGGAAGTATTGCATGTCAGTTCTGCTTGGCAGGATTTGATATGTTAGATAATTATAAAGCCATTGATGTATGGAATTCTCGGGTGCCAAGGTACCAAGAAACCTTCATT |
| OC43-Probe I.234 | AY391777.1 | OC43-Probe I | TGTGAGAGATTCCCTTCGTAATACGACTCACTATAGGGGCATGTCAGTTCTGCTTGGCAGGATTTGATATGTTAGATAATTATAAAGCCATTGATGTAGTACAGTATGAAGCTGATAGGAGAGCATTTGGAATTCTCGGGTGCCAAGGTACCAAGAAACCTTCATT |
| OC43-Probe I.235 | AY391777.1 | OC43-Probe I | TGTGAGAGATTCCCTTCGTAATACGACTCACTATAGGGATGTTAGATAATTATAAAGCCATTGATGTAGTACAGTATGAAGCTGATAGGAGAGCATTTGTTGATTATACAGGTGTGTTAAAGATTGTCTGGAATTCTCGGGTGCCAAGGTACCAAGAAACCTTCATT |
| OC43-Probe I.236 | AY391777.1 | OC43-Probe I | TGTGAGAGATTCCCTTCGTAATACGACTCACTATAGGGGTACAGTATGAAGCTGATAGGAGAGCATTTGTTGATTATACAGGTGTGTTAAAGATTGTCATTGAATTGATAGTTAGTTACGCCCTGTATTGGAATTCTCGGGTGCCAAGGTACCAAGAAACCTTCATT |
| OC43-Probe I.237 | AY391777.1 | OC43-Probe I | TGTGAGAGATTCCCTTCGTAATACGACTCACTATAGGGGTTGATTATACAGGTGTGTTAAAGATTGTCATTGAATTGATAGTTAGTTACGCCCTGTATACGGCATGGTTTTATCCATTGTTTGCCCTTTGGAATTCTCGGGTGCCAAGGTACCAAGAAACCTTCATT |
| OC43-Probe I.238 | AY391777.1 | OC43-Probe I | TGTGAGAGATTCCCTTCGTAATACGACTCACTATAGGGATTGAATTGATAGTTAGTTACGCCCTGTATACGGCATGGTTTTATCCATTGTTTGCCCTTATCAGTATTCAGATCTTGACCACTTGGCTGTGGAATTCTCGGGTGCCAAGGTACCAAGAAACCTTCATT |
| OC43-Probe I.239 | AY391777.1 | OC43-Probe I | TGTGAGAGATTCCCTTCGTAATACGACTCACTATAGGGACGGCATGGTTTTATCCATTGTTTGCCCTTATCAGTATTCAGATCTTGACCACTTGGCTGCCTGAGCTTTTTATGCTTAGTACATTACATTGGAATTCTCGGGTGCCAAGGTACCAAGAAACCTTCATT |
| OC43-Probe I.240 | AY391777.1 | OC43-Probe I | TGTGAGAGATTCCCTTCGTAATACGACTCACTATAGGGATCAGTATTCAGATCTTGACCACTTGGCTGCCTGAGCTTTTTATGCTTAGTACATTACATTGGAGTTTTAGGTTGCTGGTGGCTTTAGCTTGGAATTCTCGGGTGCCAAGGTACCAAGAAACCTTCATT |
| OC43-Probe I.241 | AY391777.1 | OC43-Probe I | TGTGAGAGATTCCCTTCGTAATACGACTCACTATAGGGCCTGAGCTTTTTATGCTTAGTACATTACATTGGAGTTTTAGGTTGCTGGTGGCTTTAGCTAATATGTTACCAGCACATGTGTTTATGAGGTGGAATTCTCGGGTGCCAAGGTACCAAGAAACCTTCATT |
| OC43-Probe I.242 | AY391777.1 | OC43-Probe I | TGTGAGAGATTCCCTTCGTAATACGACTCACTATAGGGTGGAGTTTTAGGTTGCTGGTGGCTTTAGCTAATATGTTACCAGCACATGTGTTTATGAGGTTTTATATTATTATTGCCTCTTTTATTAAGTGGAATTCTCGGGTGCCAAGGTACCAAGAAACCTTCATT |

70

| ASO ID | Accession | Group ID | Custom Array sequence |
|---|---|---|---|
| OC43-Probe 1.243 | AY391777.1 | OC43-Probe | TGTGAGAGAATCCCCTTCGTAATACGACTCACTATAGGGATAATGTTACCAGCACATGTGTTATGAGGTTTATATTATTATTATTAAGCTCTTTAGCTTGTTTAGGCATGTGCTATTGGAATTCTCGGGTGCCAAGGTACCAAGAAACCTTCATT |
| OC43-Probe 1.244 | AY391777.1 | OC43-Probe | TGTGAGAGAATCCCCTTCGTAATACGACTCACTATAGGGTTTTTATTATTATTAGCTTAGCTTGTTTATGGCATGTGTTTGTTTTGTTGTTAGTAAATCTGGTTGTTTTTGGAATTCTCGGGTGCCAAGGTACCAAGAAACCTTCATT |
| OC43-Probe 1.245 | AY391777.1 | OC43-Probe | TGTGAGAGAATCCCCTTCGTAATACGACTCACTATAGGGCTCTTTAGCTTGTTTAGGCATGTGCCTATGGTTGTTGTTGTTTGTTACAAGAGGAATCGAGTCTACGTGTTAAATGGAATTCTCGGGTGCCAAGGTACCAAGAAACCTTCATT |
| OC43-Probe 1.246 | AY391777.1 | OC43-Probe | TGTGAGAGAATCCCCTTCGTAATACGACTCACTATAGGGGGTTGTAGTAAATCTGGTTGTTTGTTTTGTTACAAGAGGAATCCGAGTCTACGTGTTAAATGTAGTGGCATGAATCTGGTTTCGGAATTCTCGGGTGCCAAGGTACCAAGAAACCTTCATT |
| OC43-Probe 1.247 | AY391777.1 | OC43-Probe | TGTGAGAGAATCCCCTTCGTAATACGACTCACTATAGGGTACAAGAGGAATCGTAGTCTACGTGTTAAATGTAGTACTATCGGTTGGTGGCATGAATGTTATGGCACTTGGAATTCTCGGGTGCCAAGGTACCAAGAAACCTTCATT |
| OC43-Probe 1.248 | AY391777.1 | OC43-Probe | TGTGAGAGAATCCCCTTCGTAATACGACTCACTATAGGGTGTAGTACTATCGTTGGTGGCATGAATGTTATGGCACTTGGCTAATGGTGGCTAATGGTCTTTTGTTCAAAACATCAATGGAATTCTCGGGTGCCAAGGTACCAAGAAACCTTCATT |
| OC43-Probe 1.249 | AY391777.1 | OC43-Probe | TGTGAGAGAATCCCCTTCGTAATACGACTCACTATAGGGATTACGATGTGTAGTACGCTTGGTGGCTAATGGTGGCACTGGCTTTTTGTTCAAAACATCAATGGAATGATTCTTATAAAACCAGGTAATTGGAATTCTCGGGTGCCAAGGTACCAAGAAACCTTCATT |
| OC43-Probe 1.250 | AY391777.1 | OC43-Probe | TGTGAGAGAATCCCCTTCGTAATACGACTCACTATAGGGCTTTTGTTCAAAACATCAATGGAATGGTTGATTCTTATAAAACCAGGTAATACTTTTATTACTGTTGAGGCGCCGCTCTCTGATCTATCTAAGGAATTGGAATTCTCGGGTGCCAAGGTACCAAGAAACCTTCATT |
| OC43-Probe 1.251 | AY391777.1 | OC43-Probe | TGTGAGAGAATCCCCTTCGTAATACGACTCACTATAGGGGATTGATTCTTATAAAACCAGGTAATACTTTTATTACTGTTGAGGCCGCTCTCGATCTATCTAAGGAATTGAAAACGGCCATTCAGTGGAATTCTCGGGTGCCAAGGTACCAAGAAACCTTCATT |
| OC43-Probe 1.252 | AY391777.1 | OC43-Probe | TGTGAGAGAATCCCCTTCGTAATACGACTCACTATAGGGACTTTTATTACTGTTGAGGCCGCTCTCGATCTATCTAAGGAATTGAAAACGGCCATTCAGTCTACAGATGTTGCTTATCATACGGTTGGTTGTTCTATGCGCTGAATTCTCGGGTGCCAAGGTACCAAGAAACCTTCATT |
| OC43-Probe 1.253 | AY391777.1 | OC43-Probe | TGTGAGAGAATCCCCTTCGTAATACGACTCACTATAGGGCTATCTAAGGAATTGAAAACGGCCATTCAGCCTACAGATGTTGCTTATCATACGGTTGGTTGTTCTATGCGCTGGTTATGCAAGTTGGTTCTCATGATCGTGATGGACAGCGCACATGAATTCTCGGGTGCCAAGGTACCAAGAAACCTTCATT |
| OC43-Probe 1.254 | AY391777.1 | OC43-Probe | TGTGAGAGAATCCCCTTCGTAATACGACTCACTATAGGGCCTACAGATGTTGCTTATCATACGGTTGTTCTATGCGCTTGGTTATGCAAGTTGGTTCTCATGATCGTGATGGACAGCGCACATATGATGTTAATGCTAGTTTGTTGTGGATTATGAATTCTCGGGTGCCAAGGTACCAAGAAACCTTCATT |
| OC43-Probe 1.255 | AY391777.1 | OC43-Probe | TGTGAGAGAATCCCCTTCGTAATACGACTCACTATAGGGGATGTTCTATGCGCCTTACAGATGGACAAGTTGGTTATGCAAGTTGGTTCTCATGATCGTGATGGACAGCGCACATATGATGTTAATGCTAGTTTGTTTGTGGATTATGAATTTGTTCTACATTCTAAGGTTGGAATTCTCGGGTGCCAAGGTACCAAGAAACCTTCATT |
| OC43-Probe 1.256 | AY391777.1 | OC43-Probe | TGTGAGAGAATCCCCTTCGTAATACGACTCACTATAGGGTGTTCTATGCGCTTGGTTATGCAAGTTGGTTCTCATGATCGTGATGGACAGCGCACATATGATGTTAATGCTAGTTTGTTTGTGGATTATGAATTTGTTCTACATTCTAAGGTTGGAATTCTCGGGTGCCAAGGTACCAAGAAACCTTCATT |
| OC43-Probe 1.257 | AY391777.1 | OC43-Probe | TGTGAGAGAATCCCCTTCGTAATACGACTCACTATAGGGTATGATGGACAAGTTGGTATGATGATGTTAATGCTAGTTTGTTTGTGTACATTCTAAGGTTGGTGGATAGTTGTGTTGGTATGATGTTGCCTAATATGCATGTTGTGTATGGAAATATGATGTGCCTAATATGCATGTTGTGTATGGAAATATGGAATTCTCGGGTGCCAAGGTACCAAGAAACCTTCATT |
| OC43-Probe 1.258 | AY391777.1 | OC43-Probe | TGTGAGAGAATCCCCTTCGTAATACGACTCACTATAGGGGATTAATAGTAATTTGCTACATTCTAAGGTTGGTGGATAGTTGTGTTGCCTAATATGCATGTTGTGTATGGAAATATGATGTTGCCTAATATGCATGTTGTGTATGGAAATGATGGGTAGTGGAAAATGATGGCTAATATGCTGATAAAGCCAATTTGGAATTCTCGGGTGCCAAGGTACCAAGAAACCTTCATT |
| OC43-Probe 1.259 | AY391777.1 | OC43-Probe | TGTGAGAGAATCCCCTTCGTAATACGACTCACTATAGGGCTACATTCTAAGGTTGGTGGATAGTTGTGTTGCCTAATATGCATGTTGTGTATGGAAATGATGGGTAGTGGAAAATGATGGCTAATAATGTGCTGATAAAGCCAATTTCTGAATGCTGCTGTATTTTATGCACAGTCTGTTTATGCACAGTCTGTGAATTCTCGGGTGCCAAGGTACCAAGAAACCTTCATT |
| OC43-Probe 1.260 | AY391777.1 | OC43-Probe | TGTGAGAGAATCCCCTTCGTAATACGACTCACTATAGGGGGTGGTGGAAAGAGTGTGCCTAATATGCATGTTGTGTATGGAAATGATGGGTAGTGGAAAATGATGGCTAACGCAATTTCTGAATGCTGCTGTATTTTATGCACAGTCTTTAATGGTTGGATAAATGGTGAATGGGAATTCTCGGGTGCCAAGGTACCAAGAAACCTTCATT |
| OC43-Probe 1.261 | AY391777.1 | OC43-Probe | TGTGAGAGAATCCCCTTCGTAATACGACTCACTATAGGGCTGTCTGTATTTTATGGTTGGATAAATGGTGAATGCTGCTGTATTTTATGCACAGTCTTTAGACCTATTTTATGTTGTTTAGACCTATTTTGTTTAGACCTATTTTGAAAATCTGATAAACTGTCTTCTTACAGAGGTACGTGCGAATTCTCGGGTGCCAAGGTACCAAGAAACCTTCATT |
| OC43-Probe 1.262 | AY391777.1 | OC43-Probe | TGTGAGAGAATCCCCTTCGTAATACGACTCACTATAGGGGGTTGTTTAGACCTATTTTATGTTGTTTAGACCTATTTTAATGGTTGATATTTTAGTTATTTAGTTTGGATATTGTTACAGAAACTGATAAACTGTCTTCTTACAGATAATCTGATAAACTATGTTTACTGGTACGTGCGTCTGTTACAACTGGAATTCTCGGGTGCCAAGGTACCAAGAAACCTTCATT |
| OC43-Probe 1.263 | AY391777.1 | OC43-Probe | TGTGAGAGAATCCCCTTCGTAATACGACTCACTATAGGGTGATATTTAAGTGTTGATTAGACCTATTTTAATGGTTTATTGTTACAGAAAATCTGATAAACTATGTTTACTGGTACGTGCGTCTGTTTATTGGATGTTTTGTCTATGTTTGATGGTGGATAGTGTGGAATTCTCGGGTGCCAAGGTACCAAGAAACCTTCATT |
| OC43-Probe 1.264 | AY391777.1 | OC43-Probe | TGTGAGAGAATCCCCTTCGTAATACGACTCACTATAGGGTCTGTTAAATCTGATAAACTATGTTTATGGATACATTTTGTCTATGTTTGTGATGTCTTTAATGTGTTATGTGGATAAAAGAGTCTTTAATAAGAGTCTTAATCTTTTAATACAGAGTGTTTATGCACAGTCTGTGGAATTCTCGGGTGCCAAGGTACCAAGAAACCTTCATT |
| OC43-Probe 1.265 | AY391777.1 | OC43-Probe | TGTGAGAGAATCCCCTTCGTAATACGACTCACTATAGGGATGTGGATGTTTTGTCTATGTTTGTGATGTCTTTAATGTGTTATGTGGATAGTGTGGATGTTTATGTGGATGGATAAAAGAGTCTTAATCTTTTAATACAGGGTACGTGCGGATTACCTTTTTAAAGTTTGGATACCTTTTTAAAGGAATTCTCGGGTGCCAAGGTACCAAGAAACCTTCATT |
| OC43-Probe 1.266 | AY391777.1 | OC43-Probe | TGTGAGAGAATCCCCTTCGTAATACGACTCACTATAGGGGGTGGATGTCTTTAATGTGTTATGTGGATAGTGTGGATGTTTATGGATGGATAGTGTGGATGTTTATGGATGGATAAAAGAGTCTTAATCTTTTAATACAGGGTACGTGCGCATTCTCTATAAAAACAGGGTACGCAGATTTATAAAGTTTGGATACCTTTTTAAAGTTTGGATACCTTTTGGAATTCTCGGGTGCCAAGGTACCAAGAAACCTTCATT |
| OC43-Probe 1.267 | AY391777.1 | OC43-Probe | TGTGAGAGAATCCCCTTCGTAATACGACTCACTATAGGGTGTAAAAGTTGTTCTATGTTCTATTGATTGATGAAGTGTGTGTTTATGGATACCTTTTTAAAGTTTGGATACCTTTTAATAGCAGGGTACGCAGATTTATAAAGTTTGGATACCTTTTTAAAGTTTGGATACCTTTTAATGATTGGATACCTTTTAATGATTGGAATTCTCGGGTGCCAAGGTACCAAGAAACCTTCATT |

71

| ASO ID | Accession | Group ID | Custom Array sequence |
|---|---|---|---|
| OC43-Probe 1.268 | AY391777.1 | OC43-Probe ! | TGTGAGAGATTCCCCTTCGTAATACGACTCACTATAGGGCAGATTTATAAAGTTTTGGATACCTTTTTAAGCTGTGCTCGTAAAAGTTGTTCTATTGATTCAGATGTTGATACTCAGATGTTGATCCAAGGTACCAAGAAACCTTCATT |
| OC43-Probe 1.269 | AY391777.1 | OC43-Probe ! | TGTGAGAGATTCCCCTTCGTAATACGACTCACTATAGGGGAGCTGTGCTCGTAAAAGTTGTTCTATTGATTCAGATGTTAGCTGATCTGCGTATCGGCAGGTCTTGGAATTCTCGGGTGCCAAGGTACCAAGAAACCTTCATT |
| OC43-Probe 1.270 | AY391777.1 | OC43-Probe ! | TGTGAGAGATTCCCCTTCGTAATACGACTCACTATAGGGTCAGATGTTAGCTGATCTGCGTATCGAATTGTAATAACTTGTGGAATTCTCGGGTGCCAAGGTACCAAGAAACCTTCATT |
| OC43-Probe 1.271 | AY391777.1 | OC43-Probe ! | TGTGAGAGATTCCCCTTCGTAATACGACTCACTATAGGGTCGTCATGTCTGCGTATCGGCAGGTCTTGAATTGACAGGATGAAAGTTGTAATAACTTGGTGCCAACATATTTGAAGAGTGCACAACATTTGAAGGTACCAAGAAACCTTCATT |
| OC43-Probe 1.272 | AY391777.1 | OC43-Probe ! | TGTGAGAGATTCCCCTTCGTAATACGACTCACTATAGGGGAATTGACGGATGAAAGTTGTAATAACTTGGTGCCAACATATTTGAAGAGTGCAGCTGATTTAGGTGTTCTGATTCAATGAAGGTACCAAGAAACCTTCATT |
| OC43-Probe 1.273 | AY391777.1 | OC43-Probe ! | TGTGAGAGATTCCCCTTCGTAATACGACTCACTATAGGGTGCCAACAATTGGACAGTGACAACATTGGTGGCAGCTGATTTAGGTGTTCTGATTCAAAATTCTGCAAAGCATGGTGCCAAGGTACCAAGAAACCTTCATT |
| OC43-Probe 1.274 | AY391777.1 | OC43-Probe ! | TGTGAGAGATTCCCCTTCGTAATACGACTCACTATAGGGTTAGGTGTTCTGATTCAAAATTCTGCAAAGCATGTGCAGGGTAATGTTCCTGTATATGGTCGTAAAATAGCTGGTGCCAAGGTACCAAGAAACCTTCATT |
| OC43-Probe 1.275 | AY391777.1 | OC43-Probe ! | TGTGAGAGATTCCCCTTCGTAATACGACTCACTATAGGGAATTCTGCAAAGCATGTGCAGGGTAATGTTCCTGTATATGGTCGTAAAATAGCTGGTGTTTAATCAGTTAGTTCTGATTCCAGCATAAATGAAGGTACCAAGAAACCTTCATT |
| OC43-Probe 1.276 | AY391777.1 | OC43-Probe ! | TGTGAGAGATTCCCCTTCGTAATACGACTCACTATAGGGCTAAAATAGCTGGTGTTTAATCAGTTAGTTCTGATTCCAGCATAAATGAAGAAAGCATGTTAGTTCTGATTTCGATTGTAAAACTGGTTTGAAACTGAAGGTACCAAGAAACCTTCATT |
| OC43-Probe 1.277 | AY391777.1 | OC43-Probe ! | TGTGAGAGATTCCCCTTCGTAATACGACTCACTATAGGGTCTGTGGATGCTTTTAATCAGTTAGTTCTGATTTCCAGCATAAATGAAGAAAGCATGGTTTGAAACTGAAAGCTGGCTAATGTCTGTTTGGTACCAAGAAACCTTCATT |
| OC43-Probe 1.278 | AY391777.1 | OC43-Probe ! | TGTGAGAGATTCCCCTTCGTAATACGACTCACTATAGGGGATTTCCAGCATAAATTGAAAACTGGTTTGTAAAACTGGTTGTAATAATAAGCAGATGGCTAATGTCTCTGTTTTAACTACACCCTTAGTCTTAAAGGGGGTGCAAGGTACCAAGAAACCTTCATT |
| OC43-Probe 1.279 | AY391777.1 | OC43-Probe ! | TGTGAGAGATTCCCCTTCGTAATACGACTCACTATAGGGTGTAAAACTGGTTGTAATAATAAGCAGATGGCTAATGTCTCTGTTTTAACTACACCCTTAGTCTTAAAGGGGGTGCAGTTTATTTTGTTATGTGTGTTTGGTACCAAGAAACCTTCATT |
| OC43-Probe 1.280 | AY391777.1 | OC43-Probe ! | TGTGAGAGATTCCCCTTCGTAATACGACTCACTATAGGGTTAGTCTTAAAGGGGGTGCAGTTTATTTTGTTATGTGTGTTTGGTCTGTTTTTGTTGTTATTGGTTCGTGTTTATTGGACTGTGGTCTTAATGCCAAGGTACCAAGAAACCTTCATT |
| OC43-Probe 1.281 | AY391777.1 | OC43-Probe ! | TGTGAGAGATTCCCCTTCGTAATACGACTCACTATAGGGTGTTTATTTTGTTATGTGTGTTTGGTCTGTTTTTGTTGTTATTGGTTCGTGTTTATTGGACTGTGGTCTTAATGCCCACTTACACAGTACAAATCAGATTTCAGGTACCAAGAAACCTTCATT |
| OC43-Probe 1.282 | AY391777.1 | OC43-Probe ! | TGTGAGAGATTCCCCTTCGTAATACGACTCACTATAGGGTTGGTCTGTTTTTGTTGTTATTGGTTCGTGTTTATTGGACTGTGGTCTTAATGCCCACTTACACAGTACAAATCAGATTTCAGCTTCCCGTTATGGAATTCAGGTACCAAGAAACCTTCATT |
| OC43-Probe 1.283 | AY391777.1 | OC43-Probe ! | TGTGAGAGATTCCCCTTCGTAATACGACTCACTATAGGGTTCGTGTTTATTGGACTGTGGTCTTAATGCCCACTTACACAGTACAAATCAGATTTCAGCTTCCCGTTATGCCGTTATATAAAGTTTTAGATAATGGTGTTGGAATTCAGGTACCAAGAAACCTTCATT |
| OC43-Probe 1.284 | AY391777.1 | OC43-Probe ! | TGTGAGAGATTCCCCTTCGTAATACGACTCACTATAGGGCTCCCGTTATGCCGTTATATAAAGTTTTAGATAATGGTGTTGGAAGTTAGATAAAGTTTTAGATAATGGTGTTGTTGGAAGATGTTTAGCGTTGAAGATGTTTGGTACCAAGAAACCTTCATT |
| OC43-Probe 1.285 | AY391777.1 | OC43-Probe ! | TGTGAGAGATTCCCCTTCGTAATACGACTCACTATAGGGCACAAATCAGATTTCAGCTTATAAAGTTTTAGATAATGGTGTTGGAAGATGTTTAGCGTTGAAGATGTTGTTTGGCGTTCAATTGACAATTGCTTGGTACCAAGAAACCTTCATT |
| OC43-Probe 1.286 | AY391777.1 | OC43-Probe ! | TGTGAGAGATTCCCCTTCGTAATACGACTCACTATAGGGGCCAGTTATAAAGATGTTATTAGAGAATGGTGTTGGAAGATGTTTGTTTGGCGTTCAATTGACAATTGCTTGGCTAACAATTTGAACAATTTGATCAATGGTGGTACCAAGAAACCTTCATT |
| OC43-Probe 1.287 | AY391777.1 | OC43-Probe ! | TGTGAGAGATTCCCCTTCGTAATACGACTCACTATAGGGGATTAGAGAATGTTAGCGTTGAACAATTTGATCAATGGTGTTGTTTGGCGTTCGGTTATTGGTTAATAGTTAAGCAGTAAGCAATTTGTGGTACCAAGAAACCTTCATT |
| OC43-Probe 1.288 | AY391777.1 | OC43-Probe ! | TGTGAGAGATTCCCCTTCGTAATACGACTCACTATAGGGGCGTTGAACAATTTGATCAATGGTGTTGTTGGTCTAAGTTATTATAGTAACAGTATGGCTCTGTCCCATTGTTTGTTCCCATTGTTTGGCTCTTGGTACCAAGAAACCTTCATT |
| OC43-Probe 1.289 | AY391777.1 | OC43-Probe ! | TGTGAGAGATTCCCCTTCGTAATACGACTCACTATAGGGTGTATGAGTCTACAATTTGGTCTAAGTTATTATAGTAACAGTATGGCTCTGTCCCATTGGCTCTTGGCTCTGTATAATAGATCAGGATTTTGGCGTCTTGGTACCAAGAAACCTTCATT |
| OC43-Probe 1.290 | AY391777.1 | OC43-Probe ! | TGTGAGAGATTCCCCTTCGTAATACGACTCACTATAGGGGGTATAGTAACAGTAACAGTAACAGTATGGCTCTGTCCCATTGGCTCTTGGCTCTGTATAATAGATCAGGATTTATGGTCTTATTTGGCGTGTAAAAGTGTTTAATGTCCACAAGTGGTACCAAGAAACCTTCATT |
| OC43-Probe 1.291 | AY391777.1 | OC43-Probe ! | TGTGAGAGATTCCCCTTCGTAATACGACTCACTATAGGGGGTTGCTGTAATAGATCAGGATTTATGGTCTTATTTGGCGTGTAAAAGTGTTTAATGTCCACAAGTGGTGCCACTTTATTTGGCTCTGCTGATGGAGTTGGTACCAAGAAACCTTCATT |
| OC43-Probe 1.292 | AY391777.1 | OC43-Probe ! | TGTGAGAGATTCCCCTTCGTAATACGACTCACTATAGGGGGACAGTGTTATTATTACACACTGGTTATCATGGTTATTCATGTGCTGATGGAGTTGGCAGTGGAGTGCAGTGGAGTGGTACCAAGAAACCTTCATT |

| ASO ID | Accession | Group ID | Custom Array sequence |
|---|---|---|---|
| OC43-Probe I.293 | AY391777.1 | OC43-Probe I | TGTGAGGAGATTCCCTTCGTAATACGACTCACTATAGGGCGATAGTGTTCGCACTTTATTACACATGATGGGCCAGTGTTATACGCCACATAGTCAAATATCGTATTGGAATTCTCGGGTGCCAAGGTACCAAGAAAACCTTCATT |
| OC43-Probe I.294 | AY391777.1 | OC43-Probe I | TGTGAGGAGATTCCCTTCGTAATACGACTCACTATAGGGACCACATAGTCAAATATCGTATTCAATTTTATGCTGATGGTGCAGTGTTATACGCCACATAGTCAGTAGTGGCCGAATTCTCGGGTGCCAAGGTACCAAGAAAACCTTCATT |
| OC43-Probe I.295 | AY391777.1 | OC43-Probe I | TGTGAGGAGATTCCCTTCGTAATACGACTCACTATAGGGTGTTATACGCCACATAGTCTAATTTTATGCTTCCTCTGCCTTGCCACTATGTTACAATGGCCTGGAATTCTCGGGTGCCAAGGTACCAAGAAAACCTTCATT |
| OC43-Probe I.296 | AY391777.1 | OC43-Probe I | TGTGAGGAGATTCCCTTCGTAATACGACTCACTATAGGGTCTAATTTTATGCTAGTGCTAGTGGCTGTGTGCTTTCCTCTGCCACTATGTTATACATGGAATTCTCGGGTGCCAAGGTACCAAGAAAACCTTCATT |
| OC43-Probe I.297 | AY391777.1 | OC43-Probe I | TGTGAGGAGATTCCCTTCGTAATACGACTCACTATAGGGTCTCTGCCACTATGTTTACATGGCCGATGGTAGTCCACAACCTTATTGTTATACAGAGGGCTTATGCAAAATGCTTCTCTGTTGATTGGAATTCTCGGGTGCCAAGGTACCAAGAAAACCTTCATT |
| OC43-Probe I.298 | AY391777.1 | OC43-Probe I | TGTGAGGAGATTCCCTTCGTAATACGACTCACTATAGGGGCTATGCAAAATGCTTATTGTTATACAGAGGGCTTATGCAAAATGGTACCTTCATTGGTGTATAATTGGAATTCTCGGGTGCCAAGGTACCAAGAAAACCTTCATT |
| OC43-Probe I.299 | AY391777.1 | OC43-Probe I | TGTGAGGAGATTCCCTTCGTAATACGACTCACTATAGGGTTATGCAAAATGCTTATGCAAAATGCTTCATTGGTGTATAATCTTCACGTGCGATAAATCTTGCTAAAGGTTTATCCGTTTCAGAAGGTT |
| OC43-Probe I.300 | AY391777.1 | OC43-Probe I | TGTGAGGAGATTCCCTTCGTAATACGACTCACTATAGGGCTTGCATTGGTGTATAATCTTGCTAAAGGTTTATCCGTTTCCAGAAGTGTTGCGAGAAGGGCTTGTACGTTTCGCTATGTC |
| OC43-Probe I.301 | AY391777.1 | OC43-Probe I | TGTGAGGAGATTCCCTTCGTAATACGACTCACTATAGGGCTTGCTAAAGGTTTATCCGTTTCCAGAAGTGTTGCGAGAAGGGCTTGTACGTATCGTGCGTACTCGTTCTATGTCGTATGTC |
| OC43-Probe I.302 | AY391777.1 | OC43-Probe I | TGTGAGGAGATTCCCTTCGTAATACGACTCACTATAGGGCCAGAAGTGTTGCGAGAAGGGCTTGTACGTATCGTGCGTACTCGTTCTATGTCGTGCAGAGTTGGATTATGTGAGGAAGCTGAGTGAGTC |
| OC43-Probe I.303 | AY391777.1 | OC43-Probe I | TGTGAGGAGATTCCCTTCGTAATACGACTCACTATAGGGATCGTGCGTACTCGTTCTATGTCGTGCGATTGGATTATGTGAGGAAGCTGAGTGAGTAGTGGTATATGTCTTAATTTTAATGGTTC |
| OC43-Probe I.304 | AY391777.1 | OC43-Probe I | TGTGAGGAGATTCCCTTCGTAATACGACTCACTATAGGGAGTTGGATTATGTGAGGAAGCTGAGTGAGTAGTGGTATATGTCTTAATTTTAATGGTTCTTGGGTGCTTAATAATGATTATTATAGATCATTGT |
| OC43-Probe I.305 | AY391777.1 | OC43-Probe I | TGTGAGGAGATTCCCTTCGTAATACGACTCACTATAGGGGGTATATGTCTTAATTTTAATGGTTCTTGGGTGCTTAATAATGATTATTATAGATCATTGTGTGTGGAATTCTCGGGTGCCAAGGTACCAAGAAAACCTTCATT |
| OC43-Probe I.306 | AY391777.1 | OC43-Probe I | TGTGAGGAGATTCCCTTCGTAATACGACTCACTATAGGGTCTTAATAATGATTATTATAGATCATTGTGTGTGGTAGAGGATGTTTTTATCAGCTATTTAAAGGTTTATGGAATTCTCGGGTGCCAAGGTACCAAGAAAACCTTCATT |
| OC43-Probe I.307 | AY391777.1 | OC43-Probe I | TGTGAGGAGATTCCCTTCGTAATACGACTCACTATAGGGCCTGGGACCTTTTAATTTATCAGCTATTTAAAGGTTTAGCAGCCTGTGGATTTTTGGCATTGGACTGGAATTCTCGGGTGCCAAGGTACCAAGAAAACCTTCATT |
| OC43-Probe I.308 | AY391777.1 | OC43-Probe I | TGTGAGGAGATTCCCTTCGTAATACGACTCACTATAGGGGATTTAATTTATCAGCTATTTAAAGGTTTAGCAGCCTGTGGATTTTTGGCATTGACTGCTAGTTCCATTGCTCGGTGTATACTGCTGTATTACTGTC |
| OC43-Probe I.309 | AY391777.1 | OC43-Probe I | TGTGAGGAGATTCCCTTCGTAATACGACTCACTATAGGGCCAGCCTGTGGATTTTTGGCATTGACTGCTAGTTCCATTGCTCGGTGTATACTGCTGTATTACTTATTACCTATGGAATTCTCGGGTGCCAAGGTACCAAGAAAACCTTCATT |
| OC43-Probe I.310 | AY391777.1 | OC43-Probe I | TGTGAGGAGATTCCCTTCGTAATACGACTCACTATAGGGCTAGTTCCATTGCTCGGTGTATACTGCTGTATTACTTATTACCTATGGTTTGGTGTTTTTGGTGATTACTGCTTTTGGCTTTGTTTAAACGTGCTTT |
| OC43-Probe I.311 | AY391777.1 | OC43-Probe I | TGTGAGGAGATTCCCTTCGTAATACGACTCACTATAGGGTAGTTCCATTGCTCTGGTGTTGGTGTTTTTGGTGATTACTGCTTTTGGCTTTGTTTAAACGTGTTTTGGTTAAACGTGTTTT |
| OC43-Probe I.312 | AY391777.1 | OC43-Probe I | TGTGAGGAGATTCCCTTCGTAATACGACTCACTATAGGGTAATTGTTTTGGTGATTACTGCTTTTGGCTTTGTTTAAACGTGTTTTGGTGATTACTGCTTTTGGTTTTGTTAAACGTGATGATTTTTGTGTTGGAATTCTCGGGTGCCAAGGTACCAAGAAAACCTTCATT |
| OC43-Probe I.313 | AY391777.1 | OC43-Probe I | TGTGAGGAGATTCCCTTCGTAATACGACTCACTATAGGGGATTGGTGGTGTGTAAAATTTATGATGCTTTTTTGTTTGTGTTCTTGTTGTGTAAGTTTATGATGCTTTTATTTTTTATGCCACGCTTATTTTTGTTATGGAATTCTCGGGTGCCAAGGTACCAAGAAAACCTTCATT |
| OC43-Probe I.314 | AY391777.1 | OC43-Probe I | TGTGAGGAGATTCCCTTCGTAATACGACTCACTATAGGGTTGTTTGTGTTCTTGTTGTGTAAGTTTATGATGCTTTTATTTTTTATGCCACGCTTATTTTTGTTATTTTTATTGTTGTTTACCCCATACTTTCTGTGTATATGCGGGTGCCAAGGTACCAAGAAAACCTTCATT |
| OC43-Probe I.315 | AY391777.1 | OC43-Probe I | TGTGAGGAGATTCCCTTCGTAATACGACTCACTATAGGGTTTTATGCCACGCTTATTTTTGTTATTTTTATTGTTGTTTACCCCATACTTTCTGTGTATATGCTATTTGTTTTCCCTTCGGAGATAAGTGTAATGCACTTCGGGTGCCAAGGTACCAAGAAAACCTTCATT |
| OC43-Probe I.316 | AY391777.1 | OC43-Probe I | TGTGAGGAGATTCCCTTCGTAATACGACTCACTATAGGGTATTGTTGTTTACCCCATACTTTCTGTGTATATGCTATTTGTTTTCCCTTCGGAGATAAGTGTAATGCACTTCGGGAGTATGTATGGCCAGTCGGGTGCCAAGGTACCAAGAAAACCTTCATT |
| OC43-Probe I.317 | AY391777.1 | OC43-Probe I | TGTGAGGAGATTCCCTTCGTAATACGACTCACTATAGGGTATTTGTTTTCCCTTCGGAGATAAGTGTAATGCACTTCGGGAGTATGTATGGCCAGTCGGTTTGTTGCTAATTGGAATTCTCGGGTGCCAAGGTACCAAGAAAACCTTCATT |

73

| ASO ID | Accession | Group ID | Custom Array sequence |
|---|---|---|---|
| OC43-Probe 1.318 | AY391777.1 | OC43-Probe I | TGTGAGAGATTCCCTTCGTAATACGACTCACTATAGGGCACTTACAAATCATGCTTATGTATGGCACTAGTTATGTATGACGACCTTTCAAATCATCTGTTGTTTGCTATATAGCTCGGGTGCCAAGAACCTTCATT |
| OC43-Probe 1.319 | AY391777.1 | OC43-Probe I | TGTGAGAGATTCCCTTCGTAATACGACTCACTATAGGGGATTAGCCTTTATGGTTTTGTTTTGCTATATAGCTGTGTTTGGGTATTTCTTACTGCAGAAAGCTTGGTTGGAATTCTCGGGTGCCAAGGTACCAAGAAACCTTCATT |
| OC43-Probe 1.320 | AY391777.1 | OC43-Probe I | TGTGAGAGATTCCCTTCGTAATACGACTCACTATAGGGGATAGCTGTTTTGTTTGCTATATAGCTCATGCTTTTGGGTATTTCTTACTGCAGAAAGCTTGGTACATTTGAATGGAATTCTCGGGTGCCAAGGTACCAAGAAACCTTCATT |
| OC43-Probe 1.321 | AY391777.1 | OC43-Probe I | TGTGAGAGATTCCCTTCGTAATACGACTCACTATAGGGTATTTTCTTACTGCAGAAAGGCTCTCACTACTTTATGATTACATTTGAAGAAATGGCTCTCACTACTTTTATGATTACAAAAGATTCTTGGAATTCTCGGGTGCCAAGGTACCAAGAAACCTTCATT |
| OC43-Probe 1.322 | AY391777.1 | OC43-Probe I | TGTGAGAGATTCCCTTCGTAATACGACTCACTATAGGGACTTCTGTTCGTAGTGATGGTACATTTGAAGAAATGGCTCTCACTACTTTTATGATTGTAAGCTTAAGGAATTCTCGGGTGCCAAGGTACCAAGAAACCTTCATT |
| OC43-Probe 1.323 | AY391777.1 | OC43-Probe I | TGTGAGAGATTCCCTTCGTAATACGACTCACTATAGGGAAATGGCTCTCACTACTTTTATGATTGTAAGGAAATGTTGTCTGATGTTGCTTTTAATAGAATTCTCGGGTGCCAAGGTACCAAGAAACCTTCATT |
| OC43-Probe 1.324 | AY391777.1 | OC43-Probe I | TGTGAGAGATTCCCTTCGTAATACGACTCACTATAGGGAAAGATTCTTATTGTAAGGAAGAATTCTTATTGTAAGGAAGAATTCTTATTGTAATAATTTGTATAATAAAATATAGGTATTACAGCTGGAATTCTCGGGTGCCAAGGTACCAAGAAACCTTCATT |
| OC43-Probe 1.325 | AY391777.1 | OC43-Probe I | TGTGAGAGATTCCCTTCGTAATACGACTCACTATAGGGTGTCTGATGTTGCTTTTAATAGAATTTGAGTTTGTATAATAAAATATAGGTATTACAGCGGTAGAGTTGGAATTCTCGGGTGCCAAGGTACCAAGAAACCTTCATT |
| OC43-Probe 1.326 | AY391777.1 | OC43-Probe I | TGTGAGAGATTCCCTTCGTAATACGACTCACTATAGGGGAGTTTGTATAATAAAATATAGGTATTACAGCGGTAGAGTTGGGTACTGCTTAAAGCAATGTGGAATTCTCGGGTGCCAAGGTACCAAGAAACCTTCATT |
| OC43-Probe 1.327 | AY391777.1 | OC43-Probe I | TGTGAGAGATTCCCTTCGTAATACGACTCACTATAGGGCTGTTTGTTCTCTCAGTTGGCTAAAGCAATGGACACATTTACCAATAATAATGGTGAATTCTCGGGTGCCAAGGTACCAAGAAACCTTCATT |
| OC43-Probe 1.328 | AY391777.1 | OC43-Probe I | TGTGAGAGATTCCCTTCGTAATACGACTCACTATAGGGGCTGCTTGCTCTCAGTTGGCTAAAGCAATGGACACATTTACCAATAATAATGGTGAGTGATGTCCGTCTGGAATTCTCGGGTGCCAAGGTACCAAGAAACCTTCATT |
| OC43-Probe 1.329 | AY391777.1 | OC43-Probe I | TGTGAGAGATTCCCTTCGTAATACGACTCACTATAGGGGGACACATTTACCAATAATAATGGTGAGTGATGTCCGTCTGTATTGTGTGGAATTCTGGGTATTGTGTGGAATTCTCGGGTGCCAAGGTACCAAGAAACCTTCATT |
| OC43-Probe 1.330 | AY391777.1 | OC43-Probe I | TGTGAGAGATTCCCTTCGTAATACGACTCACTATAGGGGCTTTACCAACCGCCTACTGCTTCCGTCTCAACTTGCAATCTGGTATTGTGTGGAATTCTACTTCTTAAGGTAGAATGGTAAATCCTACTTCTGGAATTCTCGGGTGCCAAGGTACCAAGAAACCTTCATT |
| OC43-Probe 1.331 | AY391777.1 | OC43-Probe I | TGTGAGAGATTCCCTTCGTAATACGACTCACTATAGGGGGTCAACTCATTCTTGCAATCTGGTATTGTGTGGAATTCTACTTCTTAAGGTAGAACCATGGTTTATGGTTGGACTGAATGGTTTATGGTTGGAATTCTCGGGTGCCAAGGTACCAAGAAACCTTCATT |
| OC43-Probe 1.332 | AY391777.1 | OC43-Probe I | TGTGAGAGATTCCCTTCGTAATACGACTCACTATAGGGGAAAATGGTAAATCCTACTTCTACTTCTTAAGGTAGAACCATGGTTTATGGTTGGACTGAATGGTTTATGGTTCCCAGACATGTAATATGTGGAATTCTCGGGTGCCAAGGTACCAAGAAACCTTCATT |
| OC43-Probe 1.333 | AY391777.1 | OC43-Probe I | TGTGAGAGATTCCCTTCGTAATACGACTCACTATAGGGCCATGGTTTATGGTTGGACTGAATGGTTTATGGTTCCCAGACATGTAATATGTTGTCGTTATGGTGGATGACAAGGTCTACTGCTTGGAATTCTCGGGTGCCAAGGTACCAAGAAACCTTCATT |
| OC43-Probe 1.334 | AY391777.1 | OC43-Probe I | TGTGAGAGATTCCCTTCGTAATACGACTCACTATAGGGTTCCCAGACATGTAATATGTTGTCGTTATGGTGGATGACAAGGTCTACTGCTCGTTCTTCAGATATGACTAATCCAGATTATGGAATTCTCGGGTGCCAAGGTACCAAGAAACCTTCATT |
| OC43-Probe 1.335 | AY391777.1 | OC43-Probe I | TGTGAGAGATTCCCTTCGTAATACGACTCACTATAGGGGGATGACAAGGTCTACTGCTCGTTCTTCAGATATGACTAATCCAGATTATAGTTCTGCTTCAGATATGACTAATCCAGATTATGGTGTAGAGTAACATCAAGTGGAATTCTCGGGTGCCAAGGTACCAAGAAACCTTCATT |
| OC43-Probe 1.336 | AY391777.1 | OC43-Probe I | TGTGAGAGATTCCCTTCGTAATACGACTCACTATAGGGGTCTGCTTCAGATATGACTAATCCAGATTATAGTTCTGCTTCAGATATGACTAATCCAGATTATAGTTCTGTGTATGGTAGAGTAACATCAAGTGATTGGAATTCTCGGGTGCCAAGGTACCAAGAAACCTTCATT |
| OC43-Probe 1.337 | AY391777.1 | OC43-Probe I | TGTGAGAGATTCCCTTCGTAATACGACTCACTATAGGGGATGACTAATCCAGATTATAGTTCTGTGTATGGTAGAGTAACATCAAGTGATTTGTGTATTGTTTACTGTATTGTTTGATCGTGTAAGGCTCTGGAATTCTCGGGTGCCAAGGTACCAAGAAACCTTCATT |
| OC43-Probe 1.338 | AY391777.1 | OC43-Probe I | TGTGAGAGATTCCCTTCGTAATACGACTCACTATAGGGGATTTTACTGTATTGTTTACTGTATTGTTTGATCGTGTAAGGCTCAATGTCAAGTCTTACAGTGATGTCTTATCCAATGCGGGGGTTGGAATTCTCGGGTGCCAAGGTACCAAGAAACCTTCATT |
| OC43-Probe 1.339 | AY391777.1 | OC43-Probe I | TGTGAGAGATTCCCTTCGTAATACGACTCACTATAGGGGATCGTGTAAGGCTCAATGTCAAGTCTTACAGTGATGTCTTATCAAATGCGGGGGTTGGTTACGGCCCTGACCCTGCAAAATATACATTTGGTGTGGTTAAACCTGGTGGAATTCTCGGGTGCCAAGGTACCAAGAAACCTTCATT |
| OC43-Probe 1.340 | AY391777.1 | OC43-Probe I | TGTGAGAGATTCCCTTCGTAATACGACTCACTATAGGGAAATCTCGTACGCCGCTGCAAAATTCTCGTACGCCAAAATATACATTTGGTGTGGTTAAACCTGGTGAGACTTTTACTGTTTTAGCTGCTTATAACCGTGGAATTCTCGGGTGCCAAGGTACCAAGAAACCTTCATT |
| OC43-Probe 1.341 | AY391777.1 | OC43-Probe I | TGTGAGAGATTCCCTTCGTAATACGACTCACTATAGGGTGTGGTTAAACCTGGTGAGACTTTTACTGTTTTAGCTGCTTATAACCGTGGAAAACCACAAAGGATGTAGAGACTTTTATTGGAATTCTCGGGTGCCAAGGTACCAAGAAACCTTCATT |
| OC43-Probe 1.342 | AY391777.1 | OC43-Probe I | TGTGAGAGATTCCCTTCGTAATACGACTCACTATAGGGGAGACTTTTATTGGTGTGGTTAGCTGCTTATAACCGTGGAAAACCACAAAGGATGTTATTGGAAATGTTCATGTCAACTAACGTCAGTAGTTATTGGAATTCTCGGGTGCCAAGGTACCAAGAAACCTTCATT |

| ASO ID | Accession | Group ID | Custom Array sequence |
|---|---|---|---|
| OC43-Probe I.343 | AY391777.1 | OC43-Probe I | TGTGAGAGATTCCCTTCGTAATACGACTCACTATAGGGTTAGCTGCTTATAACGGCAAACCACAAGGAGCCTTTCATGTAACTATGCGTAGTAGTTATACCATTAAGGGTTCCTTTTTATGCGGATCTTGGAATTCTCGGGTGCCAAGGTACCAAGAAACCTTCATT |
| OC43-Probe I.344 | AY391777.1 | OC43-Probe I | TGTGAGAGATTCCCTTCGTAATACGACTCACTATAGGGGCCTTTCATGTAACTATGCGTAGTAGTTATACCATTAAGGGTTCCTTTTTATGCGGATCTTGTGGATCTGTTGGTTATGTAATAATGGGTTGGAATTCTCGGGTGCCAAGGTACCAAGAAACCTTCATT |
| OC43-Probe I.345 | AY391777.1 | OC43-Probe I | TGTGAGAGATTCCCTTCGTAATACGACTCACTATAGGGGACCATTAAGGGTTCCTTTTTATGCGGATCTTGTGGATCTGTTGGTTATGTAATAATGGGTGATTGTGTTAAATTTGTTTATATGCATCAATGGAATTCTCGGGTGCCAAGGTACCAAGAAACCTTCATT |
| OC43-Probe I.346 | AY391777.1 | OC43-Probe I | TGTGAGAGATTCCCTTCGTAATACGACTCACTATAGGGTGTGGATCTGTTGGTTATGTAATAATGGGTGATTGTGTTAAATTTGTTTATATGCATCAATGGAGCTTAGTACTGGTTGTCATACTGGTTGGAATTCTCGGGTGCCAAGGTACCAAGAAACCTTCATT |
| OC43-Probe I.347 | AY391777.1 | OC43-Probe I | TGTGAGAGATTCCCTTCGTAATACGACTCACTATAGGGGATTGTGTTAAATTTGTTTATATGCATCAATGGAGCTTAGTACTGGTTGTCATACTGGTACTGACTTCAATGGGGATTTTTATGGTCCTTGGAATTCTCGGGTGCCAAGGTACCAAGAAACCTTCATT |
| OC43-Probe I.348 | AY391777.1 | OC43-Probe I | TGTGAGAGATTCCCTTCGTAATACGACTCACTATAGGGTTGGAGCTTAGTACTGGTTGTCATACTGGTACTGACTTCAATGGGGATTTTTATGGTCCTTATAAGGATGCTCAGGTTGTTCAGTTGCTCTGGAATTCTCGGGTGCCAAGGTACCAAGAAACCTTCATT |
| OC43-Probe I.349 | AY391777.1 | OC43-Probe I | TGTGAGAGATTCCCTTCGTAATACGACTCACTATAGGGACTGACTTCAATGGGGATTTTTATGGTCCTTATAAGGATGCTCAGGTTGTTCAGTTGCTCATTCAGGATTATATACAATCTGTTAATTTTTGGAATTCTCGGGTGCCAAGGTACCAAGAAACCTTCATT |
| OC43-Probe I.350 | AY391777.1 | OC43-Probe I | TGTGAGAGATTCCCTTCGTAATACGACTCACTATAGGGTATAAGGATGCTCAGGTTGTTCAGTTGCTCATTCAGGATTATATACAATCTGTTAATTTTGTAGCATGGCTTTATGCTGCTATACTTAACTGGAATTCTCGGGTGCCAAGGTACCAAGAAACCTTCATT |
| OC43-Probe I.351 | AY391777.1 | OC43-Probe I | TGTGAGAGATTCCCTTCGTAATACGACTCACTATAGGGATTCAGGATTATATACAATCTGTTAATTTTGTAGCATGGCTTTATGCTGCTATACTTAACAATTGTAATTGGTTTGTACAAAGTGATAAGTGGAATTCTCGGGTGCCAAGGTACCAAGAAACCTTCATT |
| OC43-Probe I.352 | AY391777.1 | OC43-Probe I | TGTGAGAGATTCCCTTCGTAATACGACTCACTATAGGGTAGCATGGCTTTATGCTGCTATACTTAACAATTGTAATTGGTTTGTACAAAGTGATAAGTGTTCGTAGAAGATTTTAATGTGTGGGCTTGGAATTCTCGGGTGCCAAGGTACCAAGAAACCTTCATT |
| OC43-Probe I.353 | AY391777.1 | OC43-Probe I | TGTGAGAGATTCCCTTCGTAATACGACTCACTATAGGGAATTGTAATTGGTTTGTACAAAGTGATAAGTGTTCGTAGAAGATTTTAATGTGTGGGCTCTGTCCAATGGATTTAGCCAAGTTAAATCTTGGAATTCTCGGGTGCCAAGGTACCAAGAAACCTTCATT |
| OC43-Probe I.354 | AY391777.1 | OC43-Probe I | TGTGAGAGATTCCCTTCGTAATACGACTCACTATAGGGTGTTCTGTAGAAGATTTTAATGTGTGGGCTCTGTCCAATGGATTTAGCCAAGTTAAATCTGACCTTGTTATAGATGCTTTAGCTTCTATGTGGAATTCTCGGGTGCCAAGGTACCAAGAAACCTTCATT |
| OC43-Probe I.355 | AY391777.1 | OC43-Probe I | TGTGAGAGATTCCCTTCGTAATACGACTCACTATAGGGCTGTCCAATGGATTTAGCCAAGTTAAATCTGACCTTGTTATAGATGCTTTAGCTTCTATGCTGGTGTGTCTTTGGAAACACTGTTGGCTGGAATTCTCGGGTGCCAAGGTACCAAGAAACCTTCATT |
| OC43-Probe I.356 | AY391777.1 | OC43-Probe I | TGTGAGAGATTCCCTTCGTAATACGACTCACTATAGGGGACCTTGTTATAGATGCTTTAGCTTCTATGACTGGTGTGTCTTTGGAAACACTGTTGGCTGCTATTAAGCGTCTTAAGAATGGTTTCCAATGGAATTCTCGGGTGCCAAGGTACCAAGAAACCTTCATT |
| OC43-Probe I.357 | AY391777.1 | OC43-Probe I | TGTGAGAGATTCCCTTCGTAATACGACTCACTATAGGGACTGGTGTGTCTTTGGAAACACTGTTGGCTGCTATTAAGCGTCTTAAGAATGGTTTCCAAGGACGTCAGATTATGGGTAGTTGCTCTTTTTGGAATTCTCGGGTGCCAAGGTACCAAGAAACCTTCATT |
| OC43-Probe I.358 | AY391777.1 | OC43-Probe I | TGTGAGAGATTCCCTTCGTAATACGACTCACTATAGGGGCTATTAAGCGTCTTAAGAATGGTTTCCAAGGACGTCAGATTATGGGTAGTTGCTCTTTTTGAGGATGAATTGACACCTAGCGATGTTTATTGGAATTCTCGGGTGCCAAGGTACCAAGAAACCTTCATT |
| OC43-Probe I.359 | AY391777.1 | OC43-Probe I | TGTGAGAGATTCCCTTCGTAATACGACTCACTATAGGGGGGACGTCAGATTATGGGTAGTTGCTCTTTTTGAGGATGAATTGACACCTAGCGATGTTTATCAACAACTCGCTGGTATCAAGTTACAATCATGGAATTCTCGGGTGCCAAGGTACCAAGAAACCTTCATT |
| OC43-Probe I.360 | AY391777.1 | OC43-Probe I | TGTGAGAGATTCCCTTCGTAATACGACTCACTATAGGGGAGGATGAATTGACACCTAGCGATGTTTATCAACAACTCGCTGGTATCAAGTTACAATCAAAACGCACTAGATTGTTTAAAGGCACTGTTTGGAATTCTCGGGTGCCAAGGTACCAAGAAACCTTCATT |
| OC43-Probe I.361 | AY391777.1 | OC43-Probe I | TGTGAGAGATTCCCTTCGTAATACGACTCACTATAGGGCAACAACTCGCTGGTATCAAGTTACAATCAAAACGCACTAGATTGTTTAAAGGCACTGTTTGTTGGATTATGGCTTCTACATTTTTGTTTTGGAATTCTCGGGTGCCAAGGTACCAAGAAACCTTCATT |
| OC43-Probe I.362 | AY391777.1 | OC43-Probe I | TGTGAGAGATTCCCTTCGTAATACGACTCACTATAGGGAAACGCACTAGATTGTTTAAAGGCACTGTTTGTTGGATTATGGCTTCTACATTTTTGTTTAGTTGCATAATTACAGCATTTGTGAAATGGTGGAATTCTCGGGTGCCAAGGTACCAAGAAACCTTCATT |
| OC43-Probe I.363 | AY391777.1 | OC43-Probe I | TGTGAGAGATTCCCTTCGTAATACGACTCACTATAGGGTGTTGGATTATGGCTTCTACATTTTTGTTTAGTTGCATAATTACAGCATTTGTGAAATGGACTATGTTTATGTATGTAACTACTAATATGTGGAATTCTCGGGTGCCAAGGTACCAAGAAACCTTCATT |
| OC43-Probe I.364 | AY391777.1 | OC43-Probe I | TGTGAGAGATTCCCTTCGTAATACGACTCACTATAGGGGAGTTGCATAATTACAGCATTTGTGAAATGGACTATGTTTATGTATGTAACTACTAATATGTTTAGTATTACGTTTTGTGCACTTTGTGTTTGGAATTCTCGGGTGCCAAGGTACCAAGAAACCTTCATT |
| OC43-Probe I.365 | AY391777.1 | OC43-Probe I | TGTGAGAGATTCCCTTCGTAATACGACTCACTATAGGGACTATGTTTATGTATGTAACTACTAATATGTTTAGTATTACGTTTTGTGCACTTTGTGTTATAAGTTTGGCCATGTTGTTGGTTAAGCATTGGAATTCTCGGGTGCCAAGGTACCAAGAAACCTTCATT |
| OC43-Probe I.366 | AY391777.1 | OC43-Probe I | TGTGAGAGATTCCCTTCGTAATACGACTCACTATAGGGTTTAGTATTACGTTTTGTGCACTTTGTGTTATAAGTTTGGCCATGTTGTTGGTTAAGCATAAGCATCTTTATTTGACTATGTATATAACTTGGAATTCTCGGGTGCCAAGGTACCAAGAAACCTTCATT |
| OC43-Probe I.367 | AY391777.1 | OC43-Probe I | TGTGAGAGATTCCCTTCGTAATACGACTCACTATAGGGATAAGTTTGGCCATGTTGTTGGTTAAGCATAAGCATCTTTATTTGACTATGTATATAACTCCTGTGCTTTTTACACTGTTGTATAACAACTGGAATTCTCGGGTGCCAAGGTACCAAGAAACCTTCATT |

| ASO ID | Accession | Group ID | Custom Array sequence |
|---|---|---|---|
| OC43-Probe I.368 | AY391777.1 | OC43-Probe I | TGTGAGAGATTCCCTTCGTAATACGACTCACTATAGGGAAGCATCTTTATTTGACTATGTATATAACTCCTGTGCTTTTTACACTGTTGTATAACAACTATTTGGTTGTGTACAAGCATACATTTAGATGGAATTCTCGGGTGCCAAGGTACCAAGAAACCTTCATT |
| OC43-Probe I.369 | AY391777.1 | OC43-Probe I | TGTGAGAGATTCCCTTCGTAATACGACTCACTATAGGGCCTGTGCTTTTTACACTGTTGTATAACAACTATTTGGTTGTGTACAAGCATACATTTAGAGGCTATGTCTATGCATGGCTATCATATTATTGGAATTCTCGGGTGCCAAGGTACCAAGAAACCTTCATT |
| OC43-Probe I.370 | AY391777.1 | OC43-Probe I | TGTGAGAGATTCCCTTCGTAATACGACTCACTATAGGGTATTTGGTTGTGTACAAGCATACATTTAGAGGCTATGTCTATGCATGGCTATCATATTATGTTCCATCAGTTGAGTACACTTATACTGATTGGAATTCTCGGGTGCCAAGGTACCAAGAAACCTTCATT |
| OC43-Probe I.371 | AY391777.1 | OC43-Probe I | TGTGAGAGATTCCCTTCGTAATACGACTCACTATAGGGGGGCTATGTCTATGCATGGCTATCATATTATGTTCCATCAGTTGAGTACACTTATACTGATGAAGTTATTTATGGCATGTTATTGCTTGTATGGAATTCTCGGGTGCCAAGGTACCAAGAAACCTTCATT |
| OC43-Probe I.372 | AY391777.1 | OC43-Probe I | TGTGAGAGATTCCCTTCGTAATACGACTCACTATAGGGGTTCCATCAGTTGAGTACACTTATACTGATGAAGTTATTTATGGCATGTTATTGCTTGTATGGAATGGTCTTTGTTACATTACGTAGCATTTGGAATTCTCGGGTGCCAAGGTACCAAGAAACCTTCATT |
| OC43-Probe I.373 | AY391777.1 | OC43-Probe I | TGTGAGAGATTCCCTTCGTAATACGACTCACTATAGGGGAAGTTATTTATGGCATGTTATTGCTTGTAGGAATGGTCTTTGTTACATTACGTAGCATTAACCATGATTTGTTTTCTTTTATAATGTTTTGGAATTCTCGGGTGCCAAGGTACCAAGAAACCTTCATT |
| OC43-Probe I.374 | AY391777.1 | OC43-Probe I | TGTGAGAGATTCCCTTCGTAATACGACTCACTATAGGGGGAATGGTCTTTGTTACATTACGTAGCATTAACCATGATTTGTTTTCTTTTATAATGTTTGTTGGTCGTTTGATTTCTGTTTTCTCTTTGTGGAATTCTCGGGTGCCAAGGTACCAAGAAACCTTCATT |
| OC43-Probe I.375 | AY391777.1 | OC43-Probe I | TGTGAGAGATTCCCTTCGTAATACGACTCACTATAGGGAACCATGATTTGTTTTCTTTTATAATGTTTGTTGGTCGTTTGATTTCTGTTTTCTCTTTGTGGTACAAGGGTTCTAACTTAGAGGAAGAATGGAATTCTCGGGTGCCAAGGTACCAAGAAACCTTCATT |
| OC43-Probe I.376 | AY391777.1 | OC43-Probe I | TGTGAGAGATTCCCTTCGTAATACGACTCACTATAGGGGTTGGTCGTTTGATTTCTGTTTTCTCTTTGTGGTACAAGGGTTCTAACTTAGAGGAAGAAATTCTTCTTATGTTGGCTTCCCTTTTTGGTTGGAATTCTCGGGTGCCAAGGTACCAAGAAACCTTCATT |
| OC43-Probe I.377 | AY391777.1 | OC43-Probe I | TGTGAGAGATTCCCTTCGTAATACGACTCACTATAGGGTGGTACAAGGGTTCTAACTTAGAGGAAGAAATTCTTCTTATGTTGGCTTCCCTTTTTGGTACTTACACATGGACAACAGTTTTATCTATGTGGAATTCTCGGGTGCCAAGGTACCAAGAAACCTTCATT |
| OC43-Probe I.378 | AY391777.1 | OC43-Probe I | TGTGAGAGATTCCCTTCGTAATACGACTCACTATAGGGATTCTTCTTATGTTGGCTTCCCTTTTTGGTACTTACACATGGACAACAGTTTTATCTATGGCTGTAGCAAAGGTTATTGCTAAGTGGGTTTGGAATTCTCGGGTGCCAAGGTACCAAGAAACCTTCATT |
| OC43-Probe I.379 | AY391777.1 | OC43-Probe I | TGTGAGAGATTCCCTTCGTAATACGACTCACTATAGGGACTTACACATGGACAACAGTTTTATCTATGGCTGTAGCAAAGGTTATTGCTAAGTGGGTTGCTGTGAATGTCTTGTATTTCACAGATATATGGAATTCTCGGGTGCCAAGGTACCAAGAAACCTTCATT |
| OC43-Probe I.380 | AY391777.1 | OC43-Probe I | TGTGAGAGATTCCCTTCGTAATACGACTCACTATAGGGGGCTGTAGCAAAGGTTATTGCTAAGTGGGTTGCTGTGAATGTCTTGTATTTCACAGATATACCTCAAATTAAGATAGTGCTTTTGTGCTATTGGAATTCTCGGGTGCCAAGGTACCAAGAAACCTTCATT |
| OC43-Probe I.381 | AY391777.1 | OC43-Probe I | TGTGAGAGATTCCCTTCGTAATACGACTCACTATAGGGGGCTGTGAATGTCTTGTATTTCACAGATATACCTCAAATTAAGATAGTGCTTTTGTGCTATTGTTTATTGGTTATATTATTAGCTGTTATTGGAATTCTCGGGTGCCAAGGTACCAAGAAACCTTCATT |
| OC43-Probe I.382 | AY391777.1 | OC43-Probe I | TGTGAGAGATTCCCTTCGTAATACGACTCACTATAGGGCCTCAAATTAAGATAGTGCTTTTGTGCTATTGTTTATTGGTTATATTATTAGCTGTTATTGGGGCTTGTTTTCCTTGATGAACAGTTGTGGAATTCTCGGGTGCCAAGGTACCAAGAAACCTTCATT |
| OC43-Probe I.383 | AY391777.1 | OC43-Probe I | TGTGAGAGATTCCCTTCGTAATACGACTCACTATAGGGTTGTTTATTGGTTATATTATTAGCTGTTATTGGGGCTTGTTTTCCTTGATGAACAGTTGTTTAGAATGCCTTTGGGTGTTTATAATTATTGGAATTCTCGGGTGCCAAGGTACCAAGAAACCTTCATT |
| OC43-Probe I.384 | AY391777.1 | OC43-Probe I | TGTGAGAGATTCCCTTCGTAATACGACTCACTATAGGGTGGGGCTTGTTTTCCTTGATGAACAGTTGTTTAGAATGCCTTTGGGTGTTTATAATTATAAAATTTCAGTACAGGAATTAAGATATATGTGGAATTCTCGGGTGCCAAGGTACCAAGAAACCTTCATT |
| OC43-Probe I.385 | AY391777.1 | OC43-Probe I | TGTGAGAGATTCCCTTCGTAATACGACTCACTATAGGGTTTAGAATGCCTTTGGGTGTTTATAATTATAAAATTTCAGTACAGGAATTAAGATATATGAATGCTAATGGATTGCGCCCTCCTAAGAATTGGAATTCTCGGGTGCCAAGGTACCAAGAAACCTTCATT |
| OC43-Probe I.386 | AY391777.1 | OC43-Probe I | TGTGAGAGATTCCCTTCGTAATACGACTCACTATAGGGAAAATTTCAGTACAGGAATTAAGATATATGAATGCTAATGGATTGCGCCCTCCTAAGAATAGTTTTGAAGCCCTTATGCTTAATTTTAAGTGGAATTCTCGGGTGCCAAGGTACCAAGAAACCTTCATT |
| OC43-Probe I.387 | AY391777.1 | OC43-Probe I | TGTGAGAGATTCCCTTCGTAATACGACTCACTATAGGGAATGCTAATGGATTGCGCCCTCCTAAGAATAGTTTTGAAGCCCTTATGCTTAATTTTAAGCTGTTGGGTATTGGAGGTGTTCCAATCATTGGAATTCTCGGGTGCCAAGGTACCAAGAAACCTTCATT |
| OC43-Probe I.388 | AY391777.1 | OC43-Probe I | TGTGAGAGATTCCCTTCGTAATACGACTCACTATAGGGAGTTTTGAAGCCCTTATGCTTAATTTTAAGCTGTTGGGTATTGGAGGTGTTCCAATCATTGAAGTATCTCAATTTCAATCAAAATTGACTTGGAATTCTCGGGTGCCAAGGTACCAAGAAACCTTCATT |
| OC43-Probe I.389 | AY391777.1 | OC43-Probe I | TGTGAGAGATTCCCTTCGTAATACGACTCACTATAGGGCTGTTGGGTATTGGAGGTGTTCCAATCATTGAAGTATCTCAATTTCAATCAAAATTGACTGATGTCAAATGTGCTAATGTCGTCTTGCTTTGGAATTCTCGGGTGCCAAGGTACCAAGAAACCTTCATT |
| OC43-Probe I.390 | AY391777.1 | OC43-Probe I | TGTGAGAGATTCCCTTCGTAATACGACTCACTATAGGGGAAGTATCTCAATTTCAATCAAAATTGACTGATGTCAAATGTGCTAATGTCGTCTTGCTTAATTGCTTGCAACATTTGCATGTTGCTTCTTGGAATTCTCGGGTGCCAAGGTACCAAGAAACCTTCATT |
| OC43-Probe I.391 | AY391777.1 | OC43-Probe I | TGTGAGAGATTCCCTTCGTAATACGACTCACTATAGGGGATGTCAAATGTGCTAATGTCGTCTTGCTTAATTGCTTGCAACATTTGCATGTTGCTTCTAATTCTAAGTTGTGGCATTATTGTAGCACTTGGAATTCTCGGGTGCCAAGGTACCAAGAAACCTTCATT |
| OC43-Probe I.392 | AY391777.1 | OC43-Probe I | TGTGAGAGATTCCCTTCGTAATACGACTCACTATAGGGAATTGCTTGCAACATTTGCATGTTGCTTCTAATTCTAAGTTGTGGCATTATTGTAGCACTTTGCACAATGAAATACTTGCCACTTCGGATTGGAATTCTCGGGTGCCAAGGTACCAAGAAACCTTCATT |

| ASO ID | Accession | Group ID | Custom Array sequence |
|---|---|---|---|
| OC43-Probe I.393 | AY391777.1 | OC43-Probe I | TGTGAGAGATTCCCTTCGTAATACGACTCACTATAGGGAATTCTAAGTTGTGGCATTATTGTAGCACTTTGCACAATGAAATACTTGCCACTTCGGATCTGAGTGTTGCTTTTGAAAAGCTTGCTCAGTGGAATTCTCGGGTGCCAAGGTACCAAGAAACCTTCATT |
| OC43-Probe I.394 | AY391777.1 | OC43-Probe I | TGTGAGAGATTCCCTTCGTAATACGACTCACTATAGGGTTGCACAATGAAATACTTGCCACTTCGGATCTGAGTGTTGCTTTTGAAAAGCTTGCTCAGTTATTAATTGTTTTGTTTGCTAATCCAGCTTGGAATTCTCGGGTGCCAAGGTACCAAGAAACCTTCATT |
| OC43-Probe I.395 | AY391777.1 | OC43-Probe I | TGTGAGAGATTCCCTTCGTAATACGACTCACTATAGGGCTGAGTGTTGCTTTTGAAAAGCTTGCTCAGTTATTAATTGTTTTGTTTGCTAATCCAGCTGCTGTGGATAGCAAGTGCCTGACTAGTATTTGGAATTCTCGGGTGCCAAGGTACCAAGAAACCTTCATT |
| OC43-Probe I.396 | AY391777.1 | OC43-Probe I | TGTGAGAGATTCCCTTCGTAATACGACTCACTATAGGGTTATTAATTGTTTTGTTTGCTAATCCAGCTGCTGTGGATAGCAAGTGCCTGACTAGTATTGAAGAAGTTTGCGATGATTACGCAAAGGACTGGAATTCTCGGGTGCCAAGGTACCAAGAAACCTTCATT |
| OC43-Probe I.397 | AY391777.1 | OC43-Probe I | TGTGAGAGATTCCCTTCGTAATACGACTCACTATAGGGGCTGTGGATAGCAAGTGCCTGACTAGTATTGAAGAAGTTTGCGATGATTACGCAAAGGACAATACTGTTTTGCAGGCTTTACAGAGTGAATGGAATTCTCGGGTGCCAAGGTACCAAGAAACCTTCATT |
| OC43-Probe I.398 | AY391777.1 | OC43-Probe I | TGTGAGAGATTCCCTTCGTAATACGACTCACTATAGGGGAAGAAGTTTGCGATGATTACGCAAAGGACAATACTGTTTTGCAGGCTTTACAGAGTGAATTTGTTAATATGGCTAGCTTCGTTGAATATTGGAATTCTCGGGTGCCAAGGTACCAAGAAACCTTCATT |
| OC43-Probe I.399 | AY391777.1 | OC43-Probe I | TGTGAGAGATTCCCTTCGTAATACGACTCACTATAGGGAATACTGTTTTGCAGGCTTTACAGAGTGAATTTGTTAATATGGCTAGCTTCGTTGAATATGAAGTTGCTAAGAAAAATCTTGATGAGGCGTGGAATTCTCGGGTGCCAAGGTACCAAGAAACCTTCATT |
| OC43-Probe I.400 | AY391777.1 | OC43-Probe I | TGTGAGAGATTCCCTTCGTAATACGACTCACTATAGGGTTTGTTAATATGGCTAGCTTCGTTGAATATGAAGTTGCTAAGAAAAATCTTGATGAGGCGCGTTTTAGTGGTTCTGCTAATCAACAGCAGTGGAATTCTCGGGTGCCAAGGTACCAAGAAACCTTCATT |
| OC43-Probe I.401 | AY391777.1 | OC43-Probe I | TGTGAGAGATTCCCTTCGTAATACGACTCACTATAGGGGAAGTTGCTAAGAAAAATCTTGATGAGGCGCGTTTTAGTGGTTCTGCTAATCAACAGCAGTTAAAACAGCTAGAGAAAGCCTGTAATATTTGGAATTCTCGGGTGCCAAGGTACCAAGAAACCTTCATT |
| OC43-Probe I.402 | AY391777.1 | OC43-Probe I | TGTGAGAGATTCCCTTCGTAATACGACTCACTATAGGGCGTTTTAGTGGTTCTGCTAATCAACAGCAGTTAAAACAGCTAGAGAAAGCCTGTAATATTGCTAAATCTGCTTATGAACGCGACCGTGCTTGGAATTCTCGGGTGCCAAGGTACCAAGAAACCTTCATT |
| OC43-Probe I.403 | AY391777.1 | OC43-Probe I | TGTGAGAGATTCCCTTCGTAATACGACTCACTATAGGGTTAAAACAGCTAGAGAAAGCCTGTAATATTGCTAAATCTGCTTATGAACGCGACCGTGCTGTAGCAAAAAAGTTGGAGCGTATGGCTGATTGGAATTCTCGGGTGCCAAGGTACCAAGAAACCTTCATT |
| OC43-Probe I.404 | AY391777.1 | OC43-Probe I | TGTGAGAGATTCCCTTCGTAATACGACTCACTATAGGGGCTAAATCTGCTTATGAACGCGACCGTGCTGTAGCAAAAAAGTTGGAGCGTATGGCTGATTGGCTCTCACTAATATGTATAAAGAAGCTTGGAATTCTCGGGTGCCAAGGTACCAAGAAACCTTCATT |
| OC43-Probe I.405 | AY391777.1 | OC43-Probe I | TGTGAGAGATTCCCTTCGTAATACGACTCACTATAGGGGTAGCAAAAAAGTTGGAGCGTATGGCTGATTGGCTCTCACTAATATGTATAAAGAAGCTAGAATTAATGATAAGAAGAGTAAGGTTGTTTGGAATTCTCGGGTGCCAAGGTACCAAGAAACCTTCATT |
| OC43-Probe I.406 | AY391777.1 | OC43-Probe I | TGTGAGAGATTCCCTTCGTAATACGACTCACTATAGGGTTGGCTCTCACTAATATGTATAAAGAAGCTAGAATTAATGATAAGAAGAGTAAGGTTGTTTCTGCCTTGCAAACTATGCTTTTTAGTATGTGGAATTCTCGGGTGCCAAGGTACCAAGAAACCTTCATT |
| OC43-Probe I.407 | AY391777.1 | OC43-Probe I | TGTGAGAGATTCCCTTCGTAATACGACTCACTATAGGGAGGAGAATTAATGATAAGAAGAGTAAGGTTGTTTCTGCCTTGCAAACTATGCTTTTTAGTATGGTGCGTAAGTTAGATAATCAAGCTCTGAATTGGAATTCTCGGGTGCCAAGGTACCAAGAAACCTTCATT |
| OC43-Probe I.408 | AY391777.1 | OC43-Probe I | TGTGAGAGATTCCCTTCGTAATACGACTCACTATAGGGTCTGCCTTGCAAACTATGCTTTTTAGTATGGTGCGTAAGTTAGATAATCAAGCTCTGAATTCAATATTAGATAACGCTGTGAAGGGTTGTTGGAATTCTCGGGTGCCAAGGTACCAAGAAACCTTCATT |
| OC43-Probe I.409 | AY391777.1 | OC43-Probe I | TGTGAGAGATTCCCTTCGTAATACGACTCACTATAGGGGTGCGTAAGTTAGATAATCAAGCTCTGAATTCAATATTAGATAACGCTGTGAAGGGTGTGTACCATTGAATGCAATACCTTCATTGGCATGGAATTCTCGGGTGCCAAGGTACCAAGAAACCTTCATT |
| OC43-Probe I.410 | AY391777.1 | OC43-Probe I | TGTGAGAGATTCCCTTCGTAATACGACTCACTATAGGGTCAATATTAGATAACGCTGTGAAGGGTGTGTACCATTGAATGCAATACCTTCATTGGCAGCAAATACTCTGAATATAATTGTACCAGATTGGAATTCTCGGGTGCCAAGGTACCAAGAAACCTTCATT |
| OC43-Probe I.411 | AY391777.1 | OC43-Probe I | TGTGAGAGATTCCCTTCGTAATACGACTCACTATAGGGGTACCATTGAATGCAATACCTTCATTGGCAGCAAATACTCTGAATATAATTGTACCAGATAAAAGTGTTTATGACCAGGTAGTTGATAATTGGAATTCTCGGGTGCCAAGGTACCAAGAAACCTTCATT |
| OC43-Probe I.412 | AY391777.1 | OC43-Probe I | TGTGAGAGATTCCCTTCGTAATACGACTCACTATAGGGGCAAATACTCTGAATATAATTGTACCAGATAAAAGTGTTTATGACCAGGTAGTTGATAATGTCTATGTTACCTATGCGGGTAATGTATGGTGGAATTCTCGGGTGCCAAGGTACCAAGAAACCTTCATT |
| OC43-Probe I.413 | AY391777.1 | OC43-Probe I | TGTGAGAGATTCCCTTCGTAATACGACTCACTATAGGGAAAGTGTTTATGACCAGGTAGTTGATAATGTCTATGTTACCTATGCGGGTAATGTATGGCAGATTCAAACTATCCAGGATTCAGATGGTTGGAATTCTCGGGTGCCAAGGTACCAAGAAACCTTCATT |
| OC43-Probe I.414 | AY391777.1 | OC43-Probe I | TGTGAGAGATTCCCTTCGTAATACGACTCACTATAGGGGTCTATGTTACCTATGCGGGTAATGTATGGCAGATTCAAACTATCCAGGATTCAGATGGTACAAATAAGCAGTTGAATGAGATATCTGATTGGAATTCTCGGGTGCCAAGGTACCAAGAAACCTTCATT |
| OC43-Probe I.415 | AY391777.1 | OC43-Probe I | TGTGAGAGATTCCCTTCGTAATACGACTCACTATAGGGCAGATTCAAACTATCCAGGATTCAGATGGTACAAATAAGCAGTTGAATGAGATATCTGATGATTGTAACTGGCCACTAGTTATTATTGCATGGAATTCTCGGGTGCCAAGGTACCAAGAAACCTTCATT |
| OC43-Probe I.416 | AY391777.1 | OC43-Probe I | TGTGAGAGATTCCCTTCGTAATACGACTCACTATAGGGGACAAATAAGCAGTTGAATGAGATATCTGATGATTGTAACTGGCCACTAGTTATTATTGCAAATCGGTATAATGAGGTATCTGCTACTGTTTGGAATTCTCGGGTGCCAAGGTACCAAGAAACCTTCATT |
| OC43-Probe I.417 | AY391777.1 | OC43-Probe I | TGTGAGAGATTCCCTTCGTAATACGACTCACTATAGGGGATTGTAACTGGCCACTAGTTATTATTGCAAATCGGTATAATGAGGTATCTGCTACTGTTTTGCAAAATAATGAATTAATGCCTGCTAAGTGGAATTCTCGGGTGCCAAGGTACCAAGAAACCTTCATT |

EP 4 239 083 A2

| ASO ID | Accession | Group ID | Custom Array sequence |
|---|---|---|---|
| OC43-Probe I.418 | AY391777.1 | OC43-Probe I | TGTGAGAGATTCCCTTCGTAATACGACTCACTATAGGGAATCGGTATAATGAGGTATCTGCTACTGTTTTGCAAAATAATGAATTAATGCCTGCTAAGTTGAAAATTCAGGTTGTTAATAGTGGTCCATGGAATTCTCGGGTGCCAAGGTACCAAGAAACCTTCATT |
| OC43-Probe I.419 | AY391777.1 | OC43-Probe I | TGTGAGAGATTCCCTTCGTAATACGACTCACTATAGGGGTTGCAAAATAATGAATTAATGCCTGCTAAGTTGAAAATTCAGGTTGTTAATAGTGGTCCAGATCAGACTTGTAATACACCTACTCAATGTTGGAATTCTCGGGTGCCAAGGTACCAAGAAACCTTCATT |
| OC43-Probe I.420 | AY391777.1 | OC43-Probe I | TGTGAGAGATTCCCTTCGTAATACGACTCACTATAGGGGTTGAAAATTCAGGTTGTTAATAGTGGTCCAGATCAGACTTGTAATACACCTACTCAATGTTACTATAATAATAGTAACAATGGGAAGATTTGGAATTCTCGGGTGCCAAGGTACCAAGAAACCTTCATT |
| OC43-Probe I.421 | AY391777.1 | OC43-Probe I | TGTGAGAGATTCCCTTCGTAATACGACTCACTATAGGGGATCAGACTTGTAATACACCTACTCAATGTTACTATAATAATAGTAACAATGGGAAGATTGTTTATGCTATACTTAGTGATGTTGATGGTTGGAATTCTCGGGTGCCAAGGTACCAAGAAACCTTCATT |
| OC43-Probe I.422 | AY391777.1 | OC43-Probe I | TGTGAGAGATTCCCTTCGTAATACGACTCACTATAGGGTACTATAATAATAGTAACAATGGGAAGATTGTTTATGCTATACTTAGTGATGTTGATGGTCTTAAGTATACAAAAATTCTTAAAGATGATTGGAATTCTCGGGTGCCAAGGTACCAAGAAACCTTCATT |
| OC43-Probe I.423 | AY391777.1 | OC43-Probe I | TGTGAGAGATTCCCTTCGTAATACGACTCACTATAGGGGTTTATGCTATACTTAGTGATGTTGATGGTCTTAAGTATACAAAAATTCTTAAAGATGATGGCAATTTTGTTGTTTTGGAGTTAGATCCTTGGAATTCTCGGGTGCCAAGGTACCAAGAAACCTTCATT |
| OC43-Probe I.424 | AY391777.1 | OC43-Probe I | TGTGAGAGATTCCCTTCGTAATACGACTCACTATAGGGCTTAAGTATACAAAAATTCTTAAAGATGATGGCAATTTTGTTGTTTTGGAGTTAGATCCTCCTTGTAAATTTACTGTTCAAGATGCTAAATGGAATTCTCGGGTGCCAAGGTACCAAGAAACCTTCATT |
| OC43-Probe I.425 | AY391777.1 | OC43-Probe I | TGTGAGAGATTCCCTTCGTAATACGACTCACTATAGGGGGGCAATTTTGTTGTTTTGGAGTTAGATCCTCCTTGTAAATTTACTGTTCAAGATGCTAAAGGTCTTAAAATTAAGTACCTTTATTTTGTATGGAATTCTCGGGTGCCAAGGTACCAAGAAACCTTCATT |
| OC43-Probe I.426 | AY391777.1 | OC43-Probe I | TGTGAGAGATTCCCTTCGTAATACGACTCACTATAGGGCCTTGTAAATTTACTGTTCAAGATGCTAAAGGTCTTAAAATTAAGTACCTTTATTTTGTAAAAGGTTGTAACACACTAGCAAGAGGCTGGTGGAATTCTCGGGTGCCAAGGTACCAAGAAACCTTCATT |
| OC43-Probe I.427 | AY391777.1 | OC43-Probe I | TGTGAGAGATTCCCTTCGTAATACGACTCACTATAGGGGGTCTTAAAATTAAGTACCTTTATTTTGTAAAAGGTTGTAACACACTAGCAAGAGGCTGGGTGTTGGTACAATTTCTTCTACAGTTAGATGGAATTCTCGGGTGCCAAGGTACCAAGAAACCTTCATT |
| OC43-Probe I.428 | AY391777.1 | OC43-Probe I | TGTGAGAGATTCCCTTCGTAATACGACTCACTATAGGGAAAGGTTGTAACACACTAGCAAGAGGCTGGGTGTTGGTACAATTTCTTCTACAGTTAGATTGCAAGCTGGAACTGCTACTGAATATGCTTGGAATTCTCGGGTGCCAAGGTACCAAGAAACCTTCATT |
| OC43-Probe I.429 | AY391777.1 | OC43-Probe I | TGTGAGAGATTCCCTTCGTAATACGACTCACTATAGGGGTTGTTGGTACAATTTCTTCTACAGTTAGATTGCAAGCTGGAACTGCTACTGAATATGCTTCCAACTCATCTATATTGTCTTTATGTCGTGGAATTCTCGGGTGCCAAGGTACCAAGAAACCTTCATT |
| OC43-Probe I.430 | AY391777.1 | OC43-Probe I | TGTGAGAGATTCCCTTCGTAATACGACTCACTATAGGGTTGCAAGCTGGAACTGCTACTGAATATGCTTCCAACTCATCTATATTGTCTTTATGTGCGGTTTCTGTAGATCCTAAGAAAACGTATTTATGGAATTCTCGGGTGCCAAGGTACCAAGAAACCTTCATT |
| OC43-Probe I.431 | AY391777.1 | OC43-Probe I | TGTGAGAGATTCCCTTCGTAATACGACTCACTATAGGGTCCAACTCATCTATATTGTCTTTATGTGCGGTTTCTGTAGATCCTAAGAAAACGTATTTAGATTTTATACAACAAGGAGGAACACCTATTTGGAATTCTCGGGTGCCAAGGTACCAAGAAACCTTCATT |
| OC43-Probe I.432 | AY391777.1 | OC43-Probe I | TGTGAGAGATTCCCTTCGTAATACGACTCACTATAGGGTTTTCTGTAGATCCTAAGAAAACGTATTTAGATTTTATACAACAAGGAGGAACACCTATTGCCAATTGTGTTAAAATGTTGTGTGACCATTGGAATTCTCGGGTGCCAAGGTACCAAGAAACCTTCATT |
| OC43-Probe I.433 | AY391777.1 | OC43-Probe I | TGTGAGAGATTCCCTTCGTAATACGACTCACTATAGGGGATTTTATACAACAAGGAGGAACACCTATTGCCAATTGTGTTAAAATGTTGTGTGACCATGCTGGTACCGGTATGGCCATTACTGTTAAATGGAATTCTCGGGTGCCAAGGTACCAAGAAACCTTCATT |
| OC43-Probe I.434 | AY391777.1 | OC43-Probe I | TGTGAGAGATTCCCTTCGTAATACGACTCACTATAGGGGGCCAATTGTGTTAAAATGTTGTGTGACCATGCTGGTACCGGTATGGCCATTACTGTTAAACCCGATGCTACCACTAGTCAGGATTCATATTGGAATTCTCGGGTGCCAAGGTACCAAGAAACCTTCATT |
| OC43-Probe I.435 | AY391777.1 | OC43-Probe I | TGTGAGAGATTCCCTTCGTAATACGACTCACTATAGGGGGCTGGTACCGGTATGGCCATTACTGTTAAACCCGATGCTACCACTAGTCAGGATTCATATGGTGGTGCGTCTGTTTGTATATATTGCCGCTGGAATTCTCGGGTGCCAAGGTACCAAGAAACCTTCATT |
| OC43-Probe I.436 | AY391777.1 | OC43-Probe I | TGTGAGAGATTCCCTTCGTAATACGACTCACTATAGGGCCCGATGCTACCACTAGTCAGGATTCATATGGTGGTGCGTCTGTTTGTATATATTGCCGCGCACGAGTTGAACACCCAGATGTTGATGGGTGGAATTCTCGGGTGCCAAGGTACCAAGAAACCTTCATT |
| OC43-Probe I.437 | AY391777.1 | OC43-Probe I | TGTGAGAGATTCCCTTCGTAATACGACTCACTATAGGGGGTGGTGCGTCTGTTTGTATATATTGCCGCGCACGAGTTGAACACCCAGATGTTGATGGGTTGTGCAAATTACGCGGCAAGTTTGTACAATGGAATTCTCGGGTGCCAAGGTACCAAGAAACCTTCATT |
| OC43-Probe I.438 | AY391777.1 | OC43-Probe I | TGTGAGAGATTCCCTTCGTAATACGACTCACTATAGGGGCACGAGTTGAACACCCAGATGTTGATGGGTTGTGCAAATTACGCGGCAAGTTTGTACAAGTGCCTGTAGGTATAAAAGATCCTGTGTCTTGGAATTCTCGGGTGCCAAGGTACCAAGAAACCTTCATT |
| OC43-Probe I.439 | AY391777.1 | OC43-Probe I | TGTGAGAGATTCCCTTCGTAATACGACTCACTATAGGGTTGTGCAAATTACGCGGCAAGTTTGTACAAGTGCCTGTAGGTATAAAAGATCCTGTGTCTTATGTTTTGACACATGATGTTTGTCGAGTTTGGAATTCTCGGGTGCCAAGGTACCAAGAAACCTTCATT |
| OC43-Probe I.440 | AY391777.1 | OC43-Probe I | TGTGAGAGATTCCCTTCGTAATACGACTCACTATAGGGGTGCCTGTAGGTATAAAAGATCCTGTGTCTTATGTTTTGACACATGATGTTTGTCGAGTTTGTGGATTTTGGCGGGATGGAAGTTGTTCATGGAATTCTCGGGTGCCAAGGTACCAAGAAACCTTCATT |
| OC43-Probe I.441 | AY391777.1 | OC43-Probe I | TGTGAGAGATTCCCTTCGTAATACGACTCACTATAGGGTATGTTTTGACACATGATGTTTGTCGAGTTTGTGGATTTTGGCGGGATGGAAGTTGTTCATGTGTTAGCACTGACACTACTGTTCAATCATGGAATTCTCGGGTGCCAAGGTACCAAGAAACCTTCATT |
| OC43-Probe I.442 | AY391777.1 | OC43-Probe I | TGTGAGAGATTCCCTTCGTAATACGACTCACTATAGGGTGTGGATTTTGGCGGGATGGAAGTTGTTCATGTGTTAGCACTGACACTACTGTTCAATCAAAAGATACTAATTTTTTTAAACGGGTTCGGGTGGAATTCTCGGGTGCCAAGGTACCAAGAAACCTTCATT |

| ASO ID | Accession | Group ID | Custom Array sequence |
|---|---|---|---|
| OC43-Probe I.443 | AY391777.1 | OC43-Probe I | TGTGAGAGATTCCCTTCGTAATACGACTCACTATAGGGTGTGTTAGCACTGACACTACTGTTCAATCAAAAGATACTAATTTTTTAAACGGGTTCGGGGTACGAGTGTAGATGCCCGTCTCGTACCCTTGGAATTCTCGGGTGCCAAGGTACCAAGAAACCTTCATT |
| OC43-Probe I.444 | AY391777.1 | OC43-Probe I | TGTGAGAGATTCCCTTCGTAATACGACTCACTATAGGGAAAGATACTAATTTTTTAAACGGGTTCGGGGTACGAGTGTAGATGCCCGTCTCGTACCCTGCGCCAGTGGTTTATCTACTGATGTACAATTGGAATTCTCGGGTGCCAAGGTACCAAGAAACCTTCATT |
| OC43-Probe I.445 | AY391777.1 | OC43-Probe I | TGTGAGAGATTCCCTTCGTAATACGACTCACTATAGGGGTACGAGTGTAGATGCCCGTCTCGTACCCTGCGCCAGTGGTTTATCTACTGATGTACAATTAAGGGCATTTGATATTTACAATGCTAGTGTGGAATTCTCGGGTGCCAAGGTACCAAGAAACCTTCATT |
| OC43-Probe I.446 | AY391777.1 | OC43-Probe I | TGTGAGAGATTCCCTTCGTAATACGACTCACTATAGGGGCGCCAGTGGTTTATCTACTGATGTACAATTAAGGGCATTTGATATTTACAATGCTAGTGTTGCTGGCATTGGTTTACATTTAAAAGTTATGGAATTCTCGGGTGCCAAGGTACCAAGAAACCTTCATT |
| OC43-Probe I.447 | AY391777.1 | OC43-Probe I | TGTGAGAGATTCCCTTCGTAATACGACTCACTATAGGGTAAGGGCATTTGATATTTACAATGCTAGTGTTGCTGGCATTGGTTTACATTTAAAAGTTAATTGTTGCCGTTTTCAGCGTGTTGATGAGATGGAATTCTCGGGTGCCAAGGTACCAAGAAACCTTCATT |
| OC43-Probe I.448 | AY391777.1 | OC43-Probe I | TGTGAGAGATTCCCTTCGTAATACGACTCACTATAGGGTTGCTGGCATTGGTTTACATTTAAAAGTTAATTGTTGCCGTTTTCAGCGTGTTGATGAGAACGGTGATAAATTAGATCAGTTCTTTGTTGTGGAATTCTCGGGTGCCAAGGTACCAAGAAACCTTCATT |
| OC43-Probe I.449 | AY391777.1 | OC43-Probe I | TGTGAGAGATTCCCTTCGTAATACGACTCACTATAGGGATTGTTGCCGTTTTCAGCGTGTTGATGAGAACGGTGATAAATTAGATCAGTTCTTTGTTGTTAAGAGGACAGATCTGACTATATATAATATGGAATTCTCGGGTGCCAAGGTACCAAGAAACCTTCATT |
| OC43-Probe I.450 | AY391777.1 | OC43-Probe I | TGTGAGAGATTCCCTTCGTAATACGACTCACTATAGGGACGGTGATAAATTAGATCAGTTCTTTGTTGTTAAGAGGACAGATCTGACTATATATAATAGAGATGAAATGCTATGAGCGTGTAAAAGTGGAATTCTCGGGTGCCAAGGTACCAAGAAACCTTCATT |
| OC43-Probe I.451 | AY391777.1 | OC43-Probe I | TGTGAGAGATTCCCTTCGTAATACGACTCACTATAGGGTTAAGAGGACAGATCTGACTATATATAATAGAGATGAAATGCTATGAGCGTGTAAAAGATTGTAAGTTTGTGGCTGAACACGATTTCTTGGAATTCTCGGGTGCCAAGGTACCAAGAAACCTTCATT |
| OC43-Probe I.452 | AY391777.1 | OC43-Probe I | TGTGAGAGATTCCCTTCGTAATACGACTCACTATAGGGGGAGAGATGAAATGCTATGAGCGTGTAAAAGATTGTAAGTTTGTGGCTGAACACGATTTCTTTACATTTGATGTAGAAGGTAGTCGTGTGCTGGAATTCTCGGGTGCCAAGGTACCAAGAAACCTTCATT |
| OC43-Probe I.453 | AY391777.1 | OC43-Probe I | TGTGAGAGATTCCCTTCGTAATACGACTCACTATAGGGATTGTAAGTTTGTGGCTGAACACGATTTCTTTACATTTGATGTAGAAGGTAGTCGTGTGCCACACATTGTACGCAAGGATTTAACAAAGTTGGAATTCTCGGGTGCCAAGGTACCAAGAAACCTTCATT |
| OC43-Probe I.454 | AY391777.1 | OC43-Probe I | TGTGAGAGATTCCCTTCGTAATACGACTCACTATAGGGTTACATTTGATGTAGAAGGTAGTCGTGTGCCACACATTGTACGCAAGGATTTAACAAAGTATACTATGTTGGATCTTTGCTATGCATTGCTGGAATTCTCGGGTGCCAAGGTACCAAGAAACCTTCATT |
| OC43-Probe I.455 | AY391777.1 | OC43-Probe I | TGTGAGAGATTCCCTTCGTAATACGACTCACTATAGGGCACACATTGTACGCAAGGATTTAACAAAGTATACTATGTTGGATCTTTGCTATGCATTGCGACATTTTGATCGCAATGATTGCATGCTGCTGGAATTCTCGGGTGCCAAGGTACCAAGAAACCTTCATT |
| OC43-Probe I.456 | AY391777.1 | OC43-Probe I | TGTGAGAGATTCCCTTCGTAATACGACTCACTATAGGGGATACTATGTTGGATCTTTGCTATGCATTGCGACATTTTGATCGCAATGATTGCATGCTGCTTTGTGACATTCTCTCTATATATGCTGGTTTGGAATTCTCGGGTGCCAAGGTACCAAGAAACCTTCATT |
| OC43-Probe I.457 | AY391777.1 | OC43-Probe I | TGTGAGAGATTCCCTTCGTAATACGACTCACTATAGGGGACATTTTGATCGCAATGATTGCATGCTGCTTTGTGACATTCTCTCTATATATGCTGGTTGTGAACAATCCTACTTTACTAAGAAGGATTTGGAATTCTCGGGTGCCAAGGTACCAAGAAACCTTCATT |
| OC43-Probe I.458 | AY391777.1 | OC43-Probe I | TGTGAGAGATTCCCTTCGTAATACGACTCACTATAGGGTTTGTGACATTCTCTCTATATATGCTGGTTGTGAACAATCCTACTTTACTAAGAAGGATTGGTATGATTTTGTTGAAAATCCTGATATTATGGAATTCTCGGGTGCCAAGGTACCAAGAAACCTTCATT |
| OC43-Probe I.459 | AY391777.1 | OC43-Probe I | TGTGAGAGATTCCCTTCGTAATACGACTCACTATAGGGGTGAACAATCCTACTTTACTAAGAAGGATTGGTATGATTTTGTTGAAAATCCTGATATTATTAATGTGTATAAAAAGCTAGGACCTATTTTGGAATTCTCGGGTGCCAAGGTACCAAGAAACCTTCATT |
| OC43-Probe I.460 | AY391777.1 | OC43-Probe I | TGTGAGAGATTCCCTTCGTAATACGACTCACTATAGGGGGTATGATTTTGTTGAAAATCCTGATATTATTAATGTGTATAAAAAGCTAGGACCTATTTTTAATAGAGCCCTAGTTAGCGCTACTGAGTTGGAATTCTCGGGTGCCAAGGTACCAAGAAACCTTCATT |
| OC43-Probe I.461 | AY391777.1 | OC43-Probe I | TGTGAGAGATTCCCTTCGTAATACGACTCACTATAGGGTTAATGTGTATAAAAAGCTAGGACCTATTTTTAATAGAGCCCTAGTTAGCGCTACTGAGTTTGCGGACAAATTGGTGGAGGTAGGCTTAGTGGAATTCTCGGGTGCCAAGGTACCAAGAAACCTTCATT |
| OC43-Probe I.462 | AY391777.1 | OC43-Probe I | TGTGAGAGATTCCCTTCGTAATACGACTCACTATAGGGTTAATAGAGCCCTAGTTAGCGCTACTGAGTTTGCGGACAAATTGGTGGAGGTAGGCTTAGTAGGCGTTTTAACACTTGATAATCAAGATTTGGAATTCTCGGGTGCCAAGGTACCAAGAAACCTTCATT |
| OC43-Probe I.463 | AY391777.1 | OC43-Probe I | TGTGAGAGATTCCCTTCGTAATACGACTCACTATAGGGTTGCGGACAAATTGGTGGAGGTAGGCTTAGTAGGCGTTTTAACACTTGATAATCAAGATTTAAATGGTAAATGGTATGATTTTGGTGACTTGGAATTCTCGGGTGCCAAGGTACCAAGAAACCTTCATT |
| OC43-Probe I.464 | AY391777.1 | OC43-Probe I | TGTGAGAGATTCCCTTCGTAATACGACTCACTATAGGGTAGGCGTTTTAACACTTGATAATCAAGATTTAAATGGTAAATGGTATGATTTTGGTGACTATGTTATTGCAGCCCCAGGATGTGGTGTTGTGGAATTCTCGGGTGCCAAGGTACCAAGAAACCTTCATT |
| OC43-Probe I.465 | AY391777.1 | OC43-Probe I | TGTGAGAGATTCCCTTCGTAATACGACTCACTATAGGGTAAATGGTAAATGGTATGATTTTGGTGACTATGTTATTGCAGCCCCAGGATGTGGTGTTGCTATAGCAGATTCTTATTATTCTTATATCATGGAATTCTCGGGTGCCAAGGTACCAAGAAACCTTCATT |
| OC43-Probe I.466 | AY391777.1 | OC43-Probe I | TGTGAGAGATTCCCTTCGTAATACGACTCACTATAGGGGATGTTATTGCAGCCCCAGGATGTGGTGTTGCTATAGCAGATTCTTATTATTCTTATATCATGCCTATGCTGACCATGTGTCATGCATTGGTGGAATTCTCGGGTGCCAAGGTACCAAGAAACCTTCATT |
| OC43-Probe I.467 | AY391777.1 | OC43-Probe I | TGTGAGAGATTCCCTTCGTAATACGACTCACTATAGGGCTATAGCAGATTCTTATTATTCTTATATCATGCCTATGCTGACCATGTGTCATGCATTGGTTGCGAATTGTATGTGAATAATGCTTATATGGAATTCTCGGGTGCCAAGGTACCAAGAAACCTTCATT |

| ASO ID | Accession | Group ID | Custom Array sequence |
|---|---|---|---|
| OC43-Probe I.468 | AY391777.1 | OC43-Probe I | TGTGAGAGATTCCCTTCGTAATACGACTCACTATAGGGTGCCTATGCTGACCATGTGTCATGCATTGGATTGCGAATTGTATGTGAATAATGCTTATAGACTATTTGATCTTGTACAGTATGATTTTATGGAATTCTCGGGTGCCAAGGTACCAAGAAACCTTCATT |
| OC43-Probe I.469 | AY391777.1 | OC43-Probe I | TGTGAGAGATTCCCTTCGTAATACGACTCACTATAGGGATTGCGAATTGTATGTGAATAATGCTTATAGACTATTTGATCTTGTACAGTATGATTTTACTGATTACAAGCTTGAATTGTTTAATAAGTTGGAATTCTCGGGTGCCAAGGTACCAAGAAACCTTCATT |
| OC43-Probe I.470 | AY391777.1 | OC43-Probe I | TGTGAGAGATTCCCTTCGTAATACGACTCACTATAGGGGACTATTTGATCTTGTACAGTATGATTTTACTGATTACAAGCTTGAATTGTTTAATAAGTATTTTAAGCACTGGAGTATGCCATATCATCTGGAATTCTCGGGTGCCAAGGTACCAAGAAACCTTCATT |
| OC43-Probe I.471 | AY391777.1 | OC43-Probe I | TGTGAGAGATTCCCTTCGTAATACGACTCACTATAGGGCTGATTACAAGCTTGAATTGTTTAATAAGTATTTTAAGCACTGGAGTATGCCATATCATCCTAACACTGTTGATTGTCAGGATGATCGGTTGGAATTCTCGGGTGCCAAGGTACCAAGAAACCTTCATT |
| OC43-Probe I.472 | AY391777.1 | OC43-Probe I | TGTGAGAGATTCCCTTCGTAATACGACTCACTATAGGGATTTTAAGCACTGGAGTATGCCATATCATCCTAACACTGTTGATTGTCAGGATGATCGGTGTATTATACATTGTGCTAATTTTAACATACTGGAATTCTCGGGTGCCAAGGTACCAAGAAACCTTCATT |
| OC43-Probe I.473 | AY391777.1 | OC43-Probe I | TGTGAGAGATTCCCTTCGTAATACGACTCACTATAGGGCTAACACTGTTGATTGTCAGGATGATCGGTGTATTATACATTGTGCTAATTTTAACATACTTTTTAGTATGGTTTTACCTAATACATGTTTGGAATTCTCGGGTGCCAAGGTACCAAGAAACCTTCATT |
| OC43-Probe I.474 | AY391777.1 | OC43-Probe I | TGTGAGAGATTCCCTTCGTAATACGACTCACTATAGGGGTATTATACATTGTGCTAATTTTAACATACTTTTTAGTATGGTTTTACCTAATACATGTTTGGGCCTCTTGTTAGGCAAATTTTTGTGGTGGAATTCTCGGGTGCCAAGGTACCAAGAAACCTTCATT |
| OC43-Probe I.475 | AY391777.1 | OC43-Probe I | TGTGAGAGATTCCCTTCGTAATACGACTCACTATAGGGTTTTTAGTATGGTTTTACCTAATACATGTTTGGGCCTCTTGTTAGGCAAATTTTTGTGGATGGTGTGCCTTTTGTTGTTTCAATTGGCTTGGAATTCTCGGGTGCCAAGGTACCAAGAAACCTTCATT |
| OC43-Probe I.476 | AY391777.1 | OC43-Probe I | TGTGAGAGATTCCCTTCGTAATACGACTCACTATAGGGTTGGGCCTCTTGTTAGGCAAATTTTTGTGGATGGTGTGCCTTTTGTTGTTTCAATTGGCTACCATTATAAAGAACTTGGTATTGTGATGATGGAATTCTCGGGTGCCAAGGTACCAAGAAACCTTCATT |
| OC43-Probe I.477 | AY391777.1 | OC43-Probe I | TGTGAGAGATTCCCTTCGTAATACGACTCACTATAGGGATGGTGTGCCTTTTGTTGTTTCAATTGGCTACCATTATAAAGAACTTGGTATTGTGATGAATATGGATGTGGATACACATCGTTATCGCTTGGAATTCTCGGGTGCCAAGGTACCAAGAAACCTTCATT |
| OC43-Probe I.478 | AY391777.1 | OC43-Probe I | TGTGAGAGATTCCCTTCGTAATACGACTCACTATAGGGACCATTATAAAGAACTTGGTATTGTGATGAATATGGATGTGGATACACATCGTTATCGCTTGTCTTTAAAAGACTTGCTTTTATATGCTGTGGAATTCTCGGGTGCCAAGGTACCAAGAAACCTTCATT |
| OC43-Probe I.479 | AY391777.1 | OC43-Probe I | TGTGAGAGATTCCCTTCGTAATACGACTCACTATAGGGATATGGATGTGGATACACATCGTTATCGCTTGTCTTTAAAAGACTTGCTTTTATATGCTGCTGATCCAGCTTTGCATGTAGCTTCTGCTATGGAATTCTCGGGTGCCAAGGTACCAAGAAACCTTCATT |
| OC43-Probe I.480 | AY391777.1 | OC43-Probe I | TGTGAGAGATTCCCTTCGTAATACGACTCACTATAGGGTGTCTTTAAAAGACTTGCTTTTATATGCTGCTGATCCAGCTTTGCATGTAGCTTCTGCTAGTGCATTGTATGATTTACGCCACTTGCTGTTTGGAATTCTCGGGTGCCAAGGTACCAAGAAACCTTCATT |
| OC43-Probe I.481 | AY391777.1 | OC43-Probe I | TGTGAGAGATTCCCTTCGTAATACGACTCACTATAGGGCTGATCCAGCTTTGCATGTAGCTTCTGCTAGTGCATTGTATGATTTACGCCACTTGCTGTTTTAGTGTTGCCGCTATAACAAGCGGTGTAATGAATGGAATTCTCGGGTGCCAAGGTACCAAGAAACCTTCATT |
| OC43-Probe I.482 | AY391777.1 | OC43-Probe I | TGTGAGAGATTCCCTTCGTAATACGACTCACTATAGGGGTGCATTGTATGATTTACGCCACTTGCTGTTTTAGTGTTGCCGCTATAACAAGCGGTGTAAAATTTCAAACAGTTAAACCTGGTAATTTTATGGAATTCTCGGGTGCCAAGGTACCAAGAAACCTTCATT |
| OC43-Probe I.483 | AY391777.1 | OC43-Probe I | TGTGAGAGATTCCCTTCGTAATACGACTCACTATAGGGTTAGTGTTGCCGCTATAACAAGCGGTGTAAAATTTCAAACAGTTAAACCTGGTAATTTTAATCAGGATTTTTATGATTTTGTTTTAAGTATGGAATTCTCGGGTGCCAAGGTACCAAGAAACCTTCATT |
| OC43-Probe I.484 | AY391777.1 | OC43-Probe I | TGTGAGAGATTCCCTTCGTAATACGACTCACTATAGGGAATTTCAAACAGTTAAACCTGGTAATTTTAATCAGGATTTTTATGATTTTGTTTTAAGTAAAGGCCTGCTTAAAGAGGGTAGCTCAGTTGTGGAATTCTCGGGTGCCAAGGTACCAAGAAACCTTCATT |
| OC43-Probe I.485 | AY391777.1 | OC43-Probe I | TGTGAGAGATTCCCTTCGTAATACGACTCACTATAGGGGATCAGGATTTTTATGATTTTGTTTTAAGTAAAGGCCTGCTTAAAGAGGGTAGCTCAGTTGATCTGAAGCACTTTTTCTTTACACAGGATGTGGAATTCTCGGGTGCCAAGGTACCAAGAAACCTTCATT |
| OC43-Probe I.486 | AY391777.1 | OC43-Probe I | TGTGAGAGATTCCCTTCGTAATACGACTCACTATAGGGAAGGCCTGCTTAAAGAGGGTAGCTCAGTTGATCTGAAGCACTTTTTCTTTACACAGGATGGTAATGCTGCTATTACTGATTATAATTATTTGGAATTCTCGGGTGCCAAGGTACCAAGAAACCTTCATT |
| OC43-Probe I.487 | AY391777.1 | OC43-Probe I | TGTGAGAGATTCCCTTCGTAATACGACTCACTATAGGGATCTGAAGCACTTTTTCTTTACACAGGATGGTAATGCTGCTATTACTGATTATAATTATTATAAGTATAATTTGCCCACCATGGTGGACATGGAATTCTCGGGTGCCAAGGTACCAAGAAACCTTCATT |
| OC43-Probe I.488 | AY391777.1 | OC43-Probe I | TGTGAGAGATTCCCTTCGTAATACGACTCACTATAGGGGTAATGCTGCTATTACTGATTATAATTATTATAAGTATAATTTGCCCACCATGGTGGACATTAAGCAGTTGTTGTTTGTTTTGGAAGTTGTGGAATTCTCGGGTGCCAAGGTACCAAGAAACCTTCATT |
| OC43-Probe I.489 | AY391777.1 | OC43-Probe I | TGTGAGAGATTCCCTTCGTAATACGACTCACTATAGGGGTAATGTAATTTGCCCACCATGGTGGACATTAAGCAGTTGTTGTTTGTTTTGGAAGTTGTTTATAAGTATTTTGAGATTTATGATGGTGTGGAATTCTCGGGTGCCAAGGTACCAAGAAACCTTCATT |
| OC43-Probe I.490 | AY391777.1 | OC43-Probe I | TGTGAGAGATTCCCTTCGTAATACGACTCACTATAGGGTTAAGCAGTTGTTGTTTGTTTTGGAAGTTGTTTATAAGTATTTTGAGATTTATGATGGTGGGTGTATACCGGCATCACAAGTCATTGTTATGGAATTCTCGGGTGCCAAGGTACCAAGAAACCTTCATT |
| OC43-Probe I.491 | AY391777.1 | OC43-Probe I | TGTGAGAGATTCCCTTCGTAATACGACTCACTATAGGGTTTATAAGTATTTTGAGATTTATGATGGTGGGTGTATACCGGCATCACAAGTCATTGTTAATAATTATGATAAGAGTGCTGGCTATCCATTGGAATTCTCGGGTGCCAAGGTACCAAGAAACCTTCATT |
| OC43-Probe I.492 | AY391777.1 | OC43-Probe I | TGTGAGAGATTCCCTTCGTAATACGACTCACTATAGGGGGTGTATACCGGCATCACAAGTCATTGTTAATAATTATGATAAGAGTGCTGGCTATCCATTTAACAAATTTGGAAAAGCCAGGCTCTATTTGGAATTCTCGGGTGCCAAGGTACCAAGAAACCTTCATT |

| ASO ID | Accession | Group ID | Custom Array sequence |
|---|---|---|---|
| OC43-Probe I.493 | AY391777.1 | OC43-Probe I | TGTGAGAGATTCCCTTCGTAATACGACTCACTATAGGGATAATTATGATAAGAGTGCTGGCTATCCATTTAACAAATTTGGAAAAGCCAGGCTCTATTATGAAGCATTATCATTTGAGGAACAGGATGTGGAATTCTCGGGTGCCAAGGTACCAAGAAACCTTCATT |
| OC43-Probe I.494 | AY391777.1 | OC43-Probe I | TGTGAGAGATTCCCTTCGTAATACGACTCACTATAGGGTTAACAAATTTGGAAAAGCCAGGCTCTATTATGAAGCATTATCATTTGAGGAACAGGATGAAATTTACGCTTATACTAAGCGTAATGTCCTGGAATTCTCGGGTGCCAAGGTACCAAGAAACCTTCATT |
| OC43-Probe I.495 | AY391777.1 | OC43-Probe I | TGTGAGAGATTCCCTTCGTAATACGACTCACTATAGGGATGAAGCATTATCATTTGAGGAACAGGATGAAATTTACGCTTATACTAAGCGTAATGTCCTGCCAACACTTACTCAAATGAATTTGAAATTGGAATTCTCGGGTGCCAAGGTACCAAGAAACCTTCATT |
| OC43-Probe I.496 | AY391777.1 | OC43-Probe I | TGTGAGAGATTCCCTTCGTAATACGACTCACTATAGGGAAATTTACGCTTATACTAAGCGTAATGTCCTGCCAACACTTACTCAAATGAATTTGAAATATGCTATTAGTGCTAAGAATAGAGCCCGCATGGAATTCTCGGGTGCCAAGGTACCAAGAAACCTTCATT |
| OC43-Probe I.497 | AY391777.1 | OC43-Probe I | TGTGAGAGATTCCCTTCGTAATACGACTCACTATAGGGTGCCAACACTTACTCAAATGAATTTGAAATATGCTATTAGTGCTAAGAATAGAGCCCGCACTGTTGCTGGTGTTTCCATACTTAGTACTATGGAATTCTCGGGTGCCAAGGTACCAAGAAACCTTCATT |
| OC43-Probe I.498 | AY391777.1 | OC43-Probe I | TGTGAGAGATTCCCTTCGTAATACGACTCACTATAGGGATGCTATTAGTGCTAAGAATAGAGCCCGCACTGTTGCTGGTGTTTCCATACTTAGTACTATGACTGGCAGAATGTTTCATCAAAAATGTTTGGAATTCTCGGGTGCCAAGGTACCAAGAAACCTTCATT |
| OC43-Probe I.499 | AY391777.1 | OC43-Probe I | TGTGAGAGATTCCCTTCGTAATACGACTCACTATAGGGCTGTTGCTGGTGTTTCCATACTTAGTACTATGACTGGCAGAATGTTTCATCAAAAATGTTTGAAAAGTATAGCAGCTACACGTGGTGTTCTGGAATTCTCGGGTGCCAAGGTACCAAGAAACCTTCATT |
| OC43-Probe I.500 | AY391777.1 | OC43-Probe I | TGTGAGAGATTCCCTTCGTAATACGACTCACTATAGGGTGACTGGCAGAATGTTTCATCAAAAATGTTTGAAAAGTATAGCAGCTACACGTGGTGTTCCTGTAGTTATAGGCACCACTAAATTTTATGTGGAATTCTCGGGTGCCAAGGTACCAAGAAACCTTCATT |
| OC43-Probe I.501 | AY391777.1 | OC43-Probe I | TGTGAGAGATTCCCTTCGTAATACGACTCACTATAGGGTGAAAAGTATAGCAGCTACACGTGGTGTTCCTGTAGTTATAGGCACCACTAAATTTTATGGTGGCTGGGATGATATGTTACGCCGCCTTATGGAATTCTCGGGTGCCAAGGTACCAAGAAACCTTCATT |
| OC43-Probe I.502 | AY391777.1 | OC43-Probe I | TGTGAGAGATTCCCTTCGTAATACGACTCACTATAGGGCTGTAGTTATAGGCACCACTAAATTTTATGGTGGCTGGGATGATATGTTACGCCGCCTTATTAAAGATGTTGACAATCCTGTACTTATGGTGGAATTCTCGGGTGCCAAGGTACCAAGAAACCTTCATT |
| OC43-Probe I.503 | AY391777.1 | OC43-Probe I | TGTGAGAGATTCCCTTCGTAATACGACTCACTATAGGGGTGGCTGGGATGATATGTTACGCCGCCTTATTAAAGATGTTGACAATCCTGTACTTATGGGTGGGATTATCCTAAGTGTGATCGTGCTATGGAATTCTCGGGTGCCAAGGTACCAAGAAACCTTCATT |
| OC43-Probe I.504 | AY391777.1 | OC43-Probe I | TGTGAGAGATTCCCTTCGTAATACGACTCACTATAGGGTTAAAGATGTTGACAATCCTGTACTTATGGGTGGGATTATCCTAAGTGTGATCGTGCTATGCCAAACCTACTACGTATTGTTAGTAGTTTGGAATTCTCGGGTGCCAAGGTACCAAGAAACCTTCATT |
| OC43-Probe I.505 | AY391777.1 | OC43-Probe I | TGTGAGAGATTCCCTTCGTAATACGACTCACTATAGGGGTTGGGATTATCCTAAGTGTGATCGTGCTATGCCAAACCTACTACGTATTGTTAGTAGTTTGGTATTAGCCCGAAAACATGGACATGTTTGGAATTCTCGGGTGCCAAGGTACCAAGAAACCTTCATT |
| OC43-Probe I.506 | AY391777.1 | OC43-Probe I | TGTGAGAGATTCCCTTCGTAATACGACTCACTATAGGGTGCCAAACCTACTACGTATTGTTAGTAGTTTGGTATTAGCCCGAAAACATGAGACATGTTGTTCGCAAAGCGATAGGTTTTATCGACTTGTGGAATTCTCGGGTGCCAAGGTACCAAGAAACCTTCATT |
| OC43-Probe I.507 | AY391777.1 | OC43-Probe I | TGTGAGAGATTCCCTTCGTAATACGACTCACTATAGGGTGGTATTAGCCCGAAAACATGAGACATGTTGTTCGCAAAGCGATAGGTTTTATCGACTTGCGAATGAATGCGCACAAGTTTTGAGTGAAATGGAATTCTCGGGTGCCAAGGTACCAAGAAACCTTCATT |
| OC43-Probe I.508 | AY391777.1 | OC43-Probe I | TGTGAGAGATTCCCTTCGTAATACGACTCACTATAGGGGTTCGCAAAGCGATAGGTTTTATCGACTTGCGAATGAATGCGCACAAGTTTTGAGTGAAATTGTTATGTGTGGTGGCTGTTATTATGTTATGGAATTCTCGGGTGCCAAGGTACCAAGAAACCTTCATT |
| OC43-Probe I.509 | AY391777.1 | OC43-Probe I | TGTGAGAGATTCCCTTCGTAATACGACTCACTATAGGGCGAATGAATGCGCACAAGTTTTGAGTGAAATTGTTATGTGTGGTGGCTGTTATTATGTTAAGCCTGGTGGCACTAGTAGTGGTGATGCAATGGAATTCTCGGGTGCCAAGGTACCAAGAAACCTTCATT |
| OC43-Probe I.510 | AY391777.1 | OC43-Probe I | TGTGAGAGATTCCCTTCGTAATACGACTCACTATAGGGTTGTTATGTGTGGTGGCTGTTATTATGTTAAGCCTGGTGGCACTAGTAGTGGTGATGCAACTACTGCTTTTGCTAATTCAGTCTTTAACATGGAATTCTCGGGTGCCAAGGTACCAAGAAACCTTCATT |
| OC43-Probe I.511 | AY391777.1 | OC43-Probe I | TGTGAGAGATTCCCTTCGTAATACGACTCACTATAGGGAGCCTGGTGGCACTAGTAGTGGTGATGCAACTACTGCTTTTGCTAATTCAGTCTTTAACATATGTCAAGCTGTTTCAGCCAATGTATGTGTGGAATTCTCGGGTGCCAAGGTACCAAGAAACCTTCATT |
| OC43-Probe I.512 | AY391777.1 | OC43-Probe I | TGTGAGAGATTCCCTTCGTAATACGACTCACTATAGGGCTACTGCTTTTGCTAATTCAGTCTTTAACATATGTCAAGCTGTTTCAGCCAATGTATGTGCCTTAATGTCATGCAATGGCAATAAGATTGTGGAATTCTCGGGTGCCAAGGTACCAAGAAACCTTCATT |
| OC43-Probe I.513 | AY391777.1 | OC43-Probe I | TGTGAGAGATTCCCTTCGTAATACGACTCACTATAGGGTATGTCAAGCTGTTTCAGCCAATGTATGTGCCTTAATGTCATGCAATGGCAATAAGATTGAAGATCTTAGTATACGTGCTCTTCAGAAGCTGGAATTCTCGGGTGCCAAGGTACCAAGAAACCTTCATT |
| OC43-Probe I.514 | AY391777.1 | OC43-Probe I | TGTGAGAGATTCCCTTCGTAATACGACTCACTATAGGGCCTTAATGTCATGCAATGGCAATAAGATTGAAGATCTTAGTATACGTGCTCTTCAGAAGCGCTTATACTCACATGTGTATAGAAGTGATATGGAATTCTCGGGTGCCAAGGTACCAAGAAACCTTCATT |
| OC43-Probe I.515 | AY391777.1 | OC43-Probe I | TGTGAGAGATTCCCTTCGTAATACGACTCACTATAGGGAAGATCTTAGTATACGTGCTCTTCAGAAGCGCTTATACTCACATGTGTATAGAAGTGATAAGGTTGATTCAACCTTTGTCACAGAATATTTGGAATTCTCGGGTGCCAAGGTACCAAGAAACCTTCATT |
| OC43-Probe I.516 | AY391777.1 | OC43-Probe I | TGTGAGAGATTCCCTTCGTAATACGACTCACTATAGGGGCTTATACTCACATGTGTATAGAAGTGATAAGGTTGATTCAACCTTTGTCACAGAATATTATGAATTTTTAAATAAGCATTTTAGTATGATGGAATTCTCGGGTGCCAAGGTACCAAGAAACCTTCATT |
| OC43-Probe I.517 | AY391777.1 | OC43-Probe I | TGTGAGAGATTCCCTTCGTAATACGACTCACTATAGGGGAGGTTGATTCAACCTTTGTCACAGAATATTATGAATTTTTAAATAAGCATTTTAGTATGATGATTTTGAGTGATGATGGGGTTGTGTGTTTGGAATTCTCGGGTGCCAAGGTACCAAGAAACCTTCATT |

| ASO ID | Accession | Group ID | Custom Array sequence |
|---|---|---|---|
| OC43-Probe I.518 | AY391777.1 | OC43-Probe I | TGTGAGAGATTCCCTTCGTAATACGACTCACTATAGGGATGAATTTTTAAATAAGCATTTTAGTATGATGATTTTGAGTGATGATGGGGTTGTGTGTTATAATTCTGATTATGCGTCCAAAGGGTATATGGAATTCTCGGGTGCCAAGGTACCAAGAAACCTTCATT |
| OC43-Probe I.519 | AY391777.1 | OC43-Probe I | TGTGAGAGATTCCCTTCGTAATACGACTCACTATAGGGTGATTTTGAGTGATGATGGGGTTGTGTGTTATAATTCTGATTATGCGTCCAAAGGGTATATTGCTAATATAAGTGCCTTTCAACAGGTATTGGAATTCTCGGGTGCCAAGGTACCAAGAAACCTTCATT |
| OC43-Probe I.520 | AY391777.1 | OC43-Probe I | TGTGAGAGATTCCCTTCGTAATACGACTCACTATAGGGATAAATTCTGATTATGCGTCCAAAGGGTATATTGCTAATATAAGTGCCTTTCAACAGGTATTATATTATCAAAATAACGTTTTTATGTCAGTGGAATTCTCGGGTGCCAAGGTACCAAGAAACCTTCATT |
| OC43-Probe I.521 | AY391777.1 | OC43-Probe I | TGTGAGAGATTCCCTTCGTAATACGACTCACTATAGGGTTGCTAATATAAGTGCCTTTCAACAGGTATTATATTATCAAAATAACGTTTTTATGTCAGAATCCAAATGTTGGGTTGAACATGACATAATGGAATTCTCGGGTGCCAAGGTACCAAGAAACCTTCATT |
| OC43-Probe I.522 | AY391777.1 | OC43-Probe I | TGTGAGAGATTCCCTTCGTAATACGACTCACTATAGGGTATATTATCAAAATAACGTTTTTATGTCAGAATCCAAATGTTGGGTTGAACATGACATAAATAATGGACCTCATGAATTCTGTTCACAACTGGAATTCTCGGGTGCCAAGGTACCAAGAAACCTTCATT |
| OC43-Probe I.523 | AY391777.1 | OC43-Probe I | TGTGAGAGATTCCCTTCGTAATACGACTCACTATAGGGAATCCAAATGTTGGGTTGAACATGACATAAATAATGGACCTCATGAATTCTGTTCACAACACACAATGCTTGTAAAGATGGATGGTGACGTGGAATTCTCGGGTGCCAAGGTACCAAGAAACCTTCATT |
| OC43-Probe I.524 | AY391777.1 | OC43-Probe I | TGTGAGAGATTCCCTTCGTAATACGACTCACTATAGGGATAATGGACCTCATGAATTCTGTTCACAACACACAATGCTTGTAAAGATGGATGGTGACGATGTCTACCTTCCATATCCTAATCCTAGTCTGGAATTCTCGGGTGCCAAGGTACCAAGAAACCTTCATT |
| OC43-Probe I.525 | AY391777.1 | OC43-Probe I | TGTGAGAGATTCCCTTCGTAATACGACTCACTATAGGGACACAATGCTTGTAAAGATGGATGGTGACGATGTCTACCTTCCATATCCTAATCCTAGTCGTATATTAGGAGCTGGATGTTTTGTAGATGTGGAATTCTCGGGTGCCAAGGTACCAAGAAACCTTCATT |
| OC43-Probe I.526 | AY391777.1 | OC43-Probe I | TGTGAGAGATTCCCTTCGTAATACGACTCACTATAGGGATGTCTACCTTCCATATCCTAATCCTAGTCGTATATTAGGAGCTGGATGTTTTGTAGATGATTTGTTAAAGACTGATAGTGTTCTTTTAATGGAATTCTCGGGTGCCAAGGTACCAAGAAACCTTCATT |
| OC43-Probe I.527 | AY391777.1 | OC43-Probe I | TGTGAGAGATTCCCTTCGTAATACGACTCACTATAGGGGTATATTAGGAGCTGGATGTTTTGTAGATGATTTGTTAAAGACTGATAGTGTTCTTTTAATAGAACGATTTGTAAGTCTTGCAATAGATGTGGAATTCTCGGGTGCCAAGGTACCAAGAAACCTTCATT |
| OC43-Probe I.528 | AY391777.1 | OC43-Probe I | TGTGAGAGATTCCCTTCGTAATACGACTCACTATAGGGATTTGTTAAAGACTGATAGTGTTCTTTTAATAGAACGATTTGTAAGTCTTGCAATAGATGCTTATCCACTTGTGTATCATGAAAATGAAGTGGAATTCTCGGGTGCCAAGGTACCAAGAAACCTTCATT |
| OC43-Probe I.529 | AY391777.1 | OC43-Probe I | TGTGAGAGATTCCCTTCGTAATACGACTCACTATAGGGTAGAACGATTTGTAAGTCTTGCAATAGATGCTTATCCACTTGTGTATCATGAAAATGAAGAATACCAAAAGGTTTTTCGTGTTTATTTGGTGGAATTCTCGGGTGCCAAGGTACCAAGAAACCTTCATT |
| OC43-Probe I.530 | AY391777.1 | OC43-Probe I | TGTGAGAGATTCCCTTCGTAATACGACTCACTATAGGGCTTATCCACTTGTGTATCATGAAAATGAAGAATACCAAAAGGTTTTTCGTGTTTATTTGGCGTATATAAAGAAGTTGTACAATGACCTGGTGGAATTCTCGGGTGCCAAGGTACCAAGAAACCTTCATT |
| OC43-Probe I.531 | AY391777.1 | OC43-Probe I | TGTGAGAGATTCCCTTCGTAATACGACTCACTATAGGGAATACCAAAAGGTTTTTCGTGTTTATTTGGCGTATATAAAGAAGTTGTACAATGACCTGGGTAATCAGATCTTGGATAGCTACAGTGTTATGGAATTCTCGGGTGCCAAGGTACCAAGAAACCTTCATT |
| OC43-Probe I.532 | AY391777.1 | OC43-Probe I | TGTGAGAGATTCCCTTCGTAATACGACTCACTATAGGGCGTATATAAAGAAGTTGTACAATGACCTGGGTAATCAGATCTTGGATAGCTACAGTGTTATTTTAAGTACTTGTGATGGACAAAAGTTCATGGAATTCTCGGGTGCCAAGGTACCAAGAAACCTTCATT |
| OC43-Probe I.533 | AY391777.1 | OC43-Probe I | TGTGAGAGATTCCCTTCGTAATACGACTCACTATAGGGGTAATCAGATCTTGGATAGCTACAGTGTTATTTTAAGTACTTGTGATGGACAAAAGTTCACTGATGAGTCCTTTTACAAGAACATGTATTTGGAATTCTCGGGTGCCAAGGTACCAAGAAACCTTCATT |
| OC43-Probe I.534 | AY391777.1 | OC43-Probe I | TGTGAGAGATTCCCTTCGTAATACGACTCACTATAGGGTTTTAAGTACTTGTGATGGACAAAAGTTCACTGATGAGTCCTTTTACAAGAACATGTATTTAAGAAGTGCAGTTATGCAGAGTGTTGGAGTGGAATTCTCGGGTGCCAAGGTACCAAGAAACCTTCATT |
| OC43-Probe I.535 | AY391777.1 | OC43-Probe I | TGTGAGAGATTCCCTTCGTAATACGACTCACTATAGGGCTGATGAGTCCTTTTACAAGAACATGTATTTAAGAAGTGCAGTTATGCAGAGTGTTGGAGCTTGCGTGGTCTGCTCTTCTCAAACATCATTGGAATTCTCGGGTGCCAAGGTACCAAGAAACCTTCATT |
| OC43-Probe I.536 | AY391777.1 | OC43-Probe I | TGTGAGAGATTCCCTTCGTAATACGACTCACTATAGGGTAAGAAGTGCAGTTATGCAGAGTGTTGGAGCTTGCGTGGTCTGCTCTTCTCAAACATCATTACGTTGTGGCAGTTGCATCAGAAAGCCTCTGGAATTCTCGGGTGCCAAGGTACCAAGAAACCTTCATT |
| OC43-Probe I.537 | AY391777.1 | OC43-Probe I | TGTGAGAGATTCCCTTCGTAATACGACTCACTATAGGGCTTGCGTGGTCTGCTCTTCTCAAACATCATTACGTTGTGGCAGTTGCATCAGAAAGCCTCTTCTTTGCTGCAAGTGTTGTTATGATCATGTGGAATTCTCGGGTGCCAAGGTACCAAGAAACCTTCATT |
| OC43-Probe I.538 | AY391777.1 | OC43-Probe I | TGTGAGAGATTCCCTTCGTAATACGACTCACTATAGGGTACGTTGTGGCAGTTGCATCAGAAAGCCTCTTCTTTGCTGCAAGTGTTGTTATGATCATGTTATGGCGACTGATCATAAATATGTCTTGAGTGGAATTCTCGGGTGCCAAGGTACCAAGAAACCTTCATT |
| OC43-Probe I.539 | AY391777.1 | OC43-Probe I | TGTGAGAGATTCCCTTCGTAATACGACTCACTATAGGGTTCTTTGCTGCAAGTGTTGTTATGATCATGTTATGGCGACTGATCATAAATATGTCTTGAGTGTTTCACCATATGTGTGTAATGCACCAGTGGAATTCTCGGGTGCCAAGGTACCAAGAAACCTTCATT |
| OC43-Probe I.540 | AY391777.1 | OC43-Probe I | TGTGAGAGATTCCCTTCGTAATACGACTCACTATAGGGTTATGGCGACTGATCATAAATATGTCTTGAGTGTTTCACCATATGTGTGTAATGCACCAGGATGTGATGTAAATGATGTTACCCAAATTGTTGGAATTCTCGGGTGCCAAGGTACCAAGAAACCTTCATT |
| OC43-Probe I.541 | AY391777.1 | OC43-Probe I | TGTGAGAGATTCCCTTCGTAATACGACTCACTATAGGGTGTTTCACCATATGTGTGTAATGCACCAGGATGTGATGTAAATGATGTTACCCAAATTGTATCTAGGTGGTATGTCATATTATTGTGAAGTGGAATTCTCGGGTGCCAAGGTACCAAGAAACCTTCATT |
| OC43-Probe I.542 | AY391777.1 | OC43-Probe I | TGTGAGAGATTCCCTTCGTAATACGACTCACTATAGGGGATGTGATGTAAATGATGTTACCCAAATTGTATCTAGGTGGTATGTCATATTATTGTGAAGACCATAAGCCACAATATTCATTCAAGTTGGTGGAATTCTCGGGTGCCAAGGTACCAAGAAACCTTCATT |

82

| ASO ID | Accession | Group ID | Custom Array sequence |
|---|---|---|---|
| OC43-Probe I.543 | AY391777.1 | OC43-Probe I | TGTGAGAGATTCCCTTCGTAATACGACTCACTATAGGGATCTAGGTGGTATGTCATATTATTGTGAAGACCATAAGCCACAATATTCATTCAAGTTGGTAATGAATGGTCTGGTTTTTGGTCTATATATGGAATTCTCGGGTGCCAAGGTACCAAGAAACCTTCATT |
| OC43-Probe I.544 | AY391777.1 | OC43-Probe I | TGTGAGAGATTCCCTTCGTAATACGACTCACTATAGGGACCATAAGCCACAATATTCATTCAAGTTGGTAATGAATGGTCTGGTTTTTGGTCTATATAAACAATCTTGTACAGGATCTCCGTACATAGTGGAATTCTCGGGTGCCAAGGTACCAAGAAACCTTCATT |
| OC43-Probe I.545 | AY391777.1 | OC43-Probe I | TGTGAGAGATTCCCTTCGTAATACGACTCACTATAGGGTAATGAATGGTCTGGTTTTTGGTCTATATAAACAATCTTGTACAGGATCTCCGTACATAGACGATTTTAATCGTATAGCTAGTTGTAAATTGGAATTCTCGGGTGCCAAGGTACCAAGAAACCTTCATT |
| OC43-Probe I.546 | AY391777.1 | OC43-Probe I | TGTGAGAGATTCCCTTCGTAATACGACTCACTATAGGGAACAATCTTGTACAGGATCTCCGTACATAGACGATTTTAATCGTATAGCTAGTTGTAAATGGACCGATGTGGATGATTACATACTAGCTATGGAATTCTCGGGTGCCAAGGTACCAAGAAACCTTCATT |
| OC43-Probe I.547 | AY391777.1 | OC43-Probe I | TGTGAGAGATTCCCTTCGTAATACGACTCACTATAGGGACGATTTTAATCGTATAGCTAGTTGTAAATGGACCGATGTGGATGATTACATACTAGCTAATGAATGTACAGAGCGCTTGAAATTGTTTGTGGAATTCTCGGGTGCCAAGGTACCAAGAAACCTTCATT |
| OC43-Probe I.548 | AY391777.1 | OC43-Probe I | TGTGAGAGATTCCCTTCGTAATACGACTCACTATAGGGGGACCGATGTGGATGATTACATACTAGCTAATGAATGTACAGAGCGCTTGAAATTGTTTGCTGCAGAAACGCAAAAGGCAACCGAGGAAGTGGAATTCTCGGGTGCCAAGGTACCAAGAAACCTTCATT |
| OC43-Probe I.549 | AY391777.1 | OC43-Probe I | TGTGAGAGATTCCCTTCGTAATACGACTCACTATAGGGATGAATGTACAGAGCGCTTGAAATTGTTTGCTGCAGAAACGCAAAAGGCAACCGAGGAAGCCTTTAAGCAGAGTTATGCATCAGCAACAATGGAATTCTCGGGTGCCAAGGTACCAAGAAACCTTCATT |
| OC43-Probe I.550 | AY391777.1 | OC43-Probe I | TGTGAGAGATTCCCTTCGTAATACGACTCACTATAGGGCTGCAGAAACGCAAAAGGCAACCGAGGAAGCCTTTAAGCAGAGTTATGCATCAGCAACAATACAAGAGATTGTTAGTGAGCGCGAATTGATGGAATTCTCGGGTGCCAAGGTACCAAGAAACCTTCATT |
| OC43-Probe I.551 | AY391777.1 | OC43-Probe I | TGTGAGAGATTCCCTTCGTAATACGACTCACTATAGGGCCTTTAAGCAGAGTTATGCATCAGCAACAATACAAGAGATTGTTAGTGAGCGCGAATTGATTCTCTCTTGGGAGATTGGAAAAGTTAAGCTGGAATTCTCGGGTGCCAAGGTACCAAGAAACCTTCATT |
| OC43-Probe I.552 | AY391777.1 | OC43-Probe I | TGTGAGAGATTCCCTTCGTAATACGACTCACTATAGGGTACAAGAGATTGTTAGTGAGCGCGAATTGATTCTCTCTTGGGAGATTGGAAAAGTTAAGCCACCACTTAATAAAAATTATGTTTTTACTGTGGAATTCTCGGGTGCCAAGGTACCAAGAAACCTTCATT |
| OC43-Probe I.553 | AY391777.1 | OC43-Probe I | TGTGAGAGATTCCCTTCGTAATACGACTCACTATAGGGTTCTCTCTTGGGAGATTGGAAAAGTTAAGCCACCACTTAATAAAAATTATGTTTTTACTGGCTACCATTTTACTAAAAATGGTAAGACAGTGGAATTCTCGGGTGCCAAGGTACCAAGAAACCTTCATT |
| OC43-Probe I.554 | AY391777.1 | OC43-Probe I | TGTGAGAGATTCCCTTCGTAATACGACTCACTATAGGGCACCCACTTAATAAAAATTATGTTTTTACTGGCTACCATTTTACTAAAAATGGTAAGACAGTTTTAGGTGAGTATGTTTTTGATAAGAGTGTGGAATTCTCGGGTGCCAAGGTACCAAGAAACCTTCATT |
| OC43-Probe I.555 | AY391777.1 | OC43-Probe I | TGTGAGAGATTCCCTTCGTAATACGACTCACTATAGGGGGCTACCATTTTACTAAAAATGGTAAGACAGTTTTAGGTGAGTATGTTTTTGATAAGAGTGAGTTGACTAATGGTGTGTATTATCGCGCCATGGAATTCTCGGGTGCCAAGGTACCAAGAAACCTTCATT |
| OC43-Probe I.556 | AY391777.1 | OC43-Probe I | TGTGAGAGATTCCCTTCGTAATACGACTCACTATAGGGTTTTAGGTGAGTATGTTTTTGATAAGAGTGAGTTGACTAATGGTGTGTATTATCGCGCCACAACCACTTATAAGCTATCGTAGGAGATGTGGAATTCTCGGGTGCCAAGGTACCAAGAAACCTTCATT |
| OC43-Probe I.557 | AY391777.1 | OC43-Probe I | TGTGAGAGATTCCCTTCGTAATACGACTCACTATAGGGAGTTGACTAATGGTGTGTATTATCGCGCCACAACCACTTATAAGCTATCGTAGGAGATGTTTTTGTTTAACCTCTCATTCAGTAGCTATGGAATTCTCGGGTGCCAAGGTACCAAGAAACCTTCATT |
| OC43-Probe I.558 | AY391777.1 | OC43-Probe I | TGTGAGAGATTCCCTTCGTAATACGACTCACTATAGGGCAACCACTTATAAGCTATCGTAGGAGATGTTTTTGTTTAACCTCTCATTCAGTAGCTAATTTAAGTGCTCCTACGCTTGTTCCGCAGGTGGAATTCTCGGGTGCCAAGGTACCAAGAAACCTTCATT |
| OC43-Probe I.559 | AY391777.1 | OC43-Probe I | TGTGAGAGATTCCCTTCGTAATACGACTCACTATAGGGTTTTTGTTTAACCTCTCATTCAGTAGCTAATTTAAGTGCTCCTACGCTTGTTCCGCAGGAGAATTATAGTAGTATTAGATTTGCTAGTGTGGAATTCTCGGGTGCCAAGGTACCAAGAAACCTTCATT |
| OC43-Probe I.560 | AY391777.1 | OC43-Probe I | TGTGAGAGATTCCCTTCGTAATACGACTCACTATAGGGATTTAAGTGCTCCTACGCTTGTTCCGCAGGAGAATTATAGTAGTATTAGATTTGCTAGTGTTTATAGTGTGCTTGAGACGTTTCAGAACATGGAATTCTCGGGTGCCAAGGTACCAAGAAACCTTCATT |
| OC43-Probe I.561 | AY391777.1 | OC43-Probe I | TGTGAGAGATTCCCTTCGTAATACGACTCACTATAGGGAGGAGAATTATAGTAGTATTAGATTTGCTAGTGTTTATAGTGTGCTTGAGACGTTTCAGAACAATGTTGTTAATTATCAACACATTGGTATGATGGAATTCTCGGGTGCCAAGGTACCAAGAAACCTTCATT |
| OC43-Probe I.562 | AY391777.1 | OC43-Probe I | TGTGAGAGATTCCCTTCGTAATACGACTCACTATAGGGTTTATAGTGTGCTTGAGACGTTTCAGAACAATGTTGTTAATTATCAACACATTGGTATGAAACGTTACTGCACCGTGCAAGGACCTCCTGTGGAATTCTCGGGTGCCAAGGTACCAAGAAACCTTCATT |
| OC43-Probe I.563 | AY391777.1 | OC43-Probe I | TGTGAGAGATTCCCTTCGTAATACGACTCACTATAGGGATGTTGTTAATTATCAACACATTGGTATGAAACGTTACTGCACCGTGCAAGGACCTCCTGGTACAGGGAAGTCACATCTTGCTATTGGTCTGGAATTCTCGGGTGCCAAGGTACCAAGAAACCTTCATT |
| OC43-Probe I.564 | AY391777.1 | OC43-Probe I | TGTGAGAGATTCCCTTCGTAATACGACTCACTATAGGGAACGTTACTGCACCGTGCAAGGACCTCCTGGTACAGGGAAGTCACATCTTGCTATTGGTCTTGCTGTATTCTATTGTACAGCACGTGTTGTGGAATTCTCGGGTGCCAAGGTACCAAGAAACCTTCATT |
| OC43-Probe I.565 | AY391777.1 | OC43-Probe I | TGTGAGAGATTCCCTTCGTAATACGACTCACTATAGGGGTACAGGGAAGTCACATCTTGCTATTGGTCTTGCTGTATTCTATTGTACAGCACGTGTTGTATACACAGCGGCCAGCCATGCAGCTGTTGTGGAATTCTCGGGTGCCAAGGTACCAAGAAACCTTCATT |
| OC43-Probe I.566 | AY391777.1 | OC43-Probe I | TGTGAGAGATTCCCTTCGTAATACGACTCACTATAGGGTTGCTGTATTCTATTGTACAGCACGTGTTGTATACACAGCGGCCAGCCATGCAGCTGTTGACGCATTGTGTGAAAAAGCATATAAATTTTTGGAATTCTCGGGTGCCAAGGTACCAAGAAACCTTCATT |
| OC43-Probe I.567 | AY391777.1 | OC43-Probe I | TGTGAGAGATTCCCTTCGTAATACGACTCACTATAGGGTATACACAGCGGCCAGCCATGCAGCTGTTGACGCATTGTGTGAAAAAGCATATAAATTTTTGAATATAAATGATTGCACTCGTATTGTTCTGGAATTCTCGGGTGCCAAGGTACCAAGAAACCTTCATT |

| ASO ID | Accession | Group ID | Custom Array sequence |
|---|---|---|---|
| OC43-Probe I.568 | AY391777.1 | OC43-Probe I | TGTGAGAGATTCCCTTCGTAATACGACTCACTATAGGGACGCATTGTGTAATACGACTCACTATAGGGTGGAGTGCTGCACTCGTATGATATGGAATATAAAATTTTGAAATATAAATTCTCGGGTGCCAAGGTACCAAGAAACCTTCATT |
| OC43-Probe I.569 | AY391777.1 | OC43-Probe I | TGTGAGAGATTCCCTTCGTAATACGACTCACTATAGGGTGAATATAAATAAATGATTGCACTCGTATTGTTCCGGCCAAGGTCAGGGTGGAGTGCTATGATAAGTTAAAATTAATGACCACCACTCGTAAGTTGGAATTCTCGGGTGCCAAGGTACCAAGAAACCTTCATT |
| OC43-Probe I.570 | AY391777.1 | OC43-Probe I | TGTGAGAGATTCCCTTCGTAATACGACTCACTATAGGGCCGGCCAAGGTCGTATGATAAGTTAAAATTAATGACCACCACTCGTATTAATGACCATAAATGCATTACTACCATAAATGCTGTTACTCTCGGGTGCCAAGGTACCAAGAAACCTTCATT |
| OC43-Probe I.571 | AY391777.1 | OC43-Probe I | TGTGAGAGATTCCCTTCGTAATACGACTCACTATAGGGGAGTTAAAATTAATGACCATAAATGCATTACTACCATAAATGCTGATATGGTGACTGATATGTTGTAGATGGTTGTGTAGATGGTTCTCGGGTGCCAAGGTACCAAGAAACCTTCATT |
| OC43-Probe I.572 | AY391777.1 | OC43-Probe I | TGTGAGAGATTCCCTTCGTAATACGACTCACTATAGGGGATGGTGTTTACTACCATAAATGCTGATATGGTTGTGTAGATGGTTGGAAGTTAGTATGCTTAATGGCTTTACCAATTATGACGCTTTACCAATTATGACGCTCTCGGGTGCCAAGGTACCAAGAAACCTTCATT |
| OC43-Probe I.573 | AY391777.1 | OC43-Probe I | TGTGAGAGATTCCCTTCGTAATACGACTCACTATAGGGGATGTGTTTACTACCAATTATGACGCTTTACCAATTATGACGCTTTATTAATGCTGTATTCGTATTCGTGTTATTATTCGGCGTCGGGTGCCAAGGTACCAAGAAACCTTCATT |
| OC43-Probe I.574 | AY391777.1 | OC43-Probe I | TGTGAGAGATTCCCTTCGTAATACGACTCACTATAGGGGAAGTTAGTATGCTTACCAATTATGACGCTTTATTAATGCTGTATTCGTATTGGTGATCTGCTCAATTGCCAGCACCACGTGTTATTGAGCAAGGGTCTCGGGTGCCAAGGTACCAAGAAACCTTCATT |
| OC43-Probe I.575 | AY391777.1 | OC43-Probe I | TGTGAGAGATTCCCTTCGTAATACGACTCACTATAGGGGAAGTTAGTATGTTTCTGTTATTAATGCTGTATTCGTGATCTGCTCAATTGCCAGCACCACGTGTTATTGAGCAAGGGTACACTTGAACCTAAATATTTCTCGGGTGCCAAGGTACCAAGAAACCTTCATT |
| OC43-Probe I.576 | AY391777.1 | OC43-Probe I | TGTGAGAGATTCCCTTCGTAATACGACTCACTATAGGGGTGCCAGCACACGTGTTATTGAGCAAGGGTACACTTGAACCTAAATATTTAACACTGTTGCTTAGGGCCAGTGTCTCGGGTGCCAAGGTACCAAGAAACCTTCATT |
| OC43-Probe I.577 | AY391777.1 | OC43-Probe I | TGTGAGAGATTCCCTTCGTAATACGACTCACTATAGGGGTACACTTGAACCTAAATATTTAACACTGTTGCTTAGGGCCAGTGTCTGGTACATGTTATACACTGTTGCTTAGGGCCAGTGTCTGGTACATGTTATAGATGTCTGGTCTCGGGTGCCAAGGTACCAAGAAACCTTCATT |
| OC43-Probe I.578 | AY391777.1 | OC43-Probe I | TGTGAGAGATTCCCTTCGTAATACGACTCACTATAGGGGTAACCTAAATATTTTCTTGGTACATGTTATAGATGTCTGGTACATGTTATAGATGTCCGCCTTGGATCAGTACAGTGTTGATACAGTGTCTCGGGTGCCAAGGTACCAAGAAACCTTCATT |
| OC43-Probe I.579 | AY391777.1 | OC43-Probe I | TGTGAGAGATTCCCTTCGTAATACGACTCACTATAGGGGTTACTAAGCTCATGTGTTTCTTGGTACATGTTATAGATGTCCGCCTTGGATCAGTACAGTGTTGATACAGTTTATGAAAATAAGCTTAAGGCTCTCGGGTGCCAAGGTACCAAGAAACCTTCATT |
| OC43-Probe I.580 | AY391777.1 | OC43-Probe I | TGTGAGAGATTCCCTTCGTAATACGACTCACTATAGGGGACAGTTTTCTTGGTACATGTTATAGATGTCCGCCTTGGATCAGTATGATGTCGTTATGAAAATAAGCTTAAGGCTTATGAAAATAAGCTTAAGGCTCTATTATCTCGGGTGCCAAGGTACCAAGAAACCTTCATT |
| OC43-Probe I.581 | AY391777.1 | OC43-Probe I | TGTGAGAGATTCCCTTCGTAATACGACTCACTATAGGGGTAATGGCCTTGATGTCGTTATGAAAATAAGCTTAAGGCTTATGAAAATAAGCTTAAGGCTCTATTATAAAGGGCGGTACAACACATGAAAAGTTTCTCGGGTGCCAAGGTACCAAGAAACCTTCATT |
| OC43-Probe I.582 | AY391777.1 | OC43-Probe I | TGTGAGAGATTCCCTTCGTAATACGACTCACTATAGGGGTGGTTTATGAAAATAAGCTTCATGTGTTTAAGGTCTATTATAAAGGGCGGTACAACACATGAAAAGTTTCTAGTGCTGTAAATATGCTCTCGGGTGCCAAGGTACCAAGAAACCTTCATT |
| OC43-Probe I.583 | AY391777.1 | OC43-Probe I | TGTGAGAGATTCCCTTCGTAATACGACTCACTATAGGGGATGAGAGTAGTTCATTGTTTAAGGTCTATTATAAAGGGCGGTACAACACATGAAAAGTTTCTAGTGCTGTAAATATGCTCTAGTGCTGTAAATAAGCTCTCGGGTGCCAAGGTACCAAGAAACCTTCATT |
| OC43-Probe I.584 | AY391777.1 | OC43-Probe I | TGTGAGAGATTCCCTTCGTAATACGACTCACTATAGGGGCTACAACACATGAAAAGTTTCTAGTGCTGTAAATATGCTAGTGCTGTAAATAAGCTCAGTATCAGAAATGGGCTAACCCTTTGAAGGCTAACCCTTCTCGGGTGCCAAGGTACCAAGAAACCTTCATT |
| OC43-Probe I.585 | AY391777.1 | OC43-Probe I | TGTGAGAGATTCCCTTCGTAATACGACTCACTATAGGGGCTAGTGCTGTAAATGCAGAGATTATTTGATTAATAAGTTTTTATTGGCATAAAGCTGTTTTTTATTAGCCCCTTTTATTAGCCCCATATAAATAGTCTCGGGTGCCAAGGTACCAAGAAACCTTCATT |
| OC43-Probe I.586 | AY391777.1 | OC43-Probe I | TGTGAGAGATTCCCTTCGTAATACGACTCACTATAGGGGAGTAGTGTATTATTTGATTAATAAGCTGTTTTTATTGGCATAAAGCTGTTTTTATTAGCCCATATAAATAGTCAGCCATATAATAAGTCAGCATCTCGGGTGCCAAGGTACCAAGAAACCTTCATT |
| OC43-Probe I.587 | AY391777.1 | OC43-Probe I | TGTGAGAGATTCCCTTCGTAATACGACTCACTATAGGGGAGTAGTGTGGCATAAAGTTTTTATTAGCCCCTTTTATTAGCCCATATAAATAGTCAGCATAATAATAGTCTGGGTTTATTAGCCCAAACCCCTGTGTTTTCTCGGGTGCCAAGGTACCAAGAAACCTTCATT |
| OC43-Probe I.588 | AY391777.1 | OC43-Probe I | TGTGAGAGATTCCCTTCGTAATACGACTCACTATAGGGGTGTGGCATAAAGTTTTTATTAGCCCATATAAATAGTCAGCATAATAATAGTCTGGGTTTACAAAACCCAAACCCGTGTTTGCTGCTGCTCTCGGGTGCCAAGGTACCAAGAAACCTTCATT |
| OC43-Probe I.589 | AY391777.1 | OC43-Probe I | TGTGAGAGATTCCCTTCGTAATACGACTCACTATAGGGGTGGGATTTTGGGTTTACAAAACCCAAACCCGTGTTTGCTGCTGATTTACACAGACTGCAGAAACAGCCGCATTCGTGAAATATGATTATGCTCTCGGGTGCCAAGGTACCAAGAAACCTTCATT |
| OC43-Probe I.590 | AY391777.1 | OC43-Probe I | TGTGAGAGATTCCCTTCGTAATACGACTCACTATAGGGGATGTTGGGTTTACAAAACCCAAACCCGTGTTTGCTGCTGATTTACACAGACTGCAGAAACAGCCGCATTCGTGAAATGTTATATTCACACAGACTGTTATATTCTCGGGTGCCAAGGTACCAAGAAACCTTCATT |
| OC43-Probe I.591 | AY391777.1 | OC43-Probe I | TGTGAGAGATTCCCTTCGTAATACGACTCACTATAGGGGTATATTCACAGACTGCAGAAACAGCCGCATTCGTGAAATGTTATATTCACACAGACTGTTATATTCGCTTCACAGACTGTTAATCGCTTCGAGCCAAGAAAAGTATTCTCGGGTGCCAAGGTACCAAGAAACCTTCATT |
| OC43-Probe I.592 | AY391777.1 | OC43-Probe I | TGTGAGAGATTCCCTTCGTAATACGACTCACTATAGGGGTATTCTTTGTGTGAATGTTTGTTATGTTCTCATGTTGCTATTACTCGCTTCGAGCCAAGAAAAGTATTCTTTGTGTGAATGTTGAAGCATTGGAATATGAGTAATATGCTCTCGGGTGCCAAGGTACCAAGAAACCTTCATT |

84

| ASO ID | Accession | Group ID | Custom Array sequence |
|---|---|---|---|
| OC43-Probe I.593 | AY391777.1 | OC43-Probe I | TGTGAGAGATTCCCTTCGTAATACGACTCACTATAGGGTTACTCGAGCCAAGAAAGGTATTCTTTGTGTTATGAGTAATATGCAGTTGTTTGAAGCATTACAGTTTACTACATTGACCTTAGATAAAGTGGAATTCTCGGGTGCCAAGGTACCAAGAAACCTTCATT |
| OC43-Probe I.594 | AY391777.1 | OC43-Probe I | TGTGAGAGATTCCCTTCGTAATACGACTCACTATAGGGTTATGAGTAATATGCAGTTGTTTGAAGCATTACAGTTTACTACATTGACCTTAGATAAAGTGCCACAGGCCGTCGAAACTAAAGTTCAATTGGAATTCTCGGGTGCCAAGGTACCAAGAAACCTTCATT |
| OC43-Probe I.595 | AY391777.1 | OC43-Probe I | TGTGAGAGATTCCCTTCGTAATACGACTCACTATAGGGTACAGTTTACTACATTGACCTTAGATAAAGTGCCACAGGCCGTCGAAACTAAAGTTCAATGTAGTACTAATTTATTTAAAGATTGTAGCATGGAATTCTCGGGTGCCAAGGTACCAAGAAACCTTCATT |
| OC43-Probe I.596 | AY391777.1 | OC43-Probe I | TGTGAGAGATTCCCTTCGTAATACGACTCACTATAGGGTGCCACAGGCCGTCGAAACTAAAGTTCAATGTAGTACTAATTTATTTAAAGATTGTAGCAAGAGTTATAGCGGTTATCACCCAGCTCATGTGGAATTCTCGGGTGCCAAGGTACCAAGAAACCTTCATT |
| OC43-Probe I.597 | AY391777.1 | OC43-Probe I | TGTGAGAGATTCCCTTCGTAATACGACTCACTATAGGGTAGTACTAATTTATTTAAAGATTGTAGCAAGAGTTATAGCGGTTATCACCCAGCTCATGTCCTTCATTTTTGGCAGTAGATGACAAATTGGAATTCTCGGGTGCCAAGGTACCAAGAAACCTTCATT |
| OC43-Probe I.598 | AY391777.1 | OC43-Probe I | TGTGAGAGATTCCCTTCGTAATACGACTCACTATAGGGAGAGTTATAGCGGTTATCACCCAGCTCATGCTCCTTCATTTTTGGCAGTAGATGACAAATATAAGGCAACTGGCGATTTAGCCGTGTGTCTGGAATTCTCGGGTGCCAAGGTACCAAGAAACCTTCATT |
| OC43-Probe I.599 | AY391777.1 | OC43-Probe I | TGTGAGAGATTCCCTTCGTAATACGACTCACTATAGGGCTCCTTCATTTTTGGCAGTAGATGACAAATATAAGGCAACTGGCGATTTAGCCGTGTGTCTTGGTATTGGTGATTCTGCTGTTACATATTTGGAATTCTCGGGTGCCAAGGTACCAAGAAACCTTCATT |
| OC43-Probe I.600 | AY391777.1 | OC43-Probe I | TGTGAGAGATTCCCTTCGTAATACGACTCACTATAGGGGATAAGGCAACTGGCGATTTAGCCGTGTGTCTTGGTATTGGTGATTCTGCTGTTACATATTCAAGATTAATATCACTCATGGGTTTTAAATTGGAATTCTCGGGTGCCAAGGTACCAAGAAACCTTCATT |
| OC43-Probe I.601 | AY391777.1 | OC43-Probe I | TGTGAGAGATTCCCTTCGTAATACGACTCACTATAGGGTTGGTATTGGTGATTCTGCTGTTACATATTCAAGATTAATATCACTCATGGGTTTTAAATTGGATGTTACCCTTGATGGGTATTGTAAGCTGGAATTCTCGGGTGCCAAGGTACCAAGAAACCTTCATT |
| OC43-Probe I.602 | AY391777.1 | OC43-Probe I | TGTGAGAGATTCCCTTCGTAATACGACTCACTATAGGGCAAGATTAATATCACTCATGGGTTTTAAATTGGATGTTACCCTTGATGGGTATTGTAAGCTTTTTATAACTAAAGAAGAAGCTGTTAAACTGGAATTCTCGGGTGCCAAGGTACCAAGAAACCTTCATT |
| OC43-Probe I.603 | AY391777.1 | OC43-Probe I | TGTGAGAGATTCCCTTCGTAATACGACTCACTATAGGGTGGATGTTACCCTTGATGGGTATTGTAAGCTTTTTATAACTAAAGAAGAAGCTGTTAAACGCGTGCGTGCCTGGGTTGGCTTTGATGCTGTGGAATTCTCGGGTGCCAAGGTACCAAGAAACCTTCATT |
| OC43-Probe I.604 | AY391777.1 | OC43-Probe I | TGTGAGAGATTCCCTTCGTAATACGACTCACTATAGGGTTTTTATAACTAAAGAAGAAGCTGTTAAACGCGTGCGTGCCTGGGTTGGCTTTGATGCTGAAGGTGCTCATGCCACGCGTGATAGCATTGTGGAATTCTCGGGTGCCAAGGTACCAAGAAACCTTCATT |
| OC43-Probe I.605 | AY391777.1 | OC43-Probe I | TGTGAGAGATTCCCTTCGTAATACGACTCACTATAGGGGCGTGCGTGCCTGGGTTGGCTTTGATGCTGAAGGTGCTCATGCCACGCGTGATAGCATTGGGACAAATTTCCCACTTCAATTAGGATTTTTGGAATTCTCGGGTGCCAAGGTACCAAGAAACCTTCATT |
| OC43-Probe I.606 | AY391777.1 | OC43-Probe I | TGTGAGAGATTCCCTTCGTAATACGACTCACTATAGGGGAAGGTGCTCATGCCACGCGTGATAGCATTGGGACAAATTTCCCACTTCAATTAGGATTTTCCACAGGAATTGATTTTGTTGTGGAAGCCATGGAATTCTCGGGTGCCAAGGTACCAAGAAACCTTCATT |
| OC43-Probe I.607 | AY391777.1 | OC43-Probe I | TGTGAGAGATTCCCTTCGTAATACGACTCACTATAGGGGGACAAATTTCCCACTTCAATTAGGATTTTCCACAGGAATTGATTTTGTTGTGGAAGCCACTGGTTTGTTTGCTGATAGAGATGGTTACATGGAATTCTCGGGTGCCAAGGTACCAAGAAACCTTCATT |
| OC43-Probe I.608 | AY391777.1 | OC43-Probe I | TGTGAGAGATTCCCTTCGTAATACGACTCACTATAGGGCCACAGGAATTGATTTTGTTGTGGAAGCCACTGGTTTGTTTGCTGATAGAGATGGTTACAGCTTTAAAAAGGCTGTGGCGAAAGCTCCTCTGGAATTCTCGGGTGCCAAGGTACCAAGAAACCTTCATT |
| OC43-Probe I.609 | AY391777.1 | OC43-Probe I | TGTGAGAGATTCCCTTCGTAATACGACTCACTATAGGGCTGGTTTGTTTGCTGATAGAGATGGTTACAGCTTTAAAAAGGCTGTGGCGAAAGCTCCTCCTGGTGAACAATTTAAGCACCTCATCCCTTTGGAATTCTCGGGTGCCAAGGTACCAAGAAACCTTCATT |
| OC43-Probe I.610 | AY391777.1 | OC43-Probe I | TGTGAGAGATTCCCTTCGTAATACGACTCACTATAGGGGCTTTAAAAAGGCTGTGGCGAAAGCTCCTCCTGGTGAACAATTTAAGCACCTCATCCCTTTGATGACGAGAGGTCATCGCTGGGATGTTGTGGAATTCTCGGGTGCCAAGGTACCAAGAAACCTTCATT |
| OC43-Probe I.611 | AY391777.1 | OC43-Probe I | TGTGAGAGATTCCCTTCGTAATACGACTCACTATAGGGCTGGTGAACAATTTAAGCACCTCATCCCTTTGATGACGAGAGGTCATCGCTGGGATGTTGTTAGACCTAGAATAGTACAAATGTTTGCAGTGGAATTCTCGGGTGCCAAGGTACCAAGAAACCTTCATT |
| OC43-Probe I.612 | AY391777.1 | OC43-Probe I | TGTGAGAGATTCCCTTCGTAATACGACTCACTATAGGGTGATGACGAGAGGTCATCGCTGGGATGTTGTTAGACCTAGAATAGTACAAATGTTTGCAGTCATTTAATTGATCTGTCTGATTGTGTTGTGGAATTCTCGGGTGCCAAGGTACCAAGAAACCTTCATT |
| OC43-Probe I.613 | AY391777.1 | OC43-Probe I | TGTGAGAGATTCCCTTCGTAATACGACTCACTATAGGGTTAGACCTAGAATAGTACAAATGTTTGCAGATCATTTAATTGATCTGTCTGATTGTGTTGTGCTAGTTACATGGGCAGCCAACTTTGAGCTGGAATTCTCGGGTGCCAAGGTACCAAGAAACCTTCATT |
| OC43-Probe I.614 | AY391777.1 | OC43-Probe I | TGTGAGAGATTCCCTTCGTAATACGACTCACTATAGGGGATCATTTAATTGATCTGTCTGATTGTGTTGTGCTAGTTACATGGGCAGCCAACTTTGAGCTCACTTGTCTCCGCTACTTTGCAAAAGTAGTGGAATTCTCGGGTGCCAAGGTACCAAGAAACCTTCATT |
| OC43-Probe I.615 | AY391777.1 | OC43-Probe I | TGTGAGAGATTCCCTTCGTAATACGACTCACTATAGGGTGCTAGTTACATGGGCAGCCAACTTTGAGCTCACTTGTCTCCGCTACTTTGCAAAAGTAGGGCGTGAGATTTCTTGTAATGTATGCACTATGGAATTCTCGGGTGCCAAGGTACCAAGAAACCTTCATT |
| OC43-Probe I.616 | AY391777.1 | OC43-Probe I | TGTGAGAGATTCCCTTCGTAATACGACTCACTATAGGGTCACTTGTCTCCGCTACTTTGCAAAAGTAGGGCGTGAGATTTCTTGTAATGTATGCACTAAACGTGCCACAGTTTACAATTCTAGAACTGTGGAATTCTCGGGTGCCAAGGTACCAAGAAACCTTCATT |
| OC43-Probe I.617 | AY391777.1 | OC43-Probe I | TGTGAGAGATTCCCTTCGTAATACGACTCACTATAGGGGGCGTGAGATTTCTTGTAATGTATGCACTAAACGTGCCACAGTTTACAATTCTAGAACTGGTTACTATGGTTGTTGGCGCCATAGTGTTATGGAATTCTCGGGTGCCAAGGTACCAAGAAACCTTCATT |

| ASO ID | Accession | Group ID | Custom Array sequence |
|---|---|---|---|
| OC43-Probe I.618 | AY391777.1 | OC43-Probe I | TGTGAGAGATTCCCTTCGTAATACGACTCACTATAGGGAACGTGCCACAGTTTACAATTCTAGAACTGGTTACTATGGTTGTTGGCGCCATAGTGTTACATGTGATTACTTGTATAATCCACTTATTGTGGAATTCTCGGGTGCCAAGGTACCAAGAAACCTTCATT |
| OC43-Probe I.619 | AY391777.1 | OC43-Probe I | TGTGAGAGATTCCCTTCGTAATACGACTCACTATAGGGGTTACTATGGTTGTTGGCGCCATAGTGTTACATGTGATTACTTGTATAATCCACTTATTGTTGATATTCAACAGTGGGGATATATTGGTTTGGAATTCTCGGGTGCCAAGGTACCAAGAAACCTTCATT |
| OC43-Probe I.620 | AY391777.1 | OC43-Probe I | TGTGAGAGATTCCCTTCGTAATACGACTCACTATAGGGCATGTGATTACTTGTATAATCCACTTATTGTTGATATTCAACAGTGGGGATATATTGGTTCTTTATCAAGTAATCATGATTTATATTGTATGGAATTCTCGGGTGCCAAGGTACCAAGAAACCTTCATT |
| OC43-Probe I.621 | AY391777.1 | OC43-Probe I | TGTGAGAGATTCCCTTCGTAATACGACTCACTATAGGGGTTGATATTCAACAGTGGGGATATATTGGTTCTTTATCAAGTAATCATGATTTATATTGTAGTGTCCATAAAGGAGCACATGTTGCTTCCTTGGAATTCTCGGGTGCCAAGGTACCAAGAAACCTTCATT |
| OC43-Probe I.622 | AY391777.1 | OC43-Probe I | TGTGAGAGATTCCCTTCGTAATACGACTCACTATAGGGCTTTATCAAGTAATCATGATTTATATTGTAGTGTCCATAAAGGAGCACATGTTGCTTCCTCTGATGCTATAATGACACGGTGTTTGGCCGTGGAATTCTCGGGTGCCAAGGTACCAAGAAACCTTCATT |
| OC43-Probe I.623 | AY391777.1 | OC43-Probe I | TGTGAGAGATTCCCTTCGTAATACGACTCACTATAGGGGTGTCCATAAAGGAGCACATGTTGCTTCCTCTGATGCTATAATGACACGGTGTTTGGCCGTTTATGATTGCTTTTGCAATAATATTAATTTGGAATTCTCGGGTGCCAAGGTACCAAGAAACCTTCATT |
| OC43-Probe I.624 | AY391777.1 | OC43-Probe I | TGTGAGAGATTCCCTTCGTAATACGACTCACTATAGGGCTGATGCTATAATGACACGGTGTTTGGCCGTTTATGATTGCTTTTGCAATAATATTAATTTGGAATGTGGAGTATCCCATCATTTCAAATGTGGAATTCTCGGGTGCCAAGGTACCAAGAAACCTTCATT |
| OC43-Probe I.625 | AY391777.1 | OC43-Probe I | TGTGAGAGATTCCCTTCGTAATACGACTCACTATAGGGTTTATGATTGCTTTTGCAATAATATTAATTTGGAATGTGGAGTATCCCATCATTTCAAATGAGTTAAGTATTAATACCTCTTGTAGGGTCTTGGAATTCTCGGGTGCCAAGGTACCAAGAAACCTTCATT |
| OC43-Probe I.626 | AY391777.1 | OC43-Probe I | TGTGAGAGATTCCCTTCGTAATACGACTCACTATAGGGGGAATGTGGAGTATCCCATCATTTCAAATGAGTTAAGTATTAATACCTCTTGTAGGGTCTTGCAGCGTGTGATTCTTAAAGCTGCCATGCTGGAATTCTCGGGTGCCAAGGTACCAAGAAACCTTCATT |
| OC43-Probe I.627 | AY391777.1 | OC43-Probe I | TGTGAGAGATTCCCTTCGTAATACGACTCACTATAGGGAGTTAAGTATTAATACCTCTTGTAGGGTCTTGCAGCGTGTGATTCTTAAAGCTGCCATGCTCTGCAACAGATATACTTTGTGTTTATGATATGGAATTCTCGGGTGCCAAGGTACCAAGAAACCTTCATT |
| OC43-Probe I.628 | AY391777.1 | OC43-Probe I | TGTGAGAGATTCCCTTCGTAATACGACTCACTATAGGGTGCAGCGTGTGATTCTTAAAGCTGCCATGCTCTGCAACAGATATACTTTGTGTTTATGATATTGGCAACCCAAAAGCGATTGCCTGTGTCATGGAATTCTCGGGTGCCAAGGTACCAAGAAACCTTCATT |
| OC43-Probe I.629 | AY391777.1 | OC43-Probe I | TGTGAGAGATTCCCTTCGTAATACGACTCACTATAGGGTCTGCAACAGATATACTTTGTGTTATGATATTGGCAACCCAAAAGCGATTGCCTGTGTCAAAGATTTTGATTTTAAGTTCTATGATGCCCTGGAATTCTCGGGTGCCAAGGTACCAAGAAACCTTCATT |
| OC43-Probe I.630 | AY391777.1 | OC43-Probe I | TGTGAGAGATTCCCTTCGTAATACGACTCACTATAGGGTTGGCAACCCAAAAGCGATTGCCTGTGTCAAAGATTTTGATTTTAAGTTCTATGATGCCCAACCAATTGTTAAGTCTGTTAAGACTCTTTTGGAATTCTCGGGTGCCAAGGTACCAAGAAACCTTCATT |
| OC43-Probe I.631 | AY391777.1 | OC43-Probe I | TGTGAGAGATTCCCTTCGTAATACGACTCACTATAGGGAAGATTTGATTTTAAGTTCTATGATGCCCAACCAATTGTTAAGTCTGTTAAGACTCTTTTGTATTCTTTTGAGGCACATAAGGACTCTTTGGAATTCTCGGGTGCCAAGGTACCAAGAAACCTTCATT |
| OC43-Probe I.632 | AY391777.1 | OC43-Probe I | TGTGAGAGATTCCCTTCGTAATACGACTCACTATAGGGAACCAATTGTTAAGTCTGTTAAGACTCTTTTGTATTCTTTTGAGGCACATAAGGACTCTTTAAAGACGGTTTGTGTATGTTTTGGAACTGGAATTCTCGGGTGCCAAGGTACCAAGAAACCTTCATT |
| OC43-Probe I.633 | AY391777.1 | OC43-Probe I | TGTGAGAGATTCCCTTCGTAATACGACTCACTATAGGGTGTATTCTTTTGAGGCACATAAGGACTCTTTTAAAGACGGTTTGTGTATGTTTTGGAACTGTAATGTGGATAAGTATCCACCGAATGCAGTGGAATTCTCGGGTGCCAAGGTACCAAGAAACCTTCATT |
| OC43-Probe I.634 | AY391777.1 | OC43-Probe I | TGTGAGAGATTCCCTTCGTAATACGACTCACTATAGGGTTAAAGACGGTTTGTGTATGTTTTGGAACTGTAATGTGGATAAGTATCCACCGAATGCAGTTGTATGTAGATTTGACACTAGAGTGTTGATGGAATTCTCGGGTGCCAAGGTACCAAGAAACCTTCATT |
| OC43-Probe I.635 | AY391777.1 | OC43-Probe I | TGTGAGAGATTCCCTTCGTAATACGACTCACTATAGGGGTAATGTGGATAAGTATCCACCGAATGCAGTTGTATGTAGATTTGACACTAGAGTGTTGAATAATTTAAATCTTCCTGGCTGTAATGGAGTGGAATTCTCGGGTGCCAAGGTACCAAGAAACCTTCATT |
| OC43-Probe I.636 | AY391777.1 | OC43-Probe I | TGTGAGAGATTCCCTTCGTAATACGACTCACTATAGGGGTTGTATGTAGATTTGACACTAGAGTGTTGAATAATTTAAATCTTCCTGGCTGTAATGGAGTAGTTTGTATGTTAATAAACATGCATTCCTGGAATTCTCGGGTGCCAAGGTACCAAGAAACCTTCATT |
| OC43-Probe I.637 | AY391777.1 | OC43-Probe I | TGTGAGAGATTCCCTTCGTAATACGACTCACTATAGGGATAATTTAAATCTTCCTGGCTGTAATGGAGTAGTTTGTATGTTAATAAACATGCATTCCACACTAAACCCTTTGCTAGGGCAGCCTTTGTGGAATTCTCGGGTGCCAAGGTACCAAGAAACCTTCATT |
| OC43-Probe I.638 | AY391777.1 | OC43-Probe I | TGTGAGAGATTCCCTTCGTAATACGACTCACTATAGGGGTAGTTTGTATGTTAATAAACATGCATTCCACACTAAACCCTTTGCTAGGGCAGCCTTTGAGCATTTGAAGCCTATGCCATTCTTCTATTTGGAATTCTCGGGTGCCAAGGTACCAAGAAACCTTCATT |
| OC43-Probe I.639 | AY391777.1 | OC43-Probe I | TGTGAGAGATTCCCTTCGTAATACGACTCACTATAGGGACACTAAACCCTTTGCTAGGGCAGCCTTTGAGCATTTGAAGCCTATGCCATTCTTCTATTATTCAGATACGCCTTGTGTGTATATGGATGTGGAATTCTCGGGTGCCAAGGTACCAAGAAACCTTCATT |
| OC43-Probe I.640 | AY391777.1 | OC43-Probe I | TGTGAGAGATTCCCTTCGTAATACGACTCACTATAGGGAGCATTTGAAGCCTATGCCATTCTTCTATTATTCAGATACGCCTTGTGTGTATATGGATGGCATGGATGCTAAGCCAGGTTGATTATGTACTGGAATTCTCGGGTGCCAAGGTACCAAGAAACCTTCATT |
| OC43-Probe I.641 | AY391777.1 | OC43-Probe I | TGTGAGAGATTCCCTTCGTAATACGACTCACTATAGGGATTCAGATACGCCTTGTGTGTATATGGATGGCATGGATGCTAAGCCAGGTTGATTATGTACCTTTGAAATCTGCCACGTGCATCACAAGATTGGAATTCTCGGGTGCCAAGGTACCAAGAAACCTTCATT |
| OC43-Probe I.642 | AY391777.1 | OC43-Probe I | TGTGAGAGATTCCCTTCGTAATACGACTCACTATAGGGGCATGGATGCTAAGCAGGTTGATTATGTACCTTTGAAATCTGCCACGTGCATCACAAGATGCAATTTAGGTGGTGCAGTTTGTTTAAAACTGGAATTCTCGGGTGCCAAGGTACCAAGAAACCTTCATT |

86

| ASO ID | Accession | Group ID | Custom Array sequence |
|---|---|---|---|
| OC43-Probe I.643 | AY391777.1 | OC43-Probe I | TGTGAGAGATTCCCTTCGTAATACGACTCACTATAGGGCTTTGAAATCTGCCACGTGCATCACAAGATGCAATTTAGGTGGTGCAGTTTGTTTAAAACATGCTGAAGAGTATCGTGAGTACTTAGAGTTGGAATTCTCGGGTGCCAAGGTACCAAGAAACCTTCATT |
| OC43-Probe I.644 | AY391777.1 | OC43-Probe I | TGTGAGAGATTCCCTTCGTAATACGACTCACTATAGGGGCAATTTAGGTGGTGCAGTTTGTTTAAAACATGCTGAAGAGTATCGTGAGTACTTAGAGTCTTACAATACAGCTACTACAGCAGGTTTTATGGAATTCTCGGGTGCCAAGGTACCAAGAAACCTTCATT |
| OC43-Probe I.645 | AY391777.1 | OC43-Probe I | TGTGAGAGATTCCCTTCGTAATACGACTCACTATAGGGATGCTGAAGAGTATCGTGAGTACTTAGAGTCTTACAATACAGCTACTACAGCAGGTTTTACTTTTTGGGTCTATAAGACATTTGATTTTTTGGAATTCTCGGGTGCCAAGGTACCAAGAAACCTTCATT |
| OC43-Probe I.646 | AY391777.1 | OC43-Probe I | TGTGAGAGATTCCCTTCGTAATACGACTCACTATAGGGCTTACAATACAGCTACTACAGCAGGTTTTACTTTTTGGGTCTATAAGACATTTGATTTTTATAATTTGTGGAATACGTTCACCAAGCTACTGGAATTCTCGGGTGCCAAGGTACCAAGAAACCTTCATT |
| OC43-Probe I.647 | AY391777.1 | OC43-Probe I | TGTGAGAGATTCCCTTCGTAATACGACTCACTATAGGGCTTTTTGGGTCTATAAGACATTTGATTTTTATAATTTGTGGAATACGTTCACCAAGCTACAAAGCTTGGAGAATGTTGTATATAATTTAGTGGAATTCTCGGGTGCCAAGGTACCAAGAAACCTTCATT |
| OC43-Probe I.648 | AY391777.1 | OC43-Probe I | TGTGAGAGATTCCCTTCGTAATACGACTCACTATAGGGATAATTTGTGGAATACGTTCACCAAGCTACAAAGCTTGGAGAATGTTGTATATAATTTAGTCAAGACTGGTCATTATACAGGACAGGCTGTGGAATTCTCGGGTGCCAAGGTACCAAGAAACCTTCATT |
| OC43-Probe I.649 | AY391777.1 | OC43-Probe I | TGTGAGAGATTCCCTTCGTAATACGACTCACTATAGGGAAAGCTTGGAGAATGTTGTATATAATTTAGTCAAGACTGGTCATTATACAGGACAGGCTGGTGAAATGCCTTGTGCCATTATAAATGATATGGAATTCTCGGGTGCCAAGGTACCAAGAAACCTTCATT |
| OC43-Probe I.650 | AY391777.1 | OC43-Probe I | TGTGAGAGATTCCCTTCGTAATACGACTCACTATAGGGTCAAGACTGGTCATTATACAGGACAGGCTGGTGAAATGCCTTGTGCCATTATAAATGATAAAGTTGTGGCTAAGATCGATAAGGAGGATGTGGAATTCTCGGGTGCCAAGGTACCAAGAAACCTTCATT |
| OC43-Probe I.651 | AY391777.1 | OC43-Probe I | TGTGAGAGATTCCCTTCGTAATACGACTCACTATAGGGGTGAAATGCCTTGTGCCATTATAAATGATAAAGTTGTGGCTAAGATCGATAAGGAGGATGTTGTCATTTTTATTAATAATACAACATACCTGGAATTCTCGGGTGCCAAGGTACCAAGAAACCTTCATT |
| OC43-Probe I.652 | AY391777.1 | OC43-Probe I | TGTGAGAGATTCCCTTCGTAATACGACTCACTATAGGGAAGTTGTGGCTAAGATCGATAAGGAGGATGTTGTCATTTTTATTAATAATACAACATACCCTACTAATGTGGCCGTTGAATTATTTGCCATGGAATTCTCGGGTGCCAAGGTACCAAGAAACCTTCATT |
| OC43-Probe I.653 | AY391777.1 | OC43-Probe I | TGTGAGAGATTCCCTTCGTAATACGACTCACTATAGGGTTGTCATTTTTATTAATAATACAACATACCCTACTAATGTGGCCGTTGAATTATTTGCCAAGCGCAGTGTTCGACACCACCCAGAGCTTATGGAATTCTCGGGTGCCAAGGTACCAAGAAACCTTCATT |
| OC43-Probe I.654 | AY391777.1 | OC43-Probe I | TGTGAGAGATTCCCTTCGTAATACGACTCACTATAGGGCTACTAATGTGGCCGTTGAATTATTTGCCAAGCGCAGTGTTCGACACCACCCAGAGCTTAAGCTCTTTAGAAATTTAAATATAGACGTGTTGGAATTCTCGGGTGCCAAGGTACCAAGAAACCTTCATT |
| OC43-Probe I.655 | AY391777.1 | OC43-Probe I | TGTGAGAGATTCCCTTCGTAATACGACTCACTATAGGGGAGCGCAGTGTTCGACACCACCCAGAGCTTAAGCTCTTTAGAAATTTAAATATAGACGTGTGTTGGAAGCACGTCATTTGGGATTGCTATGGAATTCTCGGGTGCCAAGGTACCAAGAAACCTTCATT |
| OC43-Probe I.656 | AY391777.1 | OC43-Probe I | TGTGAGAGATTCCCTTCGTAATACGACTCACTATAGGGGAGCTCTTTAGAAATTTAAATATAGACGTGTGTTGGAAGCACGTCATTTGGGATTATGCTAGAGAAAGTATATTTTGCAGTAATACCTATGTGGAATTCTCGGGTGCCAAGGTACCAAGAAACCTTCATT |
| OC43-Probe I.657 | AY391777.1 | OC43-Probe I | TGTGAGAGATTCCCTTCGTAATACGACTCACTATAGGGGGTTGGAAGCACGTCATTTGGGATTATGCTAGAGAAAGTATATTTTGCAGTAATACCTATGTGTCTGCATGTATACAGATTTAAAGTTCATGGAATTCTCGGGTGCCAAGGTACCAAGAAACCTTCATT |
| OC43-Probe I.658 | AY391777.1 | OC43-Probe I | TGTGAGAGATTCCCTTCGTAATACGACTCACTATAGGGGAGAAAGTATATTTTGCAGTAATACCTATGTGTCTGCATGTATACAGATTTAAAGTTCATTGATAAATTGAATGTCCTTTTTGATGGTCTGGAATTCTCGGGTGCCAAGGTACCAAGAAACCTTCATT |
| OC43-Probe I.659 | AY391777.1 | OC43-Probe I | TGTGAGAGATTCCCTTCGTAATACGACTCACTATAGGGGTGTCTGCATGTATACAGATTTAAAGTTCATTGATAAATTGAATGTCCTTTTTGATGGTCGTGATAATGGTGCTCTTGAAGCTTTTAAACTGGAATTCTCGGGTGCCAAGGTACCAAGAAACCTTCATT |
| OC43-Probe I.660 | AY391777.1 | OC43-Probe I | TGTGAGAGATTCCCTTCGTAATACGACTCACTATAGGGTTGATAAATTGAATGTCCTTTTTGATGGTCGTGATAATGGTGCTCTTGAAGCTTTTAAACGTTCTAATAATGGCGTTTACATTTCCACGATGGAATTCTCGGGTGCCAAGGTACCAAGAAACCTTCATT |
| OC43-Probe I.661 | AY391777.1 | OC43-Probe I | TGTGAGAGATTCCCTTCGTAATACGACTCACTATAGGGGTGATAATGGTGCTCTTGAAGCTTTTAAACGTTCTAATAATGGCGTTTACATTTCCACGACAAAAGTTAAGAGTCTTTCGATGATAAGAGTGGAATTCTCGGGTGCCAAGGTACCAAGAAACCTTCATT |
| OC43-Probe I.662 | AY391777.1 | OC43-Probe I | TGTGAGAGATTCCCTTCGTAATACGACTCACTATAGGGGTTCTAATAATGGCGTTTACATTTCCACGACAAAAGTTAAGAGTCTTTCGATGATAAGAGGTCCACCGCGTGCTGAATTAAATGGCGTAGTGGAATTCTCGGGTGCCAAGGTACCAAGAAACCTTCATT |
| OC43-Probe I.663 | AY391777.1 | OC43-Probe I | TGTGAGAGATTCCCTTCGTAATACGACTCACTATAGGGCAAAAGTTAAGAGTCTTTCGATGATAAGAGGTCCACCGCGTGCTGAATTAAATGGCGTAGTGGTGGACAAGGTTGGAGACACTGATTGTGTGGAATTCTCGGGTGCCAAGGTACCAAGAAACCTTCATT |
| OC43-Probe I.664 | AY391777.1 | OC43-Probe I | TGTGAGAGATTCCCTTCGTAATACGACTCACTATAGGGGTCCACCGCGTGCTGAATTAAATGGCGTAGTGGTGGACAAGGTTGGAGACACTGATTGTGTGTTTTATTTTGCTGTGCGTAAAGAAAGGTCTGGAATTCTCGGGTGCCAAGGTACCAAGAAACCTTCATT |
| OC43-Probe I.665 | AY391777.1 | OC43-Probe I | TGTGAGAGATTCCCTTCGTAATACGACTCACTATAGGGGTGGTGGACAAGGTTGGAGACACTGATTGTGTGTTTTATTTTGCTGTGCGTAAAGAAAGGTCAGGATGTCATCTTCAGCCAATTCGACAGCCTGGAATTCTCGGGTGCCAAGGTACCAAGAAACCTTCATT |
| OC43-Probe I.666 | AY391777.1 | OC43-Probe I | TGTGAGAGATTCCCTTCGTAATACGACTCACTATAGGGTGTTTTATTTTGCTGTGCGTAAAGAAAGGTCAGGATGTCATCTTCAGCCAATTCGACAGCCTGGGAGTCAGCTCTAACCAGAGCCCACAAGGTGGAATTCTCGGGTGCCAAGGTACCAAGAAACCTTCATT |
| OC43-Probe I.667 | AY391777.1 | OC43-Probe I | TGTGAGAGATTCCCTTCGTAATACGACTCACTATAGGGAGGATGTCATCTTCAGCCAATTCGACAGCCTGGGAGTCAGCTCTAACCAGAGCCCACAAGGTAATCTGGGGAGTAATGGTAAACCCGGTATGGAATTCTCGGGTGCCAAGGTACCAAGAAACCTTCATT |

| ASO ID | Accession | Group ID | Custom Array sequence |
|---|---|---|---|
| OC43-Probe I.668 | AY391777.1 | OC43-Probe I | TGTGAGAGATTCCCTTCGTAATACGACTCACTATAGGGTGGGAGTCAGCTCTAACCAGAGCCCACAAGGTAATCTGGGGAGTAATGGTAAACCCGGTAATGTCGGTGGTAATGATGCTCTGTCAATCTTGGAATTCTCGGGTGCCAAGGTACCAAGAAACCTTCATT |
| OC43-Probe I.669 | AY391777.1 | OC43-Probe I | TGTGAGAGATTCCCTTCGTAATACGACTCACTATAGGGGTAATCTGGGGAGTAATGGTAAACCCGGTAATGTCGGTGGTAATGATGCTCTGTCAATCTCTACTATCTTTACACAAAGCCGTGTTATTATGGAATTCTCGGGTGCCAAGGTACCAAGAAACCTTCATT |
| OC43-Probe I.670 | AY391777.1 | OC43-Probe I | TGTGAGAGATTCCCTTCGTAATACGACTCACTATAGGGATGTCGGTGGTAATGATGCTCTGTCAATCTCTACTATCTTTACACAAAGCCGTGTTATTAGCTCTTTTACATGTCGTACTGATATGGAAATGGAATTCTCGGGTGCCAAGGTACCAAGAAACCTTCATT |
| OC43-Probe I.671 | AY391777.1 | OC43-Probe I | TGTGAGAGATTCCCTTCGTAATACGACTCACTATAGGGCTACTATCTTTACACAAAGCCGTGTTATTAGCTCTTTTACATGTCGTACTGATATGGAAAAAGATTTTATAGCTTTAGATCAAGATGTGTTGGAATTCTCGGGTGCCAAGGTACCAAGAAACCTTCATT |
| OC43-Probe I.672 | AY391777.1 | OC43-Probe I | TGTGAGAGATTCCCTTCGTAATACGACTCACTATAGGGGCTCTTTTACATGTCGTACTGATATGGAAAAAGATTTTATAGCTTTAGATCAAGATGTGTTATTCAGAAGTATGGTTTGGAGGACTATGTGGAATTCTCGGGTGCCAAGGTACCAAGAAACCTTCATT |
| OC43-Probe I.673 | AY391777.1 | OC43-Probe I | TGTGAGAGATTCCCTTCGTAATACGACTCACTATAGGGAAGATTTTATAGCTTTAGATCAAGATGTGTTTATTCAGAAGTATGGTTTGGAGGACTATGCCTTTGAACACATTGTTTATGGTAACTTCATGGAATTCTCGGGTGCCAAGGTACCAAGAAACCTTCATT |
| OC43-Probe I.674 | AY391777.1 | OC43-Probe I | TGTGAGAGATTCCCTTCGTAATACGACTCACTATAGGGTTATTCAGAAGTATGGTTTGGAGGACTATGCCTTTGAACACATTGTTTATGGTAACTTCAACCAGAAGATTATTGGTGGTTTGCATTTGTTGGAATTCTCGGGTGCCAAGGTACCAAGAAACCTTCATT |
| OC43-Probe I.675 | AY391777.1 | OC43-Probe I | TGTGAGAGATTCCCTTCGTAATACGACTCACTATAGGGCCTTTGAACACATTGTTTATGGTAACTTCAACCAGAAGATTATTGGTGGTTTGCATTTGTTAATAGGCTTGTACCGAAGACAGCAAACTTTGGAATTCTCGGGTGCCAAGGTACCAAGAAACCTTCATT |
| OC43-Probe I.676 | AY391777.1 | OC43-Probe I | TGTGAGAGATTCCCTTCGTAATACGACTCACTATAGGGACCAGAAGATTATTGGTGGTTTGCATTTGTTAATAGGCTTGTACCGAAGACAGCAAACTTCCAATCTGGTTGTTCAGGAGTTTGTTTCATTGGAATTCTCGGGTGCCAAGGTACCAAGAAACCTTCATT |
| OC43-Probe I.677 | AY391777.1 | OC43-Probe I | TGTGAGAGATTCCCTTCGTAATACGACTCACTATAGGGTAATAGGCTTGTACCGAAGACAGCAAACTTCCAATCTGGTTGTTCAGGAGTTTGTTTCATATGACTCCAGCATACACTCTTATTTTATCATGGAATTCTCGGGTGCCAAGGTACCAAGAAACCTTCATT |
| OC43-Probe I.678 | AY391777.1 | OC43-Probe I | TGTGAGAGATTCCCTTCGTAATACGACTCACTATAGGGCCAATCTGGTTGTTCAGGAGTTTGTTTCATATGACTCCAGCATACACTCTTATTTTATCACTGACGAGAAGAGTGGTGGTAGTAAGAGTGTGGAATTCTCGGGTGCCAAGGTACCAAGAAACCTTCATT |
| OC43-Probe I.679 | AY391777.1 | OC43-Probe I | TGTGAGAGATTCCCTTCGTAATACGACTCACTATAGGGATGACTCCAGCATACACTCTTATTTTATCACTGACGAGAAGAGTGGTGGTAGTAAGAGTGTTTGCACTGTTATAGATATTTTGTTGGATGTGGAATTCTCGGGTGCCAAGGTACCAAGAAACCTTCATT |
| OC43-Probe I.680 | AY391777.1 | OC43-Probe I | TGTGAGAGATTCCCTTCGTAATACGACTCACTATAGGGCTGACGAGAAGAGTGGTGGTAGTAAGAGTGTTTGCACTGTTATAGATATTTTGTTGGATGATTTTGTGGCTCTTGTTAAGTCACTTAATCTGGAATTCTCGGGTGCCAAGGTACCAAGAAACCTTCATT |
| OC43-Probe I.681 | AY391777.1 | OC43-Probe I | TGTGAGAGATTCCCTTCGTAATACGACTCACTATAGGGTTTGCACTGTTATAGATATTTTGTTGGATGATTTTGTGGCTCTTGTTAAGTCACTTAATCTTAATTGTGTGAGTAAGGTTGTTAATGTTATGGAATTCTCGGGTGCCAAGGTACCAAGAAACCTTCATT |
| OC43-Probe I.682 | AY391777.1 | OC43-Probe I | TGTGAGAGATTCCCTTCGTAATACGACTCACTATAGGGATTTTGTGGCTCTTGTTAAGTCACTTAATCTTAATTGTGTGAGTAAGGTTGTTAATGTTAATGTTGATTTTAAAGATTTTCAGTTTATGTGTGGAATTCTCGGGTGCCAAGGTACCAAGAAACCTTCATT |
| OC43-Probe I.683 | AY391777.1 | OC43-Probe I | TGTGAGAGATTCCCTTCGTAATACGACTCACTATAGGGTTAATTGTGTGAGTAAGGTTGTTAATGTTAATGTTGATTTTAAAGATTTTCAGTTTATGTGTTGGTGTAACGATGAGAAAGTTATGACTTTGGAATTCTCGGGTGCCAAGGTACCAAGAAACCTTCATT |
| OC43-Probe I.684 | AY391777.1 | OC43-Probe I | TGTGAGAGATTCCCTTCGTAATACGACTCACTATAGGGATGTTGATTTTAAAGATTTTCAGTTTATGTGTTGGTGTAACGATGAGAAAGTTATGACTTTCTATCCTCGTTTGCAAGCTGCATCTGACTTGGAATTCTCGGGTGCCAAGGTACCAAGAAACCTTCATT |
| OC43-Probe I.685 | AY391777.1 | OC43-Probe I | TGTGAGAGATTCCCTTCGTAATACGACTCACTATAGGGTTTGGTGTAACGATGAGAAAGTTATGACTTTCTATCCTCGTTTGCAAGCTGCATCTGACTGGAAGCCTGGTTATTCTATGCCTGTATTATTGGAATTCTCGGGTGCCAAGGTACCAAGAAACCTTCATT |
| OC43-Probe I.686 | AY391777.1 | OC43-Probe I | TGTGAGAGATTCCCTTCGTAATACGACTCACTATAGGGTCTATCCTCGTTTGCAAGCTGCATCTGACTGGAAGCCTGGTTATTCTATGCCTGTATTATATAAGTATTTGAATTCTCCAATGGAAAGAGTGGAATTCTCGGGTGCCAAGGTACCAAGAAACCTTCATT |
| OC43-Probe I.687 | AY391777.1 | OC43-Probe I | TGTGAGAGATTCCCTTCGTAATACGACTCACTATAGGGGGAAGCCTGGTTATTCTATGCCTGTATTATATAAGTATTTGAATTCTCCAATGGAAAGAGTTAGTCTCTGGAATTATGGGAAGCCAGTTATGGAATTCTCGGGTGCCAAGGTACCAAGAAACCTTCATT |
| OC43-Probe I.688 | AY391777.1 | OC43-Probe I | TGTGAGAGATTCCCTTCGTAATACGACTCACTATAGGGGATAAGTATTTGAATTCTCCAATGGAAAGAGTTAGTCTCTGGAATTATGGGAAGCCAGTTACTTTGCCTACAGGCTGTATGATGAATGTTGTGGAATTCTCGGGTGCCAAGGTACCAAGAAACCTTCATT |
| OC43-Probe I.689 | AY391777.1 | OC43-Probe I | TGTGAGAGATTCCCTTCGTAATACGACTCACTATAGGGTTAGTCTCTGGAATTATGGGAAGCCAGTTACTTTGCCTACAGGCTGTATGATGAATGTTGCTAAGTATACTCAGTTATGTCAATATCTGATGGAATTCTCGGGTGCCAAGGTACCAAGAAACCTTCATT |
| OC43-Probe I.690 | AY391777.1 | OC43-Probe I | TGTGAGAGATTCCCTTCGTAATACGACTCACTATAGGGCTTTGCCTACAGGCTGTATGATGAATGTTGCTAAGTATACTCAGTTATGTCAATATCTGAATACTACAACATTAGCTGTACCTGTTAATATGGAATTCTCGGGTGCCAAGGTACCAAGAAACCTTCATT |
| OC43-Probe I.691 | AY391777.1 | OC43-Probe I | TGTGAGAGATTCCCTTCGTAATACGACTCACTATAGGGCTAAGTATACTCAGTTATGTCAATATCTGAATACTACAACATTAGCTGTACCTGTTAATATGCGAGTTTTGCATTTAGGTGCAGGTTCAGTGGAATTCTCGGGTGCCAAGGTACCAAGAAACCTTCATT |
| OC43-Probe I.692 | AY391777.1 | OC43-Probe I | TGTGAGAGATTCCCTTCGTAATACGACTCACTATAGGGGATACTACAACATTAGCTGTACCTGTTAATATGCGAGTTTTGCATTTAGGTGCAGGTTCAGAAAAAAGGAGTAGCACCGGGTTCTGCAGTTCTGGAATTCTCGGGTGCCAAGGTACCAAGAAACCTTCATT |

| ASO ID | Accession | Group ID | Custom Array sequence |
|---|---|---|---|
| OC43-Probe I.693 | AY391777.1 | OC43-Probe I | TGTGAGAGATTCCCTTCGTAATACGACTCACTATAGGGTTGCCAGTTGTCCGCTGGTTCTGCAGTTCTTAGGCGAGTAGCACCGGGTTCTGCGACTACTGGTGCCTGCTGGTCTATTCTATTCTGTAGCAGTAGTGGTTCTGGGTGCCAAGGTACCAAGAAACCTTCATT |
| OC43-Probe I.694 | AY391777.1 | OC43-Probe I | TGTGAGAGATTCCCTTCGTAATACGACTCACTATAGGGTTGCCAGTTGTCCGCTGGTTCTGCAGTTCTTAGGCGAGTAGCACCGGGTTCTGCGACTACTGGTGCCTGCTGGTCTATTCTATTCTGTAGATAACGATTTATGTATGGAATTCTCGGGTGCCAAGGTACCAAGAAACCTTCATT |
| OC43-Probe I.695 | AY391777.1 | OC43-Probe I | TGTGAGAGATTCCCTTCGTAATACGACTCACTATAGGGTTAGGCGAGTAGCACCGGGTTGCCTGCTGGTACTATTCTGTAGATAATCAACGATTTATACCCCATATTTGGGATTGGAATTCTCGGGTGCCAAGGTACCAAGAAACCTTCATT |
| OC43-Probe I.696 | AY391777.1 | OC43-Probe I | TGTGAGAGATTCCCTTCGTAATACGACTCACTATAGGGTGTAGATAACGATTTATACCCATATTTGTTAGTGACAGTGTCGCTACATATTTGATTGTCAATGGTATAACTTTACCCTTTGATATTGTCAATGGCAGTTCTCGGGTGCCAAGGTACCAAGAAACCTTCATT |
| OC43-Probe I.697 | AY391777.1 | OC43-Probe I | TGTGAGAGATTCCCTTCGTAATACGACTCACTATAGGGTGTAGATAATCAACGATTTATAACTTTACCCTTTGATATTGTCAATGGTATATTGATATGTATGGAGATTGTCAATGGCAGTTCTCGGGTGCCAAGGTACCAAGAAACCTTCATT |
| OC43-Probe I.698 | AY391777.1 | OC43-Probe I | TGTGAGAGATTCCCTTCGTAATACGACTCACTATAGGGTCGCTACATATTTCACCCTTTGATATTGTCAATGGTATAATTCTCTGATATGTATGGAGATTGATAATTACTAAGAACATAGGGAGTAGTCTCGGGTGCCAAGGTACCAAGAAACCTTCATT |
| OC43-Probe I.699 | AY391777.1 | OC43-Probe I | TGTGAGAGATTCCCTTCGTAATACGACTCACTATAGGGTGATATGTATGGAGATTGATAATTACTAAGAACATAGGGAGTAGTCTATTACTTTACACATTTCTTTACACTTACATATGATCGTCTCGGGTGCCAAGGTACCAAGAAACCTTCATT |
| OC43-Probe I.700 | AY391777.1 | OC43-Probe I | TGTGAGAGATTCCCTTCGTAATACGACTCACTATAGGGTGATAATTACTAAGAACATAGGGAGTAGTCTATTACTTTACACATTGTCATATGATCGAGACAAGTTAGCTCTGGGTGTGGCAGTGTCTCGGGTGCCAAGGTACCAAGAAACCTTCATT |
| OC43-Probe I.701 | AY391777.1 | OC43-Probe I | TGTGAGAGATTCCCTTCGTAATACGACTCACTATAGGGTGTATTACTTTACACATTGTCATATGATCGAGACAAGTTAGCTCTGGGTGTGGCAGTTTTCTTGGAATTCTCTATAAAAATAACAGAGTTTCTCGGGTGCCAAGGTACCAAGAAACCTTCATT |
| OC43-Probe I.702 | AY391777.1 | OC43-Probe I | TGTGAGAGATTCCCTTCGTAATACGACTCACTATAGGGTGCTCTGGGTGTGGCAGTTTTCTTGGAATTCTCTATAAAAATAACAGAGTTTATAAGTTAATGGTGGTGGAATTCTCGGGATGCGAGTTTCTCGGGTGCCAAGGTACCAAGAAACCTTCATT |
| OC43-Probe I.703 | AY391777.1 | OC43-Probe I | TGTGAGAGATTCCCTTCGTAATACGACTCACTATAGGGTGCTATAAAAATAACAGAGTTTATAAGTTAATGGTGGTGGAATTCTCGGGATGTGTTTTGCATTTGCAAAATGCAAATGCTCTCGGGTGCCAAGGTACCAAGAAACCTTCATT |
| OC43-Probe I.704 | AY391777.1 | OC43-Probe I | TGTGAGAGATTCCCTTCGTAATACGACTCACTATAGGGTATTAATGGGATATTTTGCATTTGCAAAATGCAAATGCTTGGGACTGTGTTTTGCATTTGGGACTGTTTTGCATTTGGACTGTGTTTTCTCGGGTGCCAAGGTACCAAGAAACCTTCATT |
| OC43-Probe I.705 | AY391777.1 | OC43-Probe I | TGTGAGAGATTCCCTTCGTAATACGACTCACTATAGGGTATTTTGCATTTGGGACTGTGTTTTGCACAAATGCAAATGCTTAATTGGCATAAATATTATTGGCATAAATATTATTGTGTAGCTCTCGGGTGCCAAGGTACCAAGAAACCTTCATT |
| OC43-Probe I.706 | AY391777.1 | OC43-Probe I | TGTGAGAGATTCCCTTCGTAATACGACTCACTATAGGGTGTAGCAAATGCAAATGCTTAATTGGCATAAATATTATTGTGTAGCTCTCGTCTCTAGTGAAGGATTTTAATTGGCATATATTGTGTAAGCTGAGAATGTTATGGCATATCCATGGAAATGTCTCGGGTGCCAAGGTACCAAGAAACCTTCATT |
| OC43-Probe I.707 | AY391777.1 | OC43-Probe I | TGTGAGAGATTCCCTTCGTAATACGACTCACTATAGGGTTTAATTGGCATATATTGTGTAAGCTGAGAATGTTATGGCATATCCATGGAAATGTTGAGATAGTAGATAGTGTTTGGGAGAAATTCTCGGGTGCCAAGGTACCAAGAAACCTTCATT |
| OC43-Probe I.708 | AY391777.1 | OC43-Probe I | TGTGAGAGATTCCCTTCGTAATACGACTCACTATAGGGTGAGAATGTTATGGCATATCCATGGAAATGTTGAGATAGTAGATAGTGTTTGGGAGAACGGGGGTGCTTATAGCCTGTTGGAATTCACAGTTTCTCGGGTGCCAAGGTACCAAGAAACCTTCATT |
| OC43-Probe I.709 | AY391777.1 | OC43-Probe I | TGTGAGAGATTCCCTTCGTAATACGACTCACTATAGGGTGATGGAATTCCACAGTTTGTTTGATATGGTACGGGGGTGCTTATAGCCTCGTTTGATATGGTTAAATTCCCGCCTAAGTTCGGGTGCCAAGGTACCAAGAAACCTTCATT |
| OC43-Probe I.710 | AY391777.1 | OC43-Probe I | TGTGAGAGATTCCCTTCGTAATACGACTCACTATAGGGTGCAGTTTGAAATCCCGCCTAAATTCCCGCCTAAGTTATGTGATATGGTTAAATTCCCGCCTAAGTTGTACTGCCGTAATAATTTAAGAGCAGTGAACTCTCGGGTGCCAAGGTACCAAGAAACCTTCATT |
| OC43-Probe I.711 | AY391777.1 | OC43-Probe I | TGTGAGAGATTCCCTTCGTAATACGACTCACTATAGGGTTGGAATGGCTAAATTCCGCGTAAGTTGCTGGCTGGTACTGCCGTAATAATTTAAGAGCAGCAGACCAGATTAATGATCATGTTTATTCCCTTCTCGGGTGCCAAGGTACCAAGAAACCTTCATT |
| OC43-Probe I.712 | AY391777.1 | OC43-Probe I | TGTGAGAGATTCCCTTCGTAATACGACTCACTATAGGGTGGTACTGCCGTAATAATTTAAGAGCAGCAGACCAGATTAATGATCATGTTTATTCCCTTCTGAAAAGGGTAAACTACTTATTAGAGATATGTCTCGGGTGCCAAGGTACCAAGAAACCTTCATT |
| OC43-Probe I.713 | AY391777.1 | OC43-Probe I | TGTGAGAGATTCCCTTCGTAATACGACTCACTATAGGGTGACCAGATTAATGATCATGTTTATTCCCTTCTTGAAAAGGGTAAACTACTTATTAGAGATACAAATAAAGAAGTTTCGTTGGTGACAGTTTGGTAAATATAAAGGTTTTATGGAATTCTCGGGTGCCAAGGTACCAAGAAACCTTCATT |
| OC43-Probe II.0 | AY391777.1 | OC43-Probe II | GTATTAGAGTTTCCCCTCTAATACGACTCACTATAGGGTGTGTCGCTTATGCTGCTTAATCATTTATCATTTCCACTTACCCATTTCAGGTTTTTTGTGTTAAATTATAAAGGTTTTATGGAATTCTCGATGAAGGAGGTGGATTGGAATTCTCATGGTAAA |
| OC43-Probe II.1 | AY391777.1 | OC43-Probe II | GTATTAGAGTTTCCCCTCTAATACGACTCACTATAGGGTGCTTATGCTGCTTAATCATTTATCATTTCCACTTACCCATTTCAGGTTTTTGTGTTAAATTATAAAGGTTTACAATTTCAAATTCTCGATGAAGGAGGTGGATTGGAATTCTCATGGTAAA |
| OC43-Probe II.2 | AY391777.1 | OC43-Probe II | GTATTAGAGTTTCCCCTCTAATACGACTCACTATAGGGTGTTTGTGTTAAATTATAAAGGTTTACAATTTCAAATTCTCGATGAAGGAGGTGGATTGGAATTCTCGAAGACTGAAGACTGGACATACAGCCTGTAAAGGTTAAATCTCATGGTAAA |
| OC43-Probe II.3 | AY391777.1 | OC43-Probe II | GTATTAGAGTTTCCCCTCTAATACGACTCACTATAGGGTGATTCTCGATGAAGGAGGTGGATTGGAATTCTCAAGGGCTAAAGGGCTAAATCTCATGGTAAAGGTGCGAAGACTGGACATACAGCCTGTAAAGGTTAAATCTCATGGTAAA |

89

| ASO ID | Accession | Group ID | Custom Array sequence |
|---|---|---|---|
| OC43-Probe II.4 | AY391777.1 | OC43-Probe II | GTATTAGAGTTTCCCCTCTAATACGACTCACTATAGGGTGTAAAATACAAACAGCGCCACACATTAGTCTTACTATGCTGGACATACAGCCTGAAGACTATAAAAGTGTTGATGTCGCTATTCAAGAATGGAATTCTCGGGTGCCAAGGGCTAAATCTCATGGTAAA |
| OC43-Probe II.5 | AY391777.1 | OC43-Probe II | GTATTAGAGTTTCCCCTCTAATACGACTCACTATAGGGCTTACTATGCTGGACATACAGCCTGAAGACTATAAAAGTGTTGATGTCGCTATTCAAGAAGTTATTGATGATATGCATTGGGGTGATGGTTGGAATTCTCGGGTGCCAAGGGCTAAATCTCATGGTAAA |
| OC43-Probe II.6 | AY391777.1 | OC43-Probe II | GTATTAGAGTTTCCCCTCTAATACGACTCACTATAGGGTATAAAAGTGTTGATGTCGCTATTCAAGAAGTTATTGATGATATGCATTGGGGTGATGGTTTTCAGATTAAATTTGAGAATCCTCACATCTGGAATTCTCGGGTGCCAAGGGCTAAATCTCATGGTAAA |
| OC43-Probe II.7 | AY391777.1 | OC43-Probe II | GTATTAGAGTTTCCCCTCTAATACGACTCACTATAGGGGTTATTGATGATATGCATTGGGGTGATGGTTTTCAGATTAAATTTGAGAATCCTCACATCCTAGGAAGATGCATAGTTTTAGATGTTAAATGGAATTCTCGGGTGCCAAGGGCTAAATCTCATGGTAAA |
| OC43-Probe II.8 | AY391777.1 | OC43-Probe II | GTATTAGAGTTTCCCCTCTAATACGACTCACTATAGGGTTTCAGATTAAATTTGAGAATCCTCACATCCTAGGAAGATGCATAGTTTTAGATGTTAAAGGTGTAGAAGAATTGCATGACGATTTAGTTTGGAATTCTCGGGTGCCAAGGGCTAAATCTCATGGTAAA |
| OC43-Probe II.9 | AY391777.1 | OC43-Probe II | GTATTAGAGTTTCCCCTCTAATACGACTCACTATAGGGCTAGGAAGATGCATAGTTTTAGATGTTAAAGGTGTAGAAGAATTGCATGACGATTTAGTTAATTACATTCGTGATAAAGGTTGTGTTGCTTGGAATTCTCGGGTGCCAAGGGCTAAATCTCATGGTAAA |
| OC43-Probe II.10 | AY391777.1 | OC43-Probe II | GTATTAGAGTTTCCCCTCTAATACGACTCACTATAGGGGGTGTAGAAGAATTGCATGACGATTTAGTTAATTACATTCGTGATAAAGGTTGTGTTGCTGACCAATCCAGGAAATGGATTGGCCATTGCTGGAATTCTCGGGTGCCAAGGGCTAAATCTCATGGTAAA |
| OC43-Probe II.11 | AY391777.1 | OC43-Probe II | GTATTAGAGTTTCCCCTCTAATACGACTCACTATAGGGAATTACATTCGTGATAAAGGTTGTGTTGCTGACCAATCCAGGAAATGGATTGGCCATTGCACCATAGCTCAACTCACGGATGCAGCACTGTGGAATTCTCGGGTGCCAAGGGCTAAATCTCATGGTAAA |
| OC43-Probe II.12 | AY391777.1 | OC43-Probe II | GTATTAGAGTTTCCCCTCTAATACGACTCACTATAGGGGACCAATCCAGGAAATGGATTGGCCATTGCACCATAGCTCAACTCACGGATGCAGCACTGTCCATTAAGGAAAATGTTGATTTTATAAACTGGAATTCTCGGGTGCCAAGGGCTAAATCTCATGGTAAA |
| OC43-Probe II.13 | AY391777.1 | OC43-Probe II | GTATTAGAGTTTCCCCTCTAATACGACTCACTATAGGGACCCATAGCTCAACTCACGGATGCAGCACTGTCCATTAAGGAAAATGTTGATTTTATAAACAGCATGCAATTCAATTATAAAATCACCATCTGGAATTCTCGGGTGCCAAGGGCTAAATCTCATGGTAAA |
| OC43-Probe II.14 | AY391777.1 | OC43-Probe II | GTATTAGAGTTTCCCCTCTAATACGACTCACTATAGGGTCCATTAAGGAAAATGTTGATTTTATAAACAGCATGCAATTCAATTATAAAATCACCATCAACCCCTCATCACCGGCTAGACTTGAAATATGGAATTCTCGGGTGCCAAGGGCTAAATCTCATGGTAAA |
| OC43-Probe II.15 | AY391777.1 | OC43-Probe II | GTATTAGAGTTTCCCCTCTAATACGACTCACTATAGGGAGCATGCAATTCAATTATAAAATCACCATCAACCCCTCATCACCGGCTAGACTTGAAATAGTTAAGCTCGGTGCTGAAAAGAAAGATGGTTGGAATTCTCGGGTGCCAAGGGCTAAATCTCATGGTAAA |
| OC43-Probe II.16 | AY391777.1 | OC43-Probe II | GTATTAGAGTTTCCCCTCTAATACGACTCACTATAGGGAACCCCTCATCACCGGCTAGACTTGAAATAGTTAAGCTCGGTGCTGAAAAGAAAGATGGTTTTTATGAAACCATAGTTAGTCACTGGATGTGGAATTCTCGGGTGCCAAGGGCTAAATCTCATGGTAAA |
| OC43-Probe II.17 | AY391777.1 | OC43-Probe II | GTATTAGAGTTTCCCCTCTAATACGACTCACTATAGGGGTTAAGCTCGGTGCTGAAAAGAAAGATGGTTTTTATGAAACCATAGTTAGTCACTGGATGGGAATTCGTTTTGAATACACATCACCCACTTGGAATTCTCGGGTGCCAAGGGCTAAATCTCATGGTAAA |
| OC43-Probe II.18 | AY391777.1 | OC43-Probe II | GTATTAGAGTTTCCCCTCTAATACGACTCACTATAGGGTTTTATGAAACCATAGTTAGTCACTGGATGGGAATTCGTTTTGAATACACATCACCCACTGATAAGCTAGCTATGATTATGGGTTATTGTTGGAATTCTCGGGTGCCAAGGGCTAAATCTCATGGTAAA |
| OC43-Probe II.19 | AY391777.1 | OC43-Probe II | GTATTAGAGTTTCCCCTCTAATACGACTCACTATAGGGGGGAATTCGTTTTGAATACACATCACCCACTGATAAGCTAGCTATGATTATGGGTTATTGTTGTTAGATGTGGTACGTAAAGAGCTAGAATGGAATTCTCGGGTGCCAAGGGCTAAATCTCATGGTAAA |
| OC43-Probe II.20 | AY391777.1 | OC43-Probe II | GTATTAGAGTTTCCCCTCTAATACGACTCACTATAGGGGATAAGCTAGCTATGATTATGGGTTATTGTTGTTAGATGTGGTACGTAAAGAGCTAGAAGAAGGCGATCTTCCCGAGAATGATGATGATTGGAATTCTCGGGTGCCAAGGGCTAAATCTCATGGTAAA |
| OC43-Probe II.21 | AY391777.1 | OC43-Probe II | GTATTAGAGTTTCCCCTCTAATACGACTCACTATAGGGTGTTTAGATGTGGTACGTAAAGAGCTAGAAGAAGGCGATCTTCCCGAGAATGATGATGATGCTTGGTTTAAGCTATCGTACCATTATGAATGGAATTCTCGGGTGCCAAGGGCTAAATCTCATGGTAAA |
| OC43-Probe II.22 | AY391777.1 | OC43-Probe II | GTATTAGAGTTTCCCCTCTAATACGACTCACTATAGGGGAAGGCGATCTTCCCGAGAATGATGATGATGCTTGGTTTAAGCTATCGTACCATTATGAAAACAATTCTTGGTTCTTCCGACATGTCTACTGGAATTCTCGGGTGCCAAGGGCTAAATCTCATGGTAAA |
| OC43-Probe II.23 | AY391777.1 | OC43-Probe II | GTATTAGAGTTTCCCCTCTAATACGACTCACTATAGGGCTTGGTTTAAGCTATCGTACCATTATGAAAACAATTCTTGGTTCTTCCGACATGTCTACAGGAAAAGTTTTCATTTCCGTAAGGCTTGTTGGAATTCTCGGGTGCCAAGGGCTAAATCTCATGGTAAA |
| OC43-Probe II.24 | AY391777.1 | OC43-Probe II | GTATTAGAGTTTCCCCTCTAATACGACTCACTATAGGGAACAATTCTTGGTTCTTCCGACATGTCTACAGGAAAAGTTTTCATTTCCGTAAGGCTTGTCAAAATTTAGATTGTAATTGTTTGGGGTTTTGGAATTCTCGGGTGCCAAGGGCTAAATCTCATGGTAAA |
| OC43-Probe II.25 | AY391777.1 | OC43-Probe II | GTATTAGAGTTTCCCCTCTAATACGACTCACTATAGGGAGGAAAAGTTTTCATTTCCGTAAGGCTTGTCAAAATTTAGATTGTAATTGTTTGGGGTTTTATGAATCTTCAGTTGAAGAATATTAAACTTGGAATTCTCGGGTGCCAAGGGCTAAATCTCATGGTAAA |
| OC43-Probe II.26 | AY391777.1 | OC43-Probe II | GTATTAGAGTTTCCCCTCTAATACGACTCACTATAGGGCAAAATTTAGATTGTAATTGTTTGGGGTTTTATGAATCTTCAGTTGAAGAATATTAAACTCAGTGAAAATGTTTTTGCTTCCTAGATTTATGGAATTCTCGGGTGCCAAGGGCTAAATCTCATGGTAAA |
| OC43-Probe II.27 | AY391777.1 | OC43-Probe II | GTATTAGAGTTTCCCCTCTAATACGACTCACTATAGGGTATGAATCTTCAGTTGAAGAATATTAAACTCAGTGAAAATGTTTTTGCTTCCTAGATTTATTCTAGTTAGCTGCATAATTGGTAGCTTAGTGGAATTCTCGGGTGCCAAGGGCTAAATCTCATGGTAAA |
| OC43-Probe II.28 | AY391777.1 | OC43-Probe II | GTATTAGAGTTTCCCCTCTAATACGACTCACTATAGGGCAGTGAAAATGTTTTTGCTTCCTAGATTTATTCTAGTTAGCTGCATAATTGGTAGCTTAGGTTTTTACAACCCTCCTACCAATGTTGTTTTGGAATTCTCGGGTGCCAAGGGCTAAATCTCATGGTAAA |

| ASO ID | Accession | Group ID | Custom Array sequence |
|---|---|---|---|
| OC43-Probe II.29 | AY391777.1 | OC43-Probe II | GTATTAGAGAGTTTCCCCTCTAATACGACTCACTATAGGGTTCTAGTTAGCTGCATAATGGTTTTACAACCCTCTACCAATGTTGTTTCGGCATGTAAATGGAGAATTGGGTTTTATTGTTGGCGAATCTCGGGTGCCAAGGGCTAAATCTCATGGTAAA |
| OC43-Probe II.30 | AY391777.1 | OC43-Probe II | GTATTAGAGAGTTTCCCCTCTAATACGACTCACTATAGGGTTTTACAACCCTCTACCAATGTTGTTCGGCATGTAAATGGAGAATTGTTCGAGATGTAATCATATCTCTATATGGAATTCTCGGGTGCCAAGGGCTAAATCTCATGGTAAA |
| OC43-Probe II.31 | AY391777.1 | OC43-Probe II | GTATTAGAGAGTTTCCCCTCTAATACGACTCACTATAGGGCGCATGTAAATGGAGAATTGGTTTTTATTTGGTGACAGTCGTTCAGAATGTTATTTGGTGTAATCAACCCCATAATTATTCTTAATATCAACCCCATAATTATTCTCGGGTGCCAAGGGCTAAATCTCATGGTAAA |
| OC43-Probe II.32 | AY391777.1 | OC43-Probe II | GTATTAGAGAGTTTCCCCTCTAATACGACTCACTATAGGGTGACAGTCGTTCAGATTGTAATCGTAATCATATTGTTAATATCAACCCCATAATTCTTAATATGGACCTTAAATCTCGTGTGATTCTCGGGTGCCAAGGGCTAAATCTCATGGTAAA |
| OC43-Probe II.33 | AY391777.1 | OC43-Probe II | GTATTAGAGAGTTTCCCCTCTAATACGACTCACTATAGGGTGTTAATATCAACCCCATAATTATTCTTATATGGACCTTAAAATATCTTTATATGGACCTTAAATCTCGTGTGATTCTGGTAAAATATCTCGGGTGCCAAGGGCTAAATCTCATGGTAAA |
| OC43-Probe II.34 | AY391777.1 | OC43-Probe II | GTATTAGAGAGTTTCCCCTCTAATACGACTCACTATAGGGTGGACCTTAAATCTCGTGTGATTCTGGTAAAATATCTTTAGGAGTTTCACTTTACCGATTTTTCACTTTACCGATTTTTATAATTACACAGGCGAAGGTCAACAAATTATTCTCATGGTAAA |
| OC43-Probe II.35 | AY391777.1 | OC43-Probe II | GTATTAGAGAGTTTCCCCTCTAATACGACTCACTATAGGGTAAAATATCTTTAGGAGTTTCACTTTACCGATTTTTATAATTACACAGGCGAAGGTCAACAAATTATTTTTATGGAGGGTGTTAAATTTCGGGTGCCAAGGGCTAAATCTCATGGTAAA |
| OC43-Probe II.36 | AY391777.1 | OC43-Probe II | GTATTAGAGAGTTTCCCCTCTAATACGACTCACTATAGGGTTTTCACTTTACCGATTTTTATAATTACACAGGCGAAGGTCAACAAATTATTTTTATGGAGGGTGTTAAATTTCGGGTGCCAAGGGCTAAATCTCATGGTAAA |
| OC43-Probe II.37 | AY391777.1 | OC43-Probe II | GTATTAGAGAGTTTCCCCTCTAATACGACTCACTATAGGGTATAATTACACAGGCGAAGGTCAACAAATTATTTTTATGGAGGGTGTTAAATTTGCAACCGTTCGTGGTGCCAAGGGCTAAATCTCATGGTAAA |
| OC43-Probe II.38 | AY391777.1 | OC43-Probe II | GTATTAGAGAGTTTCCCCTCTAATACGACTCACTATAGGGTTTTTATGGAGGGTGTTAAATGCCAATTTTACGCTTCTGGTAGTAGCAGAATAAAGGCTTGGAATTCTCGGGTGCCAAGGGCTAAATCTCATGGTAAA |
| OC43-Probe II.39 | AY391777.1 | OC43-Probe II | GTATTAGAGAGTTTCCCCTCTAATACGACTCACTATAGGGTGTTAAATGCAACCGTTCTGGTAGTAGCAGAATAAAGGCTTGTTTATACTCAGGTTTATAAGAATATGGTGGAATTCTCGGGTGCCAAGGGCTAAATCTCATGGTAAA |
| OC43-Probe II.40 | AY391777.1 | OC43-Probe II | GTATTAGAGAGTTTCCCCTCTAATACGACTCACTATAGGGTAATGCAACCGTTCTGGTAGTAGCAGAATAAAGGCTTGTTTATACTCAGGTTTATAAGAATATGGCTGTGTATCGCGAGCCTTACTTTGTTCATGGTAAA |
| OC43-Probe II.41 | AY391777.1 | OC43-Probe II | GTATTAGAGAGTTTCCCCTCTAATACGACTCACTATAGGGTGTTTATACTCAGGTTTATAAGAATATGGCTGTGTATCGCGAGCCTTACTTTGTTTAATGGTCCGCCACAAGTGGAATTCTCGGGTGCCAAGGGCTAAATCTCATGGTAAA |
| OC43-Probe II.42 | AY391777.1 | OC43-Probe II | GTATTAGAGAGTTTCCCCTCTAATACGACTCACTATAGGGTCTGTGTATCGCGAGCCTTACTTTGTTTAATGGTCCGCCACAAGTGGATTTTAGTGGAATCTCGGGTGCCAAGGGCTAAATCTCATGGTAAA |
| OC43-Probe II.43 | AY391777.1 | OC43-Probe II | GTATTAGAGAGTTTCCCCTCTAATACGACTCACTATAGGGTGTATGCGAGCCTACCATATGTTTAATGACCTTACTTTGTAAATCGCATATATTAATTGTCTTTAATAACCTGCATATATTAATTCTCGGGTGCCAAGGGCTAAATCTCATGGTAAA |
| OC43-Probe II.44 | AY391777.1 | OC43-Probe II | GTATTAGAGAGTTTCCCCTCTAATACGACTCACTATAGGGTCTACCATATGTTTAATGACCTTACTTTGTCTTTAATAACCTGCATATATTAATTGTCTTAATAACCTGCGATATAGCTCTGGGGATTATTATTAAGGTGGAATTCTCGGGTGCCAAGGGCTAAATCTCATGGTAAA |
| OC43-Probe II.45 | AY391777.1 | OC43-Probe II | GTATTAGAGAGTTTCCCCTCTAATACGACTCACTATAGGGTCTTTAATAACCTGCGATATAGCTCTGGGGATTATTATTGTTATTGTCAAGCTGAAGCTGAATCTCTTTATTTGCAGGTTGTGTGGAATTCTCGGGTGCCAAGGGCTAAATCTCATGGTAAA |
| OC43-Probe II.46 | AY391777.1 | OC43-Probe II | GTATTAGAGAGTTTCCCCTCTAATACGACTCACTATAGGGTCTGGGGATTATTATTGTTATTTATTTGCAGGTTGAAGCTGAATCTCTTTATTTGTATTTTTGTACCACTTTGTTTTATTTGCAGGTTGTGTGGAATTCTCGGGTGCCAAGGGCTAAATCTCATGGTAAA |
| OC43-Probe II.47 | AY391777.1 | OC43-Probe II | GTATTAGAGAGTTTCCCCTCTAATACGACTCACTATAGGGTTATTGTCAGGTTGTGAAGCTGAGAGTGATATCGGTGATACTTTGTGATTTTTATTGTGACGAGTATATCGTTTAAAGTGACGAGTATATCTCGGGTGCCAAGGGCTAAATCTCATGGTAAA |
| OC43-Probe II.48 | AY391777.1 | OC43-Probe II | GTATTAGAGAGTTTCCCCTCTAATACGACTCACTATAGGGTGAAGCTGAGAGTGATATCGGTGATACTTTGTGATTTTTTGTGGAATACAAAGTATTTGTGTTTTAAAGGGCAAGTTTTTGTGTTTTCTCGGGTGCCAAGGGCTAAATCTCATGGTAAA |
| OC43-Probe II.49 | AY391777.1 | OC43-Probe II | GTATTAGAGAGTTTCCCCTCTAATACGACTCACTATAGGGTGATACTTTGTGATTTTTTGTGGAATACAAAGTATTTAAAGGGCAAGTTTTTGTATTTTTAAAGGGCAAGTTTTTAATGATGATGAGTCAATTTAATGATGATGAGTCATGGTAAA |
| OC43-Probe II.50 | AY391777.1 | OC43-Probe II | GTATTAGAGAGTTTCCCCTCTAATACGACTCACTATAGGGTGGAATACAAAGTATTTTGTCGAATACAAAGTATTAATGATGAGTCAATTTATGATCTCAATTCTCACAGAAACCATTACCACTGGTTTTGATCTTATGGTAAA |
| OC43-Probe II.51 | AY391777.1 | OC43-Probe II | GTATTAGAGAGTTTCCCCTCTAATACGACTCACTATAGGGTGTCGAATTTATGGTCTCAATTCTCACAGAAACCATTACCACTGGTTTTGATCTTATGGTTTTATTTAGTTTACCCTCTCGGGTATGGAATTCTCGGGTGCCAAGGGCTAAATCTCATGGTAAA |
| OC43-Probe II.52 | AY391777.1 | OC43-Probe II | GTATTAGAGAGTTTCCCCTCTAATACGACTCACTATAGGGTTCTCACAGAAACCATTACCACTGGTTTTGATCTTATGGTTTTATTTAGTTTATTATTAGTTAGTGTTCAAATGAGCAATCTGTCTTATGGAATTCTCGGGTGCCAAGGGCTAAATCTCATGGTAAA |
| OC43-Probe II.53 | AY391777.1 | OC43-Probe II | GTATTAGAGAGTTTCCCCTCTAATACGACTCACTATAGGGTGATCTTATGGTTTTATTTAGTTTATTATTAGTTAGTGTTCAAATGAGCAATCTGTTGTTCAACTGGTAATTATTAGCCCATTTCAAATGAGCAATCTGTCTTATGGAATTCTCGGGTGCCAAGGGCTAAATCTCATGGTAAA |

| ASO ID | Accession | Group ID | Custom Array sequence |
|---|---|---|---|
| OC43-Probe II.54 | AY391777.1 | OC43-Probe II | GTATTAGAGTTTCCCCTCTAATACGACTCACTATAGGGATTATTTAGCCATTTCAAATGAGCTATTGTTAACTGTTCCTACGAAAGCAATCTGTCTTAATAAGCGTAAGGATTTTACGCCTGTACAGGTGGAATTCTCGGGTGCCAAGGGCTAAATCTCATGGTAAA |
| OC43-Probe II.55 | AY391777.1 | OC43-Probe II | GTATTAGAGTTTCCCCTCTAATACGACTCACTATAGGGTAACTGTTCCTACGAAAGCAATCTGTCTTAATAAGCGTAAGGATTTTACGCCTGTACAGGTTGTTGATTCGCCGGTGGAACAATGCCAGGCTGGAATTCTCGGGTGCCAAGGGCTAAATCTCATGGTAAA |
| OC43-Probe II.56 | AY391777.1 | OC43-Probe II | GTATTAGAGTTTCCCCTCTAATACGACTCACTATAGGGATAAGCGTAAGGATTTTACGCCTGTACAGGTTGTTGATTCGCCGGTGGAACAATGCCAGGCAGTCTGATAACATGACGGCGGTTGCTTGTCTGGAATTCTCGGGTGCCAAGGGCTAAATCTCATGGTAAA |
| OC43-Probe II.57 | AY391777.1 | OC43-Probe II | GTATTAGAGTTTCCCCTCTAATACGACTCACTATAGGGTTGTTGATTCGCCGGTGGAACAATGCCAGGCAGTCTGATAACATGACGGCGGTTGCTTGTCAACCTCCGTACTGTTATTTTCGTAATTCTCATGGAATTCTCGGGTGCCAAGGGCTAAATCTCATGGTAAA |
| OC43-Probe II.58 | AY391777.1 | OC43-Probe II | GTATTAGAGTTTCCCCTCTAATACGACTCACTATAGGGAGTCTGATAACATGACGGCGGTTGCTTGTCAACCTCCGTACTGTTATTTTCGTAATTCTACTACCAACTATGTTGGTGTTTATGATATTATGGAATTCTCGGGTGCCAAGGGCTAAATCTCATGGTAAA |
| OC43-Probe II.59 | AY391777.1 | OC43-Probe II | GTATTAGAGTTTCCCCTCTAATACGACTCACTATAGGGAACCTCCGTACTGTTATTTTCGTAATTCTACTACCAACTATGTTGGTGTTTATGATATTAATCATGGAGATGCTGGTTTTACTAGCATACTGGAATTCTCGGGTGCCAAGGGCTAAATCTCATGGTAAA |
| OC43-Probe II.60 | AY391777.1 | OC43-Probe II | GTATTAGAGTTTCCCCTCTAATACGACTCACTATAGGGCTACCAACTATGTTGGTGTTTATGATATTAATCATGGAGATGCTGGTTTTACTAGCATACTTAGTGGTTTGTTATATAATTCACCTTGTTTGGAATTCTCGGGTGCCAAGGGCTAAATCTCATGGTAAA |
| OC43-Probe II.61 | AY391777.1 | OC43-Probe II | GTATTAGAGTTTCCCCTCTAATACGACTCACTATAGGGATCATGGAGATGCTGGTTTTACTAGCATACTTAGTGGTTTGTTATATAATTCACCTTGTTTTTCGCAGCAAGGCGTTTTTAGGTATGATATGGAATTCTCGGGTGCCAAGGGCTAAATCTCATGGTAAA |
| OC43-Probe II.62 | AY391777.1 | OC43-Probe II | GTATTAGAGTTTCCCCTCTAATACGACTCACTATAGGGTTAGTGGTTTGTTATATAATTCACCTTGTTTTTCGCAGCAAGGCGTTTTTAGGTATGATAATGTTAGCAGTGTCTGGCCTCTCTACCCCTTGGAATTCTCGGGTGCCAAGGGCTAAATCTCATGGTAAA |
| OC43-Probe II.63 | AY391777.1 | OC43-Probe II | GTATTAGAGTTTCCCCTCTAATACGACTCACTATAGGGTTTCGCAGCAAGGCGTTTTTAGGTATGATAATGTTAGCAGTGTCTGGCCTCTCTACCCCTATGGCAGATGTCCCACTGCTGCTGATATTATGGAATTCTCGGGTGCCAAGGGCTAAATCTCATGGTAAA |
| OC43-Probe II.64 | AY391777.1 | OC43-Probe II | GTATTAGAGTTTCCCCTCTAATACGACTCACTATAGGGATGTTAGCAGTGTCTGGCCTCTCTACCCCTATGGCAGATGTCCCACTGCTGCTGATATTAATATCCCTGATTTACCCATTTGTGTGTATGTGGAATTCTCGGGTGCCAAGGGCTAAATCTCATGGTAAA |
| OC43-Probe II.65 | AY391777.1 | OC43-Probe II | GTATTAGAGTTTCCCCTCTAATACGACTCACTATAGGGATGGCAGATGTCCCACTGCTGCTGATATTAATATCCCTGATTTACCCATTTGTGTGTATGATCCGCTACCAGTTATTTTGCTTGGCATTCTGGAATTCTCGGGTGCCAAGGGCTAAATCTCATGGTAAA |
| OC43-Probe II.66 | AY391777.1 | OC43-Probe II | GTATTAGAGTTTCCCCTCTAATACGACTCACTATAGGGATATCCCTGATTTACCCATTTGTGTGTATGATCCGCTACCAGTTATTTTGCTTGGCATTCTTTTGGGCGTTGCGATTGTAATTATTGTAGTGGAATTCTCGGGTGCCAAGGGCTAAATCTCATGGTAAA |
| OC43-Probe II.67 | AY391777.1 | OC43-Probe II | GTATTAGAGTTTCCCCTCTAATACGACTCACTATAGGGATCCGCTACCAGTTATTTTGCTTGGCATTCTTTTGGGCGTTGCGATTGTAATTATTGTAGTTTTGTTGTTATATTTTATGGTGGATAATGTGGAATTCTCGGGTGCCAAGGGCTAAATCTCATGGTAAA |
| OC43-Probe II.68 | AY391777.1 | OC43-Probe II | GTATTAGAGTTTCCCCTCTAATACGACTCACTATAGGGTTTTGGGCGTTGCGATTGTAATTATTGTAGTTTTGTTGTTATATTTTATGGTGGATAATGTTACTAGGCTGCATGATGCTTAGACCATAATGGAATTCTCGGGTGCCAAGGGCTAAATCTCATGGTAAA |
| OC43-Probe II.69 | AY391777.1 | OC43-Probe II | GTATTAGAGTTTCCCCTCTAATACGACTCACTATAGGGTTTTGTTGTTATATTTTATGGTGGATAATGTTACTAGGCTGCATGATGCTTAGACCATAATCTAAACATGTTTTTGATACTTTTAATTTCTGGAATTCTCGGGTGCCAAGGGCTAAATCTCATGGTAAA |
| OC43-Probe II.70 | AY391777.1 | OC43-Probe II | GTATTAGAGTTTCCCCTCTAATACGACTCACTATAGGGTTACTAGGCTGCATGATGCTTAGACCATAATCTAAACATGTTTTTGATACTTTTAATTTCCTTACCAACGGCTTTTGCTGTTATAGGAGATGGAATTCTCGGGTGCCAAGGGCTAAATCTCATGGTAAA |
| OC43-Probe II.71 | AY391777.1 | OC43-Probe II | GTATTAGAGTTTCCCCTCTAATACGACTCACTATAGGGTCTAAACATGTTTTTGATACTTTTAATTTCCTTACCAACGGCTTTTGCTGTTATAGGAGATTTAAAGTGTACTTCAGATACTAGTTATATTGGAATTCTCGGGTGCCAAGGGCTAAATCTCATGGTAAA |
| OC43-Probe II.72 | AY391777.1 | OC43-Probe II | GTATTAGAGTTTCCCCTCTAATACGACTCACTATAGGGCTTACCAACGGCTTTTGCTGTTATAGGAGATTTAAAGTGTACTTCAGATACTAGTTATATTAATGATAAAGACACCGGTCCTCCTCCTTGGAATTCTCGGGTGCCAAGGGCTAAATCTCATGGTAAA |
| OC43-Probe II.73 | AY391777.1 | OC43-Probe II | GTATTAGAGTTTCCCCTCTAATACGACTCACTATAGGGTTTAAAGTGTACTTCAGATACTAGTTATATTAATGATAAAGACACCGGTCCTCCTCCTATAAGTACTGATACTGTTGATGTTACTAATGGTGGAATTCTCGGGTGCCAAGGGCTAAATCTCATGGTAAA |
| OC43-Probe II.74 | AY391777.1 | OC43-Probe II | GTATTAGAGTTTCCCCTCTAATACGACTCACTATAGGGTAATGATAAAGACACCGGTCCTCCTCCTATAAGTACTGATACTGTTGATGTTACTAATGGTTTGGGTACTTATTATGTTTTAGATCGTGTTGGAATTCTCGGGTGCCAAGGGCTAAATCTCATGGTAAA |
| OC43-Probe II.75 | AY391777.1 | OC43-Probe II | GTATTAGAGTTTCCCCTCTAATACGACTCACTATAGGGAAGTACTGATACTGTTGATGTTACTAATGGTTTGGGTACTTATTATGTTTTAGATCGTGTGTATTTAAATACTACGTTGTTTCTTAATGGTGGAATTCTCGGGTGCCAAGGGCTAAATCTCATGGTAAA |
| OC43-Probe II.76 | AY391777.1 | OC43-Probe II | GTATTAGAGTTTCCCCTCTAATACGACTCACTATAGGGTTTGGGTACTTATTATGTTTTAGATCGTGTGTATTTAAATACTACGTTGTTTCTTAATGGTTATTACCCTACTTCAGGTTCCACATATCGTGGAATTCTCGGGTGCCAAGGGCTAAATCTCATGGTAAA |
| OC43-Probe II.77 | AY391777.1 | OC43-Probe II | GTATTAGAGTTTCCCCTCTAATACGACTCACTATAGGGGTATTTAAATACTACGTTGTTTCTTAATGGTTATTACCCTACTTCAGGTTCCACATATCGTAATATGGCACTGAAGGGAAGTGTACTATTTGGAATTCTCGGGTGCCAAGGGCTAAATCTCATGGTAAA |
| OC43-Probe II.78 | AY391777.1 | OC43-Probe II | GTATTAGAGTTTCCCCTCTAATACGACTCACTATAGGGTTATTACCCTACTTCAGGTTCCACATATCGTAATATGGCACTGAAGGGAAGTGTACTATTGAGCAGACTATGGTTTAAACCACCATTTCTTGGAATTCTCGGGTGCCAAGGGCTAAATCTCATGGTAAA |

93

| ASO ID | Accession | Group ID | Custom Array sequence |
|---|---|---|---|
| OC43-Probe II.79 | AY391777.1 | OC43-Probe II | GTATTAGAGTTTCCCCTCTAATACGACTCACTATAGGGTAATATGGCACTGAAGGGAAGTGTACTATTGAGCAGACTATGGTTTAAACCACCATTTCTTTCTGATTTTATTAATGGTATTTTTGCTAATGGAATTCTCGGGTGCCAAGGGCTAAATCTCATGGTAAA |
| OC43-Probe II.80 | AY391777.1 | OC43-Probe II | GTATTAGAGTTTCCCCTCTAATACGACTCACTATAGGGGAGCAGACTATGGTTTAAACCACCATTTCTTTCTGATTTTATTAATGGTATTTTTGCTAAGGTCAAAAATACCAAGGTTATTAAAGATCGTGGAATTCTCGGGTGCCAAGGGCTAAATCTCATGGTAAA |
| OC43-Probe II.81 | AY391777.1 | OC43-Probe II | GTATTAGAGTTTCCCCTCTAATACGACTCACTATAGGGTTCTGATTTTATTAATGGTATTTTTGCTAAGGTCAAAAATACCAAGGTTATTAAAGATCGTGTAATGTATAGTGAGTTCCCTGCTATAACTGGAATTCTCGGGTGCCAAGGGCTAAATCTCATGGTAAA |
| OC43-Probe II.82 | AY391777.1 | OC43-Probe II | GTATTAGAGTTTCCCCTCTAATACGACTCACTATAGGGGGTCAAAAATACCAAGGTTATTAAAGATCGTGTAATGTATAGTGAGTTCCCTGCTATAACTATAGGTAGTACTTTTGTAAATACATCCTATGGAATTCTCGGGTGCCAAGGGCTAAATCTCATGGTAAA |
| OC43-Probe II.83 | AY391777.1 | OC43-Probe II | GTATTAGAGTTTCCCCTCTAATACGACTCACTATAGGGTGTAATGTATAGTGAGTTCCCTGCTATAACTATAGGTAGTACTTTTGTAAATACATCCTATAGTGTGGTAGTACAACCACGTACAATCAATGGAATTCTCGGGTGCCAAGGGCTAAATCTCATGGTAAA |
| OC43-Probe II.84 | AY391777.1 | OC43-Probe II | GTATTAGAGTTTCCCCTCTAATACGACTCACTATAGGGTATAGGTAGTACTTTTGTAAATACATCCTATAGTGTGGTAGTACAACCACGTACAATCAATTCAACACAGGATGGTTATAATAAATTACATGGAATTCTCGGGTGCCAAGGGCTAAATCTCATGGTAAA |
| OC43-Probe II.85 | AY391777.1 | OC43-Probe II | GTATTAGAGTTTCCCCTCTAATACGACTCACTATAGGGTAGTGTGGTAGTACAACCACGTACAATCAATTCAACACAGGATGGTTATAATAAATTACAAGGTCTTTTAGAGGTCTCTGTTTGCCAGTATGGAATTCTCGGGTGCCAAGGGCTAAATCTCATGGTAAA |
| OC43-Probe II.86 | AY391777.1 | OC43-Probe II | GTATTAGAGTTTCCCCTCTAATACGACTCACTATAGGGTTCAACACAGGATGGTTATAATAAATTACAAGGTCTTTTAGAGGTCTCTGTTTGCCAGTATAATATGTGCGAGTACCCACAAACGATTTGTGGAATTCTCGGGTGCCAAGGGCTAAATCTCATGGTAAA |
| OC43-Probe II.87 | AY391777.1 | OC43-Probe II | GTATTAGAGTTTCCCCTCTAATACGACTCACTATAGGGAGGTCTTTTAGAGGTCTCTGTTTGCCAGTATAATATGTGCGAGTACCCACAAACGATTTGTCATCCTAACCTGGGTAATCATCGCAAAGATGGAATTCTCGGGTGCCAAGGGCTAAATCTCATGGTAAA |
| OC43-Probe II.88 | AY391777.1 | OC43-Probe II | GTATTAGAGTTTCCCCTCTAATACGACTCACTATAGGGTAATATGTGCGAGTACCCACAAACGATTTGTCATCCTAACCTGGGTAATCATCGCAAAGAACTATGGCATTTGGATACAGGTGTTGTTTCTGGAATTCTCGGGTGCCAAGGGCTAAATCTCATGGTAAA |
| OC43-Probe II.89 | AY391777.1 | OC43-Probe II | GTATTAGAGTTTCCCCTCTAATACGACTCACTATAGGGTCATCCTAACCTGGGTAATCATCGCAAAGAACTATGGCATTTGGATACAGGTGTTGTTTCCTGTTTATATAAGCGTAATTTCACATATGATGGAATTCTCGGGTGCCAAGGGCTAAATCTCATGGTAAA |
| OC43-Probe II.90 | AY391777.1 | OC43-Probe II | GTATTAGAGTTTCCCCTCTAATACGACTCACTATAGGGGACTATGGCATTTGGATACAGGTGTTGTTTCCTGTTTATATAAGCGTAATTTCACATATGATGTGAATGCTGATTATTTGTATTTTCATTTTGGAATTCTCGGGTGCCAAGGGCTAAATCTCATGGTAAA |
| OC43-Probe II.91 | AY391777.1 | OC43-Probe II | GTATTAGAGTTTCCCCTCTAATACGACTCACTATAGGGCTGTTTATATAAGCGTAATTTCACATATGATGTGAATGCTGATTATTTGTATTTTCATTTTTATCAAGAAGGTGGTACTTTTTATGCATATGGAATTCTCGGGTGCCAAGGGCTAAATCTCATGGTAAA |
| OC43-Probe II.92 | AY391777.1 | OC43-Probe II | GTATTAGAGTTTCCCCTCTAATACGACTCACTATAGGGTGTGAATGCTGATTATTTGTATTTTCATTTTTATCAAGAAGGTGGTACTTTTTATGCATATTTTACAGACACTGGTGTTGTTACTAAGTTTGGAATTCTCGGGTGCCAAGGGCTAAATCTCATGGTAAA |
| OC43-Probe II.93 | AY391777.1 | OC43-Probe II | GTATTAGAGTTTCCCCTCTAATACGACTCACTATAGGGTTATCAAGAAGGTGGTACTTTTTATGCATATTTTACAGACACTGGTGTTGTTACTAAGTTTTTGTTTAATGTTTATTTAGGCATGGCGCTTGGAATTCTCGGGTGCCAAGGGCTAAATCTCATGGTAAA |
| OC43-Probe II.94 | AY391777.1 | OC43-Probe II | GTATTAGAGTTTCCCCTCTAATACGACTCACTATAGGGTTTTACAGACACTGGTGTTGTTACTAAGTTTTTGTTTAATGTTTATTTAGGCATGGCGCTTTCACACTATTATGTCATGCCTCTGACTTGTGGAATTCTCGGGTGCCAAGGGCTAAATCTCATGGTAAA |
| OC43-Probe II.95 | AY391777.1 | OC43-Probe II | GTATTAGAGTTTCCCCTCTAATACGACTCACTATAGGGTTTGTTTAATGTTTATTTAGGCATGGCGCTTTCACACTATTATGTCATGCCTCTGACTTGTAATAGTAAGGTTAAGAATGGTTTTACTTTTGGAATTCTCGGGTGCCAAGGGCTAAATCTCATGGTAAA |
| OC43-Probe II.96 | AY391777.1 | OC43-Probe II | GTATTAGAGTTTCCCCTCTAATACGACTCACTATAGGGTTCACACTATTATGTCATGCCTCTGACTTGTAATAGTAAGGTTAAGAATGGTTTTACTTTTAGAATATTGGGTTACACCTCTCACTTCTAGTGGAATTCTCGGGTGCCAAGGGCTAAATCTCATGGTAAA |
| OC43-Probe II.97 | AY391777.1 | OC43-Probe II | GTATTAGAGTTTCCCCTCTAATACGACTCACTATAGGGTAATAGTAAGGTTAAGAATGGTTTTACTTTTAGAATATTGGGTTACACCTCTCACTTCTAGACAATATTTACTCGCTTTCAATCAAGATGGTGGAATTCTCGGGTGCCAAGGGCTAAATCTCATGGTAAA |
| OC43-Probe II.98 | AY391777.1 | OC43-Probe II | GTATTAGAGTTTCCCCTCTAATACGACTCACTATAGGGAGAATATTGGGTTACACCTCTCACTTCTAGACAATATTTACTCGCTTTCAATCAAGATGGTATTATTTTTAATGCTGTTGATTGTATGAGTGGAATTCTCGGGTGCCAAGGGCTAAATCTCATGGTAAA |
| OC43-Probe II.99 | AY391777.1 | OC43-Probe II | GTATTAGAGTTTCCCCTCTAATACGACTCACTATAGGGACAATATTTACTCGCTTTCAATCAAGATGGTATTATTTTTAATGCTGTTGATTGTATGAGTGATTTTATGAGTGAGATTAAGTGTAAAACTGGAATTCTCGGGTGCCAAGGGCTAAATCTCATGGTAAA |
| OC43-Probe II.100 | AY391777.1 | OC43-Probe II | GTATTAGAGTTTCCCCTCTAATACGACTCACTATAGGGTATTATTTTTAATGCTGTTGATTGTATGAGTGATTTTATGAGTGAGATTAAGTGTAAAACACAATCTATAGCACCACCTACTGGTGTTTATGGAATTCTCGGGTGCCAAGGGCTAAATCTCATGGTAAA |
| OC43-Probe II.101 | AY391777.1 | OC43-Probe II | GTATTAGAGTTTCCCCTCTAATACGACTCACTATAGGGTGATTTTATGAGTGAGATTAAGTGTAAAACACAATCTATAGCACCACCTACTGGTGTTTATGAATTAAACGGTTACACTGTTCAGCCAATTGGAATTCTCGGGTGCCAAGGGCTAAATCTCATGGTAAA |
| OC43-Probe II.102 | AY391777.1 | OC43-Probe II | GTATTAGAGTTTCCCCTCTAATACGACTCACTATAGGGACAATCTATAGCACCACCTACTGGTGTTTATGAATTAAACGGTTACACTGTTCAGCCAATCGCAGATGTTTACCGACGTAAACTTAATCTTGGAATTCTCGGGTGCCAAGGGCTAAATCTCATGGTAAA |
| OC43-Probe II.103 | AY391777.1 | OC43-Probe II | GTATTAGAGTTTCCCCTCTAATACGACTCACTATAGGGTGAATTAAACGGTTACACTGTTCAGCCAATCGCAGATGTTTACCGACGTAAACTTAATCTTCCCAATTGCAATATAGAAGCTTGGCTTAATGGAATTCTCGGGTGCCAAGGGCTAAATCTCATGGTAAA |

| ASO ID | Accession | Group ID | Custom Array sequence |
|---|---|---|---|
| OC43-Probe II.104 | AY391777.1 | OC43-Probe II | GTATTAGAGTTTCCCCTCTAATACGACTCACTATAGGGCGCAGATGTTTACCGACGTAAACTTAATCTTCCCAATTGCAATATAGAAGCTTGGCTTAATGATAAGTCGGTGCCCTCTCCATTAAATTGTGGAATTCTCGGGTGCCAAGGGCTAAATCTCATGGTAAA |
| OC43-Probe II.105 | AY391777.1 | OC43-Probe II | GTATTAGAGTTTCCCCTCTAATACGACTCACTATAGGGTCCCAATTGCAATATAGAAGCTTGGCTTAATGATAAGTCGGTGCCCTCTCCATTAAATTGGGAACGTAAGACATTTTCAAATTGTAATTTTGGAATTCTCGGGTGCCAAGGGCTAAATCTCATGGTAAA |
| OC43-Probe II.106 | AY391777.1 | OC43-Probe II | GTATTAGAGTTTCCCCTCTAATACGACTCACTATAGGGTGATAAGTCGGTGCCCTCTCCATTAAATTGGGAACGTAAGACATTTTCAAATTGTAATTTTAATATGAGCAGCCTGATGTCTTTTATTCATGGAATTCTCGGGTGCCAAGGGCTAAATCTCATGGTAAA |
| OC43-Probe II.107 | AY391777.1 | OC43-Probe II | GTATTAGAGTTTCCCCTCTAATACGACTCACTATAGGGGGAACGTAAGACATTTTCAAATTGTAATTTTAATATGAGCAGCCTGATGTCTTTTATTCAGGCAGACTCATTTACTTGTAATAATATTGATGGAATTCTCGGGTGCCAAGGGCTAAATCTCATGGTAAA |
| OC43-Probe II.108 | AY391777.1 | OC43-Probe II | GTATTAGAGTTTCCCCTCTAATACGACTCACTATAGGGTAATATGAGCAGCCTGATGTCTTTTATTCAGGCAGACTCATTTACTTGTAATAATATTGATGCTGCTAAGATATATGGTATGTGTTTTTCTGGAATTCTCGGGTGCCAAGGGCTAAATCTCATGGTAAA |
| OC43-Probe II.109 | AY391777.1 | OC43-Probe II | GTATTAGAGTTTCCCCTCTAATACGACTCACTATAGGGGGCAGACTCATTTACTTGTAATAATATTGATGCTGCTAAGATATATGGTATGTGTTTTTCCAGCATAACTATAGATAAGTTTGCTATACCTGGAATTCTCGGGTGCCAAGGGCTAAATCTCATGGTAAA |
| OC43-Probe II.110 | AY391777.1 | OC43-Probe II | GTATTAGAGTTTCCCCTCTAATACGACTCACTATAGGGTGCTGCTAAGATATATGGTATGTGTTTTTCCAGCATAACTATAGATAAGTTTGCTATACCCAATGGCAGGAAGGTTGACCTACAATTGGGTGGAATTCTCGGGTGCCAAGGGCTAAATCTCATGGTAAA |
| OC43-Probe II.111 | AY391777.1 | OC43-Probe II | GTATTAGAGTTTCCCCTCTAATACGACTCACTATAGGGCAGCATAACTATAGATAAGTTTGCTATACCCAATGGCAGGAAGGTTGACCTACAATTGGGTAATTTGGGCTATTTGCAGTCATTTAACTATGGAATTCTCGGGTGCCAAGGGCTAAATCTCATGGTAAA |
| OC43-Probe II.112 | AY391777.1 | OC43-Probe II | GTATTAGAGTTTCCCCTCTAATACGACTCACTATAGGGCAATGGCAGGAAGGTTGACCTACAATTGGGTAATTTGGGCTATTTGCAGTCATTTAACTATAGAATTGATACTACTGCAACAAGTTGTCATGGAATTCTCGGGTGCCAAGGGCTAAATCTCATGGTAAA |
| OC43-Probe II.113 | AY391777.1 | OC43-Probe II | GTATTAGAGTTTCCCCTCTAATACGACTCACTATAGGGTAATTTGGGCTATTTGCAGTCATTTAACTATAGAATTGATACTACTGCAACAAGTTGTCAGTTGTATTATAATTTACCTGCTGCTAATGTTGGAATTCTCGGGTGCCAAGGGCTAAATCTCATGGTAAA |
| OC43-Probe II.114 | AY391777.1 | OC43-Probe II | GTATTAGAGTTTCCCCTCTAATACGACTCACTATAGGGTAGAATTGATACTACTGCAACAAGTTGTCAGTTGTATTATAATTTACCTGCTGCTAATGTTTCTGTTAGCAGGTTTAATCCTTCTACTTGTGGAATTCTCGGGTGCCAAGGGCTAAATCTCATGGTAAA |
| OC43-Probe II.115 | AY391777.1 | OC43-Probe II | GTATTAGAGTTTCCCCTCTAATACGACTCACTATAGGGGTTGTATTATAATTTACCTGCTGCTAATGTTTCTGTTAGCAGGTTTAATCCTTCTACTTGGAATAAGAGATTTGGTTTTATAGAAGATTCTGGAATTCTCGGGTGCCAAGGGCTAAATCTCATGGTAAA |
| OC43-Probe II.116 | AY391777.1 | OC43-Probe II | GTATTAGAGTTTCCCCTCTAATACGACTCACTATAGGGTTCTGTTAGCAGGTTTAATCCTTCTACTTGGAATAAGAGATTTGGTTTTATAGAAGATTCTGTTTTTAAGCCTCGACCTGCAGGTGTTCTTGGAATTCTCGGGTGCCAAGGGCTAAATCTCATGGTAAA |
| OC43-Probe II.117 | AY391777.1 | OC43-Probe II | GTATTAGAGTTTCCCCTCTAATACGACTCACTATAGGGGAATAAGAGATTTGGTTTTATAGAAGATTCTGTTTTTAAGCCTCGACCTGCAGGTGTTCTTACTAATCATGATGTAGTTTATGCACAACATGGAATTCTCGGGTGCCAAGGGCTAAATCTCATGGTAAA |
| OC43-Probe II.118 | AY391777.1 | OC43-Probe II | GTATTAGAGTTTCCCCTCTAATACGACTCACTATAGGGTGTTTTTAAGCCTCGACCTGCAGGTGTTCTTACTAATCATGATGTAGTTTATGCACAACACTGTTTCAAAGCTCCTAAAAAATTTCTGTCCTGGAATTCTCGGGTGCCAAGGGCTAAATCTCATGGTAAA |
| OC43-Probe II.119 | AY391777.1 | OC43-Probe II | GTATTAGAGTTTCCCCTCTAATACGACTCACTATAGGGTACTAATCATGATGTAGTTTATGCACAACACTGTTTCAAAGCTCCTAAAAAATTTCTGTCCGTGTAAATTGAATGGTTCGTGTGTAGGTAGTGGAATTCTCGGGTGCCAAGGGCTAAATCTCATGGTAAA |
| OC43-Probe II.120 | AY391777.1 | OC43-Probe II | GTATTAGAGTTTCCCCTCTAATACGACTCACTATAGGGCTGTTTCAAAGCTCCTAAAAAATTTCTGTCCGTGTAAATTGAATGGTTCGTGTGTAGGTAGTGGTCCTGGTAAAAATAATGGTATAGGCACTGGAATTCTCGGGTGCCAAGGGCTAAATCTCATGGTAAA |
| OC43-Probe II.121 | AY391777.1 | OC43-Probe II | GTATTAGAGTTTCCCCTCTAATACGACTCACTATAGGGGTGTAAATTGAATGGTTCGTGTGTAGGTAGTGGTCCTGGTAAAAATAATGGTATAGGCACTTGTCCTGCAGGTACTAATTATTTAACTTGTGGAATTCTCGGGTGCCAAGGGCTAAATCTCATGGTAAA |
| OC43-Probe II.122 | AY391777.1 | OC43-Probe II | GTATTAGAGTTTCCCCTCTAATACGACTCACTATAGGGTGGTCCTGGTAAAAATAATGGTATAGGCACTTGTCCTGCAGGTACTAATTATTTAACTTGTGATAATTTGTGCACTCCTGATCCTATTACTGGAATTCTCGGGTGCCAAGGGCTAAATCTCATGGTAAA |
| OC43-Probe II.123 | AY391777.1 | OC43-Probe II | GTATTAGAGTTTCCCCTCTAATACGACTCACTATAGGGTTGTCCTGCAGGTACTAATTATTTAACTTGTGATAATTTGTGCACTCCTGATCCTATTACATTTAAAGCTACAGGTACTTATAAGTGCCCTGGAATTCTCGGGTGCCAAGGGCTAAATCTCATGGTAAA |
| OC43-Probe II.124 | AY391777.1 | OC43-Probe II | GTATTAGAGTTTCCCCTCTAATACGACTCACTATAGGGTGATAATTTGTGCACTCCTGATCCTATTACATTTAAAGCTACAGGTACTTATAAGTGCCCCCAAACTAAATCTTTAGTTGGCATAGGTGATGGAATTCTCGGGTGCCAAGGGCTAAATCTCATGGTAAA |
| OC43-Probe II.125 | AY391777.1 | OC43-Probe II | GTATTAGAGTTTCCCCTCTAATACGACTCACTATAGGGATTTAAAGCTACAGGTACTTATAAGTGCCCCCAAACTAAATCTTTAGTTGGCATAGGTGAGCACTGTTCGGGTCTTGCTGTTAAAAGTGATGGAATTCTCGGGTGCCAAGGGCTAAATCTCATGGTAAA |
| OC43-Probe II.126 | AY391777.1 | OC43-Probe II | GTATTAGAGTTTCCCCTCTAATACGACTCACTATAGGGCCAAACTAAATCTTTAGTTGGCATAGGTGAGCACTGTTCGGGTCTTGCTGTTAAAAGTGATTATTGTGGAGGCAATTCTTGTACTTGCCGTGGAATTCTCGGGTGCCAAGGGCTAAATCTCATGGTAAA |
| OC43-Probe II.127 | AY391777.1 | OC43-Probe II | GTATTAGAGTTTCCCCTCTAATACGACTCACTATAGGGGCACTGTTCGGGTCTTGCTGTTAAAAGTGATTATTGTGGAGGCAATTCTTGTACTTGCCGACCACAAGCATTTTTGGGTTGGTCTGCAGATGGAATTCTCGGGTGCCAAGGGCTAAATCTCATGGTAAA |
| OC43-Probe II.128 | AY391777.1 | OC43-Probe II | GTATTAGAGTTTCCCCTCTAATACGACTCACTATAGGGTTATTGTGGAGGCAATTCTTGTACTTGCCGACCACAAGCATTTTTGGGTTGGTCTGCAGACTCTTGTTTACAAGGAGACAAGTGTAATATTGGAATTCTCGGGTGCCAAGGGCTAAATCTCATGGTAAA |

94

| ASO ID | Accession | Group ID | Custom Array sequence |
|---|---|---|---|
| OC43-Probe II.129 | AY391777.1 | OC43-Probe II | GTATTAGAGTTTCCCCTCTAATACGACTCACTATAGGGACCACAAGCATTTTTGGGTTGGTCTGCAGACTCTTGTTTACAAGGAGACAAGTGTAATATTTTTGCTAATTTTATTTTGCATGATGTTAATGGAATTCTCGGGTGCCAAGGGCTAAATCTCATGGTAAA |
| OC43-Probe II.130 | AY391777.1 | OC43-Probe II | GTATTAGAGTTTCCCCTCTAATACGACTCACTATAGGGCTCTTGTTTACAAGGAGACAAGTGTAATATTTTTGCTAATTTTATTTTGCATGATGTTAATAGTGGTCTTACTTGTTCTACTGATTTACATGGAATTCTCGGGTGCCAAGGGCTAAATCTCATGGTAAA |
| OC43-Probe II.131 | AY391777.1 | OC43-Probe II | GTATTAGAGTTTCCCCTCTAATACGACTCACTATAGGGTTTTGCTAATTTTATTTTGCATGATGTTAATAGTGGTCTTACTTGTTCTACTGATTTACAAAAAGCTAACACAGACATAATTCTTGGTGTTGGAATTCTCGGGTGCCAAGGGCTAAATCTCATGGTAAA |
| OC43-Probe II.132 | AY391777.1 | OC43-Probe II | GTATTAGAGTTTCCCCTCTAATACGACTCACTATAGGGTAGTGGTCTTACTTGTTCTACTGATTTACAAAAAGCTAACACAGACATAATTCTTGGTGTTTGTGTTAATTATGACCTCTATGGTATTTTTGGAATTCTCGGGTGCCAAGGGCTAAATCTCATGGTAAA |
| OC43-Probe II.133 | AY391777.1 | OC43-Probe II | GTATTAGAGTTTCCCCTCTAATACGACTCACTATAGGGAAAAGCTAACACAGACATAATTCTTGGTGTTTGTGTTAATTATGACCTCTATGGTATTTTTAGGCCAAGGCATTTTTGTTGAGGTTAATGCTGGAATTCTCGGGTGCCAAGGGCTAAATCTCATGGTAAA |
| OC43-Probe II.134 | AY391777.1 | OC43-Probe II | GTATTAGAGTTTCCCCTCTAATACGACTCACTATAGGGTTGTGTTAATTATGACCTCTATGGTATTTTTAGGCCAAGGCATTTTTGTTGAGGTTAATGCGACTTATTATAATAGTTGGCAGAACCTTTTTGGAATTCTCGGGTGCCAAGGGCTAAATCTCATGGTAAA |
| OC43-Probe II.135 | AY391777.1 | OC43-Probe II | GTATTAGAGTTTCCCCTCTAATACGACTCACTATAGGGAGGCCAAGGCATTTTTGTTGAGGTTAATGCGACTTATTATAATAGTTGGCAGAACCTTTTATATGATTCTAATGGTAATCTCTACGGTTTTGGAATTCTCGGGTGCCAAGGGCTAAATCTCATGGTAAA |
| OC43-Probe II.136 | AY391777.1 | OC43-Probe II | GTATTAGAGTTTCCCCTCTAATACGACTCACTATAGGGGACTTATTATAATAGTTGGCAGAACCTTTTATATGATTCTAATGGTAATCTCTACGGTTTTAGAGACTACATAACAAACAGAACTTTTATTGGAATTCTCGGGTGCCAAGGGCTAAATCTCATGGTAAA |
| OC43-Probe II.137 | AY391777.1 | OC43-Probe II | GTATTAGAGTTTCCCCTCTAATACGACTCACTATAGGGATATGATTCTAATGGTAATCTCTACGGTTTTAGAGACTACATAACAAACAGAACTTTTATGATTCGTAGTTGCTATAGCGGTCGTGTTTCTGGAATTCTCGGGTGCCAAGGGCTAAATCTCATGGTAAA |
| OC43-Probe II.138 | AY391777.1 | OC43-Probe II | GTATTAGAGTTTCCCCTCTAATACGACTCACTATAGGGTAGAGACTACATAACAAACAGAACTTTTATGATTCGTAGTTGCTATAGCGGTCGTGTTTCTGCGGCCTTTCACGCTAACTCTTCCGAACCTGGAATTCTCGGGTGCCAAGGGCTAAATCTCATGGTAAA |
| OC43-Probe II.139 | AY391777.1 | OC43-Probe II | GTATTAGAGTTTCCCCTCTAATACGACTCACTATAGGGGATTCGTAGTTGCTATAGCGGTCGTGTTTCTGCGGCCTTTCACGCTAACTCTTCCGAACCAGCATTGCTATTTCGGAATATTAAATGCAATGGAATTCTCGGGTGCCAAGGGCTAAATCTCATGGTAAA |
| OC43-Probe II.140 | AY391777.1 | OC43-Probe II | GTATTAGAGTTTCCCCTCTAATACGACTCACTATAGGGTGCGGCCTTTCACGCTAACTCTTCCGAACCAGCATTGCTATTTCGGAATATTAAATGCAACTACGTTTTTAATAATAGTCTTACACGACATGGAATTCTCGGGTGCCAAGGGCTAAATCTCATGGTAAA |
| OC43-Probe II.141 | AY391777.1 | OC43-Probe II | GTATTAGAGTTTCCCCTCTAATACGACTCACTATAGGGAGCATTGCTATTTCGGAATATTAAATGCAACTACGTTTTTAATAATAGTCTTACACGACAGCTGCAACCCATTAACTATTTTGATAGTTATGGAATTCTCGGGTGCCAAGGGCTAAATCTCATGGTAAA |
| OC43-Probe II.142 | AY391777.1 | OC43-Probe II | GTATTAGAGTTTCCCCTCTAATACGACTCACTATAGGGCTACGTTTTTAATAATAGTCTTACACGACAGCTGCAACCCATTAACTATTTTGATAGTTATCTTGGTTGTGTTGTCAATGCTTATAATAGTGGAATTCTCGGGTGCCAAGGGCTAAATCTCATGGTAAA |
| OC43-Probe II.143 | AY391777.1 | OC43-Probe II | GTATTAGAGTTTCCCCTCTAATACGACTCACTATAGGGGCTGCAACCCATTAACTATTTTGATAGTTATCTTGGTTGTGTTGTCAATGCTTATAATAGTACTGCTATTTCTGTTCAAACATGTGATCTTGGAATTCTCGGGTGCCAAGGGCTAAATCTCATGGTAAA |
| OC43-Probe II.144 | AY391777.1 | OC43-Probe II | GTATTAGAGTTTCCCCTCTAATACGACTCACTATAGGGTCTTGGTTGTGTTGTCAATGCTTATAATAGTACTGCTATTTCTGTTCAAACATGTGATCTCACAGTAGGTAGTGGTTACTGTGTGGATTATGGAATTCTCGGGTGCCAAGGGCTAAATCTCATGGTAAA |
| OC43-Probe II.145 | AY391777.1 | OC43-Probe II | GTATTAGAGTTTCCCCTCTAATACGACTCACTATAGGGTACTGCTATTTCTGTTCAAACATGTGATCTCACAGTAGGTAGTGGTTACTGTGTGGATTACTCTAAAAACAGACGAAGTCGTGGAGCGATTGGAATTCTCGGGTGCCAAGGGCTAAATCTCATGGTAAA |
| OC43-Probe II.146 | AY391777.1 | OC43-Probe II | GTATTAGAGTTTCCCCTCTAATACGACTCACTATAGGGCACAGTAGGTAGTGGTTACTGTGTGGATTACTCTAAAAACAGACGAAGTCGTGGAGCGATTACCACTGGTTATCGGTTTACTAATTTTGATGGAATTCTCGGGTGCCAAGGGCTAAATCTCATGGTAAA |
| OC43-Probe II.147 | AY391777.1 | OC43-Probe II | GTATTAGAGTTTCCCCTCTAATACGACTCACTATAGGGCTCTAAAAACAGACGAAGTCGTGGAGCGATTACCACTGGTTATCGGTTTACTAATTTTGAGCCATTTACTGTTAATTCAGTAAACGATAGTGGAATTCTCGGGTGCCAAGGGCTAAATCTCATGGTAAA |
| OC43-Probe II.148 | AY391777.1 | OC43-Probe II | GTATTAGAGTTTCCCCTCTAATACGACTCACTATAGGGTACCACTGGTTATCGGTTTACTAATTTTGAGCCATTTACTGTTAATTCAGTAAACGATAGTTTAGAACCTGTAGGTGGTTTGTATGAAATTGGAATTCTCGGGTGCCAAGGGCTAAATCTCATGGTAAA |
| OC43-Probe II.149 | AY391777.1 | OC43-Probe II | GTATTAGAGTTTCCCCTCTAATACGACTCACTATAGGGGCCATTTACTGTTAATTCAGTAAACGATAGTTTAGAACCTGTAGGTGGTTTGTATGAAATTCAAATACCTTCAGAGTTTACTATAGGTAATGGAATTCTCGGGTGCCAAGGGCTAAATCTCATGGTAAA |
| OC43-Probe II.150 | AY391777.1 | OC43-Probe II | GTATTAGAGTTTCCCCTCTAATACGACTCACTATAGGGTTTAGAACCTGTAGGTGGTTTGTATGAAATTCAAATACCTTCAGAGTTTACTATAGGTAATATGGAGGAGTTTATTCAAACAAGCTCCTGGAATTCTCGGGTGCCAAGGGCTAAATCTCATGGTAAA |
| OC43-Probe II.151 | AY391777.1 | OC43-Probe II | GTATTAGAGTTTCCCCTCTAATACGACTCACTATAGGGTCAAATACCTTCAGAGTTTACTATAGGTAATATGGAGGAGTTTATTCAAACAAGCTCTCCTAAAGTTACTATTGATTGTGCTGCATTTGTTGGAATTCTCGGGTGCCAAGGGCTAAATCTCATGGTAAA |
| OC43-Probe II.152 | AY391777.1 | OC43-Probe II | GTATTAGAGTTTCCCCTCTAATACGACTCACTATAGGGTATGGAGGAGTTTATTCAAACAAGCTCTCCTAAAGTTACTATTGATTGTGCTGCATTTGTCTGTGGTGATTATGCAGCATGTAAATCACATGGAATTCTCGGGTGCCAAGGGCTAAATCTCATGGTAAA |
| OC43-Probe II.153 | AY391777.1 | OC43-Probe II | GTATTAGAGTTTCCCCTCTAATACGACTCACTATAGGGTAAAGTTACTATTGATTGTGCTGCATTTGTCTGTGGTGATTATGCAGCATGTAAATCACAGTTGGTTGAATATGGTAGTTTCTGTGATAATGGAATTCTCGGGTGCCAAGGGCTAAATCTCATGGTAAA |

| ASO ID | Accession | Group ID | Custom Array sequence |
|---|---|---|---|
| OC43-Probe II.154 | AY391777.1 | OC43-Probe II | GTATTAGAGTTTCCCCTCTAATACGACTCACTATAGGGCTGTGGTGATTATGCAGCATGTAAATCACAGTTGGTTGAATATGGTAGTTTCTGTGATAACATTAATGCCATACTCACAGAAGTAAATGATGGAATTCTCGGGTGCCAAGGGCTAAATCTCATGGTAAA |
| OC43-Probe II.155 | AY391777.1 | OC43-Probe II | GTATTAGAGTTTCCCCTCTAATACGACTCACTATAGGGGTTGGTTGAATATGGTAGTTTCTGTGATAACATTAATGCCATACTCACAGAAGTAAATGAACTACTTGACACTACACAGTTGCAAGTAGCTGGAATTCTCGGGTGCCAAGGGCTAAATCTCATGGTAAA |
| OC43-Probe II.156 | AY391777.1 | OC43-Probe II | GTATTAGAGTTTCCCCTCTAATACGACTCACTATAGGGCATTAATGCCATACTCACAGAAGTAAATGAACTACTTGACACTACACAGTTGCAAGTAGCTAATAGTTTAATGAATGGTGTTACTCTTAGTGGAATTCTCGGGTGCCAAGGGCTAAATCTCATGGTAAA |
| OC43-Probe II.157 | AY391777.1 | OC43-Probe II | GTATTAGAGTTTCCCCTCTAATACGACTCACTATAGGGACTACTTGACACTACACAGTTGCAAGTAGCTAATAGTTTAATGAATGGTGTTACTCTTAGCACTAAGCTTAAAGATGGCGTTAATTTCAATGGAATTCTCGGGTGCCAAGGGCTAAATCTCATGGTAAA |
| OC43-Probe II.158 | AY391777.1 | OC43-Probe II | GTATTAGAGTTTCCCCTCTAATACGACTCACTATAGGGTAATAGTTTAATGAATGGTGTTACTCTTAGCACTAAGCTTAAAGATGGCGTTAATTTCAATGTAGACGACATCAATTTTTCCCCTGTATTTGGAATTCTCGGGTGCCAAGGGCTAAATCTCATGGTAAA |
| OC43-Probe II.159 | AY391777.1 | OC43-Probe II | GTATTAGAGTTTCCCCTCTAATACGACTCACTATAGGGCACTAAGCTTAAAGATGGCGTTAATTTCAATGTAGACGACATCAATTTTTCCCCTGTATTAGGTTGTCTAGGCAGCGAATGTAGTAAAGCTGGAATTCTCGGGTGCCAAGGGCTAAATCTCATGGTAAA |
| OC43-Probe II.160 | AY391777.1 | OC43-Probe II | GTATTAGAGTTTCCCCTCTAATACGACTCACTATAGGGTGTGACGACATCAATTTTTCCCCTGTATTAGGTTGTCTAGGCAGCGAATGTAGTAAAGCTTCCAGTAGATCTGCTATAGAGGATTTACTTGGAATTCTCGGGTGCCAAGGGCTAAATCTCATGGTAAA |
| OC43-Probe II.161 | AY391777.1 | OC43-Probe II | GTATTAGAGTTTCCCCTCTAATACGACTCACTATAGGGAGGTTGTCTAGGCAGCGAATGTAGTAAAGCTTCCAGTAGATCTGCTATAGAGGATTTACTTTTTGATAAAGTAAAGTTATCTGATGTCGGTGGAATTCTCGGGTGCCAAGGGCTAAATCTCATGGTAAA |
| OC43-Probe II.162 | AY391777.1 | OC43-Probe II | GTATTAGAGTTTCCCCTCTAATACGACTCACTATAGGGTTCCAGTAGATCTGCTATAGAGGATTTACTTTTTGATAAAGTAAAGTTATCTGATGTCGGTTTTGTTGAGGCTTATAATAATTGTACAGGTGGAATTCTCGGGTGCCAAGGGCTAAATCTCATGGTAAA |
| OC43-Probe II.163 | AY391777.1 | OC43-Probe II | GTATTAGAGTTTCCCCTCTAATACGACTCACTATAGGGTTTTGATAAAGTAAAGTTATCTGATGTCGGTTTTGTTGAGGCTTATAATAATTGTACAGGAGGTGCCGAAATTAGGGACCTCATTTGTGTTGGAATTCTCGGGTGCCAAGGGCTAAATCTCATGGTAAA |
| OC43-Probe II.164 | AY391777.1 | OC43-Probe II | GTATTAGAGTTTCCCCTCTAATACGACTCACTATAGGGTTTTGTTGAGGCTTATAATAATTGTACAGGAGGTGCCGAAATTAGGGACCTCATTTGTGTGCAAAGTTATAAAGGCATCAAAGTGTTGCCTGGAATTCTCGGGTGCCAAGGGCTAAATCTCATGGTAAA |
| OC43-Probe II.165 | AY391777.1 | OC43-Probe II | GTATTAGAGTTTCCCCTCTAATACGACTCACTATAGGGAGGTGCCGAAATTAGGGACCTCATTTGTGTGCAAAGTTATAAAGGCATCAAAGTGTTGCCTCCACTGCTCTCAGAAAATCAGATCAGTGGTGGAATTCTCGGGTGCCAAGGGCTAAATCTCATGGTAAA |
| OC43-Probe II.166 | AY391777.1 | OC43-Probe II | GTATTAGAGTTTCCCCTCTAATACGACTCACTATAGGGCAAAGTTATAAAGGCATCAAAGTGTTGCCTCCACTGCTCTCAGAAAATCAGATCAGTGGATACACTTTGGCTGCCACCTCTGCTAGTCTTGGAATTCTCGGGTGCCAAGGGCTAAATCTCATGGTAAA |
| OC43-Probe II.167 | AY391777.1 | OC43-Probe II | GTATTAGAGTTTCCCCTCTAATACGACTCACTATAGGGTCCACTGCTCTCAGAAAATCAGATCAGTGGATACACTTTGGCTGCCACCTCTGCTAGTCTATTTCCTCTTTGGACAGCAGCAGCAGGTGTTGGAATTCTCGGGTGCCAAGGGCTAAATCTCATGGTAAA |
| OC43-Probe II.168 | AY391777.1 | OC43-Probe II | GTATTAGAGTTTCCCCTCTAATACGACTCACTATAGGGATACACTTTGGCTGCCACCTCTGCTAGTCTATTTCCTCTTTGGACAGCAGCAGCAGGTGTACCATTTTATTTAAATGTTCAGTATCGCATTGGAATTCTCGGGTGCCAAGGGCTAAATCTCATGGTAAA |
| OC43-Probe II.169 | AY391777.1 | OC43-Probe II | GTATTAGAGTTTCCCCTCTAATACGACTCACTATAGGGATTTCCTCTTTGGACAGCAGCAGCAGGTGTACCATTTTATTTAAATGTTCAGTATCGCATTAATGGGCTTGGTGTCACCATGGATGTGCTTGGAATTCTCGGGTGCCAAGGGCTAAATCTCATGGTAAA |
| OC43-Probe II.170 | AY391777.1 | OC43-Probe II | GTATTAGAGTTTCCCCTCTAATACGACTCACTATAGGGACCATTTTATTTAAATGTTCAGTATCGCATTAATGGGCTTGGTGTCACCATGGATGTGCTAAGTCAAAATCAAAAGCTTATTGCTAATGCTGGAATTCTCGGGTGCCAAGGGCTAAATCTCATGGTAAA |
| OC43-Probe II.171 | AY391777.1 | OC43-Probe II | GTATTAGAGTTTCCCCTCTAATACGACTCACTATAGGGTAATGGGCTTGGTGTCACCATGGATGTGCTAAGTCAAAATCAAAAGCTTATTGCTAATGCATTTAACAATGCCCTTTATGCTATTCAGGATGGAATTCTCGGGTGCCAAGGGCTAAATCTCATGGTAAA |
| OC43-Probe II.172 | AY391777.1 | OC43-Probe II | GTATTAGAGTTTCCCCTCTAATACGACTCACTATAGGGAAGTCAAAATCAAAAGCTTATTGCTAATGCATTTAACAATGCCCTTTATGCTATTCAGGAAGGGTTCGATGCAACTAATTCTGCTTTAGTTGGAATTCTCGGGTGCCAAGGGCTAAATCTCATGGTAAA |
| OC43-Probe II.173 | AY391777.1 | OC43-Probe II | GTATTAGAGTTTCCCCTCTAATACGACTCACTATAGGGATTTAACAATGCCCTTTATGCTATTCAGGAAGGGTTCGATGCAACTAATTCTGCTTTAGTTAAAATTCAAGCTGTTGTTAATGCAAATGCTGGAATTCTCGGGTGCCAAGGGCTAAATCTCATGGTAAA |
| OC43-Probe II.174 | AY391777.1 | OC43-Probe II | GTATTAGAGTTTCCCCTCTAATACGACTCACTATAGGGAGGGGTTCGATGCAACTAATTCTGCTTTAGTTAAAATTCAAGCTGTTGTTAATGCAAATGCTGAAGCTCTTAATAACTTATTGCAACAACTTGGAATTCTCGGGTGCCAAGGGCTAAATCTCATGGTAAA |
| OC43-Probe II.175 | AY391777.1 | OC43-Probe II | GTATTAGAGTTTCCCCTCTAATACGACTCACTATAGGGTAAAATTCAAGCTGTTGTTAATGCAAATGCTGAAGCTCTTAATAACTTATTGCAACAACTCTCTAATAGATTTGGTGCTATAAAGTGCTTCTGGAATTCTCGGGTGCCAAGGGCTAAATCTCATGGTAAA |
| OC43-Probe II.176 | AY391777.1 | OC43-Probe II | GTATTAGAGTTTCCCCTCTAATACGACTCACTATAGGGTGAAGCTCTTAATAACTTATTGCAACAACTCTCTAATAGATTTGGTGCTATAAGTGCTTCTTTACAAGAAATTCTATCTAGACTTGATGCTGGAATTCTCGGGTGCCAAGGGCTAAATCTCATGGTAAA |
| OC43-Probe II.177 | AY391777.1 | OC43-Probe II | GTATTAGAGTTTCCCCTCTAATACGACTCACTATAGGGCTCTAATAGATTTGGTGCTATAAGTGCTTCTTTACAAGAAATTCTATCTAGACTTGATGCTCTTGAAGCGGAAGCTCAGATAGATAGACTTGGAATTCTCGGGTGCCAAGGGCTAAATCTCATGGTAAA |
| OC43-Probe II.178 | AY391777.1 | OC43-Probe II | GTATTAGAGTTTCCCCTCTAATACGACTCACTATAGGGTTTACAAGAAATTCTATCTAGACTTGATGCTCTTGAAGCGGAAGCTCAGATAGATAGACTTATTAATGGTCGTCTTACCGCTCTTAATGCTGGAATTCTCGGGTGCCAAGGGCTAAATCTCATGGTAAA |

| ASO ID | Accession | Group ID | Custom Array sequence |
|---|---|---|---|
| OC43-Probe II.179 | AY391777.1 | OC43-Probe II | GTATTAGAGTTTCCCCTCTAATACGACTCACTATAGGGTCTTGAAGCGGAAGCTCAGATAGATAGACTTATTAATGGTCGTCTTACCGCTCTTAATGCTTATGTTTCTCAACAGCTTAGTGATTCTACTGGAATTCTCGGGTGCCAAGGGCTAAATCTCATGGTAAA |
| OC43-Probe II.180 | AY391777.1 | OC43-Probe II | GTATTAGAGTTTCCCCTCTAATACGACTCACTATAGGGTATTAATGGTCGTCTTACCGCTCTTAATGCTTATGTTTCTCAACAGCTTAGTGATTCTACACTGGTAAAATTTAGTGCAGCACAAGCTATTGGAATTCTCGGGTGCCAAGGGCTAAATCTCATGGTAAA |
| OC43-Probe II.181 | AY391777.1 | OC43-Probe II | GTATTAGAGTTTCCCCTCTAATACGACTCACTATAGGGTTATGTTTCTCAACAGCTTAGTGATTCTACACTGGTAAAATTTAGTGCAGCACAAGCTATGGAGAAGGTTAATGAATGTGTCAAAAGCCATGGAATTCTCGGGTGCCAAGGGCTAAATCTCATGGTAAA |
| OC43-Probe II.182 | AY391777.1 | OC43-Probe II | GTATTAGAGTTTCCCCTCTAATACGACTCACTATAGGGACTGGTAAAATTTAGTGCAGCACAAGCTATGGAGAAGGTTAATGAATGTGTCAAAAGCCAATCATCTAGGATAAATTTCTGTGGTAATGGTGGAATTCTCGGGTGCCAAGGGCTAAATCTCATGGTAAA |
| OC43-Probe II.183 | AY391777.1 | OC43-Probe II | GTATTAGAGTTTCCCCTCTAATACGACTCACTATAGGGGGAGAAGGTTAATGAATGTGTCAAAAGCCAATCATCTAGGATAAATTTCTGTGGTAATGGTAATCATATTATATCATTAGTGCAGAATGCTGGAATTCTCGGGTGCCAAGGGCTAAATCTCATGGTAAA |
| OC43-Probe II.184 | AY391777.1 | OC43-Probe II | GTATTAGAGTTTCCCCTCTAATACGACTCACTATAGGGATCATCTAGGATAAATTTCTGTGGTAATGGTAATCATATTATATCATTAGTGCAGAATGCTCCATATGGTTTGTATTTTATCCACTTTAGTGGAATTCTCGGGTGCCAAGGGCTAAATCTCATGGTAAA |
| OC43-Probe II.185 | AY391777.1 | OC43-Probe II | GTATTAGAGTTTCCCCTCTAATACGACTCACTATAGGGTAATCATATTATATCATTAGTGCAGAATGCTCCATATGGTTTGTATTTTATCCACTTTAGTTATGTCCCTACTAAGTATGTCACAGCGAGTGGAATTCTCGGGTGCCAAGGGCTAAATCTCATGGTAAA |
| OC43-Probe II.186 | AY391777.1 | OC43-Probe II | GTATTAGAGTTTCCCCTCTAATACGACTCACTATAGGGTCCATATGGTTTGTATTTTATCCACTTTAGTTATGTCCCTACTAAGTATGTCACAGCGAGGGTTAGTCCTGGTCTGTGCATTGCTGGTGATGGAATTCTCGGGTGCCAAGGGCTAAATCTCATGGTAAA |
| OC43-Probe II.187 | AY391777.1 | OC43-Probe II | GTATTAGAGTTTCCCCTCTAATACGACTCACTATAGGGTTATGTCCCTACTAAGTATGTCACAGCGAGGGTTAGTCCTGGTCTGTGCATTGCTGGTGATAGAGGTATAGCTCCTAAGAGTGGTTATTTTGGAATTCTCGGGTGCCAAGGGCTAAATCTCATGGTAAA |
| OC43-Probe II.188 | AY391777.1 | OC43-Probe II | GTATTAGAGTTTCCCCTCTAATACGACTCACTATAGGGGGGTTAGTCCTGGTCTGTGCATTGCTGGTGATAGAGGTATAGCTCCTAAGAGTGGTTATTTTGTTAATGTAAATAATACTTGGATGTACACTGGAATTCTCGGGTGCCAAGGGCTAAATCTCATGGTAAA |
| OC43-Probe II.189 | AY391777.1 | OC43-Probe II | GTATTAGAGTTTCCCCTCTAATACGACTCACTATAGGGTAGAGGTATAGCTCCTAAGAGTGGTTATTTTGTTAATGTAAATAATACTTGGATGTACACTGGTAGTGGTTACTACTACCCTGAACCTATTGGAATTCTCGGGTGCCAAGGGCTAAATCTCATGGTAAA |
| OC43-Probe II.190 | AY391777.1 | OC43-Probe II | GTATTAGAGTTTCCCCTCTAATACGACTCACTATAGGGTGTTAATGTAAATAATACTTGGATGTACACTGGTAGTGGTTACTACTACCCTGAACCTATAACTGAAAATAATGTTGTTGTTATGGAGTACTGGAATTCTCGGGTGCCAAGGGCTAAATCTCATGGTAAA |
| OC43-Probe II.191 | AY391777.1 | OC43-Probe II | GTATTAGAGTTTCCCCTCTAATACGACTCACTATAGGGTGGTAGTGGTTACTACTACCCTGAACCTATAACTGAAAATAATGTTGTTGTTATGGAGTACCTGCGCTGTTAATTATACTAAAGCGCCGTATGGAATTCTCGGGTGCCAAGGGCTAAATCTCATGGTAAA |
| OC43-Probe II.192 | AY391777.1 | OC43-Probe II | GTATTAGAGTTTCCCCTCTAATACGACTCACTATAGGGAACTGAAAATAATGTTGTTGTTATGGAGTACCTGCGCTGTTAATTATACTAAAGCGCCGTATGTAATGCTGAACACTTCAATACCCAACCTTGGAATTCTCGGGTGCCAAGGGCTAAATCTCATGGTAAA |
| OC43-Probe II.193 | AY391777.1 | OC43-Probe II | GTATTAGAGTTTCCCCTCTAATACGACTCACTATAGGGCTGCGCTGTTAATTATACTAAAGCGCCGTATGTAATGCTGAACACTTCAATACCCAACCTTCCTGATTTTAAGGAAGAGTTGGATCAATGTGGAATTCTCGGGTGCCAAGGGCTAAATCTCATGGTAAA |
| OC43-Probe II.194 | AY391777.1 | OC43-Probe II | GTATTAGAGTTTCCCCTCTAATACGACTCACTATAGGGTGTAATGCTGAACACTTCAATACCCAACCTTCCTGATTTTAAGGAAGAGTTGGATCAATGGTTTAAAAATCAAACATCAGTGGCACCAGATGGAATTCTCGGGTGCCAAGGGCTAAATCTCATGGTAAA |
| OC43-Probe II.195 | AY391777.1 | OC43-Probe II | GTATTAGAGTTTCCCCTCTAATACGACTCACTATAGGGTCCTGATTTTAAGGAAGAGTTGGATCAATGGTTTAAAAATCAAACATCAGTGGCACCAGATTTGTCACTTGATTATATAAATGTTACATTTGGAATTCTCGGGTGCCAAGGGCTAAATCTCATGGTAAA |
| OC43-Probe II.196 | AY391777.1 | OC43-Probe II | GTATTAGAGTTTCCCCTCTAATACGACTCACTATAGGGGGTTTAAAAATCAAACATCAGTGGCACCAGATTTGTCACTTGATTATATAAATGTTACATTCTTGGACCTACAAGTTGAAATGAATAGGTTTGGAATTCTCGGGTGCCAAGGGCTAAATCTCATGGTAAA |
| OC43-Probe II.197 | AY391777.1 | OC43-Probe II | GTATTAGAGTTTCCCCTCTAATACGACTCACTATAGGGTTTGTCACTTGATTATATAAATGTTACATTCTTGGACCTACAAGTTGAAATGAATAGGTTACAGGAGGCAATAAAAGTCTTAAATCAGAGTGGAATTCTCGGGTGCCAAGGGCTAAATCTCATGGTAAA |
| OC43-Probe II.198 | AY391777.1 | OC43-Probe II | GTATTAGAGTTTCCCCTCTAATACGACTCACTATAGGGCTTGGACCTACAAGTTGAAATGAATAGGTTACAGGAGGCAATAAAAGTCTTAAATCAGAGTCACATCAATCTCAAGGACATTGGTACATATGGAATTCTCGGGTGCCAAGGGCTAAATCTCATGGTAAA |
| OC43-Probe II.199 | AY391777.1 | OC43-Probe II | GTATTAGAGTTTCCCCTCTAATACGACTCACTATAGGGACAGGAGGCAATAAAAGTCTTAAATCAGAGCTACATCAATCTCAAGGACATTGGTACATATGAATATTATGTAAAATGGCCTTGGTATGTTGGAATTCTCGGGTGCCAAGGGCTAAATCTCATGGTAAA |
| OC43-Probe II.200 | AY391777.1 | OC43-Probe II | GTATTAGAGTTTCCCCTCTAATACGACTCACTATAGGGCTACATCAATCTCAAGGACATTGGTACATATGAATATTATGTAAAATGGCCTTGGTATGTATGGCTTTTAATCTGCCTTGCTGGTGTAGCTGGAATTCTCGGGTGCCAAGGGCTAAATCTCATGGTAAA |
| OC43-Probe II.201 | AY391777.1 | OC43-Probe II | GTATTAGAGTTTCCCCTCTAATACGACTCACTATAGGGTGAATATTATGTAAAATGGCCTTGGTATGTATGGCTTTTAATCTGCCTTGCTGGTGTAGCTAGTGCTTGTTTTTACTATTCTTCATATGCTGTGGAATTCTCGGGTGCCAAGGGCTAAATCTCATGGTAAA |
| OC43-Probe II.202 | AY391777.1 | OC43-Probe II | GTATTAGAGTTTCCCCTCTAATACGACTCACTATAGGGATGGCTTTTAATCTGCCTTGCTGGTGGTAGCTATGCTTGTTTTTACTATTCTTCATATGCTGTTGTACAGGATGTGGGACTAGTTGTTTTTAATGGAATTCTCGGGTGCCAAGGGCTAAATCTCATGGTAAA |
| OC43-Probe II.203 | AY391777.1 | OC43-Probe II | GTATTAGAGTTTCCCCTCTAATACGACTCACTATAGGGTATGCTTGTTTTTACTATTCTTCATATGCTGTTGTACAGGATGTGGGACTAGTTGTTTTTAAGAAATGTGGTGGTTGTTGTGATGATTATACTGGAATTCTCGGGTGCCAAGGGCTAAATCTCATGGTAAA |

97

| ASO ID | Accession | Group ID | Custom Array sequence |
|---|---|---|---|
| OC43-Probe II.204 | AY391777.1 | OC43-Probe II | GTATTAGAGTTTCCCCTCTAATACGACTCACTATAGGGTTGTACAGGATGTGGGACTAGTTGTTTTAAGAAATGTGGTGGTTGTTGTGATGATTATACTGGATACCAGGAGTTAGTAATCAAAACTTCTGGAATTCTCGGGTGCCAAGGGCTAAATCTCATGGTAAA |
| OC43-Probe II.205 | AY391777.1 | OC43-Probe II | GTATTAGAGTTTCCCCTCTAATACGACTCACTATAGGGGAAATGTGGTGGTTGTTGTGATGATTATACTGGATACCAGGAGTTAGTAATCAAAACTTCACATGACGACTAAGTTCGTCTTTGATTCATTGGAATTCTCGGGTGCCAAGGGCTAAATCTCATGGTAAA |
| OC43-Probe II.206 | AY391777.1 | OC43-Probe II | GTATTAGAGTTTCCCCTCTAATACGACTCACTATAGGGTGGATACCAGGAGTTAGTAATCAAAACTTCACATGACGACTAAGTTCGTCTTTGATTCATTGCACTGATCTCTTGTTAGATCTTTTTGCATGGAATTCTCGGGTGCCAAGGGCTAAATCTCATGGTAAA |
| OC43-Probe II.207 | AY391777.1 | OC43-Probe II | GTATTAGAGTTTCCCCTCTAATACGACTCACTATAGGGACATGACGACTAAGTTCGTCTTTGATTCATTGCACTGATCTCTTGTTAGATCTTTTTGCAATCTAGCATTTGTTAAAGTTCTTAAGGCCATGGAATTCTCGGGTGCCAAGGGCTAAATCTCATGGTAAA |
| OC43-Probe II.208 | AY391777.1 | OC43-Probe II | GTATTAGAGTTTCCCCTCTAATACGACTCACTATAGGGTGCACTGATCTCTTGTTAGATCTTTTTGCAATCTAGCATTTGTTAAAGTTCTTAAGGCCACGCCCTATTAATGGACATTTGGAGACCTGATGGAATTCTCGGGTGCCAAGGGCTAAATCTCATGGTAAA |
| OC43-Probe II.209 | AY391777.1 | OC43-Probe II | GTATTAGAGTTTCCCCTCTAATACGACTCACTATAGGGATCTAGCATTTGTTAAAGTTCTTAAGGCCACGCCCTATTAATGGACATTTGGAGACCTGAGAAGAAATATCTCCGTTATATTAACGGTTTTGGAATTCTCGGGTGCCAAGGGCTAAATCTCATGGTAAA |
| OC43-Probe II.210 | AY391777.1 | OC43-Probe II | GTATTAGAGTTTCCCCTCTAATACGACTCACTATAGGGCGCCCTATTAATGGACATTTGGAGACCTGAGAAGAAATATCTCCGTTATATTAACGGTTTTAATGTCTCAGAATTAGAAGATGCTTGTTTTGGAATTCTCGGGTGCCAAGGGCTAAATCTCATGGTAAA |
| OC43-Probe II.211 | AY391777.1 | OC43-Probe II | GTATTAGAGTTTCCCCTCTAATACGACTCACTATAGGGGAAGAAATATCTCCGTTATATTAACGGTTTTAATGTCTCAGAATTAGAAGATGCTTGTTTTAAATTTAACTATCAATTTCCTAAAGTAGGTGGAATTCTCGGGTGCCAAGGGCTAAATCTCATGGTAAA |
| OC43-Probe II.212 | AY391777.1 | OC43-Probe II | GTATTAGAGTTTCCCCTCTAATACGACTCACTATAGGGTAATGTCTCAGAATTAGAAGATGCTTGTTTTAAATTTAACTATCAATTTCCTAAAGTAGGATATTGTAGAGTTCCTAGTCATGCTTGGTGTGGAATTCTCGGGTGCCAAGGGCTAAATCTCATGGTAAA |
| OC43-Probe II.213 | AY391777.1 | OC43-Probe II | GTATTAGAGTTTCCCCTCTAATACGACTCACTATAGGGTAAATTTAACTATCAATTTCCTAAAGTAGGATATTGTAGAGTTCCTAGTCATGCTTGGTGCCGTAATCAAGGTAGATTTTGTGCTACATTTGGAATTCTCGGGTGCCAAGGGCTAAATCTCATGGTAAA |
| OC43-Probe II.214 | AY391777.1 | OC43-Probe II | GTATTAGAGTTTCCCCTCTAATACGACTCACTATAGGGATATTGTAGAGTTCCTAGTCATGCTTGGTGCCGTAATCAAGGTAGATTTTGTGCTACATTCACTCTTTATGGTAAATCCAAACATTATGATGGAATTCTCGGGTGCCAAGGGCTAAATCTCATGGTAAA |
| OC43-Probe II.215 | AY391777.1 | OC43-Probe II | GTATTAGAGTTTCCCCTCTAATACGACTCACTATAGGGCCGTAATCAAGGTAGATTTTGTGCTACATTCACTCTTTATGGTAAATCCAAACATTATGATAAATATTTTGGAGTAATAAATGGTTTCACTGGAATTCTCGGGTGCCAAGGGCTAAATCTCATGGTAAA |
| OC43-Probe II.216 | AY391777.1 | OC43-Probe II | GTATTAGAGTTTCCCCTCTAATACGACTCACTATAGGGCACTCTTTATGGTAAATCCAAACATTATGATAAATATTTTGGAGTAATAAATGGTTTCACGCATTCGCTAATACTGTAGAGGATGCTGTTGGAATTCTCGGGTGCCAAGGGCTAAATCTCATGGTAAA |
| OC43-Probe II.217 | AY391777.1 | OC43-Probe II | GTATTAGAGTTTCCCCTCTAATACGACTCACTATAGGGTAAATATTTTGGAGTAATAAATGGTTTCACGCATTCGCTAATACTGTAGAGGATGCTGTTAACAAACTGGTTTTCTTAGCTGTTGACTTTGGAATTCTCGGGTGCCAAGGGCTAAATCTCATGGTAAA |
| OC43-Probe II.218 | AY391777.1 | OC43-Probe II | GTATTAGAGTTTCCCCTCTAATACGACTCACTATAGGGAGCATTCGCTAATACTGTAGAGGATGCTGTTAACAAACTGGTTTTCTTAGCTGTTGACTTTATTACCTGGCGCAGACAGGAGTTAAATGTTGGAATTCTCGGGTGCCAAGGGCTAAATCTCATGGTAAA |
| OC43-Probe II.219 | AY391777.1 | OC43-Probe II | GTATTAGAGTTTCCCCTCTAATACGACTCACTATAGGGTAACAAACTGGTTTTCTTAGCTGTTGACTTTATTACCTGGCGCAGACAGGAGTTAAATGTTTATGGCTGATGCTTATCTTGCAGACACTGTGGAATTCTCGGGTGCCAAGGGCTAAATCTCATGGTAAA |
| OC43-Probe II.220 | AY391777.1 | OC43-Probe II | GTATTAGAGTTTCCCCTCTAATACGACTCACTATAGGGTATTACCTGGCGCAGACAGGAGTTAAATGTTTATGGCTGATGCTTATCTTGCAGACACTGTGTGGTATGTGGGGCAAATAATTTTTATAGTGGAATTCTCGGGTGCCAAGGGCTAAATCTCATGGTAAA |
| OC43-Probe II.221 | AY391777.1 | OC43-Probe II | GTATTAGAGTTTCCCCTCTAATACGACTCACTATAGGGTTATGGCTGATGCTTATCTTGCAGACACTGTGTGGTATGTGGGGCAAATAATTTTTATAGTTGCCATTTGTTTATTGGTTACAATAGTTGTGGAATTCTCGGGTGCCAAGGGCTAAATCTCATGGTAAA |
| OC43-Probe II.222 | AY391777.1 | OC43-Probe II | GTATTAGAGTTTCCCCTCTAATACGACTCACTATAGGGTGTGGTATGTGGGGCAAATAATTTTTATAGTTGCCATTTGTTTATTGGTTACAATAGTTGTAGTGGCATTTTGGCAACTTTTAAATTGTTGGAATTCTCGGGTGCCAAGGGCTAAATCTCATGGTAAA |
| OC43-Probe II.223 | AY391777.1 | OC43-Probe II | GTATTAGAGTTTCCCCTCTAATACGACTCACTATAGGGTTGCCATTTGTTTATTGGTTACAATAGTTGTAGTGGCATTTTGGCAACTTTTAAATTGTGTATTCAACTTTGCGGTATGTGTAATACCTTGGAATTCTCGGGTGCCAAGGGCTAAATCTCATGGTAAA |
| OC43-Probe II.224 | AY391777.1 | OC43-Probe II | GTATTAGAGTTTCCCCTCTAATACGACTCACTATAGGGTAGTGGCATTTTGGCAACTTTTAAATTGTGTATTCAACTTTGCGGTATGTGTAATACCTTAGTACTGTCCCCTTCTATTTATGTGTTTATGGAATTCTCGGGTGCCAAGGGCTAAATCTCATGGTAAA |
| OC43-Probe II.225 | AY391777.1 | OC43-Probe II | GTATTAGAGTTTCCCCTCTAATACGACTCACTATAGGGTATTCAACTTTGCGGTATGTGTAATACCTTAGTACTGTCCCCTTCTATTTATGTGTTTAATAGAGGTAGGCAGTTTTATGAGTTTTACATGGAATTCTCGGGTGCCAAGGGCTAAATCTCATGGTAAA |
| OC43-Probe II.226 | AY391777.1 | OC43-Probe II | GTATTAGAGTTTCCCCTCTAATACGACTCACTATAGGGTAGTACTGTCCCCTTCTATTTATGTGTTTAATAGAGGTAGGCAGTTTTATGAGTTTTACAATGATGTAAAACCACCAGTCCTTGATGTGGTGGAATTCTCGGGTGCCAAGGGCTAAATCTCATGGTAAA |
| OC43-Probe II.227 | AY391777.1 | OC43-Probe II | GTATTAGAGTTTCCCCTCTAATACGACTCACTATAGGGATAGAGGTAGGCAGTTTTATGAGTTTTACAATGATGTAAAACCACCAGTCCTTGATGTGGATGACGTTTAGGTAATCCAAACATTATGAGTGGAATTCTCGGGTGCCAAGGGCTAAATCTCATGGTAAA |
| OC43-Probe II.228 | AY391777.1 | OC43-Probe II | GTATTAGAGTTTCCCCTCTAATACGACTCACTATAGGGATGATGTAAAACCACCAGTCCTTGATGTGGATGACGTTTAGGTAATCCAAACATTATGAGTAGTAAAACTACACCAGCACCAGTTTATATTGGAATTCTCGGGTGCCAAGGGCTAAATCTCATGGTAAA |

| ASO ID | Accession | Group ID | Custom Array sequence |
|---|---|---|---|
| OC43-Probe II.229 | AY391777.1 | OC43-Probe II | GTATTAGAGTTTCCCCTCTAATACGACTCACTATAGGGATGACGTTTAGGTAATCCAAACATTATGAGTAGTAAAACTACACCAGCACCAGTTTATATCTGGACTGCTGATGAAGCTATTAAATTCCTTGGAATTCTCGGGTGCCAAGGGCTAAATCTCATGGTAAA |
| OC43-Probe II.230 | AY391777.1 | OC43-Probe II | GTATTAGAGTTTCCCCTCTAATACGACTCACTATAGGGTAGTAAAACTACACCAGCACCAGTTTATATCTGGACTGCTGATGAAGCTATTAAATTCCTAAAGGAATGGAATTTTTCTTTGGGTATTATTGGAATTCTCGGGTGCCAAGGGCTAAATCTCATGGTAAA |
| OC43-Probe II.231 | AY391777.1 | OC43-Probe II | GTATTAGAGTTTCCCCTCTAATACGACTCACTATAGGGCTGGACTGCTGATGAAGCTATTAAATTCCTAAAGGAATGGAATTTTTCTTTGGGTATTATACTACTTTTTATTACAATCATATTGCAATTTGGAATTCTCGGGTGCCAAGGGCTAAATCTCATGGTAAA |
| OC43-Probe II.232 | AY391777.1 | OC43-Probe II | GTATTAGAGTTTCCCCTCTAATACGACTCACTATAGGGAAAGGAATGGAATTTTTCTTTGGGTATTATACTACTTTTTATTACAATCATATTGCAATTTGGATATACAAGTCGCAGTATGTTTGTTTATGGAATTCTCGGGTGCCAAGGGCTAAATCTCATGGTAAA |
| OC43-Probe II.233 | AY391777.1 | OC43-Probe II | GTATTAGAGTTTCCCCTCTAATACGACTCACTATAGGGACTACTTTTTATTACAATCATATTGCAATTTGGATATACAAGTCGCAGTATGTTTGTTTATGTTATTAAGATGATTATTTTGTGGCTTATTGGAATTCTCGGGTGCCAAGGGCTAAATCTCATGGTAAA |
| OC43-Probe II.234 | AY391777.1 | OC43-Probe II | GTATTAGAGTTTCCCCTCTAATACGACTCACTATAGGGTGGATATACAAGTCGCAGTATGTTTGTTTATGTTATTAAGATGATTATTTTGTGGCTTATGTGGCCCCTTACTATAATCTTAACTATTTTGGAATTCTCGGGTGCCAAGGGCTAAATCTCATGGTAAA |
| OC43-Probe II.235 | AY391777.1 | OC43-Probe II | GTATTAGAGTTTCCCCTCTAATACGACTCACTATAGGGTGTTATTAAGATGATTATTTTGTGGCTTATGTGGCCCCTTACTATAATCTTAACTATTTTCAATTGCGTATACGCATTGAATAATGTGTATGGAATTCTCGGGTGCCAAGGGCTAAATCTCATGGTAAA |
| OC43-Probe II.236 | AY391777.1 | OC43-Probe II | GTATTAGAGTTTCCCCTCTAATACGACTCACTATAGGGTGGCCCCTTACTATAATCTTAACTATTTTCAATTGCGTATACGCATTGAATAATGTGTATCTTGGCCTTTCTATAGTTTTTACCATAGTTGGAATTCTCGGGTGCCAAGGGCTAAATCTCATGGTAAA |
| OC43-Probe II.237 | AY391777.1 | OC43-Probe II | GTATTAGAGTTTCCCCTCTAATACGACTCACTATAGGGCAATTGCGTATACGCATTGAATAATGTGTATCTTGGCCTTTCTATAGTTTTTACCATAGTGGCCATTATTATGTGGATTGTGTATTTTGTTGGAATTCTCGGGTGCCAAGGGCTAAATCTCATGGTAAA |
| OC43-Probe II.238 | AY391777.1 | OC43-Probe II | GTATTAGAGTTTCCCCTCTAATACGACTCACTATAGGGTCTTGGCCTTTCTATAGTTTTTACCATAGTGGCCATTATTATGTGGATTGTGTATTTTGTGAATAGTATCAGGTTGTTTATTAGAACTGGTGGAATTCTCGGGTGCCAAGGGCTAAATCTCATGGTAAA |
| OC43-Probe II.239 | AY391777.1 | OC43-Probe II | GTATTAGAGTTTCCCCTCTAATACGACTCACTATAGGGGGCCATTATTATGTGGATTGTGTATTTTGTGAATAGTATCAGGTTGTTTATTAGAACTGGAAGTTTTTGGAGTTTCAACCCAGAAACAAATGGAATTCTCGGGTGCCAAGGGCTAAATCTCATGGTAAA |
| OC43-Probe II.240 | AY391777.1 | OC43-Probe II | GTATTAGAGTTTCCCCTCTAATACGACTCACTATAGGGGAATAGTATCAGGTTGTTTATTAGAACTGGAAGTTTTTGGAGTTTCAACCCAGAAACAAACAACTTGATGTGTATAGATATGAAAGGAACTGGAATTCTCGGGTGCCAAGGGCTAAATCTCATGGTAAA |
| OC43-Probe II.241 | AY391777.1 | OC43-Probe II | GTATTAGAGTTTCCCCTCTAATACGACTCACTATAGGGAAGTTTTTGGAGTTTCAACCCAGAAACAAACAACTTGATGTGTATAGATATGAAAGGAACAATGTATGTTAGGCCGATAATTGAGGACTATGGAATTCTCGGGTGCCAAGGGCTAAATCTCATGGTAAA |
| OC43-Probe II.242 | AY391777.1 | OC43-Probe II | GTATTAGAGTTTCCCCTCTAATACGACTCACTATAGGGCAACTTGATGTGTATAGATATGAAAGGAACAATGTATGTTAGGCCGATAATTGAGGACTATCATACTCTGACGGTCACAATAATACGCGGTGGAATTCTCGGGTGCCAAGGGCTAAATCTCATGGTAAA |
| OC43-Probe II.243 | AY391777.1 | OC43-Probe II | GTATTAGAGTTTCCCCTCTAATACGACTCACTATAGGGAATGTATGTTAGGCCGATAATTGAGGACTATCATACTCTGACGGTCACAATAATACGCGGCCATCTTTACATTCAAGGTATAAAACTAGGTGGAATTCTCGGGTGCCAAGGGCTAAATCTCATGGTAAA |
| OC43-Probe II.244 | AY391777.1 | OC43-Probe II | GTATTAGAGTTTCCCCTCTAATACGACTCACTATAGGGTCATACTCTGACGGTCACAATAATACGCGGCCATCTTTACATTCAAGGTATAAAACTAGGTACTGGCTATTCTTTGGCAGATTTGCCAGCTGGAATTCTCGGGTGCCAAGGGCTAAATCTCATGGTAAA |
| OC43-Probe II.245 | AY391777.1 | OC43-Probe II | GTATTAGAGTTTCCCCTCTAATACGACTCACTATAGGGCCATCTTTACATTCAAGGTATAAAACTAGGTACTGGCTATTCTTTGGCAGATTTGCCAGCTTATATGACTGTTGCTAAGGTTACACACCTTGGAATTCTCGGGTGCCAAGGGCTAAATCTCATGGTAAA |
| OC43-Probe II.246 | AY391777.1 | OC43-Probe II | GTATTAGAGTTTCCCCTCTAATACGACTCACTATAGGGTACTGGCTATTCTTTGGCAGATTTGCCAGCTTATATGACTGTTGCTAAGGTTACACACCTGTGCACATATAAGCGTGGTTTTCTTGACAGTGGAATTCTCGGGTGCCAAGGGCTAAATCTCATGGTAAA |
| OC43-Probe II.247 | AY391777.1 | OC43-Probe II | GTATTAGAGTTTCCCCTCTAATACGACTCACTATAGGGTTATATGACTGTTGCTAAGGTTACACACCTGTGCACATATAAGCGTGGTTTTCTTGACAGGATAAGCGATACTAGTGGTTTTGCTGTTTATGGAATTCTCGGGTGCCAAGGGCTAAATCTCATGGTAAA |
| OC43-Probe II.248 | AY391777.1 | OC43-Probe II | GTATTAGAGTTTCCCCTCTAATACGACTCACTATAGGGTGCACATATAAGCGTGGTTTTCTTGACAGGATAAGCGATACTAGTGGTTTTGCTGTTTATGTTAAGTCCAAAGTCGGTAATTACCGACTTGGAATTCTCGGGTGCCAAGGGCTAAATCTCATGGTAAA |
| OC43-Probe II.249 | AY391777.1 | OC43-Probe II | GTATTAGAGTTTCCCCTCTAATACGACTCACTATAGGGGATAAGCGATACTAGTGGTTTTGCTGTTTATGTTAAGTCCAAAGTCGGTAATTACCGACTGCCATCAACCCAAAAGGGTTCTGGCATGGATGGAATTCTCGGGTGCCAAGGGCTAAATCTCATGGTAAA |
| OC43-Probe II.250 | AY391777.1 | OC43-Probe II | GTATTAGAGTTTCCCCTCTAATACGACTCACTATAGGGTGTTAAGTCCAAAGTCGGTAATTACCGACTGCCATCAACCCAAAAGGGTTCTGGCATGGACACCGCATTGTTGAGAAATAATATCTAAATTGGAATTCTCGGGTGCCAAGGGCTAAATCTCATGGTAAA |
| OC43-Probe II.251 | AY391777.1 | OC43-Probe II | GTATTAGAGTTTCCCCTCTAATACGACTCACTATAGGGGCCATCAACCCAAAAGGGTTCTGGCATGGACACCGCATTGTTGAGAAATAATATCTAAATTTTAAGGATGTCTTTTACTCCTGGTAAGCATGGAATTCTCGGGTGCCAAGGGCTAAATCTCATGGTAAA |
| OC43-Probe II.252 | AY391777.1 | OC43-Probe II | GTATTAGAGTTTCCCCTCTAATACGACTCACTATAGGGCACCGCATTGTTGAGAAATAATATCTAAATTTTAAGGATGTCTTTTACTCCTGGTAAGCAATCCAGTAGTAGAGCGTCCTCTGGAAATCGTGGAATTCTCGGGTGCCAAGGGCTAAATCTCATGGTAAA |
| OC43-Probe II.253 | AY391777.1 | OC43-Probe II | GTATTAGAGTTTCCCCTCTAATACGACTCACTATAGGGTTTAAGGATGTCTTTTACTCCTGGTAAGCAATCCAGTAGTAGAGCGTCCTCTGGAAATCGTTCTGGTAATGGCATCCTCAAGTGGGCCGATGGAATTCTCGGGTGCCAAGGGCTAAATCTCATGGTAAA |

100

| ASO ID | Accession | Group ID | Custom Array sequence |
|---|---|---|---|
| OC43-Probe II.254 | AY391777.1 | OC43-Probe II | GTATTAGAGTTTCCCCTCTAATACGACTCACTATAGGGATCCAGTAGTAGAGCGTCCTCTGGAAATCGTTCTGGTAATGGCATCCTCAAGTGGGCCGATCAGTCCGACCAGTTTAGAAATGTTCAAACTGGAATTCTCGGGTGCCAAGGGCTAAATCTCATGGTAAA |
| OC43-Probe II.255 | AY391777.1 | OC43-Probe II | GTATTAGAGTTTCCCCTCTAATACGACTCACTATAGGGTTCTGGTAATGGCATCCTCAAGTGGGCCGATCAGTCCGACCAGTTTAGAAATGTTCAAACCAGGGGTAGAAGAGCTCAACCCAAGCAAACTGGAATTCTCGGGTGCCAAGGGCTAAATCTCATGGTAAA |
| OC43-Probe II.256 | AY391777.1 | OC43-Probe II | GTATTAGAGTTTCCCCTCTAATACGACTCACTATAGGGTCAGTCCGACCAGTTTAGAAATGTTCAAACCAGGGGTAGAAGAGCTCAACCCAAGCAAACTGCTACCTCTCAGCAACCATCAGGAGGGAATGGAATTCTCGGGTGCCAAGGGCTAAATCTCATGGTAAA |
| OC43-Probe II.257 | AY391777.1 | OC43-Probe II | GTATTAGAGTTTCCCCTCTAATACGACTCACTATAGGGCAGGGGTAGAAGAGCTCAACCCAAGCAAACTGCTACCTCTCAGCAACCATCAGGAGGGAATGTTGTACCCTACTATTCTTGGTTCTCTGGTGGAATTCTCGGGTGCCAAGGGCTAAATCTCATGGTAAA |
| OC43-Probe II.258 | AY391777.1 | OC43-Probe II | GTATTAGAGTTTCCCCTCTAATACGACTCACTATAGGGTGCTACCTCTCAGCAACCATCAGGAGGGAATGTTGTACCCTACTATTCTTGGTTCTCTGGAATTACTCAGTTTCAAAAGGGAAAGGAGTTTGGAATTCTCGGGTGCCAAGGGCTAAATCTCATGGTAAA |
| OC43-Probe II.259 | AY391777.1 | OC43-Probe II | GTATTAGAGTTTCCCCTCTAATACGACTCACTATAGGGTGTTGTACCCTACTATTCTTGGTTCTCTGGAATTACTCAGTTTCAAAAGGGAAAGGAGTTTGAGTTTGTAGAAGGACAAGGTGTGCCTATTGGAATTCTCGGGTGCCAAGGGCTAAATCTCATGGTAAA |
| OC43-Probe II.260 | AY391777.1 | OC43-Probe II | GTATTAGAGTTTCCCCTCTAATACGACTCACTATAGGGAATTACTCAGTTTCAAAAGGGAAAGGAGTTTGAGTTTGTAGAAGGACAAGGTGTGCCTATTGCACCAGGAGTCCCAGCTACTGAAGCTAATGGAATTCTCGGGTGCCAAGGGCTAAATCTCATGGTAAA |
| OC43-Probe II.261 | AY391777.1 | OC43-Probe II | GTATTAGAGTTTCCCCTCTAATACGACTCACTATAGGGTGAGTTTGTAGAAGGACAAGGTGTGCCTATTGCACCAGGAGTCCCAGCTACTGAAGCTAAGGGGTACTGGTACAGACACAACAGACGTTCTGGAATTCTCGGGTGCCAAGGGCTAAATCTCATGGTAAA |
| OC43-Probe II.262 | AY391777.1 | OC43-Probe II | GTATTAGAGTTTCCCCTCTAATACGACTCACTATAGGGTGCACCAGGAGTCCCAGCTACTGAAGCTAAGGGGTACTGGTACAGACACAACAGACGTTCTTTTAAAACAGCCGATGGCAACCAGCGTCATGGAATTCTCGGGTGCCAAGGGCTAAATCTCATGGTAAA |
| OC43-Probe II.263 | AY391777.1 | OC43-Probe II | GTATTAGAGTTTCCCCTCTAATACGACTCACTATAGGGGGGGGTACTGGTACAGACACAACAGACGTTCTTTTAAAACAGCCGATGGCAACCAGCGTCAACTGCTGCCACGATGGTATTTTTACTATCTTGGAATTCTCGGGTGCCAAGGGCTAAATCTCATGGTAAA |
| OC43-Probe II.264 | AY391777.1 | OC43-Probe II | GTATTAGAGTTTCCCCTCTAATACGACTCACTATAGGGTTTTAAAACAGCCGATGGCAACCAGCGTCAACTGCTGCCACGATGGTATTTTTACTATCTGGGAACAGGACCGCATGCTAAAGACCAGTATGGAATTCTCGGGTGCCAAGGGCTAAATCTCATGGTAAA |
| OC43-Probe II.265 | AY391777.1 | OC43-Probe II | GTATTAGAGTTTCCCCTCTAATACGACTCACTATAGGGACTGCTGCCACGATGGTATTTTTACTATCTGGGAACAGGACCGCATGCTAAAGACCAGTACGGCACCGATATTGACGGAGTCTACTGGGTTGGAATTCTCGGGTGCCAAGGGCTAAATCTCATGGTAAA |
| OC43-Probe II.266 | AY391777.1 | OC43-Probe II | GTATTAGAGTTTCCCCTCTAATACGACTCACTATAGGGGGGGAACAGGACCGCATGCTAAAGACCAGTACGGCACCGATATTGACGGAGTCTACTGGGTCGCTAGCAACCAGGCTGATGTCAATACCCCTGGAATTCTCGGGTGCCAAGGGCTAAATCTCATGGTAAA |
| OC43-Probe II.267 | AY391777.1 | OC43-Probe II | GTATTAGAGTTTCCCCTCTAATACGACTCACTATAGGGCGGCACCGATATTGACGGAGTCTACTGGGTCGCTAGCAACCAGGCTGATGTCAATACCCCGGCTGACATTGTCGATCGGGACCCAAGTAGTGGAATTCTCGGGTGCCAAGGGCTAAATCTCATGGTAAA |
| OC43-Probe II.268 | AY391777.1 | OC43-Probe II | GTATTAGAGTTTCCCCTCTAATACGACTCACTATAGGGCGCTAGCAACCAGGCTGATGTCAATACCCCGGCTGACATTGTCGATCGGGACCCAAGTAGCGATGAGGCTATTCCGACTAGGTTTCCGCCTGGAATTCTCGGGTGCCAAGGGCTAAATCTCATGGTAAA |
| OC43-Probe II.269 | AY391777.1 | OC43-Probe II | GTATTAGAGTTTCCCCTCTAATACGACTCACTATAGGGGGGCTGACATTGTCGATCGGGACCCAAGTAGCGATGAGGCTATTCCGACTAGGTTTCCGCCTGGCACGGTACTCCCTCAGGGTTACTATATTGGAATTCTCGGGTGCCAAGGGCTAAATCTCATGGTAAA |
| OC43-Probe II.270 | AY391777.1 | OC43-Probe II | GTATTAGAGTTTCCCCTCTAATACGACTCACTATAGGGCGATGAGGCTATTCCGACTAGGTTTCCGCCTGGCACGGTACTCCCTCAGGGTTACTATATTGAAGGCTCAGGAAGGTCTGCTCCTAATTCTGGAATTCTCGGGTGCCAAGGGCTAAATCTCATGGTAAA |
| OC43-Probe II.271 | AY391777.1 | OC43-Probe II | GTATTAGAGTTTCCCCTCTAATACGACTCACTATAGGGTGGCACGGTACTCCCTCAGGGTTACTATATTGAAGGCTCAGGAAGGTCTGCTCCTAATTCCAGATCTACTTCGCGCACATCCAGCAGAGCTGGAATTCTCGGGTGCCAAGGGCTAAATCTCATGGTAAA |
| OC43-Probe II.272 | AY391777.1 | OC43-Probe II | GTATTAGAGTTTCCCCTCTAATACGACTCACTATAGGGTGAAGGCTCAGGAAGGTCTGCTCCTAATTCCAGATCTACTTCGCGCACATCCAGCAGAGCCTCTAGTGCAGGATCGCGTAGTAGAGCCAATGGAATTCTCGGGTGCCAAGGGCTAAATCTCATGGTAAA |
| OC43-Probe II.273 | AY391777.1 | OC43-Probe II | GTATTAGAGTTTCCCCTCTAATACGACTCACTATAGGGCAGATCTACTTCGCGCACATCCAGCAGAGCCTCTAGTGCAGGATCGCGTAGTAGAGCCAATTCTGGCAATAGAACCCCTACCTCTGGTGTTGGAATTCTCGGGTGCCAAGGGCTAAATCTCATGGTAAA |
| OC43-Probe II.274 | AY391777.1 | OC43-Probe II | GTATTAGAGTTTCCCCTCTAATACGACTCACTATAGGGCTCTAGTGCAGGATCGCGTAGTAGAGCCAATTCTGGCAATAGAACCCCTACCTCTGGTGTAACACCTGACATGGCTGATCAAATTGCTAGTGGAATTCTCGGGTGCCAAGGGCTAAATCTCATGGTAAA |
| OC43-Probe II.275 | AY391777.1 | OC43-Probe II | GTATTAGAGTTTCCCCTCTAATACGACTCACTATAGGGTTCTGGCAATAGAACCCCTACCTCTGGTGTAACACCTGACATGGCTGATCAAATTGCTAGTCTTGTTCTGGCAAAACTTGGCAAGGATGCTGGAATTCTCGGGTGCCAAGGGCTAAATCTCATGGTAAA |
| OC43-Probe II.276 | AY391777.1 | OC43-Probe II | GTATTAGAGTTTCCCCTCTAATACGACTCACTATAGGGAACACCTGACATGGCTGATCAAATTGCTAGTCTTGTTCTGGCAAAACTTGGCAAGGATGCCACTAAACCTCAGCAAGTAACTAAGCATACTGGAATTCTCGGGTGCCAAGGGCTAAATCTCATGGTAAA |
| OC43-Probe II.277 | AY391777.1 | OC43-Probe II | GTATTAGAGTTTCCCCTCTAATACGACTCACTATAGGGTCTTGTTCTGGCAAAACTTGGCAAGGATGCCACTAAACCTCAGCAAGTAACTAAGCATACTGCCAAAGAAGTCAGACAGAAAATTTTGAATGGAATTCTCGGGTGCCAAGGGCTAAATCTCATGGTAAA |
| OC43-Probe II.278 | AY391777.1 | OC43-Probe II | GTATTAGAGTTTCCCCTCTAATACGACTCACTATAGGGCACTAAACCTCAGCAAGTAACTAAGCATACTGCCAAAGAAGTCAGACAGAAAATTTTGAATAAGCCCCGCCAGAAGAGGAGCCCCAATAATGGAATTCTCGGGTGCCAAGGGCTAAATCTCATGGTAAA |

| ASO ID | Accession | Group ID | Custom Array sequence |
|---|---|---|---|
| OC43-Probe II.279 | AY391777.1 | OC43-Probe II | GTATTAGAGTTTCCCCTCTAATACGACTCACTATAGGGTGCCAAAGAAGTCAGACAGAAAATTTTGAATAAGCCCCGCCAGAAGAGGAGCCCCAATAAACAATGCACTGTTCAGCAGTGTTTTGGTAATGGAATTCTCGGGTGCCAAGGGCTAAATCTCATGGTAAA |
| OC43-Probe II.280 | AY391777.1 | OC43-Probe II | GTATTAGAGTTTCCCCTCTAATACGACTCACTATAGGGTAAGCCCCGCCAGAAGAGGAGCCCCAATAAACAATGCACTGTTCAGCAGTGTTTTGGTAAGAGAGGCCCTAATCAGAATTTTGGTGGTGGTGGAATTCTCGGGTGCCAAGGGCTAAATCTCATGGTAAA |
| OC43-Probe II.281 | AY391777.1 | OC43-Probe II | GTATTAGAGTTTCCCCTCTAATACGACTCACTATAGGGACAATGCACTGTTCAGCAGTGTTTTGGTAAGAGAGGCCCTAATCAGAATTTTGGTGGTGGAGAAATGTTAAAACTTGGAACTAGTGACCCTGGAATTCTCGGGTGCCAAGGGCTAAATCTCATGGTAAA |
| OC43-Probe II.282 | AY391777.1 | OC43-Probe II | GTATTAGAGTTTCCCCTCTAATACGACTCACTATAGGGGAGAGGCCCTAATCAGAATTTTGGTGGTGGAGAAATGTTAAAACTTGGAACTAGTGACCCACAGTTCCCCATTCTTGCAGAACTCGCACCTGGAATTCTCGGGTGCCAAGGGCTAAATCTCATGGTAAA |
| OC43-Probe II.283 | AY391777.1 | OC43-Probe II | GTATTAGAGTTTCCCCTCTAATACGACTCACTATAGGGAGAAATGTTAAAACTTGGAACTAGTGACCCACAGTTCCCCATTCTTGCAGAACTCGCACCCACAGCTGGTGCGTTTTCTTTGGATCAAGTGGAATTCTCGGGTGCCAAGGGCTAAATCTCATGGTAAA |
| OC43-Probe II.284 | AY391777.1 | OC43-Probe II | GTATTAGAGTTTCCCCTCTAATACGACTCACTATAGGGACAGTTCCCCATTCTTGCAGAACTCGCACCCACAGCTGGTGCGTTTTCTTTGGATCAAGATTAGAGTTGGCCAAAGTGCAGAATTTATCTGGAATTCTCGGGTGCCAAGGGCTAAATCTCATGGTAAA |
| OC43-Probe II.285 | AY391777.1 | OC43-Probe II | GTATTAGAGTTTCCCCTCTAATACGACTCACTATAGGGCACAGCTGGTGCGTTTTTCTTTGGATCAAGATTAGAGTTGGCCAAAGTGCAGAATTTATCTGGGAATCCTGACGAGCCCCAGAAGGATGTTGGAATTCTCGGGTGCCAAGGGCTAAATCTCATGGTAAA |
| OC43-Probe II.286 | AY391777.1 | OC43-Probe II | GTATTAGAGTTTCCCCTCTAATACGACTCACTATAGGGATTAGAGTTGGCCAAAGTGCAGAATTTATCTGGGAATCCTGACGAGCCCCAGAAGGATGTTTATGAATTGCGCTATAACGGCGCAATTAGTGGAATTCTCGGGTGCCAAGGGCTAAATCTCATGGTAAA |
| OC43-Probe II.287 | AY391777.1 | OC43-Probe II | GTATTAGAGTTTCCCCTCTAATACGACTCACTATAGGGTGGGAATCCTGACGAGCCCCAGAAGGATGTTTATGAATTGCGCTATAACGGCGCAATTAGGTTTGACAGTACACTTTCAGGTTTTGAGACTGGAATTCTCGGGTGCCAAGGGCTAAATCTCATGGTAAA |
| OC43-Probe II.288 | AY391777.1 | OC43-Probe II | GTATTAGAGTTTCCCCTCTAATACGACTCACTATAGGGTTATGAATTGCGCTATAACGGCGCAATTAGGTTTGACAGTACACTTTCAGGTTTTGAGACCATAATGAAGGTGCTGAATGAGAATTTGAATGGAATTCTCGGGTGCCAAGGGCTAAATCTCATGGTAAA |
| OC43-Probe II.289 | AY391777.1 | OC43-Probe II | GTATTAGAGTTTCCCCTCTAATACGACTCACTATAGGGGTTTGACAGTACACTTTCAGGTTTTGAGACCATAATGAAGGTGCTGAATGAGAATTTGAATGCCTATCAACAACAAGATGGTATGATGAATGGAATTCTCGGGTGCCAAGGGCTAAATCTCATGGTAAA |
| OC43-Probe II.290 | AY391777.1 | OC43-Probe II | GTATTAGAGTTTCCCCTCTAATACGACTCACTATAGGGCATAATGAAGGTGCTGAATGAGAATTTGAATGCCTATCAACAACAAGATGGTATGATGAATATGAGTCCAAAACCACAGCGTCAGCGTGGTGGAATTCTCGGGTGCCAAGGGCTAAATCTCATGGTAAA |
| OC43-Probe II.291 | AY391777.1 | OC43-Probe II | GTATTAGAGTTTCCCCTCTAATACGACTCACTATAGGGTGCCTATCAACAACAAGATGGTATGATGAATATGAGTCCAAAACCACAGCGTCAGCGTGGTCATAAGAATGGACAAGGAGAAAATGATAATGGAATTCTCGGGTGCCAAGGGCTAAATCTCATGGTAAA |
| OC43-Probe II.292 | AY391777.1 | OC43-Probe II | GTATTAGAGTTTCCCCTCTAATACGACTCACTATAGGGTATGAGTCCAAAACCACAGCGTCAGCGTGGTCATAAGAATGGACAAGGAGAAAATGATAATATAAGTGTTGCAGTGCCCAAAAGCCGCGTTGGAATTCTCGGGTGCCAAGGGCTAAATCTCATGGTAAA |
| OC43-Probe II.293 | AY391777.1 | OC43-Probe II | GTATTAGAGTTTCCCCTCTAATACGACTCACTATAGGGTCATAAGAATGGACAAGGAGAAAATGATAATATAAGTGTTGCAGTGCCCAAAAGCCGCGTGCAGCAAAATAAGAGTAGAGAGTTGACTGCTGGAATTCTCGGGTGCCAAGGGCTAAATCTCATGGTAAA |
| OC43-Probe II.294 | AY391777.1 | OC43-Probe II | GTATTAGAGTTTCCCCTCTAATACGACTCACTATAGGGTATAAGTGTTGCAGTGCCCAAAAGCCGCGTGCAGCAAAATAAGAGTAGAGAGTTGACTGCAGAGGACATCAGCCTTCTTAAGAAGATGGATGGAATTCTCGGGTGCCAAGGGCTAAATCTCATGGTAAA |
| OC43-Probe II.295 | AY391777.1 | OC43-Probe II | GTATTAGAGTTTCCCCTCTAATACGACTCACTATAGGGGCAGCAAAATAAGAGTAGAGAGTTGACTGCAGAGGACATCAGCCTTCTTAAGAAGATGGATGAGCCCTATACTGAAGACACCTCAGAAATTGGAATTCTCGGGTGCCAAGGGCTAAATCTCATGGTAAA |
| OC43-Probe II.296 | AY391777.1 | OC43-Probe II | GTATTAGAGTTTCCCCTCTAATACGACTCACTATAGGGAGAGGACATCAGCCTTCTTAAGAAGATGGATGAGCCCTATACTGAAGACACCTCAGAAATATAAGAGAATGAACCTTATGTCGGCATCTGTGGAATTCTCGGGTGCCAAGGGCTAAATCTCATGGTAAA |
| OC43-Probe II.297 | AY391777.1 | OC43-Probe II | GTATTAGAGTTTCCCCTCTAATACGACTCACTATAGGGTGAGCCCTATACTGAAGACACCTCAGAAATATAAGAGAATGAACCTTATGTCGGCATCTGGTGGTAACCCCTCGCAGAAAAGTCGAGATATGGAATTCTCGGGTGCCAAGGGCTAAATCTCATGGTAAA |
| OC43-Probe II.298 | AY391777.1 | OC43-Probe II | GTATTAGAGTTTCCCCTCTAATACGACTCACTATAGGGATAAGAGAATGAACCTTATGTCGGCATCTGGTGGTAACCCCTCGCAGAAAAGTCGAGATAAGGCACTCTCTATCAGAATGGATGTCTTGCTGGAATTCTCGGGTGCCAAGGGCTAAATCTCATGGTAAA |
| OC43-Probe II.299 | AY391777.1 | OC43-Probe II | GTATTAGAGTTTCCCCTCTAATACGACTCACTATAGGGTGGTAACCCCTCGCAGAAAAGTCGAGATAAGGCACTCTCTATCAGAATGGATGTCTTGCTGCTATAATAGATAGAGAAGGTTATAGCAGTGGAATTCTCGGGTGCCAAGGGCTAAATCTCATGGTAAA |
| OC43-Probe II.300 | AY391777.1 | OC43-Probe II | GTATTAGAGTTTCCCCTCTAATACGACTCACTATAGGGAGGCACTCTCTATCAGAATGGATGTCTTGCTGCTATAATAGATAGAGAAGGTTATAGCAGACTATAGATTAATTAGTTGAAAGTTTTGTGTGGAATTCTCGGGTGCCAAGGGCTAAATCTCATGGTAAA |
| OC43-Probe II.301 | AY391777.1 | OC43-Probe II | GTATTAGAGTTTCCCCTCTAATACGACTCACTATAGGGTGCTATAATAGATAGAGAAGGTTATAGCAGACTATAGATTAATTAGTTGAAAGTTTTGTGTTGTAATGTATAGTGTTGGAGAAAGTGAAATGGAATTCTCGGGTGCCAAGGGCTAAATCTCATGGTAAA |
| OC43-Probe II.302 | AY391777.1 | OC43-Probe II | GTATTAGAGTTTCCCCTCTAATACGACTCACTATAGGGACTATAGATTAATTAGTTGAAAGTTTTGTGTTGTAATGTATAGTGTTGGAGAAAGTGAAAGACTTGCGGAAGTAATTGCCGACAAGTGCCTGGAATTCTCGGGTGCCAAGGGCTAAATCTCATGGTAAA |
| OC43-Probe II.303 | AY391777.1 | OC43-Probe II | GTATTAGAGTTTCCCCTCTAATACGACTCACTATAGGGTTGTAATGTATAGTGTTGGAGAAAGTGAAAGACTTGCGGAAGTAATTGCCGACAAGTGCCCAAGGGAAGAGCCAGCATGTTAAGTTACCATGGAATTCTCGGGTGCCAAGGGCTAAATCTCATGGTAAA |

| ASO ID | Accession | Group ID | Custom Array sequence |
|---|---|---|---|
| OC43-Probe II.304 | AY391777.1 | OC43-Probe II | GTATTAGAAGTTTCCCCTCTAATACGACTCATATAGGGACTTGTGCGGAAGCTAATCCACCAGTAATGAATGAAGTAATTGGAATTCTCGGGTGCCAAGGGCTAAATCTCATGGTAAA |
| rRNA.0 | NR_003286 | rRNA_28S_18S | GAGGACTAGGACAGAGACGTAATACGACTCACTATAGGGTACCTGGTTGATCCTGCCAGTAGCATATGCTTGTCTCAAAGATTAAGCCATGCATGTCTAAGTACGCACGGCCGGTACAGTGAAACTGCGTAACTGGATAACTGGAGATTAGATGTC |

| ASO ID | Accession | Group ID | Custom Array sequence |
|---|---|---|---|
| rRNA.24 | NR_003286 | rRNA_28S_18S | GAGGACTAGGAACAGACGTAATACGACTCACTATAGGGGCCACCGCCCGTCCCCGCCCCTTGCCTCTCGGCGCCCCCTCGATGCTCTTAGCTGAGTGTCCCGCGGGGCCCGAAGCGTTTACTTTGAAATGGAATTCTCGGGTGCCAAGGATGGAAGATTTAGATGTC |
| rRNA.25 | NR_003286 | rRNA_28S_18S | GAGGACTAGGAACAGACGTAATACGACTCACTATAGGGGGGCGCCCCCTCGATGCTCTTAGCTGAGTGTCCCGCGGGGCCCGAAGCGTTTACTTTGAAAAAATTAGAGTGTTCAAAGCAGGCCCGAGCCTGGAATTCTCGGGTGCCAAGGATGGAAGATTTAGATGTC |
| rRNA.26 | NR_003286 | rRNA_28S_18S | GAGGACTAGGAACAGACGTAATACGACTCACTATAGGGCCCGCGGGGCCCGAAGCGTTTACTTTGAAAAAATTAGAGTGTTCAAAGCAGGCCCGAGCCGCCTGGATACCGCAGCTAGGAATAATGGAATGGAATTCTCGGGTGCCAAGGATGGAAGATTTAGATGTC |
| rRNA.27 | NR_003286 | rRNA_28S_18S | GAGGACTAGGAACAGACGTAATACGACTCACTATAGGGAAATTAGAGTGTTCAAAGCAGGCCCGAGCCGCCTGGATACCGCAGCTAGGAATAATGGAATAGGACCGCGGTTCTATTTTGTTGGTTTTCTGGAATTCTCGGGTGCCAAGGATGGAAGATTTAGATGTC |
| rRNA.28 | NR_003286 | rRNA_28S_18S | GAGGACTAGGAACAGACGTAATACGACTCACTATAGGGGCCTGGATACCGCAGCTAGGAATAATGGAATAGGACCGCGGTTCTATTTTGTTGGTTTTCGGAACTGAGGCCATGATTAAGAGGGACGGCTGGAATTCTCGGGTGCCAAGGATGGAAGATTTAGATGTC |
| rRNA.29 | NR_003286 | rRNA_28S_18S | GAGGACTAGGAACAGACGTAATACGACTCACTATAGGGTAGGACCGCGGTTCTATTTTGTTGGTTTTCGGAACTGAGGCCATGATTAAGAGGGACGGCCGGGGGCATTCGTATTGCGCCGCTAGAGGTTGGAATTCTCGGGTGCCAAGGATGGAAGATTTAGATGTC |
| rRNA.30 | NR_003286 | rRNA_28S_18S | GAGGACTAGGAACAGACGTAATACGACTCACTATAGGGGGGAACTGAGGCCATGATTAAGAGGGACGGCCGGGGGCATTCGTATTGCGCCGCTAGAGGTGAAATTCTTGGACCGGCGCAAGACGGACCATGGAATTCTCGGGTGCCAAGGATGGAAGATTTAGATGTC |
| rRNA.31 | NR_003286 | rRNA_28S_18S | GAGGACTAGGAACAGACGTAATACGACTCACTATAGGGCGGGGGCATTCGTATTGCGCCGCTAGAGGTGAAATTCTTGGACCGGCGCAAGACGGACCAGAGCGAAAGCATTTGCCAAGAATGTTTTCATGGAATTCTCGGGTGCCAAGGATGGAAGATTTAGATGTC |
| rRNA.32 | NR_003286 | rRNA_28S_18S | GAGGACTAGGAACAGACGTAATACGACTCACTATAGGGGAAATTCTTGGACCGGCGCAAGACGGACCAGAGCGAAAGCATTTGCCAAGAATGTTTTCATTAATCAAGAACGAAAGTCGGAGGTTCGAATGGAATTCTCGGGTGCCAAGGATGGAAGATTTAGATGTC |
| rRNA.33 | NR_003286 | rRNA_28S_18S | GAGGACTAGGAACAGACGTAATACGACTCACTATAGGGGAGCGAAAGCATTTGCCAAGAATGTTTTCATTAATCAAGAACGAAAGTCGGAGGTTCGAAGACGATCAGATACCGTCGTAGTTCCGACCATGGAATTCTCGGGTGCCAAGGATGGAAGATTTAGATGTC |
| rRNA.34 | NR_003286 | rRNA_28S_18S | GAGGACTAGGAACAGACGTAATACGACTCACTATAGGGGTTAATCAAGAACGAAAGTCGGAGGTTCGAAGACGATCAGATACCGTCGTAGTTCCGACCATAAACGATGCCGACCGGCGATGCGGCGGCGTGGAATTCTCGGGTGCCAAGGATGGAAGATTTAGATGTC |
| rRNA.35 | NR_003286 | rRNA_28S_18S | GAGGACTAGGAACAGACGTAATACGACTCACTATAGGGGACGATCAGATACCGTCGTAGTTCCGACCATAAACGATGCCGACCGGCGATGCGGCGGCGTTATTCCCATGACCCGCCGGGCAGCTTCCGTGGAATTCTCGGGTGCCAAGGATGGAAGATTTAGATGTC |
| rRNA.36 | NR_003286 | rRNA_28S_18S | GAGGACTAGGAACAGACGTAATACGACTCACTATAGGGTAAACGATGCCGACCGGCGATGCGGCGGCGTTATTCCCATGACCCGCCGGGCAGCTTCCGGGAAACCAAAGTCTTTGGGTTCCGGGGGGTGGAATTCTCGGGTGCCAAGGATGGAAGATTTAGATGTC |
| rRNA.37 | NR_003286 | rRNA_28S_18S | GAGGACTAGGAACAGACGTAATACGACTCACTATAGGGTTATTCCCATGACCCGCCGGGCAGCTTCCGGGAAACCAAAGTCTTTGGGTTCCGGGGGGAGTATGGTTGCAAAGCTGAAACTTAAAGGAATGGAATTCTCGGGTGCCAAGGATGGAAGATTTAGATGTC |
| rRNA.38 | NR_003286 | rRNA_28S_18S | GAGGACTAGGAACAGACGTAATACGACTCACTATAGGGGGAAACCAAAGTCTTTGGGTTCCGGGGGGAGTATGGTTGCAAAGCTGAAACTTAAAGGAATTGACGGAAGGGCACCACCAGGAGTGGAGCTGGAATTCTCGGGTGCCAAGGATGGAAGATTTAGATGTC |
| rRNA.39 | NR_003286 | rRNA_28S_18S | GAGGACTAGGAACAGACGTAATACGACTCACTATAGGGGTATGGTTGCAAAGCTGAAACTTAAAGGAATTGACGGAAGGGCACCACCAGGAGTGGAGCCTGCGGCTTAATTTGACTCAACACGGGAAATGGAATTCTCGGGTGCCAAGGATGGAAGATTTAGATGTC |
| rRNA.40 | NR_003286 | rRNA_28S_18S | GAGGACTAGGAACAGACGTAATACGACTCACTATAGGGGTTGACGGAAGGGCACCACCAGGAGTGGAGCCTGCGGCTTAATTTGACTCAACACGGGAAACCTCACCCGGCCCGGACACGGACAGGATTGTGGAATTCTCGGGTGCCAAGGATGGAAGATTTAGATGTC |
| rRNA.41 | NR_003286 | rRNA_28S_18S | GAGGACTAGGAACAGACGTAATACGACTCACTATAGGGCTGCGGCTTAATTTGACTCAACACGGGAAACCTCACCCGGCCCGGACACGGACAGGATTGACAGATTGATAGCTCTTTCTCGATTCCGTGTGGAATTCTCGGGTGCCAAGGATGGAAGATTTAGATGTC |
| rRNA.42 | NR_003286 | rRNA_28S_18S | GAGGACTAGGAACAGACGTAATACGACTCACTATAGGGCCTCACCCGGCCCGGACACGGACAGGATTGACAGATTGATAGCTCTTTCTCGATTCCGTGGGTGGTGGTGCATGGCCGTTCTTAGTTGGTTGGAATTCTCGGGTGCCAAGGATGGAAGATTTAGATGTC |
| rRNA.43 | NR_003286 | rRNA_28S_18S | GAGGACTAGGAACAGACGTAATACGACTCACTATAGGGGACAGATTGATAGCTCTTTCTCGATTCCGTGGGTGGTGGTGCATGGCCGTTCTTAGTTGGTGGAGCGATTTGTCTGGTTAATTCCGATAACTGGAATTCTCGGGTGCCAAGGATGGAAGATTTAGATGTC |
| rRNA.44 | NR_003286 | rRNA_28S_18S | GAGGACTAGGAACAGACGTAATACGACTCACTATAGGGGGGTGGTGGTGCATGGCCGTTCTTAGTTGGTGGAGCGATTTGTCTGGTTAATTCCGATAACGAACGAGACTCTGGCATGCTAACTAGTTACTGGAATTCTCGGGTGCCAAGGATGGAAGATTTAGATGTC |
| rRNA.45 | NR_003286 | rRNA_28S_18S | GAGGACTAGGAACAGACGTAATACGACTCACTATAGGGGGAGCGATTTGTCTGGTTAATTCCGATAACGAACGAGACTCTGGCATGCTAACTAGTTACGCGACCCCCGAGCGGTCGGCGTCCCCCAACTGGAATTCTCGGGTGCCAAGGATGGAAGATTTAGATGTC |
| rRNA.46 | NR_003286 | rRNA_28S_18S | GAGGACTAGGAACAGACGTAATACGACTCACTATAGGGGAACGAGACTCTGGCATGCTAACTAGTTACGCGACCCCCGAGCGGTCGGCGTCCCCCAACTTCTTAGAGGGACAAGTGGCGTTCAGCCACTGGAATTCTCGGGTGCCAAGGATGGAAGATTTAGATGTC |
| rRNA.47 | NR_003286 | rRNA_28S_18S | GAGGACTAGGAACAGACGTAATACGACTCACTATAGGGGCGACCCCCGAGCGGTCGGCGTCCCCCAACTTCTTAGAGGGACAAGTGGCGTTCAGCCACCCGAGATTGAGCAATAACAGGTCTGTGATGTGGAATTCTCGGGTGCCAAGGATGGAAGATTTAGATGTC |
| rRNA.48 | NR_003286 | rRNA_28S_18S | GAGGACTAGGAACAGACGTAATACGACTCACTATAGGGTTCTTAGAGGGACAAGTGGCGTTCAGCCACCCGAGATTGAGCAATAACAGGTCTGTGATGCCCTTAGATGTCCGGGGCTGCACGCGCGCTTGGAATTCTCGGGTGCCAAGGATGGAAGATTTAGATGTC |

| ASO ID | Accession | Group ID | Custom Array sequence |
|---|---|---|---|
| rRNA.49 | NR_003286 | rRNA_28S_18S | GAGGACTAGGAACAGACGTAATACGACTCACTATAGGGCCGAGATTGAGCAATAACAGGTCTGTGATGCCCTTAGATGTCCGGGGCTGCACGCGCGCTACACTGACTGGCTCACCCTTGGAATTCTCGGGTGCCAAGGATGGAAGATTTAGATGTC |
| rRNA.50 | NR_003286 | rRNA_28S_18S | GAGGACTAGGAACAGACGTAATACGACTCACTATAGGGCCTTAGATGTCCGGGGCTGCACGCGCGCTACACTGACTGGCTCAGCGTGTGCCTACTCCGTTGAACCCCCATTCGTGATGGGGATTGCAATGATTCCCATGTCAAGGATGGAAGATTTAGATGTC |
| rRNA.51 | NR_003286 | rRNA_28S_18S | GAGGACTAGGAACAGACGTAATACGACTCACTATAGGGCCAGGCGCGGCCTACCCTACGCGCGGTGCCTACTCCGTTGAACCCCCATTCGTGATGGGGATTGCAATTATTCCCCATGAACGAGGAATTCCCAGTAAGTTGCGGTCATAAGCTTGCGTTGATTAAGTCCCTGCCTTTGTACACACCGCCCGTCGCTACTACCGATTGGATGGTTTAGTGAGGCCCTCGGATCGGCCCACGTGAGAAGATTTAGATGTC |
| rRNA.52 | NR_003286 | rRNA_28S_18S | GAGGACTAGGAACAGACGTAATACGACTCACTATAGGGCCATTCGTGATGGGGATCGGGGATTGCAATTATTCCCCATGTGAACGAGGAATTCCCAGTAAGTGCGGGTCATAAGCTTGCGTTGATTAAGTCCCTGCCTTTGTACACACCGCCCGTCGCTACTACCGATTGGATGGTTTAGTGAGGCCCTCGGATCGGCCCACGTGAGAAGATTTAGATGTC |
| rRNA.53 | NR_003286 | rRNA_28S_18S | GAGGACTAGGAACAGACGTAATACGACTCACTATAGGGCCATTCGTGATGGGGATCGGGGATTGCAATTATTCCCCATGAACGAGGAATTCCCAGTAAGTGCGGGTCATAAGCTTGCGTTGATTAAGTCCCTGCCTTTGTACACACCGCCCGTCGCTACTACCGATTGGATGGTTTAGTGAGGCCCTCGGATCGGCCCACGTGAGAAGATTTAGATGTC |
| rRNA.54 | NR_003286 | rRNA_28S_18S | GAGGACTAGGAACAGACGTAATACGACTCACTATAGGGCCATTCGTGATGGGGATCGGGGATTGCAATTATTCCCCATGAACGAGGAATTCCCAGTAAGTGCGGGTCATAAGCTTGCGTTGATTAAGTCCCTGCCTTTGTACACACCGCCCGTCGCTACTACCGATTGGATGGTTTAGTGAGGCCCTCGGATCGGCCCACGTGAGAAGATTTAGATGTC |
| rRNA.55 | NR_003286 | rRNA_28S_18S | GAGGACTAGGAACAGACGTAATACGACTCACTATAGGGCCCTCGGATCGGCCCACGTGAGAAGATTTAGATGTC |
| rRNA.56 | NR_003286 | rRNA_28S_18S | GAGGACTAGGAACAGACGTAATACGACTCACTATAGGGCCTCGGATCGGCCCACGTGAGAAGATTTAGATGTC |
| rRNA.57 | NR_003286 | rRNA_28S_18S | GAGGACTAGGAACAGACGTAATACGACTCACTATAGGGCCTCGGATCGGCCCACGTGAGAAGGTCGGATGTGATGGGGAGTCGATCTGCAATTCGTGATGGGGATTGCAATTATTCCCCATGTGAACGAGGAATTCCCAGTAAGTGCGGGTCATAAGCTTGCGTTGATTAAGTCACAGGAAGATGAGGAAGATTTAGATGTC |
| rRNA.58 | NR_003286 | rRNA_28S_18S | GAGGACTAGGAACAGACGTAATACGACTCACTATAGGGCTCGGATCGGCCCACGTGAGAAGGTCGGATCTGAGCAGCTGCGAACTTGACTATCTAGAGGAAGTAAAAGTCGTTGGAATTCTCGGGTGCCAAGGATGGAAGATTTAGATGTC |
| rRNA.59 | NR_003286 | rRNA_28S_18S | GAGGACTAGGAACAGACGTAATACGACTCACTATAGGGCCCTGGCCGGACCGGCTGACCTGCAATTATTCCCCGTAGGTGAACGTGCGGAATGGGGAATTCTCGGGTGCCAAGGATGGAAGATTTAGATGTC |
| rRNA.60 | NR_003287 | rRNA_28S_18S | GAGGACTAGGAACAGACGTAATACGACTCACTATAGGGCCCTGGCCGGACCGGCTGACCTGCAATTATTCCCCGTAGGTGAACGTGCGGAATGGGGAATTCTCGGGTGCCAAGGATGGAAGATTTAGATGTC |
| rRNA.61 | NR_003287 | rRNA_28S_18S | GAGGACTAGGAACAGACGTAATACGACTCACTATAGGGCCTCAGATCAGACGTGGCGACCCGCTGAATTTAAGCATATTAGTCAGCGGAGGAAAGAAACTAACCAGGATTCCCTCAGTAACGGCGAGTGAACAGGGAAGAGCCCAGCGCCGAATTCTCGGGTGCCAAGGATGGAAGATTTAGATGTC |
| rRNA.62 | NR_003287 | rRNA_28S_18S | GAGGACTAGGAACAGACGTAATACGACTCACTATAGGGCCTCAGATCAGACGTGGCGACCCGCTGAATTTAAGCATATTAGTCAGCGGAGGAAAGAAACTAACCAGGATTCCCTCAGTAACGGCGAGTGAACAGGGAAGAGCCCAGCGCCGAATTCTCGGGTGCCAAGGATGGAAGATTTAGATGTC |
| rRNA.63 | NR_003287 | rRNA_28S_18S | GAGGACTAGGAACAGACGTAATACGACTCACTATAGGGCCAGCGCGGCGGGGGTACGGCCGGACCCGCTCCCCGGCCGCTGGGGGAATCCGTCTGGCCGTACGGAGGTGCGTGGGGGGCCCAAGTCCTTCTGATCTGAGGCCGTGAGGTGGGGCCCAAGTCCTTCTGATCTGAGGCCGTGAGGTGGGGCCCAAGTGAAGATTTAGATGTC |
| rRNA.64 | NR_003287 | rRNA_28S_18S | GAGGACTAGGAACAGACGTAATACGACTCACTATAGGGCCTCCCCGGCCGTCCCCCTCCTCCGGGGGGCCGGGCCACCGGGGCCCAAGTCCTTCTGATCTTTGGGTGGACGGCCTGAGAGCGCCGGGCCCCGGCCGCCCAGTCCGGGGTGGAAGCTTCGGGTGCCAAGGATGGAAGATTTAGATGTC |
| rRNA.65 | NR_003287 | rRNA_28S_18S | GAGGACTAGGAACAGACGTAATACGACTCACTATAGGGCCTCCCCGGCCGTCCCCCTCCTCCGGGGGGCCGGGCCACCGGGCCCGTCGGCCGTACGGAGGTGCGTGGGGGGCCCCAAGTCCTTCTGATCTTTGGGTGGACGGCCTGAGAGGCCCTGAGGTGGGGCCCAAGTGAAGATTTAGATGTC |
| rRNA.66 | NR_003287 | rRNA_28S_18S | GAGGACTAGGAACAGACGTAATACGACTCACTATAGGGCCAGCGCGGCGCCCCGGGTGTGGGCCGCCGCCGCGGGCTCTTCTGATCCGGGCCGGGGTGAGGCCCGGGCCGGGGTCTTCTGGGATGCATGCCGGAATGCATGGGAATGCTCGGGTGCCAAGGATGGAAGATTTAGATGTC |
| rRNA.67 | NR_003287 | rRNA_28S_18S | GAGGACTAGGAACAGACGTAATACGACTCACTATAGGGCCGTGACGCGCATGAATGGATGAACGAGATTCCCGGCTCTTCTGATGTGGAGTCGCATGGTGTTGGGATAGCATGGGAATGCTCGGGTGCCAAGGATGGAAGATTTAGATGTC |
| rRNA.68 | NR_003287 | rRNA_28S_18S | GAGGACTAGGAACAGACGTAATACGACTCACTATAGGGCCGCCCCGGGGTTGCTTGGGAATGCAGCCCAAAGCGGGTGGTAAACTCCATCTAAGGCTAAATACCGGCACGAGACCGATAGTCAACAAGGAGAGGGAAGATTTAGATGTC |
| rRNA.69 | NR_003287 | rRNA_28S_18S | GAGGACTAGGAACAGACGTAATACGACTCACTATAGGGCCAGCCCAAAGCGGGTGGTAAACTCCATCTAAGGCTAAATACCGGCACGAGACCGATAGTCAACAAGGAGAGGGAAGATTTAGATGTC |
| rRNA.70 | NR_003287 | rRNA_28S_18S | GAGGACTAGGAACAGACGTAATACGACTCACTATAGGGCCGATGAATTCTGCTTCCAGGGGGAATTAGGAGCACGAGGGAAAGTTGAAAAGAACTGGATAAGAGACCGGTGAAATGGAAGATTTAGATGTC |
| rRNA.71 | NR_003287 | rRNA_28S_18S | GAGGACTAGGAACAGACGTAATACGACTCACTATAGGGCCTAAATACCCGCAGAGCCGATAGTCAACAAGTACCGTAAGGGAAAGTTGAAAAGAACTTTGAAAAGAGAGTTCAAGAGGGCGTGAAACCGTTAAGAGGTAAACGGGTGGGGTCTCCGCGGAGGATTACGGGAAGATTTAGATGTC |
| rRNA.72 | NR_003287 | rRNA_28S_18S | GAGGACTAGGAACAGACGTAATACGACTCACTATAGGGCCTAAGAGGTAAACGGGTGGGGTCTCCGCGGCGTGAAACCCGCAGTCCCGGCCGCTGGGGAATTCTGCTTCCAGGGTGCCAAGGATGGAAGATTTAGATGTC |
| rRNA.73 | NR_003287 | rRNA_28S_18S | GAGGACTAGGAACAGACGTAATACGACTCACTATAGGGCCTGTGAAAGCGTGAAGAGAGTTCAAGAGGTAAACGGGTGGGGTCTCCGCGGCGTGAAACCCGCAGTCCCGGCCGCTGGGGAATTCTGCTTCCAGGGTGCCAAGGATGGAAGATTTAGATGTC |

104

| ASO ID | Accession | Group ID | Custom Array sequence |
|---|---|---|---|
| rRNA.74 | NR_003287 | rRNA_28S_18S | GAGGACTAGGAACAGACGTAATACGACTCACTATAGGGCCGTTAAGAGGTAAACGGGTGGGGTCCGCGCAGTCCGCCCGGAGGATTCAACCCGGCGGCGGGTCCGGCCGTGTCGGCGGCCCGGCGGATTGGAATTCTCGGGTGCCAAGGATGGAAGATTTAGATGTC |
| rRNA.75 | NR_003287 | rRNA_28S_18S | GAGGACTAGGAACAGACGTAATACGACTCACTATAGGGCAGTCCGCCCGGAGGATTCAACCCGGCGGCGGGTCCGGCCGTGTCGGCGGCCCGGCGGATCTTTCCCGCCCCCCGTTCCTCCCGACCCCCTTGGAATTCTCGGGTGCCAAGGATGGAAGATTTAGATGTC |
| rRNA.76 | NR_003287 | rRNA_28S_18S | GAGGACTAGGAACAGACGTAATACGACTCACTATAGGGGGGGTCCGGCCGTGTCGGCGGCCCGGCGGATCTTTCCCGCCCCCCGTTCCTCCCGACCCCTCCACCCGCCCTCCCCTTCCCCCGCCGCCCCTTGGAATTCTCGGGTGCCAAGGATGGAAGATTTAGATGTC |
| rRNA.77 | NR_003287 | rRNA_28S_18S | GAGGACTAGGAACAGACGTAATACGACTCACTATAGGGCTTTCCCGCCCCCCGTTCCTCCCGACCCCTCCACCCGCCCTCCCCTTCCCCCGCCGCCCCTCCTCCTCCTCCCCCGGAGGGGGCGGGCTCCGTGGAATTCTCGGGTGCCAAGGATGGAAGATTTAGATGTC |
| rRNA.78 | NR_003287 | rRNA_28S_18S | GAGGACTAGGAACAGACGTAATACGACTCACTATAGGGCCACCCGCCCTCCCTTCCCCCGCCGCCCCTCCTCCTCCTCCCCCGGAGGGGGCGGGCTCCGGCGGGTGCGGGGGTGGGCGGGCGGGGCCGGTGGAATTCTCGGGTGCCAAGGATGGAAGATTTAGATGTC |
| rRNA.79 | NR_003287 | rRNA_28S_18S | GAGGACTAGGAACAGACGTAATACGACTCACTATAGGGCCTCCTCCTCCCCCGGAGGGGGCGGGCTCCGGCGGGTGCGGGGGTGGGCGGGCGGGGCCGGGGGTGGGGTCGGCGGGGGACCGTCCCCCGATGGAATTCTCGGGTGCCAAGGATGGAAGATTTAGATGTC |
| rRNA.80 | NR_003287 | rRNA_28S_18S | GAGGACTAGGAACAGACGTAATACGACTCACTATAGGGGCGGGTGCGGGGGTGGGCGGGCGGGGCCGGGGGTGGGGTCGGCGGGGGACCGTCCCCCGACCGGCGACCGGCCGCCGCCGCGGGCGCATTTCTGGAATTCTCGGGTGCCAAGGATGGAAGATTTAGATGTC |
| rRNA.81 | NR_003287 | rRNA_28S_18S | GAGGACTAGGAACAGACGTAATACGACTCACTATAGGGGGGGTGGGGTCGGCGGGGGACCGTCCCCCGACCGGCGACCGGCCGCCGCCGCGGGCGCATTTCCACCGCGGCGGTGCGCCGCGACCGGCTCCGTGGAATTCTCGGGTGCCAAGGATGGAAGATTTAGATGTC |
| rRNA.82 | NR_003287 | rRNA_28S_18S | GAGGACTAGGAACAGACGTAATACGACTCACTATAGGGCCGGCGACCGGCCGCCGCCGCGGGCGCATTTCCACCGCGGCGGTGCGCCGCGACCGGCTCCGGGACGGCTGGGAAGGCCCGGCGGGGAAGGTTGGAATTCTCGGGTGCCAAGGATGGAAGATTTAGATGTC |
| rRNA.83 | NR_003287 | rRNA_28S_18S | GAGGACTAGGAACAGACGTAATACGACTCACTATAGGGCACCGCGGCGGTGCGCCGCGACCGGCTCCGGGACGGCTGGGAAGGCCCGGCGGGGAAGGTGGCTCGGGGGGGCCCCGTCCGTCCGTCCGTCTGGAATTCTCGGGTGCCAAGGATGGAAGATTTAGATGTC |
| rRNA.84 | NR_003287 | rRNA_28S_18S | GAGGACTAGGAACAGACGTAATACGACTCACTATAGGGGGGACGGCTGGGAAGGCCCGGCGGGGAAGGTGGCTCGGGGGGGCCCCGTCCGTCCGTCCGTCGGTCCTCCTCCTCCCCCGTCTCCGCCCCCTGGAATTCTCGGGTGCCAAGGATGGAAGATTTAGATGTC |
| rRNA.85 | NR_003287 | rRNA_28S_18S | GAGGACTAGGAACAGACGTAATACGACTCACTATAGGGGGCTCGGGGGGGCCCCGTCCGTCCGTCCGTCGGTCCTCCTCCTCCCCCGTCTCCGCCCCCGGCCCCGCGTCCTCCCTCGGGAGGGCGCGCTGGAATTCTCGGGTGCCAAGGATGGAAGATTTAGATGTC |
| rRNA.86 | NR_003287 | rRNA_28S_18S | GAGGACTAGGAACAGACGTAATACGACTCACTATAGGGCGTCCTCCTCCTCCCCCGTCTCCGCCCCCGGCCCCGCGTCCTCCCTCGGGAGGGCGCGCGGGTCGGGGCGGCGGCGGCGGCGGCGGCGGTGGAATTCTCGGGTGCCAAGGATGGAAGATTTAGATGTC |
| rRNA.87 | NR_003287 | rRNA_28S_18S | GAGGACTAGGAACAGACGTAATACGACTCACTATAGGGGGGCCCCGCGTCCTCCCTCGGGAGGGCGCGCGGGTCGGGGCGGCGGCGGCGGCGGCGGCGGTGGCGGCGGCGGCGGCGGCGGCGGGACCGAAACTGGAATTCTCGGGTGCCAAGGATGGAAGATTTAGATGTC |
| rRNA.88 | NR_003287 | rRNA_28S_18S | GAGGACTAGGAACAGACGTAATACGACTCACTATAGGGGGGGTCGGGGCGGCGGCGGCGGCGGCGGCGGTGGCGGCGGCGGCGGCGGCGGCGGGACCGAAACCCCCCCGAGTGTTACAGCCCCCCGGCAGTGGAATTCTCGGGTGCCAAGGATGGAAGATTTAGATGTC |
| rRNA.89 | NR_003287 | rRNA_28S_18S | GAGGACTAGGAACAGACGTAATACGACTCACTATAGGGCGGCGGCGGCGGCGGCGGCGGGACCGAAACCCCCCCGAGTGTTACAGCCCCCCGGCAGCAGCACTCGCCGAATCCCGGGGCCGAGGGATGGAATTCTCGGGTGCCAAGGATGGAAGATTTAGATGTC |
| rRNA.90 | NR_003287 | rRNA_28S_18S | GAGGACTAGGAACAGACGTAATACGACTCACTATAGGGCCCCCCCGAGTGTTACAGCCCCCCGGCAGCAGCACTCGCCGAATCCCGGGGCCGAGGGAGCGAGACCCGTCGCCGCGCTCTCCCCCCTCTGGAATTCTCGGGTGCCAAGGATGGAAGATTTAGATGTC |
| rRNA.91 | NR_003287 | rRNA_28S_18S | GAGGACTAGGAACAGACGTAATACGACTCACTATAGGGCAGCACTCGCCGAATCCCGGGGCCGAGGGAGCGAGACCCGTCGCCGCGCTCTCCCCCCTCCCGGCGCCCACCCCCGCGGGGAATCCCCGTGGAATTCTCGGGTGCCAAGGATGGAAGATTTAGATGTC |
| rRNA.92 | NR_003287 | rRNA_28S_18S | GAGGACTAGGAACAGACGTAATACGACTCACTATAGGGGGCGAGACCCGTCGCCGCGCTCTCCCCCCTCCCGGCGCCCACCCCCGCGGGGAATCCCCGCGAGGGGGGTCTCCCCCGCGGGGGCGCGCCTGGAATTCTCGGGTGCCAAGGATGGAAGATTTAGATGTC |
| rRNA.93 | NR_003287 | rRNA_28S_18S | GAGGACTAGGAACAGACGTAATACGACTCACTATAGGGCCGGCGCCCACCCCCGCGGGGAATCCCCGCGAGGGGGGTCTCCCCCGCGGGGGCGCGCCGGCGTCTCCTCGTGGGGGGGCCCGGGCCACCTGGAATTCTCGGGTGCCAAGGATGGAAGATTTAGATGTC |
| rRNA.94 | NR_003287 | rRNA_28S_18S | GAGGACTAGGAACAGACGTAATACGACTCACTATAGGGCGAGGGGGGTCTCCCCCGCGGGGGCGCGCCGGCGTCTCCTCGTGGGGGGGCCCGGGCCACCCCTCCCACGGCGCGACCGCTCTCCCACCCCTGGAATTCTCGGGTGCCAAGGATGGAAGATTTAGATGTC |
| rRNA.95 | NR_003287 | rRNA_28S_18S | GAGGACTAGGAACAGACGTAATACGACTCACTATAGGGGCGTCTCCTCGTGGGGGGGCCGGGCCACCCCTCCCACGGCGCGACCGCTCTCCCACCCCTCCTCCCCGCGCCCCCGCCCCGGCGACGGGTGGAATTCTCGGGTGCCAAGGATGGAAGATTTAGATGTC |
| rRNA.96 | NR_003287 | rRNA_28S_18S | GAGGACTAGGAACAGACGTAATACGACTCACTATAGGGCCTCCCACGGCGCGACCGCTCTCCCACCCCTCCTCCCCGCGCCCCCGCCCCGGCGACGGGGGGGGTGCCGCGCGCGGGTCGGGGGGCGGGTGGAATTCTCGGGTGCCAAGGATGGAAGATTTAGATGTC |
| rRNA.97 | NR_003287 | rRNA_28S_18S | GAGGACTAGGAACAGACGTAATACGACTCACTATAGGGTCCTCCCCGCGCCCCCGCCCCGGCGACGGGGGGGGTGCCGCGCGCGGGTCGGGGGGCGGGGCGGACTGTCCCCAGTGCGCCCCGGGCGGGTGGAATTCTCGGGTGCCAAGGATGGAAGATTTAGATGTC |
| rRNA.98 | NR_003287 | rRNA_28S_18S | GAGGACTAGGAACAGACGTAATACGACTCACTATAGGGGGGGGGTGCCGCGCGCGGGTCGGGGGGCGGGGCGGACTGTCCCCAGTGCGCCCCGGGCGGGTCGCGCCGTCGGGCCCGGGGGAGGTTCTCTTGGAATTCTCGGGTGCCAAGGATGGAAGATTTAGATGTC |

| ASO ID | Accession | Group ID | Custom Array sequence |
|---|---|---|---|
| rRNA.99 | NR_003287 | rRNA_28S_18S | GAGGACTAGGAACAGACGTAATACGACTCACTATAGGGGCGGACTGTCCCCAGTGCGCCCCGGGCGGGTCGCGCCGTCGGGCCCGGGGGAGGTTCTCTCGGGGCCACGCGCGCGTCCCCCGAAGAGGGTGGAATTCTCGGGTGCCAAGGATGGAAGATTTAGATGTC |
| rRNA.100 | NR_003287 | rRNA_28S_18S | GAGGACTAGGAACAGACGTAATACGACTCACTATAGGGTCGCGCCGTCGGGCCCGGGGGAGGTTCTCTCGGGGCCACGCGCGCGTCCCCCGAAGAGGGGGACGGCGGAGCGAGCGCACGGGGTCGGCGTGGAATTCTCGGGTGCCAAGGATGGAAGATTTAGATGTC |
| rRNA.101 | NR_003287 | rRNA_28S_18S | GAGGACTAGGAACAGACGTAATACGACTCACTATAGGGCGGGGCCACGCGCGCGTCCCCCGAAGAGGGGGACGGCGGAGCGAGCGCACGGGGTCGGCGGCGACGTCGGCTACCCACCCGACCCGTCTTTGGAATTCTCGGGTGCCAAGGATGGAAGATTTAGATGTC |
| rRNA.102 | NR_003287 | rRNA_28S_18S | GAGGACTAGGAACAGACGTAATACGACTCACTATAGGGGGACGGCGGAGCGAGCGCACGGGGTCGGCGGCGACGTCGGCTACCCACCCGACCCGTCTTGAAACACGGACCAAGGAGTCTAACACGTGCTGGAATTCTCGGGTGCCAAGGATGGAAGATTTAGATGTC |
| rRNA.103 | NR_003287 | rRNA_28S_18S | GAGGACTAGGAACAGACGTAATACGACTCACTATAGGGGCGACGTCGGCTACCCACCCGACCCGTCTTGAAACACGGACCAAGGAGTCTAACACGTGCGCGAGTCGGGGGCTCGCACGAAAGCCGCCGTGGAATTCTCGGGTGCCAAGGATGGAAGATTTAGATGTC |
| rRNA.104 | NR_003287 | rRNA_28S_18S | GAGGACTAGGAACAGACGTAATACGACTCACTATAGGGGCAAACACGGACCAAGGAGTCTAACACGTGCGCGAGTCGGGGGCTCGCACGAAAGCCGCCGTGGCGCAATGAAGGTGAAGGCCGGCGCGCTTGGAATTCTCGGGTGCCAAGGATGGAAGATTTAGATGTC |
| rRNA.105 | NR_003287 | rRNA_28S_18S | GAGGACTAGGAACAGACGTAATACGACTCACTATAGGGGCGAGTCGGGGGCTCGCACGAAAGCCGCCGTGGCGCAATGAAGGTGAAGGCCGGCGCGCTCGCCGGCCGAGGTGGGATCCCGAGGCCTCTTGGAATTCTCGGGTGCCAAGGATGGAAGATTTAGATGTC |
| rRNA.106 | NR_003287 | rRNA_28S_18S | GAGGACTAGGAACAGACGTAATACGACTCACTATAGGGTGGCGCAATGAAGGTGAAGGCCGGCGCGCTCGCCGGCCGAGGTGGGATCCCGAGGCCTCTCCAGTCCGCCGAGGGCGCACCACCGGCCCGTGGAATTCTCGGGTGCCAAGGATGGAAGATTTAGATGTC |
| rRNA.107 | NR_003287 | rRNA_28S_18S | GAGGACTAGGAACAGACGTAATACGACTCACTATAGGGCGCCGGCCGAGGTGGGATCCCGAGGCCTCTCCAGTCCGCCGAGGGCGCACCACCGGCCCGTCTCGCCCGCCGCGCCGGGGAGGTGGAGCATGGAATTCTCGGGTGCCAAGGATGGAAGATTTAGATGTC |
| rRNA.108 | NR_003287 | rRNA_28S_18S | GAGGACTAGGAACAGACGTAATACGACTCACTATAGGGCCAGTCCGCCGAGGGCGCACCACCGGCCCGTCTCGCCCGCCGCGCCGGGGAGGTGGAGCACGAGCGCACGTGTTAGGACCCGAAAGATGGTGGAATTCTCGGGTGCCAAGGATGGAAGATTTAGATGTC |
| rRNA.109 | NR_003287 | rRNA_28S_18S | GAGGACTAGGAACAGACGTAATACGACTCACTATAGGGTCTCGCCCGCCGCGCCGGGGAGGTGGAGCACGAGCGCACGTGTTAGGACCCGAAAGATGGTGAACTATGCCTGGGCAGGGCGAAGCCAGATGGAATTCTCGGGTGCCAAGGATGGAAGATTTAGATGTC |
| rRNA.110 | NR_003287 | rRNA_28S_18S | GAGGACTAGGAACAGACGTAATACGACTCACTATAGGGCGAGCGCACGTGTTAGGACCCGAAAGATGGTGAACTATGCCTGGGCAGGGCGAAGCCAGAGGAAACTCTGGTGGAGGTCCGTAGCGGTCCTGGAATTCTCGGGTGCCAAGGATGGAAGATTTAGATGTC |
| rRNA.111 | NR_003287 | rRNA_28S_18S | GAGGACTAGGAACAGACGTAATACGACTCACTATAGGGTGAACTATGCCTGGGCAGGGCGAAGCCAGAGGAAACTCTGGTGGAGGTCCGTAGCGGTCCTGACGTGCAAATCGGTCGTCCGACCTGGGTTGGAATTCTCGGGTGCCAAGGATGGAAGATTTAGATGTC |
| rRNA.112 | NR_003287 | rRNA_28S_18S | GAGGACTAGGAACAGACGTAATACGACTCACTATAGGGGGGAAACTCTGGTGGAGGTCCGTAGCGGTCCTGACGTGCAAATCGGTCGTCCGACCTGGGTATAGGGGCGAAAGACTAATCGAACCATCTATGGAATTCTCGGGTGCCAAGGATGGAAGATTTAGATGTC |
| rRNA.113 | NR_003287 | rRNA_28S_18S | GAGGACTAGGAACAGACGTAATACGACTCACTATAGGGTGACGTGCAAATCGGTCGTCCGACCTGGGTATAGGGGCGAAAGACTAATCGAACCATCTAGTAGCTGGTTCCCTCCGAAGTTTCCCTCAGTGGAATTCTCGGGTGCCAAGGATGGAAGATTTAGATGTC |
| rRNA.114 | NR_003287 | rRNA_28S_18S | GAGGACTAGGAACAGACGTAATACGACTCACTATAGGGATAGGGGCGAAAGACTAATCGAACCATCTAGTAGCTGGTTCCCTCCGAAGTTTCCCTCAGGATAGCTGGCGCTCTCGCAGACCCGACGCATGGAATTCTCGGGTGCCAAGGATGGAAGATTTAGATGTC |
| rRNA.115 | NR_003287 | rRNA_28S_18S | GAGGACTAGGAACAGACGTAATACGACTCACTATAGGGGTAGCTGGTTCCCTCCGAAGTTTCCCTCAGGATAGCTGGCGCTCTCGCAGACCCGACGCACCCCCGCCACGCAGTTTTATCCGGTAAAGCTGGAATTCTCGGGTGCCAAGGATGGAAGATTTAGATGTC |
| rRNA.116 | NR_003287 | rRNA_28S_18S | GAGGACTAGGAACAGACGTAATACGACTCACTATAGGGGATAGCTGGCGCTCTCGCAGACCCGACGCACCCCCGCCACGCAGTTTTATCCGGTAAAGCGAATGATTAGAGGTCTTGGGGCCGAAACGATGGAATTCTCGGGTGCCAAGGATGGAAGATTTAGATGTC |
| rRNA.117 | NR_003287 | rRNA_28S_18S | GAGGACTAGGAACAGACGTAATACGACTCACTATAGGGCCCCCGCCACGCAGTTTTATCCGGTAAAGCGAATGATTAGAGGTCTTGGGGCCGAAACGATCTCAACCTATTCTCAAACTTTAAATGGGTTGGAATTCTCGGGTGCCAAGGATGGAAGATTTAGATGTC |
| rRNA.118 | NR_003287 | rRNA_28S_18S | GAGGACTAGGAACAGACGTAATACGACTCACTATAGGGGAATGATTAGAGGTCTTGGGGCCGAAACGATCTCAACCTATTCTCAAACTTTAAATGGGTAAGAAGCCCGGCTCGCTGGCGTGGAGCCGGTGGAATTCTCGGGTGCCAAGGATGGAAGATTTAGATGTC |
| rRNA.119 | NR_003287 | rRNA_28S_18S | GAGGACTAGGAACAGACGTAATACGACTCACTATAGGGTCTCAACCTATTCTCAAACTTTAAATGGGTAAGAAGCCCGGCTCGCTGGCGTGGAGCCGGGCGTGGAATGCGAGTGCCTAGTGGGCCACTTGGAATTCTCGGGTGCCAAGGATGGAAGATTTAGATGTC |
| rRNA.120 | NR_003287 | rRNA_28S_18S | GAGGACTAGGAACAGACGTAATACGACTCACTATAGGGGAAGAAGCCCGGCTCGCTGGCGTGGAGCCGGGCGTGGAATGCGAGTGCCTAGTGGGCCACTTTTGGTAAGCAGAACTGGCGCTGCGGGATGTGGAATTCTCGGGTGCCAAGGATGGAAGATTTAGATGTC |
| rRNA.121 | NR_003287 | rRNA_28S_18S | GAGGACTAGGAACAGACGTAATACGACTCACTATAGGGGCGTGGAATGCGAGTGCCTAGTGGGCCACTTTTGGTAAGCAGAACTGGCGCTGCGGGATGAACCGAACGCCGGGTTAAGGCGCCCGATGCTGGAATTCTCGGGTGCCAAGGATGGAAGATTTAGATGTC |
| rRNA.122 | NR_003287 | rRNA_28S_18S | GAGGACTAGGAACAGACGTAATACGACTCACTATAGGGTTTGGTAAGCAGAACTGGCGCTGCGGGATGAACCGAACGCCGGGTTAAGGCGCCCGATGCCGACGCTCATCAGACCCCAGAAAAGGTGTTTGGAATTCTCGGGTGCCAAGGATGGAAGATTTAGATGTC |
| rRNA.123 | NR_003287 | rRNA_28S_18S | GAGGACTAGGAACAGACGTAATACGACTCACTATAGGGAACCGAACGCCGGGTTAAGGCGCCCGATGCCGACGCTCATCAGACCCCAGAAAAGGTGTTGGTTGATATAGACAGCAGGACGGTGGCCATTGGAATTCTCGGGTGCCAAGGATGGAAGATTTAGATGTC |

EP 4 239 083 A2

| ASO ID | Accession | Group ID | Custom Array sequence |
|---|---|---|---|
| rRNA.124 | NR_003287 | rRNA_28S_18S | GAGGACTAGGAACAGACGTAATACGACTCACTATAGGGCGACGCTCATCAGACCCCAGAAAAGGTGTTGGTTGATATAGACAGCAGGACGGTGGCCATGGAAGTCGGAATCCGCTAAGGAGTGTGTAATGGAATTCTCGGGTGCCAAGGATGGAAGATTTAGATGTC |
| rRNA.125 | NR_003287 | rRNA_28S_18S | GAGGACTAGGAACAGACGTAATACGACTCACTATAGGGGGGTTGATATAGACAGCAGGACGGTGGCCATGGAAGTCGGAATCCGCTAAGGAGTGTGTAACAACTCACCTGCCGAATCAACTAGCCCTGATGGAATTCTCGGGTGCCAAGGATGGAAGATTTAGATGTC |
| rRNA.126 | NR_003287 | rRNA_28S_18S | GAGGACTAGGAACAGACGTAATACGACTCACTATAGGGGGGAAGTCGGAATCCGCTAAGGAGTGTGTAACAACTCACCTGCCGAATCAACTAGCCCTGAAAATGGATGGCGCTGGAGCGTCGGGCCCATTGGAATTCTCGGGTGCCAAGGATGGAAGATTTAGATGTC |
| rRNA.127 | NR_003287 | rRNA_28S_18S | GAGGACTAGGAACAGACGTAATACGACTCACTATAGGGCAACTCACCTGCCGAATCAACTAGCCCTGAAAATGGATGGCGCTGGAGCGTCGGGCCCATACCCGGCCGTCGCCGGCAGTCGAGAGTGGATGGAATTCTCGGGTGCCAAGGATGGAAGATTTAGATGTC |
| rRNA.128 | NR_003287 | rRNA_28S_18S | GAGGACTAGGAACAGACGTAATACGACTCACTATAGGGAAATGGATGGCGCTGGAGCGTCGGGCCCATACCCGGCCGTCGCCGGCAGTCGAGAGTGGACGGGAGCGGCGGGGGCGGCGCGCGCGCGCGTGGAATTCTCGGGTGCCAAGGATGGAAGATTTAGATGTC |
| rRNA.129 | NR_003287 | rRNA_28S_18S | GAGGACTAGGAACAGACGTAATACGACTCACTATAGGGACCCGGCCGTCGCCGGCAGTCGAGAGTGGACGGGAGCGGCGGGGGCGGCGCGCGCGCGCGCGTGTGGTGTGCGTCGGAGGGCGGCGGCGGCGGCTGGAATTCTCGGGTGCCAAGGATGGAAGATTTAGATGTC |
| rRNA.130 | NR_003287 | rRNA_28S_18S | GAGGACTAGGAACAGACGTAATACGACTCACTATAGGGCGGGAGCGGCGGGGGCGGCGCGCGCGCGCGCGTGTGGTGTGCGTCGGAGGGCGGCGGCGGCGGCGGCGGGGGTGTGGGGTCCTTCTGGAATTCTCGGGTGCCAAGGATGGAAGATTTAGATGTC |
| rRNA.131 | NR_003287 | rRNA_28S_18S | GAGGACTAGGAACAGACGTAATACGACTCACTATAGGGCGCGTGTGGTGTGCGTCGGAGGGCGGCGGCGGCGGCGGCGGCGGGGGTGTGGGTCCTTCCCCCGCCCCCCCCCCACGCCTCCTCCCCTTGGAATTCTCGGGTGCCAAGGATGGAAGATTTAGATGTC |
| rRNA.132 | NR_003287 | rRNA_28S_18S | GAGGACTAGGAACAGACGTAATACGACTCACTATAGGGGGGCGGCGGCGGCGGGGGTGTGGGGTCCTTCCCCCGCCCCCCCCCCACGCCTCCTCCCCTCCTCCCGCCCACGCCCCGCTCCCCGCCCCCTGGAATTCTCGGGTGCCAAGGATGGAAGATTTAGATGTC |
| rRNA.133 | NR_003287 | rRNA_28S_18S | GAGGACTAGGAACAGACGTAATACGACTCACTATAGGGCCCCGCCCCCCCCCCACGCCTCCTCCCCTCCTCCCGCCCACGCCCCGCTCCCCGCCCCCGGAGCCCCGCGGACGCTACGCCGCGACGAGTGGAATTCTCGGGTGCCAAGGATGGAAGATTTAGATGTC |
| rRNA.134 | NR_003287 | rRNA_28S_18S | GAGGACTAGGAACAGACGTAATACGACTCACTATAGGGCCTCCCGCCCACGCCCCGCTCCCCGCCCCCGGAGCCCCGCGGACGCTACGCCGCGACGAGTAGGAGGGCCGCTGCGGTGAGCCTTGAAGCTGGAATTCTCGGGTGCCAAGGATGGAAGATTTAGATGTC |
| rRNA.135 | NR_003287 | rRNA_28S_18S | GAGGACTAGGAACAGACGTAATACGACTCACTATAGGGGGGAGCCCCGCGGACGCTACGCCGCGACGAGTAGGAGGGCCGCTGCGGTGAGCCTTGAAGCCTAGGGCGCGGGCCCGGGTGGAGCCGCCGCTGGAATTCTCGGGTGCCAAGGATGGAAGATTTAGATGTC |
| rRNA.136 | NR_003287 | rRNA_28S_18S | GAGGACTAGGAACAGACGTAATACGACTCACTATAGGGTAGGAGGGCCGCTGCGGTGAGCCTTGAAGCCTAGGGCGCGGGCCCGGGTGGAGCCGCCGCAGGTGCAGATCTTGGTGGTAGTAGCAAATATGGAATTCTCGGGTGCCAAGGATGGAAGATTTAGATGTC |
| rRNA.137 | NR_003287 | rRNA_28S_18S | GAGGACTAGGAACAGACGTAATACGACTCACTATAGGGCTAGGGCGCGGGCCCGGGTGGAGCCGCCGCAGGTGCAGATCTTGGTGGTAGTAGCAAATATTCAAACGAGAACTTTGAAGGCCGAAGTGGTGGAATTCTCGGGTGCCAAGGATGGAAGATTTAGATGTC |
| rRNA.138 | NR_003287 | rRNA_28S_18S | GAGGACTAGGAACAGACGTAATACGACTCACTATAGGGAGGTGCAGATCTTGGTGGTAGTAGCAAATATTCAAACGAGAACTTTGAAGGCCGAAGTGGAGAAGGGTTCCATGTGAACAGCAGTTGAACTGGAATTCTCGGGTGCCAAGGATGGAAGATTTAGATGTC |
| rRNA.139 | NR_003287 | rRNA_28S_18S | GAGGACTAGGAACAGACGTAATACGACTCACTATAGGGTTCAAACGAGAACTTTGAAGGCCGAAGTGGAGAAGGGTTCCATGTGAACAGCAGTTGAACATGGGTCAGTCGGTCCTGAGAGATGGGCGATGGAATTCTCGGGTGCCAAGGATGGAAGATTTAGATGTC |
| rRNA.140 | NR_003287 | rRNA_28S_18S | GAGGACTAGGAACAGACGTAATACGACTCACTATAGGGAGAAGGGTTCCATGTGAACAGCAGTTGAACATGGGTCAGTCGGTCCTGAGAGATGGGCGAGCGCCGTTCCGAAGGGACGGGCGATGGCCTTGGAATTCTCGGGTGCCAAGGATGGAAGATTTAGATGTC |
| rRNA.141 | NR_003287 | rRNA_28S_18S | GAGGACTAGGAACAGACGTAATACGACTCACTATAGGGATGGGTCAGTCGGTCCTGAGAGATGGGCGAGCGCCGTTCCGAAGGGACGGGCGATGGCCTCCGTTGCCCTCGGCCGATCGAAAGGGAGTCTGGAATTCTCGGGTGCCAAGGATGGAAGATTTAGATGTC |
| rRNA.142 | NR_003287 | rRNA_28S_18S | GAGGACTAGGAACAGACGTAATACGACTCACTATAGGGGCGCCGTTCCGAAGGGACGGGCGATGGCCTCCGTTGCCCTCGGCCGATCGAAAGGGAGTCGGGTTCAGATCCCCGAATCCGGAGTGGCGGTGGAATTCTCGGGTGCCAAGGATGGAAGATTTAGATGTC |
| rRNA.143 | NR_003287 | rRNA_28S_18S | GAGGACTAGGAACAGACGTAATACGACTCACTATAGGGCCGTTGCCCTCGGCCGATCGAAAGGGAGTCGGGTTCAGATCCCCGAATCCGGAGTGGCGGAGATGGGCGCCGCGAGGCGTCCAGTGCGGTTGGAATTCTCGGGTGCCAAGGATGGAAGATTTAGATGTC |
| rRNA.144 | NR_003287 | rRNA_28S_18S | GAGGACTAGGAACAGACGTAATACGACTCACTATAGGGGGGGTTCAGATCCCCGAATCCGGAGTGGCGGAGATGGGCGCCGCGAGGCGTCCAGTGCGGTAACGCGACCGATCCCGGAGAAGCCGGCGGGTGGAATTCTCGGGTGCCAAGGATGGAAGATTTAGATGTC |
| rRNA.145 | NR_003287 | rRNA_28S_18S | GAGGACTAGGAACAGACGTAATACGACTCACTATAGGGAGATGGGCGCCGCGAGGCGTCCAGTGCGGTAACGCGACCGATCCCGGAGAAGCCGGCGGGAGCCCCGGGGAGAGTTCTCTTTTCTTTGTGTGGAATTCTCGGGTGCCAAGGATGGAAGATTTAGATGTC |
| rRNA.146 | NR_003287 | rRNA_28S_18S | GAGGACTAGGAACAGACGTAATACGACTCACTATAGGGAACGCGACCGATCCCGGAGAAGCCGGCGGGAGCCCCGGGGAGAGTTCTCTTTTCTTTGTGAAGGGCAGGGCGCCCTGGAATGGGTTCGCCTGGAATTCTCGGGTGCCAAGGATGGAAGATTTAGATGTC |
| rRNA.147 | NR_003287 | rRNA_28S_18S | GAGGACTAGGAACAGACGTAATACGACTCACTATAGGGAGCCCCGGGGAGAGTTCTCTTTTCTTTGTGAAGGGCAGGGCGCCCTGGAATGGGTTCGCCCCGAGAGAGGGGCCCGTGCCTTGGAAAGCGTGGAATTCTCGGGTGCCAAGGATGGAAGATTTAGATGTC |
| rRNA.148 | NR_003287 | rRNA_28S_18S | GAGGACTAGGAACAGACGTAATACGACTCACTATAGGGAAGGGCAGGGCGCCCTGGAATGGGTTCGCCCCGAGAGAGGGGCCCGTGCCTTGGAAAGCGTGCGGTTCCGGCGGCGTCCGGTGAGCTCTTGGAATTCTCGGGTGCCAAGGATGGAAGATTTAGATGTC |

| ASO ID | Accession | Group ID | Custom Array sequence |
|---|---|---|---|
| rRNA.149 | NR_003287 | rRNA_28S_18S | GAGGACTAGGAACAGACGTAATACGACTCACTATAGGGCCGAGAGAGGGGCCCGTGCCTTGGAAAGCGTCGCGGTTCCGGCGGCGTCCGGTGAGCTCTCGCTGGCCCTTGAAAATCCGGGGGAGAGGGTGGAATTCTCGGGTGCCAAGGATGGAAGATTTAGATGTC |
| rRNA.150 | NR_003287 | rRNA_28S_18S | GAGGACTAGGAACAGACGTAATACGACTCACTATAGGGTCGCGGTTCCGGCGGCGTCCGGTGAGCTCTCGCTGGCCCTTGAAAATCCGGGGGAGAGGGTGTAAATCTCGCGCCGGGCCGTACCCATATTGGAATTCTCGGGTGCCAAGGATGGAAGATTTAGATGTC |
| rRNA.151 | NR_003287 | rRNA_28S_18S | GAGGACTAGGAACAGACGTAATACGACTCACTATAGGGCGCTGGCCCTTGAAAATCCGGGGGAGAGGGTGTAAATCTCGCGCCGGGCCGTACCCATATCCGCAGCAGGTCTCCAAGGTGAACAGCCTCTGGAATTCTCGGGTGCCAAGGATGGAAGATTTAGATGTC |
| rRNA.152 | NR_003287 | rRNA_28S_18S | GAGGACTAGGAACAGACGTAATACGACTCACTATAGGGTGTAAATCTCGCGCCGGGCCGTACCCATATCCGCAGCAGGTCTCCAAGGTGAACAGCCTCTGGCATGTTGGAACAATGTAGGTAAGGGAATGGAATTCTCGGGTGCCAAGGATGGAAGATTTAGATGTC |
| rRNA.153 | NR_003287 | rRNA_28S_18S | GAGGACTAGGAACAGACGTAATACGACTCACTATAGGGCCGCAGCAGGTCTCCAAGGTGAACAGCCTCTGGCATGTTGGAACAATGTAGGTAAGGGAAGTCGGCAAGCCGGATCCGTAACTTCGGGATTGGAATTCTCGGGTGCCAAGGATGGAAGATTTAGATGTC |
| rRNA.154 | NR_003287 | rRNA_28S_18S | GAGGACTAGGAACAGACGTAATACGACTCACTATAGGGTGGCATGTTGGAACAATGTAGGTAAGGGAAGTCGGCAAGCCGGATCCGTAACTTCGGGATAAGGATTGGCTCTAAGGGCTGGGTCGGTCGTGGAATTCTCGGGTGCCAAGGATGGAAGATTTAGATGTC |
| rRNA.155 | NR_003287 | rRNA_28S_18S | GAGGACTAGGAACAGACGTAATACGACTCACTATAGGGGTCGGCAAGCCGGATCCGTAACTTCGGGATAAGGATTGGCTCTAAGGGCTGGGTCGGTCGGGCTGGGGCGCGAAGCGGGGCTGGGCGCGCTGGAATTCTCGGGTGCCAAGGATGGAAGATTTAGATGTC |
| rRNA.156 | NR_003287 | rRNA_28S_18S | GAGGACTAGGAACAGACGTAATACGACTCACTATAGGGAAGGATTGGCTCTAAGGGCTGGGTCGGTCGGGCTGGGGCGCGAAGCGGGGCTGGGCGCGCGCCGGCTGGACGAGGCGCCGCCGCCCCCTGGAATTCTCGGGTGCCAAGGATGGAAGATTTAGATGTC |
| rRNA.157 | NR_003287 | rRNA_28S_18S | GAGGACTAGGAACAGACGTAATACGACTCACTATAGGGGGCTGGGGCGCGAAGCGGGGCTGGGCGCGCGCCGCGGCTGGACGAGGCGCCGCCGCCCCCCCCACGCCCGGGGCACCCCCCTCGCGGCCCTGGAATTCTCGGGTGCCAAGGATGGAAGATTTAGATGTC |
| rRNA.158 | NR_003287 | rRNA_28S_18S | GAGGACTAGGAACAGACGTAATACGACTCACTATAGGGGCCGCGGCTGGACGAGGCGCCGCCGCCCCCCCCACGCCCGGGGCACCCCCCTCGCGGCCCTCCCCCGCCCCACCCCGCGCGCGCCGCTCGTGGAATTCTCGGGTGCCAAGGATGGAAGATTTAGATGTC |
| rRNA.159 | NR_003287 | rRNA_28S_18S | GAGGACTAGGAACAGACGTAATACGACTCACTATAGGGCCCACGCCCGGGGCACCCCCCTCGCGGCCCTCCCCCGCCCCACCCCGCGCGCGCCGCTCGCTCCCTCCCCGCCCCGCGCCCTCTCTCTCTTGGAATTCTCGGGTGCCAAGGATGGAAGATTTAGATGTC |
| rRNA.160 | NR_003287 | rRNA_28S_18S | GAGGACTAGGAACAGACGTAATACGACTCACTATAGGGTCCCCCGCCCCACCCCGCGCGCGCCGCTCGCTCCCTCCCCGCCCCGCGCCCTCTCTCTCTCTCTCTCCCCCGCTCCCCGTCCTCCCCCCTTGGAATTCTCGGGTGCCAAGGATGGAAGATTTAGATGTC |
| rRNA.161 | NR_003287 | rRNA_28S_18S | GAGGACTAGGAACAGACGTAATACGACTCACTATAGGGCTCCCTCCCCGCCCCGCGCCCTCTCTCTCTCTCTCTCCCCCGCTCCCCGTCCTCCCCCCTCCCCGGGGGAGCGCCGCGTGGGGCGGCGGTGGAATTCTCGGGTGCCAAGGATGGAAGATTTAGATGTC |
| rRNA.162 | NR_003287 | rRNA_28S_18S | GAGGACTAGGAACAGACGTAATACGACTCACTATAGGGCTCTCTCCCCCGCTCCCCGTCCTCCCCCCTCCCCGGGGGAGCGCCGCGTGGGGCGGCGGCGGGGGGAGAAGGGTCGGGGCGGCAGGGGCTGGAATTCTCGGGTGCCAAGGATGGAAGATTTAGATGTC |
| rRNA.163 | NR_003287 | rRNA_28S_18S | GAGGACTAGGAACAGACGTAATACGACTCACTATAGGGCCCCGGGGGAGCGCCGCGTGGGGCGGCGGCGGGGGGAGAAGGGTCGGGGCGGCAGGGGCCGGCGGCGGCCGCCGCGGGGCCCCGGCGGTGGAATTCTCGGGTGCCAAGGATGGAAGATTTAGATGTC |
| rRNA.164 | NR_003287 | rRNA_28S_18S | GAGGACTAGGAACAGACGTAATACGACTCACTATAGGGCGGGGGGAGAAGGGTCGGGGCGGCAGGGGCCGGCGGCGGCCGCCGCGGGGCCCCGGCGGCGGGGGCACGGTCCCCCGCGAGGGGGGCCCTGGAATTCTCGGGTGCCAAGGATGGAAGATTTAGATGTC |
| rRNA.165 | NR_003287 | rRNA_28S_18S | GAGGACTAGGAACAGACGTAATACGACTCACTATAGGGCGGCGGCGGCCCGCCGCGGGGCCCCGGCGGCGGGGGCACGGTCCCCCGCGAGGGGGGCCCGGGCACCCGGGGGGGCCGGCGGCGGCGGCGATGGAATTCTCGGGTGCCAAGGATGGAAGATTTAGATGTC |
| rRNA.166 | NR_003287 | rRNA_28S_18S | GAGGACTAGGAACAGACGTAATACGACTCACTATAGGGCGGGGGCACGGTCCCCCGCGAGGGGGGCCCGGGCACCCGGGGGGGCCGGCGGCGGCGGCGGCGACTCTGGACGCGAGCCGGGCCCTTCCCGTGGTGGAATTCTCGGGTGCCAAGGATGGAAGATTTAGATGTC |
| rRNA.167 | NR_003287 | rRNA_28S_18S | GAGGACTAGGAACAGACGTAATACGACTCACTATAGGGGGGGCACCCGGGGGGCCGGCGGCGGCGGCGGCGACTCTGGACGCGAGCCGGGCCCTTCCCGTGGATCGCCCCAGCTGCGGCGGGCGTCGCGGCCTGGAATTCTCGGGTGCCAAGGATGGAAGATTTAGATGTC |
| rRNA.168 | NR_003287 | rRNA_28S_18S | GAGGACTAGGAACAGACGTAATACGACTCACTATAGGGCTCTGGACGCGAGCCGGGCCCTTCCCGTGGATCGCCCCAGCTGCGGCGGGCGTCGCGGCCTGGACCCCCGGGGAGCCCGGCGGGCGCCGGCGCTGGAATTCTCGGGTGCCAAGGATGGAAGATTTAGATGTC |
| rRNA.169 | NR_003287 | rRNA_28S_18S | GAGGACTAGGAACAGACGTAATACGACTCACTATAGGGATCGCCCCAGCTGCGGCGGGCGTCGCGGCCTGGACCCCCGGGGAGCCCGGCGGGCGCCGGCGCGCCCCCCCCCCCCACCCCACGTCTCGTCGCGCGTGGAATTCTCGGGTGCCAAGGATGGAAGATTTAGATGTC |
| rRNA.170 | NR_003287 | rRNA_28S_18S | GAGGACTAGGAACAGACGTAATACGACTCACTATAGGGGCCCCCGGGGAGCCCGGCGGGCGCCGGCGCGCCCCCCCCCCCACCCCACGTCTCGTCGCGCGCGGTCCGCTGGGGGCGGGGAGCGGTCGTGGAATTCTCGGGTGCCAAGGATGGAAGATTTAGATGTC |
| rRNA.171 | NR_003287 | rRNA_28S_18S | GAGGACTAGGAACAGACGTAATACGACTCACTATAGGGGCCCCCCCCCCCCACCCCACGTCTCGTCGCGCGCGCGTCCGCTGGGGGCGGGGAGCGGTCGGGCGGCGGCGGTCGGCGGGCGGCGGGGCGGTGGAATTCTCGGGTGCCAAGGATGGAAGATTTAGATGTC |
| rRNA.172 | NR_003287 | rRNA_28S_18S | GAGGACTAGGAACAGACGTAATACGACTCACTATAGGGCGCGCGTCCGCTGGGGGCGGGGAGCGGTCGGGCGGCGGCGGTCGGCGGGCGGCGGGGCGGGGCGGTTCGTCCCCCCGCCCTACCCCCCGTGGAATTCTCGGGTGCCAAGGATGGAAGATTTAGATGTC |
| rRNA.173 | NR_003287 | rRNA_28S_18S | GAGGACTAGGAACAGACGTAATACGACTCACTATAGGGGGCGGCGGCGGTCGGCGGGCGGCGGGGCGGGGCGGTTCGTCCCCCCGCCCTACCCCCCCGGCCCCGTCCGCCCCCCGTTCCCCCCTCCTCTGGAATTCTCGGGTGCCAAGGATGGAAGATTTAGATGTC |

| ASO ID | Accession | Group ID | Custom Array sequence |
|---|---|---|---|
| rRNA.174 | NR_003287 | rRNA_28S_18S | GAGGACTAGGAACAGACGTAATACGACTCACTATAGGGGGCGGTTCGTCCCCCCGCCCTACCCCCCCGGCCCCGTCCGCCCCCCGTTCCCCCCTCCTCCTCGGCGCGCGGCGGCGGCGGCGGGCGGCGTGGAATTCTCGGGTGCCAAGGATGGAAGATTTAGATGTC |
| rRNA.175 | NR_003287 | rRNA_28S_18S | GAGGACTAGGAACAGACGTAATACGACTCACTATAGGGGGCCCCGTCCGCCCCCCGTTCCCCCCTCCTCCTCGGCGCGCGGCGGCGGCGGCGGGCGGCGGAGGGGCCGCGGGCCGGTCCCCCCCGCCGGTGGAATTCTCGGGTGCCAAGGATGGAAGATTTAGATGTC |
| rRNA.176 | NR_003287 | rRNA_28S_18S | GAGGACTAGGAACAGACGTAATACGACTCACTATAGGGCTCGGCGCGCGGCGGCGGCGGCGGGCGGCGGAGGGGCCGCGGGCCGGTCCCCCCCGCCGGGTCCGCCCCCGGGGCCGCGGTTCCGCGCGGCGCCTCGCCTCGGCCGGCGCCTAGCAGCCGTGGAATTCTCGGGTGCCAAGGATGGAAGATTTAGATGTC |
| rRNA.177 | NR_003287 | rRNA_28S_18S | GAGGACTAGGAACAGACGTAATACGACTCACTATAGGGGAGGGGCCGCGGGCCGGTCCCCCCCGCCGGGTCCGCCCCCGGGGCCGCGGTTCCGCGCGGCGCCTCGCCTCGGCCGGCGCCTAGCAGCCGTGGAATTCTCGGGTGCCAAGGATGGAAGATTTAGATGTC |
| rRNA.178 | NR_003287 | rRNA_28S_18S | GAGGACTAGGAACAGACGTAATACGACTCACTATAGGGGTCCGCCCCCGGGGCCGCGGTTCCGCGCGGCGCCTCGCCTCGGCCGGCGCCTAGCAGCCGACTTAGAACTGGTGCGGACCAGGGGAATCCTGGAATTCTCGGGTGCCAAGGATGGAAGATTTAGATGTC |
| rRNA.179 | NR_003287 | rRNA_28S_18S | GAGGACTAGGAACAGACGTAATACGACTCACTATAGGGCGCCTCGCCTCGGCCGGCGCCTAGCAGCCGACTTAGAACTGGTGCGGACCAGGGGAATCCGACTGTTTAATTAAAACAAAGCATCGCGAATGGAATTCTCGGGTGCCAAGGATGGAAGATTTAGATGTC |
| rRNA.180 | NR_003287 | rRNA_28S_18S | GAGGACTAGGAACAGACGTAATACGACTCACTATAGGGACTTAGAACTGGTGCGGACCAGGGGAATCCGACTGTTTAATTAAAACAAAGCATCGCGAAGGCCCGCGGCGGGTGTTGACGCGATGTGATTGGAATTCTCGGGTGCCAAGGATGGAAGATTTAGATGTC |
| rRNA.181 | NR_003287 | rRNA_28S_18S | GAGGACTAGGAACAGACGTAATACGACTCACTATAGGGGACTGTTTAATTAAAACAAAGCATCGCGAAGGCCCGCGGCGGGTGTTGACGCGATGTGATTTCTGCCCAGTGCTCTGAATGTCAAAGTGATGGAATTCTCGGGTGCCAAGGATGGAAGATTTAGATGTC |
| rRNA.182 | NR_003287 | rRNA_28S_18S | GAGGACTAGGAACAGACGTAATACGACTCACTATAGGGGGGCCCGCGGCGGGTGTTGACGCGATGTGATTTCTGCCCAGTGCTCTGAATGTCAAAGTGAAGAAATTCAATGAAGCGCGGGTAAACGGCGTGGAATTCTCGGGTGCCAAGGATGGAAGATTTAGATGTC |
| rRNA.183 | NR_003287 | rRNA_28S_18S | GAGGACTAGGAACAGACGTAATACGACTCACTATAGGGTTCTGCCCAGTGCTCTGAATGTCAAAGTGAAGAAATTCAATGAAGCGCGGGTAAACGGCGGGAGTAACTATGACTCTCTTAAGGTAGCCATGGAATTCTCGGGTGCCAAGGATGGAAGATTTAGATGTC |
| rRNA.184 | NR_003287 | rRNA_28S_18S | GAGGACTAGGAACAGACGTAATACGACTCACTATAGGGAGAAATTCAATGAAGCGCGGGTAAACGGCGGGAGTAACTATGACTCTCTTAAGGTAGCCAAATGCCTCGTCATCTAATTAGTGACGCGCATGGAATTCTCGGGTGCCAAGGATGGAAGATTTAGATGTC |
| rRNA.185 | NR_003287 | rRNA_28S_18S | GAGGACTAGGAACAGACGTAATACGACTCACTATAGGGGGGAGTAACTATGACTCTCTTAAGGTAGCCAAATGCCTCGTCATCTAATTAGTGACGCGCATGAATGGATGAACGAGATTCCCACTGTCCCTGGAATTCTCGGGTGCCAAGGATGGAAGATTTAGATGTC |
| rRNA.186 | NR_003287 | rRNA_28S_18S | GAGGACTAGGAACAGACGTAATACGACTCACTATAGGGAATGCCTCGTCATCTAATTAGTGACGCGCATGAATGGATGAACGAGATTCCCACTGTCCCTACCTACTATCCAGCGAAACCACAGCCAAGTGGAATTCTCGGGTGCCAAGGATGGAAGATTTAGATGTC |
| rRNA.187 | NR_003287 | rRNA_28S_18S | GAGGACTAGGAACAGACGTAATACGACTCACTATAGGGTGAATGGATGAACGAGATTCCCACTGTCCCTACCTACTATCCAGCGAAACCACAGCCAAGGGAACGGGCTTGGCGGAATCAGCGGGGAAATGGAATTCTCGGGTGCCAAGGATGGAAGATTTAGATGTC |
| rRNA.188 | NR_003287 | rRNA_28S_18S | GAGGACTAGGAACAGACGTAATACGACTCACTATAGGGTACCTACTATCCAGCGAAACCACAGCCAAGGGAACGGGCTTGGCGGAATCAGCGGGGAAAGAAGACCCTGTTGAGCTTGACTCTAGTCTGTGGAATTCTCGGGTGCCAAGGATGGAAGATTTAGATGTC |
| rRNA.189 | NR_003287 | rRNA_28S_18S | GAGGACTAGGAACAGACGTAATACGACTCACTATAGGGGGGAACGGGCTTGGCGGAATCAGCGGGGAAAGAAGACCCTGTTGAGCTTGACTCTAGTCTGGCACGGTGAAGAGACATGAGAGGTGTAGAATGGAATTCTCGGGTGCCAAGGATGGAAGATTTAGATGTC |
| rRNA.190 | NR_003287 | rRNA_28S_18S | GAGGACTAGGAACAGACGTAATACGACTCACTATAGGGGAAGACCCTGTTGAGCTTGACTCTAGTCTGGCACGGTGAAGAGACATGAGAGGTGTAGAATAAGTGGGAGGCCCCCGGCGCCCCCCCGGTTGGAATTCTCGGGTGCCAAGGATGGAAGATTTAGATGTC |
| rRNA.191 | NR_003287 | rRNA_28S_18S | GAGGACTAGGAACAGACGTAATACGACTCACTATAGGGGCACGGTGAAGAGACATGAGAGGTGTAGAATAAGTGGGAGGCCCCCGGCGCCCCCCCGGTGTCCCCGCGAGGGGCCCGGGGCGGGGTCCGCTGGAATTCTCGGGTGCCAAGGATGGAAGATTTAGATGTC |
| rRNA.192 | NR_003287 | rRNA_28S_18S | GAGGACTAGGAACAGACGTAATACGACTCACTATAGGGTAAGTGGGAGGCCCCCGGCGCCCCCCCGGTGTCCCCGCGAGGGGCCCGGGGCGGGGTCCGCCGGCCCTGCGGGCCGCCGGTGAAATACCATGGAATTCTCGGGTGCCAAGGATGGAAGATTTAGATGTC |
| rRNA.193 | NR_003287 | rRNA_28S_18S | GAGGACTAGGAACAGACGTAATACGACTCACTATAGGGGTCCCCGCGAGGGGCCCGGGGCGGGGTCCGCCGGCCCTGCGGGCCGCCGGTGAAATACCACTACTCTGATCGTTTTTTCACTGACCCGGTTGGAATTCTCGGGTGCCAAGGATGGAAGATTTAGATGTC |
| rRNA.194 | NR_003287 | rRNA_28S_18S | GAGGACTAGGAACAGACGTAATACGACTCACTATAGGGCCGGCCCTGCGGGCCGCCGGTGAAATACCACTACTCTGATCGTTTTTTCACTGACCCGGTGAGGCGGGGGGGGCGAGCCCCGAGGGGGCTCTTGGAATTCTCGGGTGCCAAGGATGGAAGATTTAGATGTC |
| rRNA.195 | NR_003287 | rRNA_28S_18S | GAGGACTAGGAACAGACGTAATACGACTCACTATAGGGCTACTCTGATCGTTTTTTCACTGACCCGGTGAGGCGGGGGGGGCGAGCCCCGAGGGGCTCTCGCTTCTGGCGCCAAGCGCCCGGCCGCGCGTGGAATTCTCGGGTGCCAAGGATGGAAGATTTAGATGTC |
| rRNA.196 | NR_003287 | rRNA_28S_18S | GAGGACTAGGAACAGACGTAATACGACTCACTATAGGGGAGGCGGGGGGGGCGAGCCCCGAGGGGCTCTCGCTTCTGGCGCCAAGCGCCCGGCCGCGCGCCGGCCGGGCGCGACCCGCTCCGGGGACAGTGGAATTCTCGGGTGCCAAGGATGGAAGATTTAGATGTC |
| rRNA.197 | NR_003287 | rRNA_28S_18S | GAGGACTAGGAACAGACGTAATACGACTCACTATAGGGCGCTTCTGGCGCCAAGCGCCCGGCCGCGCGCCGGCCGGGCGCGACCCGCTCCGGGGACAGTGCCAGGTGGGGAGTTTGACTGGGGCGGTATGGAATTCTCGGGTGCCAAGGATGGAAGATTTAGATGTC |
| rRNA.198 | NR_003287 | rRNA_28S_18S | GAGGACTAGGAACAGACGTAATACGACTCACTATAGGGCCGGCCGGGCGCGACCCGCTCCGGGGACAGTGCCAGGTGGGGAGTTTGACTGGGGCGGTACACCTGTCAAACGGTAACGCAGGTGTCCTATGGAATTCTCGGGTGCCAAGGATGGAAGATTTAGATGTC |

| ASO ID | Accession | Group ID | Custom Array sequence |
|---|---|---|---|
| rRNA.199 | NR_003287 | rRNA_28S_18S | GAGGACTAGGAACAGACGTAATACGACTCACTATAGGGTGCCAGGTGGGGAGTTTGACTGGGGCGGTACACCTGTCAAACGGTAACGCAGGTGTCCTAAGGCGAGCTCAGGGAGGACAGAAACCTCCCTGGAATTCTCGGGTGCCAAGGATGGAAGATTTAGATGTC |
| rRNA.200 | NR_003287 | rRNA_28S_18S | GAGGACTAGGAACAGACGTAATACGACTCACTATAGGGCACCTGTCAAACGGTAACGCAGGTGTCCTAAGGCGAGCTCAGGGAGGACAGAAACCTCCCGTGGAGCAGAAGGGCAAAAGCTCGCTTGATTGGAATTCTCGGGTGCCAAGGATGGAAGATTTAGATGTC |
| rRNA.201 | NR_003287 | rRNA_28S_18S | GAGGACTAGGAACAGACGTAATACGACTCACTATAGGGAGGCGAGCTCAGGGAGGACAGAAACCTCCCGTGGAGCAGAAGGGCAAAAGCTCGCTTGATCTTGATTTTCAGTACGAATACAGACCGTGATGGAATTCTCGGGTGCCAAGGATGGAAGATTTAGATGTC |
| rRNA.202 | NR_003287 | rRNA_28S_18S | GAGGACTAGGAACAGACGTAATACGACTCACTATAGGGGTGGAGCAGAAGGGCAAAAGCTCGCTTGATCTTGATTTTCAGTACGAATACAGACCGTGAAAGCGGGGCCTCACGATCCTTCTGACCTTTTGGAATTCTCGGGTGCCAAGGATGGAAGATTTAGATGTC |
| rRNA.203 | NR_003287 | rRNA_28S_18S | GAGGACTAGGAACAGACGTAATACGACTCACTATAGGGCTTGATTTTCAGTACGAATACAGACCGTGAAAGCGGGGCCTCACGATCCTTCTGACCTTTTGGGTTTTAAGCAGGAGGTGTCAGAAAAGTTGGAATTCTCGGGTGCCAAGGATGGAAGATTTAGATGTC |
| rRNA.204 | NR_003287 | rRNA_28S_18S | GAGGACTAGGAACAGACGTAATACGACTCACTATAGGGAAGCGGGGCCTCACGATCCTTCTGACCTTTTGGGTTTTAAGCAGGAGGTGTCAGAAAAGTTACCACAGGGATAACTGGCTTGTGGCGGCCTGGAATTCTCGGGTGCCAAGGATGGAAGATTTAGATGTC |
| rRNA.205 | NR_003287 | rRNA_28S_18S | GAGGACTAGGAACAGACGTAATACGACTCACTATAGGGTGGGTTTTAAGCAGGAGGTGTCAGAAAAGTTACCACAGGGATAACTGGCTTGTGGCGGCCAAGCGTTCATAGCGACGTCGCTTTTTGATCTGGAATTCTCGGGTGCCAAGGATGGAAGATTTAGATGTC |
| rRNA.206 | NR_003287 | rRNA_28S_18S | GAGGACTAGGAACAGACGTAATACGACTCACTATAGGGTACCACAGGGATAACTGGCTTGTGGCGGCCAAGCGTTCATAGCGACGTCGCTTTTTGATCCTTCGATGTCGGCTCTTCCTATCATTGTGATGGAATTCTCGGGTGCCAAGGATGGAAGATTTAGATGTC |
| rRNA.207 | NR_003287 | rRNA_28S_18S | GAGGACTAGGAACAGACGTAATACGACTCACTATAGGGAAGCGTTCATAGCGACGTCGCTTTTTGATCCTTCGATGTCGGCTCTTCCTATCATTGTGAAGCAGAATTCACCAAGCGTTGGATTGTTCATGGAATTCTCGGGTGCCAAGGATGGAAGATTTAGATGTC |
| rRNA.208 | NR_003287 | rRNA_28S_18S | GAGGACTAGGAACAGACGTAATACGACTCACTATAGGGCTTCGATGTCGGCTCTTCCTATCATTGTGAAGCAGAATTCACCAAGCGTTGGATTGTTCACCCACTAATAGGGAACGTGAGCTGGGTTTATGGAATTCTCGGGTGCCAAGGATGGAAGATTTAGATGTC |
| rRNA.209 | NR_003287 | rRNA_28S_18S | GAGGACTAGGAACAGACGTAATACGACTCACTATAGGGAGCAGAATTCACCAAGCGTTGGATTGTTCACCCACTAATAGGGAACGTGAGCTGGGTTTAGACCGTCGTGAGACAGGTTAGTTTTACCCTTGGAATTCTCGGGTGCCAAGGATGGAAGATTTAGATGTC |
| rRNA.210 | NR_003287 | rRNA_28S_18S | GAGGACTAGGAACAGACGTAATACGACTCACTATAGGGCCCACTAATAGGGAACGTGAGCTGGGTTTAGACCGTCGTGAGACAGGTTAGTTTTACCCTACTGATGATGTGTTGTTGCCATGGTAATCCTGGAATTCTCGGGTGCCAAGGATGGAAGATTTAGATGTC |
| rRNA.211 | NR_003287 | rRNA_28S_18S | GAGGACTAGGAACAGACGTAATACGACTCACTATAGGGGACCGTCGTGAGACAGGTTAGTTTTACCCTACTGATGATGTGTTGTTGCCATGGTAATCCTGCTCAGTACGAGAGGAACCGCAGGTTCAGTGGAATTCTCGGGTGCCAAGGATGGAAGATTTAGATGTC |
| rRNA.212 | NR_003287 | rRNA_28S_18S | GAGGACTAGGAACAGACGTAATACGACTCACTATAGGGGACTGATGATGTGTTGTTGCCATGGTAATCCTGCTCAGTACGAGAGGAACCGCAGGTTCAGACATTTGGTGTATGTGCTTGGCTGAGGAGCTGGAATTCTCGGGTGCCAAGGATGGAAGATTTAGATGTC |
| rRNA.213 | NR_003287 | rRNA_28S_18S | GAGGACTAGGAACAGACGTAATACGACTCACTATAGGGTGCTCAGTACGAGAGGAACCGCAGGTTCAGACATTTGGTGTATGTGCTTGGCTGAGGAGCCAATGGGGCGAAGCTACCATCTGTGGGATTGGAATTCTCGGGTGCCAAGGATGGAAGATTTAGATGTC |
| rRNA.214 | NR_003287 | rRNA_28S_18S | GAGGACTAGGAACAGACGTAATACGACTCACTATAGGGACATTTGGTGTATGTGCTTGGCTGAGGAGCCAATGGGGCGAAGCTACCATCTGTGGGATTATGACTGAACGCCTCTAAGTCAGAATCCCGTGGAATTCTCGGGTGCCAAGGATGGAAGATTTAGATGTC |
| rRNA.215 | NR_003287 | rRNA_28S_18S | GAGGACTAGGAACAGACGTAATACGACTCACTATAGGGCAATGGGGCGAAGCTACCATCTGTGGGATTATGACTGAACGCCTCTAAGTCAGAATCCCGCCCAGGCGGAACGATACGGCAGCGCCGCGGTGGAATTCTCGGGTGCCAAGGATGGAAGATTTAGATGTC |
| rRNA.216 | NR_003287 | rRNA_28S_18S | GAGGACTAGGAACAGACGTAATACGACTCACTATAGGGATGACTGAACGCCTCTAAGTCAGAATCCCGCCCAGGCGGAACGATACGGCAGCGCCGCGGAGCCTCGGTTGGCCTCGGATAGCCGGTCCCTGGAATTCTCGGGTGCCAAGGATGGAAGATTTAGATGTC |
| rRNA.217 | NR_003287 | rRNA_28S_18S | GAGGACTAGGAACAGACGTAATACGACTCACTATAGGGCCCAGGCGGAACGATACGGCAGCGCCGCGGAGCCTCGGTTGGCCTCGGATAGCCGGTCCCCGCCTGTCCCCGCCGGCGGGCCGCCCCCCTGGAATTCTCGGGTGCCAAGGATGGAAGATTTAGATGTC |
| rRNA.218 | NR_003287 | rRNA_28S_18S | GAGGACTAGGAACAGACGTAATACGACTCACTATAGGGAGCCTCGGTTGGCCTCGGATAGCCGGTCCCCGCCTGTCCCCGCCGGCGGGCCGCCCCCCCTCCACGCGCCCCGCGCGCGGGAGGGCTGGAATTCTCGGGTGCCAAGGATGGAAGATTTAGATGTC |
| rRNA.219 | NR_003287 | rRNA_28S_18S | GAGGACTAGGAACAGACGTAATACGACTCACTATAGGGCCGCCTGTCCCCGCCGGCGGGCCGCCCCCCCTCCACGCGCCCCGCGCGCGGGAGGGCGCGTGCCCCGCCGCGCGCCGGGACCGGGGTTGGAATTCTCGGGTGCCAAGGATGGAAGATTTAGATGTC |
| rRNA.220 | NR_003287 | rRNA_28S_18S | GAGGACTAGGAACAGACGTAATACGACTCACTATAGGGCCTCCACGCGCCCCGCGCGCGCGGGAGGGCGCGTGCCCCGCCGCGCGCCGGGACCGGGGTCCGGTGCGGAGTGCCCTTCGTCCTGGGAAATGGAATTCTCGGGTGCCAAGGATGGAAGATTTAGATGTC |
| rRNA.221 | NR_003287 | rRNA_28S_18S | GAGGACTAGGAACAGACGTAATACGACTCACTATAGGGGCGTGCCCCGCCGCGCGCGGGGACCGGGGTCCGGTGCGGAGTGCCCTTCGTCCTGGGAAACGGGGCGCGGCTGGAAAGGCGGCCGCCCCCTGGAATTCTCGGGTGCCAAGGATGGAAGATTTAGATGTC |
| rRNA.222 | NR_003287 | rRNA_28S_18S | GAGGACTAGGAACAGACGTAATACGACTCACTATAGGGCCGGTGCGGAGTGCCCTTCGTCCTGGGAAACGGGGCGCGGCTGGAAAGGCGGCCGCCCCCTCGCCCGTCACGCACCGCACGTTCGTGGGGTGGAATTCTCGGGTGCCAAGGATGGAAGATTTAGATGTC |
| rRNA.223 | NR_003287 | rRNA_28S_18S | GAGGACTAGGAACAGACGTAATACGACTCACTATAGGGCGGGGCGCGGCTGGAAAGGCGGCCGCCCCCTCGCCCGTCACGCACCGCACGTTCGTGGGGAACCTGGCGCTAAACCATTCGTAGACGACCTGGAATTCTCGGGTGCCAAGGATGGAAGATTTAGATGTC |

| ASO ID | Accession | Group ID | Custom Array sequence |
|---|---|---|---|
| rRNA.224 | NR_003287 | rRNA_28S_18S | GAGGACTAGGAACAGACGTAATACGACTCACTATAGGGTCGCCCGTCACGCACCGCACGTTCGTGGGGAACCTGGCGCTAAACCATTCGTAGACGACCTGCTTCTGGGTCGGGGTTTCGTACGTAGCATGGAATTCTCGGGTGCCAAGGATGGAAGATTTAGATGTC |
| rRNA.225 | NR_003287 | rRNA_28S_18S | GAGGACTAGGAACAGACGTAATACGACTCACTATAGGGAACCTGGCGCTAAACCATTCGTAGACGACCTGCTTCTGGGTCGGGGTTTCGTACGTAGCAGAGCAGCTCCCTCGCTGCGATCTATTGAAATGGAATTCTCGGGTGCCAAGGATGGAAGATTTAGATGTC |
| rRNA.226 | NR_003286 | rRNA_28S_18S | GAGGACTAGGAACAGACGTAATACGACTCACTATAGGGTACCTGGTTGATCCTGCCAGTAGCATATGCTTGTCTCAAAGATTAAGCCATGCATGTCTAAGTACGCACGGCCGGTACAGTGAAACTGCGTGGAATTCTCGGGTGCCAAGGATGGAAGATTTAGATGTC |
| rRNA.227 | NR_003286 | rRNA_28S_18S | GAGGACTAGGAACAGACGTAATACGACTCACTATAGGGTTGTCTCAAAGATTAAGCCATGCATGTCTAAGTACGCACGGCCGGTACAGTGAAACTGCGAATGGCTCATTAAATCAGTTATGGTTCCTTTGGAATTCTCGGGTGCCAAGGATGGAAGATTTAGATGTC |
| rRNA.228 | NR_003286 | rRNA_28S_18S | GAGGACTAGGAACAGACGTAATACGACTCACTATAGGGAGTACGCACGGCCGGTACAGTGAAACTGCGAATGGCTCATTAAATCAGTTATGGTTCCTTTGGTCGCTCGCTCCTCTCCTACTTGGATAATGGAATTCTCGGGTGCCAAGGATGGAAGATTTAGATGTC |
| rRNA.229 | NR_003286 | rRNA_28S_18S | GAGGACTAGGAACAGACGTAATACGACTCACTATAGGGAATGGCTCATTAAATCAGTTATGGTTCCTTTGGTCGCTCGCTCCTCTCCTACTTGGATAACTGTGGTAATTCTAGAGCTAATACATGCCGTGGAATTCTCGGGTGCCAAGGATGGAAGATTTAGATGTC |
| rRNA.230 | NR_003286 | rRNA_28S_18S | GAGGACTAGGAACAGACGTAATACGACTCACTATAGGGTGGTCGCTCGCTCCTCTCCTACTTGGATAACTGTGGTAATTCTAGAGCTAATACATGCCGACGGGCGCTGACCCCCTTCGCGGGGGGGATTGGAATTCTCGGGTGCCAAGGATGGAAGATTTAGATGTC |
| rRNA.231 | NR_003286 | rRNA_28S_18S | GAGGACTAGGAACAGACGTAATACGACTCACTATAGGGCTGTGGTAATTCTAGAGCTAATACATGCCGACGGGCGCTGACCCCCTTCGCGGGGGGGATGCGTGCATTTATCAGATCAAAACCAACCCGTGGAATTCTCGGGTGCCAAGGATGGAAGATTTAGATGTC |
| rRNA.232 | NR_003286 | rRNA_28S_18S | GAGGACTAGGAACAGACGTAATACGACTCACTATAGGGACGGGCGCTGACCCCCTTCGCGGGGGGGATGCGTGCATTTATCAGATCAAAACCAACCCGGTCAGCCCCTCTCCGGCCCCGGCCGGGGGGTGGAATTCTCGGGTGCCAAGGATGGAAGATTTAGATGTC |
| rRNA.233 | NR_003286 | rRNA_28S_18S | GAGGACTAGGAACAGACGTAATACGACTCACTATAGGGGCGTGCATTTATCAGATCAAAACCAACCCGGTCAGCCCCTCTCCGGCCCCGGCCGGGGGGCGGGCGCCGGCGGCTTTGGTGACTCTAGATTGGAATTCTCGGGTGCCAAGGATGGAAGATTTAGATGTC |
| rRNA.234 | NR_003286 | rRNA_28S_18S | GAGGACTAGGAACAGACGTAATACGACTCACTATAGGGTCAGCCCCTCTCCGGCCCCGGCCGGGGGGCGGGCGCCGGCGGCTTTGGTGACTCTAGATAACCTCGGGCCGATCGCACGCCCCCCGTGGTGGAATTCTCGGGTGCCAAGGATGGAAGATTTAGATGTC |
| rRNA.235 | NR_003286 | rRNA_28S_18S | GAGGACTAGGAACAGACGTAATACGACTCACTATAGGGCGGGCGCCGGCGGCTTTGGTGACTCTAGATAACCTCGGGCCGATCGCACGCCCCCCGTGGCGGCGACGACCCATTCGAACGTCTGCCCTATGGAATTCTCGGGTGCCAAGGATGGAAGATTTAGATGTC |
| rRNA.236 | NR_003286 | rRNA_28S_18S | GAGGACTAGGAACAGACGTAATACGACTCACTATAGGGAACCTCGGGCCGATCGCACGCCCCCCGTGGCGGCGACGACCCATTCGAACGTCTGCCCTATCAACTTTCGATGGTAGTCGCCGTGCCTACTGGAATTCTCGGGTGCCAAGGATGGAAGATTTAGATGTC |
| rRNA.237 | NR_003286 | rRNA_28S_18S | GAGGACTAGGAACAGACGTAATACGACTCACTATAGGGCGGCGACGACCCATTCGAACGTCTGCCCTATCAACTTTCGATGGTAGTCGCCGTGCCTACCATGGTGACCACGGGTGACGGGGAATCAGGTGGAATTCTCGGGTGCCAAGGATGGAAGATTTAGATGTC |
| rRNA.238 | NR_003286 | rRNA_28S_18S | GAGGACTAGGAACAGACGTAATACGACTCACTATAGGGTCAACTTTCGATGGTAGTCGCCGTGCCTACCATGGTGACCACGGGTGACGGGGAATCAGGGTTCGATTCCGGAGAGGGAGCCTGAGAAACTGGAATTCTCGGGTGCCAAGGATGGAAGATTTAGATGTC |
| rRNA.239 | NR_003286 | rRNA_28S_18S | GAGGACTAGGAACAGACGTAATACGACTCACTATAGGGCATGGTGACCACGGGTGACGGGGAATCAGGGTTCGATTCCGGAGAGGGAGCCTGAGAAACGGCTACCACATCCAAGGAAGGCAGCAGGCGTGGAATTCTCGGGTGCCAAGGATGGAAGATTTAGATGTC |
| rRNA.240 | NR_003286 | rRNA_28S_18S | GAGGACTAGGAACAGACGTAATACGACTCACTATAGGGGTTCGATTCCGGAGAGGGAGCCTGAGAAACGGCTACCACATCCAAGGAAGGCAGCAGGCGCGCAAATTACCCACTCCCGACCCGGGGAGGTGGAATTCTCGGGTGCCAAGGATGGAAGATTTAGATGTC |
| rRNA.241 | NR_003286 | rRNA_28S_18S | GAGGACTAGGAACAGACGTAATACGACTCACTATAGGGGGCTACCACATCCAAGGAAGGCAGCAGGCGCGCAAATTACCCACTCCCGACCCGGGGAGGTAGTGACGAAAAATAACAATACAGGACTCTTGGAATTCTCGGGTGCCAAGGATGGAAGATTTAGATGTC |
| rRNA.242 | NR_003286 | rRNA_28S_18S | GAGGACTAGGAACAGACGTAATACGACTCACTATAGGGCGCAAATTACCCACTCCCGACCCGGGGAGGTAGTGACGAAAAATAACAATACAGGACTCTTTCGAGGCCCTGTAATTGGAATGAGTCCACTGGAATTCTCGGGTGCCAAGGATGGAAGATTTAGATGTC |
| rRNA.243 | NR_003286 | rRNA_28S_18S | GAGGACTAGGAACAGACGTAATACGACTCACTATAGGGTAGTGACGAAAAATAACAATACAGGACTCTTTCGAGGCCCTGTAATTGGAATGAGTCCACTTTAAATCCTTTAACGAGGATCCATTGGAGTGGAATTCTCGGGTGCCAAGGATGGAAGATTTAGATGTC |
| rRNA.244 | NR_003286 | rRNA_28S_18S | GAGGACTAGGAACAGACGTAATACGACTCACTATAGGGTTCGAGGCCCTGTAATTGGAATGAGTCCACTTTAAATCCTTTAACGAGGATCCATTGGAGGGCAAGTCTGGTGCCAGCAGCCGCGGTAATTGGAATTCTCGGGTGCCAAGGATGGAAGATTTAGATGTC |
| rRNA.245 | NR_003286 | rRNA_28S_18S | GAGGACTAGGAACAGACGTAATACGACTCACTATAGGGTTTAAATCCTTTAACGAGGATCCATTGGAGGGCAAGTCTGGTGCCAGCAGCCGCGGTAATTCCAGCTCCAATAGCGTATATTAAAGTTGCTGGAATTCTCGGGTGCCAAGGATGGAAGATTTAGATGTC |
| rRNA.246 | NR_003286 | rRNA_28S_18S | GAGGACTAGGAACAGACGTAATACGACTCACTATAGGGGGCAAGTCTGGTGCCAGCAGCCGCGGTAATTCCAGCTCCAATAGCGTATATTAAAGTTGCTGCAGTTAAAAAGCTCGTAGTTGGATCTTGTGGAATTCTCGGGTGCCAAGGATGGAAGATTTAGATGTC |
| rRNA.247 | NR_003286 | rRNA_28S_18S | GAGGACTAGGAACAGACGTAATACGACTCACTATAGGGTCCAGCTCCAATAGCGTATATTAAAGTTGCTGCAGTTAAAAAGCTCGTAGTTGGATCTTGGGAGCGGGCGGGCGGTCCGCCGCGAGGCGATGGAATTCTCGGGTGCCAAGGATGGAAGATTTAGATGTC |
| rRNA.248 | NR_003286 | rRNA_28S_18S | GAGGACTAGGAACAGACGTAATACGACTCACTATAGGGTGCAGTTAAAAAGCTCGTAGTTGGATCTTGGGAGCGGGCGGGCGGTCCGCCGCGAGGCGAGCCACCGCCCGTCCCCGCCCCTTGCCTCTCTGGAATTCTCGGGTGCCAAGGATGGAAGATTTAGATGTC |

| ASO ID | Accession | Group ID | Custom Array sequence |
|---|---|---|---|
| rRNA.249 | NR_003286 | rRNA_28S_18S | GAGGACTAGGAACAGACGTAATACGACTCACTATAGGGAGGCGGTGTCCGCGCGAGGCGAGCCACCGCCGCCCCTCCGATGTCTCTGCCCTCTCGGCGCCCCCTCGAGTGCTTCTAGCTGAGTGTCCCGCGGGGCCCGAAGCGTTTACTTTGAAAAAATTAGAGTGTTCAAAGCAGGCCCGAGCCGCCTGGATACCGCAGCTAGGAATAATGGAATAGGATTAGAGATGTC |
| rRNA.250 | NR_003286 | rRNA_28S_18S | GAGGACTAGGAACAGACGTAATACGACTCACTATAGGGCCGCCCCCCTGCCTCTCGGCGCCCCCTCGATGTCTCTGCCCTCGAGTGCTTCTAGCTGAGTGTCCCGCGGGGCCCGAAGCGTTTACTTTGAAAAAATTAGAGTGTTCAAAGCAGGCCCGAGCCGCCTGGATACCGCAGCTAGGAATAATGGAATAGGATTAGAGATGTC |
| rRNA.251 | NR_003286 | rRNA_28S_18S | GAGGACTAGGAACAGACGTAATACGACTCACTATAGGGCCTCGATGTCTCTGCCCTCTCGGCGCCCCCTCGATGTCTCTGCCCTCGAGTGCTTCTAGCTGAGTGTCCCGCGGGGCCCGAAGCGTTTACTTACTTTGAAAAAATTAGAGTGTTCAAAGCAGGCCCGAGCCGCCTGGATACCGCAGCTAGGAATAATGGAATAGGATTAGAGATGTC |
| rRNA.252 | NR_003286 | rRNA_28S_18S | GAGGACTAGGAACAGACGTAATACGACTCACTATAGGGCTCTGCCCTCTCGGCGCCCCCTCGATGTCTCTGCCCTCTCGGCGCCCCCTCGAGTGCTTCTAGCTGAGTGTCCCGCGGGGCCCGAAGCGTTTACTTACTTTGAAAAAATTAGAGTGTTCAAAGCAGGCCCGAGCCGCCTGGATACCGCAGCTAGGAATAATGGAATAGGATTAGAGATGTC |
| rRNA.253 | NR_003286 | rRNA_28S_18S | GAGGACTAGGAACAGACGTAATACGACTCACTATAGGGAAATTAGAGTGTTCAAAGCAGGCCCGAGCCGCCTGGATACCGCAGCTAGGAATAATGGAATAGGACCGGTTCTATTTTGTTGGTTTTCGGAACTGAGGCCATGATTAAGAGGGACGGCCGGGGGCATTCTATTAGAGATGTC |
| rRNA.254 | NR_003286 | rRNA_28S_18S | GAGGACTAGGAACAGACGTAATACGACTCACTATAGGGGCTAGGAATAATGGAATAGGACCGGTTCTATTTTGTTGGTTTTCGGAACTGAGGCCATGATTAAGAGGGACGGCCGGGGGCATTCGTATTGCGCCGCTAGAGGTGAAATTCTTGGACCGGCGCAAGACGGACCAGAGCGAAAGCATTAGAGATGTC |
| rRNA.255 | NR_003286 | rRNA_28S_18S | GAGGACTAGGAACAGACGTAATACGACTCACTATAGGGCCATGATTAAGAGGGACGGCCGGGGGCATTCGTATTGCGCCGCTAGAGGTGAAATTCTTGGACCGGCGCAAGACGGACCAGAGCGAAAGCATTTCATTCAATTCGGCGCAAGACGATCAAGACATGGAATTAGAGATGTC |
| rRNA.256 | NR_003286 | rRNA_28S_18S | GAGGACTAGGAACAGACGTAATACGACTCACTATAGGGGCCGGGGGCATTCGTATTGCGCCGCTAGAGGTGAAATTCTTGGACCGGCGCAAGACGGACCAGAGCGAAAGCATTTCATTCAATTCAAGACGATCAAGACATGGAATTAGAGATGTC |
| rRNA.257 | NR_003286 | rRNA_28S_18S | GAGGACTAGGAACAGACGTAATACGACTCACTATAGGGGTGAAATTCTTGGACCGGCGCAAGACGGACCAGAGCGAAAGCATTTCATTCAATTCAAGACGATCAAGACATGGAATTCAGAATACCGTCGTAGTTCCGACCATAAAACGATGCCGACCGGCGATGCGGCGGCGTTATTCCCATGTC |
| rRNA.258 | NR_003286 | rRNA_28S_18S | GAGGACTAGGAACAGACGTAATACGACTCACTATAGGGCAGAGCGAAAGCATTTCATTCAATTCAAGACGATCAAGACATGGAATTCAGAATACCGTCGTAGTTCCGACCATAAAACGATGCCGACCGGCGATGCGGCGGCGTTATTCCCATGCCGCAGCTTCCGGGGAATTAGAGATGTC |
| rRNA.259 | NR_003286 | rRNA_28S_18S | GAGGACTAGGAACAGACGTAATACGACTCACTATAGGGTCAATTCAAGACGATCAAGACATGGAATTCAGAATACCGTCGTAGTTCCGACCATAAAACGATGCCGACCGGCGATGCGGCGGCGTTATTCCCATGCCGCAGCTTCCGGGGAATTAGAGATGTC |
| rRNA.260 | NR_003286 | rRNA_28S_18S | GAGGACTAGGAACAGACGTAATACGACTCACTATAGGGGTTAATCAAGAACGAAAGTCGGAGGTTCGAAGACGATCAGATACCGTCGTAGTTCCGACCATAAAACGATGCCGACCGGCGATGCGGCGGCGTTATTCCCATGACCCGCCGGGCAGCTTCCGGGGAATTAGAGATGTC |
| rRNA.261 | NR_003286 | rRNA_28S_18S | GAGGACTAGGAACAGACGTAATACGACTCACTATAGGGTTCGAAGACGATCAGATACCGTCGTAGTTCCGACCATAAAACGATGCCGACCGGCGATGCGGCGGCGTTATTCCCATGACCCGCCGGGCAGCTTCCGTGGAATTAGAGATGTC |
| rRNA.262 | NR_003286 | rRNA_28S_18S | GAGGACTAGGAACAGACGTAATACGACTCACTATAGGGTAAACGATGCCGGCGGCGTTATTCCCATGACCCGCCGGGCAGCTTCCGTGGAATTCCGTGGAAACCAAAGTCTTTGGGTTCCGGGGGGAGTATGGTTGCAAAGCTGAAACTTAAAGGAATTGACGGAAGATTAGAGATGTC |
| rRNA.263 | NR_003286 | rRNA_28S_18S | GAGGACTAGGAACAGACGTAATACGACTCACTATAGGGTATTCCCATGACCCGCCGGGCAGCTTCCGGGGGGAGTATGGTTGCAAAGCTGAAACTTAAAGGAATTGACGGAAGGGCACCACCAGGACTGGAAGATTAGAGATGTC |
| rRNA.264 | NR_003286 | rRNA_28S_18S | GAGGACTAGGAACAGACGTAATACGACTCACTATAGGGTCTTTGGGTTCCGGGGGGAGTATGGTTGCAAAGCTGAAACTTAAAGGAATTGACGGAAGGGCACCACCAGGAGTGGAGCCTGCGGCTTAATTTGACTCAACACGGGAAATGGAAGATTAGAGATGTC |
| rRNA.265 | NR_003286 | rRNA_28S_18S | GAGGACTAGGAACAGACGTAATACGACTCACTATAGGGTGGTTGCAAAGCTGAAACTTAAAGGAATTGACGGAAGGGCACCACCAGGAGTGGAGCCTGCGGCTTAATTTGACTCAACACGGGAAACCTCACCCGGCCCGGACACGGACAGGATTAGAGATGTC |
| rRNA.266 | NR_003286 | rRNA_28S_18S | GAGGACTAGGAACAGACGTAATACGACTCACTATAGGGGGAATTGACGGAAGGGCACCACCAGGAGTGGAGCCTGCGGCTTAATTTGACTCAACACGGGAAACCTCACCCGGCCCGGACACGGACAGGATTGACAGATTGATAGCTCTTTCTCGATTCCGTGAGATTAGAGATGTC |
| rRNA.267 | NR_003286 | rRNA_28S_18S | GAGGACTAGGAACAGACGTAATACGACTCACTATAGGGCCTGCGGCTTAATTTGACTCAACACGGGAAACCTCACCCGGCCCGGACACGGACAGGATTGACAGATTGATAGCTCTTTCTCGATTCCGTGGGTGGTGGTGCATGGCCGTTCTTAGTTGGTGGAGCGATTTGTCTGGTTAATTCCGATAACTGGAGATTAGAGATGTC |
| rRNA.268 | NR_003286 | rRNA_28S_18S | GAGGACTAGGAACAGACGTAATACGACTCACTATAGGGACGGGAAACCTCACCCGGCCCGGACACGGACAGGATTGACAGATTGATAGCTCTTTCTCGATTCCGTGGGTGGTGGTGCATGGCCGTTCTTAGTTGGTGGAGCGATTTGTCTGGTTAATTCCGATAACGAACGAGACTCTGGTTAATTAGAGATGTC |
| rRNA.269 | NR_003286 | rRNA_28S_18S | GAGGACTAGGAACAGACGTAATACGACTCACTATAGGGACAGGATTGACAGATTGATAGCTCTTTCTCGATTCCGTGGGTGGTGGTGCATGGCCGTTCTTAGTTGGTGGAGCGATTTGTCTGGTTAATTCCGATAACGAACGAGACTCTGGCATGCTAACTAGTTACGCGACCCCCGAGCGGTCGGCGTCCCCCAACTTCTTAGAGATGTC |
| rRNA.270 | NR_003286 | rRNA_28S_18S | GAGGACTAGGAACAGACGTAATACGACTCACTATAGGGTGGGTGGTGGTGCATGGCCGTTCTTAGTTGGTGGAGCGATTTGTCTGGTTAATTCCGATAACGAACGAGACTCTGGCATGCTAACTAGTTACGCGACCCCCGAGCGGTCGGCGTCCCCCAACTTCTTAGAGGGACAAGTGGCGTTCAGCCACTGGAATTAGAGATGTC |
| rRNA.271 | NR_003286 | rRNA_28S_18S | GAGGACTAGGAACAGACGTAATACGACTCACTATAGGGCGATTTGTCTGGTTAATTCCGATAACGAACGAGACTCTGGCATGCTAACTAGTTACGCGACCCCCGAGCGGTCGGCGTCCCCCAACTTCTTAGAGGGACAAGTGGCGTTCAGCCACCCGAGATTGAGCAATAACAGGTCTGTGATGCCCTTAGATGTC |
| rRNA.272 | NR_003286 | rRNA_28S_18S | GAGGACTAGGAACAGACGTAATACGACTCACTATAGGGAACGAACGAGACTCTGGCATGCTAACTAGTTACGCGACCCCCGAGCGGTCGGCGTCCCCCAACTTCTTAGAGGGACAAGTGGCGTTCAGCCACCCGAGATTGAGCAATAACAGGTCTGTGATGCCCTTAGATGTGCTGGGCTGCCAAGGTGCCAAGGATGGAAGAGATTTAGAGATGTC |
| rRNA.273 | NR_003286 | rRNA_28S_18S | GAGGACTAGGAACAGACGTAATACGACTCACTATAGGGCCCCCGAGCGGTCGGCGTCCCCCAACTTCTTAGAGGGACAAGTGGCGTTCAGCCACCCGAGATTGAGCAATAACAGGTCTGTGATGCCCTTAGATGTGCTGGGCTGCCAAGGTGCCAAGGATGGAAGAGATTTAGAGATGTC |

| ASO ID | Accession | Group ID | Custom Array sequence |
|---|---|---|---|
| rRNA.274 | NR_003286 | rRNA_28S_18S | GAGGACTAGGAACAGACGTAATACGACTCACTATAGGGTTCTTAGAGGGACAAGTGGCGTTCAGCCACCCGAGATTGAGCAATAACAGGTCTGTGATGCCCTTAGATGTCCGGGGCTGCACGCGCGCTTGGAATTCTCGGGTGCCAAGGATGGAAGATTTAGATGTC |
| rRNA.275 | NR_003286 | rRNA_28S_18S | GAGGACTAGGAACAGACGTAATACGACTCACTATAGGGCCGAGATTGAGCAATAACAGGTCTGTGATGCCCTTAGATGTCCGGGGCTGCACGCGCGCTACACTGACTGGCTCAGCGTGTGCCTACCCTTGGAATTCTCGGGTGCCAAGGATGGAAGATTTAGATGTC |
| rRNA.276 | NR_003286 | rRNA_28S_18S | GAGGACTAGGAACAGACGTAATACGACTCACTATAGGGCCCTTAGATGTCCGGGGCTGCACGCGCGCTACACTGACTGGCTCAGCGTGTGCCTACCCTACGCCGGCAGGCGCGGGTAACCCGTTGAACTGGAATTCTCGGGTGCCAAGGATGGAAGATTTAGATGTC |
| rRNA.277 | NR_003286 | rRNA_28S_18S | GAGGACTAGGAACAGACGTAATACGACTCACTATAGGGACACTGACTGGCTCAGCGTGTGCCTACCCTACGCCGGCAGGCGCGGGTAACCCGTTGAACCCCATTCGTGATGGGGATCGGGGATTGCAATGGAATTCTCGGGTGCCAAGGATGGAAGATTTAGATGTC |
| rRNA.278 | NR_003286 | rRNA_28S_18S | GAGGACTAGGAACAGACGTAATACGACTCACTATAGGGACGCCGGCAGGCGCGGGTAACCCGTTGAACCCCATTCGTGATGGGGATCGGGGATTGCAATTATTCCCCATGAACGAGGAATTCCCAGTATGGAATTCTCGGGTGCCAAGGATGGAAGATTTAGATGTC |
| rRNA.279 | NR_003286 | rRNA_28S_18S | GAGGACTAGGAACAGACGTAATACGACTCACTATAGGGCCCATTCGTGATGGGGATCGGGGATTGCAATTATTCCCCATGAACGAGGAATTCCCAGTAAGTGCGGGTCATAAGCTTGCGTTGATTAAGTGGAATTCTCGGGTGCCAAGGATGGAAGATTTAGATGTC |
| rRNA.280 | NR_003286 | rRNA_28S_18S | GAGGACTAGGAACAGACGTAATACGACTCACTATAGGGTTATTCCCCATGAACGAGGAATTCCCAGTAAGTGCGGGTCATAAGCTTGCGTTGATTAAGTCCCTGCCCTTTGTACACACCGCCCGTCGCTGGAATTCTCGGGTGCCAAGGATGGAAGATTTAGATGTC |
| rRNA.281 | NR_003286 | rRNA_28S_18S | GAGGACTAGGAACAGACGTAATACGACTCACTATAGGGAGTGCGGGTCATAAGCTTGCGTTGATTAAGTCCCTGCCCTTTGTACACACCGCCCGTCGCTACTACCGATTGGATGGTTTAGTGAGGCCCTGGAATTCTCGGGTGCCAAGGATGGAAGATTTAGATGTC |
| rRNA.282 | NR_003286 | rRNA_28S_18S | GAGGACTAGGAACAGACGTAATACGACTCACTATAGGGTCCCTGCCCTTTGTACACACCGCCCGTCGCTACTACCGATTGGATGGTTTAGTGAGGCCCTCGGATCGGCCCCGCCGGGGTCGGCCCACGTGGAATTCTCGGGTGCCAAGGATGGAAGATTTAGATGTC |
| rRNA.283 | NR_003286 | rRNA_28S_18S | GAGGACTAGGAACAGACGTAATACGACTCACTATAGGGTACTACCGATTGGATGGTTTAGTGAGGCCCTCGGATCGGCCCCGCCGGGGTCGGCCCACGGCCCTGGCGGAGCGCTGAGAAGACGGTCGATGGAATTCTCGGGTGCCAAGGATGGAAGATTTAGATGTC |
| rRNA.284 | NR_003286 | rRNA_28S_18S | GAGGACTAGGAACAGACGTAATACGACTCACTATAGGGTCGGATCGGCCCCGCCGGGGTCGGCCCACAGGCCCTGGCGGAGCGCTGAGAAGACGGTCGAACTTGACTATCTAGAGGAAGTAAAAGTCGTTGGAATTCTCGGGTGCCAAGGATGGAAGATTTAGATGTC |
| rRNA.285 | NR_003286 | rRNA_28S_18S | GAGGACTAGGAACAGACGTAATACGACTCACTATAGGGCCCTGGCGGAGCGCTGAGAAGACGGTCGAACTTGACTATCTAGAGGAAGTAAAAGTCGTAACAAGGTTTCCGTAGGTGAACCTGCGGAATGGAATTCTCGGGTGCCAAGGATGGAAGATTTAGATGTC |
| rRNA.286 | NR_003287 | rRNA_28S_18S | GAGGACTAGGAACAGACGTAATACGACTCACTATAGGGCGCGACCTCAGATCAGACGTGGCGACCCGCTGAATTTAAGCATATTAGTCAGCGGAGGAGAAGAAACTAACCAGGATTCCCTCAGTAACGTGGAATTCTCGGGTGCCAAGGATGGAAGATTTAGATGTC |
| rRNA.287 | NR_003287 | rRNA_28S_18S | GAGGACTAGGAACAGACGTAATACGACTCACTATAGGGTGAATTTAAGCATATTAGTCAGCGGAGGAGAAGAAACTAACCAGGATTCCCTCAGTAACGGCGAGTGAACAGGGAAGAGCCCAGCGCCGATGGAATTCTCGGGTGCCAAGGATGGAAGATTTAGATGTC |
| rRNA.288 | NR_003287 | rRNA_28S_18S | GAGGACTAGGAACAGACGTAATACGACTCACTATAGGGAAGAAACTAACCAGGATTCCCTCAGTAACGGCGAGTGAACAGGGAAGAGCCCAGCGCCGAATCCCCGCCCCGCGGCGGGGCGCGGGACATTGGAATTCTCGGGTGCCAAGGATGGAAGATTTAGATGTC |
| rRNA.289 | NR_003287 | rRNA_28S_18S | GAGGACTAGGAACAGACGTAATACGACTCACTATAGGGGCGAGTGAACAGGGAAGAGCCCAGCGCCGAATCCCCGCCCCGCGGCGGGGCGCGGGACATGTGGCGTACGGAAGACCCGCTCCCCGGCGCTGGAATTCTCGGGTGCCAAGGATGGAAGATTTAGATGTC |
| rRNA.290 | NR_003287 | rRNA_28S_18S | GAGGACTAGGAACAGACGTAATACGACTCACTATAGGGATCCCCGCCCCGCGGCGGGGCGCGGGACATGTGGCGTACGGAAGACCCGCTCCCCGGCGCCGCTCGTGGGGGGCCCAAGTCCTTCTGATCTGGAATTCTCGGGTGCCAAGGATGGAAGATTTAGATGTC |
| rRNA.291 | NR_003287 | rRNA_28S_18S | GAGGACTAGGAACAGACGTAATACGACTCACTATAGGGGTGGCGTACGGAAGACCCGCTCCCCGGCGCCGCTCGTGGGGGGCCCAAGTCCTTCTGATCGAGGCCCAGCCCGTGGACGGTGTGAGGCCGTGGAATTCTCGGGTGCCAAGGATGGAAGATTTAGATGTC |
| rRNA.292 | NR_003287 | rRNA_28S_18S | GAGGACTAGGAACAGACGTAATACGACTCACTATAGGGCGCTCGTGGGGGGCCCAAGTCCTTCTGATCGAGGCCCAGCCCGTGGACGGTGTGAGGCCGGTAGCGGCCCCCGGCGCGCCGGGCCCGGGTTGGAATTCTCGGGTGCCAAGGATGGAAGATTTAGATGTC |
| rRNA.293 | NR_003287 | rRNA_28S_18S | GAGGACTAGGAACAGACGTAATACGACTCACTATAGGGGAGGCCCAGCCCGTGGACGGTGTGAGGCCGGTAGCGGCCCCCGGCGCGCCGGGCCCGGGTCTTCCCGGAGTCGGGTTGCTTGGGAATGCATGGAATTCTCGGGTGCCAAGGATGGAAGATTTAGATGTC |
| rRNA.294 | NR_003287 | rRNA_28S_18S | GAGGACTAGGAACAGACGTAATACGACTCACTATAGGGTAGCGGCCCCCGGCGCGCCGGGCCCGGGTCTTCCCGGAGTCGGGTTGCTTGGGAATGCAGCCCAAAGCGGGTGGTAAACTCCATCTAAGTGGAATTCTCGGGTGCCAAGGATGGAAGATTTAGATGTC |
| rRNA.295 | NR_003287 | rRNA_28S_18S | GAGGACTAGGAACAGACGTAATACGACTCACTATAGGGCTTCCCGGAGTCGGGTTGCTTGGGAATGCAGCCCAAAGCGGGTGGTAAACTCCATCTAAGGCTAAATACCGGCACGAGACCGATAGTCAATGGAATTCTCGGGTGCCAAGGATGGAAGATTTAGATGTC |
| rRNA.296 | NR_003287 | rRNA_28S_18S | GAGGACTAGGAACAGACGTAATACGACTCACTATAGGGCCCAAAGCGGGTGGTAAACTCCATCTAAGGCTAAATACCGGCACGAGACCGATAGTCAACAAGTACCGTAAGGGAAAGTTGAAAAGAACTGGAATTCTCGGGTGCCAAGGATGGAAGATTTAGATGTC |
| rRNA.297 | NR_003287 | rRNA_28S_18S | GAGGACTAGGAACAGACGTAATACGACTCACTATAGGGGCTAAATACCGGCACGAGACCGATAGTCAACAAGTACCGTAAGGGAAAGTTGAAAAGAACTTTGAAGAGAGAGTTCAAGAGGGCGTGAAATGGAATTCTCGGGTGCCAAGGATGGAAGATTTAGATGTC |
| rRNA.298 | NR_003287 | rRNA_28S_18S | GAGGACTAGGAACAGACGTAATACGACTCACTATAGGGCAAGTACCGTAAGGGAAAGTTGAAAAGAACTTTGAAGAGAGAGTTCAAGAGGGCGTGAAACCGTTAAGAGGTAAACGGGTGGGGTCCGCGTGGAATTCTCGGGTGCCAAGGATGGAAGATTTAGATGTC |

114

| ASO ID | Accession | Group ID | Custom Array sequence |
|---|---|---|---|
| rRNA.299 | NR_003287 | rRNA_28S_18S | GAGGACTAGGAACAGACGTAATACGACTCACTATAGGGTTTGAAGAGAGAGTTCAAGAGGGCGTGAAACCGTTAAGAGGTAAACGGGTGGGGTCCGCGCAGTCCGCCCGGAGGATTCAACCCGGCGGCTGGAATTCTCGGGTGCCAAGGATGGAAGATTTAGATGTC |
| rRNA.300 | NR_003287 | rRNA_28S_18S | GAGGACTAGGAACAGACGTAATACGACTCACTATAGGGCCGTTAAGAGGTAAACGGGTGGGGTCCGCGCAGTCCGCCCGGAGGATTCAACCCGGCGGCGGGTCCGGCCGTGTCGGCGGCCCGGCGGATTGGAATTCTCGGGTGCCAAGGATGGAAGATTTAGATGTC |
| rRNA.301 | NR_003287 | rRNA_28S_18S | GAGGACTAGGAACAGACGTAATACGACTCACTATAGGGCAGTCCGCCCGGAGGATTCAACCCGGCGGCGGGTCCGGCCGTGTCGGCGGCCCGGCGGATCTTTCCCGCCCCCCGTTCCTCCCGACCCCTTGGAATTCTCGGGTGCCAAGGATGGAAGATTTAGATGTC |
| rRNA.302 | NR_003287 | rRNA_28S_18S | GAGGACTAGGAACAGACGTAATACGACTCACTATAGGGGGGTCCGGCCGTGTCGGCGGCCCGGCGGATCTTTCCCGCCCCCCGTTCCTCCCGACCCCTCCACCCGCCCTCCCTTCCCCCGCCGCCCCCTTGGAATTCTCGGGTGCCAAGGATGGAAGATTTAGATGTC |
| rRNA.303 | NR_003287 | rRNA_28S_18S | GAGGACTAGGAACAGACGTAATACGACTCACTATAGGGCTTTCCCGCCCCCCGTTCCTCCCGACCCCTCCACCCGCCCTCCCTTCCCCCGCCGCCCCTCCTCCTCCTCCCCCGGAGGGGGCGGGCTCCGTGGAATTCTCGGGTGCCAAGGATGGAAGATTTAGATGTC |
| rRNA.304 | NR_003287 | rRNA_28S_18S | GAGGACTAGGAACAGACGTAATACGACTCACTATAGGGCCACCCGCCCTCCCTTCCCCCGCCGCCCCTCCTCCTCCTCCCCCGGAGGGGGCGGGCTCCGGCGGGTGCGGGGGTGGGCGGGCGGGGCCGGTGGAATTCTCGGGTGCCAAGGATGGAAGATTTAGATGTC |
| rRNA.305 | NR_003287 | rRNA_28S_18S | GAGGACTAGGAACAGACGTAATACGACTCACTATAGGGCCTCCTCCTCCCCCGGAGGGGGCGGGCTCCGGCGGGTGCGGGGGTGGGCGGGCGGGGCCGGGGGTGGGGTCGGCGGGGGACCGTCCCCCGATGGAATTCTCGGGTGCCAAGGATGGAAGATTTAGATGTC |
| rRNA.306 | NR_003287 | rRNA_28S_18S | GAGGACTAGGAACAGACGTAATACGACTCACTATAGGGGCGGGTGCGGGGGTGGGCGGGCGGGGCCGGGGGTGGGGTCGGCGGGGGACCGTCCCCCGACCGGCGACCGGCCGCCGCCGGGGCGCATTTCTGGAATTCTCGGGTGCCAAGGATGGAAGATTTAGATGTC |
| rRNA.307 | NR_003287 | rRNA_28S_18S | GAGGACTAGGAACAGACGTAATACGACTCACTATAGGGGGGTGGGGTCGGCGGGGGACCGTCCCCCGACCGGCGACCGGCCGCCGCCGGGGCGCATTTCCACCGCGGCGGTGCGCCGCGACCGGCTCCGTGGAATTCTCGGGTGCCAAGGATGGAAGATTTAGATGTC |
| rRNA.308 | NR_003287 | rRNA_28S_18S | GAGGACTAGGAACAGACGTAATACGACTCACTATAGGGCCGGCGACCGGCCGCCGCCGGGGCGCATTTCCACCGCGGCGGTGCGCCGCGACCGGCTCCGGGACGGCTGGGAAGGCCCGGCGGGGAAGGTTGGAATTCTCGGGTGCCAAGGATGGAAGATTTAGATGTC |
| rRNA.309 | NR_003287 | rRNA_28S_18S | GAGGACTAGGAACAGACGTAATACGACTCACTATAGGGCACCGCGGCGGTGCGCCGCGACCGGCTCCGGGACGGCTGGGAAGGCCCGGCGGGGAAGGTGGCTCGGGGGGCCCCGTCCGTCCGTCCGTCTGGAATTCTCGGGTGCCAAGGATGGAAGATTTAGATGTC |
| rRNA.310 | NR_003287 | rRNA_28S_18S | GAGGACTAGGAACAGACGTAATACGACTCACTATAGGGGACGGCTGGGAAGGCCCGGCGGGGAAGGTGGCTCGGGGGGCCCCGTCCGTCCGTCCGTCCGTCCTCCTCCTCCCCCGTCTCCGCCCCCTGGAATTCTCGGGTGCCAAGGATGGAAGATTTAGATGTC |
| rRNA.311 | NR_003287 | rRNA_28S_18S | GAGGACTAGGAACAGACGTAATACGACTCACTATAGGGGGCTCGGGGGGCCCCGTCCGTCCGTCCGTCCGTCCTCCTCCTCCCCCGTCTCCGCCCCCCGGCCCCGCGTCCTCCCTCGGGAGGGCGCGCTGGAATTCTCGGGTGCCAAGGATGGAAGATTTAGATGTC |
| rRNA.312 | NR_003287 | rRNA_28S_18S | GAGGACTAGGAACAGACGTAATACGACTCACTATAGGGCGTCCTCCTCCTCCCCCGTCTCCGCCCCCCGGCCCCGCGTCCTCCCTCGGGAGGGCGCGCGGGTCGGGGCGGCGGCGGCGGCGGCGGCGGTGGTGGAATTCTCGGGTGCCAAGGATGGAAGATTTAGATGTC |
| rRNA.313 | NR_003287 | rRNA_28S_18S | GAGGACTAGGAACAGACGTAATACGACTCACTATAGGGGCCCCGCGTCCTCCCTCGGGAGGGCGCGCGGGTCGGGGCGGCGGCGGCGGCGGCGGCGGTGGCGGCGGCGGCGGCGGGACCGAAACTGGAATTCTCGGGTGCCAAGGATGGAAGATTTAGATGTC |
| rRNA.314 | NR_003287 | rRNA_28S_18S | GAGGACTAGGAACAGACGTAATACGACTCACTATAGGGGGGTCGGGGCGGCGGCGGCGGCGGCGGCGGTGGCGGCGGCGGCGGCGGCGGCGGGACCGAAACCCCCCCGAGTGTTACAGCCCCCCGGCAGTGGAATTCTCGGGTGCCAAGGATGGAAGATTTAGATGTC |
| rRNA.315 | NR_003287 | rRNA_28S_18S | GAGGACTAGGAACAGACGTAATACGACTCACTATAGGGCGGCGGCGGCGGCGGCGGCGGCGGGACCGAAACCCCCCCGAGTGTTACAGCCCCCCGGCAGCAGCACTCGCCGAATCCCGGGGCCGAGGGATGGAATTCTCGGGTGCCAAGGATGGAAGATTTAGATGTC |
| rRNA.316 | NR_003287 | rRNA_28S_18S | GAGGACTAGGAACAGACGTAATACGACTCACTATAGGGCCCCCCGAGTGTTACAGCCCCCCGGCAGCAGCACTCGCCGAATCCCGGGGCCGAGGGAGCGAGACCCGTCGCCGCGCTCTCCCCCCTCTGGAATTCTCGGGTGCCAAGGATGGAAGATTTAGATGTC |
| rRNA.317 | NR_003287 | rRNA_28S_18S | GAGGACTAGGAACAGACGTAATACGACTCACTATAGGGCAGCACTCGCCGAATCCCGGGGCCGAGGGAGCGAGACCCGTCGCCGCGCTCTCCCCCCTCCCGGCGCCCACCCCCGCGGGGAATCCCCGTGGAATTCTCGGGTGCCAAGGATGGAAGATTTAGATGTC |
| rRNA.318 | NR_003287 | rRNA_28S_18S | GAGGACTAGGAACAGACGTAATACGACTCACTATAGGGGCGAGACCCGTCGCCGCGCTCTCCCCCCTCCCGGCGCCCACCCCCGCGGGGAATCCCCGCGAGGGGGGTCTCCCCGCGGGGGCGCGCTGGAATTCTCGGGTGCCAAGGATGGAAGATTTAGATGTC |
| rRNA.319 | NR_003287 | rRNA_28S_18S | GAGGACTAGGAACAGACGTAATACGACTCACTATAGGGCCGGCGCCCACCCCCGCGGGGAATCCCCGCGAGGGGGGTCTCCCCGCGGGGGCGCGCCGGCGTCTCCTCGTGGGGGGGCCGGGCCACCTGGAATTCTCGGGTGCCAAGGATGGAAGATTTAGATGTC |
| rRNA.320 | NR_003287 | rRNA_28S_18S | GAGGACTAGGAACAGACGTAATACGACTCACTATAGGGCGAGGGGGGTCTCCCCGCGGGGGCGCGCCGGCGTCTCCTCGTGGGGGGGCCGGGCCACCCCTCCCACGGCGCGACCGCTCTCCCACCCCTGGAATTCTCGGGTGCCAAGGATGGAAGATTTAGATGTC |
| rRNA.321 | NR_003287 | rRNA_28S_18S | GAGGACTAGGAACAGACGTAATACGACTCACTATAGGGGGCGTCTCCTCGTGGGGGGGCCGGGCCACCCCTCCCACGGCGCGACCGCTCTCCCACCCCTCCTCCCCCGCGCCCCCGCCCCGGCGACGGGTGGAATTCTCGGGTGCCAAGGATGGAAGATTTAGATGTC |
| rRNA.322 | NR_003287 | rRNA_28S_18S | GAGGACTAGGAACAGACGTAATACGACTCACTATAGGGCCTCCCACGGCGCGACCGCTCTCCCACCCCTCCTCCCCCGCGCCCCCGCCCCGGCGACGGGGGGGGTGCCGCGCGCGGGTCGGGGGGCGGGTGGAATTCTCGGGTGCCAAGGATGGAAGATTTAGATGTC |
| rRNA.323 | NR_003287 | rRNA_28S_18S | GAGGACTAGGAACAGACGTAATACGACTCACTATAGGGTCCTCCCCGCGCCCCCGCCCCGGCGACGGGGGGGGTGCCGCGCGCGGGTCGGGGGGCGGGGCGGACTGTCCCCAGTGCGCCCCGGGCGGGTGGAATTCTCGGGTGCCAAGGATGGAAGATTTAGATGTC |

| ASO ID | Accession | Group ID | Custom Array sequence |
|---|---|---|---|
| rRNA.324 | NR_003287 | rRNA_28S_18S | GAGGACTAGGAACAGACGTAATACGACTCACTATAGGGGGGGGGTGCCGCGCGCGGGTCGGGGGGCGGGGCGGACTGTCCCCAGTGCGCCCCGGGCGGGTCGCGCCGTCGGGCCCGGGGGAGGTTCTCTTGGAATTCTCGGGTGCCAAGGATGGAAGATTTAGATGTC |
| rRNA.325 | NR_003287 | rRNA_28S_18S | GAGGACTAGGAACAGACGTAATACGACTCACTATAGGGGCGGACTGTCCCCAGTGCGCCCCGGGCGGGTCGCGCCGTCGGGCCCGGGGGAGGTTCTCTCGGGGCCCACGCGCGCGTCCCCCGAAGAGGGTGGAATTCTCGGGTGCCAAGGATGGAAGATTTAGATGTC |
| rRNA.326 | NR_003287 | rRNA_28S_18S | GAGGACTAGGAACAGACGTAATACGACTCACTATAGGGTCGCGCCGTCGGGCCCGGGGGAGGTTCTCTCGGGGCCCACGCGCGCGTCCCCCGAAGAGGGGGACGGCGGAGCGAGCGCACGGGGTCGGCGTGGAATTCTCGGGTGCCAAGGATGGAAGATTTAGATGTC |
| rRNA.327 | NR_003287 | rRNA_28S_18S | GAGGACTAGGAACAGACGTAATACGACTCACTATAGGGCGGGGCCCACGCGCGCGTCCCCCGAAGAGGGGGACGGCGGAGCGAGCGCACGGGGTCGGCGGCGACGTCGGCTACCCACCCGACCCGTCTTTGGAATTCTCGGGTGCCAAGGATGGAAGATTTAGATGTC |
| rRNA.328 | NR_003287 | rRNA_28S_18S | GAGGACTAGGAACAGACGTAATACGACTCACTATAGGGGGGACGGCGGAGCGAGCGCACGGGGTCGGCGGCGACGTCGGCTACCCACCCGACCCGTCTTGAAACACGGACCAAGGAGTCTAACACGTGCTGGAATTCTCGGGTGCCAAGGATGGAAGATTTAGATGTC |
| rRNA.329 | NR_003287 | rRNA_28S_18S | GAGGACTAGGAACAGACGTAATACGACTCACTATAGGGGCGACGTCGGCTACCCACCCGACCCGTCTTGAAACACGGACCAAGGAGTCTAACACGTGCGCGAGTCGGGGGCTCGCACGAAAGCCGCCGTGGAATTCTCGGGTGCCAAGGATGGAAGATTTAGATGTC |
| rRNA.330 | NR_003287 | rRNA_28S_18S | GAGGACTAGGAACAGACGTAATACGACTCACTATAGGGGAAACACGGACCAAGGAGTCTAACACGTGCGCGAGTCGGGGGCTCGCACGAAAGCCGCCGTGGCGCAATGAAGGTGAAGGCCGGCGCGCTTGGAATTCTCGGGTGCCAAGGATGGAAGATTTAGATGTC |
| rRNA.331 | NR_003287 | rRNA_28S_18S | GAGGACTAGGAACAGACGTAATACGACTCACTATAGGGGCGAGTCGGGGGCTCGCACGAAAGCCGCCGTGGCGCAATGAAGGTGAAGGCCGGCGCGCTCGCCGGCCGAGGTGGGATCCCGAGGCCTCTTGGAATTCTCGGGTGCCAAGGATGGAAGATTTAGATGTC |
| rRNA.332 | NR_003287 | rRNA_28S_18S | GAGGACTAGGAACAGACGTAATACGACTCACTATAGGGTGGCGCAATGAAGGTGAAGGCCGGCGCGTCGCCGGCCGAGGTGGGATCCCGAGGCCTCTCCAGTCCGCCGAGGGCGCACCACCGGCCCGTGGAATTCTCGGGTGCCAAGGATGGAAGATTTAGATGTC |
| rRNA.333 | NR_003287 | rRNA_28S_18S | GAGGACTAGGAACAGACGTAATACGACTCACTATAGGGCGCCGGCCGAGGTGGGATCCCGAGGCCTCTCCAGTCCGCCGAGGGCGCACCACCGGCCCGTCTCGCCCGCCGCGCCGGGGAGGTGGAGCATGGAATTCTCGGGTGCCAAGGATGGAAGATTTAGATGTC |
| rRNA.334 | NR_003287 | rRNA_28S_18S | GAGGACTAGGAACAGACGTAATACGACTCACTATAGGGCCAGTCCGCCGAGGGCGCACCACCGGCCCGTCTCGCCCGCCGCGCCGGGGAGGTGGAGCACGAGCGCACGTGTTAGGACCCGAAAGATGGTGGAATTCTCGGGTGCCAAGGATGGAAGATTTAGATGTC |
| rRNA.335 | NR_003287 | rRNA_28S_18S | GAGGACTAGGAACAGACGTAATACGACTCACTATAGGGTCTCGCCCGCCGCGCCGGGGAGGTGGAGCACGAGCGCACGTGTTAGGACCCGAAAGATGGTGAACTATGCCTGGGCAGGGCGAAGCCAGATGGAATTCTCGGGTGCCAAGGATGGAAGATTTAGATGTC |
| rRNA.336 | NR_003287 | rRNA_28S_18S | GAGGACTAGGAACAGACGTAATACGACTCACTATAGGGCGAGCGCACGTGTTAGGACCCGAAAGATGGTGAACTATGCCTGGGCAGGGCGAAGCCAGAGGAAACTCTGGTGGAGGTCCGTAGCGGTCCTGGAATTCTCGGGTGCCAAGGATGGAAGATTTAGATGTC |
| rRNA.337 | NR_003287 | rRNA_28S_18S | GAGGACTAGGAACAGACGTAATACGACTCACTATAGGGTGAACTATGCCTGGGCAGGGCGAAGCCAGAGGAAACTCTGGTGGAGGTCCGTAGCGGTCCTGACGTGCAAATCGGTCGTCCGACCTGGGTTGGAATTCTCGGGTGCCAAGGATGGAAGATTTAGATGTC |
| rRNA.338 | NR_003287 | rRNA_28S_18S | GAGGACTAGGAACAGACGTAATACGACTCACTATAGGGGGAAACTCTGGTGGAGGTCCGTAGCGGTCCTGACGTGCAAATCGGTCGTCCGACCTGGGTATAGGGGCGAAAGACTAATCGAACCATCTATGGAATTCTCGGGTGCCAAGGATGGAAGATTTAGATGTC |
| rRNA.339 | NR_003287 | rRNA_28S_18S | GAGGACTAGGAACAGACGTAATACGACTCACTATAGGGTGACGTGCAAATCGGTCGTCCGACCTGGGTATAGGGGCGAAAGACTAATCGAACCATCTAGTAGCTGGTTCCCTCCGAAGTTTCCCTCAGTGGAATTCTCGGGTGCCAAGGATGGAAGATTTAGATGTC |
| rRNA.340 | NR_003287 | rRNA_28S_18S | GAGGACTAGGAACAGACGTAATACGACTCACTATAGGGATAGGGGCGAAAGACTAATCGAACCATCTAGTAGCTGGTTCCCTCCGAAGTTTCCCTCAGGATAGCTGGCGCTCTCGCAGACCCGACGCATGGAATTCTCGGGTGCCAAGGATGGAAGATTTAGATGTC |
| rRNA.341 | NR_003287 | rRNA_28S_18S | GAGGACTAGGAACAGACGTAATACGACTCACTATAGGGGTAGCTGGTTCCCTCCGAAGTTTCCCTCAGGATAGCTGGCGCTCTCGCAGACCCGACGCACCCCCGCCACGCAGTTTTATCCGGTAAAGCTGGAATTCTCGGGTGCCAAGGATGGAAGATTTAGATGTC |
| rRNA.342 | NR_003287 | rRNA_28S_18S | GAGGACTAGGAACAGACGTAATACGACTCACTATAGGGGATAGCTGGCGCTCTCGCAGACCCGACGCACCCCCGCCACGCAGTTTTATCCGGTAAAGCGAATGATTAGAGGTCTTGGGGCCGAAACGATGGAATTCTCGGGTGCCAAGGATGGAAGATTTAGATGTC |
| rRNA.343 | NR_003287 | rRNA_28S_18S | GAGGACTAGGAACAGACGTAATACGACTCACTATAGGGCCCCCGCCACGCAGTTTTATCCGGTAAAGCGAATGATTAGAGGTCTTGGGGCCGAAACGATCTCAACCTATTCTCAAACTTTAAATGGGTTGGAATTCTCGGGTGCCAAGGATGGAAGATTTAGATGTC |
| rRNA.344 | NR_003287 | rRNA_28S_18S | GAGGACTAGGAACAGACGTAATACGACTCACTATAGGGGAATGATTAGAGGTCTTGGGGCCGAAACGATCTCAACCTATTCTCAAACTTTAAATGGGTAAGAAGCCCGGCTCGCTGGCGTGGAGCCGGTGGAATTCTCGGGTGCCAAGGATGGAAGATTTAGATGTC |
| rRNA.345 | NR_003287 | rRNA_28S_18S | GAGGACTAGGAACAGACGTAATACGACTCACTATAGGGTCTCAACCTATTCTCAAACTTTAAATGGGTAAGAAGCCCGGCTCGCTGGCGTGGAGCCGGGCGTGGAATGCGAGTGCCTAGTGGGCCACTTGGAATTCTCGGGTGCCAAGGATGGAAGATTTAGATGTC |
| rRNA.346 | NR_003287 | rRNA_28S_18S | GAGGACTAGGAACAGACGTAATACGACTCACTATAGGGAAGAAGCCCGGCTCGCTGGCGTGGAGCCGGGCGTGGAATGCGAGTGCCTAGTGGGCCACTTTTGGTAAGCAGAACTGGCGCTGCGGGATGTGGAATTCTCGGGTGCCAAGGATGGAAGATTTAGATGTC |
| rRNA.347 | NR_003287 | rRNA_28S_18S | GAGGACTAGGAACAGACGTAATACGACTCACTATAGGGGCGTGGAATGCGAGTGCCTAGTGGGCCACTTTTGGTAAGCAGAACTGGCGCTGCGGGATGAACCGAACGCCGGGTTAAGGCGCCCGATGCTGGAATTCTCGGGTGCCAAGGATGGAAGATTTAGATGTC |
| rRNA.348 | NR_003287 | rRNA_28S_18S | GAGGACTAGGAACAGACGTAATACGACTCACTATAGGGTTTGGTAAGCAGAACTGGCGCTGCGGGATGAACCGAACGCCGGGTTAAGGCGCCCGATGCCGACGCTCATCAGACCCCAGAAAAGGTGTTTGGAATTCTCGGGTGCCAAGGATGGAAGATTTAGATGTC |

| ASO ID | Accession | Group ID | Custom Array sequence |
|---|---|---|---|
| rRNA.349 | NR_003287 | rRNA_28S_18S | GAGGACTAGGAACAGACGTAATACGACTCACTATAGGGAACCGAACGCCGGGTTAAGGCGCCCGATGCCGACGCTCATCAGACCCCAGAAAAGGTGTTGGTTGATATAGACAGCAGGACGGTGGCCATTGGAATTCTCGGGTGCCAAGGATGGAAGATTTAGATGTC |
| rRNA.350 | NR_003287 | rRNA_28S_18S | GAGGACTAGGAACAGACGTAATACGACTCACTATAGGGCGACGCTCATCAGACCCCAGAAAAGGTGTTGGTTGATATAGACAGCAGGACGGTGGCCATGGAAGTCGGAATCCGCTAAGGAGTGTGTAATGGAATTCTCGGGTGCCAAGGATGGAAGATTTAGATGTC |
| rRNA.351 | NR_003287 | rRNA_28S_18S | GAGGACTAGGAACAGACGTAATACGACTCACTATAGGGGGTTGATATAGACAGCAGGACGGTGGCCATGGAAGTCGGAATCCGCTAAGGAGTGTGTAACAACTCACCTGCCGAATCAACTAGCCCTGATGGAATTCTCGGGTGCCAAGGATGGAAGATTTAGATGTC |
| rRNA.352 | NR_003287 | rRNA_28S_18S | GAGGACTAGGAACAGACGTAATACGACTCACTATAGGGGGGAAGTCGGAATCCGCTAAGGAGTGTGTAACAACTCACCTGCCGAATCAACTAGCCCTGAAAATGGATGGCGCTGGAGCGTCGGGCCCATTGGAATTCTCGGGTGCCAAGGATGGAAGATTTAGATGTC |
| rRNA.353 | NR_003287 | rRNA_28S_18S | GAGGACTAGGAACAGACGTAATACGACTCACTATAGGGCAACTCACCTGCCGAATCAACTAGCCCTGAAAATGGATGGCGCTGGAGCGTCGGGCCCATACCCGGCCGTCGCCGGCAGTCGAGAGTGGATGGAATTCTCGGGTGCCAAGGATGGAAGATTTAGATGTC |
| rRNA.354 | NR_003287 | rRNA_28S_18S | GAGGACTAGGAACAGACGTAATACGACTCACTATAGGGAAATGGATGGCGCTGGAGCGTCGGGCCCATACCCGGCCGTCGCCGGCAGTCGAGAGTGGACGGGAGCGGCGGGGGCGGCGCGCGCGCGTGGAATTCTCGGGTGCCAAGGATGGAAGATTTAGATGTC |
| rRNA.355 | NR_003287 | rRNA_28S_18S | GAGGACTAGGAACAGACGTAATACGACTCACTATAGGGACCCGGCCGTCGCCGGCAGTCGAGAGTGGACGGGAGCGGCGGGGGCGGCGCGCGCGCGCGCGTGTGGTGTGCGTCGGAGGGCGGCGGCTGGAATTCTCGGGTGCCAAGGATGGAAGATTTAGATGTC |
| rRNA.356 | NR_003287 | rRNA_28S_18S | GAGGACTAGGAACAGACGTAATACGACTCACTATAGGGCGGGAGCGGCGGGGGCGGCGCGCGCGCGCGCGCGTGTGGTGTGCGTCGGAGGGCGGCGGCGGCGGCGGCGGCGGGGGTGTGGGGTCCTTCTGGAATTCTCGGGTGCCAAGGATGGAAGATTTAGATGTC |
| rRNA.357 | NR_003287 | rRNA_28S_18S | GAGGACTAGGAACAGACGTAATACGACTCACTATAGGGCGCGTGTGGTGTGCGTCGGAGGGCGGCGGCGGCGGCGGCGGCGGGGGTGTGGGGTCCTTCCCCCGCCCCCCCCCCCACGCCTCCTCCCCTTGGAATTCTCGGGTGCCAAGGATGGAAGATTTAGATGTC |
| rRNA.358 | NR_003287 | rRNA_28S_18S | GAGGACTAGGAACAGACGTAATACGACTCACTATAGGGGGCGGCGGCGGCGGGGGTGTGGGGTCCTTCCCCCGCCCCCCCCCCCACGCCTCCTCCCCTCCTCCCGCCCACGCCCCCGCTCCCCGCCCCCTGGAATTCTCGGGTGCCAAGGATGGAAGATTTAGATGTC |
| rRNA.359 | NR_003287 | rRNA_28S_18S | GAGGACTAGGAACAGACGTAATACGACTCACTATAGGGCCCCGCCCCCCCCCCCACGCCTCCTCCCCTCCTCCCGCCCACGCCCCCGCTCCCCGCCCCCGGAGCCCCGCGGACGCTACGCCGCGACGAGTGGAATTCTCGGGTGCCAAGGATGGAAGATTTAGATGTC |
| rRNA.360 | NR_003287 | rRNA_28S_18S | GAGGACTAGGAACAGACGTAATACGACTCACTATAGGGCCTCCCGCCCACGCCCCGCTCCCCGCCCCCGGAGCCCCGCGGACGCTACGCCGCGACGAGTAGGAGGGCCGCTGCGGTGAGCCTTGAAGCTGGAATTCTCGGGTGCCAAGGATGGAAGATTTAGATGTC |
| rRNA.361 | NR_003287 | rRNA_28S_18S | GAGGACTAGGAACAGACGTAATACGACTCACTATAGGGGGAGCCCCGCGGACGCTACGCCGCGACGAGTAGGAGGGCCGCTGCGGTGAGCCTTGAAGCCTAGGGCGCGGGCCCGGGTGGAGCCGCCGCTGGAATTCTCGGGTGCCAAGGATGGAAGATTTAGATGTC |
| rRNA.362 | NR_003287 | rRNA_28S_18S | GAGGACTAGGAACAGACGTAATACGACTCACTATAGGGTAGGAGGGCCGCTGCGGTGAGCCTTGAAGCCTAGGGCGCGGGCCCGGGTGGAGCCGCCGCAGGTGCAGATCTTGGTGGTAGTAGCAAATATGGAATTCTCGGGTGCCAAGGATGGAAGATTTAGATGTC |
| rRNA.363 | NR_003287 | rRNA_28S_18S | GAGGACTAGGAACAGACGTAATACGACTCACTATAGGGCTAGGGCGCGGGCCCGGGTGGAGCCGCCGCAGGTGCAGATCTTGGTGGTAGTAGCAAATATTCAAACGAGAACTTTGAAGGCCGAAGTGGTGGAATTCTCGGGTGCCAAGGATGGAAGATTTAGATGTC |
| rRNA.364 | NR_003287 | rRNA_28S_18S | GAGGACTAGGAACAGACGTAATACGACTCACTATAGGGGAGGTGCAGATCTTGGTGGTAGTAGCAAATATTCAAACGAGAACTTTGAAGGCCGAAGTGGAGAAGGGTTCCATGTGAACAGCAGTTGAACTGGAATTCTCGGGTGCCAAGGATGGAAGATTTAGATGTC |
| rRNA.365 | NR_003287 | rRNA_28S_18S | GAGGACTAGGAACAGACGTAATACGACTCACTATAGGGTTCAAACGAGAACTTTGAAGGCCGAAGTGGAGAAGGGTTCCATGTGAACAGCAGTTGAACATGGGTCAGTCGGTCCTGAGAGATGGGCGATGGAATTCTCGGGTGCCAAGGATGGAAGATTTAGATGTC |
| rRNA.366 | NR_003287 | rRNA_28S_18S | GAGGACTAGGAACAGACGTAATACGACTCACTATAGGGAGAAGGGTTCCATGTGAACAGCAGTTGAACATGGGTCAGTCGGTCCTGAGAGATGGGCGAGCGCCGTTCCGAAGGGACGGGCGATGGCCTTGGAATTCTCGGGTGCCAAGGATGGAAGATTTAGATGTC |
| rRNA.367 | NR_003287 | rRNA_28S_18S | GAGGACTAGGAACAGACGTAATACGACTCACTATAGGGATGGGTCAGTCGGTCCTGAGAGATGGGCGAGCGCCGTTCCGAAGGGACGGGCGATGGCCTCCGTTGCCCTCGGCCGATCGAAAGGGAGTCTGGAATTCTCGGGTGCCAAGGATGGAAGATTTAGATGTC |
| rRNA.368 | NR_003287 | rRNA_28S_18S | GAGGACTAGGAACAGACGTAATACGACTCACTATAGGGCGCCGTTCCGAAGGGACGGGCGATGGCCTCCGTTGCCCTCGGCCGATCGAAAGGGAGTCGGGTTCAGATCCCCGAATCCGGAGTGGCGGTGGAATTCTCGGGTGCCAAGGATGGAAGATTTAGATGTC |
| rRNA.369 | NR_003287 | rRNA_28S_18S | GAGGACTAGGAACAGACGTAATACGACTCACTATAGGGCCGTTGCCCTCGGCCGATCGAAAGGGAGTCGGGTTCAGATCCCCGAATCCGGAGTGGCGGAGATGGGCGCCGCGAGGCGTCCAGTGCGGTTGGAATTCTCGGGTGCCAAGGATGGAAGATTTAGATGTC |
| rRNA.370 | NR_003287 | rRNA_28S_18S | GAGGACTAGGAACAGACGTAATACGACTCACTATAGGGGGGTTCAGATCCCCGAATCCGGAGTGGCGGAGATGGGCGCCGCGAGGCGTCCAGTGCGGTAACGCGACCGATCCCGGAGAAGCCGGCGGGTGGAATTCTCGGGTGCCAAGGATGGAAGATTTAGATGTC |
| rRNA.371 | NR_003287 | rRNA_28S_18S | GAGGACTAGGAACAGACGTAATACGACTCACTATAGGGAGATGGGCGCCGCGAGGCGTCCAGTGCGGTAACGCGACCGATCCCGGAGAAGCCGGCGGGAGCCCCGGGGAGAGTTCTCTTTTCTTTGTGTGGAATTCTCGGGTGCCAAGGATGGAAGATTTAGATGTC |
| rRNA.372 | NR_003287 | rRNA_28S_18S | GAGGACTAGGAACAGACGTAATACGACTCACTATAGGGAACGCGACCGATCCCGGAGAAGCCGGCGGGAGCCCCGGGGAGAGTTCTCTTTTCTTTGTGAAGGGCAGGGCGCCCTGGAATGGGTTCGCCTGGAATTCTCGGGTGCCAAGGATGGAAGATTTAGATGTC |
| rRNA.373 | NR_003287 | rRNA_28S_18S | GAGGACTAGGAACAGACGTAATACGACTCACTATAGGGAGCCCCGGGGAGAGTTCTCTTTTCTTTGTGAAGGGCAGGGCGCCCTGGAATGGGTTCGCCCCGAGAGAGGGGCCCGTGCCTTGGAAAGCGTGGAATTCTCGGGTGCCAAGGATGGAAGATTTAGATGTC |

| ASO ID | Accession | Group ID | Custom Array sequence |
|---|---|---|---|
| rRNA.374 | NR_003287 | rRNA_28S_18S | GAGGACTAGGAACAGACGTAATACGACTCACTATAGGGAAGGGCAGGGCGCCCTGGAATGGGTTCGCCCCGAGAGAGGGGCCCGTGCCTTGGAAAGCGTCGCGGTTCCGGCGGCGTCCGGTGAGCTCTTGGAATTCTCGGGTGCCAAGGATGGAAGATTTAGATGTC |
| rRNA.375 | NR_003287 | rRNA_28S_18S | GAGGACTAGGAACAGACGTAATACGACTCACTATAGGGCCGAGAGAGGGGCCCGTGCCTTGGAAAGCGTCGCGGTTCCGGCGGCGTCCGGTGAGCTCTCGCTGGCCCTTGAAAATCCGGGGGAGAGGGTGGAATTCTCGGGTGCCAAGGATGGAAGATTTAGATGTC |
| rRNA.376 | NR_003287 | rRNA_28S_18S | GAGGACTAGGAACAGACGTAATACGACTCACTATAGGGTCGCGGTTCCGGCGGCGTCCGGTGAGCTCTCGCTGGCCCTTGAAAATCCGGGGGAGAGGGTGTAAATCTCGCGCCGGGCCGTACCCATATTGGAATTCTCGGGTGCCAAGGATGGAAGATTTAGATGTC |
| rRNA.377 | NR_003287 | rRNA_28S_18S | GAGGACTAGGAACAGACGTAATACGACTCACTATAGGGCGCTGGCCCTTGAAAATCCGGGGGAGAGGGTGTAAATCTCGCGCCGGGCCGTACCCATATCCGCAGCAGGTCTCCAAGGTGAACAGCCTCTGGAATTCTCGGGTGCCAAGGATGGAAGATTTAGATGTC |
| rRNA.378 | NR_003287 | rRNA_28S_18S | GAGGACTAGGAACAGACGTAATACGACTCACTATAGGGTGTAAATCTCGCGCCGGGCCGTACCCATATCCGCAGCAGGTCTCCAAGGTGAACAGCCTCTGGCATGTTGGAACAATGTAGGTAAGGGAATGGAATTCTCGGGTGCCAAGGATGGAAGATTTAGATGTC |
| rRNA.379 | NR_003287 | rRNA_28S_18S | GAGGACTAGGAACAGACGTAATACGACTCACTATAGGGCCGCAGCAGGTCTCCAAGGTGAACAGCCTCTGGCATGTTGGAACAATGTAGGTAAGGGAAGTCGGCAAGCCGGATCCGTAACTTCGGGATTGGAATTCTCGGGTGCCAAGGATGGAAGATTTAGATGTC |
| rRNA.380 | NR_003287 | rRNA_28S_18S | GAGGACTAGGAACAGACGTAATACGACTCACTATAGGGTGGCATGTTGGAACAATGTAGGTAAGGGAAGTCGGCAAGCCGGATCCGTAACTTCGGGATAAGGATTGGCTCTAAAGGGCTGGGTCGGTCGTGGAATTCTCGGGTGCCAAGGATGGAAGATTTAGATGTC |
| rRNA.381 | NR_003287 | rRNA_28S_18S | GAGGACTAGGAACAGACGTAATACGACTCACTATAGGGGTCGGCAAGCCGGATCCGTAACTTCGGGATAAGGATTGGCTCTAAAGGGCTGGGTCGGTCGGGCTGGGGCGCGAAGCGGGGCTGGGCGCGCTGGAATTCTCGGGTGCCAAGGATGGAAGATTTAGATGTC |
| rRNA.382 | NR_003287 | rRNA_28S_18S | GAGGACTAGGAACAGACGTAATACGACTCACTATAGGGAAGGATTGGCTCTAAGGGCTGGGTCGGTCGGGCTGGGGCGCGAAGCGGGGCTGGGCGCGCGACCGCGGCTGGACGAGGCGCCGCCGCCCCCTGGAATTCTCGGGTGCCAAGGATGGAAGATTTAGATGTC |
| rRNA.383 | NR_003287 | rRNA_28S_18S | GAGGACTAGGAACAGACGTAATACGACTCACTATAGGGGGCTGGGGCGCGAAGCGGGGCTGGGCGCGCGACCGCGGCTGGACGAGGCGCCGCCGCCCCCCCCACGCCCGGGGCACCCCCCTCGCGGCCCTGGAATTCTCGGGTGCCAAGGATGGAAGATTTAGATGTC |
| rRNA.384 | NR_003287 | rRNA_28S_18S | GAGGACTAGGAACAGACGTAATACGACTCACTATAGGGCCGGCGGCTGGACGAGGCGCCGCCGCCCCCCCCACGCCCGGGGCACCCCCCTCGCGGCCCTCCCCCGCCCCCACCCCGCGCGCGCCGCTCGTGGAATTCTCGGGTGCCAAGGATGGAAGATTTAGATGTC |
| rRNA.385 | NR_003287 | rRNA_28S_18S | GAGGACTAGGAACAGACGTAATACGACTCACTATAGGGCCCCACGCCCGGGGCACCCCCCTCGCGGCCCTCCCCCGCCCCACCCCGCGCGCGCCGCTCGCTCCCTCCCCGCCCCGCGCCCTCTCTCTCTTGGAATTCTCGGGTGCCAAGGATGGAAGATTTAGATGTC |
| rRNA.386 | NR_003287 | rRNA_28S_18S | GAGGACTAGGAACAGACGTAATACGACTCACTATAGGGTCCCCCGCCCCACCCCGCGCGCGCCGCTCGCTCCCTCCCCGCCCCGCGCCCTCTCTCTCTCTCTCTCCCCCGCTCCCCGTCCTCCCCCCTTGGAATTCTCGGGTGCCAAGGATGGAAGATTTAGATGTC |
| rRNA.387 | NR_003287 | rRNA_28S_18S | GAGGACTAGGAACAGACGTAATACGACTCACTATAGGGCTCCCTCCCCGCCCCGCGCCCTCTCTCTCTCTCTCTCCCCCGCTCCCCGTCCTCCCCCCTCCCCGGGGGAGCGCCGCGTGGGGGCGGCGGTGGAATTCTCGGGTGCCAAGGATGGAAGATTTAGATGTC |
| rRNA.388 | NR_003287 | rRNA_28S_18S | GAGGACTAGGAACAGACGTAATACGACTCACTATAGGGCTCTCTCCCCGCTCCCCGTCCTCCCCCCTCCCCGGGGGAGCGCCGCGTGGGGGCGGCGGCGGGGGGAGAAGGGTCGGGGCGGCAGGGGCTGGAATTCTCGGGTGCCAAGGATGGAAGATTTAGATGTC |
| rRNA.389 | NR_003287 | rRNA_28S_18S | GAGGACTAGGAACAGACGTAATACGACTCACTATAGGGCCCCGGGGGAGCGCCGCGTGGGGGCGGCGGCGGGGGGAGAAGGGTCGGGGCGGCAGGGGCCGGCGGCGGCCCGCCGCGGGGCCCCGGCGGTGGAATTCTCGGGTGCCAAGGATGGAAGATTTAGATGTC |
| rRNA.390 | NR_003287 | rRNA_28S_18S | GAGGACTAGGAACAGACGTAATACGACTCACTATAGGGCGGGGGGAGAAGGGTCGGGGCGGCAGGGGCCGGCGGCGGCCCGCCGCGGGGCCCCGGCGGCGGGGGCACGGTCCCCCGCGAGGGGGGGCCCTGGAATTCTCGGGTGCCAAGGATGGAAGATTTAGATGTC |
| rRNA.391 | NR_003287 | rRNA_28S_18S | GAGGACTAGGAACAGACGTAATACGACTCACTATAGGGCGGCGGCGGCCCGCCGCGGGGCCCCGGCGGCGGGGGCACGGTCCCCCGCGAGGGGGGGCCCGGGCACCCGGGGGGCCGGCGGCGGCGGCGATGGAATTCTCGGGTGCCAAGGATGGAAGATTTAGATGTC |
| rRNA.392 | NR_003287 | rRNA_28S_18S | GAGGACTAGGAACAGACGTAATACGACTCACTATAGGGCGGGGGCACGGTCCCCCGCGAGGGGGGGCCCGGGCACCCGGGGGGCCGGCGGCGGCGGCGACTCTGGACGCGCGAGCCGGGCCCTTCCCGTGGTGGAATTCTCGGGTGCCAAGGATGGAAGATTTAGATGTC |
| rRNA.393 | NR_003287 | rRNA_28S_18S | GAGGACTAGGAACAGACGTAATACGACTCACTATAGGGGGGGCACCCGGGGGGCCGGCGGCGGCGGCGACTCTGGACGCGCGAGCCGGGCCCTTCCCGTGGATCGCCCCAGCTGCGGCGGGCGTCGCGGCCTGGAATTCTCGGGTGCCAAGGATGGAAGATTTAGATGTC |
| rRNA.394 | NR_003287 | rRNA_28S_18S | GAGGACTAGGAACAGACGTAATACGACTCACTATAGGGCTCTGGACGCGCGAGCCGGGCCCTTCCCGTGGATCGCCCCAGCTGCGGCGGGCGTCGCGGCCGCCCCCCGGGGAGCCCGGCGGGCGCCGGCGCTGGAATTCTCGGGTGCCAAGGATGGAAGATTTAGATGTC |
| rRNA.395 | NR_003287 | rRNA_28S_18S | GAGGACTAGGAACAGACGTAATACGACTCACTATAGGGGATCGCCCCAGCTGCGGCGGGCGTCGCGGCCGCCCCCCGGGGAGCCCGGCGGGCGCCGGCGCGCCCCCCCCCCCACCCCACGTCTCGTCGCGCGTGGAATTCTCGGGTGCCAAGGATGGAAGATTTAGATGTC |
| rRNA.396 | NR_003287 | rRNA_28S_18S | GAGGACTAGGAACAGACGTAATACGACTCACTATAGGGGCCCCCGGGGAGCCCGGCGGGCGCCGGCGCGCCCCCCCCCCCACCCCACGTCTCGTCGCGCGCGCGTCCGCTGGGGGCGGGGAGCGGTCGTGGAATTCTCGGGTGCCAAGGATGGAAGATTTAGATGTC |
| rRNA.397 | NR_003287 | rRNA_28S_18S | GAGGACTAGGAACAGACGTAATACGACTCACTATAGGGGCCCCCCCCCCCACCCCACGTCTCGTCGCGCGCGCGTCCGCTGGGGGCGGGGAGCGGTCGGGCGGCGGCGGTCGGCGGGCGGCGGGGGCGGTGGAATTCTCGGGTGCCAAGGATGGAAGATTTAGATGTC |
| rRNA.398 | NR_003287 | rRNA_28S_18S | GAGGACTAGGAACAGACGTAATACGACTCACTATAGGGCGCGCGTCCGCTGGGGGCGGGGAGCGGTCGGGCGGCGGCGGTCGGCGGGCGGCGGGGGCGGGCGGTTCGTCCCCCCGCCCTACCCCCCCGTGGAATTCTCGGGTGCCAAGGATGGAAGATTTAGATGTC |

118

| ASO ID | Accession | Group ID | Custom Array sequence |
|---|---|---|---|
| rRNA.399 | NR_003287 | rRNA_28S_18S | GAGGACTAGGAACAGACGTAATACGACTCACTATAGGGGGCGGCGGCGGTCGGCGGGCGGCGGGGCGGGGCGGTTCGTCCCCCCGCCCTACCCCCCCGGCCCCGTCCGCCCCCCGTTCCCCCCTCCTCTGGAATTCTCGGGTGCCAAGGATGGAAGATTTAGATGTC |
| rRNA.400 | NR_003287 | rRNA_28S_18S | GAGGACTAGGAACAGACGTAATACGACTCACTATAGGGGGCGGTTCGTCCCCCCGCCCTACCCCCCCGGCCCCGTCCGCCCCCCGTTCCCCCCTCCTCCTCGGCGCGCGGCGGCGGCGGCGGGGCGGCGGTGGAATTCTCGGGTGCCAAGGATGGAAGATTTAGATGTC |
| rRNA.401 | NR_003287 | rRNA_28S_18S | GAGGACTAGGAACAGACGTAATACGACTCACTATAGGGGGCCCCGTCCGCCCCCCGTTCCCCCCTCCTCCTCGGCGCGCGGCGGCGGCGGCGGGGCGGCGGAGGGGCCGCGGGCCGGTCCCCCCCGCCGGTGGAATTCTCGGGTGCCAAGGATGGAAGATTTAGATGTC |
| rRNA.402 | NR_003287 | rRNA_28S_18S | GAGGACTAGGAACAGACGTAATACGACTCACTATAGGGGCTCGGCGCGCGGCGGCGGCGGCGGGGCGGCGGAGGGGCCGCGGGCCGGTCCCCCCCGCCGGGTCCGCCCCCGGGGCCGCGGTTCCGCGCGGTGGAATTCTCGGGTGCCAAGGATGGAAGATTTAGATGTC |
| rRNA.403 | NR_003287 | rRNA_28S_18S | GAGGACTAGGAACAGACGTAATACGACTCACTATAGGGGGAGGGGCCGCGGGCCGGTCCCCCCCGCCGGGTCCGCCCCCGGGGCCGCGGTTCCGCGCGGCGCCTCGCCTCGGCCGGCGCCTAGCAGCCGTGGAATTCTCGGGTGCCAAGGATGGAAGATTTAGATGTC |
| rRNA.404 | NR_003287 | rRNA_28S_18S | GAGGACTAGGAACAGACGTAATACGACTCACTATAGGGCGGTCCGCCCCCGGGGCCGCGGTTCCGCGCGGCGCCTCGCCTCGGCCGGCGCCTAGCAGCCGACTTAGAACTGGTGCGGACCCAGGGGAATCCTGGAATTCTCGGGTGCCAAGGATGGAAGATTTAGATGTC |
| rRNA.405 | NR_003287 | rRNA_28S_18S | GAGGACTAGGAACAGACGTAATACGACTCACTATAGGGCGCCTCGCCTCGGCCGGCGCCTAGCAGCCGACTTAGAACTGGTGCGGACCCAGGGGAATCCGACTGTTTAATTAAAACAAAGCATCGCGAATGGAATTCTCGGGTGCCAAGGATGGAAGATTTAGATGTC |
| rRNA.406 | NR_003287 | rRNA_28S_18S | GAGGACTAGGAACAGACGTAATACGACTCACTATAGGGACTTAGAACTGGTGCGGACCCAGGGGAATCCGACTGTTTAATTAAAACAAAGCATCGCGAAGGCCCGCGGCGGGGTGTTGACGCGATGTGATTGGAATTCTCGGGTGCCAAGGATGGAAGATTTAGATGTC |
| rRNA.407 | NR_003287 | rRNA_28S_18S | GAGGACTAGGAACAGACGTAATACGACTCACTATAGGGGGACTGTTTAATTAAAACAAAGCATCGCGAAGGCCCGCGGCGGGGTGTTGACGCGATGTGATTTCTGCCCAGTGCTCTGAATGTCAAAGTGATGGAATTCTCGGGTGCCAAGGATGGAAGATTTAGATGTC |
| rRNA.408 | NR_003287 | rRNA_28S_18S | GAGGACTAGGAACAGACGTAATACGACTCACTATAGGGGGCCCGCGGCGGGGTGTTGACGCGATGTGATTTCTGCCCAGTGCTCTGAATGTCAAAGTGAAGAAATTCAATGAAGCGCGGGTAAACGGCGTGGAATTCTCGGGTGCCAAGGATGGAAGATTTAGATGTC |
| rRNA.409 | NR_003287 | rRNA_28S_18S | GAGGACTAGGAACAGACGTAATACGACTCACTATAGGGTTCTGCCCAGTGCTCTGAATGTCAAAGTGAAGAAATTCAATGAAGCGCGGGTAAACGGCGGGAGTAACTATGACTCTCTTAAGGTAGCCATGGAATTCTCGGGTGCCAAGGATGGAAGATTTAGATGTC |
| rRNA.410 | NR_003287 | rRNA_28S_18S | GAGGACTAGGAACAGACGTAATACGACTCACTATAGGGAGAAATTCAATGAAGCGCGGGTAAACGGCGGGAGTAACTATGACTCTCTTAAGGTAGCCAAATGCCTCGTCATCTAATTAGTGACGCGCATGGAATTCTCGGGTGCCAAGGATGGAAGATTTAGATGTC |
| rRNA.411 | NR_003287 | rRNA_28S_18S | GAGGACTAGGAACAGACGTAATACGACTCACTATAGGGGGAGTAACTATGACTCTCTTAAGGTAGCCAAATGCCTCGTCATCTAATTAGTGACGCGCATGAATGGATGAACGAGATTCCCACTGTCCCTGGAATTCTCGGGTGCCAAGGATGGAAGATTTAGATGTC |
| rRNA.412 | NR_003287 | rRNA_28S_18S | GAGGACTAGGAACAGACGTAATACGACTCACTATAGGGAATGCCTCGTCATCTAATTAGTGACGCGCATGAATGGATGAACGAGATTCCCACTGTCCCTACCTACTATCCAGCGAAACCACAGCCAAGTGGAATTCTCGGGTGCCAAGGATGGAAGATTTAGATGTC |
| rRNA.413 | NR_003287 | rRNA_28S_18S | GAGGACTAGGAACAGACGTAATACGACTCACTATAGGGTGAATGGATGAACGAGATTCCCACTGTCCCTACCTACTATCCAGCGAAACCACAGCCAAGGGAACGGGCTTGGCGGAATCAGCGGGGAAATGGAATTCTCGGGTGCCAAGGATGGAAGATTTAGATGTC |
| rRNA.414 | NR_003287 | rRNA_28S_18S | GAGGACTAGGAACAGACGTAATACGACTCACTATAGGGTACCTACTATCCAGCGAAACCACAGCCAAGGGAACGGGCTTGGCGGAATCAGCGGGGAAAGAAGACCCTGTTGAGCTTGACTCTAGTCTGTGGAATTCTCGGGTGCCAAGGATGGAAGATTTAGATGTC |
| rRNA.415 | NR_003287 | rRNA_28S_18S | GAGGACTAGGAACAGACGTAATACGACTCACTATAGGGGGAACGGGCTTGGCGGAATCAGCGGGGAAAGAAGACCCTGTTGAGCTTGACTCTAGTCTGGCACGGTGAAGAGACATGAGAGGTGTAGAATGGAATTCTCGGGTGCCAAGGATGGAAGATTTAGATGTC |
| rRNA.416 | NR_003287 | rRNA_28S_18S | GAGGACTAGGAACAGACGTAATACGACTCACTATAGGGGAAGACCCTGTTGAGCTTGACTCTAGTCTGGCACGGTGAAGAGACATGAGAGGTGTAGAATAAGTGGGAGGCCCCCGGCGCCCCCCCGGTTGGAATTCTCGGGTGCCAAGGATGGAAGATTTAGATGTC |
| rRNA.417 | NR_003287 | rRNA_28S_18S | GAGGACTAGGAACAGACGTAATACGACTCACTATAGGGCACGGTGAAGAGACATGAGAGGTGTAGAATAAGTGGGAGGCCCCCGGCGCCCCCCCGGTGTCCCCGCGAGGGGCCCGGGGCGGGGTCCGTGGAATTCTCGGGTGCCAAGGATGGAAGATTTAGATGTC |
| rRNA.418 | NR_003287 | rRNA_28S_18S | GAGGACTAGGAACAGACGTAATACGACTCACTATAGGGTAAGTGGGAGGCCCCCGGCGCCCCCCCGGTGTCCCCGCGAGGGGCCCGGGGCGGGGTCCGCCGGCCCTGCGGGCCGCCGGTGAAATACCATGGAATTCTCGGGTGCCAAGGATGGAAGATTTAGATGTC |
| rRNA.419 | NR_003287 | rRNA_28S_18S | GAGGACTAGGAACAGACGTAATACGACTCACTATAGGGTCCCCGCGAGGGGCCCGGGGCGGGGTCCGCCGGCCCTGCGGGCCGCCGGTGAAATACCACTACTCTGATCGTTTTTTCACTGACCCGGTTGGAATTCTCGGGTGCCAAGGATGGAAGATTTAGATGTC |
| rRNA.420 | NR_003287 | rRNA_28S_18S | GAGGACTAGGAACAGACGTAATACGACTCACTATAGGGCCGGCCCTGCGGGCCGCCGGTGAAATACCACTACTCTGATCGTTTTTTCACTGACCCGGTGAGGCGGGGGGGCGAGCCCCGAGGGGGCTCTTGGAATTCTCGGGTGCCAAGGATGGAAGATTTAGATGTC |
| rRNA.421 | NR_003287 | rRNA_28S_18S | GAGGACTAGGAACAGACGTAATACGACTCACTATAGGGCTACTCTGATCGTTTTTTCACTGACCCGGTGAGGCGGGGGGGCGAGCCCCGAGGGGCTCTCGCTTCTGGCGCCAAGCGCCCGGCCCGCGCGTGGAATTCTCGGGTGCCAAGGATGGAAGATTTAGATGTC |
| rRNA.422 | NR_003287 | rRNA_28S_18S | GAGGACTAGGAACAGACGTAATACGACTCACTATAGGGGAGGCGGGGGGGGCGAGCCCCGAGGGGCTCTCGCTTCTGGCGCCAAGCGCCCGGCCCGCGCGCCGGCCGGGCGCGACCCGCTCCGGGGACAGTGGAATTCTCGGGTGCCAAGGATGGAAGATTTAGATGTC |
| rRNA.423 | NR_003287 | rRNA_28S_18S | GAGGACTAGGAACAGACGTAATACGACTCACTATAGGGCGCTTCTGGCGCCAAGCGCCCGGCCCGCGCGCCGGCCGGGCGCGACCCGCTCCGGGGACAGTGCCAGGTGGGGAGTTTGACTGGGGCGGTATGGAATTCTCGGGTGCCAAGGATGGAAGATTTAGATGTC |

| ASO ID | Accession | Group ID | Custom Array sequence |
|---|---|---|---|
| rRNA.424 | NR_003287 | rRNA_28S_18S | GAGGACTAGGACTCACTATAGCGCTAATACGACTCACTATAGGGGCCGCCCGGGCGCGGACCGCGCCGCCCGGGGCAGAGTGCCAGGGTGGGGGACGTTTGACTGGGGCGGTACACCTGTCAAACGGTAACGCAGGTGTCCTATGGATGAGAATTCTCAAACGATGGAAGATTTAGATGTC |
| rRNA.425 | NR_003287 | rRNA_28S_18S | GAGGACTAGGACTCACTATAGCGCTAATACGACTCACTATAGGGGCCGCCCGGGCGCGGACCGCGCCGCCCGGGGCAGAGTGCCAGGGTGGGGGCGCGGTACCAGGGTTTGACTGGGCGGTACACCTGTCAAACGGTAACGCAGGAGAGCAGAAACCTCCCGTGGAGCAGAGAGGGCAAAAGCTCGGAATTCTCGGGTGCCAAGGATGGAAGATTTAGATGTC |
| rRNA.426 | NR_003287 | rRNA_28S_18S | GAGGACTAGGAACAGAGACGTAATACGACTCACTATAGGGAGCGCACCTGTCTAAACGGTAACGCAGGTGTCCTAAGGCGAGCTCAGGGAGCAGAAAACCTCCCGTGGAGCAGAGAGGGACAAAAGCTCGCTTGATCTTGATTTTCAGTACGGAGAATTCTCGGGTGCCAAGGATGGAAGATTTAGATGTC |
| rRNA.427 | NR_003287 | rRNA_28S_18S | GAGGACTAGGAACAGAGACGTAATACGACTCACTATAGGGAGCTCACTATAGGGAGCGCACCTGTCTAAACGGTAACGCAGGTGTCCTAAGGCGAGCTCAGGGAGCAGAGAGGACAAAAGCTCGCTTGATCTTGATTTTCAGTACGGAGGGGCCTCACGATCCTTCGAACCTTTGCTTGATTTCGGGTGCCAAGGATGGAAGATTTAGATGTC |
| rRNA.428 | NR_003287 | rRNA_28S_18S | GAGGACTAGGAACAGAGACGTAATACGACTCACTATAGGGTGGAGCAGAAGGGCAAAAGCTCGCTTGATCTTGATTTTCAGTACGGAAGGCGGGGCCTCACGATCCTTCTGACCTTTTGGGTTTTAAGCAGGAATCTCTGACCTTTGATTTCGGGTGCCAAGGATGGAAGATTTAGATGTC |
| rRNA.429 | NR_003287 | rRNA_28S_18S | GAGGACTAGGACTCACTATAGGGGCCTCACGATCCTTCTGACCTTTTGGGTTTTAAGCAGTACGGAAGGCGGGGCCTCACGATCCTTCTGACCTTTTGGGTTTTAAGCAGGAATCTCTGACCTTTGATTTCGGGTGCCAAGGATGGAAGATTTAGATGTC |
| rRNA.430 | NR_003287 | rRNA_28S_18S | GAGGACTAGGACTCACTATAGGGGCCTCACGATCCTTCTGACCTTTTGGGTTTTAAGCAGGAATCTCTGACCTTTGAAACGGTACCACAGGATAACTGGCTTGTGGCGGCCAAGCGTTCATAGCGACGTCGCTTTTCGGGTGCCAAGGATGGAAGATTTAGATGTC |
| rRNA.431 | NR_003287 | rRNA_28S_18S | GAGGACTAGGAACAGAGACGTAATACGACTCACTATAGGGTGGCTTGTGGCGGCCAAGCGTTCATAGCGACGTCGCTTTTCGATCGTTCGATGTCGGCTCTTCCTATCATTGTGAAGCAGAATTCACCAAGCGTTGGATCTTCGGGTGCCAAGGATGGAAGATTTAGATGTC |
| rRNA.432 | NR_003287 | rRNA_28S_18S | GAGGACTAGGAACAGAGACGTAATACGACTCACTATAGGGTACCACAGGATAACTGGCTTGTGGCGGCCAAGCGTTCATAGCGACGTCGCTTTTCGATCGTTCGATGTCGGCTCTTCCTATCATTGTGAAGCAGAATTCACCAAGCGTTGAATTGTTCATGGATGGAAGATTTAGATGTC |
| rRNA.433 | NR_003287 | rRNA_28S_18S | GAGGACTAGGAACAGAGACGTAATACGACTCACTATAGGGTCGATGTCGGCTCTTCCTATCATTGTGAAGCAGAATTCACCAAGCGTTGAATTGTTCACCCACTAATAGGGGAACGTGAGCTGGGTTTATGGATGGAAGATTTAGATGTC |
| rRNA.434 | NR_003287 | rRNA_28S_18S | GAGGACTAGGAACAGAGACGTAATACGACTCACTATAGGGTCTTCCTATCATTGTGAAGCAGAATTCACCAAGCGTTGGATTGTTCACCCACTAATAGGGGAACGTGAGCTGGGTTTAGACCGTCTGTGATGACCTTAGCCTTTACCCTACTGATGGAAGATTTAGATGTC |
| rRNA.435 | NR_003287 | rRNA_28S_18S | GAGGACTAGGAACAGAGACGTAATACGACTCACTATAGGGCCCACTAATAGGGGAACGTGAGCTGGGTTTAGACCGTCGTGAGACAGGTTAGTTTTACCCTACTGATGTTGTTGCCATGGTAATTCTGCTCAGTACGAGAGGAACCGCAGGTTCAGTGGATGGAAGATTTAGATGTC |
| rRNA.436 | NR_003287 | rRNA_28S_18S | GAGGACTAGGAACAGAGACGTAATACGACTCACTATAGGGTTAGACCGTCGTGAGACAGGTTAGTTTTACCCTACTGATGTTGTTGCCATGGTAATTCTGCTCAGTACGAGAGGAACCGCAGGTTCAGTGGATGGAAGATTTAGATGTC |
| rRNA.437 | NR_003287 | rRNA_28S_18S | GAGGACTAGGAACAGAGACGTAATACGACTCACTATAGGGGTTAGTTTTACCCTACTGATGTTGTTGCCATGGTAATTCTGCTCAGTACGAGAGGAACCGCAGGTTCAGTGGATTTGGTATGTCTAGTAGACAGCATTTGGTCTGGATGGAAGATTTAGATGTC |
| rRNA.438 | NR_003287 | rRNA_28S_18S | GAGGACTAGGAACAGAGACGTAATACGACTCACTATAGGGGTGTTGCCATGGTAATTCTGCTCAGTACGAGAGGAACCGCAGGTTCAGTGGATTTGGTATGTGTATGTCTAGTAGACAGCATTTGGTCTGGACATTTGGTATGGCCGAAGCTACCATCTGTGGGATTGGATGGAAGATTTAGATGTC |
| rRNA.439 | NR_003287 | rRNA_28S_18S | GAGGACTAGGAACAGAGACGTAATACGACTCACTATAGGGTCTGCTGGCTGGCTGAGGAGCCAATGGGAGGATTTGGTATGTGTATGTCTAGTAGACAGCATTTGGTCTGGATTGGTATGGCCGAAGCTACCATCTGTGGGATTGGATGGAAGATTTAGATGTC |
| rRNA.440 | NR_003287 | rRNA_28S_18S | GAGGACTAGGAACAGAGACGTAATACGACTCACTATAGGGGACATTTGGTATGGCCGAAGCTACCATCTGTGGGATTGGTATGGCCGAAGCTACCATCTGTGGGATTATGACTCAGTGGGATTATGACTGAACGCCTCTAAGTCAGAATCCCGCCCAGGCGGGATGGAAGATTTAGATGTC |
| rRNA.441 | NR_003287 | rRNA_28S_18S | GAGGACTAGGAACAGAGACGTAATACGACTCACTATAGGGGATTGGTATGGCCGAAGCTACCATCTGTGGGATTATGACTGAACGCCTCTAAGTCAGAATCCCGCCCAGGCGGCGACGATACGGCAGCGCCGCCAGGCCTCGGTTGGATGGAAGATTTAGATGTC |
| rRNA.442 | NR_003287 | rRNA_28S_18S | GAGGACTAGGAACAGAGACGTAATACGACTCACTATAGGGGACTGAACGCCTCTAAGTCAGAATCCCGCCCAGGCGGCGACGATACGGCAGCGCCGCCAGGCCTCGGTTGGCTCGGATAGCCGGTCCTGCCCCGTCCCCGGCGGGATGGAAGATTTAGATGTC |
| rRNA.443 | NR_003287 | rRNA_28S_18S | GAGGACTAGGAACAGAGACGTAATACGACTCACTATAGGGCCGCCAGGCCTCGGTTGGCTCGGATAGCCGGTCCTGCCCCGTCCCCGGCGGGATAGCCGGTCCTGCCCCGTCCCCGGCGGCGCGCCGGGATAGGAAGCTGGATGGAAGATTTAGATGTC |
| rRNA.444 | NR_003287 | rRNA_28S_18S | GAGGACTAGGAACAGAGACGTAATACGACTCACTATAGGGCTCGGATAGCCGGTCCTGCCCCGTCCCCGGCGGCGCGCCGGGATAGGAAGCTGGATGGAAGCTGGCGGGACGGGGAGGAGGCTGGATGGAAGATTTAGATGTC |
| rRNA.445 | NR_003287 | rRNA_28S_18S | GAGGACTAGGAACAGAGACGTAATACGACTCACTATAGGGCCCCGTCCCCGGCGGCGCGCCGGGATAGGAAGCTGGCGGGACGGGGAGGAGGCTGGATGGAAATGGAAGATTCTCGGGTGCCAAGGATGGAAGATTTAGATGTC |
| rRNA.446 | NR_003287 | rRNA_28S_18S | GAGGACTAGGAACAGAGACGTAATACGACTCACTATAGGGCGCCCGGGATAGGAAGCTGGCGGGACGGGGAGGAGGCTGGATGGAAATGGAAGATTCTCGGGTGCCAAGGATGGAAGATTCTCGGGTGCCAAGGATGGAAGATTTAGATGTC |
| rRNA.447 | NR_003287 | rRNA_28S_18S | GAGGACTAGGAACAGAGACGTAATACGACTCACTATAGGGGGTGCTCGGTCTCTGGGAAAACGGGCGCCCCTTCGTCTGGGAGTGCCCTTCGTCTGGGAAAACGGGCGCCGCCCCTCGTCTGGGAGTGCCCTTCGTCTGGGTGGGATGGAAGATTTAGATGTC |
| rRNA.448 | NR_003287 | rRNA_28S_18S | GAGGACTAGGAACAGAGACGTAATACGACTCACTATAGGGGGGGACGGTGCCCTTCGTCTGGGAGTGCCCTTCGTCTGGGAAAGGCGGGCCGCCCCCTCGCCCGCCGGGCGCCGCCCCCTCGTCTGGGTGGGTGGGATGGAAGATTTAGATGTC |

| ASO ID | Accession | Group ID | Custom Array sequence |
|---|---|---|---|
| rRNA.449 | NR_003287 | rRNA_28S_18S | GAAGGACTAGGAACAGACGTAATACGACTCACTATAGGGCGGGGGCGCGGCGTGGAAAGGCGGCGCCCCCTCCGCCGTCACGCACCGCACGTTCGTGGGAACCTGGCGCTAAACCATTCGTAGACGACTGGAATTCTCGGGTGCCAAGGATGGAAGGATTTAGATGTC |
| rRNA.450 | NR_003287 | rRNA_28S_18S | GAAGGACTAGGAACAGACGTAATACGACTCACTATAGGGTCGGCCACGCACCGCACGTTCGTGGGAACCTGGCGCTAAACCATTCGTAGACGACTGGAATTCTCGGGTGCCAAGGATGGAAGGATTTAGAGTGTC |
| rRNA.451 | NR_003287 | rRNA_28S_18S | GAAGGACTAGGAACAGACGTAATACGACTCACTATAGGGAACCTGGCGCTAAACCATTCGTAGAGAGCAGCTCCCTCCGCTGCGATCTATTGAAATGGAATTCTCGGGTGCCAAGGATGGAAGGATTTAGATGTC |

The SARS-CoV-2 transcriptome consists of (1) a genomic RNA (gRNA) encoding 16 nonstructural proteins (nsps) and (2) multiple subgenomic RNAs (sgRNAs) that encode structural and accessory proteins (Sola et al., 2015). The sgRNAs are more abundant than the gRNA (Kim et al., 2020a). The first pool ("Probe I") consists of 707 oligos tiles every 30 nucleotides across the ORF1ab region (266:21553, NC_045512.2) and thus hybridizes specifically with the gRNA molecules (FIG. 1b). The second pool of 275 oligos ("Probe II") covers the remaining region (21563:29872, NC_045512.2) which is shared by both the gRNA and sgRNAs.

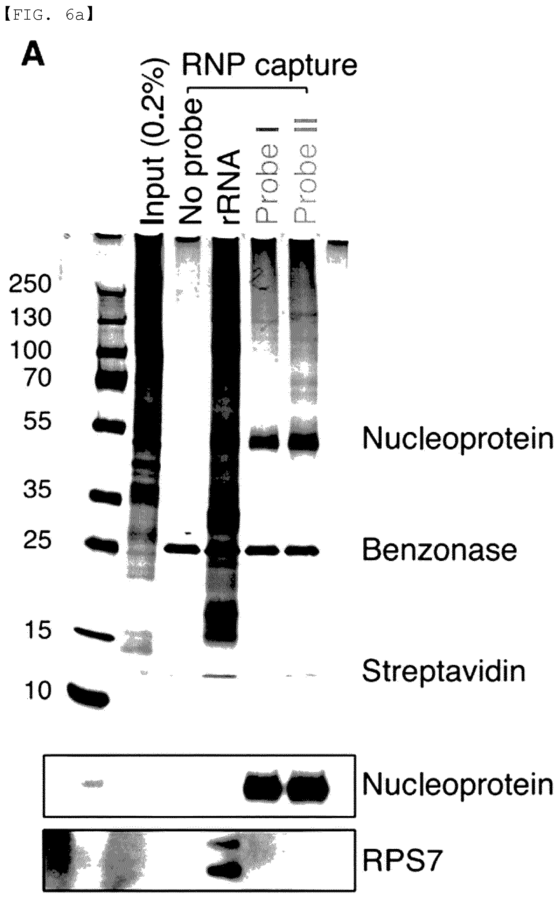

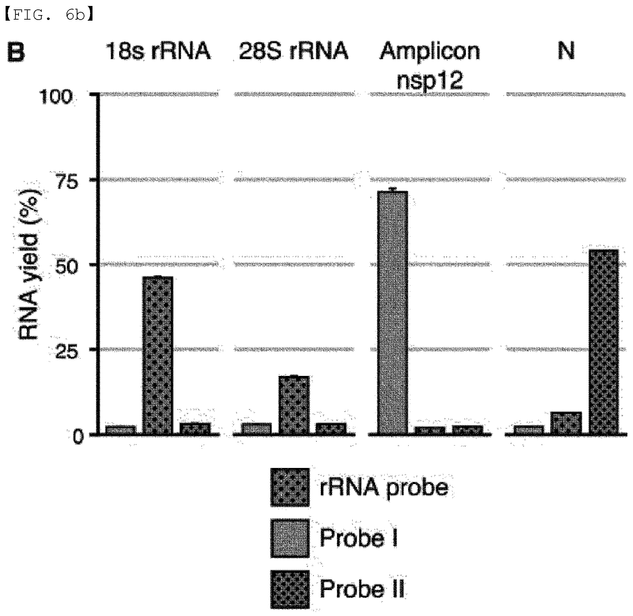

To first check whether our method specifically captures the viral RNP complexes, we compared the resulting purification from Vero cells infected with SARS-CoV-2 (BetaCoV/Korea/KCDC03/2020) at MOI 0.1 for 24 hours (Kim et al., 2020b) by either Probe I or Probe II. As negative controls, we pulled-down without probes ("no probe" control) or with the control probes (for either 18S or 28S rRNA). Protein composition of each RNP sample was distinct as shown by silver staining and western blotting (FIG. 6a) with prominent SARSCoV- 2 N protein associated with Probes I and II, as expected. Enrichment of SARS-CoV-2 RNAs were confirmed by RT-qPCR (FIG. 6b), suggesting that our protocol purfies specific RNP complexes. Note that SARS-CoV-2 gRNA was not enriched in the Probe II experiment, hinting at the excess amount of sgRNAs over gRNA in our culture condition.

**2. Comprehensive identification of proteins binding to the SARS-CoV-2 transcripts**

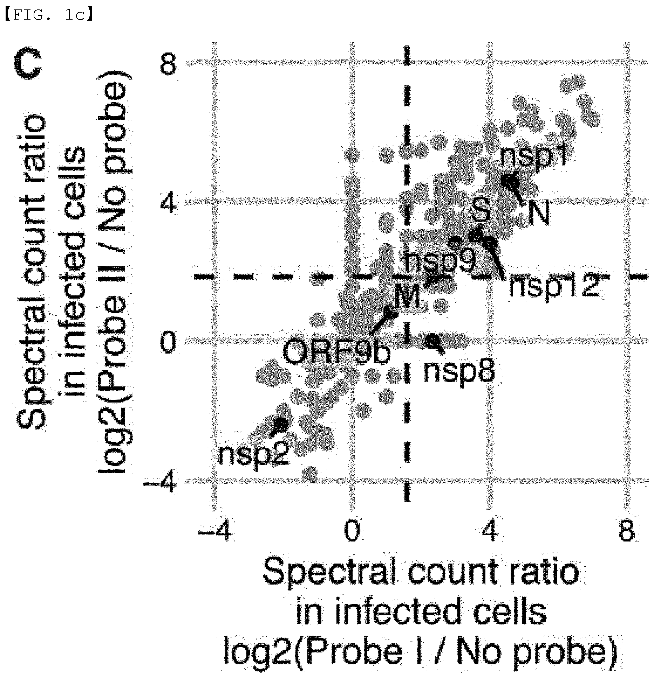

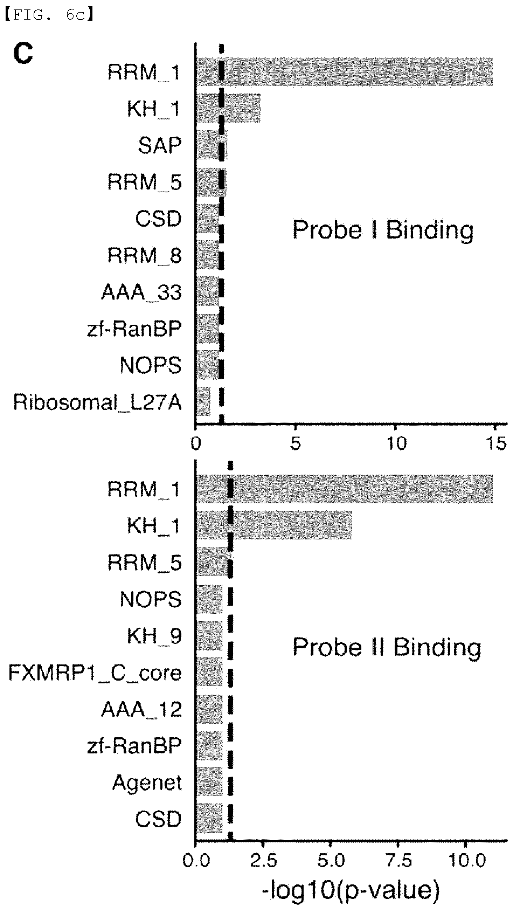

We conducted Label-free quantification (LFQ) by liquid chromatography with tandem mass spectrometry (LC-MS/MS) and identified 429 host proteins and 9 viral proteins in total (FIG. 1c). As highly abundant proteins may nonspecifically coprecipitate during the RNP capture experiment, we statistically modelled this protein background as a multinomial distribution and assessed the probability (i.e. p-value) of the quantity of the identified protein in the RNP capture (e.g. Probe I) experiment over the protein background of the control (e.g. no-probe) experiment (see Methods for details). This unweighted spectral count analysis resulted in 199 and 220 proteins that are overrepresented in the Probe I and Probe II sample, respectively (FDR < 10%, Table 2). Protein domain enrichment analysis revealed that these proteins indeed harbor RNAbinding domains such as RNA recognition motif (RRM) domain and K Homology (KH) domain (FIG. 6c). Of note, unlike the cellular mRNA interactome (Castello et al., 2012; Gerstberger et al., 2014), the RNA-binding repertoire of SARS-CoV-2 RNAs showed a depletion of DEAD/DEAH box helicase domains and an enrichment of KH domain.

[Table 2]

| Protein IDs | vervet.uniprot | gname | species | target | background | adj.p | vervet.gname | human.gname |
|---|---|---|---|---|---|---|---|---|
| ncbi\|YP_009724397 | YP_009724397.2 | N | SARS2 | 680 | 27 | 1.0E-470 | NA | NA |
| A0A0D9RB84 | A0A0D9RB84 | KHSRP | CHLSB | 128 | 1 | 4.239E-174 | KHSRP | KHSRP |
| A0A0D9RNX3;A0AC | A0A0D9RNX3 | HNRNPA2B1 | CHLSB | 116 | 1 | 3.858E-153 | NA | HNRNPA2B1 |
| A0A0D9RJZ5 | A0A0D9RJZ5 | PTBP1 | CHLSB | 108 | 1 | 1.797E-139 | PTBP1 | PTBP1 |
| A0A0D9RQH8 | A0A0D9RQH8 | FUBP3 | CHLSB | 94 | 1 | 4.619E-116 | FUBP3 | FUBP3 |
| A0A0D9RVK4 | A0A0D9RVK4 | HNRNPC | CHLSB | 109 | 2 | 3.625E-109 | HNRNPC | HNRNPC |
| A0A0D9R7T1 | A0A0D9R7T1 | HNRNPH1 | CHLSB | 78 | 1 | 1.851E-90 | HNRNPH1 | HNRNPH1 |
| A0A0D9R723;A0A0 | A0A0D9R723 | ELAVL1 | CHLSB | 77 | 1 | 5.951E-89 | ELAVL1 | ELAVL1 |
| A0A0D9RXQ9 | A0A0D9RXQ9 | SYNCRIP | CHLSB | 76 | 1 | 1.919E-87 | SYNCRIP | SYNCRIP |
| A0A0D9R8Y5;A0A0 | A0A0D9R8Y5 | CNBP* | CHLSB | 74 | 1 | 2.184E-84 | CNBP | CNBP |
| A0A0D9R6L1 | A0A0D9R6L1 | HNRNPM | CHLSB | 71 | 1 | 8.076E-80 | HNRNPM | HNRNPM |
| A0A0D9RFM7;A0AC | A0A0D9RFM7 | RPS2 | CHLSB | 69 | 1 | 8.171E-77 | RPS2 | RPS2 |
| A0A0D9R2V4 | A0A0D9R2V4 | NCL | CHLSB | 117 | 5 | 9.979E-77 | NCL | NCL |
| A0A0D9QZF1;A0AC | A0A0D9QZF1 | HNRNPA1 | CHLSB | 97 | 3 | 1.463E-76 | HNRNPA1 | HNRNPA1 |
| A0A0D9RKP7 | A0A0D9RKP7 | LRPPRC* | CHLSB | 109 | 5 | 1.473E-68 | LRPPRC* | LRPPRC |
| A0A0D9RPE9;A0A0 | A0A0D9RPE9 | HNRNPA3 | CHLSB | 59 | 1 | 4.111E-62 | HNRNPA3 | HNRNPA3 |
| A0A0D9REQ6 | A0A0D9REQ6 | G3BP1 | CHLSB | 56 | 1 | 7.497E-58 | G3BP1 | G3BP1 |
| A0A0D9RKW3 | A0A0D9RKW3 | MATR3* | CHLSB | 56 | 1 | 7.497E-58 | NA | MATR3 |
| A0A0D9R5A7;A0A0 | A0A0D9R5A7 | HNRNPK* | CHLSB | 55 | 1 | 1.878E-56 | HNRNPK* | HNRNPK |
| A0A0D9RA32 | A0A0D9RA32 | HNRNPU | CHLSB | 75 | 3 | 8.083E-52 | HNRNPU | HNRNPU |
| A0A0D9S7U2 | A0A0D9S7U2 | SFPQ | CHLSB | 50 | 1 | 1.672E-49 | SFPQ | SFPQ |
| A0A0D9S1G6;A0AC | A0A0D9S1G6 | RPS3A | CHLSB | 48 | 1 | 8.748E-47 | RPS3A | RPS3A |
| A0A0D9S3H8;A0A0 | A0A0D9S3H8 | HNRNPAB* | CHLSB | 44 | 1 | 1.964E-41 | HNRNPAB* | HNRNPAB |
| A0A0D9QVC3 | A0A0D9QVC3 | HNRNPL | CHLSB | 43 | 1 | 3.926E-40 | HNRNPL | HNRNPL |
| A0A0D9R8L5;A0A0 | A0A0D9R8L5 | YBX3 | CHLSB | 41 | 1 | 1.531E-37 | YBX3 | YBX3 |
| A0A0D9QWR4 | A0A0D9QWR4 | HNRNPD | CHLSB | 37 | 1 | 1.580E-32 | HNRNPD | HNRNPD |
| A0A0D9R403 | A0A0D9R403 | FAM120A | CHLSB | 37 | 1 | 1.580E-32 | FAM120A | FAM120A |
| A0A0D9RG28 | A0A0D9RG28 | NONO* | CHLSB | 37 | 1 | 1.580E-32 | NONO* | NONO |
| A0A0D9S679 | A0A0D9S679 | CSDE1 | CHLSB | 37 | 1 | 1.580E-32 | CSDE1 | CSDE1 |
| A0A0D9S7K7 | A0A0D9S7K7 | YBX1 | CHLSB | 37 | 1 | 1.580E-32 | YBX1 | YBX1 |
| A0A0D9S3T6;A0A0 | A0A0D9S3T6 | RPS11 | CHLSB | 35 | 1 | 4.613E-30 | RPS11 | RPS11 |
| A0A0D9RI90 | A0A0D9RI90 | RALY | CHLSB | 33 | 1 | 1.208E-27 | RALY | RALY |
| A0A0D9QVI6 | A0A0D9QVI6 | DDX5 | CHLSB | 32 | 1 | 1.692E-26 | DDX5 | DDX5 |
| A0A0D9RNG4 | A0A0D9RNG4 | IGF2BP2 | CHLSB | 32 | 1 | 1.692E-26 | IGF2BP2 | IGF2BP2 |
| A0A0D9S6X7 | A0A0D9S6X7 | FUBP1 | CHLSB | 32 | 1 | 1.692E-26 | FUBP1 | FUBP1 |
| A0A0D9S897 | A0A0D9S897 | HNRNPR | CHLSB | 32 | 1 | 1.692E-26 | HNRNPR | HNRNPR |
| A0A0D9QZI9;A0A0I | A0A0D9QZI9 | RPL18A | CHLSB | 31 | 1 | 2.387E-25 | RPL18A | RPL18A |
| A0A0D9R833 | A0A0D9R833 | ZC3HAV1 | CHLSB | 31 | 1 | 2.387E-25 | ZC3HAV1 | ZC3HAV1 |
| A0A0D9REA3 | A0A0D9REA3 | MRPL13 | CHLSB | 31 | 1 | 2.387E-25 | MRPL13 | MRPL13 |
| A0A0D9RHW7;A0A | A0A0D9RHW7 | PABPC1 | CHLSB | 29 | 1 | 4.954E-23 | PABPC1 | PABPC1 |
| A0A0D9QWR3 | A0A0D9QWR3 | HNRNPDL | CHLSB | 28 | 1 | 6.557E-22 | HNRNPDL | HNRNPDL |
| A0A0D9S8N2;A0A0 | A0A0D9S8N2 | TARDBP* | CHLSB | 28 | 1 | 6.557E-22 | NA | TARDBP |
| A0A0D9R787;A0A0 | A0A0D9R787 | RPL8 | CHLSB | 66 | 7 | 9.424E-22 | RPL8 | RPL8 |
| A0A0D9QXD5 | A0A0D9QXD5 | G3BP2 | CHLSB | 26 | 1 | 1.067E-19 | G3BP2 | G3BP2 |
| A0A0D9S6D9 | A0A0D9S6D9 | RPS5 | CHLSB | 26 | 1 | 1.067E-19 | RPS5 | RPS5 |
| A0A0D9S020 | A0A0D9S020 | EIF4H | CHLSB | 25 | 1 | 1.305E-18 | EIF4H | EIF4H |
| A0A0D9R2U6 | A0A0D9R2U6 | DDX17 | CHLSB | 24 | 1 | 1.477E-17 | DDX17 | DDX17 |
| A0A0D9RGJ5 | A0A0D9RGJ5 | RPS14 | CHLSB | 24 | 1 | 1.477E-17 | RPS14 | RPS14 |
| A0A0D9SDY5;A0A0 | A0A0D9SDY5 | RPL22 | CHLSB | 24 | 1 | 1.477E-17 | RPL22 | RPL22 |
| A0A0D9R5E6 | A0A0D9R5E6 | HNRNPH3 | CHLSB | 23 | 1 | 1.552E-16 | HNRNPH3 | HNRNPH3 |
| A0A0D9RGN3 | A0A0D9RGN3 | MRPL2 | CHLSB | 23 | 1 | 1.552E-16 | MRPL2 | MRPL2 |
| A0A0D9RIR4 | A0A0D9RIR4 | PTBP3 | CHLSB | 23 | 1 | 1.552E-16 | PTBP3 | PTBP3 |
| A0A0D9RQ30 | A0A0D9RQ30 | RPS20* | CHLSB | 23 | 1 | 1.552E-16 | RPS20 | RPS20 |
| ncbi\|YP_009725297 | YP_009725297.1 | nsp1 | SARS2 | 23 | 1 | 1.552E-16 | NA | NA |
| A0A0D9RIM7 | A0A0D9RIM7 | SRSF7 | CHLSB | 21 | 1 | 1.809E-14 | SRSF7 | SRSF7 |
| A0A0D9R0F6;A0A0 | A0A0D9R0F6 | CELF1 | CHLSB | 20 | 1 | 1.720E-13 | CELF1 | CELF1 |
| A0A0D9R790 | A0A0D9R790 | MRPL11 | CHLSB | 20 | 1 | 1.720E-13 | MRPL11 | MRPL11 |
| A0A0D9RC52 | A0A0D9RC52 | SAFB | CHLSB | 20 | 1 | 1.720E-13 | SAFB | SAFB |
| A0A0D9S4M9 | A0A0D9S4M9 | ALYREF | CHLSB | 20 | 1 | 1.720E-13 | ALYREF | ALYREF |
| A0A0D9R5W1 | A0A0D9R5W1 | MRPL44 | CHLSB | 19 | 1 | 1.545E-12 | MRPL44 | MRPL44 |
| A0A0D9RDB1 | A0A0D9RDB1 | MYEF2 | CHLSB | 19 | 1 | 1.545E-12 | MYEF2 | MYEF2 |
| A0A0D9RHP8;A0A0 | A0A0D9RHP8 | MBNL1 | CHLSB | 19 | 1 | 1.545E-12 | MBNL1 | MBNL1 |
| A0A0D9S7Y1;A0A0 | A0A0D9S7Y1 | KHDRBS1 | CHLSB | 19 | 1 | 1.545E-12 | KHDRBS1 | KHDRBS1 |
| A0A0D9SC26;A0A0 | A0A0D9SC26 | RPL17* | CHLSB | 19 | 1 | 1.545E-12 | RPL17* | RPL17 |
| A0A0D9S7H2 | A0A0D9S7H2 | RPS8 | CHLSB | 41 | 5 | 2.978E-12 | RPS8 | RPS8 |
| A0A0D9RMB4;A0A | A0A0D9RMB4 | RPL7* | CHLSB | 25 | 2 | 7.667E-12 | RPL7* | RPL7 |
| A0A0D9RFF7;A0A0 | A0A0D9RFF7 | RPS4* | CHLSB | 18 | 1 | 1.312E-11 | RPS4X | RPS4X |
| A0A0D9RLC1 | A0A0D9RLC1 | RBM3 | CHLSB | 18 | 1 | 1.312E-11 | RBM3 | RBM3 |
| A0A0D9RWG7 | A0A0D9RWG7 | RPL14* | CHLSB | 18 | 1 | 1.312E-11 | RPL14* | RPL14 |
| A0A0D9S6R4;A0A0 | A0A0D9S6R4 | RPL5* | CHLSB | 18 | 1 | 1.312E-11 | RPL5 | RPL5 |
| A0A0D9R4I5 | A0A0D9R4I5 | SF1 | CHLSB | 17 | 1 | 1.095E-10 | SF1 | SF1 |
| A0A0D9RAL5;A0A0 | A0A0D9RAL5 | RPS6 | CHLSB | 17 | 1 | 1.095E-10 | RPS6 | RPS6 |
| A0A0D9RNA2;A0AC | A0A0D9RNA2 | DDX3X | CHLSB | 17 | 1 | 1.095E-10 | DDX3X | DDX3X |
| A0A0D9S713 | A0A0D9S713 | SERBP1 | CHLSB | 17 | 1 | 1.095E-10 | SERBP1 | SERBP1 |
| A0A0D9RCS8 | A0A0D9RCS8 | SND1 | CHLSB | 16 | 1 | 8.366E-10 | SND1 | SND1 |
| A0A0D9RD33 | A0A0D9RD33 | SRSF3 | CHLSB | 16 | 1 | 8.366E-10 | SRSF3 | SRSF3 |

| Protein IDs | vervet.uniprot | gname | species | target | background | adj.p | vervet.gname | human.gname |
|---|---|---|---|---|---|---|---|---|
| A0A0D9RH50 | A0A0D9RH50 | SLIRP | CHLSB | 16 | 1 | 8.366E-10 | SLIRP | SLIRP |
| A0A0D9RJE1 | A0A0D9RJE1 | DHX9 | CHLSB | 16 | 1 | 8.366E-10 | DHX9 | DHX9 |
| A0A0D9S6D8 | A0A0D9S6D8 | MOV10 | CHLSB | 16 | 1 | 8.366E-10 | MOV10 | MOV10 |
| A0A0D9SA63 | A0A0D9SA63 | RPL35* | CHLSB | 16 | 1 | 8.366E-10 | RPL35* | RPL35 |
| ncbi|YP_009725307 | YP_009725307.1 | nsp12 | SARS2 | 16 | 1 | 8.366E-10 | NA | NA |
| A0A0D9QZB7 | A0A0D9QZB7 | MRPL27 | CHLSB | 15 | 1 | 6.229E-09 | MRPL27 | MRPL27 |
| A0A0D9R1R4 | A0A0D9R1R4 | RPL24 | CHLSB | 15 | 1 | 6.229E-09 | RPL24 | RPL24 |
| A0A0D9R3S5 | A0A0D9R3S5 | ILF3 | CHLSB | 15 | 1 | 6.229E-09 | ILF3 | ILF3 |
| A0A0D9RG34 | A0A0D9RG34 | NONO* | CHLSB | 15 | 1 | 6.229E-09 | NONO* | NONO |
| A0A0D9S9H0 | A0A0D9S9H0 | PURA | CHLSB | 15 | 1 | 6.229E-09 | PURA | PURA |
| A0A0D9QXT8 | A0A0D9QXT8 | GRSF1 | CHLSB | 14 | 1 | 4.290E-08 | GRSF1 | GRSF1 |
| A0A0D9R6X7 | A0A0D9R6X7 | SSBP1 | CHLSB | 14 | 1 | 4.290E-08 | SSBP1 | SSBP1 |
| A0A0D9RNH2;A0A( | A0A0D9RNH2 | SRSF6 | CHLSB | 14 | 1 | 4.290E-08 | SRSF6 | SRSF6 |
| A0A0D9RTU4 | A0A0D9RTU4 | MAP4* | CHLSB | 14 | 1 | 4.290E-08 | MAP4* | MAP4 |
| A0A0D9S8U1 | A0A0D9S8U1 | LRRC47 | CHLSB | 14 | 1 | 4.290E-08 | LRRC47 | LRRC47 |
| A0A0D9SCE0 | A0A0D9SCE0 | RPL21* | CHLSB | 14 | 1 | 4.290E-08 | RPL21* | RPL21 |
| A0A0D9SE18 | A0A0D9SE18 | RPL3* | CHLSB | 14 | 1 | 4.290E-08 | RPL3* | RPL3 |
| A0A0D9RN36;A0A( | A0A0D9RN36 | RPL29* | CHLSB | 32 | 5 | 1.792E-07 | NA | RPL29 |
| A0A0D9RBD8;A0A( | A0A0D9RBD8 | NPM1 | CHLSB | 13 | 1 | 2.789E-07 | NPM1 | NPM1 |
| A0A0D9RI73 | A0A0D9RI73 | DAZAP1 | CHLSB | 13 | 1 | 2.789E-07 | DAZAP1 | DAZAP1 |
| A0A0D9RI76 | A0A0D9RI76 | MRPS18A | CHLSB | 13 | 1 | 2.789E-07 | MRPS18A | MRPS18A |
| A0A0D9RNI1 | A0A0D9RNI1 | TRA2B | CHLSB | 13 | 1 | 2.789E-07 | TRA2B | TRA2B |
| A0A0D9RQA3 | A0A0D9RQA3 | MRPL15 | CHLSB | 13 | 1 | 2.789E-07 | MRPL15 | MRPL15 |
| A0A0D9RTV5 | A0A0D9RTV5 | DHX30 | CHLSB | 13 | 1 | 2.789E-07 | DHX30 | DHX30 |
| A0A0D9QZS8 | A0A0D9QZS8 | MRPL43 | CHLSB | 12 | 1 | 1.627E-06 | MRPL43 | MRPL43 |
| A0A0D9RAB6 | A0A0D9RAB6 | RPS24* | CHLSB | 12 | 1 | 1.627E-06 | RPS24* | RPS24 |
| A0A0D9RIJ2 | A0A0D9RIJ2 | CIRBP | CHLSB | 12 | 1 | 1.627E-06 | CIRBP | CIRBP |
| A0A0D9RK55 | A0A0D9RK55 | MRPL28 | CHLSB | 12 | 1 | 1.627E-06 | MRPL28 | MRPL28 |
| A0A0D9RQZ1;A0A( | A0A0D9RQZ1 | RPS23 | CHLSB | 12 | 1 | 1.627E-06 | RPS23 | RPS23 |
| A0A0D9RU12 | A0A0D9RU12 | PABPN1 | CHLSB | 12 | 1 | 1.627E-06 | PABPN1 | PABPN1 |
| A0A0D9RWW2;A0A | A0A0D9RWW2 | RBM47 | CHLSB | 12 | 1 | 1.627E-06 | RBM47 | RBM47 |
| A0A0D9S9G6 | A0A0D9S9G6 | HNRNPA0 | CHLSB | 12 | 1 | 1.627E-06 | HNRNPA0 | HNRNPA0 |
| A0A0D9S9I2;A0A0C | A0A0D9S9I2 | RPS7* | CHLSB | 12 | 1 | 1.627E-06 | RPS7* | RPS7 |
| A0A0D9SCC6;A0A( | A0A0D9SCC6 | ILF2* | CHLSB | 12 | 1 | 1.627E-06 | ILF2* | ILF2 |
| A0A0D9SCR9 | A0A0D9SCR9 | RPL31* | CHLSB | 12 | 1 | 1.627E-06 | RPL31* | RPL31 |
| ncbi|YP_009724390 | YP_009724390.1 | S | SARS2 | 12 | 1 | 1.627E-06 | NA | NA |
| ncbi|YP_009724393 | YP_009724393.1 | M | SARS2 | 36 | 7 | 4.899E-06 | NA | NA |
| A0A0D9R014 | A0A0D9R014 | SRSF1* | CHLSB | 11 | 1 | 9.545E-06 | SRSF1* | SRSF1 |
| A0A0D9RT27 | A0A0D9RT27 | RBMS1 | CHLSB | 11 | 1 | 9.545E-06 | RBMS1 | RBMS1 |
| A0A0D9SE44 | A0A0D9SE44 | RPS26* | CHLSB | 11 | 1 | 9.545E-06 | RPS26* | RPS26 |
| A0A0D9R0F2 | A0A0D9R0F2 | HDLBP | CHLSB | 23 | 4 | 4.205E-05 | HDLBP | HDLBP |
| A0A0D9REP7 | A0A0D9REP7 | EEF2 | CHLSB | 19 | 3 | 4.941E-05 | EEF2 | EEF2 |
| A0A0D9QXH3 | A0A0D9QXH3 | TIAL1 | CHLSB | 10 | 1 | 5.020E-05 | TIAL1 | TIAL1 |
| A0A0D9R0F3 | A0A0D9R0F3 | XRCC6 | CHLSB | 10 | 1 | 5.020E-05 | XRCC6 | XRCC6 |
| A0A0D9R6B6 | A0A0D9R6B6 | EIF3D EIF3S7 | CHLSB | 10 | 1 | 5.020E-05 | EIF3D | EIF3D |
| A0A0D9RXB8 | A0A0D9RXB8 | PCBP2* | CHLSB | 10 | 1 | 5.020E-05 | PCBP2* | PCBP2 |
| A0A0D9S7P0 | A0A0D9S7P0 | PABPC4 | CHLSB | 10 | 1 | 5.020E-05 | PABPC4 | PABPC4 |
| A0A0D9SAS2;A0A( | A0A0D9SAS2 | RPL28* | CHLSB | 10 | 1 | 5.020E-05 | RPL28* | RPL28 |
| A0A0D9QXG7 | A0A0D9QXG7 | MRPS7 | CHLSB | 9 | 1 | 2.420E-04 | MRPS7 | MRPS7 |
| A0A0D9R1V9 | A0A0D9R1V9 | ATXN2L | CHLSB | 9 | 1 | 2.420E-04 | ATXN2L | ATXN2L |
| A0A0D9R7E8 | A0A0D9R7E8 | PARP12 | CHLSB | 9 | 1 | 2.420E-04 | PARP12 | PARP12 |
| A0A0D9R7L9 | A0A0D9R7L9 | RBM14* | CHLSB | 9 | 1 | 2.420E-04 | RBM14* | RBM14 |
| A0A0D9RBS5 | A0A0D9RBS5 | EWSR1 | CHLSB | 9 | 1 | 2.420E-04 | EWSR1 | EWSR1 |
| A0A0D9RL59 | A0A0D9RL59 | FXR1 | CHLSB | 9 | 1 | 2.420E-04 | FXR1 | FXR1 |
| A0A0D9S773 | A0A0D9S773 | MRPL37 | CHLSB | 9 | 1 | 2.420E-04 | MRPL37 | MRPL37 |
| A0A0D9SAB7 | A0A0D9SAB7 | RPL5* | CHLSB | 9 | 1 | 2.420E-04 | RPL5* | RPL5 |
| A0A0D9SCL8;A0A0 | A0A0D9SCL8 | HIST1H2BB | CHLSB | 9 | 1 | 2.420E-04 | H2BC3 | H2BC3 |
| A0A0D9RL86 | A0A0D9RL86 | RPL4* | CHLSB | 36 | 9 | 7.218E-04 | RPL4* | RPL4 |
| A0A0D9R5N6 | A0A0D9R5N6 | FAU | CHLSB | 8 | 1 | 1.069E-03 | FAU | FAU |
| A0A0D9RC61 | A0A0D9RC61 | SAFB2 | CHLSB | 8 | 1 | 1.069E-03 | SAFB2 | SAFB2 |
| A0A0D9RJV6 | A0A0D9RJV6 | RPL22L1 | CHLSB | 8 | 1 | 1.069E-03 | RPL22L1 | RPL22L1 |
| A0A0D9RNC9;A0A( | A0A0D9RNC9 | IGF2BP3 | CHLSB | 8 | 1 | 1.069E-03 | IGF2BP3 | IGF2BP3 |
| A0A0D9RXV6 | A0A0D9RXV6 | MRPS5 | CHLSB | 8 | 1 | 1.069E-03 | MRPS5 | MRPS5 |
| A0A0D9S0E1 | A0A0D9S0E1 | DDX3Y | CHLSB | 8 | 1 | 1.069E-03 | DDX3Y | DDX3Y |
| A0A0D9SDL6;A0A0 | A0A0D9SDL6 | RPS17* | CHLSB | 8 | 1 | 1.069E-03 | RPS17* | RPS17 |
| ncbi|YP_009725305 | YP_009725305.1 | nsp9 | SARS2 | 8 | 1 | 1.069E-03 | NA | NA |
| A0A0D9QYJ1 | A0A0D9QYJ1 | SRSF2 | CHLSB | 12 | 2 | 1.437E-03 | SRSF2 | SRSF2 |
| A0A0D9SDL0 | A0A0D9SDL0 | PCBP1 | CHLSB | 12 | 2 | 1.437E-03 | PCBP1 | PCBP1 |
| A0A0D9QV93 | A0A0D9QV93 | RPS3 | CHLSB | 7 | 1 | 4.001E-03 | RPS3 | RPS3 |
| A0A0D9QYW2 | A0A0D9QYW2 | PA2G4* | CHLSB | 7 | 1 | 4.001E-03 | PA2G4* | PA2G4 |
| A0A0D9QZT6;A0A( | A0A0D9QZT6 | EIF4B | CHLSB | 7 | 1 | 4.001E-03 | EIF4B | EIF4B |
| A0A0D9R949 | A0A0D9R949 | PUF60 | CHLSB | 7 | 1 | 4.001E-03 | PUF60 | PUF60 |
| A0A0D9RDI7;A0A0I | A0A0D9RDI7 | RPL26 | CHLSB | 7 | 1 | 4.001E-03 | RPL26 | RPL26 |
| A0A0D9RDL0 | A0A0D9RDL0 | RPUSD3 | CHLSB | 7 | 1 | 4.001E-03 | RPUSD3 | RPUSD3 |
| A0A0D9RDR4;A0A( | A0A0D9RDR4 | GAPDH* | CHLSB | 7 | 1 | 4.001E-03 | NA | GAPDH |
| A0A0D9RHG5;A0A( | A0A0D9RHG5 | FAM98A* | CHLSB | 7 | 1 | 4.001E-03 | FAM98A* | FAM98A |

123

| Protein IDs | vervet.uniprot | gname | species | target | background | adj.p | vervet.gname | human.gname |
|---|---|---|---|---|---|---|---|---|
| A0A0D9RIV1 | A0A0D9RIV1 | RPL30 | CHLSB | 7 | 1 | 4.001E-03 | RPL30 | RPL30 |
| A0A0D9RY63 | A0A0D9RY63 | MBNL2 | CHLSB | 7 | 1 | 4.001E-03 | MBNL2 | MBNL2 |
| A0A0D9RZS5 | A0A0D9RZS5 | PSPC1 | CHLSB | 7 | 1 | 4.001E-03 | PSPC1 | PSPC1 |
| A0A0D9S618 | A0A0D9S618 | U2AF2 | CHLSB | 7 | 1 | 4.001E-03 | U2AF2 | U2AF2 |
| A0A0D9S7S6 | A0A0D9S7S6 | THRAP3 | CHLSB | 7 | 1 | 4.001E-03 | THRAP3 | THRAP3 |
| A0A0D9S913 | A0A0D9S913 | RPS27* | CHLSB | 7 | 1 | 4.001E-03 | RPS27* | RPS27 |
| A0A0D9SAG1;A0A( | A0A0D9SAG1 | RPL23A* | CHLSB | 7 | 1 | 4.001E-03 | RPL23A* | RPL23A |
| A0A0D9SBN8 | A0A0D9SBN8 | RPS9* | CHLSB | 7 | 1 | 4.001E-03 | RPS9* | RPS9 |
| A0A0D9SBZ3;A0A0 | A0A0D9SBZ3 | RPL6* | CHLSB | 7 | 1 | 4.001E-03 | RPL6* | RPL6 |
| A0A0D9SDR8;A0A( | A0A0D9SDR8 | RPL23* | CHLSB | 7 | 1 | 4.001E-03 | RPL23* | RPL23 |
| A0A0D9S9I8 | A0A0D9S9I8 | RPL13* | CHLSB | 14 | 3 | 6.550E-03 | RPL13* | RPL13 |
| A0A0D9RE27 | A0A0D9RE27 | PPP1CB | CHLSB | 17 | 4 | 8.061E-03 | PPP1CB | PPP1CB |
| A0A0D9R2G8 | A0A0D9R2G8 | GADD45GIP1 | CHLSB | 6 | 1 | 1.384E-02 | GADD45GIP1 | GADD45GIP1 |
| A0A0D9R300 | A0A0D9R300 | HNRNPUL2* | CHLSB | 6 | 1 | 1.384E-02 | NA | HNRNPUL2 |
| A0A0D9R4U0 | A0A0D9R4U0 | MRPL4 | CHLSB | 6 | 1 | 1.384E-02 | MRPL4 | MRPL4 |
| A0A0D9R508 | A0A0D9R508 | SHFL | CHLSB | 6 | 1 | 1.384E-02 | SHFL | SHFL |
| A0A0D9R7C2 | A0A0D9R7C2 | RSL1D1 | CHLSB | 6 | 1 | 1.384E-02 | RSL1D1 | RSL1D1 |
| A0A0D9R7L4 | A0A0D9R7L4 | RTCB | CHLSB | 6 | 1 | 1.384E-02 | RTCB | RTCB |
| A0A0D9RIK4 | A0A0D9RIK4 | QKI | CHLSB | 6 | 1 | 1.384E-02 | QKI | QKI |
| A0A0D9RNK1 | A0A0D9RNK1 | TRA2A | CHLSB | 6 | 1 | 1.384E-02 | TRA2A | TRA2A |
| A0A0D9S4J2 | A0A0D9S4J2 | SRSF9 | CHLSB | 6 | 1 | 1.384E-02 | SRSF9 | SRSF9 |
| A0A0D9S7Z5 | A0A0D9S7Z5 | PUM1 | CHLSB | 6 | 1 | 1.384E-02 | PUM1 | PUM1 |
| A0A0D9SA22;A0A0 | A0A0D9SA22 | RPSA | CHLSB | 6 | 1 | 1.384E-02 | RPSA | RPSA |
| A0A0D9SAF5 | A0A0D9SAF5 | HNRNPA1L2* | CHLSB | 6 | 1 | 1.384E-02 | HNRNPA1L2* | HNRNPA1L2 |
| A0A0D9SBP6 | A0A0D9SBP6 | MARS2 | CHLSB | 6 | 1 | 1.384E-02 | MARS2 | MARS2 |
| A0A0D9SDA4;A0A( | A0A0D9SDA4 | RBMX* | CHLSB | 6 | 1 | 1.384E-02 | NA | RBMXL2 |
| CON__P35908;COf | CON__P35908 | NA | NA | 6 | 1 | 1.384E-02 | NA | NA |
| A0A0D9RJU1 | A0A0D9RJU1 | VIM | CHLSB | 16 | 4 | 1.587E-02 | VIM | VIM |
| A0A0D9QWP9;A0A | A0A0D9QWP9 | RPL27A* | CHLSB | 18 | 5 | 3.073E-02 | RPL27A* | RPL27A |
| A0A0D9QUW2 | A0A0D9QUW2 | FBL | CHLSB | 5 | 1 | 4.233E-02 | FBL | FBL |
| A0A0D9QZK8 | A0A0D9QZK8 | PCBP2* | CHLSB | 5 | 1 | 4.233E-02 | PCBP2* | PCBP2 |
| A0A0D9R027 | A0A0D9R027 | POLDIP3 | CHLSB | 5 | 1 | 4.233E-02 | POLDIP3 | POLDIP3 |
| A0A0D9R082 | A0A0D9R082 | FUS | CHLSB | 5 | 1 | 4.233E-02 | FUS | FUS |
| A0A0D9R1Q0 | A0A0D9R1Q0 | TAF15 | CHLSB | 5 | 1 | 4.233E-02 | TAF15 | TAF15 |
| A0A0D9RBR0;A0A( | A0A0D9RBR0 | MRPL23* | CHLSB | 5 | 1 | 4.233E-02 | NA | MRPL23 |
| A0A0D9REC9;A0A( | A0A0D9REC9 | PGK2* | CHLSB | 5 | 1 | 4.233E-02 | PGK2* | PGK2 |
| A0A0D9RJV9;A0A0 | A0A0D9RJV9 | EZR | CHLSB | 5 | 1 | 4.233E-02 | EZR | EZR |
| A0A0D9RKT0 | A0A0D9RKT0 | SRSF5 | CHLSB | 5 | 1 | 4.233E-02 | SRSF5 | SRSF5 |
| A0A0D9RU64 | A0A0D9RU64 | EDF1 | CHLSB | 5 | 1 | 4.233E-02 | EDF1 | EDF1 |
| A0A0D9RZW6 | A0A0D9RZW6 | HSPB1 | CHLSB | 5 | 1 | 4.233E-02 | HSPB1 | HSPB1 |
| A0A0D9S0W9 | A0A0D9S0W9 | HNRNPUL1 | CHLSB | 5 | 1 | 4.233E-02 | HNRNPUL1 | HNRNPUL1 |
| A0A0D9S2I7 | A0A0D9S2I7 | CKAP4 | CHLSB | 5 | 1 | 4.233E-02 | CKAP4 | CKAP4 |
| A0A0D9SBE5;A0A( | A0A0D9SBE5 | RPL3* | CHLSB | 5 | 1 | 4.233E-02 | RPL3* | RPL3 |
| A0A0D9SDP4;A0A( | A0A0D9SDP4 | MRPL20* | CHLSB | 5 | 1 | 4.233E-02 | MRPL20* | MRPL20 |
| A0A0D9SDS3;A0A( | A0A0D9SDS3 | RPL11* | CHLSB | 5 | 1 | 4.233E-02 | RPL11* | RPL11 |
| ncbi|YP_009725304 | YP_009725304.1 | nsp8 | SARS2 | 5 | 1 | 4.233E-02 | NA | NA |
| A0A0D9S9U1;A0A0 | A0A0D9S9U1 | RPL7A* | CHLSB | 20 | 6 | 4.541E-02 | RPL7A* | RPL7A |

| Protein IDs | vervet.uniprot | gname | species | target | background | adj.p | vervet.gname | human.gname |
|---|---|---|---|---|---|---|---|---|
| ncbi\|YP_009724397 | YP_009724397.2 | N | SARS2 | 615 | 27 | 1.0E-377 | NA | NA |
| A0A0D9RQH8 | A0A0D9RQH8 | FUBP3 | CHLSB | 173 | 1 | 2.000E-250 | FUBP3 | FUBP3 |
| A0A0D9RXQ9 | A0A0D9RXQ9 | SYNCRIP | CHLSB | 159 | 1 | 1.248E-224 | SYNCRIP | SYNCRIP |
| A0A0D9R0F2 | A0A0D9R0F2 | HDLBP | CHLSB | 207 | 4 | 1.366E-193 | HDLBP | HDLBP |
| A0A0D9RJZ5 | A0A0D9RJZ5 | PTBP1 | CHLSB | 116 | 1 | 1.076E-148 | PTBP1 | PTBP1 |
| A0A0D9RHW7;A0A | A0A0D9RHW7 | PABPC1 | CHLSB | 114 | 1 | 2.203E-145 | PABPC1 | PABPC1 |
| A0A0D9R403 | A0A0D9R403 | FAM120A | CHLSB | 97 | 1 | 2.813E-117 | FAM120A | FAM120A |
| A0A0D9S679 | A0A0D9S679 | CSDE1 | CHLSB | 92 | 1 | 2.763E-109 | CSDE1 | CSDE1 |
| A0A0D9S7K7 | A0A0D9S7K7 | YBX1 | CHLSB | 90 | 1 | 3.782E-106 | YBX1 | YBX1 |
| A0A0D9RNX3;A0AC | A0A0D9RNX3 | HNRNPA2B1 | CHLSB | 88 | 1 | 5.017E-103 | NA | HNRNPA2B1 |
| A0A0D9RB84 | A0A0D9RB84 | KHSRP | CHLSB | 82 | 1 | 1.109E-93 | KHSRP | KHSRP |
| A0A0D9RFM7;A0AC | A0A0D9RFM7 | RPS2 | CHLSB | 81 | 1 | 3.301E-92 | RPS2 | RPS2 |
| A0A0D9RNG4 | A0A0D9RNG4 | IGF2BP2 | CHLSB | 81 | 1 | 3.301E-92 | IGF2BP2 | IGF2BP2 |
| A0A0D9R723;A0A0I | A0A0D9R723 | ELAVL1 | CHLSB | 74 | 1 | 1.439E-81 | ELAVL1 | ELAVL1 |
| A0A0D9R8L5;A0A0I | A0A0D9R8L5 | YBX3 | CHLSB | 73 | 1 | 4.267E-80 | YBX3 | YBX3 |
| A0A0D9RIR4 | A0A0D9RIR4 | PTBP3 | CHLSB | 68 | 1 | 1.052E-72 | PTBP3 | PTBP3 |
| A0A0D9RCS8 | A0A0D9RCS8 | SND1 | CHLSB | 66 | 1 | 8.321E-70 | SND1 | SND1 |
| A0A0D9R2V4 | A0A0D9R2V4 | NCL | CHLSB | 114 | 5 | 1.577E-69 | NCL | NCL |
| A0A0D9R7T1 | A0A0D9R7T1 | HNRNPH1 | CHLSB | 61 | 1 | 1.170E-62 | HNRNPH1 | HNRNPH1 |
| A0A0D9RVK4 | A0A0D9RVK4 | HNRNPC | CHLSB | 76 | 2 | 1.411E-62 | HNRNPC | HNRNPC |
| A0A0D9S6D8 | A0A0D9S6D8 | MOV10 | CHLSB | 56 | 1 | 1.112E-55 | MOV10 | MOV10 |
| A0A0D9REQ6 | A0A0D9REQ6 | G3BP1 | CHLSB | 54 | 1 | 6.056E-53 | G3BP1 | G3BP1 |
| A0A0D9R6L1 | A0A0D9R6L1 | HNRNPM | CHLSB | 53 | 1 | 1.291E-51 | HNRNPM | HNRNPM |
| A0A0D9RKW3 | A0A0D9RKW3 | MATR3* | CHLSB | 53 | 1 | 1.291E-51 | NA | MATR3 |
| A0A0D9R5A7;A0A0 | A0A0D9R5A7 | HNRNPK* | CHLSB | 52 | 1 | 2.828E-50 | HNRNPK* | HNRNPK |
| A0A0D9RPE9;A0A0 | A0A0D9RPE9 | HNRNPA3 | CHLSB | 51 | 1 | 6.092E-49 | HNRNPA3 | HNRNPA3 |
| A0A0D9QZF1;A0A0 | A0A0D9QZF1 | HNRNPA1 | CHLSB | 74 | 3 | 4.302E-48 | HNRNPA1 | HNRNPA1 |
| A0A0D9RNA2;A0AC | A0A0D9RNA2 | DDX3X | CHLSB | 49 | 1 | 2.679E-46 | DDX3X | DDX3X |
| A0A0D9R833 | A0A0D9R833 | ZC3HAV1 | CHLSB | 48 | 1 | 5.466E-45 | ZC3HAV1 | ZC3HAV1 |
| A0A0D9RL59 | A0A0D9RL59 | FXR1 | CHLSB | 47 | 1 | 1.094E-43 | FXR1 | FXR1 |
| A0A0D9RWW2;A0A | A0A0D9RWW2 | RBM47 | CHLSB | 46 | 1 | 2.147E-42 | RBM47 | RBM47 |
| A0A0D9QZ01 | A0A0D9QZ01 | UPF1 | CHLSB | 45 | 1 | 4.130E-41 | UPF1 | UPF1 |
| A0A0D9R1V0 | A0A0D9R1V0 | EIF3C EIF3S8 | CHLSB | 44 | 1 | 7.338E-40 | NA | EIF3C |
| A0A0D9R8Y5;A0A0 | A0A0D9R8Y5 | CNBP* | CHLSB | 44 | 1 | 7.338E-40 | CNBP | CNBP |
| A0A0D9S3H8;A0A0 | A0A0D9S3H8 | HNRNPAB* | CHLSB | 44 | 1 | 7.338E-40 | HNRNPAB* | HNRNPAB |
| A0A0D9R6W1 | A0A0D9R6W1 | PUM2 | CHLSB | 43 | 1 | 1.356E-38 | PUM2 | PUM2 |
| A0A0D9RA32 | A0A0D9RA32 | HNRNPU | CHLSB | 62 | 3 | 2.463E-36 | HNRNPU | HNRNPU |
| A0A0D9S1G6;A0A0 | A0A0D9S1G6 | RPS3A | CHLSB | 41 | 1 | 4.337E-36 | RPS3A | RPS3A |
| A0A0D9QXL4 | A0A0D9QXL4 | EIF3A EIF3S10 | CHLSB | 40 | 1 | 7.309E-35 | EIF3A | EIF3A |
| A0A0D9REI2 | A0A0D9REI2 | LARP1 | CHLSB | 40 | 1 | 7.309E-35 | LARP1 | LARP1 |
| A0A0D9S7P0 | A0A0D9S7P0 | PABPC4 | CHLSB | 38 | 1 | 1.989E-32 | PABPC4 | PABPC4 |
| A0A0D9S7U2 | A0A0D9S7U2 | SFPQ | CHLSB | 38 | 1 | 1.989E-32 | SFPQ | SFPQ |
| A0A0D9SDY5;A0A0 | A0A0D9SDY5 | RPL22 | CHLSB | 38 | 1 | 1.989E-32 | RPL22 | RPL22 |
| A0A0D9R787;A0A0I | A0A0D9R787 | RPL8 | CHLSB | 82 | 7 | 3.269E-30 | RPL8 | RPL8 |
| A0A0D9QVC3 | A0A0D9QVC3 | HNRNPL | CHLSB | 35 | 1 | 7.679E-29 | HNRNPL | HNRNPL |
| A0A0D9RNC9;A0AC | A0A0D9RNC9 | IGF2BP3 | CHLSB | 35 | 1 | 7.679E-29 | IGF2BP3 | IGF2BP3 |
| A0A0D9QXD5 | A0A0D9QXD5 | G3BP2 | CHLSB | 34 | 1 | 1.096E-27 | G3BP2 | G3BP2 |
| A0A0D9RGJ5 | A0A0D9RGJ5 | RPS14 | CHLSB | 34 | 1 | 1.096E-27 | RPS14 | RPS14 |
| A0A0D9S3T6;A0A0I | A0A0D9S3T6 | RPS11 | CHLSB | 34 | 1 | 1.096E-27 | RPS11 | RPS11 |
| A0A0D9R6B6 | A0A0D9R6B6 | EIF3D EIF3S7 | CHLSB | 33 | 1 | 1.556E-26 | EIF3D | EIF3D |
| A0A0D9RTU4 | A0A0D9RTU4 | MAP4* | CHLSB | 33 | 1 | 1.556E-26 | MAP4* | MAP4 |
| A0A0D9RHP8;A0A0 | A0A0D9RHP8 | MBNL1 | CHLSB | 32 | 1 | 2.150E-25 | MBNL1 | MBNL1 |
| A0A0D9S897 | A0A0D9S897 | HNRNPR | CHLSB | 32 | 1 | 2.150E-25 | HNRNPR | HNRNPR |
| A0A0D9QWR4 | A0A0D9QWR4 | HNRNPD | CHLSB | 31 | 1 | 2.888E-24 | HNRNPD | HNRNPD |
| A0A0D9S020 | A0A0D9S020 | EIF4H | CHLSB | 31 | 1 | 2.888E-24 | EIF4H | EIF4H |
| A0A0D9S713 | A0A0D9S713 | SERBP1 | CHLSB | 30 | 1 | 3.766E-23 | SERBP1 | SERBP1 |
| A0A0D9S8N2;A0A0 | A0A0D9S8N2 | TARDBP* | CHLSB | 30 | 1 | 3.766E-23 | NA | TARDBP |
| A0A0D9QZI9;A0A0[ | A0A0D9QZI9 | RPL18A | CHLSB | 29 | 1 | 4.846E-22 | RPL18A | RPL18A |
| A0A0D9SDL0 | A0A0D9SDL0 | PCBP1 | CHLSB | 38 | 2 | 7.935E-22 | PCBP1 | PCBP1 |
| A0A0D9R1V9 | A0A0D9R1V9 | ATXN2L | CHLSB | 28 | 1 | 5.744E-21 | ATXN2L | ATXN2L |
| A0A0D9RFF7;A0A0 | A0A0D9RFF7 | RPS4X* | CHLSB | 28 | 1 | 5.744E-21 | RPS4X | RPS4X |
| A0A0D9RI90 | A0A0D9RI90 | RALY | CHLSB | 28 | 1 | 5.744E-21 | RALY | RALY |
| A0A0D9RQ30 | A0A0D9RQ30 | RPS20* | CHLSB | 27 | 1 | 6.712E-20 | RPS20 | RPS20 |
| A0A0D9S0E1 | A0A0D9S0E1 | DDX3Y | CHLSB | 27 | 1 | 6.712E-20 | DDX3Y | DDX3Y |
| A0A0D9S6D9 | A0A0D9S6D9 | RPS5 | CHLSB | 27 | 1 | 6.712E-20 | RPS5 | RPS5 |
| A0A0D9QXH3 | A0A0D9QXH3 | TIAL1 | CHLSB | 26 | 1 | 7.819E-19 | TIAL1 | TIAL1 |
| A0A0D9QWR3 | A0A0D9QWR3 | HNRNPDL | CHLSB | 25 | 1 | 8.658E-18 | HNRNPDL | HNRNPDL |
| A0A0D9R0F6;A0A0 | A0A0D9R0F6 | CELF1 | CHLSB | 25 | 1 | 8.658E-18 | CELF1 | CELF1 |
| A0A0D9S7H2 | A0A0D9S7H2 | RPS8 | CHLSB | 51 | 5 | 4.797E-17 | RPS8 | RPS8 |
| A0A0D9QVI6 | A0A0D9QVI6 | DDX5 | CHLSB | 24 | 1 | 8.741E-17 | DDX5 | DDX5 |
| A0A0D9QZT6;A0A0 | A0A0D9QZT6 | EIF4B | CHLSB | 24 | 1 | 8.741E-17 | EIF4B | EIF4B |
| A0A0D9R508 | A0A0D9R508 | SHFL | CHLSB | 24 | 1 | 8.741E-17 | SHFL | SHFL |
| A0A0D9RAL5;A0A0 | A0A0D9RAL5 | RPS6 | CHLSB | 24 | 1 | 8.741E-17 | RPS6 | RPS6 |
| ncbi\|YP_009725297 | YP_009725297.1 | nsp1 | SARS2 | 24 | 1 | 8.741E-17 | NA | NA |
| A0A0D9RG49 | A0A0D9RG49 | FXR2 | CHLSB | 23 | 1 | 9.114E-16 | FXR2 | FXR2 |
| A0A0D9RWG7 | A0A0D9RWG7 | RPL14* | CHLSB | 22 | 1 | 9.007E-15 | RPL14* | RPL14 |
| A0A0D9S3L7 | A0A0D9S3L7 | ATXN2 | CHLSB | 22 | 1 | 9.007E-15 | ATXN2 | ATXN2 |
| A0A0D9RLC1 | A0A0D9RLC1 | RBM3 | CHLSB | 21 | 1 | 8.432E-14 | RBM3 | RBM3 |
| A0A0D9S6X7 | A0A0D9S6X7 | FUBP1 | CHLSB | 21 | 1 | 8.432E-14 | FUBP1 | FUBP1 |
| A0A0D9SBN8 | A0A0D9SBN8 | RPS9* | CHLSB | 21 | 1 | 8.432E-14 | RPS9* | RPS9 |
| A0A0D9QZW0 | A0A0D9QZW0 | TRIM25 | CHLSB | 20 | 1 | 7.386E-13 | TRIM25 | TRIM25 |
| A0A0D9RD33 | A0A0D9RD33 | SRSF3 | CHLSB | 20 | 1 | 7.386E-13 | SRSF3 | SRSF3 |
| A0A0D9RG28 | A0A0D9RG28 | NONO* | CHLSB | 20 | 1 | 7.386E-13 | NONO* | NONO |

| Protein IDs | vervet.uniprot | gname | species | target | background | adj.p | vervet.gname | human.gname |
|---|---|---|---|---|---|---|---|---|
| A0A0D9S7Z5 | A0A0D9S7Z5 | PUM1 | CHLSB | 20 | 1 | 7.386E-13 | PUM1 | PUM1 |
| A0A0D9SC26;A0A0 | A0A0D9SC26 | RPL17* | CHLSB | 20 | 1 | 7.386E-13 | RPL17* | RPL17 |
| A0A0D9RKP7 | A0A0D9RKP7 | LRPPRC* | CHLSB | 44 | 5 | 8.228E-13 | LRPPRC* | LRPPRC |
| A0A0D9R7K5 | A0A0D9R7K5 | SQSTM1 | CHLSB | 19 | 1 | 6.340E-12 | SQSTM1 | SQSTM1 |
| A0A0D9S9H0 | A0A0D9S9H0 | PURA | CHLSB | 19 | 1 | 6.340E-12 | PURA | PURA |
| A0A0D9R4K7 | A0A0D9R4K7 | FMR1 | CHLSB | 18 | 1 | 5.250E-11 | FMR1 | FMR1 |
| A0A0D9RMM1 | A0A0D9RMM1 | EIF4G1 | CHLSB | 18 | 1 | 5.250E-11 | EIF4G1 | EIF4G1 |
| A0A0D9QV93 | A0A0D9QV93 | RPS3 | CHLSB | 17 | 1 | 4.004E-10 | RPS3 | RPS3 |
| A0A0D9R5E6 | A0A0D9R5E6 | HNRNPH3 | CHLSB | 17 | 1 | 4.004E-10 | HNRNPH3 | HNRNPH3 |
| A0A0D9R7E8 | A0A0D9R7E8 | PARP12 | CHLSB | 17 | 1 | 4.004E-10 | PARP12 | PARP12 |
| A0A0D9RBD8;A0A0 | A0A0D9RBD8 | NPM1 | CHLSB | 17 | 1 | 4.004E-10 | NPM1 | NPM1 |
| A0A0D9S5G4 | A0A0D9S5G4 | UBAP2L | CHLSB | 17 | 1 | 4.004E-10 | UBAP2L | UBAP2L |
| A0A0D9R1R4 | A0A0D9R1R4 | RPL24 | CHLSB | 16 | 1 | 2.962E-09 | RPL24 | RPL24 |
| A0A0D9RAB6 | A0A0D9RAB6 | RPS24* | CHLSB | 16 | 1 | 2.962E-09 | RPS24* | RPS24 |
| A0A0D9RIM7 | A0A0D9RIM7 | SRSF7 | CHLSB | 16 | 1 | 2.962E-09 | SRSF7 | SRSF7 |
| A0A0D9R2U6 | A0A0D9R2U6 | DDX17 | CHLSB | 15 | 1 | 1.975E-08 | DDX17 | DDX17 |
| A0A0D9R3S5 | A0A0D9R3S5 | ILF3 | CHLSB | 15 | 1 | 1.975E-08 | ILF3 | ILF3 |
| A0A0D9R790 | A0A0D9R790 | MRPL11 | CHLSB | 15 | 1 | 1.975E-08 | MRPL11 | MRPL11 |
| A0A0D9RIJ2 | A0A0D9RIJ2 | CIRBP | CHLSB | 15 | 1 | 1.975E-08 | CIRBP | CIRBP |
| A0A0D9RT27 | A0A0D9RT27 | RBMS1 | CHLSB | 15 | 1 | 1.975E-08 | RBMS1 | RBMS1 |
| A0A0D9SA22;A0A0 | A0A0D9SA22 | RPSA | CHLSB | 15 | 1 | 1.975E-08 | RPSA | RPSA |
| A0A0D9SE18 | A0A0D9SE18 | RPL3* | CHLSB | 15 | 1 | 1.975E-08 | RPL3* | RPL3 |
| A0A0D9SE44 | A0A0D9SE44 | RPS26* | CHLSB | 15 | 1 | 1.975E-08 | RPS26* | RPS26 |
| A0A0D9QYF8 | A0A0D9QYF8 | RBMS2 | CHLSB | 14 | 1 | 1.282E-07 | RBMS2 | RBMS2 |
| A0A0D9R7D0 | A0A0D9R7D0 | ZC3H7A | CHLSB | 14 | 1 | 1.282E-07 | ZC3H7A | ZC3H7A |
| A0A0D9R7L4 | A0A0D9R7L4 | RTCB | CHLSB | 14 | 1 | 1.282E-07 | RTCB | RTCB |
| A0A0D9RIY1 | A0A0D9RIY1 | FAM120C | CHLSB | 14 | 1 | 1.282E-07 | FAM120C | FAM120C |
| A0A0D9SA63 | A0A0D9SA63 | RPL35* | CHLSB | 14 | 1 | 1.282E-07 | RPL35* | RPL35 |
| A0A0D9RN36;A0A0 | A0A0D9RN36 | RPL29* | CHLSB | 33 | 5 | 4.345E-07 | NA | RPL29 |
| A0A0D9RMB4;A0A0 | A0A0D9RMB4 | RPL7* | CHLSB | 19 | 2 | 5.495E-07 | RPL7* | RPL7 |
| A0A0D9RDB1 | A0A0D9RDB1 | MYEF2 | CHLSB | 13 | 1 | 7.628E-07 | MYEF2 | MYEF2 |
| A0A0D9RQZ1;A0A0 | A0A0D9RQZ1 | RPS23 | CHLSB | 13 | 1 | 7.628E-07 | RPS23 | RPS23 |
| A0A0D9S4M9 | A0A0D9S4M9 | ALYREF | CHLSB | 13 | 1 | 7.628E-07 | ALYREF | ALYREF |
| A0A0D9S7Y1;A0A0 | A0A0D9S7Y1 | KHDRBS1 | CHLSB | 13 | 1 | 7.628E-07 | KHDRBS1 | KHDRBS1 |
| A0A0D9S8U1 | A0A0D9S8U1 | LRRC47 | CHLSB | 13 | 1 | 7.628E-07 | LRRC47 | LRRC47 |
| A0A0D9SCE0 | A0A0D9SCE0 | RPL21* | CHLSB | 13 | 1 | 7.628E-07 | RPL21* | RPL21 |
| A0A0D9RDR4;A0A0 | A0A0D9RDR4 | GAPDH* | CHLSB | 12 | 1 | 4.402E-06 | NA | GAPDH |
| A0A0D9RJV9;A0A0 | A0A0D9RJV9 | EZR | CHLSB | 12 | 1 | 4.402E-06 | EZR | EZR |
| A0A0D9RTV5 | A0A0D9RTV5 | DHX30 | CHLSB | 12 | 1 | 4.402E-06 | DHX30 | DHX30 |
| A0A0D9RXB8 | A0A0D9RXB8 | PCBP2* | CHLSB | 12 | 1 | 4.402E-06 | PCBP2* | PCBP2 |
| A0A0D9R4M7 | A0A0D9R4M7 | BICC1 | CHLSB | 11 | 1 | 2.225E-05 | BICC1 | BICC1 |
| A0A0D9RC52 | A0A0D9RC52 | SAFB | CHLSB | 11 | 1 | 2.225E-05 | SAFB | SAFB |
| A0A0D9RCA8 | A0A0D9RCA8 | UBAP2 | CHLSB | 11 | 1 | 2.225E-05 | UBAP2 | UBAP2 |
| A0A0D9REA3 | A0A0D9REA3 | MRPL13 | CHLSB | 11 | 1 | 2.225E-05 | MRPL13 | MRPL13 |
| A0A0D9RG34 | A0A0D9RG34 | NONO* | CHLSB | 11 | 1 | 2.225E-05 | NONO* | NONO |
| A0A0D9RGN3 | A0A0D9RGN3 | MRPL2 | CHLSB | 11 | 1 | 2.225E-05 | MRPL2 | MRPL2 |
| A0A0D9RI73 | A0A0D9RI73 | DAZAP1 | CHLSB | 11 | 1 | 2.225E-05 | DAZAP1 | DAZAP1 |
| A0A0D9RJE1 | A0A0D9RJE1 | DHX9 | CHLSB | 11 | 1 | 2.225E-05 | DHX9 | DHX9 |
| A0A0D9RRJ6 | A0A0D9RRJ6 | TIA1 | CHLSB | 11 | 1 | 2.225E-05 | TIA1 | TIA1 |
| A0A0D9RU12 | A0A0D9RU12 | PABPN1 | CHLSB | 11 | 1 | 2.225E-05 | PABPN1 | PABPN1 |
| A0A0D9S9I2;A0A0D | A0A0D9S9I2 | RPS7* | CHLSB | 11 | 1 | 2.225E-05 | RPS7* | RPS7 |
| A0A0D9SCL8;A0A0 | A0A0D9SCL8 | HIST1H2BB | CHLSB | 11 | 1 | 2.225E-05 | H2BC3 | H2BC3 |
| A0A0D9RE27 | A0A0D9RE27 | PPP1CB | CHLSB | 24 | 4 | 6.088E-05 | PPP1CB | PPP1CB |
| A0A0D9QZA0 | A0A0D9QZA0 | CAPRIN1 | CHLSB | 10 | 1 | 1.087E-04 | CAPRIN1 | CAPRIN1 |
| A0A0D9R0Y0 | A0A0D9R0Y0 | LARP4 | CHLSB | 10 | 1 | 1.087E-04 | LARP4 | LARP4 |
| A0A0D9R3U8 | A0A0D9R3U8 | NUFIP2 | CHLSB | 10 | 1 | 1.087E-04 | NUFIP2 | NUFIP2 |
| A0A0D9R4W4;A0A0 | A0A0D9R4W4 | EIF3G EIF3S4 | CHLSB | 10 | 1 | 1.087E-04 | NA | EIF3G |
| A0A0D9R7L9 | A0A0D9R7L9 | RBM14* | CHLSB | 10 | 1 | 1.087E-04 | RBM14* | RBM14 |
| A0A0D9RZS5 | A0A0D9RZS5 | PSPC1 | CHLSB | 10 | 1 | 1.087E-04 | PSPC1 | PSPC1 |
| A0A0D9S9I8 | A0A0D9S9I8 | RPL13* | CHLSB | 19 | 3 | 1.558E-04 | RPL13* | RPL13 |
| A0A0D9S8Q7;A0A0 | A0A0D9S8Q7 | ENO1 | CHLSB | 22 | 4 | 3.992E-04 | ENO1 | ENO1 |
| A0A0D9REP7 | A0A0D9REP7 | EEF2 | CHLSB | 18 | 3 | 4.413E-04 | EEF2 | EEF2 |
| A0A0D9R014 | A0A0D9R014 | SRSF1* | CHLSB | 9 | 1 | 4.794E-04 | SRSF1* | SRSF1 |
| A0A0D9R0F3 | A0A0D9R0F3 | XRCC6 | CHLSB | 9 | 1 | 4.794E-04 | XRCC6 | XRCC6 |
| A0A0D9RDI7;A0A0I | A0A0D9RDI7 | RPL26 | CHLSB | 9 | 1 | 4.794E-04 | RPL26 | RPL26 |
| A0A0D9RIV1 | A0A0D9RIV1 | RPL30 | CHLSB | 9 | 1 | 4.794E-04 | RPL30 | RPL30 |
| A0A0D9S6R4;A0A0 | A0A0D9S6R4 | RPL5* | CHLSB | 9 | 1 | 4.794E-04 | RPL5* | RPL5 |
| A0A0D9S9G6 | A0A0D9S9G6 | HNRNPA0 | CHLSB | 9 | 1 | 4.794E-04 | HNRNPA0 | HNRNPA0 |
| A0A0D9SCR9 | A0A0D9SCR9 | RPL31* | CHLSB | 9 | 1 | 4.794E-04 | RPL31* | RPL31 |
| A0A0D9QX96 | A0A0D9QX96 | LSM14A* | CHLSB | 8 | 1 | 1.893E-03 | LSM14A* | LSM14A |
| A0A0D9QXG7 | A0A0D9QXG7 | MRPS7 | CHLSB | 8 | 1 | 1.893E-03 | MRPS7 | MRPS7 |
| A0A0D9QXK1;A0A0 | A0A0D9QXK1 | RPS13 | CHLSB | 8 | 1 | 1.893E-03 | RPS13 | RPS13 |
| A0A0D9R1Q0 | A0A0D9R1Q0 | TAF15 | CHLSB | 8 | 1 | 1.893E-03 | TAF15 | TAF15 |
| A0A0D9RGD1;A0A0 | A0A0D9RGD1 | EIF4A1 | CHLSB | 8 | 1 | 1.893E-03 | EIF4A1 | EIF4A1 |
| A0A0D9RHG5;A0A0 | A0A0D9RHG5 | FAM98A* | CHLSB | 8 | 1 | 1.893E-03 | FAM98A* | FAM98A |
| A0A0D9RNI1 | A0A0D9RNI1 | TRA2B | CHLSB | 8 | 1 | 1.893E-03 | TRA2B | TRA2B |
| A0A0D9RU64 | A0A0D9RU64 | EDF1 | CHLSB | 8 | 1 | 1.893E-03 | EDF1 | EDF1 |
| A0A0D9RV44 | A0A0D9RV44 | HELZ2 | CHLSB | 8 | 1 | 1.893E-03 | HELZ2 | HELZ2 |
| A0A0D9SAB7 | A0A0D9SAB7 | RPL5* | CHLSB | 8 | 1 | 1.893E-03 | RPL5* | RPL5 |
| A0A0D9SAF5 | A0A0D9SAF5 | HNRNPA1L2* | CHLSB | 8 | 1 | 1.893E-03 | HNRNPA1L2* | HNRNPA1L2 |
| A0A0D9SBZ3;A0A0 | A0A0D9SBZ3 | RPL6* | CHLSB | 8 | 1 | 1.893E-03 | RPL6* | RPL6 |
| A0A0D9SDL6;A0A0 | A0A0D9SDL6 | RPS17* | CHLSB | 8 | 1 | 1.893E-03 | RPS17* | RPS17 |
| ncbi\|YP_009724390 | YP_009724390.1 | S | SARS2 | 8 | 1 | 1.893E-03 | NA | NA |

| Protein IDs | vervet.uniprot | gname | species | target | background | adj.p | vervet.gname | human.gname |
|---|---|---|---|---|---|---|---|---|
| A0A0D9QWP9;A0A | A0A0D9QWP9 | RPL27A* | CHLSB | 23 | 5 | 3.050E-03 | RPL27A | RPL27A |
| A0A0D9QZK8 | A0A0D9QZK8 | PCBP2* | CHLSB | 7 | 1 | 6.820E-03 | PCBP2* | PCBP2 |
| A0A0D9QZS8 | A0A0D9QZS8 | MRPL43 | CHLSB | 7 | 1 | 6.820E-03 | MRPL43 | MRPL43 |
| A0A0D9R082 | A0A0D9R082 | FUS | CHLSB | 7 | 1 | 6.820E-03 | FUS | FUS |
| A0A0D9R5N6 | A0A0D9R5N6 | FAU | CHLSB | 7 | 1 | 6.820E-03 | FAU | FAU |
| A0A0D9R6X7 | A0A0D9R6X7 | SSBP1 | CHLSB | 7 | 1 | 6.820E-03 | SSBP1 | SSBP1 |
| A0A0D9RBS5 | A0A0D9RBS5 | EWSR1 | CHLSB | 7 | 1 | 6.820E-03 | EWSR1 | EWSR1 |
| A0A0D9RI76 | A0A0D9RI76 | MRPS18A | CHLSB | 7 | 1 | 6.820E-03 | MRPS18A | MRPS18A |
| A0A0D9RJV6 | A0A0D9RJV6 | RPL22L1 | CHLSB | 7 | 1 | 6.820E-03 | RPL22L1 | RPL22L1 |
| A0A0D9RNK1 | A0A0D9RNK1 | TRA2A | CHLSB | 7 | 1 | 6.820E-03 | TRA2A | TRA2A |
| A0A0D9RY63 | A0A0D9RY63 | MBNL2 | CHLSB | 7 | 1 | 6.820E-03 | MBNL2 | MBNL2 |
| A0A0D9SBE5;A0A0 | A0A0D9SBE5 | RPL3* | CHLSB | 7 | 1 | 6.820E-03 | RPL3* | RPL3 |
| ncbi|YP_009725305 | YP_009725305.1 | nsp9 | SARS2 | 7 | 1 | 6.820E-03 | NA | NA |
| ncbi|YP_009725307 | YP_009725307.1 | nsp12 | SARS2 | 7 | 1 | 6.820E-03 | NA | NA |
| A0A0D9QYJ1 | A0A0D9QYJ1 | SRSF2 | CHLSB | 11 | 2 | 7.984E-03 | SRSF2 | SRSF2 |
| A0A0D9S9U1;A0A0 | A0A0D9S9U1 | RPL7A* | CHLSB | 24 | 6 | 1.346E-02 | RPL7A* | RPL7A |
| A0A0D9RJU1 | A0A0D9RJU1 | VIM | CHLSB | 17 | 4 | 1.921E-02 | VIM | VIM |
| A0A0D9QZB7 | A0A0D9QZB7 | MRPL27 | CHLSB | 6 | 1 | 2.204E-02 | MRPL27 | MRPL27 |
| A0A0D9R4I5 | A0A0D9R4I5 | SF1 | CHLSB | 6 | 1 | 2.204E-02 | SF1 | SF1 |
| A0A0D9RAJ1 | A0A0D9RAJ1 | CRIP2 | CHLSB | 6 | 1 | 2.204E-02 | CRIP2 | CRIP2 |
| A0A0D9RC61 | A0A0D9RC61 | SAFB2 | CHLSB | 6 | 1 | 2.204E-02 | SAFB2 | SAFB2 |
| A0A0D9RIK4 | A0A0D9RIK4 | QKI | CHLSB | 6 | 1 | 2.204E-02 | QKI | QKI |
| A0A0D9RNQ9 | A0A0D9RNQ9 | LARP4B | CHLSB | 6 | 1 | 2.204E-02 | LARP4B | LARP4B |
| A0A0D9RVN0;A0A0 | A0A0D9RVN0 | TUBB4B | CHLSB | 6 | 1 | 2.204E-02 | TUBB4B | TUBB4B |
| A0A0D9S2A5 | A0A0D9S2A5 | ACLY | CHLSB | 6 | 1 | 2.204E-02 | ACLY | ACLY |
| A0A0D9S618 | A0A0D9S618 | U2AF2 | CHLSB | 6 | 1 | 2.204E-02 | U2AF2 | U2AF2 |
| A0A0D9S7S6 | A0A0D9S7S6 | THRAP3 | CHLSB | 6 | 1 | 2.204E-02 | THRAP3 | THRAP3 |
| A0A0D9S913 | A0A0D9S913 | RPS27* | CHLSB | 6 | 1 | 2.204E-02 | RPS27* | RPS27 |
| A0A0D9SAS2;A0A0 | A0A0D9SAS2 | RPL28* | CHLSB | 6 | 1 | 2.204E-02 | RPL28* | RPL28 |
| ncbi|YP_009724393 | YP_009724393.1 | M | SARS2 | 26 | 7 | 2.370E-02 | NA | NA |
| A0A0D9SCL0;A0A0 | A0A0D9SCL0 | HIST1H3A | CHLSB | 13 | 3 | 2.926E-02 | HIST1H3A | H3C1 |
| A0A0D9S3C9;A0A0 | A0A0D9S3C9 | RPL19 | CHLSB | 19 | 5 | 3.845E-02 | RPL19 | RPL19 |
| A0A0D9R6E6 | A0A0D9R6E6 | MYH9 | CHLSB | 9 | 2 | 4.648E-02 | MYH9 | MYH9 |
| A0A0D9RL86 | A0A0D9RL86 | RPL4* | CHLSB | 30 | 9 | 5.835E-02 | RPL4* | RPL4 |
| A0A0D9QYW2 | A0A0D9QYW2 | PA2G4* | CHLSB | 5 | 1 | 6.113E-02 | PA2G4* | PA2G4 |
| A0A0D9R1C6 | A0A0D9R1C6 | SPATS2 | CHLSB | 5 | 1 | 6.113E-02 | SPATS2 | SPATS2 |
| A0A0D9R300 | A0A0D9R300 | HNRNPUL2* | CHLSB | 5 | 1 | 6.113E-02 | NA | HNRNPUL2 |
| A0A0D9REC9;A0A0 | A0A0D9REC9 | PGK2* | CHLSB | 5 | 1 | 6.113E-02 | PGK2* | PGK2 |
| A0A0D9REE8 | A0A0D9REE8 | GEMIN5 | CHLSB | 5 | 1 | 6.113E-02 | GEMIN5 | GEMIN5 |
| A0A0D9RH50 | A0A0D9RH50 | SLIRP | CHLSB | 5 | 1 | 6.113E-02 | SLIRP | SLIRP |
| A0A0D9RI58 | A0A0D9RI58 | RPS15 | CHLSB | 5 | 1 | 6.113E-02 | RPS15 | RPS15 |
| A0A0D9RK34 | A0A0D9RK34 | FNDC3B | CHLSB | 5 | 1 | 6.113E-02 | FNDC3B | FNDC3B |
| A0A0D9RNH2;A0A0 | A0A0D9RNH2 | SRSF6 | CHLSB | 5 | 1 | 6.113E-02 | SRSF6 | SRSF6 |
| A0A0D9RTH8 | A0A0D9RTH8 | RBPMS | CHLSB | 5 | 1 | 6.113E-02 | RBPMS | RBPMS |
| A0A0D9S4I2 | A0A0D9S4I2 | MSI1 | CHLSB | 5 | 1 | 6.113E-02 | MSI1 | MSI1 |
| A0A0D9S5N0 | A0A0D9S5N0 | CHTOP | CHLSB | 5 | 1 | 6.113E-02 | CHTOP | CHTOP |
| A0A0D9S8B7 | A0A0D9S8B7 | EIF4G3 | CHLSB | 5 | 1 | 6.113E-02 | EIF4G3 | EIF4G3 |
| A0A0D9SC51 | A0A0D9SC51 | RPL32 | CHLSB | 5 | 1 | 6.113E-02 | RPL32 | RPL32 |
| A0A0D9SCC6;A0A0 | A0A0D9SCC6 | ILF2* | CHLSB | 5 | 1 | 6.113E-02 | ILF2* | ILF2 |
| A0A0D9SDR8;A0A0 | A0A0D9SDR8 | RPL23* | CHLSB | 5 | 1 | 6.113E-02 | RPL23* | RPL23 |
| A0A0D9SE08 | A0A0D9SE08 | PURB | CHLSB | 5 | 1 | 6.113E-02 | PURB | PURB |
| REV__A0A0D9RA0 | REV__A0A0D9RA0 | NA | NA | 5 | 1 | 6.113E-02 | NA | NA |
| A0A0D9RT22;A0A0 | A0A0D9RT22 | PPIA | CHLSB | 32 | 10 | 7.512E-02 | PPIA | PPIA |
| A0A0D9RKH2 | A0A0D9RKH2 | PFN1 | CHLSB | 8 | 2 | 9.407E-02 | PFN1 | PFN1 |

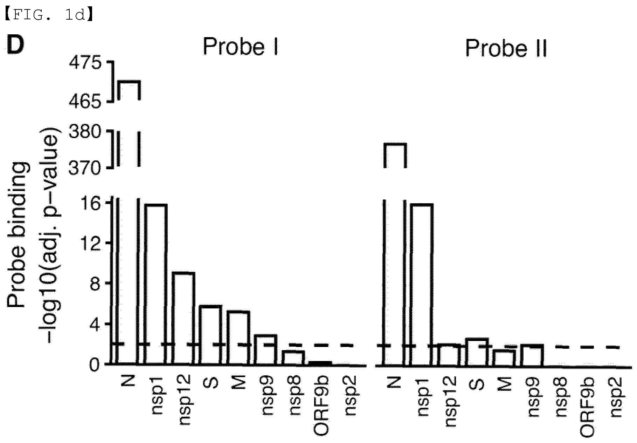

As for viral proteins, the N protein was the most strongly enriched one, as expected (FIG. 1d). The nsp1 protein was also statistically enriched in both Probe I and Probe II experiments.

Nsp12, S, M, and nsp9 were detected more with Probe I than with Probe II. Coronavirus nsp9 is a single-strand RNA binding protein (Egloff et al., 2004; Sutton et al., 2004) essential for viral replication (Miknis et al., 2009). Nsp1 is one of the major virulence factors that suppresses host translation by binding to the 40S ribosomal subunit (Thoms et al., 2020). While nsp1 is mostly studied in the context of host gene expression (Narayanan et al., 2008), our result hints at the direct role of nsp1 on the transcripts of SARS-CoV-2.

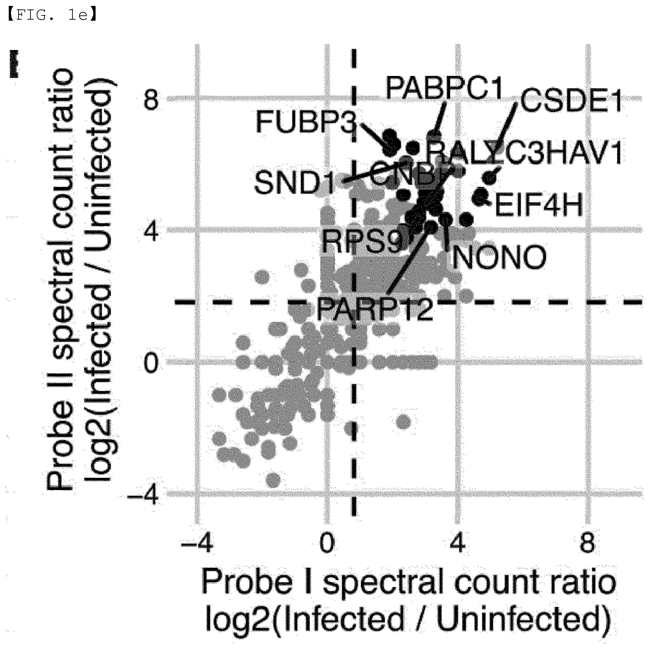

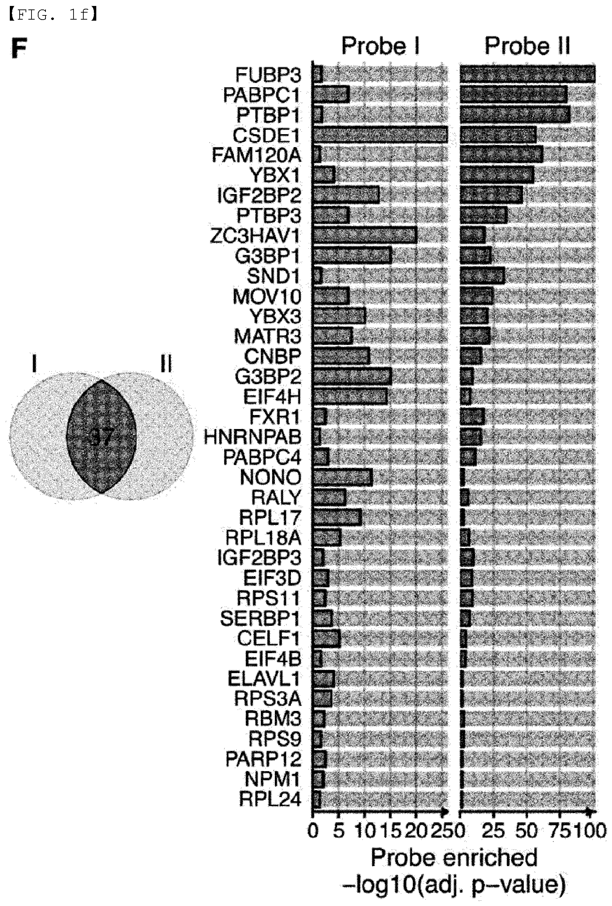

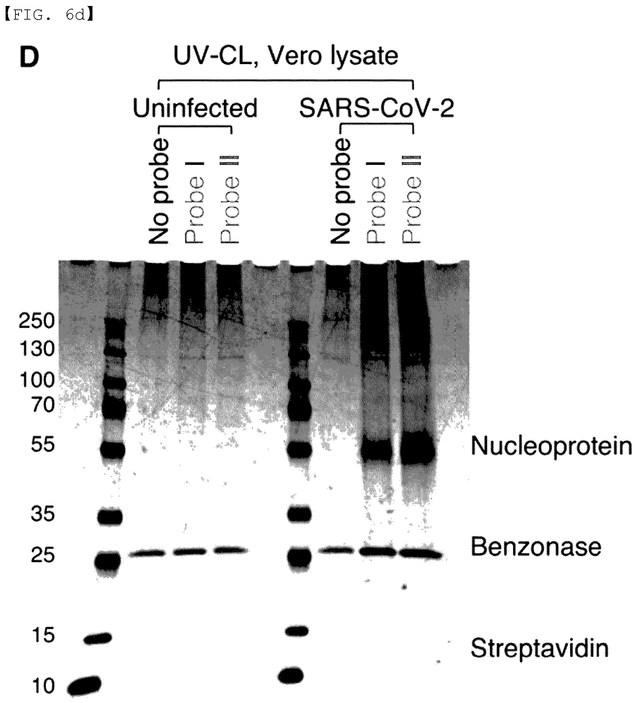

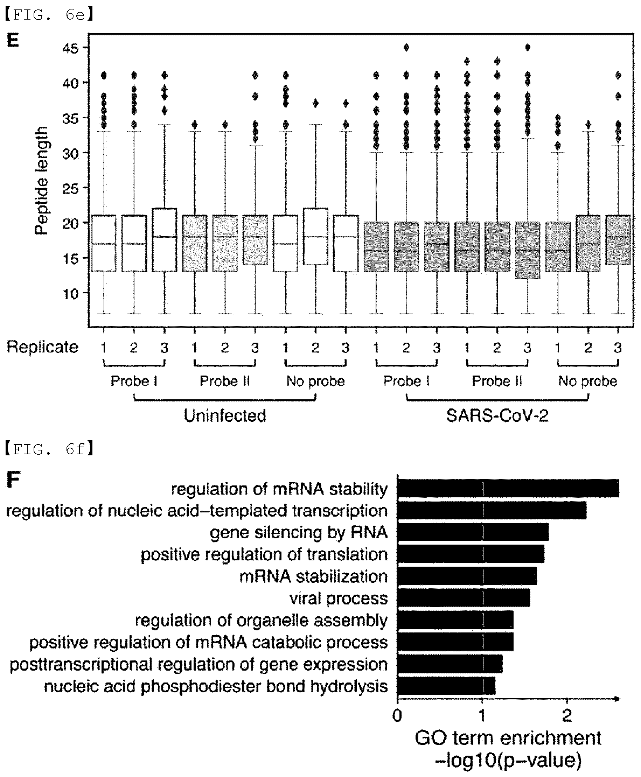

To delineate the host proteins that are enriched in the SARS-CoV-2 RNP complex, we employed an additional negative control experiment with uninfected cells (FIG. 1e). In effect, this control provides a conservative background of host proteins as shown by silver staining (FIG. 6d). Distributions of peptide length were consistent across technical replicates (FIG. 6e), demonstrating the robustness of the"on-bead" digestion step. Spectral count analysis against the "uninfected probe control" resulted in 74 and 72 proteins that are enriched in the infected samples with Probe I and Probe II, respectively (FDR < 5%, FIGs. 1f-h). In combination, we define these 109 proteins as the "SARS-CoV-2 RNA interactome." 37 host proteins such as CSDE1 (Unr), EIF4H, FUBP3, G3BP2, PABPC1, ZC3HAV1 were enriched in both the Probe I and Probe II RNP capture experiments on infected cells, thus identifying a robust set of the "core SARS-CoV-2 RNA interactome." Gene ontology (GO) term enrichment analysis revealed that these host factors are involved in RNA stability control, mRNA function, and viral process (FIG. 6f) .

## 3. Conservation of betacoronavirus RNP complexes

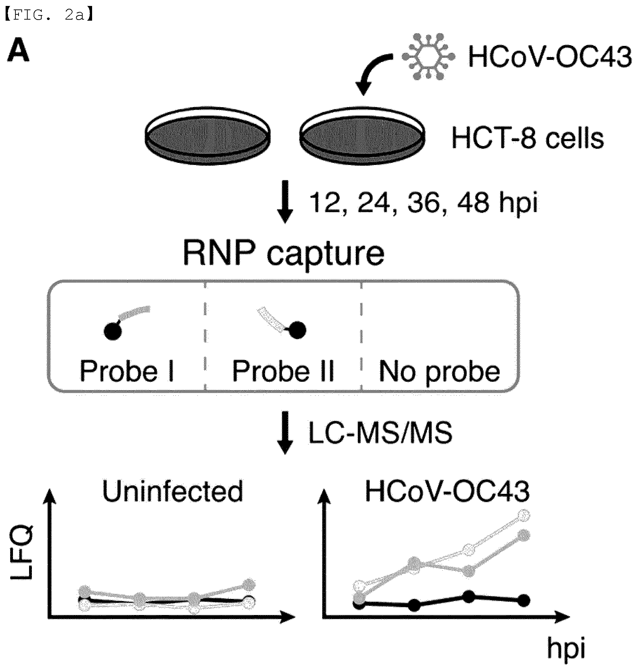

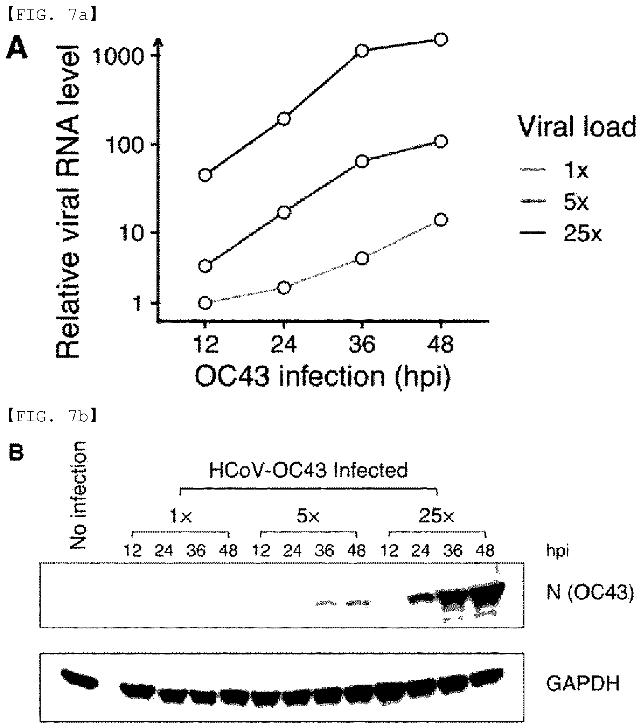

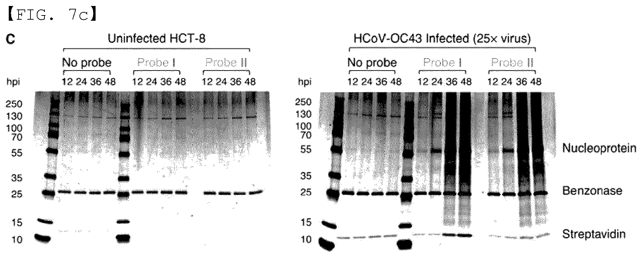

To investigate the evolutionary conservation of the RNA-protein interactions in coronaviruses, we conducted RNP capture on HCoV-OC43 that belongs to the lineage A of genus betacoronavirus. HCoV-OC43 shows 54.2% nucleotide homology to SARS-CoV-2 which belongs to lineage B. We profiled the HCoV-OC43 RNP complexes at multiple time points: 12, 24, 36, and 48 hours post-infection (FIG. 2a, 7a and 7b). Two antisense probe sets (i.e. OC43 Probe I and OC43 Probe II) were designed in a similar manner as in SARS-CoV-2 experiments. OC43 Probe I hybridizes only with the gRNA of HCoV-OC43, while Probe II captures both gRNA and sgRNA molecules. As negative controls, the no-probe control on infected cells and the probe control on uninfected cells were used and confirmed by silver staining (FIG. 7c).

Unweighted spectral count analysis against the no-probe control revealed 133, 167, 192, and 160 proteins that are overrepresented in the OC43 Probe I experiment at 12, 24, 36, and 48 hours post-infection (hpi), respectively (FDR < 10%, Table 3).

[Table 3]

| Protein IDs | gname | species | target | type | backgrou | adj.p |
|---|---|---|---|---|---|---|
| P33469 | N | CVHOC | 87 | n_12_v | 1 | 1.937E-135 |
| Q04837 | SSBP1 | HUMAN | 34 | n_12_v | 1 | 9.094E-40 |
| P19338 | NCL | HUMAN | 34 | n_12_v | 4 | 4.656E-20 |
| P11387;Q969P6 | TOP1 | HUMAN | 16 | n_12_v | 1 | 4.005E-14 |
| P42704;Q9NP80 | LRPPRC | HUMAN | 65 | n_12_v | 22 | 1.250E-13 |
| P07910;O60812;B2 | HNRNPC | HUMAN | 20 | n_12_v | 2 | 1.250E-13 |
| P09874 | PARP1 | HUMAN | 14 | n_12_v | 1 | 7.012E-12 |
| P22626 | HNRNPA2B1 | HUMAN | 18 | n_12_v | 2 | 1.114E-11 |
| P15880 | RPS2 | HUMAN | 24 | n_12_v | 4 | 1.892E-11 |
| P09651;Q32P51 | HNRNPA1 | HUMAN | 13 | n_12_v | 1 | 7.105E-11 |
| P52272 | HNRNPM | HUMAN | 13 | n_12_v | 1 | 7.105E-11 |
| Q92945 | KHSRP | HUMAN | 17 | n_12_v | 2 | 7.511E-11 |
| Q00839 | HNRNPU | HUMAN | 15 | n_12_v | 2 | 6.012E-09 |
| P02545 | LMNA | HUMAN | 11 | n_12_v | 1 | 1.069E-08 |
| P46777 | RPL5 | HUMAN | 11 | n_12_v | 1 | 1.069E-08 |
| P26373 | RPL13 | HUMAN | 13 | n_12_v | 2 | 3.328E-07 |
| P62917 | RPL8 | HUMAN | 18 | n_12_v | 4 | 4.631E-07 |
| P26599 | PTBP1 | HUMAN | 9 | n_12_v | 1 | 1.197E-06 |
| Q02878 | RPL6 | HUMAN | 9 | n_12_v | 1 | 1.197E-06 |
| Q15717;Q12926;P2 | ELAVL1 | HUMAN | 9 | n_12_v | 1 | 1.197E-06 |
| O60506 | SYNCRIP | HUMAN | 14 | n_12_v | 3 | 5.771E-06 |
| P09429;B2RPK0 | HMGB1 | HUMAN | 8 | n_12_v | 1 | 1.108E-05 |
| Q96I24 | FUBP3 | HUMAN | 8 | n_12_v | 1 | 1.108E-05 |
| P31943 | HNRNPH1 | HUMAN | 13 | n_12_v | 3 | 2.596E-05 |
| P61978 | HNRNPK | HUMAN | 13 | n_12_v | 3 | 2.596E-05 |
| P62081 | RPS7 | HUMAN | 10 | n_12_v | 2 | 6.821E-05 |
| O75534 | CSDE1 | HUMAN | 7 | n_12_v | 1 | 8.737E-05 |
| P31942 | HNRNPH3 | HUMAN | 7 | n_12_v | 1 | 8.737E-05 |
| P36578 | RPL4 | HUMAN | 19 | n_12_v | 7 | 2.571E-04 |
| P18124 | RPL7 | HUMAN | 11 | n_12_v | 3 | 5.049E-04 |
| A0AV96;Q8TBY0;Q | RBM47 | HUMAN | 6 | n_12_v | 1 | 5.367E-04 |
| O76021 | RSL1D1 | HUMAN | 6 | n_12_v | 1 | 5.367E-04 |
| P23246 | SFPQ | HUMAN | 6 | n_12_v | 1 | 5.367E-04 |
| P46781 | RPS9 | HUMAN | 6 | n_12_v | 1 | 5.367E-04 |
| P62263 | RPS14 | HUMAN | 6 | n_12_v | 1 | 5.367E-04 |
| Q01831 | XPC | HUMAN | 6 | n_12_v | 1 | 5.367E-04 |
| Q08945 | SSRP1 | HUMAN | 6 | n_12_v | 1 | 5.367E-04 |
| Q5JTH9 | RRP12 | HUMAN | 6 | n_12_v | 1 | 5.367E-04 |
| Q9UIG0 | BAZ1B | HUMAN | 6 | n_12_v | 1 | 5.367E-04 |
| Q02543 | RPL18A | HUMAN | 8 | n_12_v | 2 | 1.407E-03 |
| P16403 | HIST1H1C | HUMAN | 5 | n_12_v | 1 | 2.967E-03 |
| P16989 | YBX3 | HUMAN | 5 | n_12_v | 1 | 2.967E-03 |
| P18621 | RPL17 | HUMAN | 5 | n_12_v | 1 | 2.967E-03 |
| P27694 | RPA1 | HUMAN | 5 | n_12_v | 1 | 2.967E-03 |
| P62750 | RPL23A | HUMAN | 5 | n_12_v | 1 | 2.967E-03 |
| Q01455 | M | CVHOC | 5 | n_12_v | 1 | 2.967E-03 |
| Q07955 | SRSF1 | HUMAN | 5 | n_12_v | 1 | 2.967E-03 |
| Q14980 | NUMA1 | HUMAN | 5 | n_12_v | 1 | 2.967E-03 |
| Q15365 | PCBP1 | HUMAN | 5 | n_12_v | 1 | 2.967E-03 |
| Q5SSJ5 | HP1BP3 | HUMAN | 5 | n_12_v | 1 | 2.967E-03 |
| Q7KZF4 | SND1 | HUMAN | 5 | n_12_v | 1 | 2.967E-03 |
| Q99729 | HNRNPAB | HUMAN | 5 | n_12_v | 1 | 2.967E-03 |
| Q9Y5B9 | SUPT16H | HUMAN | 5 | n_12_v | 1 | 2.967E-03 |
| P52597 | HNRNPF | HUMAN | 9 | n_12_v | 3 | 4.805E-03 |
| Q86V81 | ALYREF | HUMAN | 7 | n_12_v | 2 | 4.975E-03 |
| P62424 | RPL7A | HUMAN | 12 | n_12_v | 5 | 7.821E-03 |
| Q07020 | RPL18 | HUMAN | 12 | n_12_v | 5 | 7.821E-03 |
| P47914 | RPL29 | HUMAN | 10 | n_12_v | 4 | 1.145E-02 |
| P39023;Q92901 | RPL3 | HUMAN | 13 | n_12_v | 6 | 1.171E-02 |
| 852-2750 | NSP3 | CVHOC | 4 | n_12_v | 1 | 1.171E-02 |
| O14979 | HNRNPDL | HUMAN | 4 | n_12_v | 1 | 1.171E-02 |
| O43390 | HNRNPR | HUMAN | 4 | n_12_v | 1 | 1.171E-02 |
| O75367;Q9P0M6 | H2AFY | HUMAN | 4 | n_12_v | 1 | 1.171E-02 |
| P11388 | TOP2A | HUMAN | 4 | n_12_v | 1 | 1.171E-02 |

| Protein IDs | gname | species | target | type | backgrou | adj.p |
|---|---|---|---|---|---|---|
| P14866 | HNRNPL | HUMAN | 4 | n_12_v | 1 | 1.171E-02 |
| P16402 | HIST1H1D | HUMAN | 4 | n_12_v | 1 | 1.171E-02 |
| P17480 | UBTF | HUMAN | 4 | n_12_v | 1 | 1.171E-02 |
| P17844 | DDX5 | HUMAN | 4 | n_12_v | 1 | 1.171E-02 |
| P23396 | RPS3 | HUMAN | 4 | n_12_v | 1 | 1.171E-02 |
| P27816 | MAP4 | HUMAN | 4 | n_12_v | 1 | 1.171E-02 |
| P35251 | RFC1 | HUMAN | 4 | n_12_v | 1 | 1.171E-02 |
| P43243 | MATR3 | HUMAN | 4 | n_12_v | 1 | 1.171E-02 |
| P46778 | RPL21 | HUMAN | 4 | n_12_v | 1 | 1.171E-02 |
| P51991 | HNRNPA3 | HUMAN | 4 | n_12_v | 1 | 1.171E-02 |
| P62899 | RPL31 | HUMAN | 4 | n_12_v | 1 | 1.171E-02 |
| Q12905 | ILF2 | HUMAN | 4 | n_12_v | 1 | 1.171E-02 |
| Q14103 | HNRNPD | HUMAN | 4 | n_12_v | 1 | 1.171E-02 |
| Q15637 | SF1 | HUMAN | 4 | n_12_v | 1 | 1.171E-02 |
| Q16629 | SRSF7 | HUMAN | 4 | n_12_v | 1 | 1.171E-02 |
| Q5T653 | MRPL2 | HUMAN | 4 | n_12_v | 1 | 1.171E-02 |
| Q6P5R6 | RPL22L1 | HUMAN | 4 | n_12_v | 1 | 1.171E-02 |
| Q8IYB3 | SRRM1 | HUMAN | 4 | n_12_v | 1 | 1.171E-02 |
| Q8NC51 | SERBP1 | HUMAN | 4 | n_12_v | 1 | 1.171E-02 |
| Q92841 | DDX17 | HUMAN | 4 | n_12_v | 1 | 1.171E-02 |
| Q96PK6 | RBM14 | HUMAN | 4 | n_12_v | 1 | 1.171E-02 |
| Q9BYD1 | MRPL13 | HUMAN | 4 | n_12_v | 1 | 1.171E-02 |
| Q9P2E9 | RRBP1 | HUMAN | 4 | n_12_v | 1 | 1.171E-02 |
| P62805 | HIST1H4A | HUMAN | 17 | n_12_v | 9 | 1.240E-02 |
| P42766 | RPL35 | HUMAN | 6 | n_12_v | 2 | 1.307E-02 |
| P46782 | RPS5 | HUMAN | 6 | n_12_v | 2 | 1.307E-02 |
| Q14498 | RBM39 | HUMAN | 6 | n_12_v | 2 | 1.307E-02 |
| P62753 | RPS6 | HUMAN | 12 | n_12_v | 6 | 2.215E-02 |
| P16401 | HIST1H1B | HUMAN | 13 | n_12_v | 7 | 3.161E-02 |
| Q8WUH6 | TMEM263 | HUMAN | 7 | n_12_v | 3 | 3.296E-02 |
| P62701;P22090;Q8 | RPS4X | HUMAN | 5 | n_12_v | 2 | 4.177E-02 |
| O00571 | DDX3X | HUMAN | 3 | n_12_v | 1 | 4.177E-02 |
| O60841 | EIF5B | HUMAN | 3 | n_12_v | 1 | 4.177E-02 |
| O75494;Q8WXF0 | SRSF10 | HUMAN | 3 | n_12_v | 1 | 4.177E-02 |
| P05455 | SSB | HUMAN | 3 | n_12_v | 1 | 4.177E-02 |
| P06748 | NPM1 | HUMAN | 3 | n_12_v | 1 | 4.177E-02 |
| P11940;Q9H361;Q9 | PABPC1 | HUMAN | 3 | n_12_v | 1 | 4.177E-02 |
| P12956 | XRCC6 | HUMAN | 3 | n_12_v | 1 | 4.177E-02 |
| P17096 | HMGA1 | HUMAN | 3 | n_12_v | 1 | 4.177E-02 |
| P26368 | U2AF2 | HUMAN | 3 | n_12_v | 1 | 4.177E-02 |
| P27695 | APEX1 | HUMAN | 3 | n_12_v | 1 | 4.177E-02 |
| P30876 | POLR2B | HUMAN | 3 | n_12_v | 1 | 4.177E-02 |
| P32322 | PYCR1 | HUMAN | 3 | n_12_v | 1 | 4.177E-02 |
| P35659 | DEK | HUMAN | 3 | n_12_v | 1 | 4.177E-02 |
| P38159;O75526;Q8 | RBMX | HUMAN | 3 | n_12_v | 1 | 4.177E-02 |
| P46013 | MKI67 | HUMAN | 3 | n_12_v | 1 | 4.177E-02 |
| P53999 | SUB1 | HUMAN | 3 | n_12_v | 1 | 4.177E-02 |
| P56134 | ATP5J2 | HUMAN | 3 | n_12_v | 1 | 4.177E-02 |
| P62633 | CNBP | HUMAN | 3 | n_12_v | 1 | 4.177E-02 |
| P83731 | RPL24 | HUMAN | 3 | n_12_v | 1 | 4.177E-02 |
| Q00341 | HDLBP | HUMAN | 3 | n_12_v | 1 | 4.177E-02 |
| Q12906 | ILF3 | HUMAN | 3 | n_12_v | 1 | 4.177E-02 |
| Q13151 | HNRNPA0 | HUMAN | 3 | n_12_v | 1 | 4.177E-02 |
| Q13243 | SRSF5 | HUMAN | 3 | n_12_v | 1 | 4.177E-02 |
| Q13263 | TRIM28 | HUMAN | 3 | n_12_v | 1 | 4.177E-02 |
| Q13427 | PPIG | HUMAN | 3 | n_12_v | 1 | 4.177E-02 |
| Q13595 | TRA2A | HUMAN | 3 | n_12_v | 1 | 4.177E-02 |
| Q14684 | RRP1B | HUMAN | 3 | n_12_v | 1 | 4.177E-02 |
| Q15366;P57721 | PCBP2 | HUMAN | 3 | n_12_v | 1 | 4.177E-02 |
| Q15424 | SAFB | HUMAN | 3 | n_12_v | 1 | 4.177E-02 |
| Q96T88 | UHRF1 | HUMAN | 3 | n_12_v | 1 | 4.177E-02 |
| Q9BUJ2 | HNRNPUL1 | HUMAN | 3 | n_12_v | 1 | 4.177E-02 |
| Q9BWF3;Q9BQ04 | RBM4 | HUMAN | 3 | n_12_v | 1 | 4.177E-02 |
| Q9GZT3 | SLIRP | HUMAN | 3 | n_12_v | 1 | 4.177E-02 |
| Protein IDs | gname | species | target | type | backgrou | adj.p |
| Q9HCK4 | ROBO2 | HUMAN | 3 | n_12_v | 1 | 4.177E-02 |
| Q9NWH9 | SLTM | HUMAN | 3 | n_12_v | 1 | 4.177E-02 |
| Q9P258 | RCC2 | HUMAN | 3 | n_12_v | 1 | 4.177E-02 |
| Q9UKM9 | RALY | HUMAN | 3 | n_12_v | 1 | 4.177E-02 |
| Q9Y2W1 | THRAP3 | HUMAN | 3 | n_12_v | 1 | 4.177E-02 |

130

| Protein IDs | gname | species | target | type | backgrou | adj.p |
|---|---|---|---|---|---|---|
| P33469 | N | CVHOC | 252 | n_24_v | 2 | 0.000E+00 |
| P52272 | HNRNPM | HUMAN | 34 | n_24_v | 1 | 1.310E-34 |
| P22626 | HNRNPA2B1 | HUMAN | 46 | n_24_v | 3 | 1.950E-31 |
| P42704;Q9NP80 | LRPPRC | HUMAN | 117 | n_24_v | 25 | 2.510E-28 |
| P14866 | HNRNPL | HUMAN | 27 | n_24_v | 1 | 2.531E-25 |
| Q92945 | KHSRP | HUMAN | 53 | n_24_v | 6 | 6.121E-25 |
| Q96I24 | FUBP3 | HUMAN | 26 | n_24_v | 1 | 3.831E-24 |
| P07910;O60812;B2| | HNRNPC | HUMAN | 47 | n_24_v | 5 | 2.035E-23 |
| P11387;Q969P6 | TOP1 | HUMAN | 25 | n_24_v | 1 | 6.088E-23 |
| P43243 | MATR3 | HUMAN | 24 | n_24_v | 1 | 1.078E-21 |
| O60506 | SYNCRIP | HUMAN | 43 | n_24_v | 5 | 3.692E-20 |
| P51991 | HNRNPA3 | HUMAN | 29 | n_24_v | 2 | 6.333E-20 |
| O43390 | HNRNPR | HUMAN | 27 | n_24_v | 2 | 7.304E-18 |
| P09651;Q32P51 | HNRNPA1 | HUMAN | 31 | n_24_v | 3 | 4.272E-17 |
| Q04837 | SSBP1 | HUMAN | 31 | n_24_v | 3 | 4.272E-17 |
| P26599 | PTBP1 | HUMAN | 20 | n_24_v | 1 | 6.703E-17 |
| Q12906 | ILF3 | HUMAN | 19 | n_24_v | 1 | 1.002E-15 |
| P31943 | HNRNPH1 | HUMAN | 27 | n_24_v | 3 | 1.259E-13 |
| P19338 | NCL | HUMAN | 53 | n_24_v | 12 | 1.525E-12 |
| P23246 | SFPQ | HUMAN | 16 | n_24_v | 1 | 2.407E-12 |
| Q99729 | HNRNPAB | HUMAN | 16 | n_24_v | 1 | 2.407E-12 |
| Q15717;Q12926;P2 | ELAVL1 | HUMAN | 21 | n_24_v | 2 | 3.475E-12 |
| P61978 | HNRNPK | HUMAN | 28 | n_24_v | 4 | 1.579E-11 |
| P31942 | HNRNPH3 | HUMAN | 15 | n_24_v | 1 | 2.603E-11 |
| Q13148 | TARDBP | HUMAN | 15 | n_24_v | 1 | 2.603E-11 |
| P17844 | DDX5 | HUMAN | 19 | n_24_v | 2 | 1.956E-10 |
| O14979 | HNRNPDL | HUMAN | 14 | n_24_v | 1 | 2.817E-10 |
| P09874 | PARP1 | HUMAN | 14 | n_24_v | 1 | 2.817E-10 |
| P46777 | RPL5 | HUMAN | 31 | n_24_v | 6 | 2.069E-09 |
| P62917 | RPL8 | HUMAN | 39 | n_24_v | 9 | 2.276E-09 |
| 852-2750 | NSP3 | CVHOC | 13 | n_24_v | 1 | 2.718E-09 |
| A0AV96;Q8TBY0;Q | RBM47 | HUMAN | 13 | n_24_v | 1 | 2.718E-09 |
| Q9UKM9 | RALY | HUMAN | 13 | n_24_v | 1 | 2.718E-09 |
| P52597 | HNRNPF | HUMAN | 17 | n_24_v | 2 | 8.200E-09 |
| Q07666;Q5VWX1 | KHDRBS1 | HUMAN | 12 | n_24_v | 1 | 2.530E-08 |
| Q08211 | DHX9 | HUMAN | 12 | n_24_v | 1 | 2.530E-08 |
| Q15233 | NONO | HUMAN | 12 | n_24_v | 1 | 2.530E-08 |
| Q00839 | HNRNPU | HUMAN | 29 | n_24_v | 6 | 2.530E-08 |
| P15880 | RPS2 | HUMAN | 30 | n_24_v | 7 | 2.091E-07 |
| P83731 | RPL24 | HUMAN | 11 | n_24_v | 1 | 2.238E-07 |
| Q13243 | SRSF5 | HUMAN | 11 | n_24_v | 1 | 2.238E-07 |
| Q96AE4 | FUBP1 | HUMAN | 11 | n_24_v | 1 | 2.238E-07 |
| P47914 | RPL29 | HUMAN | 14 | n_24_v | 2 | 1.750E-06 |
| P36334 | S | CVHOC | 10 | n_24_v | 1 | 1.786E-06 |
| P62995 | TRA2B | HUMAN | 10 | n_24_v | 1 | 1.786E-06 |
| Q14103 | HNRNPD | HUMAN | 10 | n_24_v | 1 | 1.786E-06 |
| Q6PKG0 | LARP1 | HUMAN | 10 | n_24_v | 1 | 1.786E-06 |
| Q9NZB2;Q5T035 | FAM120A | HUMAN | 10 | n_24_v | 1 | 1.786E-06 |
| P67809 | YBX1 | HUMAN | 17 | n_24_v | 3 | 2.424E-06 |
| P36578 | RPL4 | HUMAN | 34 | n_24_v | 10 | 5.332E-06 |
| O75534 | CSDE1 | HUMAN | 9 | n_24_v | 1 | 1.238E-05 |
| P35637 | FUS | HUMAN | 9 | n_24_v | 1 | 1.238E-05 |
| P62633 | CNBP | HUMAN | 9 | n_24_v | 1 | 1.238E-05 |
| Q13151 | HNRNPA0 | HUMAN | 9 | n_24_v | 1 | 1.238E-05 |
| Q5T653 | MRPL2 | HUMAN | 9 | n_24_v | 1 | 1.238E-05 |
| Q7Z2W4 | ZC3HAV1 | HUMAN | 9 | n_24_v | 1 | 1.238E-05 |
| Q8N163 | CCAR2 | HUMAN | 9 | n_24_v | 1 | 1.238E-05 |
| Q8NC51 | SERBP1 | HUMAN | 9 | n_24_v | 1 | 1.238E-05 |
| Q02878 | RPL6 | HUMAN | 18 | n_24_v | 4 | 2.841E-05 |
| Q7L2E3 | DHX30 | HUMAN | 12 | n_24_v | 2 | 3.777E-05 |
| Q86V81 | ALYREF | HUMAN | 12 | n_24_v | 2 | 3.777E-05 |
| P11940;Q9H361;Q9 | PABPC1 | HUMAN | 8 | n_24_v | 1 | 7.500E-05 |
| P27694 | RPA1 | HUMAN | 8 | n_24_v | 1 | 7.500E-05 |
| P38159;O75526;Q8 | RBMX | HUMAN | 8 | n_24_v | 1 | 7.500E-05 |

| Protein IDs | gname | species | target | type | backgrou | adj.p |
|---|---|---|---|---|---|---|
| P60866 | RPS20 | HUMAN | 8 | n_24_v | 1 | 7.500E-05 |
| Q07955 | SRSF1 | HUMAN | 8 | n_24_v | 1 | 7.500E-05 |
| Q12905 | ILF2 | HUMAN | 8 | n_24_v | 1 | 7.500E-05 |
| Q14011 | CIRBP | HUMAN | 8 | n_24_v | 1 | 7.500E-05 |
| Q7KZF4 | SND1 | HUMAN | 8 | n_24_v | 1 | 7.500E-05 |
| Q96PK6 | RBM14 | HUMAN | 8 | n_24_v | 1 | 7.500E-05 |
| Q9BUJ2 | HNRNPUL1 | HUMAN | 8 | n_24_v | 1 | 7.500E-05 |
| Q9GZT3 | SLIRP | HUMAN | 8 | n_24_v | 1 | 7.500E-05 |
| Q9HCE1 | MOV10 | HUMAN | 8 | n_24_v | 1 | 7.500E-05 |
| Q01455 | M | CVHOC | 14 | n_24_v | 3 | 1.306E-04 |
| P46781 | RPS9 | HUMAN | 11 | n_24_v | 2 | 1.490E-04 |
| P62081 | RPS7 | HUMAN | 11 | n_24_v | 2 | 1.490E-04 |
| Q15366;P57721 | PCBP2 | HUMAN | 11 | n_24_v | 2 | 1.490E-04 |
| O00571 | DDX3X | HUMAN | 7 | n_24_v | 1 | 4.383E-04 |
| O75494;Q8WXF0 | SRSF10 | HUMAN | 7 | n_24_v | 1 | 4.383E-04 |
| P27635;Q96L21 | RPL10 | HUMAN | 7 | n_24_v | 1 | 4.383E-04 |
| Q01844 | EWSR1 | HUMAN | 7 | n_24_v | 1 | 4.383E-04 |
| Q15365 | PCBP1 | HUMAN | 7 | n_24_v | 1 | 4.383E-04 |
| Q15424 | SAFB | HUMAN | 7 | n_24_v | 1 | 4.383E-04 |
| Q7L014 | DDX46 | HUMAN | 7 | n_24_v | 1 | 4.383E-04 |
| Q92841 | DDX17 | HUMAN | 7 | n_24_v | 1 | 4.383E-04 |
| Q9P258 | RCC2 | HUMAN | 7 | n_24_v | 1 | 4.383E-04 |
| P62701;P22090;Q8 | RPS4X | HUMAN | 9 | n_24_v | 2 | 2.271E-03 |
| O00425;Q9NZI8 | IGF2BP3 | HUMAN | 6 | n_24_v | 1 | 2.271E-03 |
| O15371 | EIF3D | HUMAN | 6 | n_24_v | 1 | 2.271E-03 |
| P16989 | YBX3 | HUMAN | 6 | n_24_v | 1 | 2.271E-03 |
| P23396 | RPS3 | HUMAN | 6 | n_24_v | 1 | 2.271E-03 |
| P42766 | RPL35 | HUMAN | 6 | n_24_v | 1 | 2.271E-03 |
| P62263 | RPS14 | HUMAN | 6 | n_24_v | 1 | 2.271E-03 |
| P98179 | RBM3 | HUMAN | 6 | n_24_v | 1 | 2.271E-03 |
| Q07065 | CKAP4 | HUMAN | 6 | n_24_v | 1 | 2.271E-03 |
| Q12849 | GRSF1 | HUMAN | 6 | n_24_v | 1 | 2.271E-03 |
| Q14498 | RBM39 | HUMAN | 6 | n_24_v | 1 | 2.271E-03 |
| Q15056 | EIF4H | HUMAN | 6 | n_24_v | 1 | 2.271E-03 |
| Q1KMD3 | HNRNPUL2 | HUMAN | 6 | n_24_v | 1 | 2.271E-03 |
| Q92879;O95319 | CELF1 | HUMAN | 6 | n_24_v | 1 | 2.271E-03 |
| Q9BYD1 | MRPL13 | HUMAN | 6 | n_24_v | 1 | 2.271E-03 |
| Q9Y3I0 | RTCB | HUMAN | 6 | n_24_v | 1 | 2.271E-03 |
| P62280 | RPS11 | HUMAN | 11 | n_24_v | 3 | 4.359E-03 |
| P02545 | LMNA | HUMAN | 8 | n_24_v | 2 | 8.279E-03 |
| Q00341 | HDLBP | HUMAN | 8 | n_24_v | 2 | 8.279E-03 |
| O95758 | PTBP3 | HUMAN | 5 | n_24_v | 1 | 1.027E-02 |
| P09429;B2RPK0 | HMGB1 | HUMAN | 5 | n_24_v | 1 | 1.027E-02 |
| P0DMV9;P0DMV8 | HSPA1B | HUMAN | 5 | n_24_v | 1 | 1.027E-02 |
| P11021 | HSPA5 | HUMAN | 5 | n_24_v | 1 | 1.027E-02 |
| P16402 | HIST1H1D | HUMAN | 5 | n_24_v | 1 | 1.027E-02 |
| P18583 | SON | HUMAN | 5 | n_24_v | 1 | 1.027E-02 |
| P22087 | FBL | HUMAN | 5 | n_24_v | 1 | 1.027E-02 |
| P51114 | FXR1 | HUMAN | 5 | n_24_v | 1 | 1.027E-02 |
| Q08945 | SSRP1 | HUMAN | 5 | n_24_v | 1 | 1.027E-02 |
| Q14152 | EIF3A | HUMAN | 5 | n_24_v | 1 | 1.027E-02 |
| Q15637 | SF1 | HUMAN | 5 | n_24_v | 1 | 1.027E-02 |
| Q86U42;A6NDY0 | PABPN1 | HUMAN | 5 | n_24_v | 1 | 1.027E-02 |
| Q8WWM7 | ATXN2L | HUMAN | 5 | n_24_v | 1 | 1.027E-02 |
| Q8WXF1 | PSPC1 | HUMAN | 5 | n_24_v | 1 | 1.027E-02 |
| Q92499 | DDX1 | HUMAN | 5 | n_24_v | 1 | 1.027E-02 |
| Q9NVS2 | MRPS18A | HUMAN | 5 | n_24_v | 1 | 1.027E-02 |
| Q9UIG0 | BAZ1B | HUMAN | 5 | n_24_v | 1 | 1.027E-02 |
| P46782 | RPS5 | HUMAN | 10 | n_24_v | 3 | 1.136E-02 |
| P18124 | RPL7 | HUMAN | 16 | n_24_v | 6 | 1.464E-02 |
| P40429;Q6NVV1 | RPL13A | HUMAN | 13 | n_24_v | 5 | 3.426E-02 |
| 247-851 | NSP2 | CVHOC | 4 | n_24_v | 1 | 3.618E-02 |
| CON__Q9N2I2 | NA | NA | 4 | n_24_v | 1 | 3.618E-02 |
| O43776 | NARS | HUMAN | 4 | n_24_v | 1 | 3.618E-02 |

| Protein IDs | gname | species | target | type | backgrou | adj.p |
|---|---|---|---|---|---|---|
| O75367;Q9P0M6 | H2AFY | HUMAN | 4 | n_24_v | 1 | 3.618E-02 |
| O75822 | EIF3J | HUMAN | 4 | n_24_v | 1 | 3.618E-02 |
| P05455 | SSB | HUMAN | 4 | n_24_v | 1 | 3.618E-02 |
| P08708 | RPS17 | HUMAN | 4 | n_24_v | 1 | 3.618E-02 |
| P12956 | XRCC6 | HUMAN | 4 | n_24_v | 1 | 3.618E-02 |
| P16615;Q93084;O1 | ATP2A2 | HUMAN | 4 | n_24_v | 1 | 3.618E-02 |
| P23588 | EIF4B | HUMAN | 4 | n_24_v | 1 | 3.618E-02 |
| P26368 | U2AF2 | HUMAN | 4 | n_24_v | 1 | 3.618E-02 |
| P26583 | HMGB2 | HUMAN | 4 | n_24_v | 1 | 3.618E-02 |
| P42681 | TXK | HUMAN | 4 | n_24_v | 1 | 3.618E-02 |
| P53999 | SUB1 | HUMAN | 4 | n_24_v | 1 | 3.618E-02 |
| P63241;Q6IS14;Q9( | EIF5A | HUMAN | 4 | n_24_v | 1 | 3.618E-02 |
| Q01085 | TIAL1 | HUMAN | 4 | n_24_v | 1 | 3.618E-02 |
| Q13247 | SRSF6 | HUMAN | 4 | n_24_v | 1 | 3.618E-02 |
| Q13310 | PABPC4 | HUMAN | 4 | n_24_v | 1 | 3.618E-02 |
| Q13838 | DDX39B | HUMAN | 4 | n_24_v | 1 | 3.618E-02 |
| Q15084 | PDIA6 | HUMAN | 4 | n_24_v | 1 | 3.618E-02 |
| Q6Y7W6 | GIGYF2 | HUMAN | 4 | n_24_v | 1 | 3.618E-02 |
| Q8N1G4 | LRRC47 | HUMAN | 4 | n_24_v | 1 | 3.618E-02 |
| Q92804 | TAF15 | HUMAN | 4 | n_24_v | 1 | 3.618E-02 |
| Q92900 | UPF1 | HUMAN | 4 | n_24_v | 1 | 3.618E-02 |
| Q96AG4 | LRRC59 | HUMAN | 4 | n_24_v | 1 | 3.618E-02 |
| Q99575 | POP1 | HUMAN | 4 | n_24_v | 1 | 3.618E-02 |
| Q9BYD3 | MRPL4 | HUMAN | 4 | n_24_v | 1 | 3.618E-02 |
| Q9BZE1 | MRPL37 | HUMAN | 4 | n_24_v | 1 | 3.618E-02 |
| Q9H6T0;Q6NXG1 | ESRP2 | HUMAN | 4 | n_24_v | 1 | 3.618E-02 |
| Q9H7E9 | C8orf33 | HUMAN | 4 | n_24_v | 1 | 3.618E-02 |
| Q9NWH9 | SLTM | HUMAN | 4 | n_24_v | 1 | 3.618E-02 |
| Q9NYQ8 | FAT2 | HUMAN | 4 | n_24_v | 1 | 3.618E-02 |
| Q9UN86 | G3BP2 | HUMAN | 4 | n_24_v | 1 | 3.618E-02 |
| Q9UQ80 | PA2G4 | HUMAN | 4 | n_24_v | 1 | 3.618E-02 |
| Q9Y3Y2 | CHTOP | HUMAN | 4 | n_24_v | 1 | 3.618E-02 |
| Q9Y520 | PRRC2C | HUMAN | 4 | n_24_v | 1 | 3.618E-02 |
| Q9Y5W8 | SNX13 | HUMAN | 4 | n_24_v | 1 | 3.618E-02 |
| O60832 | DKC1 | HUMAN | 6 | n_24_v | 2 | 5.868E-02 |
| Q6P2Q9 | PRPF8 | HUMAN | 6 | n_24_v | 2 | 5.868E-02 |
| P27816 | MAP4 | HUMAN | 8 | n_24_v | 3 | 6.373E-02 |
| P62750 | RPL23A | HUMAN | 8 | n_24_v | 3 | 6.373E-02 |
| P62424 | RPL7A | HUMAN | 20 | n_24_v | 10 | 9.226E-02 |

| Protein IDs | gname | species | target | type | backgrou | adj.p |
|---|---|---|---|---|---|---|
| P33469 | N | CVHOC | 386 | n_36_v | 25 | 4.954E-236 |
| P14866 | HNRNPL | HUMAN | 59 | n_36_v | 3 | 1.074E-41 |
| P42704;Q9NP80 | LRPPRC | HUMAN | 175 | n_36_v | 32 | 3.948E-41 |
| Q96I24 | FUBP3 | HUMAN | 49 | n_36_v | 2 | 2.056E-39 |
| O60506 | SYNCRIP | HUMAN | 90 | n_36_v | 10 | 1.287E-36 |
| Q12906 | ILF3 | HUMAN | 39 | n_36_v | 2 | 5.592E-28 |
| O43390 | HNRNPR | HUMAN | 70 | n_36_v | 9 | 5.290E-25 |
| P43243 | MATR3 | HUMAN | 57 | n_36_v | 6 | 1.236E-24 |
| Q00341 | HDLBP | HUMAN | 26 | n_36_v | 1 | 3.014E-22 |
| Q9HCE1 | MOV10 | HUMAN | 26 | n_36_v | 1 | 3.014E-22 |
| P61978 | HNRNPK | HUMAN | 65 | n_36_v | 9 | 9.610E-22 |
| Q00839 | HNRNPU | HUMAN | 78 | n_36_v | 13 | 3.876E-21 |
| Q1KMD3 | HNRNPUL2 | HUMAN | 24 | n_36_v | 1 | 6.605E-20 |
| P09651;Q32P51 | HNRNPA1 | HUMAN | 57 | n_36_v | 8 | 5.772E-19 |
| Q9NZB2;Q5T035 | FAM120A | HUMAN | 23 | n_36_v | 1 | 9.118E-19 |
| P07910;O60812;B2I | HNRNPC | HUMAN | 69 | n_36_v | 12 | 6.450E-18 |
| Q9UKM9 | RALY | HUMAN | 29 | n_36_v | 2 | 6.625E-18 |
| P52597 | HNRNPF | HUMAN | 39 | n_36_v | 4 | 9.652E-18 |
| Q15717;Q12926;P2 | ELAVL1 | HUMAN | 38 | n_36_v | 4 | 5.846E-17 |
| Q92945 | KHSRP | HUMAN | 58 | n_36_v | 10 | 1.954E-15 |
| P51991 | HNRNPA3 | HUMAN | 36 | n_36_v | 4 | 1.954E-15 |
| Q8N163 | CCAR2 | HUMAN | 20 | n_36_v | 1 | 1.954E-15 |
| P52272 | HNRNPM | HUMAN | 53 | n_36_v | 9 | 1.836E-14 |
| P36334 | S | CVHOC | 24 | n_36_v | 2 | 2.659E-13 |
| Q5T653 | MRPL2 | HUMAN | 18 | n_36_v | 1 | 2.935E-13 |
| P26599 | PTBP1 | HUMAN | 60 | n_36_v | 12 | 3.334E-13 |
| A0AV96;Q8TBY0;Q | RBM47 | HUMAN | 28 | n_36_v | 3 | 1.124E-12 |
| O00425;Q9NZI8 | IGF2BP3 | HUMAN | 17 | n_36_v | 1 | 3.176E-12 |
| P22626 | HNRNPA2B1 | HUMAN | 69 | n_36_v | 16 | 4.246E-12 |
| P11940;Q9H361;Q9 | PABPC1 | HUMAN | 22 | n_36_v | 2 | 1.302E-11 |
| Q9NVS2 | MRPS18A | HUMAN | 16 | n_36_v | 1 | 3.294E-11 |
| P17844 | DDX5 | HUMAN | 30 | n_36_v | 4 | 3.687E-11 |
| Q6PKG0 | LARP1 | HUMAN | 21 | n_36_v | 2 | 8.705E-11 |
| Q08211 | DHX9 | HUMAN | 29 | n_36_v | 4 | 1.723E-10 |
| Q92879;O95319 | CELF1 | HUMAN | 15 | n_36_v | 1 | 3.166E-10 |
| P98179 | RBM3 | HUMAN | 14 | n_36_v | 1 | 3.146E-09 |
| Q13148 | TARDBP | HUMAN | 29 | n_36_v | 5 | 2.784E-08 |
| Q13084 | MRPL28 | HUMAN | 13 | n_36_v | 1 | 2.784E-08 |
| Q9GZT3 | SLIRP | HUMAN | 13 | n_36_v | 1 | 2.784E-08 |
| Q15233 | NONO | HUMAN | 28 | n_36_v | 5 | 1.008E-07 |
| P27635;Q96L21 | RPL10 | HUMAN | 21 | n_36_v | 3 | 1.177E-07 |
| Q86V81 | ALYREF | HUMAN | 17 | n_36_v | 2 | 1.232E-07 |
| Q9BZE1 | MRPL37 | HUMAN | 17 | n_36_v | 2 | 1.232E-07 |
| P48634 | PRRC2A | HUMAN | 12 | n_36_v | 1 | 2.026E-07 |
| P55795 | HNRNPH2 | HUMAN | 12 | n_36_v | 1 | 2.026E-07 |
| Q13310 | PABPC4 | HUMAN | 12 | n_36_v | 1 | 2.026E-07 |
| Q15084 | PDIA6 | HUMAN | 12 | n_36_v | 1 | 2.026E-07 |
| Q92499 | DDX1 | HUMAN | 12 | n_36_v | 1 | 2.026E-07 |
| Q04837 | SSBP1 | HUMAN | 24 | n_36_v | 4 | 2.170E-07 |
| 852-2750 | NSP3 | CVHOC | 23 | n_36_v | 4 | 8.545E-07 |
| O75494;Q8WXF0 | SRSF10 | HUMAN | 11 | n_36_v | 1 | 1.421E-06 |
| Q14671 | PUM1 | HUMAN | 11 | n_36_v | 1 | 1.421E-06 |
| Q7Z2W4 | ZC3HAV1 | HUMAN | 11 | n_36_v | 1 | 1.421E-06 |
| Q96EP5 | DAZAP1 | HUMAN | 11 | n_36_v | 1 | 1.421E-06 |
| Q9BYD1 | MRPL13 | HUMAN | 11 | n_36_v | 1 | 1.421E-06 |
| Q9BYK8 | HELZ2 | HUMAN | 11 | n_36_v | 1 | 1.421E-06 |
| Q9P015 | MRPL15 | HUMAN | 11 | n_36_v | 1 | 1.421E-06 |
| P31943 | HNRNPH1 | HUMAN | 42 | n_36_v | 11 | 1.804E-06 |
| P38159;O75526;Q8 | RBMX | HUMAN | 15 | n_36_v | 2 | 2.783E-06 |
| P62633 | CNBP | HUMAN | 15 | n_36_v | 2 | 2.783E-06 |
| Q01455 | M | CVHOC | 25 | n_36_v | 5 | 3.088E-06 |
| O14979 | HNRNPDL | HUMAN | 18 | n_36_v | 3 | 7.082E-06 |
| P31483 | TIA1 | HUMAN | 10 | n_36_v | 1 | 9.740E-06 |
| Q9NWH9 | SLTM | HUMAN | 10 | n_36_v | 1 | 9.740E-06 |

| Protein IDs | gname | species | target | type | backgrou | adj.p |
|---|---|---|---|---|---|---|
| P31942 | HNRNPH3 | HUMAN | 17 | n_36_v | 3 | 2.758E-05 |
| P62917 | RPL8 | HUMAN | 66 | n_36_v | 23 | 3.217E-05 |
| Q99729 | HNRNPAB | HUMAN | 20 | n_36_v | 4 | 3.238E-05 |
| P11387;Q969P6 | TOP1 | HUMAN | 13 | n_36_v | 2 | 5.956E-05 |
| Q01085 | TIAL1 | HUMAN | 9 | n_36_v | 1 | 5.956E-05 |
| Q13283 | G3BP1 | HUMAN | 9 | n_36_v | 1 | 5.956E-05 |
| Q14152 | EIF3A | HUMAN | 9 | n_36_v | 1 | 5.956E-05 |
| Q9NZ01 | TECR | HUMAN | 9 | n_36_v | 1 | 5.956E-05 |
| Q9P0M9 | MRPL27 | HUMAN | 9 | n_36_v | 1 | 5.956E-05 |
| Q9Y383;Q9NQ29 | LUC7L2 | HUMAN | 9 | n_36_v | 1 | 5.956E-05 |
| O75534 | CSDE1 | HUMAN | 16 | n_36_v | 3 | 9.022E-05 |
| Q12905 | ILF2 | HUMAN | 16 | n_36_v | 3 | 9.022E-05 |
| Q7KZF4 | SND1 | HUMAN | 16 | n_36_v | 3 | 9.022E-05 |
| P19338 | NCL | HUMAN | 70 | n_36_v | 26 | 1.162E-04 |
| P62277 | RPS13 | HUMAN | 12 | n_36_v | 2 | 2.348E-04 |
| Q15637 | SF1 | HUMAN | 12 | n_36_v | 2 | 2.348E-04 |
| Q07065 | CKAP4 | HUMAN | 15 | n_36_v | 3 | 3.033E-04 |
| Q07955 | SRSF1 | HUMAN | 15 | n_36_v | 3 | 3.033E-04 |
| Q13151 | HNRNPA0 | HUMAN | 15 | n_36_v | 3 | 3.033E-04 |
| Q96AE4 | FUBP1 | HUMAN | 15 | n_36_v | 3 | 3.033E-04 |
| P13010 | XRCC5 | HUMAN | 8 | n_36_v | 1 | 3.191E-04 |
| P26368 | U2AF2 | HUMAN | 8 | n_36_v | 1 | 3.191E-04 |
| Q15056 | EIF4H | HUMAN | 8 | n_36_v | 1 | 3.191E-04 |
| Q80872 | 2a | CVHOC | 8 | n_36_v | 1 | 3.191E-04 |
| Q9BRJ2 | MRPL45 | HUMAN | 8 | n_36_v | 1 | 3.191E-04 |
| P23246 | SFPQ | HUMAN | 33 | n_36_v | 10 | 3.520E-04 |
| Q96PK6 | RBM14 | HUMAN | 20 | n_36_v | 5 | 5.913E-04 |
| O95758 | PTBP3 | HUMAN | 11 | n_36_v | 2 | 8.600E-04 |
| P49207 | RPL34 | HUMAN | 14 | n_36_v | 3 | 9.482E-04 |
| Q9P2E9 | RRBP1 | HUMAN | 22 | n_36_v | 6 | 1.016E-03 |
| P67809 | YBX1 | HUMAN | 19 | n_36_v | 5 | 1.547E-03 |
| P62841 | RPS15 | HUMAN | 7 | n_36_v | 1 | 1.653E-03 |
| Q13501 | SQSTM1 | HUMAN | 7 | n_36_v | 1 | 1.653E-03 |
| Q15424 | SAFB | HUMAN | 7 | n_36_v | 1 | 1.653E-03 |
| Q96AG4 | LRRC59 | HUMAN | 7 | n_36_v | 1 | 1.653E-03 |
| Q9BY77 | POLDIP3 | HUMAN | 7 | n_36_v | 1 | 1.653E-03 |
| Q14103 | HNRNPD | HUMAN | 16 | n_36_v | 4 | 2.132E-03 |
| P16989 | YBX3 | HUMAN | 13 | n_36_v | 3 | 2.787E-03 |
| Q9BUJ2 | HNRNPUL1 | HUMAN | 13 | n_36_v | 3 | 2.787E-03 |
| P22087 | FBL | HUMAN | 10 | n_36_v | 2 | 2.858E-03 |
| P25398 | RPS12 | HUMAN | 10 | n_36_v | 2 | 2.858E-03 |
| P35637 | FUS | HUMAN | 10 | n_36_v | 2 | 2.858E-03 |
| P62995 | TRA2B | HUMAN | 10 | n_36_v | 2 | 2.858E-03 |
| Q86U42;A6NDY0 | PABPN1 | HUMAN | 10 | n_36_v | 2 | 2.858E-03 |
| Q15365 | PCBP1 | HUMAN | 15 | n_36_v | 4 | 5.483E-03 |
| O15371 | EIF3D | HUMAN | 6 | n_36_v | 1 | 6.824E-03 |
| P23588 | EIF4B | HUMAN | 6 | n_36_v | 1 | 6.824E-03 |
| P39019 | RPS19 | HUMAN | 6 | n_36_v | 1 | 6.824E-03 |
| P46783;Q9NQ39 | RPS10 | HUMAN | 6 | n_36_v | 1 | 6.824E-03 |
| Q00577 | PURA | HUMAN | 6 | n_36_v | 1 | 6.824E-03 |
| Q14011 | CIRBP | HUMAN | 6 | n_36_v | 1 | 6.824E-03 |
| Q8N1G4 | LRRC47 | HUMAN | 6 | n_36_v | 1 | 6.824E-03 |
| Q8NCA5;Q52LJ0 | FAM98A | HUMAN | 6 | n_36_v | 1 | 6.824E-03 |
| Q8NE71 | ABCF1 | HUMAN | 6 | n_36_v | 1 | 6.824E-03 |
| Q92804 | TAF15 | HUMAN | 6 | n_36_v | 1 | 6.824E-03 |
| Q9BYD6 | MRPL1 | HUMAN | 6 | n_36_v | 1 | 6.824E-03 |
| Q9BYN8 | MRPS26 | HUMAN | 6 | n_36_v | 1 | 6.824E-03 |
| Q9BZZ5 | API5 | HUMAN | 6 | n_36_v | 1 | 6.824E-03 |
| Q9H6T0;Q6NXG1 | ESRP2 | HUMAN | 6 | n_36_v | 1 | 6.824E-03 |
| Q9UN86 | G3BP2 | HUMAN | 6 | n_36_v | 1 | 6.824E-03 |
| Q9Y2X3 | NOP58 | HUMAN | 6 | n_36_v | 1 | 6.824E-03 |
| Q9Y6M1 | IGF2BP2 | HUMAN | 6 | n_36_v | 1 | 6.824E-03 |
| Q8WXF1 | PSPC1 | HUMAN | 12 | n_36_v | 3 | 6.895E-03 |
| P62241 | RPS8 | HUMAN | 37 | n_36_v | 14 | 7.009E-03 |

| Protein IDs | gname | species | target | type | backgrou | adj.p |
|---|---|---|---|---|---|---|
| P15880 | RPS2 | HUMAN | 51 | n_36_v | 21 | 7.769E-03 |
| P08621 | SNRNP70 | HUMAN | 9 | n_36_v | 2 | 8.533E-03 |
| P62701;P22090;Q8 | RPS4X | HUMAN | 19 | n_36_v | 6 | 9.999E-03 |
| Q14258 | TRIM25 | HUMAN | 14 | n_36_v | 4 | 1.188E-02 |
| Q15366;P57721 | PCBP2 | HUMAN | 16 | n_36_v | 5 | 1.696E-02 |
| P18124 | RPL7 | HUMAN | 27 | n_36_v | 10 | 1.766E-02 |
| O00571 | DDX3X | HUMAN | 8 | n_36_v | 2 | 2.495E-02 |
| Q13243 | SRSF5 | HUMAN | 8 | n_36_v | 2 | 2.495E-02 |
| Q92900 | UPF1 | HUMAN | 8 | n_36_v | 2 | 2.495E-02 |
| Q9H0D6 | XRN2 | HUMAN | 8 | n_36_v | 2 | 2.495E-02 |
| Q9Y2W1 | THRAP3 | HUMAN | 8 | n_36_v | 2 | 2.495E-02 |
| Q07666;Q5VWX1 | KHDRBS1 | HUMAN | 13 | n_36_v | 4 | 2.495E-02 |
| 2751-3246 | NSP4 | CVHOC | 5 | n_36_v | 1 | 2.495E-02 |
| O00567 | NOP56 | HUMAN | 5 | n_36_v | 1 | 2.495E-02 |
| O75822 | EIF3J | HUMAN | 5 | n_36_v | 1 | 2.495E-02 |
| P09429;B2RPK0 | HMGB1 | HUMAN | 5 | n_36_v | 1 | 2.495E-02 |
| P19525 | EIF2AK2 | HUMAN | 5 | n_36_v | 1 | 2.495E-02 |
| P62318 | SNRPD3 | HUMAN | 5 | n_36_v | 1 | 2.495E-02 |
| Q06787 | FMR1 | HUMAN | 5 | n_36_v | 1 | 2.495E-02 |
| Q14157 | UBAP2L | HUMAN | 5 | n_36_v | 1 | 2.495E-02 |
| Q4G0J3 | LARP7 | HUMAN | 5 | n_36_v | 1 | 2.495E-02 |
| Q7Z7H8 | MRPL10 | HUMAN | 5 | n_36_v | 1 | 2.495E-02 |
| Q8N983 | MRPL43 | HUMAN | 5 | n_36_v | 1 | 2.495E-02 |
| Q8TAE8 | GADD45GIP1 | HUMAN | 5 | n_36_v | 1 | 2.495E-02 |
| Q8TB72 | PUM2 | HUMAN | 5 | n_36_v | 1 | 2.495E-02 |
| Q96EY7 | PTCD3 | HUMAN | 5 | n_36_v | 1 | 2.495E-02 |
| Q96GC5 | MRPL48 | HUMAN | 5 | n_36_v | 1 | 2.495E-02 |
| Q9BYD3 | MRPL4 | HUMAN | 5 | n_36_v | 1 | 2.495E-02 |
| Q9NX20 | MRPL16 | HUMAN | 5 | n_36_v | 1 | 2.495E-02 |
| Q9UHX1 | PUF60 | HUMAN | 5 | n_36_v | 1 | 2.495E-02 |
| Q7L2E3 | DHX30 | HUMAN | 15 | n_36_v | 5 | 3.184E-02 |
| P62847 | RPS24 | HUMAN | 10 | n_36_v | 3 | 4.122E-02 |
| P83731 | RPL24 | HUMAN | 19 | n_36_v | 7 | 4.176E-02 |
| P62280 | RPS11 | HUMAN | 23 | n_36_v | 9 | 4.676E-02 |
| P18621 | RPL17 | HUMAN | 14 | n_36_v | 5 | 6.601E-02 |
| Q969Q0;P83881 | RPL36AL | HUMAN | 7 | n_36_v | 2 | 6.639E-02 |
| Q92841 | DDX17 | HUMAN | 16 | n_36_v | 6 | 7.259E-02 |
| P61247 | RPS3A | HUMAN | 22 | n_36_v | 9 | 8.078E-02 |
| 247-851 | NSP2 | CVHOC | 4 | n_36_v | 1 | 8.375E-02 |
| O43670 | ZNF207 | HUMAN | 4 | n_36_v | 1 | 8.375E-02 |
| O60869 | EDF1 | HUMAN | 4 | n_36_v | 1 | 8.375E-02 |
| P00558 | PGK1 | HUMAN | 4 | n_36_v | 1 | 8.375E-02 |
| P62140 | PPP1CB | HUMAN | 4 | n_36_v | 1 | 8.375E-02 |
| Q5T6F2 | UBAP2 | HUMAN | 4 | n_36_v | 1 | 8.375E-02 |
| Q6P1L8 | MRPL14 | HUMAN | 4 | n_36_v | 1 | 8.375E-02 |
| Q8IUX4;Q9NRW3;C | APOBEC3F | HUMAN | 4 | n_36_v | 1 | 8.375E-02 |
| Q8WVM0 | TFB1M | HUMAN | 4 | n_36_v | 1 | 8.375E-02 |
| Q92615 | LARP4B | HUMAN | 4 | n_36_v | 1 | 8.375E-02 |
| Q93077;Q7L7L0;P0 | HIST1H2AC | HUMAN | 4 | n_36_v | 1 | 8.375E-02 |
| Q96DH6 | MSI2 | HUMAN | 4 | n_36_v | 1 | 8.375E-02 |
| Q99700 | ATXN2 | HUMAN | 4 | n_36_v | 1 | 8.375E-02 |
| Q9BWF3;Q9BQ04 | RBM4 | HUMAN | 4 | n_36_v | 1 | 8.375E-02 |
| Q9BZE4 | GTPBP4 | HUMAN | 4 | n_36_v | 1 | 8.375E-02 |
| Q9HD20 | ATP13A1 | HUMAN | 4 | n_36_v | 1 | 8.375E-02 |
| Q9NQ50 | MRPL40 | HUMAN | 4 | n_36_v | 1 | 8.375E-02 |
| Q9UH17;P31941 | APOBEC3B | HUMAN | 4 | n_36_v | 1 | 8.375E-02 |
| Q9Y3I0 | RTCB | HUMAN | 4 | n_36_v | 1 | 8.375E-02 |
| Q9Y3Y2 | CHTOP | HUMAN | 4 | n_36_v | 1 | 8.375E-02 |
| REV__Q9Y2B1 | NA | HUMAN | 4 | n_36_v | 1 | 8.375E-02 |
| P62750 | RPL23A | HUMAN | 9 | n_36_v | 3 | 8.402E-02 |
| P62937 | PPIA | HUMAN | 9 | n_36_v | 3 | 8.402E-02 |
| Q14498 | RBM39 | HUMAN | 9 | n_36_v | 3 | 8.402E-02 |
| Q8NC51 | SERBP1 | HUMAN | 9 | n_36_v | 3 | 8.402E-02 |
| Q96T37 | RBM15 | HUMAN | 9 | n_36_v | 3 | 8.402E-02 |

| Protein IDs | gname | species | target | type | backgrou | adj.p |
|---|---|---|---|---|---|---|
| P33469 | N | CVHOC | 439 | n_48_v | 38 | 2.033E-176 |
| P51991 | HNRNPA3 | HUMAN | 52 | n_48_v | 1 | 3.606E-52 |
| Q92945 | KHSRP | HUMAN | 90 | n_48_v | 5 | 5.553E-51 |
| P22626 | HNRNPA2B1 | HUMAN | 98 | n_48_v | 7 | 3.599E-46 |
| P14866 | HNRNPL | HUMAN | 59 | n_48_v | 2 | 1.699E-45 |
| O60506 | SYNCRIP | HUMAN | 88 | n_48_v | 7 | 3.992E-38 |
| Q15717;Q12926;P2 | ELAVL1 | HUMAN | 50 | n_48_v | 2 | 2.066E-35 |
| P09651;Q32P51 | HNRNPA1 | HUMAN | 82 | n_48_v | 7 | 1.504E-33 |
| O43390 | HNRNPR | HUMAN | 63 | n_48_v | 4 | 5.709E-33 |
| Q00839 | HNRNPU | HUMAN | 80 | n_48_v | 8 | 2.696E-28 |
| P42704;Q9NP80 | LRPPRC | HUMAN | 156 | n_48_v | 28 | 1.033E-25 |
| Q96I24 | FUBP3 | HUMAN | 47 | n_48_v | 3 | 8.476E-25 |
| Q12906 | ILF3 | HUMAN | 38 | n_48_v | 2 | 3.914E-23 |
| Q99729 | HNRNPAB | HUMAN | 37 | n_48_v | 2 | 3.470E-22 |
| P52272 | HNRNPM | HUMAN | 65 | n_48_v | 7 | 1.416E-21 |
| P43243 | MATR3 | HUMAN | 41 | n_48_v | 3 | 1.067E-19 |
| Q1KMD3 | HNRNPUL2 | HUMAN | 33 | n_48_v | 2 | 1.735E-18 |
| Q9HCE1 | MOV10 | HUMAN | 25 | n_48_v | 1 | 1.735E-18 |
| P61978 | HNRNPK | HUMAN | 64 | n_48_v | 8 | 4.102E-18 |
| P26599 | PTBP1 | HUMAN | 49 | n_48_v | 5 | 1.766E-17 |
| Q9UKM9 | RALY | HUMAN | 31 | n_48_v | 2 | 9.893E-17 |
| P36334 | S | CVHOC | 23 | n_48_v | 1 | 2.110E-16 |
| Q8N163 | CCAR2 | HUMAN | 23 | n_48_v | 1 | 2.110E-16 |
| Q15233 | NONO | HUMAN | 36 | n_48_v | 3 | 8.450E-16 |
| Q9NZB2;Q5T035 | FAM120A | HUMAN | 29 | n_48_v | 2 | 4.944E-15 |
| P31943 | HNRNPH1 | HUMAN | 49 | n_48_v | 6 | 1.679E-14 |
| O75534 | CSDE1 | HUMAN | 20 | n_48_v | 1 | 2.463E-13 |
| Q8WXF1 | PSPC1 | HUMAN | 20 | n_48_v | 1 | 2.463E-13 |
| A0AV96;Q8TBY0;Q | RBM47 | HUMAN | 26 | n_48_v | 2 | 1.476E-12 |
| Q14103 | HNRNPD | HUMAN | 26 | n_48_v | 2 | 1.476E-12 |
| P07910;O60812;B2I | HNRNPC | HUMAN | 92 | n_48_v | 19 | 3.255E-12 |
| P52597 | HNRNPF | HUMAN | 36 | n_48_v | 4 | 3.655E-12 |
| P17844 | DDX5 | HUMAN | 30 | n_48_v | 3 | 1.967E-11 |
| Q07666;Q5VWX1 | KHDRBS1 | HUMAN | 18 | n_48_v | 1 | 1.983E-11 |
| Q96EP5 | DAZAP1 | HUMAN | 18 | n_48_v | 1 | 1.983E-11 |
| P38159;O75526;Q8 | RBMX | HUMAN | 17 | n_48_v | 1 | 1.656E-10 |
| P98179 | RBM3 | HUMAN | 17 | n_48_v | 1 | 1.656E-10 |
| Q07955 | SRSF1 | HUMAN | 17 | n_48_v | 1 | 1.656E-10 |
| Q7Z2W4 | ZC3HAV1 | HUMAN | 17 | n_48_v | 1 | 1.656E-10 |
| P11940;Q9H361;Q9 | PABPC1 | HUMAN | 23 | n_48_v | 2 | 2.868E-10 |
| Q13148 | TARDBP | HUMAN | 28 | n_48_v | 3 | 3.948E-10 |
| Q92879;O95319 | CELF1 | HUMAN | 16 | n_48_v | 1 | 1.356E-09 |
| P62633 | CNBP | HUMAN | 22 | n_48_v | 2 | 1.572E-09 |
| Q01455 | M | CVHOC | 30 | n_48_v | 4 | 1.535E-08 |
| 852-2750 | NSP3 | CVHOC | 29 | n_48_v | 4 | 5.731E-08 |
| Q96T37 | RBM15 | HUMAN | 14 | n_48_v | 1 | 8.108E-08 |
| Q96PK6 | RBM14 | HUMAN | 19 | n_48_v | 2 | 2.270E-07 |
| P23246 | SFPQ | HUMAN | 39 | n_48_v | 7 | 3.056E-07 |
| Q92900 | UPF1 | HUMAN | 13 | n_48_v | 1 | 5.610E-07 |
| Q5T653 | MRPL2 | HUMAN | 18 | n_48_v | 2 | 1.031E-06 |
| Q96AE4 | FUBP1 | HUMAN | 18 | n_48_v | 2 | 1.031E-06 |
| P12956 | XRCC6 | HUMAN | 12 | n_48_v | 1 | 3.321E-06 |
| Q15637 | SF1 | HUMAN | 12 | n_48_v | 1 | 3.321E-06 |
| Q8WWM7 | ATXN2L | HUMAN | 12 | n_48_v | 1 | 3.321E-06 |
| Q92499 | DDX1 | HUMAN | 12 | n_48_v | 1 | 3.321E-06 |
| Q9BZE1 | MRPL37 | HUMAN | 12 | n_48_v | 1 | 3.321E-06 |
| Q9NVS2 | MRPS18A | HUMAN | 12 | n_48_v | 1 | 3.321E-06 |
| P31942 | HNRNPH3 | HUMAN | 16 | n_48_v | 2 | 1.825E-05 |
| Q00341 | HDLBP | HUMAN | 16 | n_48_v | 2 | 1.825E-05 |
| Q9GZT3 | SLIRP | HUMAN | 16 | n_48_v | 2 | 1.825E-05 |
| Q01085 | TIAL1 | HUMAN | 11 | n_48_v | 1 | 1.838E-05 |
| Q13310 | PABPC4 | HUMAN | 11 | n_48_v | 1 | 1.838E-05 |
| Q15424 | SAFB | HUMAN | 11 | n_48_v | 1 | 1.838E-05 |
| Q92804 | TAF15 | HUMAN | 11 | n_48_v | 1 | 1.838E-05 |

| Protein IDs | gname | species | target | type | backgrou | adj.p |
|---|---|---|---|---|---|---|
| Q9BUJ2 | HNRNPUL1 | HUMAN | 11 | n_48_v | 1 | 1.838E-05 |
| Q9NWH9 | SLTM | HUMAN | 11 | n_48_v | 1 | 1.838E-05 |
| Q08211 | DHX9 | HUMAN | 24 | n_48_v | 4 | 1.887E-05 |
| P61247 | RPS3A | HUMAN | 31 | n_48_v | 6 | 2.138E-05 |
| O00425;Q9NZI8 | IGF2BP3 | HUMAN | 15 | n_48_v | 2 | 6.674E-05 |
| P13010 | XRCC5 | HUMAN | 10 | n_48_v | 1 | 9.845E-05 |
| P55795 | HNRNPH2 | HUMAN | 10 | n_48_v | 1 | 9.845E-05 |
| P62937 | PPIA | HUMAN | 10 | n_48_v | 1 | 9.845E-05 |
| Q13084 | MRPL28 | HUMAN | 10 | n_48_v | 1 | 9.845E-05 |
| Q86V81 | ALYREF | HUMAN | 18 | n_48_v | 3 | 2.305E-04 |
| O95758 | PTBP3 | HUMAN | 14 | n_48_v | 2 | 2.436E-04 |
| P27635;Q96L21 | RPL10 | HUMAN | 21 | n_48_v | 4 | 4.386E-04 |
| O75494;Q8WXF0 | SRSF10 | HUMAN | 9 | n_48_v | 1 | 4.449E-04 |
| O75822 | EIF3J | HUMAN | 9 | n_48_v | 1 | 4.449E-04 |
| P39019 | RPS19 | HUMAN | 9 | n_48_v | 1 | 4.449E-04 |
| P48634 | PRRC2A | HUMAN | 9 | n_48_v | 1 | 4.449E-04 |
| P62277 | RPS13 | HUMAN | 9 | n_48_v | 1 | 4.449E-04 |
| P82675 | MRPS5 | HUMAN | 9 | n_48_v | 1 | 4.449E-04 |
| Q14011 | CIRBP | HUMAN | 9 | n_48_v | 1 | 4.449E-04 |
| Q14151 | SAFB2 | HUMAN | 9 | n_48_v | 1 | 4.449E-04 |
| Q15056 | EIF4H | HUMAN | 9 | n_48_v | 1 | 4.449E-04 |
| Q96AG4 | LRRC59 | HUMAN | 9 | n_48_v | 1 | 4.449E-04 |
| Q9H0D6 | XRN2 | HUMAN | 9 | n_48_v | 1 | 4.449E-04 |
| Q9UHX1 | PUF60 | HUMAN | 9 | n_48_v | 1 | 4.449E-04 |
| O14979 | HNRNPDL | HUMAN | 17 | n_48_v | 3 | 6.038E-04 |
| Q9BYD1 | MRPL13 | HUMAN | 13 | n_48_v | 2 | 7.568E-04 |
| Q04837 | SSBP1 | HUMAN | 16 | n_48_v | 3 | 1.767E-03 |
| P10599 | TXN | HUMAN | 8 | n_48_v | 1 | 1.986E-03 |
| P19525 | EIF2AK2 | HUMAN | 8 | n_48_v | 1 | 1.986E-03 |
| P26368 | U2AF2 | HUMAN | 8 | n_48_v | 1 | 1.986E-03 |
| P35637 | FUS | HUMAN | 8 | n_48_v | 1 | 1.986E-03 |
| Q01844 | EWSR1 | HUMAN | 8 | n_48_v | 1 | 1.986E-03 |
| Q14152 | EIF3A | HUMAN | 8 | n_48_v | 1 | 1.986E-03 |
| Q9BYK8 | HELZ2 | HUMAN | 8 | n_48_v | 1 | 1.986E-03 |
| P19338 | NCL | HUMAN | 77 | n_48_v | 25 | 2.191E-03 |
| P11387;Q969P6 | TOP1 | HUMAN | 12 | n_48_v | 2 | 2.304E-03 |
| P49207 | RPL34 | HUMAN | 12 | n_48_v | 2 | 2.304E-03 |
| Q13151 | HNRNPA0 | HUMAN | 12 | n_48_v | 2 | 2.304E-03 |
| Q6PKG0 | LARP1 | HUMAN | 19 | n_48_v | 4 | 2.304E-03 |
| Q8NC51 | SERBP1 | HUMAN | 19 | n_48_v | 4 | 2.304E-03 |
| Q7KZF4 | SND1 | HUMAN | 25 | n_48_v | 6 | 2.940E-03 |
| P62917 | RPL8 | HUMAN | 67 | n_48_v | 22 | 5.912E-03 |
| P55265 | ADAR | HUMAN | 11 | n_48_v | 2 | 7.237E-03 |
| P51116 | FXR2 | HUMAN | 7 | n_48_v | 1 | 7.477E-03 |
| Q00577 | PURA | HUMAN | 7 | n_48_v | 1 | 7.477E-03 |
| Q80872 | 2a | CVHOC | 7 | n_48_v | 1 | 7.477E-03 |
| Q8NE71 | ABCF1 | HUMAN | 7 | n_48_v | 1 | 7.477E-03 |
| Q9BRJ2 | MRPL45 | HUMAN | 7 | n_48_v | 1 | 7.477E-03 |
| Q9BY77 | POLDIP3 | HUMAN | 7 | n_48_v | 1 | 7.477E-03 |
| Q9P015 | MRPL15 | HUMAN | 7 | n_48_v | 1 | 7.477E-03 |
| Q9P0M9 | MRPL27 | HUMAN | 7 | n_48_v | 1 | 7.477E-03 |
| Q9Y3I0 | RTCB | HUMAN | 7 | n_48_v | 1 | 7.477E-03 |
| Q9Y6M1 | IGF2BP2 | HUMAN | 7 | n_48_v | 1 | 7.477E-03 |
| P13645;CON__P13 | KRT10 | HUMAN | 14 | n_48_v | 3 | 1.018E-02 |
| P84103 | SRSF3 | HUMAN | 14 | n_48_v | 3 | 1.018E-02 |
| Q12905 | ILF2 | HUMAN | 14 | n_48_v | 3 | 1.018E-02 |
| P62888 | RPL30 | HUMAN | 10 | n_48_v | 2 | 1.887E-02 |
| P62995 | TRA2B | HUMAN | 10 | n_48_v | 2 | 1.887E-02 |
| Q5BKZ1 | ZNF326 | HUMAN | 10 | n_48_v | 2 | 1.887E-02 |
| Q9Y2W1 | THRAP3 | HUMAN | 10 | n_48_v | 2 | 1.887E-02 |
| O15371 | EIF3D | HUMAN | 6 | n_48_v | 1 | 2.514E-02 |
| O60832 | DKC1 | HUMAN | 6 | n_48_v | 1 | 2.514E-02 |
| P25398 | RPS12 | HUMAN | 6 | n_48_v | 1 | 2.514E-02 |
| Q13247 | SRSF6 | HUMAN | 6 | n_48_v | 1 | 2.514E-02 |

| Protein IDs | gname | species | target | type | backgrou | adj.p |
|---|---|---|---|---|---|---|
| Q14157 | UBAP2L | HUMAN | 6 | n_48_v | 1 | 2.514E-02 |
| Q14671 | PUM1 | HUMAN | 6 | n_48_v | 1 | 2.514E-02 |
| Q14694 | USP10 | HUMAN | 6 | n_48_v | 1 | 2.514E-02 |
| Q4G0J3 | LARP7 | HUMAN | 6 | n_48_v | 1 | 2.514E-02 |
| Q6P1L8 | MRPL14 | HUMAN | 6 | n_48_v | 1 | 2.514E-02 |
| Q8IUX4;Q9NRW3;C | APOBEC3F | HUMAN | 6 | n_48_v | 1 | 2.514E-02 |
| Q8N983 | MRPL43 | HUMAN | 6 | n_48_v | 1 | 2.514E-02 |
| Q96GC5 | MRPL48 | HUMAN | 6 | n_48_v | 1 | 2.514E-02 |
| Q99575 | POP1 | HUMAN | 6 | n_48_v | 1 | 2.514E-02 |
| Q9BWF3;Q9BQ04 | RBM4 | HUMAN | 6 | n_48_v | 1 | 2.514E-02 |
| Q9H6T0;Q6NXG1 | ESRP2 | HUMAN | 6 | n_48_v | 1 | 2.514E-02 |
| Q9Y3Y2 | CHTOP | HUMAN | 6 | n_48_v | 1 | 2.514E-02 |
| P18124 | RPL7 | HUMAN | 29 | n_48_v | 9 | 4.050E-02 |
| P22087 | FBL | HUMAN | 9 | n_48_v | 2 | 4.532E-02 |
| P23588 | EIF4B | HUMAN | 9 | n_48_v | 2 | 4.532E-02 |
| P62841 | RPS15 | HUMAN | 9 | n_48_v | 2 | 4.532E-02 |
| Q9P2E9 | RRBP1 | HUMAN | 17 | n_48_v | 5 | 7.875E-02 |
| 247-851 | NSP2 | CVHOC | 5 | n_48_v | 1 | 7.875E-02 |
| 3926-4122 | NSP8 | CVHOC | 5 | n_48_v | 1 | 7.875E-02 |
| O00567 | NOP56 | HUMAN | 5 | n_48_v | 1 | 7.875E-02 |
| P09001 | MRPL3 | HUMAN | 5 | n_48_v | 1 | 7.875E-02 |
| P26038 | MSN | HUMAN | 5 | n_48_v | 1 | 7.875E-02 |
| Q13435 | SF3B2 | HUMAN | 5 | n_48_v | 1 | 7.875E-02 |
| Q13573 | SNW1 | HUMAN | 5 | n_48_v | 1 | 7.875E-02 |
| Q13838 | DDX39B | HUMAN | 5 | n_48_v | 1 | 7.875E-02 |
| Q8IY67 | RAVER1 | HUMAN | 5 | n_48_v | 1 | 7.875E-02 |
| Q96QR8;Q9UJV8 | PURB | HUMAN | 5 | n_48_v | 1 | 7.875E-02 |
| Q9BYD3 | MRPL4 | HUMAN | 5 | n_48_v | 1 | 7.875E-02 |
| Q9H089 | LSG1 | HUMAN | 5 | n_48_v | 1 | 7.875E-02 |
| Q9NX20 | MRPL16 | HUMAN | 5 | n_48_v | 1 | 7.875E-02 |
| Q9Y3D9 | MRPS23 | HUMAN | 5 | n_48_v | 1 | 7.875E-02 |
| P62280 | RPS11 | HUMAN | 27 | n_48_v | 9 | 9.892E-02 |

For the OC43 Probe II experiment, 119, 189, 194, and 185 proteins were overrepresented at each respective infection time point of 12, 24, 36, and 48 hpi (FDR < 10%, Table 4).

[Table 4]

| Protein IDs | gname | species | target | type | background | adj.p |
|---|---|---|---|---|---|---|
| P33469 | N | CVHOC | 148 | n_12_v | 1 | 3.530E-236 |
| Q04837 | SSBP1 | HUMAN | 44 | n_12_v | 1 | 6.879E-48 |
| P11387;Q969P6 | TOP1 | HUMAN | 31 | n_12_v | 1 | 1.790E-29 |
| Q7KZF4 | SND1 | HUMAN | 27 | n_12_v | 1 | 2.664E-24 |
| P09651;Q32P51 | HNRNPA1 | HUMAN | 24 | n_12_v | 1 | 1.389E-20 |
| P19338 | NCL | HUMAN | 41 | n_12_v | 4 | 1.860E-20 |
| P07910;O60812;I | HNRNPC | HUMAN | 30 | n_12_v | 2 | 9.022E-20 |
| P52272 | HNRNPM | HUMAN | 22 | n_12_v | 1 | 2.495E-18 |
| P22626 | HNRNPA2B1 | HUMAN | 28 | n_12_v | 2 | 7.845E-18 |
| O60506 | SYNCRIP | HUMAN | 33 | n_12_v | 3 | 1.010E-17 |
| P09874 | PARP1 | HUMAN | 21 | n_12_v | 1 | 2.888E-17 |
| Q00839 | HNRNPU | HUMAN | 24 | n_12_v | 2 | 4.848E-14 |
| P42704;Q9NP80 | LRPPRC | HUMAN | 84 | n_12_v | 22 | 1.391E-13 |
| Q92945 | KHSRP | HUMAN | 23 | n_12_v | 2 | 3.603E-13 |
| P26599 | PTBP1 | HUMAN | 17 | n_12_v | 1 | 8.533E-13 |
| Q01455 | M | CVHOC | 16 | n_12_v | 1 | 9.971E-12 |
| P51991 | HNRNPA3 | HUMAN | 14 | n_12_v | 1 | 1.225E-09 |
| P62917 | RPL8 | HUMAN | 26 | n_12_v | 4 | 3.202E-09 |
| O43390 | HNRNPR | HUMAN | 13 | n_12_v | 1 | 1.140E-08 |
| P14866 | HNRNPL | HUMAN | 12 | n_12_v | 1 | 1.002E-07 |
| P27694 | RPA1 | HUMAN | 12 | n_12_v | 1 | 1.002E-07 |
| P26373 | RPL13 | HUMAN | 16 | n_12_v | 2 | 2.438E-07 |
| P23396 | RPS3 | HUMAN | 11 | n_12_v | 1 | 7.034E-07 |
| P43243 | MATR3 | HUMAN | 11 | n_12_v | 1 | 7.034E-07 |
| Q00341 | HDLBP | HUMAN | 11 | n_12_v | 1 | 7.034E-07 |
| Q96I24 | FUBP3 | HUMAN | 11 | n_12_v | 1 | 7.034E-07 |
| Q9GZT3 | SLIRP | HUMAN | 11 | n_12_v | 1 | 7.034E-07 |
| P15880 | RPS2 | HUMAN | 22 | n_12_v | 4 | 8.006E-07 |
| A0AV96;Q8TBY0 | RBM47 | HUMAN | 10 | n_12_v | 1 | 5.112E-06 |
| P46777 | RPL5 | HUMAN | 10 | n_12_v | 1 | 5.112E-06 |
| Q99729 | HNRNPAB | HUMAN | 10 | n_12_v | 1 | 5.112E-06 |
| P27635;Q96L21 | RPL10 | HUMAN | 9 | n_12_v | 1 | 3.571E-05 |
| Q02878 | RPL6 | HUMAN | 9 | n_12_v | 1 | 3.571E-05 |
| Q15717;Q12926; | ELAVL1 | HUMAN | 9 | n_12_v | 1 | 3.571E-05 |
| P61978 | HNRNPK | HUMAN | 16 | n_12_v | 3 | 3.900E-05 |
| P31943 | HNRNPH1 | HUMAN | 15 | n_12_v | 3 | 1.506E-04 |
| P17844 | DDX5 | HUMAN | 8 | n_12_v | 1 | 2.068E-04 |
| P18621 | RPL17 | HUMAN | 8 | n_12_v | 1 | 2.068E-04 |
| P23246 | SFPQ | HUMAN | 8 | n_12_v | 1 | 2.068E-04 |
| Q15365 | PCBP1 | HUMAN | 8 | n_12_v | 1 | 2.068E-04 |
| Q7Z2W4 | ZC3HAV1 | HUMAN | 8 | n_12_v | 1 | 2.068E-04 |
| O76021 | RSL1D1 | HUMAN | 7 | n_12_v | 1 | 1.028E-03 |
| P02545 | LMNA | HUMAN | 7 | n_12_v | 1 | 1.028E-03 |
| P09429;B2RPK0 | HMGB1 | HUMAN | 7 | n_12_v | 1 | 1.028E-03 |
| P11940;Q9H361; | PABPC1 | HUMAN | 7 | n_12_v | 1 | 1.028E-03 |
| P16403 | HIST1H1C | HUMAN | 7 | n_12_v | 1 | 1.028E-03 |
| P16989 | YBX3 | HUMAN | 7 | n_12_v | 1 | 1.028E-03 |
| P62269 | RPS18 | HUMAN | 7 | n_12_v | 1 | 1.028E-03 |
| P62750 | RPL23A | HUMAN | 7 | n_12_v | 1 | 1.028E-03 |
| P83731 | RPL24 | HUMAN | 7 | n_12_v | 1 | 1.028E-03 |
| Q12906 | ILF3 | HUMAN | 7 | n_12_v | 1 | 1.028E-03 |
| Q6PKG0 | LARP1 | HUMAN | 7 | n_12_v | 1 | 1.028E-03 |
| P52597 | HNRNPF | HUMAN | 13 | n_12_v | 3 | 1.351E-03 |
| P62805 | HIST1H4A | HUMAN | 27 | n_12_v | 9 | 1.591E-03 |
| Q13148 | TARDBP | HUMAN | 10 | n_12_v | 2 | 1.591E-03 |
| P36578 | RPL4 | HUMAN | 22 | n_12_v | 7 | 2.596E-03 |
| O14979 | HNRNPDL | HUMAN | 6 | n_12_v | 1 | 4.930E-03 |
| P31942 | HNRNPH3 | HUMAN | 6 | n_12_v | 1 | 4.930E-03 |
| P35637 | FUS | HUMAN | 6 | n_12_v | 1 | 4.930E-03 |
| Q1KMD3 | HNRNPUL2 | HUMAN | 6 | n_12_v | 1 | 4.930E-03 |
| Q96AE4 | FUBP1 | HUMAN | 6 | n_12_v | 1 | 4.930E-03 |

| Protein IDs | gname | species | target | type | background | adj.p |
|---|---|---|---|---|---|---|
| P18124 | RPL7 | HUMAN | 11 | n_12_v | 3 | 1.173E-02 |
| P62241 | RPS8 | HUMAN | 20 | n_12_v | 7 | 1.201E-02 |
| P16402 | HIST1H1D | HUMAN | 5 | n_12_v | 1 | 1.887E-02 |
| P26368 | U2AF2 | HUMAN | 5 | n_12_v | 1 | 1.887E-02 |
| P27816 | MAP4 | HUMAN | 5 | n_12_v | 1 | 1.887E-02 |
| P36334 | S | CVHOC | 5 | n_12_v | 1 | 1.887E-02 |
| P46781 | RPS9 | HUMAN | 5 | n_12_v | 1 | 1.887E-02 |
| P54098 | POLG | HUMAN | 5 | n_12_v | 1 | 1.887E-02 |
| P62633 | CNBP | HUMAN | 5 | n_12_v | 1 | 1.887E-02 |
| P62995 | TRA2B | HUMAN | 5 | n_12_v | 1 | 1.887E-02 |
| Q13151 | HNRNPA0 | HUMAN | 5 | n_12_v | 1 | 1.887E-02 |
| Q13283 | G3BP1 | HUMAN | 5 | n_12_v | 1 | 1.887E-02 |
| Q14980 | NUMA1 | HUMAN | 5 | n_12_v | 1 | 1.887E-02 |
| Q16629 | SRSF7 | HUMAN | 5 | n_12_v | 1 | 1.887E-02 |
| Q6P5R6 | RPL22L1 | HUMAN | 5 | n_12_v | 1 | 1.887E-02 |
| Q92804 | TAF15 | HUMAN | 5 | n_12_v | 1 | 1.887E-02 |
| Q96PK6 | RBM14 | HUMAN | 5 | n_12_v | 1 | 1.887E-02 |
| Q9NR56;Q9NUK( | MBNL1 | HUMAN | 5 | n_12_v | 1 | 1.887E-02 |
| CON__P13717 | NA | NA | 236 | n_12_v | 138 | 2.886E-02 |
| P08865 | RPSA | HUMAN | 7 | n_12_v | 2 | 4.532E-02 |
| P46782 | RPS5 | HUMAN | 7 | n_12_v | 2 | 4.532E-02 |
| P60866 | RPS20 | HUMAN | 7 | n_12_v | 2 | 4.532E-02 |
| Q02543 | RPL18A | HUMAN | 7 | n_12_v | 2 | 4.532E-02 |
| Q15287 | RNPS1 | HUMAN | 7 | n_12_v | 2 | 4.532E-02 |
| Q86V81 | ALYREF | HUMAN | 7 | n_12_v | 2 | 4.532E-02 |
| P07437 | TUBB | HUMAN | 11 | n_12_v | 4 | 5.764E-02 |
| O15371 | EIF3D | HUMAN | 4 | n_12_v | 1 | 5.764E-02 |
| O75367;Q9P0M6 | H2AFY | HUMAN | 4 | n_12_v | 1 | 5.764E-02 |
| P06748 | NPM1 | HUMAN | 4 | n_12_v | 1 | 5.764E-02 |
| P29372 | MPG | HUMAN | 4 | n_12_v | 1 | 5.764E-02 |
| P38159;O75526; | RBMX | HUMAN | 4 | n_12_v | 1 | 5.764E-02 |
| P42167;P42166 | TMPO | HUMAN | 4 | n_12_v | 1 | 5.764E-02 |
| P53999 | SUB1 | HUMAN | 4 | n_12_v | 1 | 5.764E-02 |
| P62263 | RPS14 | HUMAN | 4 | n_12_v | 1 | 5.764E-02 |
| P62829 | RPL23 | HUMAN | 4 | n_12_v | 1 | 5.764E-02 |
| P62899 | RPL31 | HUMAN | 4 | n_12_v | 1 | 5.764E-02 |
| Q07955 | SRSF1 | HUMAN | 4 | n_12_v | 1 | 5.764E-02 |
| Q12905 | ILF2 | HUMAN | 4 | n_12_v | 1 | 5.764E-02 |
| Q13523 | PRPF4B | HUMAN | 4 | n_12_v | 1 | 5.764E-02 |
| Q14152 | EIF3A | HUMAN | 4 | n_12_v | 1 | 5.764E-02 |
| Q15233 | NONO | HUMAN | 4 | n_12_v | 1 | 5.764E-02 |
| Q8WWM7 | ATXN2L | HUMAN | 4 | n_12_v | 1 | 5.764E-02 |
| Q8WXF1 | PSPC1 | HUMAN | 4 | n_12_v | 1 | 5.764E-02 |
| Q9BUJ2 | HNRNPUL1 | HUMAN | 4 | n_12_v | 1 | 5.764E-02 |
| Q9BYD1 | MRPL13 | HUMAN | 4 | n_12_v | 1 | 5.764E-02 |
| Q9NVV4 | MTPAP | HUMAN | 4 | n_12_v | 1 | 5.764E-02 |
| Q9NZB2;Q5T035 | FAM120A | HUMAN | 4 | n_12_v | 1 | 5.764E-02 |
| Q9UKM9 | RALY | HUMAN | 4 | n_12_v | 1 | 5.764E-02 |
| Q9UN86 | G3BP2 | HUMAN | 4 | n_12_v | 1 | 5.764E-02 |
| Q9Y5B9 | SUPT16H | HUMAN | 4 | n_12_v | 1 | 5.764E-02 |
| P16401 | HIST1H1B | HUMAN | 17 | n_12_v | 7 | 5.792E-02 |
| P05141;Q9H0C2 | SLC25A5 | HUMAN | 6 | n_12_v | 2 | 9.486E-02 |
| P11166 | SLC2A1 | HUMAN | 6 | n_12_v | 2 | 9.486E-02 |
| P35268 | RPL22 | HUMAN | 6 | n_12_v | 2 | 9.486E-02 |
| P46776 | RPL27A | HUMAN | 6 | n_12_v | 2 | 9.486E-02 |
| P84103 | SRSF3 | HUMAN | 6 | n_12_v | 2 | 9.486E-02 |
| Q14498 | RBM39 | HUMAN | 6 | n_12_v | 2 | 9.486E-02 |
| Q7L0Y3 | TRMT10C | HUMAN | 6 | n_12_v | 2 | 9.486E-02 |

| Protein IDs | gname | species | target | type | background | adj.p |
|---|---|---|---|---|---|---|
| P33469 | N | CVHOC | 211 | n_24_v | 2 | 2.35E-310 |
| Q7KZF4 | SND1 | HUMAN | 37 | n_24_v | 1 | 2.322E-38 |
| P52272 | HNRNPM | HUMAN | 34 | n_24_v | 1 | 3.060E-34 |
| P26599 | PTBP1 | HUMAN | 33 | n_24_v | 1 | 5.871E-33 |
| O60506 | SYNCRIP | HUMAN | 55 | n_24_v | 5 | 1.223E-29 |
| P22626 | HNRNPA2B1 | HUMAN | 43 | n_24_v | 3 | 6.776E-28 |
| Q96I24 | FUBP3 | HUMAN | 27 | n_24_v | 1 | 4.935E-25 |
| P14866 | HNRNPL | HUMAN | 25 | n_24_v | 1 | 1.741E-22 |
| P31943 | HNRNPH1 | HUMAN | 36 | n_24_v | 3 | 4.508E-21 |
| Q92945 | KHSRP | HUMAN | 47 | n_24_v | 6 | 1.252E-19 |
| CON__P08779;P | KRT16 | NA | 22 | n_24_v | 1 | 7.687E-19 |
| P42704;Q9NP80 | LRPPRC | HUMAN | 98 | n_24_v | 25 | 8.230E-18 |
| P52597 | HNRNPF | HUMAN | 27 | n_24_v | 2 | 1.903E-17 |
| P07910;O60812;I | HNRNPC | HUMAN | 40 | n_24_v | 5 | 2.918E-17 |
| P09651;Q32P51 | HNRNPA1 | HUMAN | 31 | n_24_v | 3 | 1.226E-16 |
| P43243 | MATR3 | HUMAN | 20 | n_24_v | 1 | 1.342E-16 |
| Q13148 | TARDBP | HUMAN | 20 | n_24_v | 1 | 1.342E-16 |
| O75534 | CSDE1 | HUMAN | 19 | n_24_v | 1 | 1.943E-15 |
| Q00839 | HNRNPU | HUMAN | 40 | n_24_v | 6 | 1.002E-14 |
| O43390 | HNRNPR | HUMAN | 24 | n_24_v | 2 | 1.082E-14 |
| P15924 | DSP | HUMAN | 24 | n_24_v | 2 | 1.082E-14 |
| Q00341 | HDLBP | HUMAN | 24 | n_24_v | 2 | 1.082E-14 |
| A0AV96;Q8TBY0 | RBM47 | HUMAN | 18 | n_24_v | 1 | 1.965E-14 |
| P11940;Q9H361; | PABPC1 | HUMAN | 18 | n_24_v | 1 | 1.965E-14 |
| Q99729 | HNRNPAB | HUMAN | 18 | n_24_v | 1 | 1.965E-14 |
| Q9NZB2;Q5T035 | FAM120A | HUMAN | 18 | n_24_v | 1 | 1.965E-14 |
| Q14103 | HNRNPD | HUMAN | 17 | n_24_v | 1 | 2.532E-13 |
| Q6PKG0 | LARP1 | HUMAN | 17 | n_24_v | 1 | 2.532E-13 |
| P51991 | HNRNPA3 | HUMAN | 22 | n_24_v | 2 | 6.592E-13 |
| P23246 | SFPQ | HUMAN | 16 | n_24_v | 1 | 3.108E-12 |
| P17844 | DDX5 | HUMAN | 21 | n_24_v | 2 | 5.192E-12 |
| Q04837 | SSBP1 | HUMAN | 25 | n_24_v | 3 | 7.951E-12 |
| P61978 | HNRNPK | HUMAN | 28 | n_24_v | 4 | 2.711E-11 |
| P31942 | HNRNPH3 | HUMAN | 15 | n_24_v | 1 | 3.310E-11 |
| Q9HCE1 | MOV10 | HUMAN | 15 | n_24_v | 1 | 3.310E-11 |
| Q12906 | ILF3 | HUMAN | 14 | n_24_v | 1 | 3.567E-10 |
| Q15233 | NONO | HUMAN | 14 | n_24_v | 1 | 3.567E-10 |
| Q9UKM9 | RALY | HUMAN | 14 | n_24_v | 1 | 3.567E-10 |
| Q08211 | DHX9 | HUMAN | 13 | n_24_v | 1 | 3.719E-09 |
| Q7Z2W4 | ZC3HAV1 | HUMAN | 13 | n_24_v | 1 | 3.719E-09 |
| Q15717;Q12926; | ELAVL1 | HUMAN | 17 | n_24_v | 2 | 1.243E-08 |
| 852-2750 | NSP3 | CVHOC | 12 | n_24_v | 1 | 3.315E-08 |
| P11387;Q969P6 | TOP1 | HUMAN | 12 | n_24_v | 1 | 3.315E-08 |
| P62633 | CNBP | HUMAN | 12 | n_24_v | 1 | 3.315E-08 |
| Q07955 | SRSF1 | HUMAN | 12 | n_24_v | 1 | 3.315E-08 |
| Q96AE4 | FUBP1 | HUMAN | 12 | n_24_v | 1 | 3.315E-08 |
| P15880 | RPS2 | HUMAN | 31 | n_24_v | 7 | 1.186E-07 |
| P62917 | RPL8 | HUMAN | 36 | n_24_v | 9 | 1.338E-07 |
| O00425;Q9NZI8 | IGF2BP3 | HUMAN | 11 | n_24_v | 1 | 2.641E-07 |
| P09874 | PARP1 | HUMAN | 11 | n_24_v | 1 | 2.641E-07 |
| P14923 | JUP | HUMAN | 11 | n_24_v | 1 | 2.641E-07 |
| P23396 | RPS3 | HUMAN | 11 | n_24_v | 1 | 2.641E-07 |
| P60866 | RPS20 | HUMAN | 11 | n_24_v | 1 | 2.641E-07 |
| P83731 | RPL24 | HUMAN | 11 | n_24_v | 1 | 2.641E-07 |
| Q1KMD3 | HNRNPUL2 | HUMAN | 11 | n_24_v | 1 | 2.641E-07 |
| Q5T749 | KPRP | HUMAN | 15 | n_24_v | 2 | 3.753E-07 |
| P19338 | NCL | HUMAN | 42 | n_24_v | 12 | 3.768E-07 |
| Q01455 | M | CVHOC | 18 | n_24_v | 3 | 7.666E-07 |
| Q02878 | RPL6 | HUMAN | 21 | n_24_v | 4 | 8.455E-07 |
| P47914 | RPL29 | HUMAN | 14 | n_24_v | 2 | 2.030E-06 |
| P16989 | YBX3 | HUMAN | 10 | n_24_v | 1 | 2.030E-06 |
| Q07666;Q5VWX | KHDRBS1 | HUMAN | 10 | n_24_v | 1 | 2.030E-06 |
| Q14498 | RBM39 | HUMAN | 10 | n_24_v | 1 | 2.030E-06 |

| Protein IDs | gname | species | target | type | background | adj.p |
|---|---|---|---|---|---|---|
| P62280 | RPS11 | HUMAN | 17 | n_24_v | 3 | 3.243E-06 |
| O14979 | HNRNPDL | HUMAN | 9 | n_24_v | 1 | 1.436E-05 |
| P11021 | HSPA5 | HUMAN | 9 | n_24_v | 1 | 1.436E-05 |
| P23588 | EIF4B | HUMAN | 9 | n_24_v | 1 | 1.436E-05 |
| P27635;Q96L21 | RPL10 | HUMAN | 9 | n_24_v | 1 | 1.436E-05 |
| P35637 | FUS | HUMAN | 9 | n_24_v | 1 | 1.436E-05 |
| Q14152 | EIF3A | HUMAN | 9 | n_24_v | 1 | 1.436E-05 |
| Q92841 | DDX17 | HUMAN | 9 | n_24_v | 1 | 1.436E-05 |
| Q9GZT3 | SLIRP | HUMAN | 9 | n_24_v | 1 | 1.436E-05 |
| P36578 | RPL4 | HUMAN | 33 | n_24_v | 10 | 2.191E-05 |
| P62081 | RPS7 | HUMAN | 12 | n_24_v | 2 | 4.772E-05 |
| P36334 | S | CVHOC | 8 | n_24_v | 1 | 8.752E-05 |
| P42766 | RPL35 | HUMAN | 8 | n_24_v | 1 | 8.752E-05 |
| P48634 | PRRC2A | HUMAN | 8 | n_24_v | 1 | 8.752E-05 |
| P62263 | RPS14 | HUMAN | 8 | n_24_v | 1 | 8.752E-05 |
| Q02413 | DSG1 | HUMAN | 8 | n_24_v | 1 | 8.752E-05 |
| Q07065 | CKAP4 | HUMAN | 8 | n_24_v | 1 | 8.752E-05 |
| Q15365 | PCBP1 | HUMAN | 8 | n_24_v | 1 | 8.752E-05 |
| Q15424 | SAFB | HUMAN | 8 | n_24_v | 1 | 8.752E-05 |
| Q8IWR0 | ZC3H7A | HUMAN | 8 | n_24_v | 1 | 8.752E-05 |
| Q8N163 | CCAR2 | HUMAN | 8 | n_24_v | 1 | 8.752E-05 |
| Q8WXF1 | PSPC1 | HUMAN | 8 | n_24_v | 1 | 8.752E-05 |
| Q92900 | UPF1 | HUMAN | 8 | n_24_v | 1 | 8.752E-05 |
| Q9BUJ2 | HNRNPUL1 | HUMAN | 8 | n_24_v | 1 | 8.752E-05 |
| P46777 | RPL5 | HUMAN | 22 | n_24_v | 6 | 1.122E-04 |
| P46781 | RPS9 | HUMAN | 11 | n_24_v | 2 | 1.908E-04 |
| O00571 | DDX3X | HUMAN | 7 | n_24_v | 1 | 5.062E-04 |
| O95758 | PTBP3 | HUMAN | 7 | n_24_v | 1 | 5.062E-04 |
| P12956 | XRCC6 | HUMAN | 7 | n_24_v | 1 | 5.062E-04 |
| Q01085 | TIAL1 | HUMAN | 7 | n_24_v | 1 | 5.062E-04 |
| Q12905 | ILF2 | HUMAN | 7 | n_24_v | 1 | 5.062E-04 |
| Q13151 | HNRNPA0 | HUMAN | 7 | n_24_v | 1 | 5.062E-04 |
| Q14011 | CIRBP | HUMAN | 7 | n_24_v | 1 | 5.062E-04 |
| Q92499 | DDX1 | HUMAN | 7 | n_24_v | 1 | 5.062E-04 |
| Q96EP5 | DAZAP1 | HUMAN | 7 | n_24_v | 1 | 5.062E-04 |
| Q96PK6 | RBM14 | HUMAN | 7 | n_24_v | 1 | 5.062E-04 |
| Q9BZE1 | MRPL37 | HUMAN | 7 | n_24_v | 1 | 5.062E-04 |
| P67809 | YBX1 | HUMAN | 13 | n_24_v | 3 | 5.593E-04 |
| P62701;P22090;C | RPS4X | HUMAN | 10 | n_24_v | 2 | 7.269E-04 |
| Q15366;P57721 | PCBP2 | HUMAN | 10 | n_24_v | 2 | 7.269E-04 |
| Q7L2E3 | DHX30 | HUMAN | 10 | n_24_v | 2 | 7.269E-04 |
| P46782 | RPS5 | HUMAN | 12 | n_24_v | 3 | 1.871E-03 |
| P35527;CON__P | KRT9 | HUMAN | 53 | n_24_v | 24 | 2.475E-03 |
| O15371 | EIF3D | HUMAN | 6 | n_24_v | 1 | 2.525E-03 |
| O75494;Q8WXF( | SRSF10 | HUMAN | 6 | n_24_v | 1 | 2.525E-03 |
| P09429;B2RPK0 | HMGB1 | HUMAN | 6 | n_24_v | 1 | 2.525E-03 |
| P38159;O75526;( | RBMX | HUMAN | 6 | n_24_v | 1 | 2.525E-03 |
| Q01844 | EWSR1 | HUMAN | 6 | n_24_v | 1 | 2.525E-03 |
| Q13310 | PABPC4 | HUMAN | 6 | n_24_v | 1 | 2.525E-03 |
| Q8NC51 | SERBP1 | HUMAN | 6 | n_24_v | 1 | 2.525E-03 |
| Q8WWM7 | ATXN2L | HUMAN | 6 | n_24_v | 1 | 2.525E-03 |
| Q92615 | LARP4B | HUMAN | 6 | n_24_v | 1 | 2.525E-03 |
| Q92879;O95319 | CELF1 | HUMAN | 6 | n_24_v | 1 | 2.525E-03 |
| Q9BZE4 | GTPBP4 | HUMAN | 6 | n_24_v | 1 | 2.525E-03 |
| Q9UGR2 | ZC3H7B | HUMAN | 6 | n_24_v | 1 | 2.525E-03 |
| Q9Y6M1 | IGF2BP2 | HUMAN | 6 | n_24_v | 1 | 2.525E-03 |
| P02545 | LMNA | HUMAN | 8 | n_24_v | 2 | 9.508E-03 |
| P62841 | RPS15 | HUMAN | 8 | n_24_v | 2 | 9.508E-03 |
| Q86V81 | ALYREF | HUMAN | 8 | n_24_v | 2 | 9.508E-03 |
| P18124 | RPL7 | HUMAN | 17 | n_24_v | 6 | 9.674E-03 |
| Q99613;B5ME19 | EIF3C | HUMAN | 5 | n_24_v | 1 | 1.121E-02 |
| O75822 | EIF3J | HUMAN | 5 | n_24_v | 1 | 1.121E-02 |
| P27694 | RPA1 | HUMAN | 5 | n_24_v | 1 | 1.121E-02 |

143

| Protein IDs | gname | species | target | type | background | adj.p |
|---|---|---|---|---|---|---|
| P53999 | SUB1 | HUMAN | 5 | n_24_v | 1 | 1.121E-02 |
| P60842;Q14240 | EIF4A1 | HUMAN | 5 | n_24_v | 1 | 1.121E-02 |
| P62906 | RPL10A | HUMAN | 5 | n_24_v | 1 | 1.121E-02 |
| P98179 | RBM3 | HUMAN | 5 | n_24_v | 1 | 1.121E-02 |
| Q13283 | G3BP1 | HUMAN | 5 | n_24_v | 1 | 1.121E-02 |
| Q14258 | TRIM25 | HUMAN | 5 | n_24_v | 1 | 1.121E-02 |
| Q14694 | USP10 | HUMAN | 5 | n_24_v | 1 | 1.121E-02 |
| Q15056 | EIF4H | HUMAN | 5 | n_24_v | 1 | 1.121E-02 |
| Q86U42;A6NDYC | PABPN1 | HUMAN | 5 | n_24_v | 1 | 1.121E-02 |
| Q86UE4 | MTDH | HUMAN | 5 | n_24_v | 1 | 1.121E-02 |
| Q92804 | TAF15 | HUMAN | 5 | n_24_v | 1 | 1.121E-02 |
| Q96T37 | RBM15 | HUMAN | 5 | n_24_v | 1 | 1.121E-02 |
| Q9H6T0;Q6NXG | ESRP2 | HUMAN | 5 | n_24_v | 1 | 1.121E-02 |
| Q9P258 | RCC2 | HUMAN | 5 | n_24_v | 1 | 1.121E-02 |
| Q9P2E9 | RRBP1 | HUMAN | 10 | n_24_v | 3 | 1.359E-02 |
| P04264;CON__P | KRT1 | HUMAN | 49 | n_24_v | 25 | 2.679E-02 |
| CON__P02533;P | KRT14 | NA | 7 | n_24_v | 2 | 2.679E-02 |
| P06748 | NPM1 | HUMAN | 7 | n_24_v | 2 | 2.679E-02 |
| P49207 | RPL34 | HUMAN | 7 | n_24_v | 2 | 2.679E-02 |
| Q6P2Q9 | PRPF8 | HUMAN | 7 | n_24_v | 2 | 2.679E-02 |
| Q9NR30 | DDX21 | HUMAN | 7 | n_24_v | 2 | 2.679E-02 |
| P13639 | EEF2 | HUMAN | 19 | n_24_v | 8 | 3.386E-02 |
| P08865 | RPSA | HUMAN | 9 | n_24_v | 3 | 3.526E-02 |
| P18621 | RPL17 | HUMAN | 9 | n_24_v | 3 | 3.526E-02 |
| P50914 | RPL14 | HUMAN | 9 | n_24_v | 3 | 3.526E-02 |
| CON__P02538;P | KRT6A | NA | 4 | n_24_v | 1 | 3.858E-02 |
| CON__P35908;C | NA | NA | 4 | n_24_v | 1 | 3.858E-02 |
| O43251;Q9NWB | RBFOX2 | HUMAN | 4 | n_24_v | 1 | 3.858E-02 |
| O60869 | EDF1 | HUMAN | 4 | n_24_v | 1 | 3.858E-02 |
| P05023;P13637;F | ATP1A1 | HUMAN | 4 | n_24_v | 1 | 3.858E-02 |
| P22087 | FBL | HUMAN | 4 | n_24_v | 1 | 3.858E-02 |
| P32969 | RPL9 | HUMAN | 4 | n_24_v | 1 | 3.858E-02 |
| P46783;Q9NQ39 | RPS10 | HUMAN | 4 | n_24_v | 1 | 3.858E-02 |
| P55795 | HNRNPH2 | HUMAN | 4 | n_24_v | 1 | 3.858E-02 |
| P62249 | RPS16 | HUMAN | 4 | n_24_v | 1 | 3.858E-02 |
| Q04637;O43432 | EIF4G1 | HUMAN | 4 | n_24_v | 1 | 3.858E-02 |
| Q08945 | SSRP1 | HUMAN | 4 | n_24_v | 1 | 3.858E-02 |
| Q12849 | GRSF1 | HUMAN | 4 | n_24_v | 1 | 3.858E-02 |
| Q13243 | SRSF5 | HUMAN | 4 | n_24_v | 1 | 3.858E-02 |
| Q13247 | SRSF6 | HUMAN | 4 | n_24_v | 1 | 3.858E-02 |
| Q13835 | PKP1 | HUMAN | 4 | n_24_v | 1 | 3.858E-02 |
| Q15084 | PDIA6 | HUMAN | 4 | n_24_v | 1 | 3.858E-02 |
| Q15637 | SF1 | HUMAN | 4 | n_24_v | 1 | 3.858E-02 |
| Q5BKZ1 | ZNF326 | HUMAN | 4 | n_24_v | 1 | 3.858E-02 |
| Q5T653 | MRPL2 | HUMAN | 4 | n_24_v | 1 | 3.858E-02 |
| Q8N1G4 | LRRC47 | HUMAN | 4 | n_24_v | 1 | 3.858E-02 |
| Q92736 | RYR2 | HUMAN | 4 | n_24_v | 1 | 3.858E-02 |
| Q96KR1 | ZFR | HUMAN | 4 | n_24_v | 1 | 3.858E-02 |
| Q9BRJ2 | MRPL45 | HUMAN | 4 | n_24_v | 1 | 3.858E-02 |
| Q9BWF3;Q9BQ0 | RBM4 | HUMAN | 4 | n_24_v | 1 | 3.858E-02 |
| Q9BYD1 | MRPL13 | HUMAN | 4 | n_24_v | 1 | 3.858E-02 |
| Q9BYD3 | MRPL4 | HUMAN | 4 | n_24_v | 1 | 3.858E-02 |
| Q9NVS2 | MRPS18A | HUMAN | 4 | n_24_v | 1 | 3.858E-02 |
| Q9NVV4 | MTPAP | HUMAN | 4 | n_24_v | 1 | 3.858E-02 |
| Q9UIG0 | BAZ1B | HUMAN | 4 | n_24_v | 1 | 3.858E-02 |
| Q9UN86 | G3BP2 | HUMAN | 4 | n_24_v | 1 | 3.858E-02 |
| Q9Y383;Q9NQ29 | LUC7L2 | HUMAN | 4 | n_24_v | 1 | 3.858E-02 |
| Q9Y3I0 | RTCB | HUMAN | 4 | n_24_v | 1 | 3.858E-02 |
| P35579;P35749 | MYH9 | HUMAN | 6 | n_24_v | 2 | 6.488E-02 |
| P40429;Q6NVV1 | RPL13A | HUMAN | 12 | n_24_v | 5 | 6.853E-02 |
| Q02543 | RPL18A | HUMAN | 12 | n_24_v | 5 | 6.853E-02 |
| P62266 | RPS23 | HUMAN | 10 | n_24_v | 4 | 7.180E-02 |
| P27816 | MAP4 | HUMAN | 8 | n_24_v | 3 | 7.180E-02 |

| Protein IDs | gname | species | target | type | background | adj.p |
|---|---|---|---|---|---|---|
| P33469 | N | CVHOC | 390 | n_36_v | 25 | 1.693E-239 |
| O60506 | SYNCRIP | HUMAN | 97 | n_36_v | 10 | 1.399E-41 |
| Q7KZF4 | SND1 | HUMAN | 57 | n_36_v | 3 | 1.374E-39 |
| Q00341 | HDLBP | HUMAN | 36 | n_36_v | 1 | 5.951E-35 |
| Q96I24 | FUBP3 | HUMAN | 42 | n_36_v | 2 | 3.135E-31 |
| P07910;O60812; | HNRNPC | HUMAN | 89 | n_36_v | 12 | 4.969E-30 |
| P52272 | HNRNPM | HUMAN | 73 | n_36_v | 9 | 4.639E-27 |
| Q92945 | KHSRP | HUMAN | 76 | n_36_v | 10 | 1.845E-26 |
| P14866 | HNRNPL | HUMAN | 42 | n_36_v | 3 | 1.396E-24 |
| P61978 | HNRNPK | HUMAN | 66 | n_36_v | 9 | 2.644E-22 |
| Q9HCE1 | MOV10 | HUMAN | 24 | n_36_v | 1 | 8.132E-20 |
| Q12906 | ILF3 | HUMAN | 31 | n_36_v | 2 | 9.929E-20 |
| Q15717;Q12926; | ELAVL1 | HUMAN | 40 | n_36_v | 4 | 2.239E-18 |
| P42704;Q9NP80 | LRPPRC | HUMAN | 126 | n_36_v | 32 | 5.954E-18 |
| P11940;Q9H361; | PABPC1 | HUMAN | 29 | n_36_v | 2 | 7.947E-18 |
| Q00839 | HNRNPU | HUMAN | 69 | n_36_v | 13 | 4.422E-16 |
| Q6PKG0 | LARP1 | HUMAN | 26 | n_36_v | 2 | 5.476E-15 |
| A0AV96;Q8TBY( | RBM47 | HUMAN | 31 | n_36_v | 3 | 6.211E-15 |
| Q9NZB2;Q5T035 | FAM120A | HUMAN | 19 | n_36_v | 1 | 3.083E-14 |
| P52597 | HNRNPF | HUMAN | 34 | n_36_v | 4 | 7.649E-14 |
| O43390 | HNRNPR | HUMAN | 52 | n_36_v | 9 | 8.658E-14 |
| P26599 | PTBP1 | HUMAN | 61 | n_36_v | 12 | 1.357E-13 |
| Q7Z2W4 | ZC3HAV1 | HUMAN | 18 | n_36_v | 1 | 3.233E-13 |
| P17844 | DDX5 | HUMAN | 33 | n_36_v | 4 | 3.552E-13 |
| P22626 | HNRNPA2B1 | HUMAN | 70 | n_36_v | 16 | 1.999E-12 |
| Q9UKM9 | RALY | HUMAN | 23 | n_36_v | 2 | 2.018E-12 |
| P43243 | MATR3 | HUMAN | 39 | n_36_v | 6 | 3.634E-12 |
| Q9BYK8 | HELZ2 | HUMAN | 16 | n_36_v | 1 | 3.662E-11 |
| O75534 | CSDE1 | HUMAN | 24 | n_36_v | 3 | 1.269E-09 |
| O00425;Q9NZI8 | IGF2BP3 | HUMAN | 14 | n_36_v | 1 | 3.313E-09 |
| Q14152 | EIF3A | HUMAN | 14 | n_36_v | 1 | 3.313E-09 |
| Q1KMD3 | HNRNPUL2 | HUMAN | 14 | n_36_v | 1 | 3.313E-09 |
| Q92879;O95319 | CELF1 | HUMAN | 14 | n_36_v | 1 | 3.313E-09 |
| Q9BYD1 | MRPL13 | HUMAN | 14 | n_36_v | 1 | 3.313E-09 |
| Q08211 | DHX9 | HUMAN | 27 | n_36_v | 4 | 3.814E-09 |
| Q86V81 | ALYREF | HUMAN | 19 | n_36_v | 2 | 3.814E-09 |
| P09651;Q32P51 | HNRNPA1 | HUMAN | 39 | n_36_v | 8 | 1.242E-08 |
| Q14103 | HNRNPD | HUMAN | 26 | n_36_v | 4 | 1.592E-08 |
| Q14011 | CIRBP | HUMAN | 13 | n_36_v | 1 | 2.688E-08 |
| Q96EP5 | DAZAP1 | HUMAN | 13 | n_36_v | 1 | 2.688E-08 |
| P51991 | HNRNPA3 | HUMAN | 25 | n_36_v | 4 | 6.365E-08 |
| Q8N163 | CCAR2 | HUMAN | 12 | n_36_v | 1 | 2.229E-07 |
| Q9GZT3 | SLIRP | HUMAN | 12 | n_36_v | 1 | 2.229E-07 |
| Q01455 | M | CVHOC | 27 | n_36_v | 5 | 3.510E-07 |
| O14979 | HNRNPDL | HUMAN | 20 | n_36_v | 3 | 5.096E-07 |
| Q92900 | UPF1 | HUMAN | 16 | n_36_v | 2 | 6.630E-07 |
| P62280 | RPS11 | HUMAN | 38 | n_36_v | 9 | 6.745E-07 |
| P62917 | RPL8 | HUMAN | 72 | n_36_v | 23 | 8.226E-07 |
| Q99613;B5ME19 | EIF3C | HUMAN | 11 | n_36_v | 1 | 1.558E-06 |
| Q13310 | PABPC4 | HUMAN | 11 | n_36_v | 1 | 1.558E-06 |
| Q5T653 | MRPL2 | HUMAN | 11 | n_36_v | 1 | 1.558E-06 |
| P49207 | RPL34 | HUMAN | 19 | n_36_v | 3 | 1.976E-06 |
| P15880 | RPS2 | HUMAN | 66 | n_36_v | 21 | 2.100E-06 |
| P19338 | NCL | HUMAN | 77 | n_36_v | 26 | 2.406E-06 |
| P62995 | TRA2B | HUMAN | 15 | n_36_v | 2 | 3.014E-06 |
| P48634 | PRRC2A | HUMAN | 10 | n_36_v | 1 | 1.061E-05 |
| P98179 | RBM3 | HUMAN | 10 | n_36_v | 1 | 1.061E-05 |
| Q99729 | HNRNPAB | HUMAN | 21 | n_36_v | 4 | 1.061E-05 |
| P11387;Q969P6 | TOP1 | HUMAN | 14 | n_36_v | 2 | 1.392E-05 |
| P36334 | S | CVHOC | 14 | n_36_v | 2 | 1.392E-05 |
| P62633 | CNBP | HUMAN | 14 | n_36_v | 2 | 1.392E-05 |
| Q96AE4 | FUBP1 | HUMAN | 17 | n_36_v | 3 | 2.877E-05 |
| Q15233 | NONO | HUMAN | 23 | n_36_v | 5 | 3.265E-05 |
| 852-2750 | NSP3 | CVHOC | 20 | n_36_v | 4 | 3.397E-05 |

| Protein IDs | gname | species | target | type | background | adj.p |
|---|---|---|---|---|---|---|
| P23246 | SFPQ | HUMAN | 36 | n_36_v | 10 | 3.980E-05 |
| O00567 | NOP56 | HUMAN | 9 | n_36_v | 1 | 6.035E-05 |
| P23588 | EIF4B | HUMAN | 9 | n_36_v | 1 | 6.035E-05 |
| Q01085 | TIAL1 | HUMAN | 9 | n_36_v | 1 | 6.035E-05 |
| Q8IWR0 | ZC3H7A | HUMAN | 9 | n_36_v | 1 | 6.035E-05 |
| Q92499 | DDX1 | HUMAN | 9 | n_36_v | 1 | 6.035E-05 |
| Q9Y2T7 | YBX2 | HUMAN | 9 | n_36_v | 1 | 6.035E-05 |
| P27635;Q96L21 | RPL10 | HUMAN | 16 | n_36_v | 3 | 9.426E-05 |
| P31942 | HNRNPH3 | HUMAN | 16 | n_36_v | 3 | 9.426E-05 |
| P38159;O75526; | RBMX | HUMAN | 12 | n_36_v | 2 | 2.420E-04 |
| Q9BZE1 | MRPL37 | HUMAN | 12 | n_36_v | 2 | 2.420E-04 |
| Q9Y2W1 | THRAP3 | HUMAN | 12 | n_36_v | 2 | 2.420E-04 |
| P31943 | HNRNPH1 | HUMAN | 36 | n_36_v | 11 | 2.520E-04 |
| Q12905 | ILF2 | HUMAN | 15 | n_36_v | 3 | 3.159E-04 |
| Q8NC51 | SERBP1 | HUMAN | 15 | n_36_v | 3 | 3.159E-04 |
| O15371 | EIF3D | HUMAN | 8 | n_36_v | 1 | 3.159E-04 |
| O75822 | EIF3J | HUMAN | 8 | n_36_v | 1 | 3.159E-04 |
| P55795 | HNRNPH2 | HUMAN | 8 | n_36_v | 1 | 3.159E-04 |
| P62841 | RPS15 | HUMAN | 8 | n_36_v | 1 | 3.159E-04 |
| Q13283 | G3BP1 | HUMAN | 8 | n_36_v | 1 | 3.159E-04 |
| Q15056 | EIF4H | HUMAN | 8 | n_36_v | 1 | 3.159E-04 |
| Q92804 | TAF15 | HUMAN | 8 | n_36_v | 1 | 3.159E-04 |
| Q9P0M9 | MRPL27 | HUMAN | 8 | n_36_v | 1 | 3.159E-04 |
| P62241 | RPS8 | HUMAN | 41 | n_36_v | 14 | 8.335E-04 |
| P22087 | FBL | HUMAN | 11 | n_36_v | 2 | 8.665E-04 |
| Q07955 | SRSF1 | HUMAN | 14 | n_36_v | 3 | 9.512E-04 |
| Q8WXF1 | PSPC1 | HUMAN | 14 | n_36_v | 3 | 9.512E-04 |
| Q13148 | TARDBP | HUMAN | 19 | n_36_v | 5 | 1.583E-03 |
| P13010 | XRCC5 | HUMAN | 7 | n_36_v | 1 | 1.593E-03 |
| Q04637;O43432 | EIF4G1 | HUMAN | 7 | n_36_v | 1 | 1.593E-03 |
| Q15084 | PDIA6 | HUMAN | 7 | n_36_v | 1 | 1.593E-03 |
| Q15424 | SAFB | HUMAN | 7 | n_36_v | 1 | 1.593E-03 |
| Q4G0J3 | LARP7 | HUMAN | 7 | n_36_v | 1 | 1.593E-03 |
| Q8N1G4 | LRRC47 | HUMAN | 7 | n_36_v | 1 | 1.593E-03 |
| Q9NVS2 | MRPS18A | HUMAN | 7 | n_36_v | 1 | 1.593E-03 |
| Q9UHX1 | PUF60 | HUMAN | 7 | n_36_v | 1 | 1.593E-03 |
| Q15365 | PCBP1 | HUMAN | 16 | n_36_v | 4 | 2.098E-03 |
| P62937 | PPIA | HUMAN | 13 | n_36_v | 3 | 2.750E-03 |
| P63244 | GNB2L1 | HUMAN | 10 | n_36_v | 2 | 2.880E-03 |
| Q13243 | SRSF5 | HUMAN | 10 | n_36_v | 2 | 2.880E-03 |
| P61247 | RPS3A | HUMAN | 27 | n_36_v | 9 | 5.158E-03 |
| P26368 | U2AF2 | HUMAN | 6 | n_36_v | 1 | 7.170E-03 |
| P39019 | RPS19 | HUMAN | 6 | n_36_v | 1 | 7.170E-03 |
| Q14671 | PUM1 | HUMAN | 6 | n_36_v | 1 | 7.170E-03 |
| Q86TB9 | PATL1 | HUMAN | 6 | n_36_v | 1 | 7.170E-03 |
| Q86UE4 | MTDH | HUMAN | 6 | n_36_v | 1 | 7.170E-03 |
| Q96AG4 | LRRC59 | HUMAN | 6 | n_36_v | 1 | 7.170E-03 |
| Q9NWH9 | SLTM | HUMAN | 6 | n_36_v | 1 | 7.170E-03 |
| Q9NX24 | NHP2 | HUMAN | 6 | n_36_v | 1 | 7.170E-03 |
| Q9UN86 | G3BP2 | HUMAN | 6 | n_36_v | 1 | 7.170E-03 |
| Q9Y520 | PRRC2C | HUMAN | 6 | n_36_v | 1 | 7.170E-03 |
| Q07065 | CKAP4 | HUMAN | 12 | n_36_v | 3 | 7.281E-03 |
| Q13151 | HNRNPA0 | HUMAN | 12 | n_36_v | 3 | 7.281E-03 |
| P67809 | YBX1 | HUMAN | 17 | n_36_v | 5 | 7.936E-03 |
| Q01844 | EWSR1 | HUMAN | 9 | n_36_v | 2 | 9.005E-03 |
| Q16629 | SRSF7 | HUMAN | 14 | n_36_v | 4 | 1.274E-02 |
| P23396 | RPS3 | HUMAN | 16 | n_36_v | 5 | 1.807E-02 |
| P84103 | SRSF3 | HUMAN | 16 | n_36_v | 5 | 1.807E-02 |
| P12956 | XRCC6 | HUMAN | 11 | n_36_v | 3 | 1.932E-02 |
| P16989 | YBX3 | HUMAN | 11 | n_36_v | 3 | 1.932E-02 |
| P62750 | RPL23A | HUMAN | 11 | n_36_v | 3 | 1.932E-02 |
| O95758 | PTBP3 | HUMAN | 8 | n_36_v | 2 | 2.560E-02 |
| Q15637 | SF1 | HUMAN | 8 | n_36_v | 2 | 2.560E-02 |
| Q99575 | POP1 | HUMAN | 8 | n_36_v | 2 | 2.560E-02 |

| Protein IDs | gname | species | target | type | background | adj.p |
|---|---|---|---|---|---|---|
| Q13263 | TRIM28 | HUMAN | 13 | n_36_v | 4 | 2.560E-02 |
| O75494;Q8WXF SRSF10 | | HUMAN | 5 | n_36_v | 1 | 2.560E-02 |
| O75533 | SF3B1 | HUMAN | 5 | n_36_v | 1 | 2.560E-02 |
| P10599 | TXN | HUMAN | 5 | n_36_v | 1 | 2.560E-02 |
| P31483 | TIA1 | HUMAN | 5 | n_36_v | 1 | 2.560E-02 |
| P46783;Q9NQ39 | RPS10 | HUMAN | 5 | n_36_v | 1 | 2.560E-02 |
| P51114 | FXR1 | HUMAN | 5 | n_36_v | 1 | 2.560E-02 |
| P51116 | FXR2 | HUMAN | 5 | n_36_v | 1 | 2.560E-02 |
| Q00577 | PURA | HUMAN | 5 | n_36_v | 1 | 2.560E-02 |
| Q13084 | MRPL28 | HUMAN | 5 | n_36_v | 1 | 2.560E-02 |
| Q14151 | SAFB2 | HUMAN | 5 | n_36_v | 1 | 2.560E-02 |
| Q14157 | UBAP2L | HUMAN | 5 | n_36_v | 1 | 2.560E-02 |
| Q80872 | 2a | CVHOC | 5 | n_36_v | 1 | 2.560E-02 |
| Q8TDD1 | DDX54 | HUMAN | 5 | n_36_v | 1 | 2.560E-02 |
| Q9H6T0;Q6NXG ESRP2 | | HUMAN | 5 | n_36_v | 1 | 2.560E-02 |
| Q9HD20 | ATP13A1 | HUMAN | 5 | n_36_v | 1 | 2.560E-02 |
| Q9Y3I0 | RTCB | HUMAN | 5 | n_36_v | 1 | 2.560E-02 |
| Q9Y3Y2 | CHTOP | HUMAN | 5 | n_36_v | 1 | 2.560E-02 |
| Q9Y5W8 | SNX13 | HUMAN | 5 | n_36_v | 1 | 2.560E-02 |
| Q92841 | DDX17 | HUMAN | 17 | n_36_v | 6 | 3.962E-02 |
| Q8WWM7 | ATXN2L | HUMAN | 10 | n_36_v | 3 | 4.268E-02 |
| Q04837 | SSBP1 | HUMAN | 12 | n_36_v | 4 | 5.758E-02 |
| Q07666;Q5VWX KHDRBS1 | | HUMAN | 12 | n_36_v | 4 | 5.758E-02 |
| O00571 | DDX3X | HUMAN | 7 | n_36_v | 2 | 6.642E-02 |
| P08708 | RPS17 | HUMAN | 7 | n_36_v | 2 | 6.642E-02 |
| P35637 | FUS | HUMAN | 7 | n_36_v | 2 | 6.642E-02 |
| Q5BKZ1 | ZNF326 | HUMAN | 7 | n_36_v | 2 | 6.642E-02 |
| P62854;Q5JNZ5 | RPS26 | HUMAN | 14 | n_36_v | 5 | 6.642E-02 |
| Q96PK6 | RBM14 | HUMAN | 14 | n_36_v | 5 | 6.642E-02 |
| Q9P2E9 | RRBP1 | HUMAN | 16 | n_36_v | 6 | 7.366E-02 |
| P83731 | RPL24 | HUMAN | 18 | n_36_v | 7 | 7.817E-02 |
| P47914 | RPL29 | HUMAN | 22 | n_36_v | 9 | 7.834E-02 |
| O43251;Q9NWB RBFOX2 | | HUMAN | 4 | n_36_v | 1 | 7.834E-02 |
| O43823 | AKAP8 | HUMAN | 4 | n_36_v | 1 | 7.834E-02 |
| O76094 | SRP72 | HUMAN | 4 | n_36_v | 1 | 7.834E-02 |
| P0DMV9;P0DMV HSPA1B | | HUMAN | 4 | n_36_v | 1 | 7.834E-02 |
| P17987 | TCP1 | HUMAN | 4 | n_36_v | 1 | 7.834E-02 |
| P19525 | EIF2AK2 | HUMAN | 4 | n_36_v | 1 | 7.834E-02 |
| P26641 | EEF1G | HUMAN | 4 | n_36_v | 1 | 7.834E-02 |
| P62136 | PPP1CA | HUMAN | 4 | n_36_v | 1 | 7.834E-02 |
| Q14694 | USP10 | HUMAN | 4 | n_36_v | 1 | 7.834E-02 |
| Q15434 | RBMS2 | HUMAN | 4 | n_36_v | 1 | 7.834E-02 |
| Q5T6F2 | UBAP2 | HUMAN | 4 | n_36_v | 1 | 7.834E-02 |
| Q71RC2 | LARP4 | HUMAN | 4 | n_36_v | 1 | 7.834E-02 |
| Q7Z2W9 | MRPL21 | HUMAN | 4 | n_36_v | 1 | 7.834E-02 |
| Q8IUX4;Q9NRW APOBEC3F | | HUMAN | 4 | n_36_v | 1 | 7.834E-02 |
| Q8NCA5;Q52LJ( FAM98A | | HUMAN | 4 | n_36_v | 1 | 7.834E-02 |
| Q8WVM0 | TFB1M | HUMAN | 4 | n_36_v | 1 | 7.834E-02 |
| Q92615 | LARP4B | HUMAN | 4 | n_36_v | 1 | 7.834E-02 |
| Q92736 | RYR2 | HUMAN | 4 | n_36_v | 1 | 7.834E-02 |
| Q99700 | ATXN2 | HUMAN | 4 | n_36_v | 1 | 7.834E-02 |
| Q9BSJ8 | ESYT1 | HUMAN | 4 | n_36_v | 1 | 7.834E-02 |
| Q9BVP2 | GNL3 | HUMAN | 4 | n_36_v | 1 | 7.834E-02 |
| Q9BWF3;Q9BQ( RBM4 | | HUMAN | 4 | n_36_v | 1 | 7.834E-02 |
| Q9BY44 | EIF2A | HUMAN | 4 | n_36_v | 1 | 7.834E-02 |
| Q9BYD3 | MRPL4 | HUMAN | 4 | n_36_v | 1 | 7.834E-02 |
| Q9BZE4 | GTPBP4 | HUMAN | 4 | n_36_v | 1 | 7.834E-02 |
| Q9H7E9 | C8orf33 | HUMAN | 4 | n_36_v | 1 | 7.834E-02 |
| Q9NUL5 | C19orf66 | HUMAN | 4 | n_36_v | 1 | 7.834E-02 |
| Q9NWU5 | MRPL22 | HUMAN | 4 | n_36_v | 1 | 7.834E-02 |
| Q9P015 | MRPL15 | HUMAN | 4 | n_36_v | 1 | 7.834E-02 |
| Q9Y2X3 | NOP58 | HUMAN | 4 | n_36_v | 1 | 7.834E-02 |
| P62847 | RPS24 | HUMAN | 9 | n_36_v | 3 | 8.109E-02 |
| P07437 | TUBB | HUMAN | 11 | n_36_v | 4 | 9.697E-02 |

| Protein IDs | gname | species | target | type | background | adj.p |
|---|---|---|---|---|---|---|
| P27816 | MAP4 | HUMAN | 11 | n_36_v | 4 | 9.697E-02 |
| P62263 | RPS14 | HUMAN | 11 | n_36_v | 4 | 9.697E-02 |

| Protein IDs | gname | species | target | type | background | adj.p |
|---|---|---|---|---|---|---|
| P33469 | N | CVHOC | 359 | n_48_v | 38 | 2.664E-117 |
| Q96I24 | FUBP3 | HUMAN | 71 | n_48_v | 3 | 1.263E-47 |
| Q92945 | KHSRP | HUMAN | 86 | n_48_v | 5 | 5.775E-47 |
| O43390 | HNRNPR | HUMAN | 76 | n_48_v | 4 | 1.293E-44 |
| P51991 | HNRNPA3 | HUMAN | 44 | n_48_v | 1 | 2.366E-41 |
| O60506 | SYNCRIP | HUMAN | 87 | n_48_v | 7 | 4.242E-37 |
| P22626 | HNRNPA2B1 | HUMAN | 86 | n_48_v | 7 | 2.234E-36 |
| Q00341 | HDLBP | HUMAN | 50 | n_48_v | 2 | 2.590E-35 |
| Q9HCE1 | MOV10 | HUMAN | 37 | n_48_v | 1 | 1.924E-32 |
| P14866 | HNRNPL | HUMAN | 46 | n_48_v | 2 | 4.353E-31 |
| Q7KZF4 | SND1 | HUMAN | 64 | n_48_v | 6 | 4.398E-24 |
| Q00839 | HNRNPU | HUMAN | 72 | n_48_v | 8 | 9.017E-23 |
| Q12906 | ILF3 | HUMAN | 36 | n_48_v | 2 | 4.233E-21 |
| Q15717;Q12926; | ELAVL1 | HUMAN | 36 | n_48_v | 2 | 4.233E-21 |
| Q14103 | HNRNPD | HUMAN | 35 | n_48_v | 2 | 3.556E-20 |
| P52272 | HNRNPM | HUMAN | 61 | n_48_v | 7 | 8.022E-19 |
| Q9NZB2;Q5T035 | FAM120A | HUMAN | 33 | n_48_v | 2 | 2.340E-18 |
| P43243 | MATR3 | HUMAN | 39 | n_48_v | 3 | 5.751E-18 |
| P61978 | HNRNPK | HUMAN | 63 | n_48_v | 8 | 2.360E-17 |
| P31943 | HNRNPH1 | HUMAN | 53 | n_48_v | 6 | 8.885E-17 |
| Q7Z2W4 | ZC3HAV1 | HUMAN | 22 | n_48_v | 1 | 3.152E-15 |
| Q96EP5 | DAZAP1 | HUMAN | 22 | n_48_v | 1 | 3.152E-15 |
| O75534 | CSDE1 | HUMAN | 21 | n_48_v | 1 | 3.371E-14 |
| P09651;Q32P51 | HNRNPA1 | HUMAN | 53 | n_48_v | 7 | 4.085E-14 |
| P26599 | PTBP1 | HUMAN | 43 | n_48_v | 5 | 1.913E-13 |
| A0AV96;Q8TBY( | RBM47 | HUMAN | 27 | n_48_v | 2 | 2.675E-13 |
| Q99729 | HNRNPAB | HUMAN | 27 | n_48_v | 2 | 2.675E-13 |
| P38159;O75526; | RBMX | HUMAN | 20 | n_48_v | 1 | 2.675E-13 |
| Q07955 | SRSF1 | HUMAN | 20 | n_48_v | 1 | 2.675E-13 |
| Q8N163 | CCAR2 | HUMAN | 20 | n_48_v | 1 | 2.675E-13 |
| Q9BYK8 | HELZ2 | HUMAN | 20 | n_48_v | 1 | 2.675E-13 |
| P36334 | S | CVHOC | 19 | n_48_v | 1 | 2.647E-12 |
| P17844 | DDX5 | HUMAN | 31 | n_48_v | 3 | 4.581E-12 |
| Q15233 | NONO | HUMAN | 31 | n_48_v | 3 | 4.581E-12 |
| P11940;Q9H361; | PABPC1 | HUMAN | 25 | n_48_v | 2 | 9.979E-12 |
| Q1KMD3 | HNRNPUL2 | HUMAN | 25 | n_48_v | 2 | 9.979E-12 |
| P42704;Q9NP80 | LRPPRC | HUMAN | 117 | n_48_v | 28 | 4.878E-11 |
| Q9UKM9 | RALY | HUMAN | 24 | n_48_v | 2 | 5.992E-11 |
| P52597 | HNRNPF | HUMAN | 34 | n_48_v | 4 | 7.618E-11 |
| P07910;O60812; | HNRNPC | HUMAN | 88 | n_48_v | 19 | 1.176E-10 |
| P12956 | XRCC6 | HUMAN | 17 | n_48_v | 1 | 1.859E-10 |
| Q14152 | EIF3A | HUMAN | 16 | n_48_v | 1 | 1.591E-09 |
| Q08211 | DHX9 | HUMAN | 31 | n_48_v | 4 | 4.950E-09 |
| P31942 | HNRNPH3 | HUMAN | 21 | n_48_v | 2 | 1.014E-08 |
| Q96AE4 | FUBP1 | HUMAN | 21 | n_48_v | 2 | 1.014E-08 |
| Q92900 | UPF1 | HUMAN | 15 | n_48_v | 1 | 1.178E-08 |
| Q9BZE1 | MRPL37 | HUMAN | 15 | n_48_v | 1 | 1.178E-08 |
| Q07666;Q5VWX | KHDRBS1 | HUMAN | 14 | n_48_v | 1 | 8.644E-08 |
| Q8WXF1 | PSPC1 | HUMAN | 14 | n_48_v | 1 | 8.644E-08 |
| Q92879;O95319 | CELF1 | HUMAN | 14 | n_48_v | 1 | 8.644E-08 |
| Q9P2E9 | RRBP1 | HUMAN | 32 | n_48_v | 5 | 2.233E-07 |
| Q6PKG0 | LARP1 | HUMAN | 28 | n_48_v | 4 | 2.233E-07 |
| P62633 | CNBP | HUMAN | 19 | n_48_v | 2 | 2.377E-07 |
| P98179 | RBM3 | HUMAN | 13 | n_48_v | 1 | 5.863E-07 |
| Q01455 | M | CVHOC | 27 | n_48_v | 4 | 7.506E-07 |
| P13645;CON__P | KRT10 | HUMAN | 22 | n_48_v | 3 | 2.256E-06 |
| Q13148 | TARDBP | HUMAN | 22 | n_48_v | 3 | 2.256E-06 |
| P13010 | XRCC5 | HUMAN | 12 | n_48_v | 1 | 3.386E-06 |
| P26368 | U2AF2 | HUMAN | 12 | n_48_v | 1 | 3.386E-06 |
| P62937 | PPIA | HUMAN | 12 | n_48_v | 1 | 3.386E-06 |
| Q00577 | PURA | HUMAN | 12 | n_48_v | 1 | 3.386E-06 |
| Q14011 | CIRBP | HUMAN | 12 | n_48_v | 1 | 3.386E-06 |
| Q8WWM7 | ATXN2L | HUMAN | 12 | n_48_v | 1 | 3.386E-06 |
| Q9Y6M1 | IGF2BP2 | HUMAN | 12 | n_48_v | 1 | 3.386E-06 |

| Protein IDs | gname | species | target | type | background | adj.p |
|---|---|---|---|---|---|---|
| Q96PK6 | RBM14 | HUMAN | 17 | n_48_v | 2 | 4.420E-06 |
| P35637 | FUS | HUMAN | 11 | n_48_v | 1 | 1.943E-05 |
| Q01085 | TIAL1 | HUMAN | 11 | n_48_v | 1 | 1.943E-05 |
| Q13310 | PABPC4 | HUMAN | 11 | n_48_v | 1 | 1.943E-05 |
| Q92499 | DDX1 | HUMAN | 11 | n_48_v | 1 | 1.943E-05 |
| Q96T37 | RBM15 | HUMAN | 11 | n_48_v | 1 | 1.943E-05 |
| Q9BUJ2 | HNRNPUL1 | HUMAN | 11 | n_48_v | 1 | 1.943E-05 |
| P61247 | RPS3A | HUMAN | 31 | n_48_v | 6 | 2.446E-05 |
| O00425;Q9NZI8 | IGF2BP3 | HUMAN | 15 | n_48_v | 2 | 7.075E-05 |
| Q13283 | G3BP1 | HUMAN | 15 | n_48_v | 2 | 7.075E-05 |
| Q5T653 | MRPL2 | HUMAN | 15 | n_48_v | 2 | 7.075E-05 |
| P23246 | SFPQ | HUMAN | 33 | n_48_v | 7 | 8.060E-05 |
| Q99613;B5ME19 | EIF3C | HUMAN | 10 | n_48_v | 1 | 1.003E-04 |
| P55795 | HNRNPH2 | HUMAN | 10 | n_48_v | 1 | 1.003E-04 |
| Q01844 | EWSR1 | HUMAN | 10 | n_48_v | 1 | 1.003E-04 |
| Q15637 | SF1 | HUMAN | 10 | n_48_v | 1 | 1.003E-04 |
| Q9H0D6 | XRN2 | HUMAN | 10 | n_48_v | 1 | 1.003E-04 |
| P19338 | NCL | HUMAN | 83 | n_48_v | 25 | 1.936E-04 |
| O14979 | HNRNPDL | HUMAN | 18 | n_48_v | 3 | 2.384E-04 |
| P62841 | RPS15 | HUMAN | 14 | n_48_v | 2 | 2.491E-04 |
| 852-2750 | NSP3 | CVHOC | 21 | n_48_v | 4 | 4.578E-04 |
| O60832 | DKC1 | HUMAN | 9 | n_48_v | 1 | 4.584E-04 |
| O75494;Q8WXF( | SRSF10 | HUMAN | 9 | n_48_v | 1 | 4.584E-04 |
| P35579;P35749 | MYH9 | HUMAN | 9 | n_48_v | 1 | 4.584E-04 |
| P48634 | PRRC2A | HUMAN | 9 | n_48_v | 1 | 4.584E-04 |
| P51114 | FXR1 | HUMAN | 9 | n_48_v | 1 | 4.584E-04 |
| Q15056 | EIF4H | HUMAN | 9 | n_48_v | 1 | 4.584E-04 |
| Q15424 | SAFB | HUMAN | 9 | n_48_v | 1 | 4.584E-04 |
| Q4G0J3 | LARP7 | HUMAN | 9 | n_48_v | 1 | 4.584E-04 |
| Q8IWR0 | ZC3H7A | HUMAN | 9 | n_48_v | 1 | 4.584E-04 |
| Q9NVS2 | MRPS18A | HUMAN | 9 | n_48_v | 1 | 4.584E-04 |
| Q9UN86 | G3BP2 | HUMAN | 9 | n_48_v | 1 | 4.584E-04 |
| Q12905 | ILF2 | HUMAN | 17 | n_48_v | 3 | 6.323E-04 |
| Q86V81 | ALYREF | HUMAN | 17 | n_48_v | 3 | 6.323E-04 |
| P49207 | RPL34 | HUMAN | 13 | n_48_v | 2 | 7.678E-04 |
| Q13151 | HNRNPA0 | HUMAN | 13 | n_48_v | 2 | 7.678E-04 |
| Q9Y2W1 | THRAP3 | HUMAN | 13 | n_48_v | 2 | 7.678E-04 |
| P84103 | SRSF3 | HUMAN | 16 | n_48_v | 3 | 1.807E-03 |
| O15371 | EIF3D | HUMAN | 8 | n_48_v | 1 | 1.976E-03 |
| P19525 | EIF2AK2 | HUMAN | 8 | n_48_v | 1 | 1.976E-03 |
| P25398 | RPS12 | HUMAN | 8 | n_48_v | 1 | 1.976E-03 |
| P46783;Q9NQ39 | RPS10 | HUMAN | 8 | n_48_v | 1 | 1.976E-03 |
| Q92804 | TAF15 | HUMAN | 8 | n_48_v | 1 | 1.976E-03 |
| Q9H6T0;Q6NXG | ESRP2 | HUMAN | 8 | n_48_v | 1 | 1.976E-03 |
| Q9NWH9 | SLTM | HUMAN | 8 | n_48_v | 1 | 1.976E-03 |
| Q9Y2T7 | YBX2 | HUMAN | 8 | n_48_v | 1 | 1.976E-03 |
| O95758 | PTBP3 | HUMAN | 12 | n_48_v | 2 | 2.382E-03 |
| P62995 | TRA2B | HUMAN | 12 | n_48_v | 2 | 2.382E-03 |
| Q13243 | SRSF5 | HUMAN | 12 | n_48_v | 2 | 2.382E-03 |
| Q16629 | SRSF7 | HUMAN | 19 | n_48_v | 4 | 2.424E-03 |
| P27635;Q96L21 | RPL10 | HUMAN | 18 | n_48_v | 4 | 5.933E-03 |
| Q8NC51 | SERBP1 | HUMAN | 18 | n_48_v | 4 | 5.933E-03 |
| O00571 | DDX3X | HUMAN | 11 | n_48_v | 2 | 7.383E-03 |
| P55265 | ADAR | HUMAN | 11 | n_48_v | 2 | 7.383E-03 |
| O75822 | EIF3J | HUMAN | 7 | n_48_v | 1 | 7.473E-03 |
| P51116 | FXR2 | HUMAN | 7 | n_48_v | 1 | 7.473E-03 |
| P62277 | RPS13 | HUMAN | 7 | n_48_v | 1 | 7.473E-03 |
| Q06787 | FMR1 | HUMAN | 7 | n_48_v | 1 | 7.473E-03 |
| Q13084 | MRPL28 | HUMAN | 7 | n_48_v | 1 | 7.473E-03 |
| Q14151 | SAFB2 | HUMAN | 7 | n_48_v | 1 | 7.473E-03 |
| Q14694 | USP10 | HUMAN | 7 | n_48_v | 1 | 7.473E-03 |
| Q99575 | POP1 | HUMAN | 7 | n_48_v | 1 | 7.473E-03 |
| Q9P0M9 | MRPL27 | HUMAN | 7 | n_48_v | 1 | 7.473E-03 |
| Q9UGR2 | ZC3H7B | HUMAN | 7 | n_48_v | 1 | 7.473E-03 |

| Protein IDs | gname | species | target | type | background | adj.p |
|---|---|---|---|---|---|---|
| Q9UHX1 | PUF60 | HUMAN | 7 | n_48_v | 1 | 7.473E-03 |
| Q9Y3I0 | RTCB | HUMAN | 7 | n_48_v | 1 | 7.473E-03 |
| Q04837 | SSBP1 | HUMAN | 14 | n_48_v | 3 | 1.056E-02 |
| Q6P2Q9 | PRPF8 | HUMAN | 20 | n_48_v | 5 | 1.278E-02 |
| Q9GZT3 | SLIRP | HUMAN | 10 | n_48_v | 2 | 1.968E-02 |
| O43143 | DHX15 | HUMAN | 13 | n_48_v | 3 | 2.540E-02 |
| Q14258 | TRIM25 | HUMAN | 13 | n_48_v | 3 | 2.540E-02 |
| P67809 | YBX1 | HUMAN | 30 | n_48_v | 9 | 2.540E-02 |
| Q04637;O43432 | EIF4G1 | HUMAN | 6 | n_48_v | 1 | 2.540E-02 |
| Q13263 | TRIM28 | HUMAN | 6 | n_48_v | 1 | 2.540E-02 |
| Q13838 | DDX39B | HUMAN | 6 | n_48_v | 1 | 2.540E-02 |
| Q14671 | PUM1 | HUMAN | 6 | n_48_v | 1 | 2.540E-02 |
| Q15149 | PLEC | HUMAN | 6 | n_48_v | 1 | 2.540E-02 |
| Q86UE4 | MTDH | HUMAN | 6 | n_48_v | 1 | 2.540E-02 |
| Q8IUX4;Q9NRW | APOBEC3F | HUMAN | 6 | n_48_v | 1 | 2.540E-02 |
| Q92615 | LARP4B | HUMAN | 6 | n_48_v | 1 | 2.540E-02 |
| Q96EY7 | PTCD3 | HUMAN | 6 | n_48_v | 1 | 2.540E-02 |
| Q96KR1 | ZFR | HUMAN | 6 | n_48_v | 1 | 2.540E-02 |
| Q99700 | ATXN2 | HUMAN | 6 | n_48_v | 1 | 2.540E-02 |
| Q9BYD3 | MRPL4 | HUMAN | 6 | n_48_v | 1 | 2.540E-02 |
| Q9BZE4 | GTPBP4 | HUMAN | 6 | n_48_v | 1 | 2.540E-02 |
| Q9H3K6 | BOLA2 | HUMAN | 6 | n_48_v | 1 | 2.540E-02 |
| Q9NUL5 | C19orf66 | HUMAN | 6 | n_48_v | 1 | 2.540E-02 |
| Q9NX24 | NHP2 | HUMAN | 6 | n_48_v | 1 | 2.540E-02 |
| Q9Y3Y2 | CHTOP | HUMAN | 6 | n_48_v | 1 | 2.540E-02 |
| P23588 | EIF4B | HUMAN | 9 | n_48_v | 2 | 4.682E-02 |
| P62888 | RPL30 | HUMAN | 9 | n_48_v | 2 | 4.682E-02 |
| Q6ZNB6 | NFXL1 | HUMAN | 12 | n_48_v | 3 | 5.246E-02 |
| O00567 | NOP56 | HUMAN | 5 | n_48_v | 1 | 7.678E-02 |
| P09327 | VIL1 | HUMAN | 5 | n_48_v | 1 | 7.678E-02 |
| P17931 | LGALS3 | HUMAN | 5 | n_48_v | 1 | 7.678E-02 |
| P39019 | RPS19 | HUMAN | 5 | n_48_v | 1 | 7.678E-02 |
| Q05639 | EEF1A2 | HUMAN | 5 | n_48_v | 1 | 7.678E-02 |
| Q13435 | SF3B2 | HUMAN | 5 | n_48_v | 1 | 7.678E-02 |
| Q14697 | GANAB | HUMAN | 5 | n_48_v | 1 | 7.678E-02 |
| Q80872 | 2a | CVHOC | 5 | n_48_v | 1 | 7.678E-02 |
| Q86TB9 | PATL1 | HUMAN | 5 | n_48_v | 1 | 7.678E-02 |
| Q8IY67 | RAVER1 | HUMAN | 5 | n_48_v | 1 | 7.678E-02 |
| Q8N983 | MRPL43 | HUMAN | 5 | n_48_v | 1 | 7.678E-02 |
| Q8NCA5;Q52LJ( | FAM98A | HUMAN | 5 | n_48_v | 1 | 7.678E-02 |
| Q8NE71 | ABCF1 | HUMAN | 5 | n_48_v | 1 | 7.678E-02 |
| Q8WVV9 | HNRNPLL | HUMAN | 5 | n_48_v | 1 | 7.678E-02 |
| Q969S3 | ZNF622 | HUMAN | 5 | n_48_v | 1 | 7.678E-02 |
| Q96AG4 | LRRC59 | HUMAN | 5 | n_48_v | 1 | 7.678E-02 |
| Q96GC5 | MRPL48 | HUMAN | 5 | n_48_v | 1 | 7.678E-02 |
| Q96QR8;Q9UJV( | PURB | HUMAN | 5 | n_48_v | 1 | 7.678E-02 |
| Q9BY44 | EIF2A | HUMAN | 5 | n_48_v | 1 | 7.678E-02 |
| Q9BYN8 | MRPS26 | HUMAN | 5 | n_48_v | 1 | 7.678E-02 |
| Q9H0J9 | PARP12 | HUMAN | 5 | n_48_v | 1 | 7.678E-02 |
| Q9Y3D9 | MRPS23 | HUMAN | 5 | n_48_v | 1 | 7.678E-02 |
| Q9Y520 | PRRC2C | HUMAN | 5 | n_48_v | 1 | 7.678E-02 |
| P62917 | RPL8 | HUMAN | 59 | n_48_v | 22 | 9.002E-02 |
| P08621 | SNRNP70 | HUMAN | 8 | n_48_v | 2 | 9.981E-02 |
| P63244 | GNB2L1 | HUMAN | 8 | n_48_v | 2 | 9.981E-02 |
| Q5BKZ1 | ZNF326 | HUMAN | 8 | n_48_v | 2 | 9.981E-02 |
| Q86U42;A6NDY( | PABPN1 | HUMAN | 8 | n_48_v | 2 | 9.981E-02 |
| Q9BYD1 | MRPL13 | HUMAN | 8 | n_48_v | 2 | 9.981E-02 |

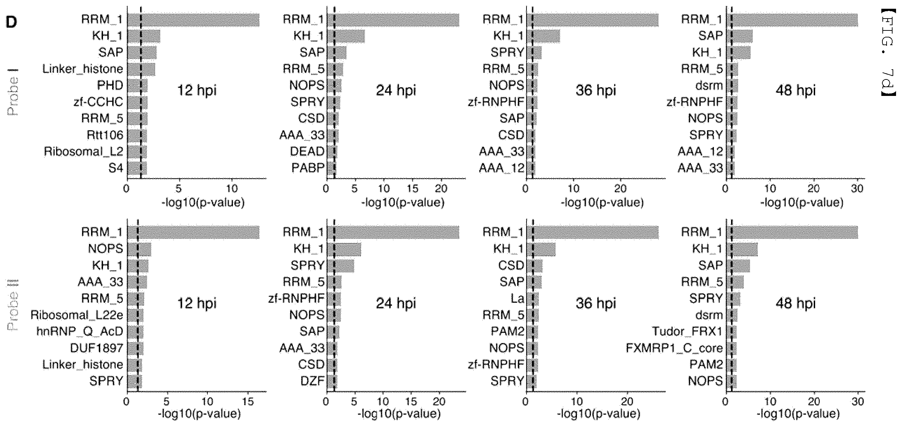

The analysis of all eight RNP capture experiments resulted in the enrichment of proteins containing canonical RNA binding domains such as the RRM domain and the KH domain (FIG. 7d), indicating the specificity of the coprecipitated RBPs.

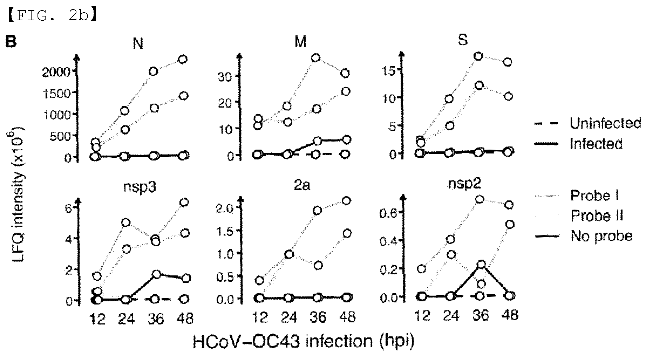

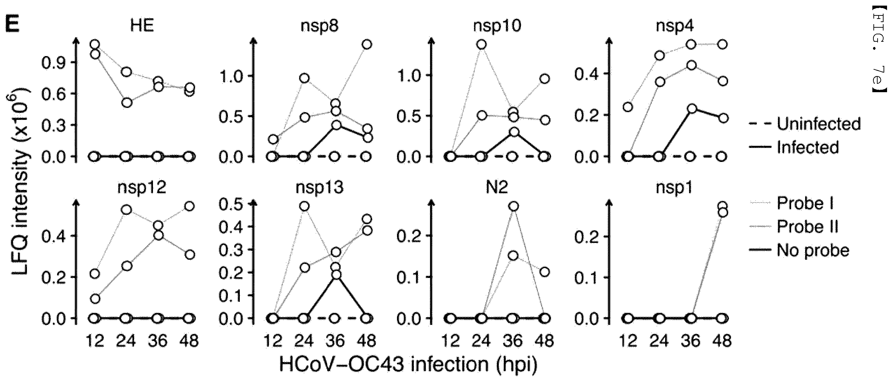

Fourteen viral proteins were detected within the HCoV-OC43 RNP complexes (FIG. 2b, FIG. 7e). Specifically, LFQ intensities for viral structural proteins N, M, and S increased over time more evidently in the gRNA-hybridizing Probe I experiment (FIG. 2b). HCoV-OC43 2a, an accessory protein unique to betacoronavirus lineage A, was also detected and increased over time, indicating that this protein of unknown function may act as an RBP. The RdRP nsp12 and the papain-like protease nsp3 also increased along with the other nsps identified in this experiment. Only a marginal amount of the HCoV-OC43 nsp1 was detected (FIG. 7e), implying the functional divergence of nsp1 in betacoronavirus lineages A and B.

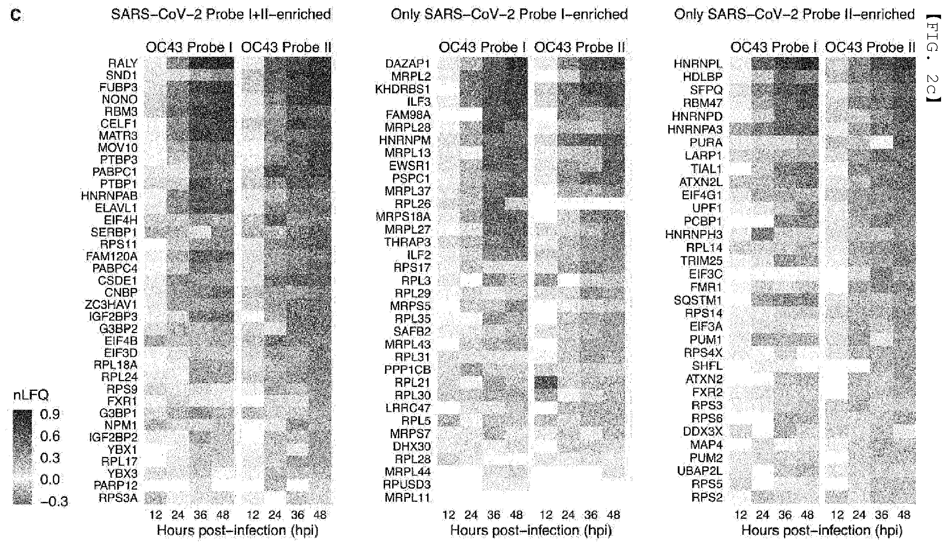

Next, the inventors compared the host factors that form the viral RNA interactome of HCoV-OC43 and SARS-CoV-2. All 107 proteins from the SARS-CoV-2 interactome were also detected in the HCoV-OC43 interactome throughout multiple infection timepoints, except for RBMS1 and DDX3Y (FIG. 2c), indicating that many of the same host proteins interact with RNAs of both HCoV-OC43 and SARS-CoV-2.

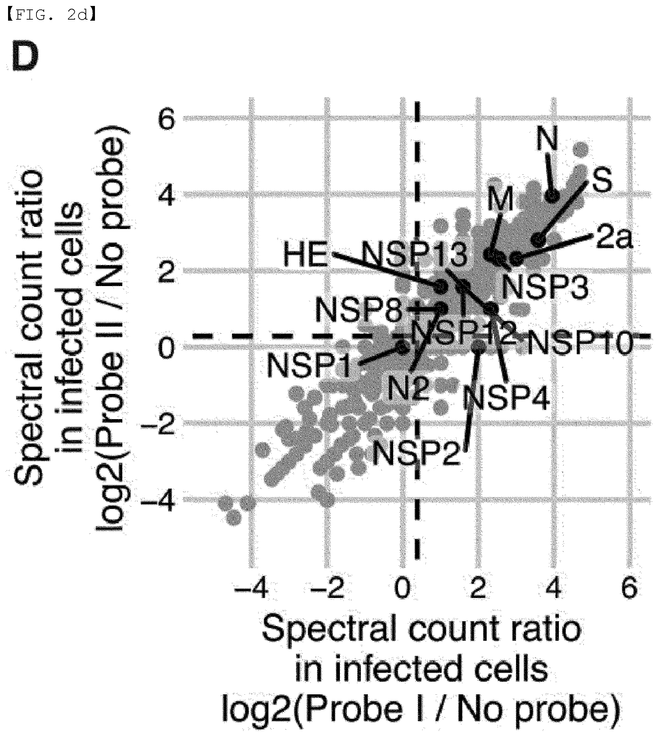

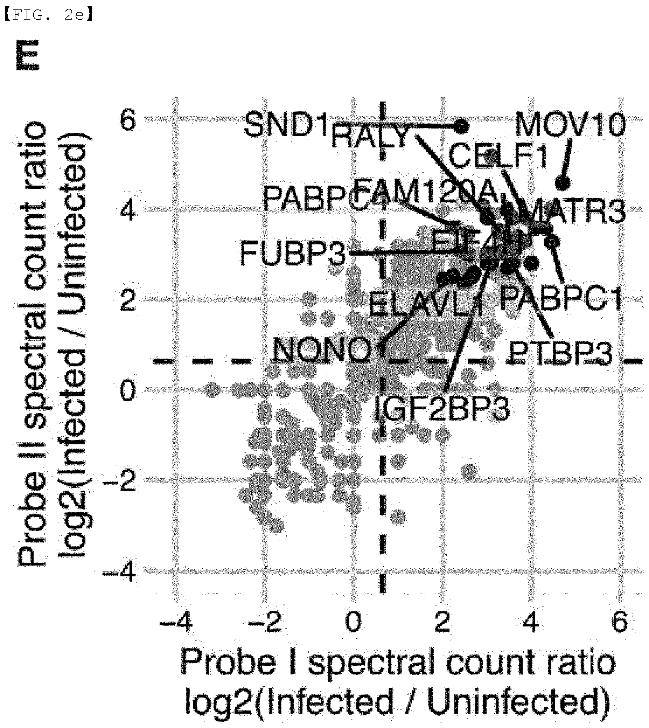

To determine the core host factors that are conserved in the coronavirus RNA interactomes, we applied our spectral count analysis on the HCoV-OC43 experiment of 36 hpi (FIG. 7a) and conducted statistical analysis in comparison to the noprobe control (FIG. 2d, FDR < 10%) and the uninfected-probe control (FIG. 2e, FDR < 5%).

The inventors identified 67 and 70 host proteins for the HCoV-OC43 Probe I and Probe II experiments, respectively (Table 5).

[Table 5]

| Protein IDs | gname | species | target | type | background | adj.p |
|---|---|---|---|---|---|---|
| P43243 | MATR3 | HUMAN | 57 | g_36_m | 4 | 4.555E-19 |
| Q9HCE1 | MOV10 | HUMAN | 26 | g_36_m | 1 | 5.504E-15 |
| P14866 | HNRNPL | HUMAN | 59 | g_36_m | 6 | 6.174E-13 |
| Q12906 | ILF3 | HUMAN | 39 | g_36_m | 3 | 2.209E-12 |
| P11940;Q9H361;Q96DU9 | PABPC1 | HUMAN | 22 | g_36_m | 1 | 9.191E-12 |
| Q9P2E9 | RRBP1 | HUMAN | 22 | g_36_m | 1 | 9.191E-12 |
| Q8N163 | CCAR2 | HUMAN | 20 | g_36_m | 1 | 4.352E-10 |
| Q9BZE1 | MRPL37 | HUMAN | 17 | g_36_m | 1 | 1.100E-07 |
| P17844 | DDX5 | HUMAN | 30 | g_36_m | 3 | 2.426E-07 |
| Q9NVS2 | MRPS18A | HUMAN | 16 | g_36_m | 1 | 5.245E-07 |
| Q9NZB2;Q5T035 | FAM120A | HUMAN | 23 | g_36_m | 2 | 5.843E-07 |
| Q9UKM9 | RALY | HUMAN | 29 | g_36_m | 3 | 6.255E-07 |
| Q92879;O95319 | CELF1 | HUMAN | 15 | g_36_m | 1 | 2.274E-06 |
| Q14258 | TRIM25 | HUMAN | 14 | g_36_m | 1 | 1.122E-05 |
| Q00341 | HDLBP | HUMAN | 26 | g_36_m | 3 | 1.669E-05 |
| Q96PK6 | RBM14 | HUMAN | 20 | g_36_m | 2 | 2.247E-05 |
| Q13084 | MRPL28 | HUMAN | 13 | g_36_m | 1 | 4.607E-05 |
| Q1KMD3 | HNRNPUL2 | HUMAN | 24 | g_36_m | 3 | 1.210E-04 |
| Q15717;Q12926;P26378 | ELAVL1 | HUMAN | 38 | g_36_m | 6 | 1.461E-04 |
| P48634 | PRRC2A | HUMAN | 12 | g_36_m | 1 | 1.461E-04 |
| P55795 | HNRNPH2 | HUMAN | 12 | g_36_m | 1 | 1.461E-04 |
| P62277 | RPS13 | HUMAN | 12 | g_36_m | 1 | 1.461E-04 |
| Q15084 | PDIA6 | HUMAN | 12 | g_36_m | 1 | 1.461E-04 |
| Q8WXF1 | PSPC1 | HUMAN | 12 | g_36_m | 1 | 1.461E-04 |
| Q92499 | DDX1 | HUMAN | 12 | g_36_m | 1 | 1.461E-04 |
| O00425;Q9NZI8 | IGF2BP3 | HUMAN | 17 | g_36_m | 2 | 4.807E-04 |
| Q96I24 | FUBP3 | HUMAN | 49 | g_36_m | 9 | 4.807E-04 |
| O95758 | PTBP3 | HUMAN | 11 | g_36_m | 1 | 4.807E-04 |
| Q14671 | PUM1 | HUMAN | 11 | g_36_m | 1 | 4.807E-04 |
| Q96EP5 | DAZAP1 | HUMAN | 11 | g_36_m | 1 | 4.807E-04 |
| Q9BYD1 | MRPL13 | HUMAN | 11 | g_36_m | 1 | 4.807E-04 |
| Q9BYK8 | HELZ2 | HUMAN | 11 | g_36_m | 1 | 4.807E-04 |
| Q9P015 | MRPL15 | HUMAN | 11 | g_36_m | 1 | 4.807E-04 |
| Q12905 | ILF2 | HUMAN | 16 | g_36_m | 2 | 1.189E-03 |
| P27635;Q96L21 | RPL10 | HUMAN | 21 | g_36_m | 3 | 1.199E-03 |
| P22087 | FBL | HUMAN | 10 | g_36_m | 1 | 1.636E-03 |
| P25398 | RPS12 | HUMAN | 10 | g_36_m | 1 | 1.636E-03 |
| P31483 | TIA1 | HUMAN | 10 | g_36_m | 1 | 1.636E-03 |
| Q9NWH9 | SLTM | HUMAN | 10 | g_36_m | 1 | 1.636E-03 |
| P62633 | CNBP | HUMAN | 15 | g_36_m | 2 | 2.754E-03 |
| Q13151 | HNRNPA0 | HUMAN | 15 | g_36_m | 2 | 2.754E-03 |
| Q96AE4 | FUBP1 | HUMAN | 15 | g_36_m | 2 | 2.754E-03 |
| A0AV96;Q8TBY0;Q9NQ94 | RBM47 | HUMAN | 28 | g_36_m | 5 | 4.908E-03 |
| P08621 | SNRNP70 | HUMAN | 9 | g_36_m | 1 | 4.923E-03 |
| Q96T37 | RBM15 | HUMAN | 9 | g_36_m | 1 | 4.923E-03 |
| Q9NZ01 | TECR | HUMAN | 9 | g_36_m | 1 | 4.923E-03 |
| Q9P0M9 | MRPL27 | HUMAN | 9 | g_36_m | 1 | 4.923E-03 |
| Q9Y383;Q9NQ29 | LUC7L2 | HUMAN | 9 | g_36_m | 1 | 4.923E-03 |
| P98179 | RBM3 | HUMAN | 14 | g_36_m | 2 | 6.381E-03 |
| Q5T653 | MRPL2 | HUMAN | 18 | g_36_m | 3 | 1.107E-02 |
| P16989 | YBX3 | HUMAN | 13 | g_36_m | 2 | 1.357E-02 |
| Q07666;Q5VWX1 | KHDRBS1 | HUMAN | 13 | g_36_m | 2 | 1.357E-02 |
| Q9BUJ2 | HNRNPUL1 | HUMAN | 13 | g_36_m | 2 | 1.357E-02 |
| P13010 | XRCC5 | HUMAN | 8 | g_36_m | 1 | 1.357E-02 |
| Q13243 | SRSF5 | HUMAN | 8 | g_36_m | 1 | 1.357E-02 |
| Q15056 | EIF4H | HUMAN | 8 | g_36_m | 1 | 1.357E-02 |
| Q9BRJ2 | MRPL45 | HUMAN | 8 | g_36_m | 1 | 1.357E-02 |
| Q9H0D6 | XRN2 | HUMAN | 8 | g_36_m | 1 | 1.357E-02 |
| Q9Y2W1 | THRAP3 | HUMAN | 8 | g_36_m | 1 | 1.357E-02 |

| Protein IDs | gname | species | target | type | background | adj.p |
|---|---|---|---|---|---|---|
| P23246 | SFPQ | HUMAN | 33 | g_36_m | 7 | 2.231E-02 |
| Q13310 | PABPC4 | HUMAN | 12 | g_36_m | 2 | 3.070E-02 |
| Q15637 | SF1 | HUMAN | 12 | g_36_m | 2 | 3.070E-02 |
| P61978 | HNRNPK | HUMAN | 65 | g_36_m | 16 | 3.332E-02 |
| Q15233 | NONO | HUMAN | 28 | g_36_m | 6 | 3.672E-02 |
| Q7KZF4 | SND1 | HUMAN | 16 | g_36_m | 3 | 3.672E-02 |
| Q13501 | SQSTM1 | HUMAN | 7 | g_36_m | 1 | 3.672E-02 |
| Q9BY77 | POLDIP3 | HUMAN | 7 | g_36_m | 1 | 3.672E-02 |

| Protein IDs | gname | species | target | type | background | adj.p |
|---|---|---|---|---|---|---|
| Q7KZF4 | SND1 | HUMAN | 57 | s_36_m | 1 | 1.044E-48 |
| Q00341 | HDLBP | HUMAN | 36 | s_36_m | 1 | 2.165E-24 |
| Q9HCE1 | MOV10 | HUMAN | 24 | s_36_m | 1 | 1.061E-12 |
| P23246 | SFPQ | HUMAN | 36 | s_36_m | 3 | 9.515E-10 |
| P17844 | DDX5 | HUMAN | 33 | s_36_m | 3 | 4.031E-08 |
| P43243 | MATR3 | HUMAN | 39 | s_36_m | 4 | 4.161E-08 |
| Q7Z2W4 | ZC3HAV1 | HUMAN | 18 | s_36_m | 1 | 5.299E-08 |
| Q96AE4 | FUBP1 | HUMAN | 17 | s_36_m | 1 | 2.740E-07 |
| Q12906 | ILF3 | HUMAN | 31 | s_36_m | 3 | 2.740E-07 |
| P14866 | HNRNPL | HUMAN | 42 | s_36_m | 5 | 4.151E-07 |
| Q96I24 | FUBP3 | HUMAN | 42 | s_36_m | 5 | 4.151E-07 |
| Q92900 | UPF1 | HUMAN | 16 | s_36_m | 1 | 8.832E-07 |
| Q9BYK8 | HELZ2 | HUMAN | 16 | s_36_m | 1 | 8.832E-07 |
| Q9P2E9 | RRBP1 | HUMAN | 16 | s_36_m | 1 | 8.832E-07 |
| Q9UKM9 | RALY | HUMAN | 23 | s_36_m | 2 | 1.280E-06 |
| P11940;Q9H361;Q96DU9 | PABPC1 | HUMAN | 29 | s_36_m | 3 | 1.671E-06 |
| P61247 | RPS3A | HUMAN | 27 | s_36_m | 3 | 1.417E-05 |
| Q08211 | DHX9 | HUMAN | 27 | s_36_m | 3 | 1.417E-05 |
| Q1KMD3 | HNRNPUL2 | HUMAN | 14 | s_36_m | 1 | 1.568E-05 |
| Q8WXF1 | PSPC1 | HUMAN | 14 | s_36_m | 1 | 1.568E-05 |
| Q92879;O95319 | CELF1 | HUMAN | 14 | s_36_m | 1 | 1.568E-05 |
| Q86V81 | ALYREF | HUMAN | 19 | s_36_m | 2 | 1.295E-04 |
| Q9NZB2;Q5T035 | FAM120A | HUMAN | 19 | s_36_m | 2 | 1.295E-04 |
| P61978 | HNRNPK | HUMAN | 66 | s_36_m | 12 | 2.042E-04 |
| O75534 | CSDE1 | HUMAN | 24 | s_36_m | 3 | 2.320E-04 |
| Q07065 | CKAP4 | HUMAN | 12 | s_36_m | 1 | 2.435E-04 |
| Q8N163 | CCAR2 | HUMAN | 12 | s_36_m | 1 | 2.435E-04 |
| Q9BZE1 | MRPL37 | HUMAN | 12 | s_36_m | 1 | 2.435E-04 |
| P22087 | FBL | HUMAN | 11 | s_36_m | 1 | 9.012E-04 |
| Q13310 | PABPC4 | HUMAN | 11 | s_36_m | 1 | 9.012E-04 |
| Q99613;B5ME19 | EIF3C | HUMAN | 11 | s_36_m | 1 | 9.012E-04 |
| Q15717;Q12926;P26378 | ELAVL1 | HUMAN | 40 | s_36_m | 7 | 2.046E-03 |
| P26599 | PTBP1 | HUMAN | 61 | s_36_m | 12 | 2.191E-03 |
| Q8WWM7 | ATXN2L | HUMAN | 10 | s_36_m | 1 | 3.120E-03 |
| P62995 | TRA2B | HUMAN | 15 | s_36_m | 2 | 6.209E-03 |
| P23588 | EIF4B | HUMAN | 9 | s_36_m | 1 | 9.047E-03 |
| Q01085 | TIAL1 | HUMAN | 9 | s_36_m | 1 | 9.047E-03 |
| Q01844 | EWSR1 | HUMAN | 9 | s_36_m | 1 | 9.047E-03 |
| Q8IWR0 | ZC3H7A | HUMAN | 9 | s_36_m | 1 | 9.047E-03 |
| Q92499 | DDX1 | HUMAN | 9 | s_36_m | 1 | 9.047E-03 |
| Q9Y2T7 | YBX2 | HUMAN | 9 | s_36_m | 1 | 9.047E-03 |
| O00425;Q9NZI8 | IGF2BP3 | HUMAN | 14 | s_36_m | 2 | 1.322E-02 |
| Q15233 | NONO | HUMAN | 23 | s_36_m | 4 | 1.723E-02 |
| O60506 | SYNCRIP | HUMAN | 97 | s_36_m | 23 | 2.158E-02 |
| A0AV96;Q8TBY0;Q9NQ94 | RBM47 | HUMAN | 31 | s_36_m | 6 | 2.158E-02 |
| O15371 | EIF3D | HUMAN | 8 | s_36_m | 1 | 2.158E-02 |
| O75822 | EIF3J | HUMAN | 8 | s_36_m | 1 | 2.158E-02 |
| O95758 | PTBP3 | HUMAN | 8 | s_36_m | 1 | 2.158E-02 |
| P55795 | HNRNPH2 | HUMAN | 8 | s_36_m | 1 | 2.158E-02 |
| P62841 | RPS15 | HUMAN | 8 | s_36_m | 1 | 2.158E-02 |
| Q15056 | EIF4H | HUMAN | 8 | s_36_m | 1 | 2.158E-02 |
| Q15637 | SF1 | HUMAN | 8 | s_36_m | 1 | 2.158E-02 |
| Q92804 | TAF15 | HUMAN | 8 | s_36_m | 1 | 2.158E-02 |
| Q99575 | POP1 | HUMAN | 8 | s_36_m | 1 | 2.158E-02 |
| Q9P0M9 | MRPL27 | HUMAN | 8 | s_36_m | 1 | 2.158E-02 |
| Q14011 | CIRBP | HUMAN | 13 | s_36_m | 2 | 2.344E-02 |
| Q96EP5 | DAZAP1 | HUMAN | 13 | s_36_m | 2 | 2.344E-02 |
| Q6PKG0 | LARP1 | HUMAN | 26 | s_36_m | 5 | 2.892E-02 |
| Q07666;Q5VWX1 | KHDRBS1 | HUMAN | 12 | s_36_m | 2 | 4.927E-02 |
| O00571 | DDX3X | HUMAN | 7 | s_36_m | 1 | 4.927E-02 |
| P08708 | RPS17 | HUMAN | 7 | s_36_m | 1 | 4.927E-02 |

| Protein IDs | gname | species | target | type | background | adj.p |
|---|---|---|---|---|---|---|
| P13010 | XRCC5 | HUMAN | 7 | s_36_m | 1 | 4.927E-02 |
| Q04637;O43432 | EIF4G1 | HUMAN | 7 | s_36_m | 1 | 4.927E-02 |
| Q15084 | PDIA6 | HUMAN | 7 | s_36_m | 1 | 4.927E-02 |
| Q15424 | SAFB | HUMAN | 7 | s_36_m | 1 | 4.927E-02 |
| Q4G0J3 | LARP7 | HUMAN | 7 | s_36_m | 1 | 4.927E-02 |
| Q5BKZ1 | ZNF326 | HUMAN | 7 | s_36_m | 1 | 4.927E-02 |
| Q8N1G4 | LRRC47 | HUMAN | 7 | s_36_m | 1 | 4.927E-02 |
| Q9NVS2 | MRPS18A | HUMAN | 7 | s_36_m | 1 | 4.927E-02 |
| Q9UHX1 | PUF60 | HUMAN | 7 | s_36_m | 1 | 4.927E-02 |

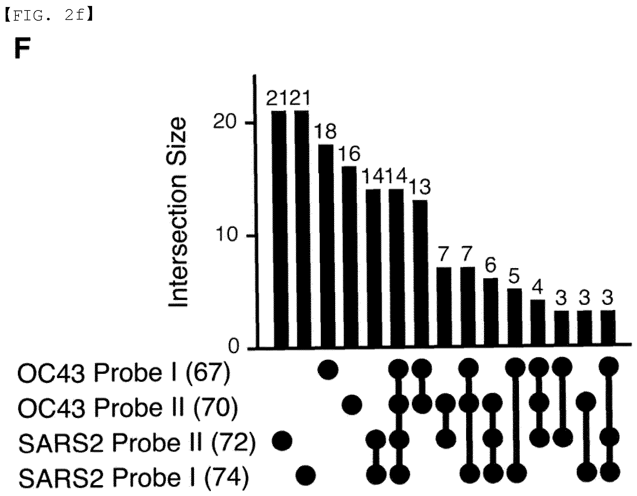

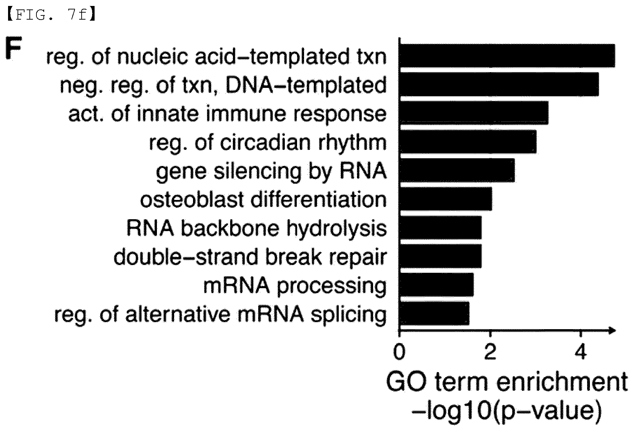

38 proteins were statistically enriched in both probe sets. GO term enrichment analysis revealed that these 38 host proteins are involved in transcriptional regulation, RNA processing, and RNA stability control (FIG. 7f). Of the 38 common proteins, 14 host proteins (CELF1, EIF4H, ELAVL1, FAM120A, FUBP3, IGF2BP3, MATR3, MOV10, NONO, PABPC1, PABPC4, PTBP3, RALY, SND1 and ZC3HAV1) were also found in the SARS-CoV-2 experiments (FIG. 2f), and thus are conserved host components of the betacoronavirus RNA interactome.

## 4. Regulatory landscape of the SARS-CoV-2 RNA interactome

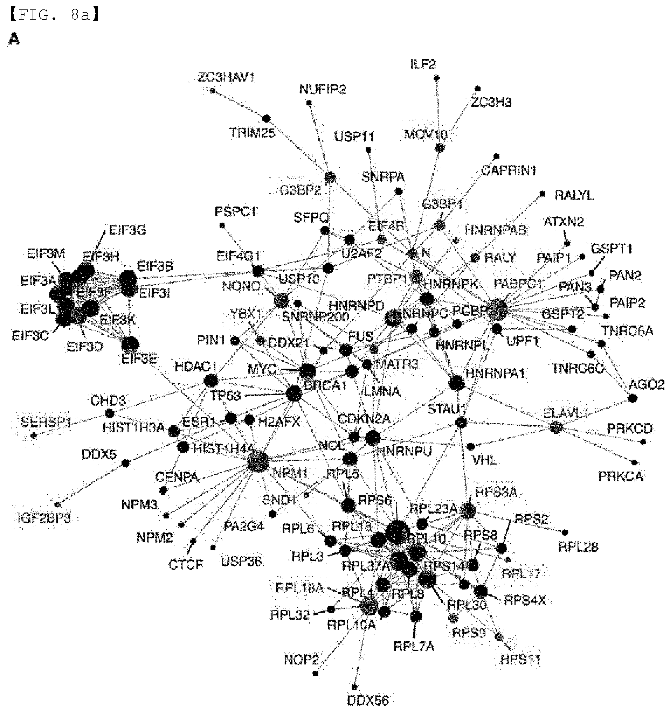

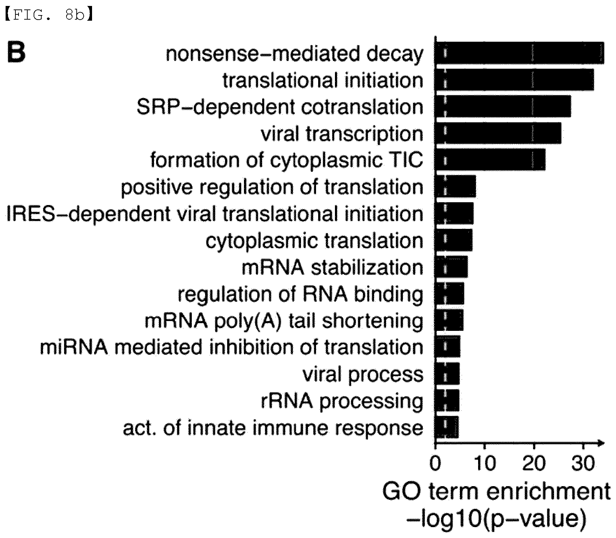

To understand the regulatory significance of the SARS-CoV-2 RNA interactome, we compiled a list of "neighboring" proteins that are known to physically interact with the factors identified in our study (see Methods for details). In particular, we generated a physical interaction network centered (or seeded) by the core SARS-CoV-2 interactome (FIG. 8a). Network analysis revealed several network hubs (e.g. NPM1 and PABPC1) and two highly connected network modules: the ribosomal subunits and the EIF3 complex. GO term enrichment analysis resulted in translation-related biological processes (FIG. 8b), most likely due to the overrepresentation of ribosomal proteins and subunits of the EIF3 complex, which reflects the active translational status of viral mRNPs.

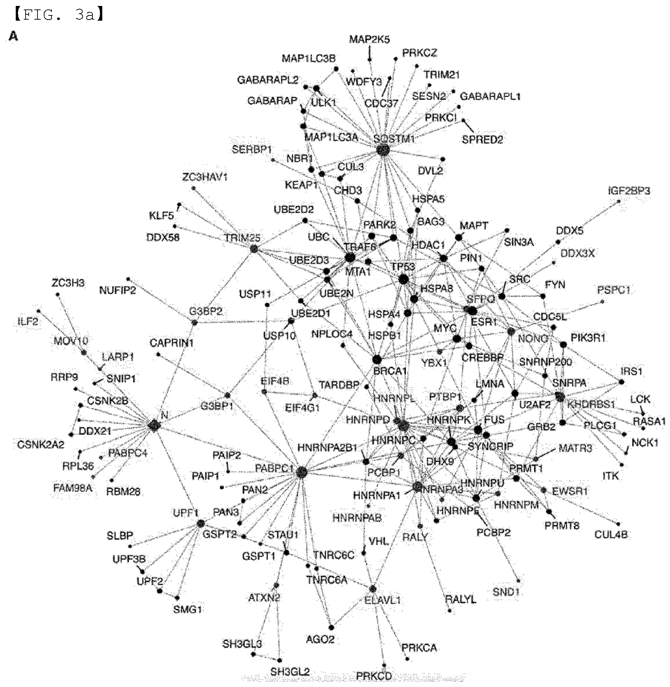

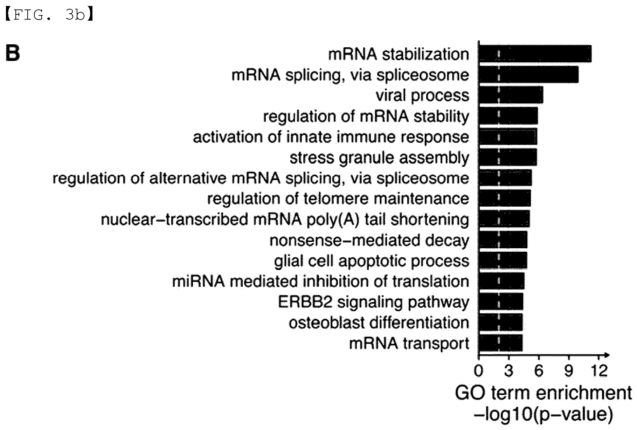

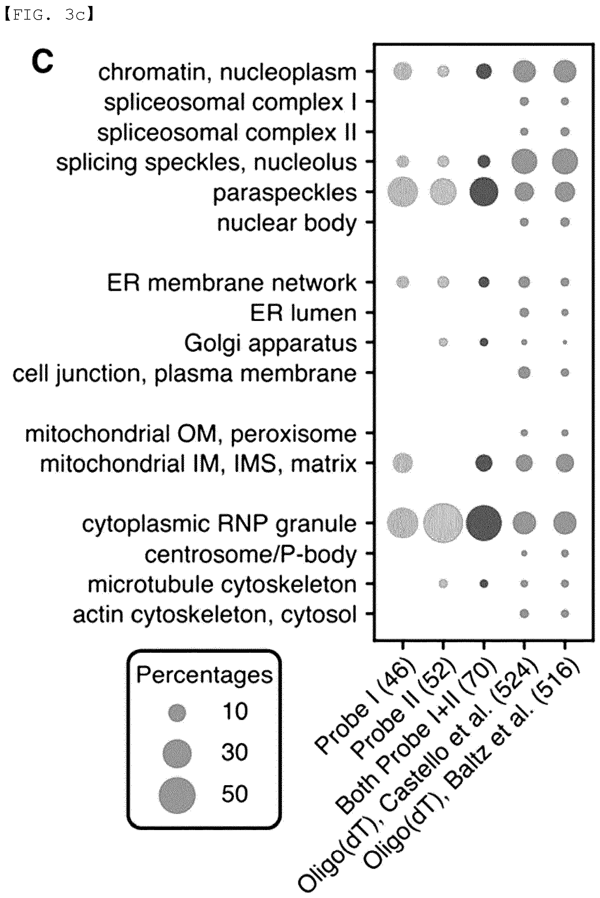

To achieve a more indepth functional perspective of the RNA interactome, we reconstructed the physical interaction network with the SARS-CoV-2 RNA interactome but excluding ribosomal proteins and EIF3 proteins (FIG. 3a). This analysis identified additional hub proteins such as TRIM25, SQSTM1, and KHDRBS1. GO term enrichment analysis revealed multiple steps of the mRNA life cycle such as mRNA splicing, mRNA export, mRNA stability, and stress granule assembly (FIG. 3b), suggesting these mRNA regulators are co-opted to assist the viral life cycle. Interestingly, we also found GO terms related to viral processes and innate immune response. In terms of intracellular localization, the SARS-CoV-2 RNA interactome is enriched by proteins localized in the paraspeckle and cytoplasmic RNP granule (e.g. stress granule) compared to the cellular mRNA interactome (FIG. 3c) (Baltz et al., 2012; Castello et al., 2012). These observations suggest the regulatory mechanisms of viral RNAs distinct from that of host mRNAs, which involve activation of host antiviral machinery and sequestration of viral RNAs.

Another way to gauge the regulatory potential of the SARS-CoV-2 RNA interactome is to examine them in the context of the transcriptional response to viral infection. For example, infected cells recognize the non-cellular RNAs by a number of cytosolic sensors such as RIG-I (DDX58) and MDA5 (IFIH1) and ultimately induces interferons which in turn up-regulates interferon-stimulated genes (ISGs) through the JAK-STAT pathway (Sa Ribero et al., 2020). Multiple studies have reported the unusually low-level of type I/III interferon responses in cell and animal model systems of SARS-CoV-2 infection (Blanco-Melo et al., 2020) and blood biopsy from COVID-19 patients (Hadjadj et al., 2020), indicating active immune evasion by SARS-CoV-2 and supporting the therapeutic potential of timely interferon treatment (Sa Ribero et al., 2020).

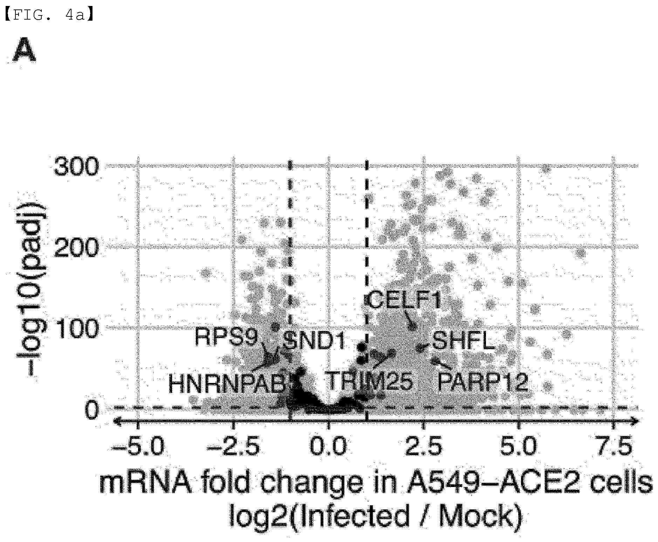

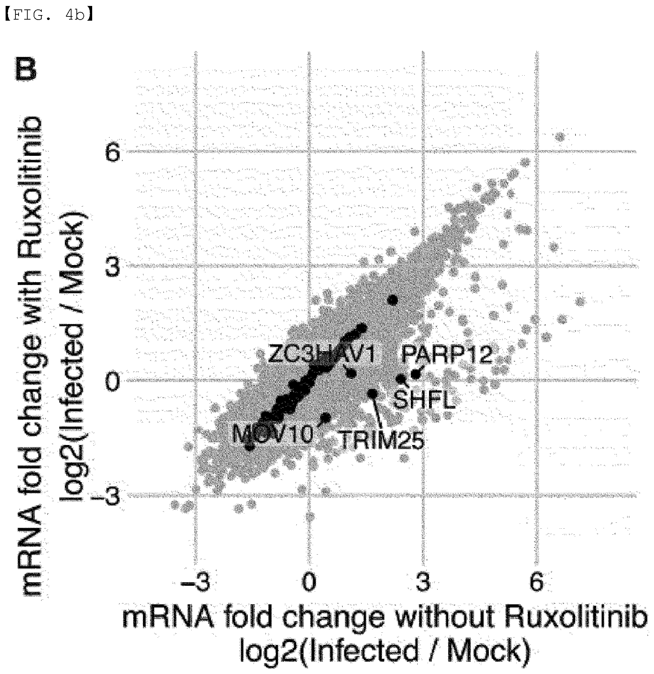

To investigate the regulation of the SARS-CoV-2 RNA interactome, we utilized published transcriptome data of SARS-CoV-2-infected cells (Blanco-Melo et al., 2020). Transcriptome analysis of ACE2-expressing A549 cells revealed host factors of SARS-CoV-2 RNA interactome that are differentially expressed after infection (FIG. 4a). Specifically, PARP12, SHFL, CELF1, and TRIM25 are up-regulated upon infection. Treatment of ruxolitinib, a JAK1 and 2 inhibitor, in infected cells suppressed the expression of 5 host factors (MOV10, PARP12, SHFL, TRIM25, and ZC3HAV1) (FIG. 4b). TRIM25 and PARP12 are part of the 62 vertebrate core ISGs (Shaw et al., 2017).

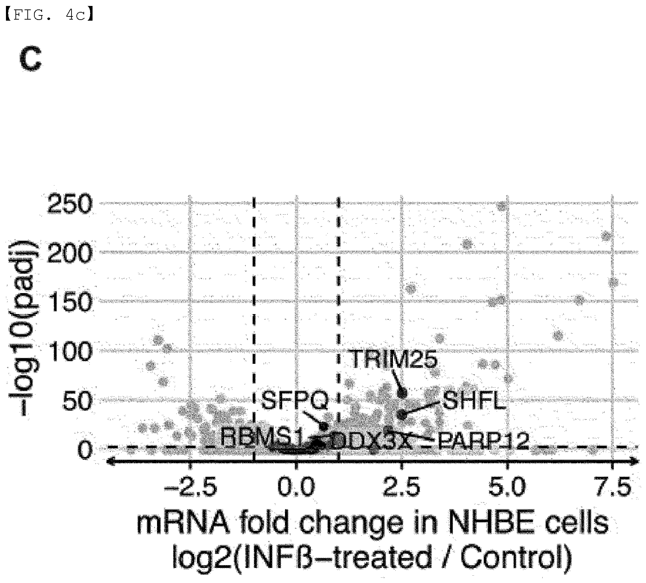

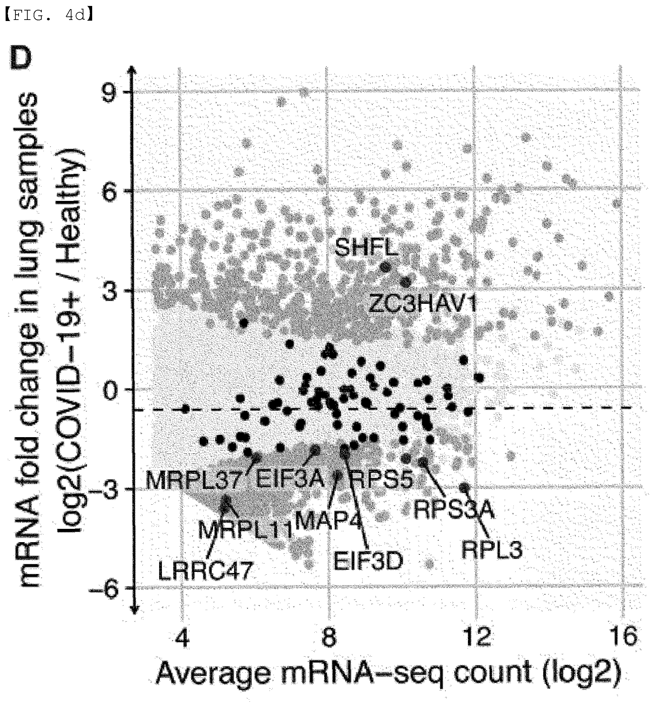

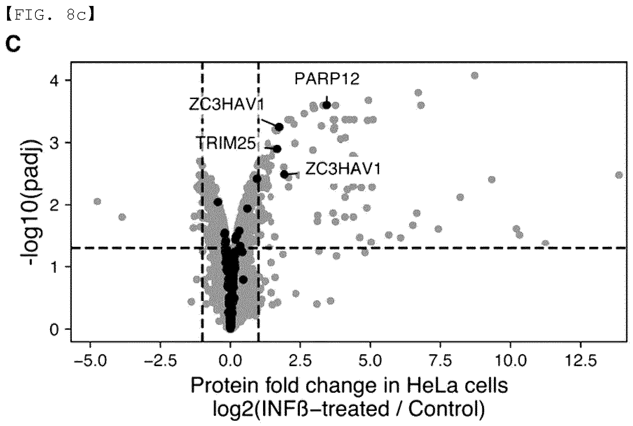

Interferon-beta (INFβ) treatment on normal human bronchial epithelial (NHBE) cells induces PARP12, SHFL, and TRIM25 (FIG. 4c). Consistently, proteome analysis of INFβ-treated cells (Kerr et al., 2020) exhibited an upregulation of PARP12, TRIM25, and ZC3HAV1 (FIG. 8c), altogether indicating that JAK-STAT signaling pathway regulates part of the SARS-CoV-2 RNA interactome. Moreover, reanalysis of mRNA-seq data of postmortem lung samples from COVID-19 patients (Blanco-Melo et al., 2020) showed up-regulation of SHFL and ZC3HAV1 (FIG. 4d), highlighting the physiological relevance of SARS-CoV-2 RNP regulation in natural human infection of SARS-CoV-2.

**5. Host factors and functional modules that regulate the SARS-CoV-2 RNAs**

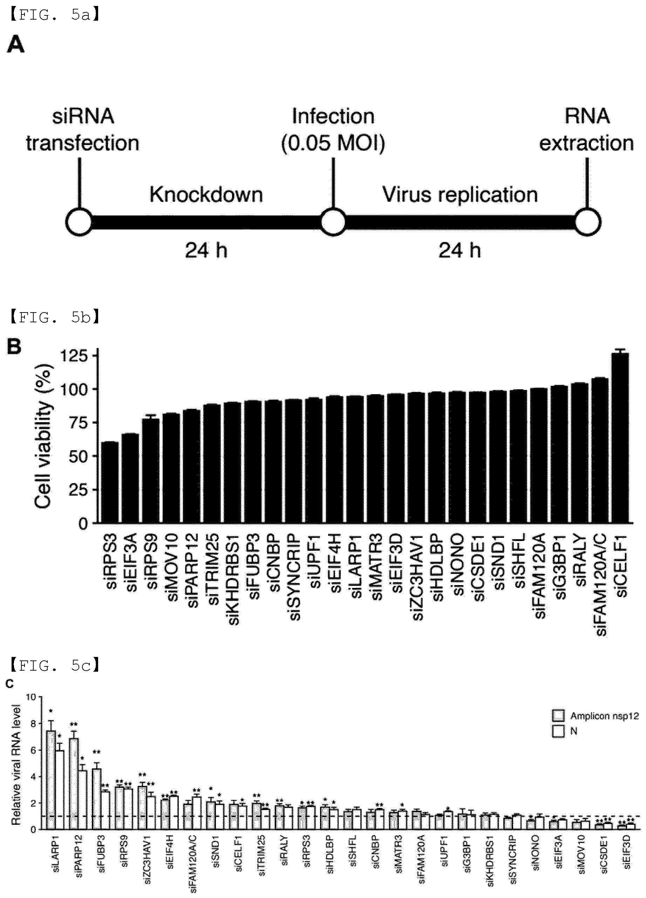

To measure the impact of these host proteins on coronavirus RNAs, we conducted knockdown experiments and infected Calu-3 cells with SARS-CoV-2 (FIG. 5a and 5b). Calu-3 cells are human lung epithelial cells and often used as a model system for coronavirus infection (Sims et al., 2008). Strategically, we selected a subset of the SARS-CoV-2 RNA interactome that covers a broad range of functional modules that we identified above: JAK-STAT signaling, mRNA transport, mRNA stability, and translation. Knockdown of host factors that are stimulated by SARS-CoV-2 infection or INFβ treatment, namely PARP12, TRIM25, ZC3HAV1, CELF1, and SHFL, led to increased viral RNAs (FIG. 5c). The result suggests that these RBPs which directly recognize the viral RNAs are induced by the interferon and JAK-STAT signaling pathway to suppress coronaviruses.

ZC3HAV1 (ZAP/PARP13) is an ISG and known to restrict the replication of many RNA viruses such as HIV-1 (Retroviridae), sindbis virus (Togaviridae), and Ebola (Filoviridae) (Goodier et al., 2015). ZC3HAV1 was previously reported to recognize CpG and recruits decay factors to degrade HIV RNAs (Takata et al., 2017). ZC3HAV1 acts as a cofactor for TRIM25, an E3 ubiquitin ligase that promotes antiviral signaling mainly through RIG-I (Choudhury et al., 2020; Gack et al., 2007). Our knockdown results indicate that both ZC3HAV1 and TRIM25 may act as antiviral factors against SARS-CoV-2 (FIG. 5c). SARS-CoV N protein was previously shown to interact with the SPRY domain of TRIM25, interfering with the activation of RIG-I (Hu et al., 2017). Further investigation is needed to understand how ZC3HAV1 and TRIM25 recognize and suppress SARS-CoV-2 transcripts and if SARS-COV-2 N protein counteracts TRIM25.

PARP12, a cytoplasmic mono-ADP-ribosylation (MARylation) enzyme, is also known to have broad antiviral activity against RNA viruses such as Venezuelan equine encephalitis virus (Togaviridae), vesicular stomatitis virus (Rhabdoviridae), Rift Valley fever virus (Phenuiviridae), encephalomyocarditis virus (Picornaviridae), and Zika virus (Flaviviridae) by multiple mechanisms including blocking cellular RNA translation (Atasheva et al., 2014; Welsby et al., 2014) or triggering proteasome-mediated destabilization of viral proteins (Li et al., 2018). ADP ribosyltransferases are evolutionarily ancient tools used for host-pathogen interactions (Fehr et al., 2020). Of note, coronavirus nsp3 carries a conserved macrodomain that can remove ADPribose to reverse the activity of PARP enzymes (Fehr et al., 2015). Knockdown of PARP12 and PARP14 was shown to increase the replication of the macrodomain-deficient mouse hepatitis virus (MHV) which belongs to the lineage A of genus betacoronavirus (Grunewald et al., 2019) which is consistent with our knockdown results (FIG. 5c). Based on our RNA interactome data, we hypothesize that the RNA-binding activity of PARP12 and its role in viral RNA recognition may explain the underlying molecular mechanism of its antiviral activity against SARS-CoV-2 transcripts.

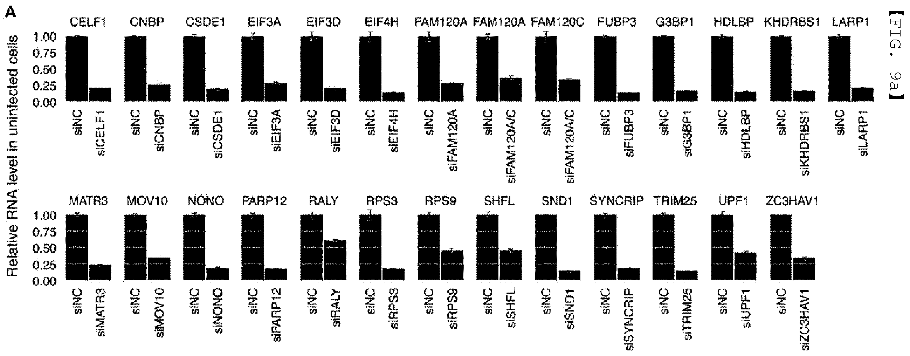

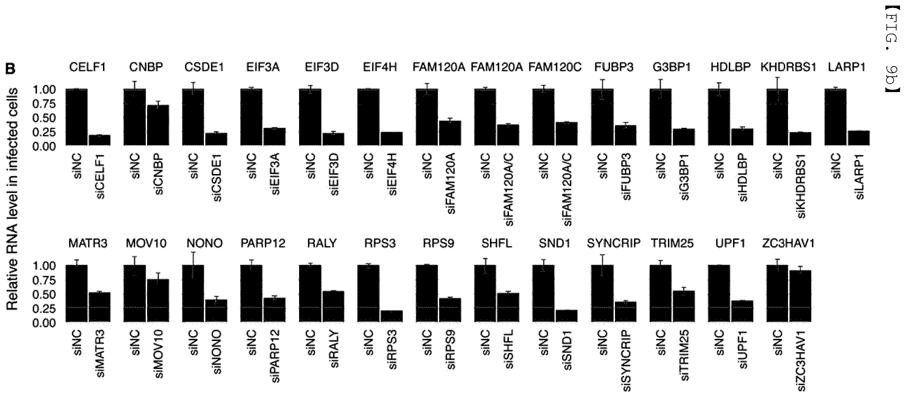

Other interferon-stimulated RBPs may also be involved in host defense against SARS-CoV-2. CELF1 (CUGBP1 Elav-like protein family 1) mediates alternative splicing and controls mRNA stability and translation (Konieczny et al., 2014). CELF1 is required for IFNβ-mediated suppression of simian immunodeficiency virus (Retroviridae) (Dudaronek et al., 2007), but its involvement in other viral infections is unknown. SHFL (Shiftless/RyDEN) was induced prominently upon viral infection and interferon treatment, and suppressed by JAK inhibitor (FIG. 4s). SHFL suppresses the translation of diverse RNA viruses, including dengue virus (Flaviviridae) and HIV (Retroviridae) (Balinsky et al., 2017; Suzuki et al., 2016; Wang et al., 2019). Under our experimental condition, upregulation of viral RNA was only modest in CELF1- and SHFL-depleted cells, but further examination is needed as the knockdown efficiency of ISGs were low in infected cells (FIG. 9b), likely because virus-induced interferon response compromised gene silencing.

Apart from the above RBPs, we identified multiple host factors that have not been previously described in the context of viral infection. In particular, LARP1 depletion resulted in a substantial upregulation of viral RNAs (FIG. 5c), suggesting that LARP1 may have an antiviral function. LARP1 stabilizes 5' TOP mRNAs encoding ribosomal proteins and translation factors, which contain the 5' terminal oligopyrimidine (5' TOP) motif in the 5' UTR (Philippe et al., 2018). LARP1 also represses the translation of 5' TOP mRNAs in response to metabolic stress in an mTORC1-dependent manner (Hong et al., 2017; Lahr et al., 2017). While much is unknown regarding the role of LARP1 during viral infection, a recent proteomics study showed that SARS-CoV-2 N protein binds to LARP1 (Gordon et al., 2020). Based on our RNP capture and knockdown results, it is conceivable that LARP1 may interfere with viral translation directly via viral RNA interaction. The role of N protein in the context of LARP1-dependent viral suppression warrants further investigation. Of note, we cannot exclude the possibility that LARP1 indirectly regulates SARS-CoV-2 RNAs via the control of 5' TOP mRNAs encoding translation machinery.

In fact, the SARS-CoV-2 RNA interactome includes specific components of the 40S and 60S ribosomal subunits and translational initiation factors (FIG. 1f-h). Knockdown experiments indicated that ribosomal proteins (RPS9 and RPS3) and translation initiation factor EIF4H may have antiviral activities (FIG. 5c). EIF4H along with EIF4B is a cofactor for RNA helicase EIF4A (Rogers et al., 2001) whose depletion results in RNA granule formation (Tauber et al., 2020). EIF4H and EIF4B were both identified as the core SARS-CoV-2 RNA interactome (FIG. 1f). EIF4H also interacts with SARS-CoV-2 nsp9 (Gordon et al., 2020), so it will be interesting to investigate the functional consequence of the EIF4H-nsp9 interaction. Together, our observations implicate that SARS-CoV-2 infection may be closely intertwined with the regulation of ribosome biogenesis, metabolic rewiring, and global translational control.

Other translation factors EIF3A, EIF3D, and CSDE1 exhibited proviral effects (FIG. 5c).

EIF3A is the RNA-binding component of the mammalian EIF3 complex and evolutionarily conserved along with EIF3B and EIF3C (Masutani et al., 2007). EIF3D is known to interact with mRNA cap and is required for specialized translation initiation (Lee et al., 2016). CSDE1 (Unr) is required for IRES-dependent translation in human rhinovirus (Picornaviridae) and poliovirus (Picornaviridae) (Anderson et al., 2007; Boussadia et al., 2003). In all, our finding suggests that SARS-CoV-2 may recruit EIF3D and CSDE1 to respectively regulate cap-dependent and IRESdependent translation initiation (Lee et al., 2017) of SARS-CoV-2 gRNA and sgRNAs.

Lastly, the coronaviral RNA interactomes are enriched with RBPs with KH domains (FIG. 6d and FIG. 7b) unlike the mRNA interactome. Depletion of FUBP3 (MARTA2) and HDLBP (Vigilin) increased the viral RNA levels (FIG. 5C), hinting at a potential antiviral role of proteins containing KH domains. HDLBP is a conserved protein that contains 14 KH domains and has been implicated in viral translation of dengue virus (Ooi et al., 2019) . FUBP3 was enriched in all four RNP capture experiments (i.e. SARS-CoV-2 Probe I/II and OC43 Probe I/II) (FIG. 2d). FUBP3 is a nuclear protein with 4 KH domains and binds to the 3' UTR of cellular mRNAs regulating mRNA localization (Blichenberg et al., 1999; Mukherjee et al., 2019). Its connection to the life cycle of coronavirus is unknown to our knowledge.

Our current study reveals a broad-spectrum of antiviral factors such as TRIM25, ZC3HAV1, PARP12, and SHFL and also many RBPs whose functions are unknown in the context of viral infection such as LARP1, FUBP3, FAM120A/C, EIF4H, RPS3, RPS9, SND1, CELF1, RALY, CNBP, EIF3A, EIF3D, and CSDE1. This list of proteins reflects constant host-pathogen interactions and opens new avenues to explore unknown mechanisms of viral life cycle and immune evasion.

Along with proteins regulating RNAs, it would also be interesting to consider the possibility of 'riboregulation' (Hentze et al., 2018) in which RNA controls its interacting proteins. Dengue virus, for example, uses its subgenomic RNA called sfRNA to sequester TRIM25 (Chapman et al., 2014). The sgRNA/gRNA ratio is a critical determinant of epidemic potential of dengue virus (Manokaran et al., 2015). Notably, coronaviruses including SARS-CoV-2 produces substantial amounts of noncanonical sgRNAs that may serve as noncoding decoys to interact with host RBPs to modulate host immune responses (Kim et al., 2020a).

There are over 3,200 human clinical studies listed for the treatment of COVID-19 as of September 2020 (ClinicalTrials.gov), yet there's no effective antiviral drug or vaccine available to stop the ongoing pandemic. This unmet medical need highlights our substantial lack of knowledge of SARS-CoV-2. Thus, redefining antiviral strategies should be contemplated beyond expeditious drug repurposing efforts. So far, collective large and multidisciplinary datasets from viral transcriptome (Kim et al., 2020a), host transcriptional response (Blanco-Melo et al., 2020), ribosome profiling (Finkel et al., 2020), whole proteomics (Bojkova et al., 2020), protein-protein interactions by co-IP (Gordon et al., 2020) and proximity labeling (St-Germain et al., 2020), phosphoproteomics (Bouhaddou et al., 2020), RNA structure (Lan et al., 2020), genome-wide CRISPR screen (Wei et al., 2020), and off-label drug screening (Chen et al., 2020) have all provided invaluable insights of the underlying biology of this novel human coronavirus. In line with these efforts, we here present the SARS-CoV-2 RNA interactome that offers insights into the host-viral interaction that regulate the life cycle of coronaviruses. Data interpretation in the context of publicly available orthogonal information has enabled the identification of proviral and antiviral protein candidates. We expect that further efforts to generate and integrate system-level data will elucidate the pathogenicity of SARS-CoV-2 and introduce new strategies to combat COVID-19.

**References**

Alexa, A., Rahnenf_hrer, J., and Lengauer, T. (2006). Improved scoring of functional groups from gene expression data by decorrelating GO graph structure. Bioinformatics 22, 1600-1607.

Anderson, E.C., Hunt, S.L., and Jackson, R.J. (2007). Internal initiation of translation from the human rhinovirus-2 internal ribosome entry site requires the binding of Unr to two distinct sites on the 5' untranslated region. J. Gen. Virol. 88, 3043-3052.

Atasheva, S., Frolova, E.I., and Frolov, I. (2014). Interferon-stimulated poly(ADP-Ribose) polymerases are potent inhibitors of cellular translation and virus replication. J. Virol. 88, 2116-2130.

Balinsky, C.A., Schmeisser, H., Wells, A.I., Ganesan, S., Jin, T., Singh, K., and Zoon, K.C. (2017). IRAV (FLJ11286), an Interferon-Stimulated Gene with Antiviral Activity against Dengue Virus, Interacts with MOV10. J. Virol. 91.

Baltz, A.G., Munschauer, M., Schwanh_usser, B., Vasile, A., Murakawa, Y., Schueler, M., Youngs, N., Penfold-Brown, D., Drew, K., Milek, M., et al. (2012). The mRNA-bound proteome and its global occupancy profile on protein-coding transcripts. Mol. Cell 46, 674-690.

Blanco-Melo, D., Nilsson-Payant, B.E., Liu, W.-C., Uhl, S., Hoagland, D., Moller, R., Jordan, T.X., Oishi, K., Panis, M., Sachs, D., et al. (2020). Imbalanced Host Response to SARS-CoV-2 Drives Development of COVID-19. Cell 181, 1036-1045.e9.

Blichenberg, A., Schwanke, B., Rehbein, M., Garner, C.C., Richter, D., and Kindler, S. (1999). Identification of a cis-acting dendritic targeting element in MAP2 mRNAs. J. Neurosci. 19, 8818-8829.

Bojkova, D., Klann, K., Koch, B., Widera, M., Krause, D., Ciesek, S., Cinatl, J., and M_nch, C. (2020). Proteomics of SARS-CoV-2-infected host cells reveals therapy targets. Nature 583, 469-472.

Bouhaddou, M., Memon, D., Meyer, B., White, K.M., Rezelj, V.V., Correa Marrero, M., Polacco, B.J., Melnyk, J.E., Ulferts, S., Kaake, R.M., et al. (2020). The Global Phosphorylation Landscape of SARS-CoV-2 Infection. Cell 182, 685-712.e19.

Boussadia, O., Niepmann, M., Cr_ancier, L., Prats, A.-C., Dautry, F., and Jacquemin-Sablon, H. (2003). Unr is required in vivo for efficient initiation of translation from the internal ribosome entry sites of both rhinovirus and poliovirus. J. Virol. 77, 3353-3359.

Bouvet, M., Debarnot, C., Imbert, I., Selisko, B., Snijder, E.J., Canard, B., and Decroly, E. (2010). In vitro reconstitution of SARS-coronavirus mRNA cap methylation. PLoS Pathog. 6, e1000863.

Castello, A., Fischer, B., Eichelbaum, K., Horos, R., Beckmann, B.M., Strein, C., Davey, N.E., Humphreys, D.T., Preiss, T., Steinmetz, L.M., et al. (2012). Insights into RNA biology from an atlas of mammalian mRNA-binding proteins. Cell 149, 1393-1406.

Chapman, E.G., Costantino, D.A., Rabe, J.L., Moon, S.L., Wilusz, J., Nix, J.C., and Kieft, J.S. (2014). The structural basis of pathogenic subgenomic flavivirus RNA (sfRNA) production. Science 344, 307-310.

Chen, C.Z., Shinn, P., Itkin, Z., Eastman, R., Bostwick, R., Rasmussen, L., Huang, R., Shen, M., Hu, X., Wilson, K.M., et al. (2020). Drug Repurposing Screen for Compounds Inhibiting the Cytopathic Effect of SARS-CoV-2. bioRxiv http://dx.doi.org/10.1101/2020.08.18.255877.

Choudhury, N.R., Heikel, G., and Michlewski, G. (2020). TRIM25 and its emerging RNA-binding roles in antiviral defense. WIREs RNA 11, 1.

Chu, C., Zhang, Q.C., da Rocha, S.T., Flynn, R.A., Bharadwaj, M., Calabrese, J.M., Magnuson, T., Heard, E., and Chang, H.Y. (2015). Systematic discovery of Xist RNA binding proteins. Cell 161, 404-416.

Cox, J., and Mann, M. (2008). MaxQuant enables high peptide identification rates, individualized ppb-range mass accuracies and proteome-wide protein quantification. Nat. Biotechnol. 26, 1367-1372.

Cox, J., Neuhauser, N., Michalski, A., Scheltema, R.A., Olsen, J.V., and Mann, M. (2011). Andromeda: a peptide search engine integrated into the MaxQuant environment. J. Proteome Res. 10, 1794-1805.

Dudaronek, J.M., Barber, S.A., and Clements, J.E. (2007). CUGBP1 is required for IFNbeta- mediated induction of dominant-negative CEBPbeta and suppression of SIV replication in macrophages. J. Immunol. 179, 7262-7269.

Egloff, M.-P., Ferron, F., Campanacci, V., Longhi, S., Rancurel, C., Dutartre, H., Snijder, E.J., Gorbalenya, A.E., Cambillau, C., and Canard, B. (2004). The severe acute respiratory syndrome-coronavirus replicative protein nsp9 is a single-stranded RNA-binding subunit unique in the RNA virus world. Proc. Natl. Acad. Sci. U. S. A. 101, 3792-3796.

El-Gebali, S., Mistry, J., Bateman, A., Eddy, S.R., Luciani, A., Potter, S.C., Qureshi, M., Richardson, L.J., Salazar, G.A., Smart, A., et al. (2019). The Pfam protein families database in 2019. Nucleic Acids Res. 47, D427-D432.

Engreitz, J.M., Pandya-Jones, A., McDonel, P., Shishkin, A., Sirokman, K., Surka, C., Kadri, S., Xing, J., Goren, A., Lander, E.S., et al. (2013). The Xist lncRNA exploits three-dimensional genome architecture to spread across the X chromosome. Science 341, 1237973.

Fehr, A.R., Athmer, J., Channappanavar, R., Phillips, J.M., Meyerholz, D.K., and Perlman, S. (2015). The nsp3 macrodomain promotes virulence in mice with coronavirus-induced encephalitis. J. Virol. 89, 1523-1536.

Fehr, A.R., Singh, S.A., Kerr, C.M., Mukai, S., Higashi, H., and Aikawa, M. (2020). The impact of PARPs and ADP-ribosylation on inflammation and host-pathogen interactions. Genes Dev. 34, 341-359.

Finkel, Y., Mizrahi, O., Nachshon, A., Weingarten-Gabbay, S., Yahalom-Ronen, Y., Tamir, H., Achdout, H., Melamed, S., Weiss, S., Israely, T., et al. (2020). The coding capacity of SARS- CoV-2. bioRxiv http://dx.doi.org/10.1101/2020.05.07.082909.

Fung, T.S., and Liu, D.X. (2019). Human Coronavirus: Host-Pathogen Interaction. Annu. Rev. Microbiol. 73, 529-557.

*446Gack, M.U., Shin, Y.C., Joo, C.-H., Urano, T., Liang, C., Sun, L., Takeuchi, O., Akira, S., Chen, Z., Inoue, S., et al. (2007). TRIM25 RING-finger E3 ubiquitin ligase is essential for RIG-I- mediated antiviral activity. Nature 446, 916-920.

Gene Ontology Consortium (2001). Creating the gene ontology resource: design and implementation. Genome Res. 11, 1425-1433.

Gerstberger, S., Hafner, M., and Tuschl, T. (2014). A census of human RNA-binding proteins. Nat. Rev. Genet. 15, 829-845.

Go, C.D., Knight, J.D.R., Rajasekharan, A., Rathod, B., Hesketh, G.G., Abe, K.T., Youn, J.-Y., Samavarchi-Tehrani, P., Zhang, H., Zhu, L.Y., et al. (2019). A proximity biotinylation map of a human cell. bioRxiv https://doi.org/10.1101/796391.

Goodier, J.L., Pereira, G.C., Cheung, L.E., Rose, R.J., and Kazazian, H.H., Jr (2015). The Broad-Spectrum Antiviral Protein ZAP Restricts Human Retrotransposition. PLoS Genet. 11, e1005252.

Gordon, D.E., Jang, G.M., Bouhaddou, M., Xu, J., Obernier, K., White, K.M., O'Meara, M.J., Rezelj, V.V., Guo, J.Z., Swaney, D. L., et al. (2020). A SARS-CoV-2 protein interaction map reveals targets for drug repurposing. Nature 583, 459-468.

de Groot, R.J., Baker, S.C., Baric, R.S., Brown, C.S., Drosten, C., Enjuanes, L., Fouchier, R.A.M., Galiano, M., Gorbalenya, A.E., Memish, Z.A., et al. (2013). Commentary: Middle East Respiratory Syndrome Coronavirus (MERS-CoV): Announcement of the Coronavirus Study Group. J. Virol. 87, 7790-7792.

Grunewald, M.E., Chen, Y., Kuny, C., Maejima, T., Lease, R., Ferraris, D., Aikawa, M., Sullivan, C.S., Perlman, S., and Fehr, A.R. (2019). The coronavirus macrodomain is required to prevent PARP-mediated inhibition of virus replication and enhancement of IFN expression. PLoS Pathog. 15, e1007756.

Hadjadj, J., Yatim, N., Barnabei, L., Corneau, A., Boussier, J., Smith, N., P_r_, H., Charbit, B., Bondet, V., Chenevier-Gobeaux, C., et al. (2020). Impaired type I interferon activity and inflammatory responses in severe COVID-19 patients. Science 369, 718-724.

Hamre, D., and Procknow, J.J. (1966). A new virus isolated from the human respiratory tract. Proc. Soc. Exp. Biol. Med. 121, 190-193.

Hentze, M.W., Castello, A., Schwarzl, T., and Preiss, T. (2018). A brave new world of RNA- binding proteins. Nat. Rev. Mol. Cell Biol. 19, 327-341.

Hong, S., Freeberg, M.A., Han, T., Kamath, A., Yao, Y., Fukuda, T., Suzuki, T., Kim, J.K., and Inoki, K. (2017). LARP1 functions as a molecular switch for mTORC1-mediated translation of an essential class of mRNAs. Elife 6.

Hu, Y., Li, W., Gao, T., Cui, Y., Jin, Y., Li, P., Ma, Q., Liu, X., and Cao, C. (2017). The Severe Acute Respiratory Syndrome Coronavirus Nucleocapsid Inhibits Type I Interferon Production by Interfering with TRIM25-Mediated RIG-I Ubiquitination. J. Virol. 91.

Kerr, C.H., Skinnider, M.A., Andrews, D.D.T., Madero, A.M., Chan, Q.W.T., Stacey, R.G., Stoynov, N., Jan, E., and Foster, L.J. (2020). Dynamic rewiring of the human interactome by interferon signaling. Genome Biol. 21, 140.

Kim, D., Lee, J.-Y., Yang, J.-S., Kim, J.W., Kim, V.N., and Chang, H. (2020a). The Architecture of SARS-CoV-2 Transcriptome. Cell 181, 914-921.e10.

Kim, J.-M., Chung, Y.-S., Jo, H.J., Lee, N.-J., Kim, M.S., Woo, S.H., Park, S., Kim, J.W., Kim, H.M., and Han, M.-G. (2020b). Identification of Coronavirus Isolated from a Patient in Korea with COVID-19. Osong Public Health Res Perspect 11, 3-7.

Konieczny, P., Stepniak-Konieczna, E., and Sobczak, K. (2014). MBNL proteins and their target RNAs, interaction and splicing regulation. Nucleic Acids Res. 42, 10873-10887.

Lahr, R.M., Fonseca, B.D., Ciotti, G.E., Al-Ashtal, H.A., Jia, J.-J., Niklaus, M.R., Blagden, S.P., Alain, T., and Berman, A.J. (2017). La-related protein 1 (LARP1) binds the mRNA cap, blocking eIF4F assembly on TOP mRNAs. Elife 6.

Lai, M.M.C., and Cavanagh, D. (1997). The Molecular Biology of Coronaviruses. In Advances in Virus Research, K. Maramorosch, F.A. Murphy, and A.J. Shatkin, eds. (Academic Press), pp. 1-100.

Lai, M.M., and Stohlman, S.A. (1981). Comparative analysis of RNA genomes of mouse hepatitis viruses. J. Virol. 38, 661-670.

Lan, T.C.T., Allan, M.F., Malsick, L.E., Khandwala, S., Nyeo, S.S.Y., Bathe, M., Griffiths, A., and Rouskin, S. (2020). Structure of the full SARS-CoV-2 RNA genome in infected cells. bioRxiv http://dx.doi.org/10.1101/2020.06.29.178343.

Langmead, B., and Salzberg, S.L. (2012). Fast gapped-read alignment with Bowtie 2. Nat. Methods 9, 357-359.

Lee, F.C.Y., and Ule, J. (2018). Advances in CLIP Technologies for Studies of Protein-RNA Interactions. Mol. Cell 69, 354-369.

Lee, A.S., Kranzusch, P.J., Doudna, J.A., and Cate, J.H.D. (2016). eIF3d is an mRNA cap- binding protein that is required for specialized translation initiation. Nature 536, 96-99.

Lee, K.-M., Chen, C.-J., and Shih, S.-R. (2017). Regulation Mechanisms of Viral IRES-Driven Translation. Trends Microbiol. 25, 546-561.

Li, L., Zhao, H., Liu, P., Li, C., Quanquin, N., Ji, X., Sun, N., Du, P., Qin, C.-F., Lu, N., et al. (2018). PARP12 suppresses Zika virus infection through PARP-dependent degradation of NS1 and NS3 viral proteins. Sci. Signal. 11.

Manokaran, G., Finol, E., Wang, C., Gunaratne, J., Bahl, J., Ong, E.Z., Tan, H.C., Sessions, O.M., Ward, A.M., Gubler, D.J., et al. (2015). Dengue subgenomic RNA binds TRIM25 to inhibit interferon expression for epidemiological fitness. Science 350, 217-221.

Masutani, M., Sonenberg, N., Yokoyama, S., and Imataka, H. (2007). Reconstitution reveals the functional core of mammalian eIF3. EMBO J. 26, 3373-3383.

McHugh, C.A., and Guttman, M. (2018). RAP-MS: A Method to Identify Proteins that Interact Directly with a Specific RNA Molecule in Cells. Methods Mol. Biol. 1649, 473-488.

McHugh, C.A., Chen, C.-K., Chow, A., Surka, C.F., Tran, C., McDonel, P., Pandya-Jones, A., Blanco, M., Burghard, C., Moradian, A., et al. (2015). The Xist lncRNA interacts directly with SHARP to silence transcription through HDAC3. Nature 521, 232-236.

Miknis, Z.J., Donaldson, E.F., Umland, T.C., Rimmer, R.A., Baric, R.S., and Schultz, L.W. (2009). Severe acute respiratory syndrome coronavirus nsp9 dimerization is essential for efficient viral growth. J. Virol. 83, 3007-3018.

Minajigi, A., Froberg, J., Wei, C., Sunwoo, H., Kesner, B., Colognori, D., Lessing, D., Payer, B., Boukhali, M., Haas, W., et al. (2015). Chromosomes. A comprehensive Xist interactome reveals cohesin repulsion and an RNA-directed chromosome conformation. Science 349.

Mukherjee, J., Hermesh, O., Eliscovich, C., Nalpas, N., Franz-Wachtel, M., Ma_ek, B., and Jansen, R.-P. (2019). β-Actin mRNA interactome mapping by proximity biotinylation. Proc. Natl. Acad. Sci. U. S. A. 116, 12863-12872.

Narayanan, K., Huang, C., Lokugamage, K., Kamitani, W., Ikegami, T., Tseng, C.-T.K., and Makino, S. (2008). Severe acute respiratory syndrome coronavirus nsp1 suppresses host gene expression, including that of type I interferon, in infected cells. J. Virol. 82, 4471-4479.

Ooi, Y.S., Majzoub, K., Flynn, R.A., Mata, M.A., Diep, J., Li, J.K., van Buuren, N., Rumachik, N., Johnson, A.G., Puschnik, A.S., et al. (2019). An RNA-centric dissection of host complexes controlling flavivirus infection. Nat Microbiol 4, 2369-2382.

Peiris, J.S.M., Lai, S.T., Poon, L.L.M., Guan, Y., Yam, L.Y.C., Lim, W., Nicholls, J., Yee, W.K.S., Yan, W.W., Cheung, M.T., et al. (2003). Coronavirus as a possible cause of severe acute respiratory syndrome. Lancet 361, 1319-1325.

Perlman, S., and Netland, J. (2009). Coronaviruses post-SARS: update on replication and pathogenesis. Nature Reviews Microbiology 7, 439-450.

Philippe, L., Vasseur, J.-J., Debart, F., and Thoreen, C.C. (2018). La-related protein 1 (LARP1) repression of TOP mRNA translation is mediated through its cap-binding domain and controlled by an adjacent regulatory region. Nucleic Acids Res. 46, 1457-1469.

Ramanathan, M., Porter, D.F., and Khavari, P.A. (2019). Methods to study RNA-protein interactions. Nat. Methods 16, 225-234.

Rogers, G.W., Jr, Richter, N.J., Lima, W.F., and Merrick, W.C. (2001). Modulation of the helicase activity of eIF4A by eIF4B, eIF4H, and eIF4F. J. Biol. Chem. 276, 30914-30922.

*486Roth, A., and Diederichs, S. (2015). Molecular biology: Rap and chirp about X inactivation. Nature 521, 170-171.

Sa Ribero, M., Jouvenet, N., Dreux, M., and Nisole, S. (2020). Interplay between SARS-CoV-2 and the type I interferon response. PLoS Pathog. 16, e1008737.

Shaw, A.E., Hughes, J., Gu, Q., Behdenna, A., Singer, J.B., Dennis, T., Orton, R.J., Varela, M., Gifford, R.J., Wilson, S.J., et al. (2017). Fundamental properties of the mammalian innate immune system revealed by multispecies comparison of type I interferon responses. PLoS Biol. 15, e2004086.

Sims, A.C., Burkett, S.E., Yount, B., and Pickles, R.J. (2008). SARS-CoV replication and pathogenesis in an in vitro model of the human conducting airway epithelium. Virus Res. 133, 33-44.

Snijder, E.J., Decroly, E., and Ziebuhr, J. (2016). The Nonstructural Proteins Directing Coronavirus RNA Synthesis and Processing. Adv. Virus Res. 96, 59-126.

Sola, I., Almaz_n, F., Z_iga, S., and Enjuanes, L. (2015). Continuous and Discontinuous RNA Synthesis in Coronaviruses. Annu. Rev. Virol. 2, 265-288.

Stark, C., Breitkreutz, B.-J., Reguly, T., Boucher, L., Breitkreutz, A., and Tyers, M. (2006). BioGRID: a general repository for interaction datasets. Nucleic Acids Res. 34, D535-D539.

St-Germain, J.R., Astori, A., and Samavarchi-Tehrani, P. (2020). A SARS-CoV-2 BioID-based virus-host membrane protein interactome and virus peptide compendium: new proteomics resources for COVID-19 research. bioRxiv https://doi.org/10.1101/2020.08.28.269175.

Sutton, G., Fry, E., Carter, L., Sainsbury, S., Walter, T., Nettleship, J., Berrow, N., Owens, R., Gilbert, R., Davidson, A., et al. (2004). The nsp9 replicase protein of SARS-coronavirus, structure and functional insights. Structure 12, 341-353.

Suzuki, Y., Chin, W.-X., Han, Q. 'en, Ichiyama, K., Lee, C.H., Eyo, Z.W., Ebina, H., Takahashi, H., Takahashi, C., Tan, B.H., et al. (2016). Characterization of RyDEN (C19orf66) as an Interferon-Stimulated Cellular Inhibitor against Dengue Virus Replication. PLoS Pathog. 12, e1005357.

Takata, M.A., Gon_alves-Carneiro, D., Zang, T.M., Soll, S.J., York, A., Blanco-Melo, D., and Bieniasz, P.D. (2017). CG dinucleotide suppression enables antiviral defence targeting non-self RNA. Nature 550, 124-127.

Tauber, D., Tauber, G., Khong, A., Van Treeck, B., Pelletier, J., and Parker, R. (2020). Modulation of RNA Condensation by the DEAD-Box Protein eIF4A. Cell 180, 411-426.e16.

Thoms, M., Buschauer, R., Ameismeier, M., Koepke, L., Denk, T., Hirschenberger, M., Kratzat, H., Hayn, M., Mackens-Kiani, T., Cheng, J., et al. (2020). Structural basis for translational shutdown and immune evasion by the Nsp1 protein of SARS-CoV-2. Science.

Ule, J., Hwang, H.-W., and Darnell, R.B. (2018). The Future of Cross-Linking and Immunoprecipitation (CLIP). Cold Spring Harb. Perspect. Biol. 10.

UniProt Consortium (2019). UniProt: a worldwide hub of protein knowledge. Nucleic Acids Res. 47, D506-D515.

**EP 4 239 083 A2**

Van Nostrand, E.L., Freese, P., Pratt, G.A., Wang, X., Wei, X., Xiao, R., Blue, S.M., Chen, J.-Y., Cody, N.A.L., Dominguez, D., et al. (2020). A large-scale binding and functional map of human RNA-binding proteins. Nature 583, 711-719.

Vijgen, L., Keyaerts, E., Mo_s, E., Thoelen, I., Wollants, E., Lemey, P., Vandamme, A.-M., and Van Ranst, M. (2005). Complete genomic sequence of human coronavirus OC43: molecular clock analysis suggests a relatively recent zoonotic coronavirus transmission event. J. Virol. 79, 1595-1604.

Wang, X., Xuan, Y., Han, Y., Ding, X., Ye, K., Yang, F., Gao, P., Goff, S.P., and Gao, G. (2019). Regulation of HIV-1 Gag-Pol Expression by Shiftless, an Inhibitor of Programmed -1 Ribosomal Frameshifting. Cell 176, 625-635.e14.

Wei, J., Alfajaro, M., Hanna, R., DeWeirdt, P., and Strine, M. (2020). Genome-wide CRISPR screen reveals host genes that regulate SARS-CoV-2 infection. bioRxivhttps://doi.org/10.1101/2020.06.16.155101.

Welsby, I., Hutin, D., Gueydan, C., Kruys, V., Rongvaux, A., and Leo, O. (2014). PARP12, an interferon-stimulated gene involved in the control of protein translation and inflammation. J. Biol. Chem. 289, 26642-26657.

Woo, P.C.Y., Lau, S.K.P., Lam, C.S.F., Lau, C.C.Y., Tsang, A.K.L., Lau, J.H.N., Bai, R., Teng, J.L.L., Tsang, C.C.C., Wang, M., et al. (2012). Discovery of seven novel Mammalian and avian coronaviruses in the genus deltacoronavirus supports bat coronaviruses as the gene source of alphacoronavirus and betacoronavirus and avian coronaviruses as the gene source of gammacoronavirus and deltacoronavirus. J. Virol. 86, 3995-4008.

Zhou, P., Yang, X.-L., Wang, X.-G., Hu, B., Zhang, L., Zhang, W., Si, H.-R., Zhu, Y., Li, B., Huang, C.-L., et al. (2020). A pneumonia outbreak associated with a new coronavirus of probable bat origin. Nature 579, 270-273.

**Claims**

1. An antiviral composition comprising one or more proteins selected from the group consisting of LARP1, FUBP3, FUBP1, FAM120A, FAM120C, EIF4H, RPS3, RPS9, SND1, CELF1, RALY, CNBP, TRIM25, ZC3HAV1, PARP12, PPP1CA, HDLBP, CNBP, TIAL, UPF1, and SHFL as an active ingredient.

2. An antiviral composition comprising nucleic acids encoding one or more proteins selected from the group consisting of LARP1, FUBP3, FUBP1, FAM120A, FAM120C, EIF4H, RPS3, RPS9, SND1, CELF1, RALY, CNBP, TRIM25, ZC3HAV1, PARP12, PPP1CA, HDLBP, CNBP, TIAL, UPF1, and SHFL as an active ingredient.

3. The antiviral composition of claim 1, wherein the virus is a coronavirus.

4. The antiviral composition of claim 3, wherein the coronavirus is selected from the group consisting of 229E, OC43, NL63, HKU1, MERS-CoV, SARS-CoV and SARS-CoV-2.

5. An antiviral composition comprising a recombinant vector that contains nucleic acids encoding one or more proteins selected from the group consisting of LARP1, FUBP3, FUBP1, FAM120A, FAM120C, EIF4H, RPS3, RPS9, SND1, CELF1, RALY, CNBP, TRIM25, ZC3HAV1, PARP12, PPP1CA, HDLBP, CNBP, TIAL, UPF1, and SHFL as an active ingredient.

6. An antiviral composition comprising an expression inhibitor or activity inhibitor of one or more proteins selected from the group consisting of EIF3A, EIF3D, CSDE1, HNRNPM, YBX3, FMR1, RTCB, and NUFIP2.

7. The antiviral composition of claim 6, wherein the expression inhibitor of the proteins is selected from the group consisting of miRNA, siRNA, shRNA, antisense oligonucleotide, CRISPRi, and combinations thereof that complementarily bind to the nucleic acid sequences encoding the proteins.

8. The antiviral composition of claim 6, wherein the activity inhibitor of the proteins is selected from the group consisting of antibodies, aptamers, small molecule compounds, and combinations thereof that specifically bind to the proteins.

9. A pharmaceutical composition for the prevention or treatment of coronavirus infection comprising the antiviral composition according to any one of claims 1 to 8.

10. A screening method for a coronavirus inhibitor comprising the following steps:

    (a) contacting coronavirus-infected cells with a candidate substance for a coronavirus inhibitor; and
    (b) analyzing whether the expression level of one or more proteins selected from the group consisting of EIF3A, EIF3D, CSDE1, HNRNPM, YBX3, FMR1, RTCB, and NUFIP2 is reduced compared to a control group of cells not treated with the candidate substance, to determine the candidate substance as a coronavirus inhibitor if the expression level of the proteins is reduced.

11. A method for establishing an RNA interactome comprising the following steps:

(a) inducing the RNA-protein cross-linking by irradiating a sample containing target RNA and protein with UV light;
(b) performing Denaturation by DNase treatment;
(c) capturing denatured RNP complexes in a sequence-specific manner using a biotinylated antisense oligonucleotide probe pool; wherein the biotinylated antisense oligonucleotides included in the probe pool are designed based on the target RNA sequence in a consecutive manner, and have the same length ranging from 45 to 135 nt, and their starting positions are spaced at regular intervals to induce partial overlap between the oligonucleotides;
(d) performing Digestion by on-bead trypsin treatment; and
(e) obtaining interactome information through analysis of the digestion products.

12. The method of claim 11, wherein the target RNA is genomic RNA (gRNA) of an RNA virus.

13. The method of claim 11, wherein the probe pool comprises a first probe pool that specifically hybridizes with a gRNA molecule and a second probe pool that hybridizes with both gRNA and gsRNA.

【FIG. 1a】

A

SARS-CoV-2

Vero cells

RNP capture

24 hpi

① UV (254 nm) — ① UV crosslinking

② RNA — Denatured protein — ② DNase treatment / Denaturation

③ Streptavidin bead — Biotin — ③ Probe hybridization / Affinity purification

④ — ④ Detergent removal / On-bead digestion

LC-MS/MS

【FIG. 1b】

B Target RNA

Antisense oligonucleotides

Biotin

30 nt    90 nt

Probe I (707)    Probe II (275)

Genomic RNA

ORF1ab

Subgenomic RNAs

Probe coverage

【FIG. 1c】

【FIG. 1d】

【FIG. 1e】

【FIG. 1f】

**F**

【FIG. 1g】

【FIG. 1h】

【FIG. 2a】

【FIG. 2b】

**c**

SARS–CoV–2 Probe I+II–enriched

Only SARS–CoV–2 Probe I–enriched

Only SARS–CoV–2 Probe II–enriched

【FIG. 2d】

【FIG. 2e】

【FIG. 2f】

# F

【FIG. 3a】

A

【FIG. 3b】

B

GO term enrichment
−log10(p−value)

【FIG. 3c】

【FIG. 4a】

**A**

【FIG. 4b】

【FIG. 4c】

C

【FIG. 4d】

【FIG. 5a】

**A**

**B**

【FIG. 5c】

**C**

【FIG. 6a】

【FIG. 6b】

【FIG. 6c】

【FIG. 6d】

【FIG. 6e】

E

Replicate — Probe I / Probe II / No probe (Uninfected); Probe I / Probe II / No probe (SARS-CoV-2)

【FIG. 6f】

F

regulation of mRNA stability
regulation of nucleic acid–templated transcription
gene silencing by RNA
positive regulation of translation
mRNA stabilization
viral process
regulation of organelle assembly
positive regulation of mRNA catabolic process
posttranscriptional regulation of gene expression
nucleic acid phosphodiester bond hydrolysis

GO term enrichment
−log10(p−value)

【FIG. 7a】

**A**

【FIG. 7b】

**B**

【FIG. 7c】

【FIG. 7e】

【FIG. 7f】

**F**

GO term enrichment
−log10(p−value)

【FIG. 8a】

**A**

【FIG. 8b】

B

GO term enrichment
−log10(p−value)

【FIG. 8c】

C

Protein fold change in HeLa cells
log2(INFß−treated / Control)

【FIG. 9a】

【FIG. 9b】

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020200144787

**Non-patent literature cited in the description**

- **SCHEIT.** Nucleotide Analogs. John Wiley, 1980
- **UHLMAN ; PEYMAN.** *Chemical Reviews,* 1990, vol. 90, 543-584
- **SMITH ; WATERMAN.** *Adv. Appl. Math.,* 1981, vol. 2, 482
- **NEEDLEMAN ; WUNSCH.** *J. Mol. Bio.,* 1970, vol. 48, 443
- **PEARSON ; LIPMAN.** *Methods in Mol. Biol.,* 1988, vol. 24, 307-31
- **HIGGINS ; SHARP.** *Gene,* 1988, vol. 73, 237-44
- **HIGGINS ; SHARP.** *CABIOS,* 1989, vol. 5, 151-3
- **CORPET et al.** *Nuc. Acids Res.,* 1988, vol. 16, 10881-90
- **HUANG et al.** *Comp. Appl. BioSci.,* 1992, vol. 8, 155-65
- **PEARSON et al.** *Meth. Mol. Biol.,* 1994, vol. 24, 307-31
- **SAMBROOK et al.** Molecular Cloning, A Laboratory Manual. Cold Spring Harbor Laboratory Press, 2001
- *Remington's Pharmaceutical Sciences,* 1995
- **ALEXA, A. ; RAHNENF_HRER, J. ; LENGAUER, T.** Improved scoring of functional groups from gene expression data by decorrelating GO graph structure. *Bioinformatics,* 2006, vol. 22, 1600-1607
- **ANDERSON, E.C. ; HUNT, S.L. ; JACKSON, R.J.** Internal initiation of translation from the human rhinovirus-2 internal ribosome entry site requires the binding of Unr to two distinct sites on the 5' untranslated region. *J. Gen. Virol.,* 2007, vol. 88, 3043-3052
- **ATASHEVA, S. ; FROLOVA, E.I. ; FROLOV, I.** Interferon-stimulated poly(ADP-Ribose) polymerases are potent inhibitors of cellular translation and virus replication. *J. Virol.,* 2014, vol. 88, 2116-2130
- **BALINSKY, C.A. ; SCHMEISSER, H. ; WELLS, A.I. ; GANESAN, S. ; JIN, T. ; SINGH, K. ; ZOON, K.C.** IRAV (FLJ11286), an Interferon-Stimulated Gene with Antiviral Activity against Dengue Virus, Interacts with MOV10. *J. Virol.,* 2017, vol. 91

- **BALTZ, A.G. ; MUNSCHAUER, M. ; SCHWANH_USSER, B. ; VASILE, A. ; MURAKAWA, Y. ; SCHUELER, M. ; YOUNGS, N. ; PENFOLD-BROWN, D. ; DREW, K. ; MILEK, M. et al.** The mRNA-bound proteome and its global occupancy profile on protein-coding transcripts. *Mol. Cell,* 2012, vol. 46, 674-690
- **BLANCO-MELO, D. ; NILSSON-PAYANT, B.E. ; LIU, W.-C. ; UHL, S. ; HOAGLAND, D. ; MOLLER, R. ; JORDAN, T.X. ; OISHI, K. ; PANIS, M. ; SACHS, D. et al.** Imbalanced Host Response to SARS-CoV-2 Drives Development of COVID-19. *Cell,* 2020, vol. 181, 1036-1045
- **BLICHENBERG, A. ; SCHWANKE, B. ; REHBEIN, M. ; GARNER, C.C. ; RICHTER, D ; KINDLER, S.** Identification of a cis-acting dendritic targeting element in MAP2 mRNAs. *J. Neurosci.,* 1999, vol. 19, 8818-8829
- **BOJKOVA, D. ; KLANN, K. ; KOCH, B. ; WIDERA, M. ; KRAUSE, D. ; CIESEK, S. ; CINATL, J. ; M_NCH, C.** Proteomics of SARS-CoV-2-infected host cells reveals therapy targets. *Nature,* 2020, vol. 583, 469-472
- **BOUHADDOU, M. ; MEMON, D. ; MEYER, B. ; WHITE, K.M. ; REZELJ, V.V. ; CORREA MARRERO, M. ; POLACCO, B.J. ; MELNYK, J.E. ; ULFERTS, S. ; KAAKE, R.M. et al.** The Global Phosphorylation Landscape of SARS-CoV-2 Infection. *Cell,* 2020, vol. 182, 685-712
- **BOUSSADIA, O. ; NIEPMANN, M. ; CR_ANCIER, L. ; PRATS, A.-C. ; DAUTRY, F. ; JACQUEMIN-SABLON, H.** Unr is required in vivo for efficient initiation of translation from the internal ribosome entry sites of both rhinovirus and poliovirus. *J. Virol.,* 2003, vol. 77, 3353-3359
- **BOUVET, M. ; DEBARNOT, C. ; IMBERT, I. ; SELISKO, B. ; SNIJDER, E.J. ; CANARD, B. ; DECROLY, E.** In vitro reconstitution of SARS-coronavirus mRNA cap methylation. *PLoS Pathog.,* 2010, vol. 6, e1000863

- **CASTELLO, A. ; FISCHER, B. ; EICHELBAUM, K. ; HOROS, R. ; BECKMANN, B.M. ; STREIN, C. ; DAVEY, N.E. ; HUMPHREYS, D.T. ; PREISS, T. ; STEINMETZ, L.M. et al.** Insights into RNA biology from an atlas of mammalian mRNA-binding proteins. *Cell,* 2012, vol. 149, 1393-1406

- **CHAPMAN, E.G. ; COSTANTINO, D.A. ; RABE, J.L. ; MOON, S.L. ; WILUSZ, J. ; NIX, J.C. ; KIEFT, J.S.** The structural basis of pathogenic subgenomic flavivirus RNA (sfRNA) production. *Science,* 2014, vol. 344, 307-310

- **CHEN, C.Z. ; SHINN, P. ; ITKIN, Z. ; EASTMAN, R. ; BOSTWICK, R. ; RASMUSSEN, L. ; HUANG, R. ; SHEN, M. ; HU, X. ; WILSON, K.M. et al.** Drug Repurposing Screen for Compounds Inhibiting the Cytopathic Effect of SARS-CoV-2. *bioRxiv,* 2020, http://dx.doi.org/10.1101/2020.08.18.255877

- **CHOUDHURY, N.R. ; HEIKEL, G. ; MICHLEWSKI, G.** TRIM25 and its emerging RNA-binding roles in antiviral defense. *WIREs RNA,* 2020, vol. 11, 1

- **CHU, C. ; ZHANG, Q.C. ; DA ROCHA, S.T. ; FLYNN, R.A. ; BHARADWAJ, M. ; CALABRESE, J.M. ; MAGNUSON, T. ; HEARD, E. ; CHANG, H.Y.** Systematic discovery of Xist RNA binding proteins. *Cell,* 2015, vol. 161, 404-416

- **COX, J. ; MANN, M.** MaxQuant enables high peptide identification rates, individualized ppb-range mass accuracies and proteome-wide protein quantification. *Nat. Biotechnol.,* 2008, vol. 26, 1367-1372

- **COX, J. ; NEUHAUSER, N. ; MICHALSKI, A. ; SCHELTEMA, R.A. ; OLSEN, J.V. ; MANN, M.** Andromeda: a peptide search engine integrated into the MaxQuant environment. *J. Proteome Res.,* 2011, vol. 10, 1794-1805

- **DUDARONEK, J.M. ; BARBER, S.A. ; CLEMENTS, J.E.** CUGBP1 is required for IFNbeta- mediated induction of dominant-negative CEBPbeta and suppression of SIV replication in macrophages. *J. Immunol.,* 2007, vol. 179, 7262-7269

- **EGLOFF, M.-P. ; FERRON, F. ; CAMPANACCI, V. ; LONGHI, S. ; RANCUREL, C. ; DUTARTRE, H. ; SNIJDER, E.J. ; GORBALENYA, A.E. ; CAMBILLAU, C. ; CANARD, B.** The severe acute respiratory syndrome-coronavirus replicative protein nsp9 is a single-stranded RNA-binding subunit unique in the RNA virus world. *Proc. Natl. Acad. Sci. U. S. A.,* 2004, vol. 101, 3792-3796

- **EL-GEBALI, S. ; MISTRY, J. ; BATEMAN, A. ; EDDY, S.R. ; LUCIANI, A. ; POTTER, S.C. ; QURESHI, M. ; RICHARDSON, L.J. ; SALAZAR, G.A. ; SMART, A. et al.** The Pfam protein families database in. *Nucleic Acids Res.,* 2019, vol. 47, D427-D432

- **ENGREITZ, J.M. ; PANDYA-JONES, A. ; MCDONEL, P. ; SHISHKIN, A. ; SIROKMAN, K. ; SURKA, C. ; KADRI, S. ; XING, J. ; GOREN, A. ; LANDER, E.S. et al.** The Xist lncRNA exploits three-dimensional genome architecture to spread across the X chromosome. *Science,* 2013, vol. 341, 1237973

- **FEHR, A.R. ; ATHMER, J. ; CHANNAPPANAVAR, R. ; PHILLIPS, J.M. ; MEYERHOLZ, D.K. ; PERLMAN, S.** The nsp3 macrodomain promotes virulence in mice with coronavirus-induced encephalitis. *J. Virol.,* 2015, vol. 89, 1523-1536

- **FEHR, A.R. ; SINGH, S.A. ; KERR, C.M. ; MUKAI, S. ; HIGASHI, H. ; AIKAWA, M.** The impact of PARPs and ADP-ribosylation on inflammation and host-pathogen interactions. *Genes Dev.,* 2020, vol. 34, 341-359

- **FINKEL, Y. ; MIZRAHI, O. ; NACHSHON, A. ; WEINGARTEN-GABBAY, S. ; YAHALOM-RONEN, Y. ; TAMIR, H ; ACHDOUT, H. ; MELAMED, S. ; WEISS, S. ; ISRAELY, T. et al.** The coding capacity of SARS- CoV-2. *bioRxiv,* 2020, http://dx.doi.org/10.1101/2020.05.07.082909

- **FUNG, T.S. ; LIU, D.X.** *Human Coronavirus: Host-Pathogen Interaction. Annu. Rev. Microbiol.,* 2019, vol. 73, 529-557

- **GACK, M.U. ; SHIN, Y.C. ; JOO, C.-H. ; URANO, T. ; LIANG, C. ; SUN, L. ; TAKEUCHI, O. ; AKIRA, S. ; CHEN, Z. ; INOUE, S. et al.** TRIM25 RING-finger E3 ubiquitin ligase is essential for RIG-I- mediated antiviral activity. *Nature,* 2007, vol. 446, 916-920

- Creating the gene ontology resource: design and implementation. *Genome Res.,* 2001, vol. 11, 1425-1433

- **GERSTBERGER, S. ; HAFNER, M. ; TUSCHL, T.** A census of human RNA-binding proteins. *Nat. Rev. Genet.,* 2014, vol. 15, 829-845

- **GO, C.D ; KNIGHT, J.D.R. ; RAJASEKHARAN, A. ; RATHOD, B. ; HESKETH, G.G. ; ABE, K.T. ; YOUN, J.-Y. ; SAMAVARCHI-TEHRANI, P. ; ZHANG, H. ; ZHU, L.Y. et al.** A proximity biotinylation map of a human cell. *bioRxiv,* 2019, https://doi.org/10.1101/796391

- **GOODIER, J.L. ; PEREIRA, G.C. ; CHEUNG, L.E. ; ROSE, R.J. ; KAZAZIAN, H.H., JR.** The Broad-Spectrum Antiviral Protein ZAP Restricts Human Retrotransposition. *PLoS Genet.,* 2015, vol. 11, e1005252

- **GORDON, D.E. ; JANG, G.M. ; BOUHADDOU, M. ; XU, J. ; OBERNIER, K. ; WHITE, K.M. ; O'MEARA, M.J. ; REZELJ, V.V. ; GUO, J.Z. ; SWANEY, D. L. et al.** A SARS-CoV-2 protein interaction map reveals targets for drug repurposing. *Nature,* 2020, vol. 583, 459-468

- **DE GROOT, R.J. ; BAKER, S.C. ; BARIC, R.S. ; BROWN, C.S. ; DROSTEN, C. ; ENJUANES, L. ; FOUCHIER, R.A.M. ; GALIANO, M. ; GORBALEN-YA, A.E. ; MEMISH, Z.A. et al.** Commentary: Middle East Respiratory Syndrome Coronavirus (MERS-CoV): Announcement of the Coronavirus Study Group. *J. Virol.,* 2013, vol. 87, 7790-7792

- **GRUNEWALD, M.E. ; CHEN, Y. ; KUNY, C. ; MAE-JIMA, T. ; LEASE, R. ; FERRARIS, D. ; AIKAWA, M. ; SULLIVAN, C.S. ; PERLMAN, S. ; FEHR, A.R.** The coronavirus macrodomain is required to prevent PARP-mediated inhibition of virus replication and enhancement of IFN expression. *PLoS Pathog.,* 2019, vol. 15, e1007756

- **HADJADJ, J. ; YATIM, N. ; BARNABEI, L. ; COR-NEAU, A. ; BOUSSIER, J. ; SMITH, N. ; P_R_, H. ; CHARBIT, B. ; BONDET, V. ; CHENEVI-ER-GOBEAUX, C. et al.** Impaired type I interferon activity and inflammatory responses in severe COV-ID-19 patients. *Science,* 2020, vol. 369, 718-724

- **HAMRE, D. ; PROCKNOW, J.J.** A new virus isolated from the human respiratory tract. *Proc. Soc. Exp. Biol. Med.,* 1966, vol. 121, 190-193

- **HENTZE, M.W. ; CASTELLO, A. ; SCHWARZL, T. ; PREISS, T.** A brave new world of RNA- binding proteins. *Nat. Rev. Mol. Cell Biol.,* 2018, vol. 19, 327-341

- **HONG, S. ; FREEBERG, M.A. ; HAN, T. ; KAMATH, A. ; YAO, Y. ; FUKUDA, T. ; SUZUKI, T. ; KIM, J.K. ; INOKI, K.** LARP1 functions as a molecular switch for mTORC1-mediated translation of an essential class of mRNAs. *Elife,* 2017, vol. 6

- **HU, Y. ; LI, W. ; GAO, T. ; CUI, Y. ; JIN, Y. ; LI, P. ; MA, Q. ; LIU, X. ; CAO, C.** The Severe Acute Respiratory Syndrome Coronavirus Nucleocapsid Inhibits Type I Interferon Production by Interfering with TRIM25-Mediated RIG-I Ubiquitination. *J. Virol.,* 2017, vol. 91

- **KERR, C.H. ; SKINNIDER, M.A. ; ANDREWS, D.D.T. ; MADERO, A.M. ; CHAN, Q.W.T. ; STACEY, R.G. ; STOYNOV, N. ; JAN, E. ; FOSTER, L.J.** Dynamic rewiring of the human interactome by interferon signaling. *Genome Biol.,* 2020, vol. 21, 140

- **KIM, D. ; LEE, J.-Y. ; YANG, J.-S. ; KIM, J.W. ; KIM, V.N. ; CHANG, H.** The Architecture of SARS-CoV-2 Transcriptome. *Cell,* 2020, vol. 181, 914-921.e10

- **KIM, J.-M. ; CHUNG, Y.-S. ; JO, H.J. ; LEE, N.-J. ; KIM, M.S. ; WOO, S.H. ; PARK, S. ; KIM, J.W. ; KIM, H.M. ; HAN, M.-G.** Identification of Coronavirus Isolated from a Patient in Korea with COVID-19. *Osong Public Health Res Perspect,* 2020, vol. 11, 3-7

- **KONIECZNY, P. ; STEPNIAK-KONIECZNA, E. ; SOBCZAK, K.** MBNL proteins and their target RNAs, interaction and splicing regulation. *Nucleic Acids Res.,* 2014, vol. 42, 10873-10887

- **LAHR, R.M. ; FONSECA, B.D. ; CIOTTI, G.E. ; AL-ASHTAL, H.A. ; JIA, J.-J. ; NIKLAUS, M.R. ; BLAGDEN, S.P. ; ALAIN, T. ; BERMAN, A.J.** La-related protein 1 (LARP1) binds the mRNA cap, blocking eIF4F assembly on TOP mRNAs. *Elife 6.,* 2017

- The Molecular Biology of Coronaviruses. **LAI, M.M.C. ; CAVANAGH, D.** Advances in Virus Research. Academic Press, 1997, 1-100

- **LAI, M.M. ; STOHLMAN, S.A.** Comparative analysis of RNA genomes of mouse hepatitis viruses. *J. Virol.,* 1981, vol. 38, 661-670

- **LAN, T.C.T. ; ALLAN, M.F. ; MALSICK, L.E. ; KHANDWALA, S. ; NYEO, S.S.Y. ; BATHE, M. ; GRIFFITHS, A. ; ROUSKIN, S.** Structure of the full SARS-CoV-2 RNA genome in infected cells. *bioRxiv,* 2020, http://dx.doi.org/10.1101/2020.06.29.178343

- **LANGMEAD, B. ; SALZBERG, S.L.** Fast gapped-read alignment with Bowtie 2. *Nat. Methods,* 2012, vol. 9, 357-359

- **LEE, F.C.Y. ; ULE, J.** Advances in CLIP Technologies for Studies of Protein-RNA Interactions. *Mol. Cell,* 2018, vol. 69, 354-369

- **LEE, A.S. ; KRANZUSCH, P.J. ; DOUDNA, J.A. ; CATE, J.H.D.** eIF3d is an mRNA cap- binding protein that is required for specialized translation initiation. *Nature,* 2016, vol. 536, 96-99

- **LEE, K.-M. ; CHEN, C.-J. ; SHIH, S.-R.** Regulation Mechanisms of Viral IRES-Driven Translation. *Trends Microbiol.,* 2017, vol. 25, 546-561

- **LI, L. ; ZHAO, H. ; LIU, P. ; LI, C. ; QUANQUIN, N. ; JI, X. ; SUN, N. ; DU, P. ; QIN, C.-F. ; LU, N. et al.** PARP12 suppresses Zika virus infection through PARP-dependent degradation of NS1 and NS3 viral proteins. *Sci. Signal,* 2018, vol. 11

- **MANOKARAN, G. ; FINOL, E. ; WANG, C. ; GU-NARATNE, J. ; BAHL, J. ; ONG, E.Z. ; TAN, H.C. ; SESSIONS, O.M. ; WARD, A.M. ; GUBLER, D.J. et al.** Dengue subgenomic RNA binds TRIM25 to inhibit interferon expression for epidemiological fitness. *Science,* 2015, vol. 350, 217-221

- **MASUTANI, M. ; SONENBERG, N. ; YOKOYAMA, S. ; IMATAKA, H.** Reconstitution reveals the functional core of mammalian eIF3. *EMBO J.,* 2007, vol. 26, 3373-3383

- **MCHUGH, C.A. ; GUTTMAN, M.** RAP-MS: A Method to Identify Proteins that Interact Directly with a Specific RNA Molecule in Cells. *Methods Mol. Biol.,* 2018, vol. 1649, 473-488

- **MCHUGH, C.A. ; CHEN, C.-K. ; CHOW, A. ; SUR-KA, C.F. ; TRAN, C. ; MCDONEL, P. ; PAN-DYA-JONES, A. ; BLANCO, M. ; BURGHARD, C. ; MORADIAN, A. et al.** The Xist lncRNA interacts directly with SHARP to silence transcription through HDAC3. *Nature,* 2015, vol. 521, 232-236

- **MIKNIS, Z.J. ; DONALDSON, E.F. ; UMLAND, T.C. ; RIMMER, R.A. ; BARIC, R.S. ; SCHULTZ, L.W.** Severe acute respiratory syndrome coronavirus nsp9 dimerization is essential for efficient viral growth. *J. Virol.,* 2009, vol. 83, 3007-3018
- **MINAJIGI, A. ; FROBERG, J. ; WEI, C. ; SUNWOO, H. ; KESNER, B. ; COLOGNORI, D. ; LESSING, D. ; PAYER, B. ; BOUKHALI, M. ; HAAS, W. et al.** Chromosomes. A comprehensive Xist interactome reveals cohesin repulsion and an RNA-directed chromosome conformation. *Science,* 2015, vol. 349
- **MUKHERJEE, J. ; HERMESH, O. ; ELISCOVICH, C. ; NALPAS, N. ; FRANZ-WACHTEL, M. ; MA_EK, B. ; JANSEN, R.-P.** β-Actin mRNA interactome mapping by proximity biotinylation. *Proc. Natl. Acad. Sci. U. S. A.,* 2019, vol. 116, 12863-12872
- **NARAYANAN, K. ; HUANG, C. ; LOKUGAMAGE, K. ; KAMITANI, W. ; IKEGAMI, T. ; TSENG, C.-T.K. ; MAKINO, S.** Severe acute respiratory syndrome coronavirus nsp1 suppresses host gene expression, including that of type I interferon, in infected cells. *J. Virol.,* 2008, vol. 82, 4471-4479
- **OOI, Y.S. ; MAJZOUB, K. ; FLYNN, R.A. ; MATA, M.A. ; DIEP, J. ; LI, J.K. ; VAN BUUREN, N. ; RUMACHIK, N. ; JOHNSON, A.G. ; PUSCHNIK, A.S. et al.** An RNA-centric dissection of host complexes controlling flavivirus infection. *Nat Microbiol,* 2019, vol. 4, 2369-2382
- **PEIRIS, J.S.M. ; LAI, S.T. ; POON, L.L.M. ; GUAN, Y. ; YAM, L.Y.C. ; LIM, W. ; NICHOLLS, J. ; YEE, W.K.S. ; YAN, W.W. ; CHEUNG, M.T. et al.** Coronavirus as a possible cause of severe acute respiratory syndrome. *Lancet,* 2003, vol. 361, 1319-1325
- **PERLMAN, S. ; NETLAND, J.** Coronaviruses post-SARS: update on replication and pathogenesis. *Nature Reviews Microbiology,* 2009, vol. 7, 439-450
- **PHILIPPE, L. ; VASSEUR, J.-J. ; DEBART, F. ; THOREEN, C.C.** La-related protein 1 (LARP1) repression of TOP mRNA translation is mediated through its cap-binding domain and controlled by an adjacent regulatory region. *Nucleic Acids Res.,* 2018, vol. 46, 1457-1469
- **RAMANATHAN, M. ; PORTER, D.F. ; KHAVARI, P.A.** Methods to study RNA-protein interactions. *Nat. Methods,* 2019, vol. 16, 225-234
- **ROGERS, G.W. ; JR, RICHTER, N.J. ; LIMA, W.F. ; MERRICK, W.C.** Modulation of the helicase activity of eIF4A by eIF4B, eIF4H, and eIF4F. *J. Biol. Chem.,* 2001, vol. 276, 30914-30922
- **ROTH, A. ; DIEDERICHS, S.** Molecular biology: Rap and chirp about X inactivation. *Nature,* 2015, vol. 521, 170-171
- **SA RIBERO, M. ; JOUVENET, N. ; DREUX, M. ; NISOLE, S.** Interplay between SARS-CoV-2 and the type I interferon response. *PLoS Pathog,* 2020, vol. 16, e1008737
- **SHAW, A.E. ; HUGHES, J. ; GU, Q. ; BEHDENNA, A. ; SINGER, J.B. ; DENNIS, T. ; ORTON, R.J. ; VARELA, M. ; GIFFORD, R.J. ; WILSON, S.J. et al.** Fundamental properties of the mammalian innate immune system revealed by multispecies comparison of type I interferon responses. *PLoS Biol.,* 2017, vol. 15, e2004086
- **SIMS, A.C. ; BURKETT, S.E. ; YOUNT, B. ; PICKLES, R.J.** SARS-CoV replication and pathogenesis in an in vitro model of the human conducting airway epithelium. *Virus Res.,* 2008, vol. 133, 33-44
- **SNIJDER, E.J. ; DECROLY, E. ; ZIEBUHR, J.** The Nonstructural Proteins Directing Coronavirus RNA Synthesis. *Processing. Adv. Virus Res.,* 2016, vol. 96, 59-126
- **SOLA, I. ; ALMAZ_N, F. ; Z_IGA, S. ; ENJUANES, L.** Continuous and Discontinuous RNA Synthesis in Coronaviruses. *Annu. Rev. Virol.,* 2015, vol. 2, 265-288
- **STARK, C. ; BREITKREUTZ, B.-J. ; REGULY, T. ; BOUCHER, L. ; BREITKREUTZ, A. ; TYERS, M.** BioGRID: a general repository for interaction datasets. *Nucleic Acids Res.,* 2006, vol. 34, D535-D539
- **ST-GERMAIN, J.R. ; ASTORI, A. ; SAMAVARCHI-TEHRANI, P.** A SARS-CoV-2 BioID-based virus-host membrane protein interactome and virus peptide compendium: new proteomics resources for COVID-19 research. *bioRxiv,* 2020, https://doi.org/10.1101/2020.08.28.269175
- **SUTTON, G. ; FRY, E. ; CARTER, L. ; SAINSBURY, S. ; WALTER, T. ; NETTLESHIP, J. ; BERROW, N. ; OWENS, R. ; GILBERT, R. ; DAVIDSON, A. et al.** The nsp9 replicase protein of SARS-coronavirus, structure and functional insights. *Structure,* 2004, vol. 12, 341-353
- **SUZUKI, Y. ; CHIN, W.-X. ; HAN, Q. 'EN ; ICHIYAMA, K. ; LEE, C.H. ; EYO, Z.W. ; EBINA, H. ; TAKAHASHI, H. ; TAKAHASHI, C. ; TAN, B.H. et al.** Characterization of RyDEN (C19orf66) as an Interferon-Stimulated Cellular Inhibitor against Dengue Virus Replication. *PLoS Pathog,* 2016, vol. 12, e1005357
- **TAKATA, M.A. ; GON_ALVES-CARNEIRO, D. ; ZANG, T.M. ; SOLL, S.J. ; YORK, A. ; BLANCO-MELO, D. ; BIENIASZ, P.D.** CG dinucleotide suppression enables antiviral defence targeting non-self RNA. *Nature,* 2017, vol. 550, 124-127
- **TAUBER, D. ; TAUBER, G. ; KHONG, A. ; VAN TREECK, B. ; PELLETIER, J. ; PARKER, R.** Modulation of RNA Condensation by the DEAD-Box Protein eIF4A. *Cell,* 2020, vol. 180, 411-426
- **THOMS, M. ; BUSCHAUER, R. ; AMEISMEIER, M. ; KOEPKE, L. ; DENK, T. ; HIRSCHENBERGER, M. ; KRATZAT, H. ; HAYN, M. ; MACKENS-KIANI, T. ; CHENG, J. et al.** Structural basis for translational shutdown and immune evasion by the Nsp1 protein of SARS-CoV-2. *Science,* 2020

- The Future of Cross-Linking and Immunoprecipitation (CLIP). **ULE, J. ; HWANG, H.-W. ; DARNELL, R.B.** Perspect. Biol. Cold Spring Harb, 2018, vol. 10
- UniProt: a worldwide hub of protein knowledge. *Nucleic Acids Res.,* vol. 47, D506-D515
- **VAN NOSTRAND, E.L. ; FREESE, P. ; PRATT, G.A. ; WANG, X. ; WEI, X. ; XIAO, R. ; BLUE, S.M. ; CHEN, J.-Y ; CODY, N.A.L. ; DOMINGUEZ, D. et al.** A large-scale binding and functional map of human RNA-binding proteins. *Nature,* 2020, vol. 583, 711-719
- **VIJGEN, L. ; KEYAERTS, E. ; MO_S, E. ; THOELEN, I. ; WOLLANTS, E. ; LEMEY, P. ; VANDAMME, A.-M. ; VAN RANST, M.** Complete genomic sequence of human coronavirus OC43: molecular clock analysis suggests a relatively recent zoonotic coronavirus transmission event. *J. Virol.,* 2005, vol. 79, 1595-1604
- **WANG, X. ; XUAN, Y. ; HAN, Y. ; DING, X. ; YE, K. ; YANG, F. ; GAO, P. ; GOFF, S.P. ; GAO, G.** Regulation of HIV-1 Gag-Pol Expression by Shiftless, an Inhibitor of Programmed -1 Ribosomal Frameshifting. *Cell,* 2019, vol. 176, 625-635.e14

- **WEI, J. ; ALFAJARO, M. ; HANNA, R. ; DEWEIRDT, P. ; STRINE, M.** Genome-wide CRISPR screen reveals host genes that regulate SARS-CoV-2 infection. *bioRxiv,* 2020, https://doi.org/10.1101/2020.06.16.155101
- **WELSBY, I. ; HUTIN, D. ; GUEYDAN, C. ; KRUYS, V. ; RONGVAUX, A. ; LEO, O.** PARP12, an interferon-stimulated gene involved in the control of protein translation and inflammation. *J. Biol. Chem.,* 2014, vol. 289, 26642-26657
- **WOO, P.C.Y. ; LAU, S.K.P. ; LAM, C.S.F. ; LAU, C.C.Y. ; TSANG, A.K.L. ; LAU, J.H.N. ; BAI, R. ; TENG, J.L.L. ; TSANG, C.C.C. ; WANG, M. et al.** Discovery of seven novel Mammalian and avian coronaviruses in the genus deltacoronavirus supports bat coronaviruses as the gene source of alphacoronavirus and betacoronavirus and avian coronaviruses as the gene source of gammacoronavirus and deltacoronavirus. *J. Virol.,* 2012, vol. 86, 3995-4008
- **ZHOU, P. ; YANG, X.-L. ; WANG, X.-G. ; HU, B. ; ZHANG, L. ; ZHANG, W. ; SI, H.-R. ; ZHU, Y. ; LI, B. ; HUANG, C.-L. et al.** A pneumonia outbreak associated with a new coronavirus of probable bat origin. *Nature,* 2020, vol. 579, 270-273